# EUROPEAN PATENT APPLICATION

(11) **EP 1 921 066 A1**
(43) Date of publication of application: **14.05.2008**
(21) Application number: 06796486.6
(22) Date of filing: 10.08.2006
(51) Int. Cl.: C07D 213/80, A61K 31/4418, A61K 31/443, A61K 31/4545, A61K 31/497, A61K 31/5377, A61K 31/5513, A61P 5/24, A61P 13/08, A61P 15/00, A61P 35/00, A61P 43/00, C07D 213/85, C07D 401/10, C07D 401/12, C07D 405/12, C07D 405/14, C07D 409/12, C07D 409/14, C07D 417/12, C07D 417/14

(54) **PYRIDYLPHENOL COMPOUND AND USE THEREOF**

(30) Priority: 11.08.2005 JP 2005232884; 21.12.2005 JP 2005368313
(71) Applicant: Takeda Pharmaceutical Company Limited, Osaka-shi, Osaka 541-0045 (JP)
(72) Inventor: SASAKI, Satoshi, 10, Wadai, Tsukuba-shi, Ibaraki 300-4247 (JP); BABA, Atsuo, 10, Wadai, Tsukuba-shi, Ibaraki 300-4247 (JP)
(74) Representative: Rickard, Timothy Mark Adrian
(86) International application number: PCT/JP2006/316129
(87) International publication number: WO 2007/018319

(57) **Abstract**

The present invention provides a compound which has metastin receptor antagonist activity and is useful for preventing and treating hormone-dependent cancer, benign prostatomegaly, endometriosis, precocious puberty, uterine myoma or the like. More specifically, the present invention provides a compound, represented by the formula: 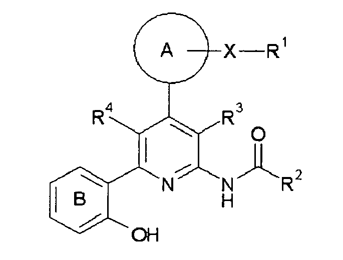 or a salt thereof, a prodrug thereof, and a pharmaceutical agent containing the same; wherein Ring A represents a 5- to 8-membered homocyclic or heterocyclic group optionally having a substituent other than formula -X-R¹ wherein X represents a bond or a spacer, and R¹ represents optionally substituted amino or an optionally substituted nitrogen-containing heterocyclic group; Ring B represents an optionally substituted benzene ring; R² represents an optionally substituted homocyclic or heterocyclic group; and R³ and R⁴ independently represent a hydrogen atom, cyano, acyl or an optionally substituted hydrocarbon group.

## Description

### TECHNICAL FIELD

The present invention relates to, for example, a compound having metastin receptor antagonist activity and a prophylactic or therapeutic function for hormone-dependent cancer or the like.

### BACKGROUND ART

Human metastin (human KiSS-1 peptide) is a peptide having 54 amino acids purified from human placenta, and is a ligand to human OT7T175 (GPR54), which is a G protein-coupled receptor (WO 00/24890). Rat metastin and mouse metastin have also been reported (WO 01/75104). Metastin has cancer metastasis suppression activity, and so is useful for preventing or treating cancer (e.g., lung cancer, gastric cancer, liver cancer, pancreatic cancer, colorectal cancer, rectal cancer, colon cancer, prostate cancer, ovary cancer, cervical cancer, breast cancer, renal cancer, bladder cancer, brain tumor, etc.). Metastin has pancreas function regulating activity, and so is useful for preventing or treating pancreas diseases (e.g., acute or chronic pancreatitis, pancreatic cancer, etc.). Metastin has placenta function regulating activity, and so is useful for preventing or treating choriocarcinoma, hydatidiform mole, invasive mole, miscarriage, fetal hypoplasia, abnormal sugar metabolism, abnormal lipid metabolism, or abnormal parturition (WO 00/24890, WO 01/75104, etc.). Metastin has a gonadotropic hormone secretion promoting or suppressing function, a sex hormone secretion promoting or suppressing function, a gonadal function improving function, an ovulation inducing or promoting function, a sex maturing function and the like, and so is useful as a gonadal function improving agent, an ovulation inducer or promoter, a gonadotropic hormone secretion promoter, a gonadotropic hormone secretion suppressor, a sex hormone secretion promoter, a sex hormone secretion suppressor, or the like; more specifically, as a prophylactic or therapeutic agent for infertility, hormone-sensitive cancer, endometriosis or the like, a follicle maturing inhibitor, a menstrual cycle-suspending agent or the like (WO 2004/080479).

As a pyridylphenol compound, the following compounds are known.
(1) WO 04/58716 describes the following compound as a KISS-1 antagonist, which is useful for treating pain, urinary organ dysfunction, urinary organ inflammation or the like. 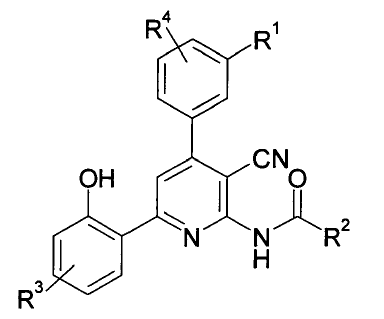 wherein
   R¹ represents amino or -NHC(= O)R⁵; R² represents a 5- or 6-membered aromatic heterocyclic group optionally having 1 to 3 substituent(s) selected from halogen, hydroxy, amino, nitro, cyano, hydroxycarbonyl, alkyl, alkoxy, alkylamino, alkoxycarbonyl, aminocarbonyl and alkylaminocarbonyl; R³ represents a hydrogen atom, halogen, hydroxy, amino, nitro, cyano, hydroxycarbonyl, alkyl, alkoxy, alkylamino, alkoxycarbonyl, aminocarbonyl or alkylaminocarbonyl; R⁴ represents a hydrogen atom, halogen, hydroxy, amino, nitro, cyano, hydroxycarbonyl, alkyl, alkoxy, alkylamino, alkoxycarbonyl, aminocarbonyl or alkylaminocarbonyl; and R⁵ represents alkyl, alkoxy, alkylamino, alkoxycarbonyl, aminocarbonyl or 5-oxotetrahydrofuran-2-yl (wherein alkyl may optionally have 1 to 3 substituent(s) selected from amino, hydroxy, alkoxy, alkylamino, alkoxycarbonyl, aminocarbonyl and alkylaminocarbonyl).
(2) WO 02/44153 describes the following compound which inhibits NF-κB activity by inhibition of IκB kinase β (IKK-β) and thus is useful for preventing or treating NF-κB activity-related diseases, especially inflammation diseases. 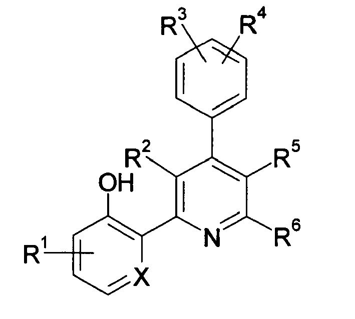 wherein
   X represents CH or N; R¹ represents H, hydroxy, etc.; R² represents H, hydroxy, halogen or C₁₋₆ alkyl; R³ represents H, amino, etc.; R⁴ represents H, -CO-NHR⁴¹, -NH-COR⁴¹, etc.; R⁴¹ represents C₁₋₆ alkyl, -NHR^{41c}, etc.; R^{41c} represents carboxy, etc.; R⁵ represents H, cyano, carboxy, etc.; R⁶ represents -NR⁶¹R⁶²; R⁶¹ represents H; C₁₋₆ alkyl, R⁶² represents H, C₁₋₆ alkyl, phenyl, benzyl or C₁₋₆ alkanoyl; and NR⁶¹R⁶² represents a saturated 5- or 6-membered ring optionally having NH or O other than N, which is bonded to R⁶¹ and R⁶².
(3) WO 02/24679 describes the following compound which inhibits NF-κB activity by inhibition of IκB kinase β (IKK-β) and thus is useful for preventing or treating NF-κB activity-related diseases, especially inflammation diseases. 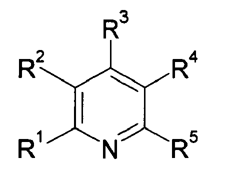 wherein R¹ represents 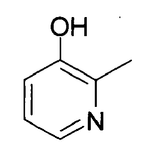 or the like; R² represents H or halogen; R³ represents H, 1,2,3,6-tetrahydropyridine or; CR³¹R³²R³³, R³¹ represents H or C₁₋₆ alkyl; R³² represents H, α-aminobenzyl, a saturated 5- to 8-membered ring, etc.; R³³ represents H, aminobenzyl, piperidinyl-C₁₋₆ alkylcarbonylamino, etc.; R³² and R³³ represent saturated a 5- to 8-membered ring, etc.; R⁴ represents cyano, etc.; R⁵ represents -NR⁵¹R⁵²; R⁵¹ represents H or C₁₋₆ alkyl; R⁵² represents H, C₁₋₆ alkyl, phenyl, benzyl or C₁₋₆ alkanoyl; NR⁵¹R⁵² represents a saturated 5- or 6-membered ring optionally having NH or O other than N, which is bonded to R⁵¹ and R⁵²; and R⁴ and R⁵ represent -R⁴⁰-CO-NH, etc.

### DISCLOSURE OF THE INVENTION

A prophylactic or therapeutic agent for hormone-dependent cancer, endometriosis, and the like, which is safer than, and superior to, the conventionally used prophylactic or therapeutic agents for such diseases are desired.

As a result of intensive various studies in order to solve the above-described problem, the present inventors synthesized, for the first time in history, a compound represented by the formula: 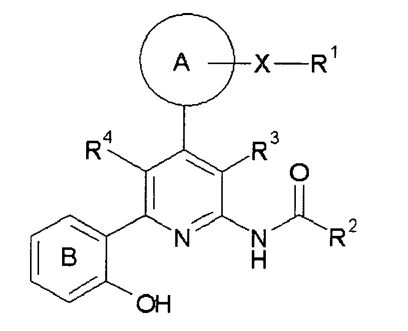 or a salt thereof (hereinafter, occasionally referred to as "compound (I)", wherein
Ring A represents a 5- to 8-membered homocyclic or heterocyclic group optionally having a substituent other than a group represented by formula -X-R¹ wherein X represents a bond or a spacer, and R¹ represents optionally substituted amino or an optionally substituted nitrogen-containing heterocyclic group; and the group represented by formula -X-R¹ may be located at any position on Ring A;
Ring B represents a benzene ring optionally having a substituent other than hydroxy;
R² represents an optionally substituted homocyclic or heterocyclic group; and
R³ and R⁴ independently represent a hydrogen atom, cyano, acyl or an optionally substituted hydrocarbon group;
with proviso that the compound or the salt thereof excludes N-[4-(3-aminophenyl)-3-cyano-6-(2-hydroxyphenyl)pyridin-2-yl]thiophene-2-carboxamide, [(3-{3-cyano-6-(2-hydroxyphenyl)-2-[(2-thienylcarbonyl)amino] pyridin-4-yl}phenyl) amino](oxo)acetic acid ethyl ester, N-(3-cyano-6-(2-hydroxyphenyl)-4- {3-[methoxyacetyl)amino]phenyl}pyridin-2-yl)thiophene-2-carboxamide, N-(3-{3-cyano-6-(2-hydroxyphenyl)-2-[(2-thienylcarbonyl)amino] pyridin-4-yl}phenyl)-5-oxotetrahydrofuran-2-carboxamide, N-[3-(cyano-6-(2-hydroxyphenyl)-4-(3-{[(5-oxotetrahydrofuran-2-yl)carbonyl]amino}phenyl) pyridin-2-yl]isoxazole-5-carboxamide, and N-[3-cyano-6-(2-hydroxyphenyl)-4-(3-{[(5-oxotetrahydrofuran-2-yl)carbonyl]amino}phenyl)pyridin-2-yl]-2-furanamide). The above compound has a chemical structural feature in including a group represented by formula -X-R¹, wherein X represents a bond or a spacer, and R¹ represents optionally substituted amino or an optionally substituted nitrogen-containing heterocyclic group, in Ring A of the structure represented by the formula: 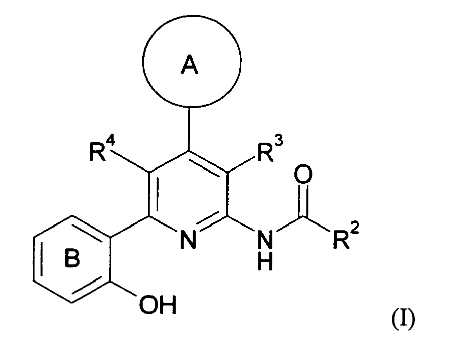 wherein
Ring A represents an optionally substituted 5- to 8-membered homocyclic or heterocyclic group;
Ring B represents a benzene ring optionally having a substituent other than hydroxy;
R² represents an optionally substituted homocyclic or heterocyclic group; and
R³ and R⁴ independently represent a hydrogen atom, cyano, acyl or an optionally substituted hydrocarbon group. The present inventors unexpectedly found that compound (I) has superb metastin receptor antagonist activity, gonadotropic hormone secretion suppression activity, sex hormone secretion suppression activity, and the like based on a specific chemical structure thereof, and that the physical properties of the compound are improved by substitutied Ring A with a group represented by formula -X-R¹. The present inventors also found that a compound represented by the formula: 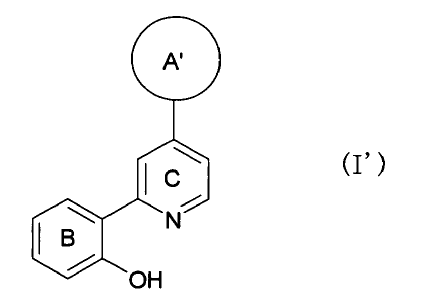 or a salt thereof (hereinafter, occasionally referred to as "compound (I')", wherein
Ring A' represents an optionally substituted 5- to 8-membered homocyclic or heterocyclic group;
Ring B represents a benzene ring optionally having a substitutent other than hydroxy; and
Ring C represents an optionally substituted or optionally condensed pyridine ring,
also unexpectedly has superb metastin receptor antagonist activity, gonadotropic hormone secretion suppression activity, sex hormone secretion suppression activity, and the like based on a specific chemical structure thereof, and is useful as a safe and superb medicament such as a gonadal function regulator or the like, and also as a prophylactic or therapeutic agent for infertility, hormone-sensitive cancer, endometriosis, precocious puberty, uterine myoma or the like. Based on such a discovery, the present inventors completed the present invention.

The present invention relates to, for example, the following.
[1] Compound (I);
[2] The compound according to [1], wherein Ring A is a benzene ring optionally having halogen;
[3] The compound according to [1], wherein the group of the formula: 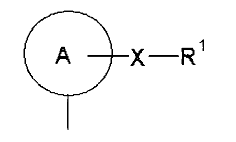 is a group represented by the formula: 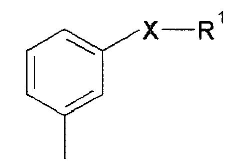 wherein each letter has the same significance as above;
[4] The compound according to [1], wherein X is a group represented by the formula: 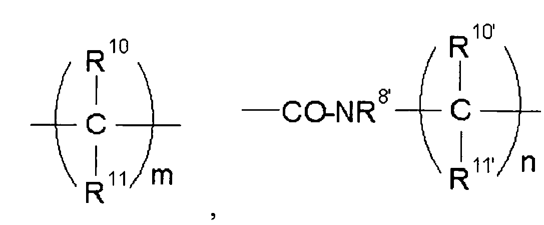 or 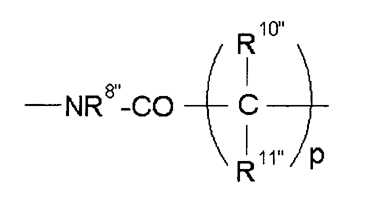 wherein
   R^{8'}, R^{8"}, R¹⁰, R^{10'}, R^{10"}, R¹¹, R^{11'} and R^{11"} independently represent a hydrogen atom, an optionally substituted hydrocarbon group, or an optionally substituted heterocyclic group;
   m represents an integer of 1 to 8; and
   n and p independently represent an integer of 0 to 6 with proviso that if m, n and p are each 2 or greater, R¹⁰, R¹¹, R^{10'}, R^{11'}, R^{10"} and R^{11"} in the repeat unit may each be the same or different;
[5] The compound according to [1], wherein R¹ is an optionally substituted 5- to 7-membered nitrogen-containing heterocyclic group;
[6] The compound according to [1], wherein R¹ is an optionally substituted non-aromatic, 5- to 7-membered nitrogen-containing heterocyclic group;
[7] The compound according to [5], wherein X is a group represented by a bond or the formula: 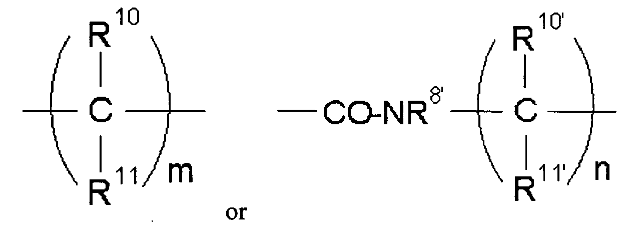 wherein
   R^{8'}, R¹⁰, R^{10'}, R¹¹ and R^{11'} independently represent a hydrogen atom, an optionally substituted hydrocarbon group, or an optionally substituted heterocyclic group;
   m represents an integer of 1 to 8; and
   n represents an integer of 0 to 6 with proviso that if m and n are each 2 or greater, R¹⁰, R¹¹, R^{10'} and R^{11'} in the repeat unit may each be the same or different;
[8] The compound according to [1], wherein Ring B is a benzene ring having halogen other than hydroxy;
[9] The compound according to [1], wherein the group of the formula: 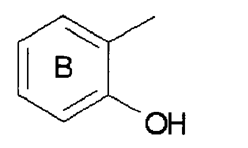 is a group represented by the formula: 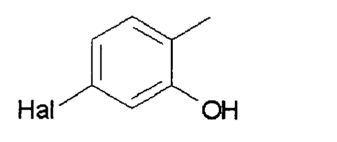 wherein Hal represents halogen;
[9a] The compound according to [1], wherein R¹ is an optionally substituted 5- to 7-membered nitrogen-containing heterocyclic group;
[10] The compound according to [1], wherein R² is an optionally substituted aromatic group;
[11] The compound according to [1], wherein R³ is cyano;
[12] The compound according to [1], which is represented by the formula: 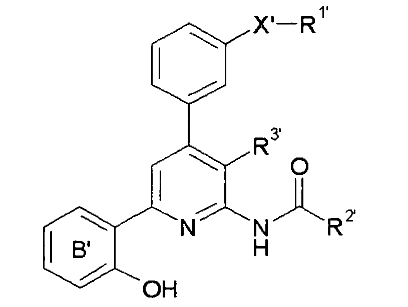 wherein Ring B' represents a benzene ring optionally having halogen other than hydroxy;
   X' represents a group expressed by a bond or the formula: 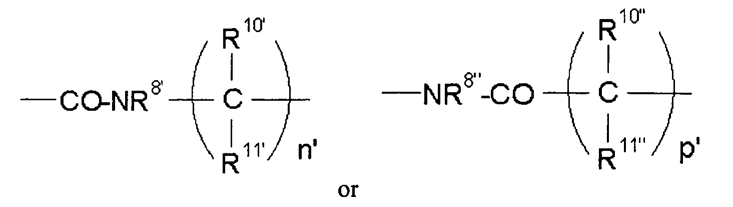 wherein R^{8'}, R^{8"}, R^{10'}, R^{10"}, R^{11'} and R^{11"} independently represent a hydrogen atom, an optionally substituted hydrocarbon group, or an optionally substituted heterocyclic group; and
   n' and p' independently represent an integer of 1 to 4 with proviso that if n' and p' are each 2 or greater, R^{10'}, R^{11'} R^{10"} and R^{11"} in the repeat unit may each be the same or different;
   R^{1'} represents optionally substituted amino or an optionally substituted 5- to 7-membered nitrogen-containing heterocyclic group;
   R^{2'} represents an optionally substituted 5- to 10-membered homocyclic or heterocyclic group; and
   R^{3'} represents a hydrogen atom or cyano;
[13] The compound according to [12], which is represented by the formula: 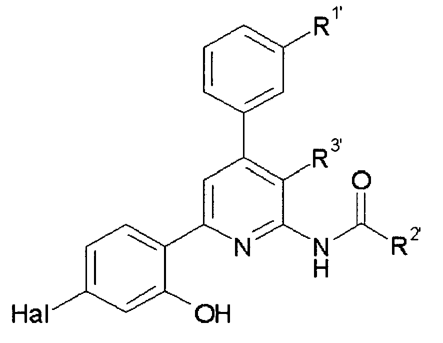 wherein Hal represents halogen, and R^{1'} represents an optionally substituted 5- to 7-membered nitrogen-containing heterocyclic group;
[14] N-[4-(3-{[(2-aminoethyl)amino]carbonyl}phenyl)-3-cyano-6-(4-fluoro-2-hydroxyphenyl)pyridin-2-yl]thiophene-2-carboxamide hydrochloride;
   N-[4-[3-({[(2S)-2-aminopropyl]amino} carbonyl)phenyl]-6-(4-chloro-2-hydroxyphenyl)-3-cyanopyridin-2-yl]isoxazole-5-carboxamide hydrochloride;
   N-[3-cyano-6-(4-fluoro-2-hydroxyphenyl)-4-(3-piperazin-1-ylphenyl) pyridin-2-yl]-2-furamide hydrochloride;
   N-[3-cyano-6-(4-fluoro-2-hydroxyphenyl)-4-(3-(((2R)pyrrolidin-2-ylacetyl) amino)phenyl)pyridin-2-yl]-2-furamide hydrochloride; or
   N-[3-cyano-4-[3-(1,4-diazepan-1-ylmethyl)phenyl]-6-(4-fluoro-2-hydroxyphenyl)pyridin-2-yl]-2-furamide dihydrochloride;
[15] A prodrug of the compound according to [1];
[16] A pharmaceutical agent comprising the compound according to [1] or a prodrug thereof;
[17] The pharmaceutical agent according to [16], which is a metastin receptor antagonist;
[18] The pharmaceutical agent according to [17], which is a gonadal function regulator;
[19] The pharmaceutical agent according to [17], which is a gonadotropic hormone secretion suppressor and/or sex hormone secretion suppressor;
[20] The pharmaceutical agent according to [16], which is a prophylactic or therapeutic agent for hormone-dependent cancer, benign prostatomegaly, endometriosis, precocious puberty or uterine myoma;
[21] A gonadal function regulator, comprising the compound (I'); and
[22] A medicament according to [21], which is a gonadotropic hormone secretion suppressor and/or sex hormone secretion suppressor.

Compounds (I) and (I') (hereinafter, may be simply referred to as a "compound according to the present invention" collectively) have, based on a specific chemical structure thereof, has superb metastin receptor (protein comprising an amino acid sequence represented by SEQ ID NO: 1, 3 or 5, a partial peptide thereof, or a salt thereof, etc.) antagonist activity, gonadotropic hormone (e.g., FSH, LH, etc.) secretion suppression activity, sex hormone [e.g., androgen (e.g., testosterone, androstenedione, etc.), estrogen (e.g., estradiol, estrone, etc.), progesterone, etc.] secretion suppression activity, and the like; is low in toxicity; and has very little side effects. Therefore, a compound according to the present invention or a prodrug thereof is useful as a safe pharmaceutical agent; for example, a metastin receptor antagonist (including an inverse agonist and a partial agonist), a gonadal function regulator, a gonadotropic hormone secretion suppressor, a sex hormone secretion suppressor, an ovulation inhibitor, an ovarium function regulator, or the like; more specifically, for example, a medicament such as a prophylactic or therapeutic agent for hormone-dependent cancer (e.g., prostate cancer, breast cancer, ovarian cancer, endometrial cancer, etc.), benign prostatomegaly (BPH), infertility, endometriosis, precocious puberty, uterine myoma or the like, a contraceptive, a follicle maturing inhibitor, a menstrual cycle-suspending agent or the like.

A compound according to the present invention or a prodrug thereof is also useful for regulating ovulation, and is usable for infertility treatment such as, for example, IVF (in vitro fertilization), artificial insemination or the like.

### BRIEF DESCRIPTION OF THE DRAWINGS

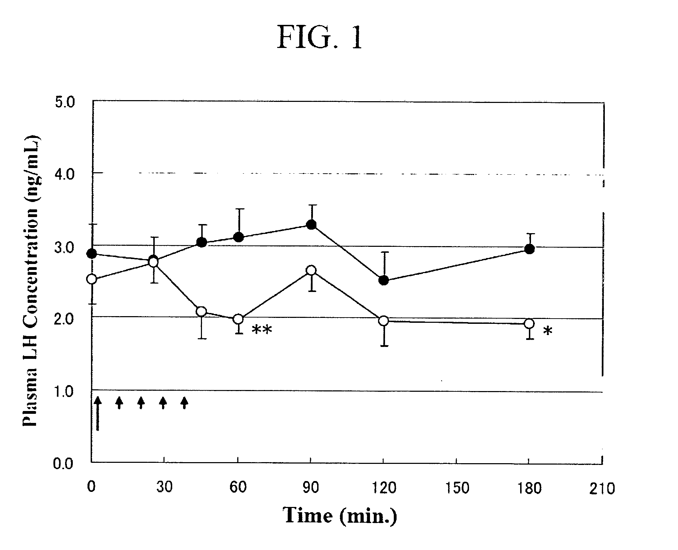

FIG 1 is a graph showing the LH concentration in the plasma of the test rats. In the figure, -●- represents the control group, -○- represents the group to which the compound was administered, and the arrow and the head thereof represent the time of administration. Student's test; **P < 0.01, *P < 0.05

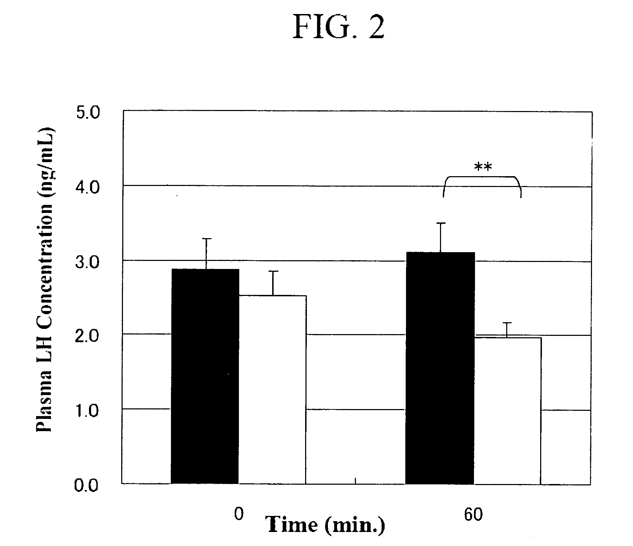

FIG 2 is a graph showing the LH concentration in the plasma of the test rats immediately before the administration and 60 minute after the start of administration. In the figure, -■- represents the control group, and -□- represents the group to which the compound was administered. Student's test; **P < 0.01

### BEST MODE FOR CARRYING OUT THE INVENTION

Herein, examples of the "hydrocarbon group" include a chain or cyclic hydrocarbon group (e.g., alkyl, alkenyl, alkynyl, cycloalkyl, cycloalkenyl, aryl, aralkyl, a polycyclic hydrocarbon group, etc.), and the like. Among them, a chain or cyclic hydrocarbon group having 1 to 19 carbon atoms and the like are preferred.

Examples of the "alkyl" described above include C₁₋₆ alkyl (e.g., methyl, ethyl, propyl, isopropyl, butyl, isobutyl, sec-butyl, tert-butyl, pentyl, neopentyl, hexyl, etc.), and the like.

Examples of the "alkenyl" described above include C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl, etc.), and the like.

Examples of the "alkynyl" described above include C₂₋₆ alkynyl (e.g., ethynyl, propargyl, 1-butynyl, 2-butynyl, 3-butynyl, 1-hexynyl, etc.), and the like.

Examples of the "cycloalkyl" described above include C₃₋₆ cycloalkyl (e.g., cyclopropyl, cyclobutyl, cyclopentyl, cyclohexyl, etc.), and the like.

Examples of the "cycloalkenyl" described above include C₅₋₆ cycloalkenyl (e.g., 1-cyclopentenyl, 3-cyclopentenyl, 4-cyclopentenyl, 1-cyclohexenyl, 3-cyclohexenyl, 4-cyclohexenyl, 1-cycloheptenyl, 3-cycloheptenyl, 4-cycloheptenyl, etc.), and the like.

Examples of the "aryl" described above include C₆₋₁₄ aryl (e.g., phenyl, 1-naphthyl, 2-naphthyl, 2-biphenylyl, 3-biphenylyl, 4-biphenylyl, 2-anthryl, 3-indenyl, etc.), and the like.

Examples of the "aralkyl" described above include C₇₋₁₉ aralkyl (e.g., benzyl, phenethyl, diphenylmethyl, trityl, 1-naphthylmethyl, 2-naphthylmethyl, 2,2-diphenylethyl, 3-phenylpropyl, 4-phenylbutyl, 5-phenylpentyl, 9-fluorenyl, etc.), and the like.

Examples of the "polycyclic hydrocarbon group" described above include a bi- to tetracyclic non-aromatic hydrocarbon group (e.g., 1-adamantyl, 2-adamantyl, decalin-1-yl, tetralin-1-yl, indan-1-yl, androstan-3-yl, 5-androsten-3-yl, etc.), and the like.

Herein, examples of the "heterocyclic group" include a monovalent group obtained by removing one optional hydrogen atom from a 5- to 14-membered (monocyclic, bicyclic or tricyclic) heterocyclic group posessing, 1 or 2 kind(s) of 1 to 4 hetero atom(s) selected from nitrogen atom, sulfur atom and oxygen atom, besides carbon atoms, preferably (i) a 5- to 14-membered (preferably 5- to 10-membered) aromatic heterocyclic group having the hetero atom(s) selected as above besides carbon atoms, (ii) a 3- to 14-membered non-aromatic heterocyclic group having the hetero atom(s) selected as above besides carbon atoms, or (iii) a 7- to 10-membered heterocyclic bridged ring having the hetero atom(s) selected as above besides carbon atoms, and the like.

Examples of the "5- to 14-membered (preferably 5- to 10-membered) aromatic heterocyclic group" described above include aromatic heterocyclic groups such as thiophene, benzo[b]thiophene, benzo[b]furan, benzimidazole, benzoxazole, benzothiazole, benzisothiazole, 1H benzotriazole, naphtho[2,3-b]thiophene, furan, pyrrole, imidazole, pyrazole, oxazole, 1,2,3-triazole, 1,2,4-triazole, tetrazole, 1,3,4-thiadiazole, 1,3,4-oxadiazole, pyridine, pyrazine, pyrimidine, pyridazine, indole, isoindole, 1H-indazole, purine, 4H-quinolizine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, isoxazole, furazan, phenoxazine, and the like; a ring formed by condensing any of these groups (preferably a monocyclic ring) with one or a plurality of (preferably 1 or 2) aromatic rings (e.g., benzene ring, pyridine ring, imidazole ring, etc.); and the like.

Examples of the "3- to 14-membered non-aromatic heterocyclic group" described above include oxirane, oxetane, tetrahydrofuran, dihydrofuran, pyran, dioxolane, dioxane, azetidine, pyrrolidine, imidazoline, pyrazolidine, pyrazoline, piperidine, piperazine, morpholine, thiomorpholine, thiazolidine, oxazolidine, oxadiazoline, thiadiazoline, triazoline, 1,4-diazepane, 1,4-oxazepane, 1,4-thiazepane, the reduced forms or partially reduced forms of any of the aromatic heterocyclic groups described above (e.g., 1,2,3,4-tetrahydroquinoline, 1,2,3,4-tetrahydroisoquinoline, indoline, etc.), and the like.

Examples of the "7- to 10-membered heterocyclic bridged ring" described above include quinuclidine, 7-azabicyclo[2.2.1]heptane, and the like.

The "heterocyclic group" is preferably a 5- to 14-membered (preferably 5- to 10-membered) (monocyclic or bicyclic) heterocyclic group having, besides carbon atoms, 1 or 2 kind(s) of preferably 1 to 4 hetero atom(s) selected from nitrogen atom, sulfur atom and oxygen atom. Specific examples include aromatic heterocyclic groups such as 2-thienyl, 3-thienyl, 2-furyl, 3-furyl, 1-pyrrolyl, 3-pyrrolyl, 1-imidazolyl, 2-imidazolyl, 2-pyridyl, 1-pyrazolyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyridazinyl, 2-thiazolyl, 2-oxazolyl, 3-isothiazolyl, 3-isoxazolyl, 5-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.; non-aromatic heterocyclic groups such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, tetrahydroazepin-1-yl, homopiperazin-1-yl, etc.; and the like.

Herein, examples of the "acyl" include groups represented by formula -(C=O)-R⁶, -(C=O)-OR⁵, -(C=O)-NR⁶R⁷, -(C=S)-NR⁶ R⁷, -SO-R⁵, -SO₂ -R⁵ or -SO₂ -NR⁶R⁷, wherein R⁵ represents an optionally substituted hydrocarbon group or an optionally substituted heterocyclic group; R⁶ represents a hydrogen atom, an optionally substituted hydrocarbon group or an optionally substituted heterocyclic group; R⁷ represents a hydrogen atom, optionally substituted amino, optionally substituted hydroxy, an optionally substituted hydrocarbon group or an optionally substituted heterocyclic group; and NR⁶R⁷ may be a cyclic amino, and the like.

Examples of the "substituent" in the "optionally substituted hydrocarbon group" and the "optionally substituted heterocyclic group" represented by R⁵, R⁶, or R⁷ include a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, 5- to 14-membered aromatic heterocyclic group-C₁₋₆ alkyl (e.g., imidazolylmethyl, indolylmethyl etc.), C₁₋₈ alkoxy-C₁₋₆ alkyl (e.g., methoxymethyl, ethoxymethyl, etc.), amino-C₁₋₆ alkyl (e.g., aminomethyl, etc.), mono- or di-C₁₋₆ alkylamino-C₁₋₆ alkyl (e.g., (e.g., methylaminomethyl, dimethylaminomethyl, etc.), di-C₁₋₆ alkylamino (e.g., dimethylamino, diethylamino, ethylmethylamino, etc.), C₇₋₁₉ aralkyloxy-carbonyl-C₁₋₆ alkyl (e.g., benzyloxycarbonylmethyl, benzyloxycarbonylethyl, etc.), optionally halogenated C₂₋₆ alkenyl, carboxy, C₂₋₆ alkenyl (e.g., 2-carboxyethenyl, 2-carboxy-2-methylethenyl, etc.), optionally halogenated C₂₋₆ alkynyl, optionally halogenated or optionally condensed C₃₋₈ cycloalkyl, optionally substituted aryl (e.g., C₆₋₁₄ aryl (e.g., phenyl, 1-naphthyl, 2-naphthyl, 2-biphenylyl, 3-biphenylyl, 4-biphenylyl, 2- anthryl, etc.) and the like), C₇₋₁₉ aralkyl (e.g., benzyl, etc.), optionally substituted aromatic heterocyclic group (e.g., 5- to 14-membered (preferably, 5- to 10-membered) (monocyclic or bicyclic) aromatic heterocyclic group (e.g., 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 5-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.) and the like); optionally substituted non-aromatic heterocyclic group (e.g., 3- to 14-membered (preferably, 5- to 10-membered) (monocyclic or bicyclic) non-aromatic heterocyclic group (e.g., 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, tetrahydroazepin-1-yl, homopiperazin-1-yl, etc.) and the like); optionally halogenated C₁₋₈ alkoxy, C₁₋₆ alkoxy-carbonyl-C₁₋₆ alkoxy (e.g., ethoxycarbonylmethyloxy, etc.), hydroxy, C₆₋₁₄ aryloxy (e.g., phenyloxy, 1-naphthyloxy, 2-naphthyloxy, etc.), C₇₋₁₆ aralkyloxy (e.g., benzyloxy, phenethyloxy, etc.), amino, hydroxyamino, mono-C₁₋₆ alkylamino (e.g., methylamino, ethylamino, etc.), mono-C₆₋₁₄ arylamino (e.g., phenylamino, 1-naphthylamino, 2-naphthylamino, etc.), di-C₁₋₆ alkylamino (e.g., dimethylamino, diethylamino, ethylmethylamino, etc.), di-C₆₋₁₄ arylamino (e.g., diphenylamino, etc.), C₁₋₆ alkyl-carbonylamino (e.g., acetylamino, propionylamino, etc.), C₁₋₆ alkyl (optionally halogenated C₁₋₆ alkyl-carbonyl)amino (e.g., methyl(trifluoroacetyl)amino, etc.), C₁₋₆ alkoxy-carbonylamino (e.g., methoxycarbonylamino, ethoxycarbonylamino, propoxycarbonylamino, butoxycarbonylamino, tert-butoxycarbonylamino, etc.), optionally substituted 5- to 7-membered saturated cyclic amino, formyl, carboxy, C₁₋₆ alkyl-carbonyl (e.g., acetyl, propionyl, etc.), C₃₋₆ cycloalkyl-carbonyl (e.g., cyclopropylcarbonyl, cyclopentylcarbonyl, cyclohexylcarbonyl, etc.), C₁₋₆ alkoxy-carbonyl (e.g., methoxycarbonyl, ethoxycarbonyl, propoxycarbonyl, tert-butoxycarbonyl, etc.), C₆₋₁₄ aryl-carbonyl (e.g., benzoyl, 1-naphthoyl, 2-naphthoyl, etc.), C₇₋₁₆ aralkyl-carbonyl (e.g., phenylacetyl, 3-phenylpropionyl, etc.), C₆₋₁₄ aryloxy-carbonyl (e.g., phenoxycarbonyl, etc.), C₇₋₁₆ aralkyloxy-carbonyl (e.g., benzyloxycarbonyl, phenethyloxycarbonyl, etc.), 5- or 6-membered heterocyclic carbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.), carbamoyl, mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), C₆₋₁₄ aryl-carbamoyl (e.g., phenylcarbamoyl, 1-naphthylcarbamoyl, 2-naphthylcarbamoyl, etc.), C₁₋₆ alkoxy-carbamoyl (e.g., methoxycarbamoyl, ethoxycarbamoyl, etc.), 5- or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl, etc.), optionally substituted mercapto, sulfo, C₁₋₆ alkylsulfonyl (e.g., methylsulfonyl, ethylsulfonyl, etc.), C₆₋₁₄ arylsulfonyl (e.g., phenylsulfonyl, 1-naphthylsulfonyl, 2-naphthylsulfonyl, etc.), formylamino, C₁₋₆ alkyl-carbonylamino (e.g., acetylamino, etc.), C₆₋₁₄ aryl-carbonylamino (e.g., benzoylamino, naphthoylamino, etc.), C₁₋₆ alkoxy-carbonylamino (e.g., methoxycarbonylamino, ethoxycarbonylamino, propoxycarbonylamino, butoxycarbonylamino, etc.), C₁₋₆ alkylsulfonylamino (e.g., methylsulfonylamino, ethylsulfonylamino, etc.), C₆₋₁₄ arylsulfonylamino (e.g., phenylsulfonylamino, 2-naphthylsulfonylamino, 1-naphthylsulfonylamino, etc.), C₁₋₆ alkyl-carbonyloxy (e.g., acetoxy, propionyloxy, etc.), C₆₋₁₄ aryl-carbonyloxy (e.g., benzoyloxy, naphthylcarbonyloxy, etc.), C₁₋₆ alkoxy-carbonyloxy (e.g., methoxycarbonyloxy, ethoxycarbonyloxy, propoxycarbonyloxy, butoxycarbonyloxy, etc.), mono-C₁₋₆ alkyl-carbamoyloxy (e.g., methylcarbamoyloxy, ethylcarbamoyloxy, etc.), di-C₁₋₆ alkyl-carbamoyloxy (e.g., dimethylcarbamoyloxy, diethylcarbamoyloxy, etc.), C₆₋₁₄ aryl-carbamoyloxy (e.g., phenylcarbamoyloxy, naphthylcarbamoyloxy, etc.), 5- or 6-membered heterocyclic carbonyloxy (e.g., nicotinoyloxy, isonicotinoyloxy, etc.), oxo, imino, C₁₋₆ alkoxy-carbonylimino (e.g., methoxycarbonylimino, ethoxycarbonylimino, propoxycarbonylimino, butoxycarbonylimino, tert-butoxycarbonylimino, etc.), C₁₋₆ alkylimino (e.g., methylimino, ethylimino etc.), and the like.

Examples of the "optionally halogenated C₁₋₆ alkyl" described above include alkyl (e.g., C₁₋₆ alkyl such as methyl, ethyl, propyl, isopropyl, butyl, isobutyl, sec-butyl, tert-butyl, pentyl, hexyl, etc.), which may optionally contain, e.g., 1 to 5, preferably 1 to 3, halogen atom(s) (e.g., fluorine, chlorine, bromine, iodine, etc.), and the like.
Specific examples include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, and the like.

Examples of the "optionally halogenated C₂₋₆ alkenyl" described above include a C₂₋₆ alkenyl (e.g., vinyl, propenyl, isopropenyl, 2-buten-1-yl, 4-penten-1-yl, 5-hexen-1-yl, etc.) which may optionally contain, e.g., 1 to 5, preferably 1 to 3, halogen atom(s) (e.g., fluorine, chlorine, bromine, iodine, etc.), and the like.

Examples of the "optionally halogenated C₂₋₆ alkynyl" described above include C₂₋₆ alkynyl (e.g., propargyl, 2-butyn-1-yl, 4-pentyn-1-yl, 5-hexyn-1-yl, etc.), which may optionally contain, e.g., 1 to 5, preferably 1 to 3, halogen atom(s) (e.g., fluorine, chlorine, bromine, iodine, etc.), and the like.

Examples of the "optionally halogenated C₃₋₈ cycloalkyl" in the "optionally halogenated or optionally condensed C₃₋₈ cycloalkyl" described above include C₃₋₆ cycloalkyl (e.g., cyclopropyl, cyclobutyl, cyclopentyl, cyclohexyl, etc.), which may optionally contain, e.g., 1 to 5, preferably 1 to 3, halogen atom(s) (e.g., fluorine, chlorine, bromine, iodine, etc.), and the like. Specific examples include cyclopropyl, cyclobutyl, cyclopentyl, cyclohexyl, 4,4-dichlorocyclohexyl, 2,2,3,3-tetrafluorocyclopentyl, 4-chlorocyclohexyl, and the like.

Examples of the "condensed C₃₋₈ cycloalkyl" in the "optionally halogenated or optionally condensed C₃₋₈ cycloalkyl" described above include 8- to 14-membered bicyclic or tricyclic C₃₋₈ cycloalkyl (e.g., 1-adamantyl, 2-adamantyl, decalin-1-yl, tetralin-1-yl, 9-fluorenyl, 1-indanyl, 1,2,3,4-tetrahydro-1-naphthyl, etc.), and the like. The "condensed C₃₋₈ cycloalkyl" may optionally be halogenated.

Examples of the "substituent" in the "optionally substituted aryl", "optionally substituted aromatic heterocyclic group" and "optionally substituted non-aromatic heterocyclic group" described above include (a) a halogen atom, (b) C₁₋₆ alkyl which may optionally contain optionally halogenated C₆₋₁₂ aryl, (c) optionally halogenated C₆₋₁₂ aryl, (d) C₁₋₆ alkoxy which may be optionally substituted with a 5- or 6-membered aromatic heterocyclic group which may optionally contain C₁₋₆ alkyl, (e) C₇₋₁₃ aralkyloxy which may optionally contain 1 to 3 substituent(s) selected from halogen, C₁₋₆ alkoxy and optionally halogenated C₁₋₆ alkyl, (f) 3- to 10-membered non-aromatic homocyclic-oxy, (g) optionally halogenated C₆₋₁₂ aryloxy, (h) 5- or 6-membered aromatic heterocyclic-oxy, (i) optionally halogenated C₆₋₁₂ aryl-carbonylamino, (j) optionally halogenated C₇₋₁₃ aralkylamino, (k) optionally halogenated C₆₋₁₂ aryl-carbonyl, (1) optionally halogenated C₇₋₁₃ aralkyl-carbonyl, (m) C₁₋₆ alkylsulfonyl, (n) 5- to 10-membered aromatic group, (o) hydroxy, (p) cyano, (q) carboxy, etc.

Examples of the "optionally halogenated C₁₋₈ alkoxy" described above include C₁₋₈ alkoxy (e.g., methoxy, ethoxy, propoxy, isopropoxy, butoxy, isobutoxy, sec-butoxy, pentyloxy, hexyloxy, etc.), which may optionally contain, e.g., 1 to 5, preferably 1 to 3, halogen atom(s) (e.g., fluorine, chlorine, bromine, iodine, etc.), and the like. Specific examples include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, and the like.

Examples of the "5- to 7-membered saturated cyclic amino" in the "optionally substituted 5- to 7-membered saturated cyclic amino" include 5- to 7-membered saturated cyclic amino, which may contain, besides one nitrogen atom and carbon atoms, 1 or 2 kind(s) of preferably 1 to 4 hetero atom(s) selected from nitrogen atom, sulfur atom and oxygen atom. Specific examples include pyrrolidin-1-yl, piperidino, piperazin-1-yl, morpholino, thiomorpholino, tetrahydroazepin-1-yl, homopiperazin-1-yl, and the like.

Examples of the "substituent" in the "optionally substituted 5- to 7-membered saturated cyclic amino" described above include C₁₋₆ alkyl (e.g., methyl, ethyl, propyl, isopropyl, butyl, isobutyl, sec-butyl, tert-butyl, pentyl, hexyl, etc.), amino C₁₋₆ alkyl (e.g., aminomethyl, aminoethyl, etc.), C₁₋₆ alkoxy (e.g., methoxy, ethoxy, propoxy, isopropoxy, butoxy, etc.), halogen atom (e.g., fluorine, chlorine, bromine, iodine, etc.), hydroxy, cyano, amino, carboxy, carbamoyl, C₁₋₆ alkoxy-carbonyl (e.g., methoxycarbonyl, ethoxycarbonyl, tert-butoxycarbonyl, etc.), C₇₋₁₄ aralkyloxy-carbonyl (e.g., benzyloxycarbonyl, etc.), C₆₋₁₄ aryl (e.g., phenyl, 1-naphthyl, 2-naphthyl, 2-biphenylyl, 3-biphenylyl, 4-biphenylyl, 2-anthryl, etc.), C₁₋₆ alkyl-carbonyl (e.g., acetyl, propionyl, etc.), C₁₋₆ alkyl-sulfonyl (e.g., methanesulfonyl, ethanesulfonyl, etc.), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), oxo, and the like. The "optionally substituted 5- to 7-membered saturated cyclic amino" may contain 1 to 5, preferably 1 to 3, of the substituents described above at any substitutable positions; with proviso that if there are two or more substituents, the substituents may be the same or different.

Examples of the "substituent" in the "optionally substituted mercapto" include a hydrocarbon group or a heterocyclic group, each of which may contain 1 to 5 substituents selected from halogen atom, C₁₋₃ alkylenedioxy, nitro, cyano, optionally halogenated C₁₋₆ alkyl, optionally halogenated C₂₋₆ alkenyl, carboxy-C₂₋₆ alkenyl, optionally halogenated C₂₋₆ alkynyl, optionally halogenated or optionally condensed C₃₋₈ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, hydroxyamino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl, carbamoyl, mono-C₁₋₆ alkyl-carbamoyl, di-C₁₋₆ alkyl-carbamoyl, C₆₋₁₄ aryl-carbamoyl, C₁₋₆ alkoxy-carbamoyl, 5- or 6-membered heterocyclic carbamoyl, sulfo, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, 5- or 6-membered heterocyclic carbonyloxy, 5- to 7-membered saturated cyclic amino, a 5- to 10-membered aromatic heterocyclic group, and 5- to 10-membered non-aromatic heterocyclic group.

Examples of the "substituent" in the "optionally substituted amino" include (i) a hydrocarbon group or a heterocyclic group, each of which may contain 1 to 5 substituent(s) selected from halogen atom, C₁₋₃ alkylenedioxy, nitro, cyano, optionally halogenated C₁₋₆ alkyl, optionally halogenated C₂₋₆ alkenyl, carboxy-C₂₋₆ alkenyl, optionally halogenated C₂₋₆ alkynyl, optionally halogenated or optionally condensed C₃₋₈ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, hydroxyamino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl, carbamoyl, mono-C₁₋₆ aralkyl-carbamoyl, di-C₁₋₆ aralkyl-carbamoyl, C₆₋₁₄ aryl-carbamoyl, C₁₋₆ alkoxy-carbamoyl, 5- or 6-membered heterocyclic carbamoyl, sulfo, C₁₋₆ alkylsulfonyl, C₆₋₁₄ arylsulfonyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, 5- or 6-membered heterocyclic carbonyloxy, 5- to 7-membered saturated cyclic amino, a 5- to 10-membered aromatic heterocyclic group, and 5- to 10-membered non-aromatic heterocyclic group; (ii) a sulfonyl substituted with a substituent selected from optionally halogenated C₁₋₆ alkyl, optionally halogenated C₂₋₆ alkenyl, carboxy C₂₋₆ alkenyl, optionally halogenated C₂₋₆ alkynyl, optionally halogenated or optionally condensed C₃₋₈ cycloalkyl, and C₆₋₁₄ aryl.

Examples of the "substituent" in the "optionally substituted hydroxy" represented by R⁷ may be, for example, the same as the examples of the "substituent" in the "optionally substituted mercapto" described above.

Examples of the "cyclic amino" represented by NR⁶R⁷ include a 5- to 7-membered saturated cyclic amino, which may contain, besides one nitrogen atom and carbon atoms, 1 or 2 kind(s) of 1 to 4 hetero atom(s) selected from nitrogen atom, sulfur atom and oxygen atom. Specific examples include pyrrolidin-1-yl, piperidino, piperazin-1-yl, morpholino, thiomorpholino, tetrahydroazepin-1-yl, homopiperazin-1-yl, and the like.

Examples of the "5- to 8-membered homocyclic group" in the "5- to 8-membered homocyclic or heterocyclic group optionally having a substituent other than a group represented by formula -X-R¹" represented by Ring A in compound (I) include C₅₋₈ cycloalkane (e.g., cyclopentane, cyclohexane, cycloheptane, cyclooctane, etc.), C₅₋₇ cycloalkene (e.g., cyclopentene, cyclopentadiene, cyclohexene, 1,3-cyclohexadiene, 1,4-cyclohexadiene, cycloheptene, 1,3-cycloheptadiene, etc.), benzene, and the like.

Examples of the "5- to 8-membered heterocyclic group" in the "5- to 8-membered homocyclic or heterocyclic group optionally having a substituent other than a group represented by formula -X-R¹" represented by Ring A include a 5- to 8-membered heterocyclic group containing, besides carbon atoms, 1 or 2 kind(s) of 1 to 4 hetero atom(s) selected from nitrogen atom, sulfur atom and oxygen atom, preferably (i) a 5- to 8-membered aromatic heterocyclic group having the hetero atom(s) selected as above besides carbon atoms; (ii) a 5- to 8-membered non-aromatic heterocyclic group having the hetero atom(s) selected as above besides carbon atoms; or (iii) a 7- to 8-membered heterocyclic bridged ring having the hetero atom(s) selected as above besides carbon atoms, and the like.

Examples of the "5- to 8-membered aromatic heterocyclic group" described above include aromatic heterocyclic groups such as thiophene, furan, pyrrole, imidazole, pyrazole, pyridine, pyrazine, pyrimidine, pyridazine , and the like.

Examples of the "5- to 8-membered non-aromatic heterocyclic group" described above include dihydropyrrole, dihydroimidazole, dihydropyrazole, dihydro-1,4-oxazine, dihydro-1,4-thioxazine, dihydrofuran, tetrahydrofuran, dihydrothiophene, pyran, dihydropyran, thiopyran, dihydrothiopyran, dihydrooxepin, dihydrothiepin, dihydroazepine, dihydro-1,4-diazepine, dihydro-1,4-oxazepine, dihydro-1,4-thiazepine, dioxorane, dioxane, azetidine, thiazolidine, oxazolidine, oxadiazoline, thiadiazoline, triazoline, 1,4-diazepane, 1,4-oxazepane, 1,4-thiazepane, pyrrolidine, imidazoline, pyrazolidine, pyrazoline, piperidine, piperazine, morpholine, thiomorpholine, tetrahydropyridine, dihydropyridine, tetrahydropyrazine, dihydropyrazine, and the like.

Examples of the "7- to 8-membered heterocyclic bridged ring" described above include quinuclidine, 7-azabicyclo[2.2.1]heptane, and the like.

The "5- to 8-membered heterocyclic group" described above is preferably a 5-to 8-membered, preferably 5- or 6-membered, heterocyclic group containing, besides carbon atoms, 1 or 2 kind(s) of preferably 1 to 3 hetero atom(s) selected from nitrogen atom, sulfur atom and oxygen atom. Specific examples include thiophene, furan, pyrrole, imidazole, pyrazole, pyridine, pyrazine, pyrimidine, pyridazine, piperidine, and the like. Examples of the "substituent" in the "5- to 8-membered homocyclic or heterocyclic group optionally having a substituent other than a group represented by formula -X-R¹" represented by Ring A may be, for example, the same as the examples of the "substituent" in the "optionally substituted hydrocarbon group".

Ring A may contain 1 to 5, preferably 1 to 3, of the substituents described above at any substitutable positions; with proviso that if there are two or more substituents, the substituents may be the same or different.

Examples of the "spacer" represented by X in compound (I) include 1 to 3 group(s) (with proviso that if there are two or three groups, such groups may be bonded in any order) selected from -O-, -S-, -CO-, -SO-, SO₂-, -NR⁸-, -CONR⁸-, -NR⁸CO-, -SO₂NR⁸-, -NR⁸SO₂-, -NR⁸CONR⁹-, optionally substituted bivalent chain hydrocarbon group (e.g., bivalent C₁₋₅ chain hydrocarbon (e.g., C₁₋₅ alkylene, C₁₋₅ alkenylene, C₁₋₅ alkynylene, etc.) and the like, optionally substituted bivalent heterocyclic group (e.g., bivalent 5- or 6-membered heterocyclic group (e.g., thiazole-2,5-diyl, thiophene-2,5-diyl, furan-2,5-diyl, pyridin-2,5-diyl, pyridin-2,4-diyl, pyrimidin-1 ,3-diyl, pyrrolidin-1 ,3-diyl, piperidin-1 ,4-diyl, piperazin-1 ,4-diyl, etc.) and the like); and optionally substituted bivalent homocyclic group (e.g., bivalent 3 to 8-membered homocyclic group, preferably bivalent 6-membered homocyclic group (e.g., 1 ,2-phenylene, 1,3-phenylene, 1,4-phenylene, cyclopentan-1,2-diyl, cyclohexan-1,2-diyl, cyclohexan-1,4-diyl, pyridin-2,5-diyl, pyridin-2,4-diyl, piperidin-1,4-diyl, etc.); and the like.

In the above formulas, R⁸ and R⁹ independently represent a hydrogen atom, an optionally substituted hydrocarbon group, or an optionally substituted heterocyclic group.

Examples of the "optionally substituted hydrocarbon group" and the "optionally substituted heterocyclic group" represented by R⁸ or R⁹ may be, for example, the same as the examples of the "optionally substituted hydrocarbon group" and the "optionally substituted heterocyclic group" represented by R⁵.

Examples of the "substituent" in the "optionally substituted bivalent chain hydrocarbon group", "optionally substituted bivalent heterocyclic group", and "optionally substituted bivalent homocyclic group" described above may be, for example, the same as the examples of the "substituent" in the "optionally substituted hydrocarbon group" represented by R⁵.

The bivalent chain hydrocarbon group, the bivalent heterocyclic group, and bivalent homocyclic group may contain 1 to 5, preferably 1 to 3, of the substituents described above at any substitutable positions; with proviso that if there are two or more substituents, the substituents may be the same or different.

Examples of the "spacer" represented by X include spacers having 1 to 8 atoms in the main chain.

Examples of such a spacer include a group represented by the formula: 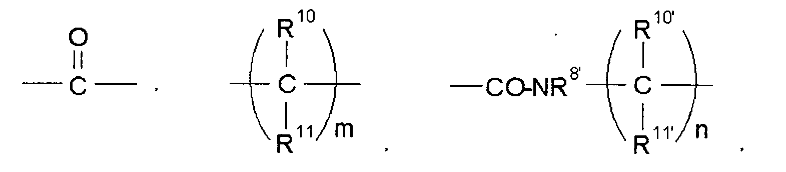 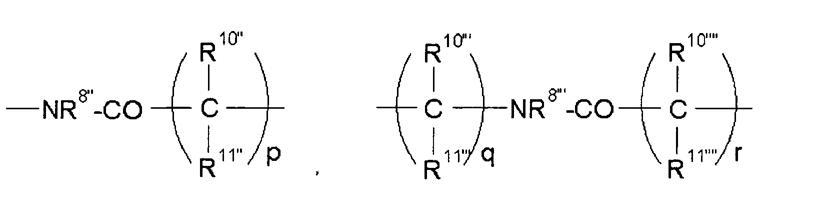 or 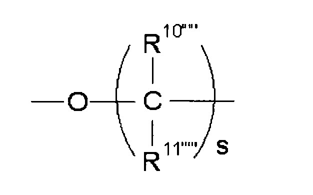 wherein
R^{8'}, R^{8"}, R^{8"'}, R¹⁰, R^{10'}, R^{10"}, R^{10'"}, R^{10""}, R^{10"'"}, R¹¹, R^{11'}, R^{11"}, R^{11"'}, R^{11""} and R^{11""'}, (i) independently represent a hydrogen atom, an optionally substituted hydrocarbon group, or an optionally substituted heterocyclic group; or (ii) R¹⁰ and R¹¹, R^{10'} and R^{11'}, R^{10"} and R^{11"}, R^{10"'} and R^{11"'}, R^{10""} and R^{11""}, and R^{10""'} and R^{11""'}, may each form a 3- to 6-membered ring together with an adjacent carbon atom;
m represents an integer of 1 to 8;
n, p and s independently represent an integer of 0 to 6;
q and r independently represent an integer of 1 to 3 with proviso that if m, n, p, q and r are each 2 or greater, R¹⁰, R^{10'}, R^{10"}, R^{10"'}, R^{10""}, R^{10"""}, R¹¹, R^{11'}, R^{11"}, R^{11"'}, R^{11"'}, R^{11""'} in the repeat unit may each be the same or different. An especially preferably group is represented by the formula: 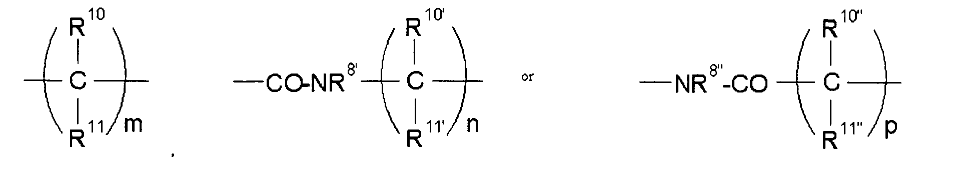 wherein each letter has the same significance as above.

Examples of the "optionally substituted hydrocarbon group" and the "optionally substituted heterocyclic group" represented by R^{8'}, R^{8"}, R^{8"'}, R¹⁰, R^{10'}, R^{10"}, R^{10"'}, R^{10""}, R^{10""'}, R¹¹, R^{11'}, R^{11"}, R^{11"'}, R^{11""} or R^{11""'} may be, for example, the same as the examples of the "optionally substituted hydrocarbon group" and the "optionally substituted heterocyclic group" represented by R⁵.

Examples of the "3- to 6-membered ring" formed by each of R¹⁰ and R¹¹, R^{10'} and R^{11'}, R^{10"} and R^{11"}, R^{10"} and R^{11"'}, R^{10""} and R^{11""}, and R^{10""'} and R^{11""'} together with an adjacent carbon atom include C₃₋₆ cycloalkane (e.g., cyclopropane, cyclobutane, cyclopentane, cyclohexane, etc.), C₃₋₆ cycloalkene (e.g., cyclopropene, cyclobutene, cyclopentene, cyclohexene, etc.), and the like.

Examples of the "substituent" of the "optionally substituted amino" represented by R¹ in compound (I) include acyl, an optionally substituted hydrocarbon group, an optionally substituted heterocyclic group, and the like. Examples of the "optionally substituted hydrocarbon group" and the "optionally substituted heterocyclic group" may be, for example, the same as the examples of the "optionally substituted hydrocarbon group" and the "optionally substituted heterocyclic group" represented by R⁵.

Amino may contain 1 or 2 of the substituents described above at any substitutable positions; with proviso that if there are two or more substituents, the substituents may be the same or different.

Examples of the "nitrogen-containing heterocyclic group" in the "optionally substituted nitrogen-containing heterocyclic group" represented by R¹ include a 5- to 14-membered (monocyclic, bicyclic or tricyclic) heterocyclic group which may optionally contain, besides one nitrogen atom and carbon atoms, 1 or 2 kind(s) of 1 to 4 hetero atom(s) selected from nitrogen atom, sulfur atom and oxygen atom, and the like.

Specific examples of such a heterocyclic group include a 5- to 10-membered nitrogen-containing heterocyclic group (e.g., 1-pyrrolyl, 3-pyrrolyl, 1-imidazolyl, 2-imidazolyl, 1-pyrazolyl, 2-pyridyl, 3-pyridyl, 4-pyridyl, 2-quinolyl, 3-quinolyl, 4-quinolyl, 5-quinolyl, 8-quinolyl, 1 -isoquinolyl, 3-isoquinolyl, 4-isoquinolyl, 5-isoquinolyl, pyrazinyl, 2-pyrimidinyl, 4-pyrimidinyl, 3- pyridazinyl, 2-thiazolyl, 2-oxazolyl, 3-isothiazolyl, 3-isoxazolyl, 1-indolyl, 2-indolyl, 3-indolyl, 2-benzothiazolyl, 1-pyrrolidinyl, 2-pyrrolidinyl, 3-pyrrolidinyl, pyrrolinyl, imidazolidinyl, 2-imidazolidinyl, 4-imidazolidinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, tetrahydroazepin-1-yl, homopiperazin-1-yl, hexamethyleneimino, 1,4-diazepan-1-yl, etc.) and the like. The cycle (preferably, monocycle) may be condensed with 1 or 2 of 5- or 6-membered aromatic ring(s) (e.g., benzene ring, pyridine ring, imidazole ring, etc.). A 5- or 6-membered nitrogen-containing heterocyclic group, a 5- or 6-membered nitrogen-containing heterocyclic group which may be optionally condensed with one 5- or 6-membered aromatic ring, and the like are especially preferable.

Examples of the "substituent" in the "optionally substituted nitrogen-containing heterocyclic group" represented by R¹ may be, for example, the same as the examples of the "substituent" in the "optionally substituted hydrocarbon group" represented by R⁵.

The nitrogen-containing heterocyclic group may contain 1 to 5, preferably 1 to 3, of the substituents described above at any substitutable positions; with proviso that if there are two or more substituents, the substituents may be the same or different.

Examples of the "substituent" in the "benzene ring optionally having a substituent other than hydroxy " represented by Ring B in compounds (I) and (I') may be, for example, the same as the examples of the "substituent" in the "optionally substituted hydrocarbon group" represented by R⁵.

Ring B may contain 1 to 5, preferably 1 to 3, of the substituents described above at any substitutable positions; with proviso that if there are two or more substituents, the substituents may be the same or different.

Examples of the "homocyclic group" in the "optionally substituted homocyclic or heterocyclic group" represented by R² in compound (I) include cycloalkyl, cycloalkenyl, aryl, a polycyclic hydrocarbon group, and the like.

Examples of the "cycloalkyl" described above preferably include C₃₋₆ cycloalkyl (e.g., cyclopropyl, cyclobutyl, cyclopentyl, cyclohexyl, etc.), and the like.

Examples of the "cycloalkenyl" include C₅₋₆ cycloalkenyl (e.g., 1-cyclopentenyl, 3-cyclopentenyl, 4-cyclopentenyl, 1-cyclohexenyl, 3-cyclohexenyl, 4-cyclohexenyl, 1-cycloheptenyl, 3-cycloheptenyl, 4-cycloheptenyl, etc.), and the like.

Examples of the "aryl" include C₆₋₁₄ aryl (e.g., phenyl, 1-naphthyl, 2-naphthyl, 2-biphenylyl, 3-biphenylyl, 4-biphenylyl, 2-anthryl, etc.), and the like.

Examples of the "polycyclic hydrocarbon group" include a bi- to tetracyclic non-aromatic hydrocarbon group (e.g., 1-adamantyl, 2-adamantyl, decalin-1-yl, tetralin-1-yl, indan-1-yl, androstan-3-yl, 5-androsten-3-yl, etc.), and the like.

Examples of the "substituent" in the "optionally substituted homocyclic or heterocyclic group" represented by R² may be, for example, the same as the examples of the "substituent" in the "optionally substituted hydrocarbon group" represented by R⁵.

The "homocyclic or heterocyclic group" may contain 1 to 5, preferably 1 to 3, of the substituents described above at any substitutable positions; with proviso that if there are two or more substituents, the substituents may be the same or different.

Examples of the "optionally substituted hydrocarbon group" represented by R³ or R⁴ in compound (I) may be, for example, the same as the examples of the "optionally substituted hydrocarbon group" represented by R⁵.

Ring A is preferably benzene or a 5- to 8-membered aromatic heterocyclic group, each of which may optionally contain halogen, and is more preferably a benzene ring.

A preferable example of 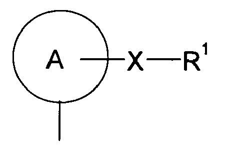 is a group represented by the formula: 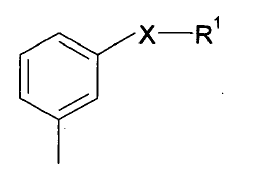 wherein each letter has the same significance as above.

X is preferably a bond or a group represented by the formula: 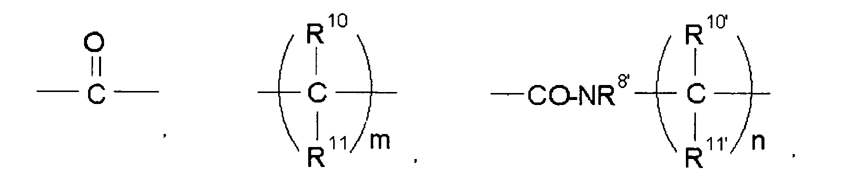 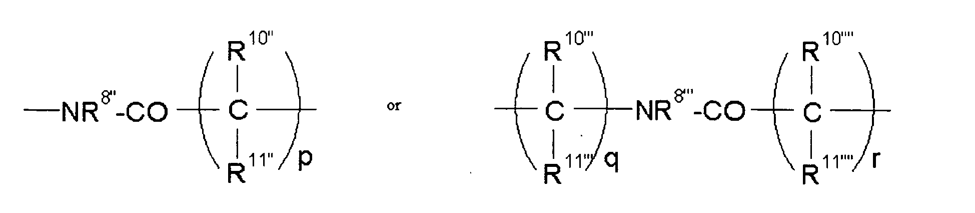 wherein each letter has the same significance as above. More preferably, X is a bond or a group represented by the formula: 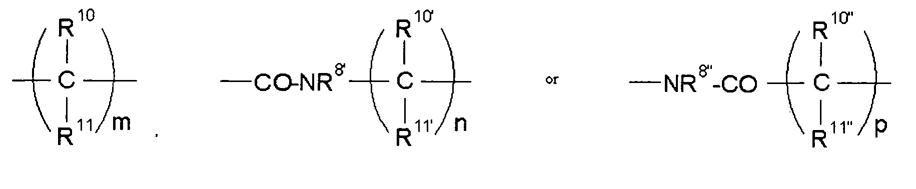 wherein each letter has the same significance as above.

Still more preferably, X is a bond or a group represented by the formula: 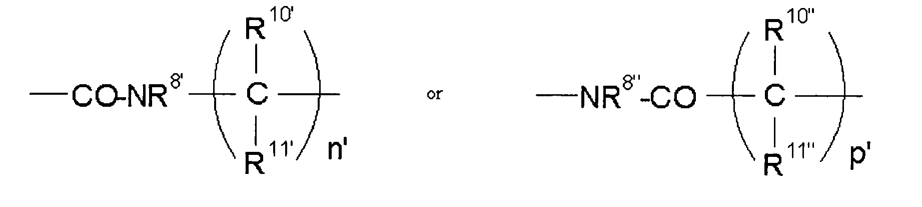 wherein each letter has the same significance as above.

In each of the above formulas, R^{8'} is preferably a hydrogen atom, C₁₋₃ alkyl, or the like.

R^{8"} is preferably a hydrogen atom, C₁₋₃ alkyl, or the like.

R^{8'"} is preferably a hydrogen atom, C₁₋₃ alkyl, or the like.

R¹⁰ is preferably a hydrogen atom, C₁₋₃ alkyl, or the like.

R^{10'} is preferably a hydrogen atom, C₁₋₃ alkyl, or the like.

R^{10"} is preferably a hydrogen atom, C₁₋₃ alkyl which may optionally contain a substituent (e.g., a 5- or 6-membered aromatic group, C₁₋₃ alkoxy, amino, mono- or di-C₁₋₆ alkylamino, etc.), or the like.
R^{10'"} is preferably a hydrogen atom, C₁₋₃ alkyl, or the like.

R^{10""} is preferably a hydrogen atom, C₁₋₃ alkyl, or the like.

R¹¹ is preferably a hydrogen atom, C₁₋₃ alkyl, or the like.

R^{11'} is preferably a hydrogen atom, C₁₋₃ alkyl, or the like.

R^{11"} is preferably a hydrogen atom, C₁₋₃ alkyl, or the like.

R^{11"'} is preferably a hydrogen atom, C₁₋₃ alkyl, or the like.

R^{11""} is preferably a hydrogen atom, C₁₋₃ alkyl, or the like.

m is preferably an integer of 1 to 4.

n is preferably an integer of 0 to 4.

n' is preferably an integer of 1 to 4.

p is preferably an integer of 1 to 4.

p' is preferably an integer of 1 to 4.

q is preferably an integer of 1 to 3.

r is preferably an integer of 1 to 3.

Still more preferably, X is a bond, -CONH-C₁₋₂ alkylene, -NHCO-C₁₋₂ alkylene, or the like.

R¹ is preferably amino which may optionally contain optionally halogenated C₁₋₆ alkyl, a 5- or 6-membered nitrogen-containing heterocyclic group which may optionally contain amino, or the like.

Ring B is preferably a benzene ring opttionally having halogen other than hydroxy.

A preferable example of 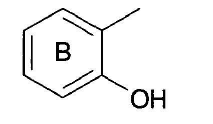 is a group represented by the formula: 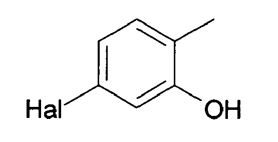 wherein Hal represents halogen (e.g., fluorine, chlorine, bromine, iodine, etc.).

R² is preferably an optionally substituted aromatic group or the like. More preferably, R² is an optionally substituted C₆₋₁₄ aryl, an optionally substituted aromatic 5- to 14-membered heterocyclic group, or the like. Still more preferably, R² is phenyl, an aromatic 5- to 10-membered heterocyclic group, or the like.

R³ is preferably a hydrogen atom, cyano, or the like.

R⁴ is preferably a hydrogen atom.

Preferable examples of compound (I) include compounds of formula (I), among the compounds represented by the formula (I), wherein if:
R² represents a 5- or 6-membered aromatic heterocyclic group which may optionally contain 1 to 3 substituent(s) selected from halogen, hydroxy, amino, nitro, cyano, hydroxycarbonyl, alkyl, alkoxy, alkylamino, alkoxycarbonyl, aminocarbonyl and alkylaminocarbonyl; and R³ is cyano, 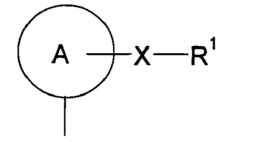 is not a group represented by the formula: 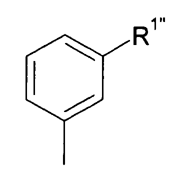
wherein R^{1"} represents amino or formula -NHCO-R^{1"'} (wherein R^{1"'} (i) alkyl, alkoxy, alkylamino, alkoxycarbonyl or aminocarbonyl which may optionally contain 1 to 3 substituent(s) selected from amino, hydroxy, alkoxy, alkylamino, alkoxycarbonyl, aminocarbonyl and alkylaminocarbonyl; or (ii) 5-oxotetrahydrofuran-2-yl).

Compound (I) is preferably, for example, a compound represented by the formula: 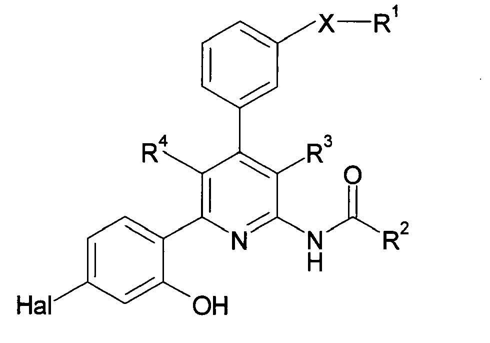 wherein X is a bond or a group represented by the formula: 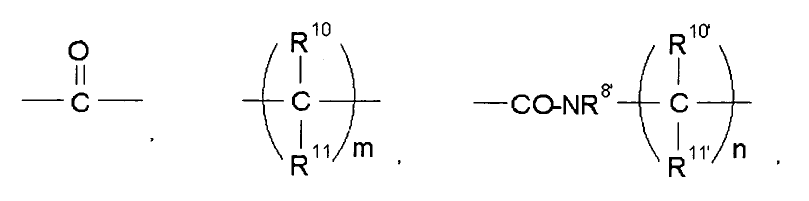 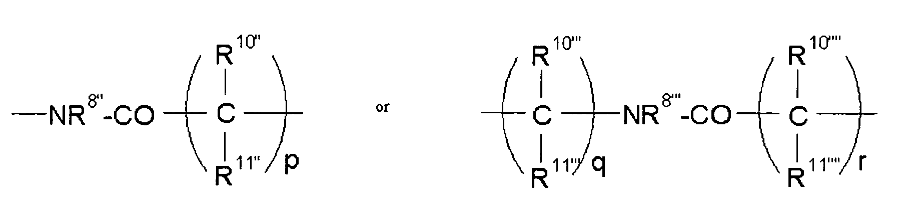 wherein R^{8'} represents a hydrogen atom or C₁₋₃ alkyl; R^{8"} represents a hydrogen atom or C₁₋₃ alkyl; R^{8"'} represents a hydrogen atom or C₁₋₃ alkyl; R¹⁰ represents a hydrogen atom or C₁₋₃ alkyl; R^{10'} represents a hydrogen atom or C₁₋₃ alkyl; R^{10"} represents a hydrogen atom or C₁₋₃ alkyl which may optionally contain a substituent (e.g., a 5- or 6-membered aromatic group, C₁₋₃ alkoxy, amino, mono- or di-C₁₋₆ alkylamino, etc.); R^{10"'} represents a hydrogen atom or C₁₋₃ alkyl; R^{10""} represents a hydrogen atom or C₁₋₃ alkyl; R¹¹ represents a hydrogen atom or C₁₋₃ alkyl; R^{11'} represents a hydrogen atom or C₁₋₃ alkyl; R^{11"} represents a hydrogen atom or C₁₋₃ alkyl; R^{11"'} represents a hydrogen atom or C₁₋₃ alkyl; R^{11""} represents a hydrogen atom or C₁₋₃ alkyl; m represents an integer of 1 to 4; n represents an integer of 0 to 4; p represents an integer of 1 to 4; q represents an integer of 1 to 3; and r represents an integer of 1 to 3;
R¹ represents amino which may optionally contain optionally halogenated C₁₋₆ alkyl, or a 5- or 6-membered nitrogen-containing heterocyclic group which may optionally contain amino;
R² represents phenyl or an aromatic 5- to 10-membered heterocyclic group;
R³ represents a hydrogen atom or cyano; and
Hal represents halogen.

Compound (I) is preferably, for example, a compound represented by the formula: 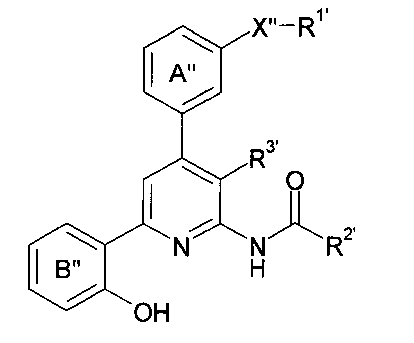 wherein Ring A" has the same significance as Ring A, Ring B" has the same significance as Ring B, X" has the same significance as X, and the other letters each have the same significance as above.

Examples of Ring A" include a benzene ring which may optionally have, other than -X-R¹; 1 to 2 substituent(s) selected from halogen, C₁₋₆ alkoxy, and mono- or di-C₁₋₆ alkylamino; and the like.

Examples of Ring B" include a benzene ring which may optionally have, other than hydroxy, 1 to 3 substituent(s) selected from halogen, optionally halogenated C₁₋₆ alkyl, and C₁₋₆ alkoxy; and the like.

Examples of R^{1'} include (1) amino which may optionally contain 1 or 2 substituent(s) selected from (a) C₁₋₆ alkyl which may optionally contain 1 to 3 substituent(s) selected from (i) hydroxy, (ii) amino which may optionally contain 1 to 2 substituent(s) selected from C₁₋₆ alkoxy-carbonyl, optionally halogenated C₁₋₆ alkyl-carbonyl, and C1-6 alkyl, (iii) imino, and (iv) C₁₋₆ alkoxy-carbonylimino, (b) C₇₋₁₉ aralkyloxy-carbonyl, (c) C₁₋₆ alkoxy-carbonyl, (d) C₁₋₆ alkyl-carbonyl, and (e) C₆₋₁₂ aryl; (2) a 5- or 6-membered nitrogen-containing heterocyclic group which may optionally contain 1 to 4 substituent(s) selected from amino, C₁₋₆ alkoxy-carbonyl, amino-C₁₋₆ alkyl, C₁₋₆ alkyl, and hydroxy; and the like.

Examples of R^{2'} include (1) a 5- or 6-membered aromatic heterocyclic group which may contain 1 to 3 substituent(s) selected from halogen, optionally halogenated C₁₋₆ alkyl, and optionally halogenated C₁₋₆ alkoxy and also may be optionally condensed with a benzene ring; (2) C₅₋₆ cycloalkyl; and the like.

Examples of R^{3'} include cyano, a hydrogen atom, C₁₋₆ alkoxy-carbonyl, carboxy, mono- or di-C₁₋₆ alkylamino-C₁₋₆ alkyl, and the like.

Examples of X" include a bond or a group represented by the formula: 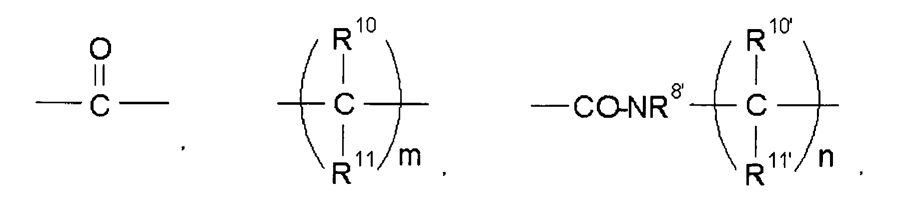 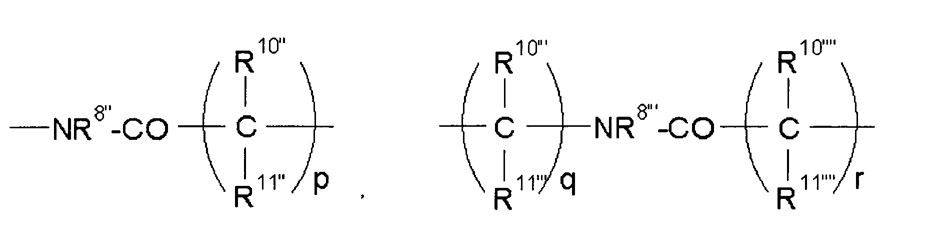 or 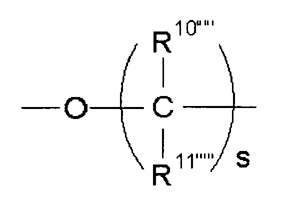 wherein each letter has the same significance as above.

Examples of R^{8'} include a hydrogen atom and the like.

Examples of R^{8"} include a hydrogen atom, C₁₋₃ alkyl, and the like.

Examples of R^{8"'} include a hydrogen atom and the like.

Examples of R¹⁰ include C₁₋₃ alkyl and the like.

Examples of R^{10'} include a hydrogen atom, C₁₋₃ alkyl, and the like.

Examples of R^{10"} include (1) a hydrogen atom, (2) C₁₋₃ alkyl which may optionally contain a substituent selected from a 5- or 6-membered aromatic group (e.g., imidazolyl, phenyl), C₁₋₃ alkoxy, amino, and mono- or di-C₁₋₆ alkylamino, (3) phenyl, and the like.

Examples of R^{10"'} include a hydrogen atom and the like.

Examples of R^{10""} include a hydrogen atom and the like.

Examples of R^{10""'} include a hydrogen atom and the like.

Examples of R¹¹ include a hydrogen atom and the like.

Examples of R^{11'} include a hydrogen atom, C₁₋₃ alkyl and the like.

Examples of R^{11"} include a hydrogen atom and the like.

Examples of R^{11"'} include a hydrogen atom and the like.

Examples of R^{11""} include a hydrogen atom and the like.

Examples of R^{11""'} include a hydrogen atom and the like.

R^{10"} and R^{11"} may form a 3- to 4-membered ring together with, for example, an adjacent carbon atom.

A preferable example of 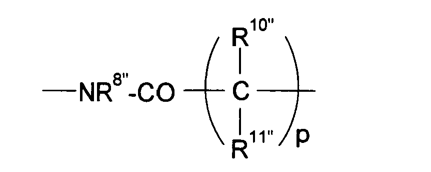 may be

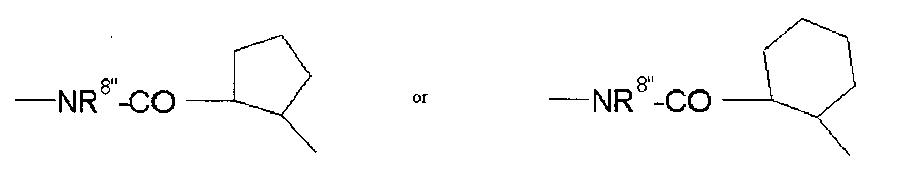

Examples of m include 1.

Examples of n include an integer of 0 to 3.

Examples of p include an integer of 0 to 3.

Examples of q include 1.

Examples of r include 2.

Examples of s include 3.

Examples of the "optionally substituted 5- to 8-membered homocyclic or heterocyclic group" represented by Ring A' in compound (I') may be the same as the examples of the "5- to 8-membered homocyclic or heterocyclic group" in the "5- to 8-membered homocyclic or heterocyclic group optionally having a substituent other than a group represented by formula -X-R¹" represented by Ring A in compound (I).

Examples of the "substituent" in the "optionally substituted 5- to 8-membered homocyclic or heterocyclic group" represented by Ring A' may be, for example, the same as the examples of (i) a group represented by -X-R¹, wherein each letter has the same significance as above, (ii) the "substituent" in the "optionally substituted hydrocarbon group" represented by R⁵; and the like.

Ring A' may contain 1 to 5, preferably 1 to 3, of the substituents described above at any substitutable positions; with proviso that if there are two or more substituents, the substituents may be the same or different.

Preferable examples of Ring B in compound (I') include a benzene ring which may optionally have halogen other than hydroxy.

Examples of the "optionally condensed pyridine ring" in the "optionally substituted or optionally condensed pyridine ring" represented by Ring C in compound (I') include a pyridine ring which may be optionally condensed with a 5- to 14-membered (preferably 5- to 10-membered) aromatic heterocyclic group, a 3- to 14-membered non-aromatic heterocyclic group, C₃₋₆ cycloalkane, C₅₋₈ cycloalkene, benzene or naphthalene. Specific examples of such a pyridine ring include the following condensed rings.

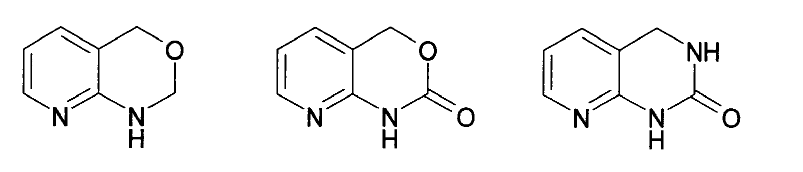

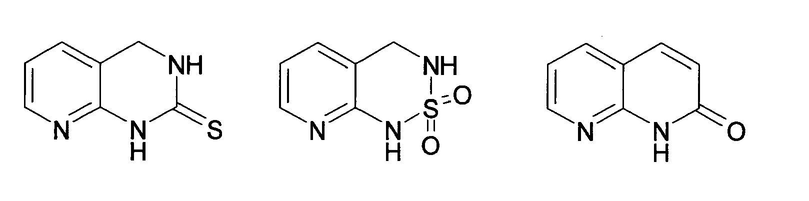

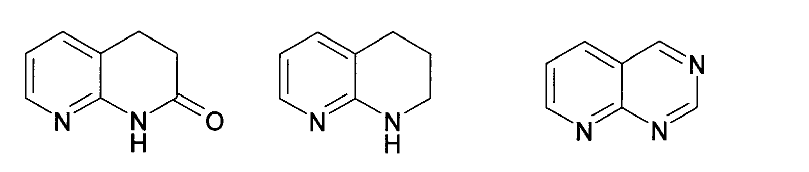

Examples of the "substituent" which may be included in the "optionally condensed pyridine ring" may be the same as the examples of the "substituent" in the "optionally substituted hydrocarbon group" represented by R⁵.

The optionally condensed pyridine ring may contain 1 to 5, preferably 1 to 3, of the substituents described above at any substitutable positions; with proviso that if there are two or more substituents, the substituents may be the same or different.

Preferable examples of compound (I') include a compound represented by the formula: 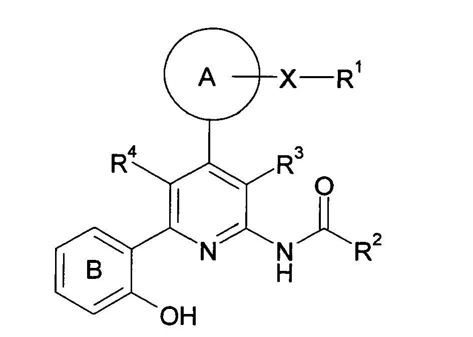 wherein each letter has the same significance as above.

Examples of salts of the compounds and intermediates thereof according to the present invention include metal salts, ammonium salts, salts with organic bases, salts with inorganic acids, salts with organic acids, salts with basic or acidic amino acids, and the like. Preferable examples of the metal salts include alkali metal salts such as sodium salts, potassium salts, etc.; alkaline earth metal salts such as calcium salts, magnesium salts, barium salts, etc.; aluminum salts; and the like. Preferable examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferable examples of the salts with inorganic acids include salts with hydrochloric acid, hydrobromic acid, nitric acid, sulfuric acid, phosphoric acid, etc. Preferable examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferable examples of the salts with basic amino acids include salts with arginine, lysine, ornithine, etc. Preferable examples of the salts with acidic amino acids include salts with aspartic acid, glutamic acid, etc.

Of these salts, pharmaceutically acceptable salts are preferred. Where the compounds have, for example, acidic functional groups, examples the pharmaceutically acceptable salts include inorganic salts such as alkali metal salts (e.g., sodium salts, potassium salts, etc.), alkaline earth metal salts (e.g., calcium salts, magnesium salts, barium salts, etc.), and the like; ammonium salts; etc. Where the compounds have basic functional groups, examples of the pharmaceutically acceptable salts include salts with inorganic acids such as hydrobromic acid, nitric acid, sulfuric acid, phosphoric acid, etc,; and salts with organic acids such as acetic acid, phthalic acid, fumaric acid, oxalic acid, tartaric acid, maleic acid, citric acid, succinic acid, methanesulfonic acid, p-toluenesulfonic acid, etc.

A compound according to the present invention may be a hydrate or a non-hydrate. Examples of the hydrate include 0.5 hydrate, 1 hydrate, 1.5 hydrate, 2 hydrate and the like.

A compound according to the present invention can be optionally obtained in a targeted R or S form by a known method, for example, asymmetric synthesis, optical resolution or the like.

A "prodrug" of a compound according to the present invention refers to a compound which is converted into a compound of the present invention by a reaction with an enzyme, gastric acid or the like under a physiological condition in a living body, namely, a compound which is changed into a compound of the present invention as a result of enzymatic oxidation, reduction, hydrolysis or the like, or a compound which is changed into a compound of the present invention as a result of hydrolysis or the like caused by gastric acid or the like. Examples of the prodrug of a compound of the present invention include a compound obtained as a result of acylation, alkylation or phosphorylation of amino in the compound of the present invention (e.g., a compound obtained as a result of eicosanoylation, alanylation, pentylaminocarbonylation, (5-methyl-2-oxo-1 ,3-dioxolene-4-yl)methoxycarbonylation, tetrahydrofuranylation, pyrrolidylmethylation, pivaloyloxymethylation, or tert-butylation of amino in the compound of the present invention, etc.); a compound obtained as a result of acylation, alkylation, phosphorylation or boration of hydroxy in the compound of the present invention (e.g., a compound obtained as a result of acetylation, palmitoylation, propanoylation, pivaloylation, succinylation, fumarylation, alanylation, or dimethylaminomethylcarbonylation of hydroxy in the compound of the present invention, etc.); a compound obtained as a result of esterification or amidation of carboxy in the compound of the present invention (e.g., a compound obtained as a result of ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1 ,3-dioxolene-4-yl)methylesterification, cyclohexyloxycarbonylethylesterification, or methylamidation of hydroxy in the compound of the present invention, etc.); and the like. These compounds can be produced from the compound of the present invention by a known method.

A prodrug of a compound according to the present invention may be a compound which is changed into a compound according to the present invention under a physiological condition as described in Iyakuhin no Kaihatsu (Development of Pharmaceuticals), Vol. 7, Bunshi Sekkei (Molecular Design), pp. 163-198, published by Hirokawa Shoten.

Now, methods for producing a compound according to the present invention will be described.

In the following production methods, alkylation reaction, amidation reaction (condensation reaction), esterification reaction, reduction reaction, reduction-type amination reaction, amination reaction, halogenation reaction, oxidation reaction and the like are performed in accordance with a known method. Examples of such a method include methods described in Organic Functional Group Preparations, 2nd edition, Academic Press, Inc. (1989), Comprehensive Organic Transformations, VCH Publishers Inc. (1989), and the like. Protection reaction and deprotection reaction are performed by a known method, for example, a method described in Protective Groups in Organic Synthesis, 3rd edition, John Wiley and Sons, Inc. (1999), or a method conformed thereto.

In each of the following production methods, in the case where a material compound can form a salt, such a compound may be used as a salt. As such a salt, the salts listed above as examples of the salt of compound (I) are used.

A target substance obtained in each of the following production methods can be isolated and purified by known separation and purification means, for example, concentration, concentration under reduced pressure, extraction with a solvent, crystallization, recrystallization, change of solvent, chromatography or the like. In the case where the production method include a plurality of steps, the intermediate obtained during the synthesis may be isolated and purified by known separation and purification means or used for the next step as a reaction mixture without being isolated or purified.

The solvents described with a generic name in the following reactions are as follows.

Solvents used as "alcohols" are, for example, methanol, ethanol, 1-propanol, 2-propanol, tert-butylalcohol, and the like.

Solvents used as "ethers" are, for example, diethylether, diisopropylether, diphenylether, tetrahydrofuran, 1,4-dioxane, 1,2-dimethoxyethane, and the like.

Solvents used as "esters" are, for example, ethyl acetate, methyl acetate, tert-butyl acetate, and the like.

Solvents used as "hydrocarbons" are, for example, benzene, toluene, xylene, cyclohexane, hexane, pentane, and the like.

Solvents used as "amides" are, for example, N,N-dimethylformamide, N,N-dimethylacetamide, hexamethylphosphoric triamide, and the like.

Solvents used as "halogenated hydrocarbons" are, for example, dichloromethane, chloroform, carbon tetrachloride, 1,2-dichloroethane, tetrachloroethylene, chlorobenzene, and the like.

Solvents used as "nitriles" are, for example, acetonitrile, propionitrile, and the like.

Solvents used as "ketones" are, for example, acetone, 2-butanone, and the like.

Solvents used as "organic acids" are, for example, formic acid, acetic acid, propionic acid, trifluoroacetic acid, methanesulfonic acid, and the like.

Solvents used as "aromatic amines " are, for example, pyridine, 2,6-lutidine, quinoline, and the like.

Solvents used as "sulfoxides " are, for example, dimethylsulfoxide, and the like.

Compound (I) can be produced by, for example, any of Method A through Method G described below or a method conformed thereto.

### <Method A>

Compound (I) is produced by the following method or a method conformed thereto.

Compound (IIa) is acylated with compound (1a-1), and then optionally treated with a base, to produce compound (I). 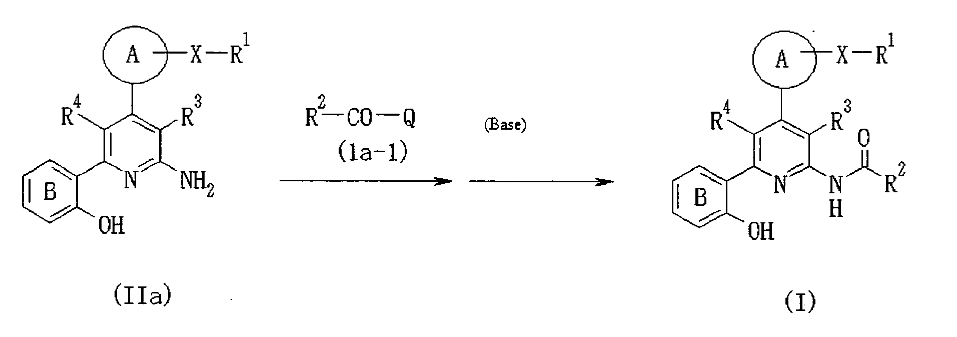 wherein Q represents a leaving group, and the other letters each have the same significance as above.

Examples of the leaving group represented by Q include a halogen atom (e.g., chlorine, bromine, iodine), optionally halogenated C₁₋₆ alkylsulfonyloxy (e.g., methylsulfonyloxy, ethylsulfonyloxy, trifluoromethylsulfonyloxy), C₆₋₁₀ arylsulfonyloxy which may be optionally substituted with C₁₋₆ alkyl (e.g., benzenesulfonyloxy, 4-toluenesulfonyloxy), methylmercapto, methanesulfonyl, and the like. A preferable example of the leaving group is a halogen atom.

A commercially available compound may be used as compound (1a-1) as it is, or compound (1a-1) may be produced by a known method or a method conformed thereto.

The acylation reaction may be performed in a solvent having no adverse influence on the reaction and optionally in the presence of a base.

Examples of such a solvent include alcohols, ethers, hydrocarbons, halogenated hydrocarbons, ketones, nitriles, amides, esters, aromatic amines, and the like. Preferable examples of such a solvent include ethers, hydrocarbons, halogenated hydrocarbons, amides, and aromatic amines. Two or more of these solvents may be mixed at an appropriate ratio.

Examples of the base include sodium hydride, potassium carbonate, sodium carbonate, potassium hydroxide, sodium hydroxide, triethylamine, diisopropylethylamine, and the like.

The amount of the base is usually 1 to 10 molar equivalent, preferably 1 to 5 molar equivalent, with respect to 1 mol of compound (IIa).

The amount of compound (1a-1) is usually 1 to 20 molar equivalent, preferably 2 to 10 molar equivalent, with respect to 1 mol of compound (IIa).

The reaction temperature is usually 0 to 100°C, preferably 20 to 60°C.

The reaction time is usually 0.5 to 100 hours, preferably 1 to 48 hours.

Where an N-diacylated form or a triacylated form is generated after the acylation reaction, compound (I) is produced by treating such a form with a base in a solvent having no adverse influence on the reaction.

Examples of such a solvent include water, alcohols, ethers, hydrocarbons, halogenated hydrocarbons, ketones, nitriles, amides, aromatic amines, and the like. Preferable examples of such a solvent are ethers, amides, and aromatic amines. Two or more of these solvents may be mixed at an appropriate ratio.

Examples of the base include potassium carbonate, sodium carbonate, potassium hydroxide, sodium hydroxide, ammonia, and the like.

The amount of the base is usually 1 to 20 molar equivalent, preferably 2 to 10 molar equivalent, with respect to 1 mol of compound (IIa).

The reaction temperature is usually 0 to 100°C, preferably 20 to 60°C.

The reaction time is usually 0.1 to 100 hours, preferably 0.5 to 24 hours.

### <Method B>

Compound (I) may be produced by the following method or a method conformed thereto.

Compound (IIb) is acylated with compound (1a-1) to obtain compound (III), and then compound (III) is subjected to deprotection reaction, to produce compound (I). 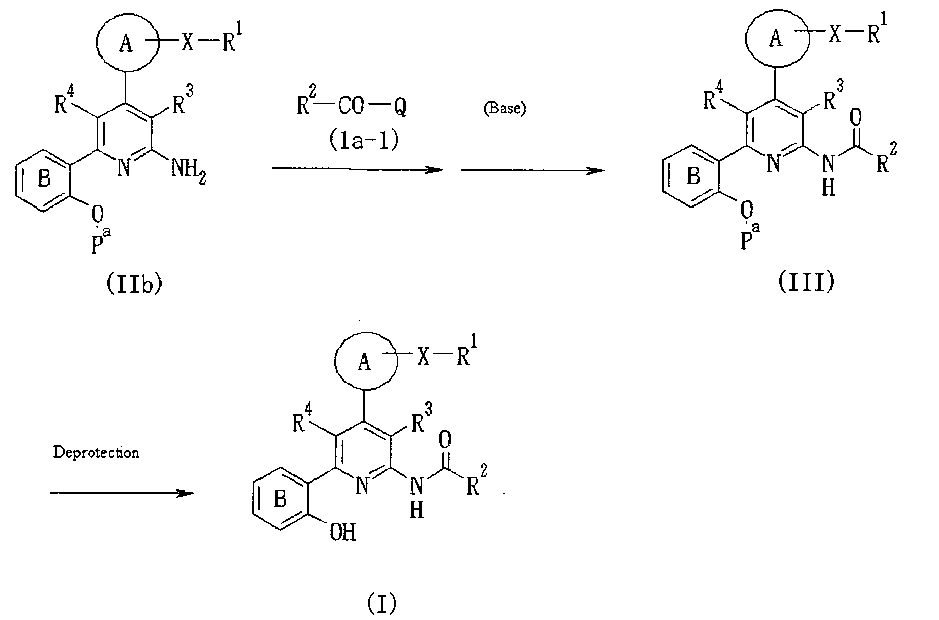 wherein P^{a} represents a protecting group, and the other letters each have the same significance as above.

Examples of the protecting group represented by P^{a} include acyl (e.g., acetyl, pivaloyl, benzoyl, etc.), alkyl (e.g., methyl, benzyl, methoxymethyl, benzyloxymethyl, phenacyl, tert-butyl, etc.), silyl (e.g., trimethylsilyl, t-butyldimethylsilyl, etc.), methanesulfonyl, toluenesulfonyl, allyl, and the like. Preferable examples of the protecting group include benzyl, tert-butyldimethylsilyl, methoxymethyl, and the like.

The acylation reaction is performed in accordance with the method described in Method A.

Where an N-diacylated compound is generated after the acylation reaction, compound (III) is produced in accordance with the method described in Method A.

The deprotection reaction is performed, when P^{a} is benzyl, using catalytic hydrogenation (e.g., Pd-C catalyst), trimethylsilane iodide or the like; when P^{a} is methoxymethyl, using hydrochloric acid, sodium iodide or the like; and when P^{a} is tert-butyldimethylsilyl, using tetrabutylammonium fluoride or the like. These reactions are performed in a solvent having no adverse influence on the reaction.

Examples of such a solvent include alcohols, ethers, halogenated hydrocarbons, hydrocarbons, amides, nitriles, aromatic amines, esters, and the like. Two or more of these solvents may be mixed at an appropriate ratio.

The reaction temperature is usually 0 to 100°C, preferably 20 to 60°C.

The reaction time is usually 0.1 to 100 hours, preferably 0.5 to 24 hours.

Among the compounds represented as compound (I), compound (1a) in which X is -Xa-Y^{a}-Z^{a}-Xb- (wherein Y^{a} represents -NR^{8"}-, -S- or -O-, Z^{a} represents any one of -CO-, -SO₂- and C₁₋₆ alkylene, Xa and Xb independently represent a bond or a spacer, and the other letters each have the same significance as above) can be produced by, for example, Method C described below or a method conformed thereto.

### <Method C>

Compound (IIc) is acylated with compound (1a-1), and then subjected to deprotection reaction to obtain compound (IIIa). The resultant compound (IIIa) and compound (1a-2) are reacted, and then subjected to deprotection reaction, to produce compound (Ia). 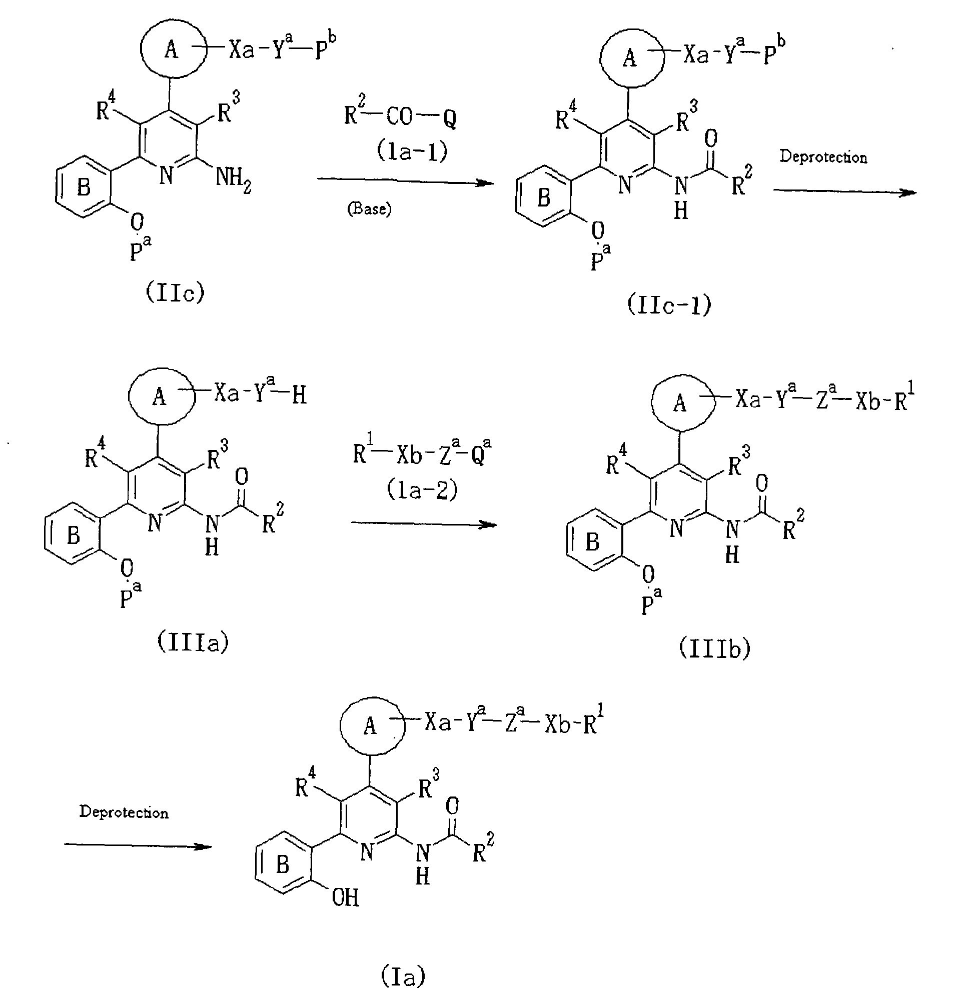 wherein P^{b} represents a protecting group, Q^{a} represents a leaving group, and the other letters each have the same significance as above.

Examples of the leaving group represented by Q^{a} include hydroxy, a halogen atom (e.g., chlorine, bromine, iodine), optionally halogenated C₁₋₆ alkylsulfonyloxy (e.g., methylsulfonyloxy, ethylsulfonyloxy, trifluoromethylsulfonyloxy), C₆₋₁₀ arylsulfonyloxy which may be optionally substituted with C₁₋₆ alkyl (e.g., benzenesulfonyloxy, 4-toluenesulfonyloxy), methylmercapto, methanesulfonyl, and the like. A preferable example of the leaving group is a halogen atom.

When Y^{a} is -NR^{8"}-, P^{b} may be, for example, formyl, C₁₋₆ alkylcarbonyl (e.g., acetyl, propionyl, etc.), C₁₋₆ alkoxycarbonyl (e.g., methoxycarbonyl, ethoxycarbonyl, tert-butoxycarbonyl), C₆₋₁₂ arylcarbonyl (e.g., benzoyl), C₇₋₁₃ aralkylcarbonyl (e.g., benzylcarbonyl), C₇₋₁₃ aralkyloxy-carbonyl (e.g., benzyloxycarbonyl, 9-fluorenylmethoxycarbonyl, etc.), trityl, phthaloyl, N,N-dimethylaminomethylene, silyl (e.g., trimethylsilyl, triethylsilyl, dimethylphenylsilyl, tert-butyldimethylsilyl, tert-butyldiethylsilyl, etc.), C₇₋₁₃ aralkyl (e.g., benzyl), C₂₋₆ alkenyl (e.g., 1-allyl), or the like. These groups may be substituted with 1 to 3 halogen atom(s) (e.g., fluorine, chlorine, bromine, iodine), C₁₋₆ alkoxy (e.g., methoxy, ethoxy, propoxy), nitro or the like. P^{b} is preferably trihalogenated acetyl, C₁₋₆ alkoxycarbonyl or C₇₋₁₃ aralkyloxycarbonyl; and is more preferably trifluoroacetyl, or tert-butoxycarbonyl.

When Y^{a} is -O-, P^{b} may be, for example, acyl (e.g., acetyl, pivaloyl, benzoyl, etc.), alkyl (e.g., methyl, benzyl, methoxymethyl, benzyloxymethyl, phenacyl, tert-butyl, etc.), silyl (e.g., trimethylsilyl, t-butyldimethylsilyl, etc.), methanesulfonyl, toluenesulfonyl, allyl, or the like. P^{b} is preferably benzyl, t-butyldimethylsilyl, methoxymethyl, or the like, and is more preferably benzyl, or methanesulfonyl.

The acylation reaction of compound (IIc) may be performed in a solvent having no adverse influence on the reaction by a method substantially the same as in Method B or a method conformed thereto.

The deprotection reaction performed after the acylation may be preformed at the same time as the treatment with a base, which is necessary when a diacylated compound is generated.

The reaction of compound (IIIa) and compound (1a-2) may be performed in a solvent having no adverse influence on the reaction and optionally in the presence of a base.

Examples of such a solvent include ethers, hydrocarbons, halogenated hydrocarbons, ketones, nitriles, amides, esters, aromatic amines, and the like. Preferable example of such a solvent are ethers, hydrocarbons, halogenated hydrocarbons, and amides. Two or more of these solvents may be mixed at an appropriate ratio.

Examples of the base include sodium hydride, potassium carbonate, sodium carbonate, potassium hydroxide, sodium hydroxide, triethylamine, diisopropylethylamine, and the like.

The amount of the base is usually 1 to 10 molar equivalent, preferably 1 to 5 molar equivalent, with respect to 1 mol of compound (IIIa).

The amount of compound (1a-2) is usually 1 to 20 molar equivalent, preferably 2 to 10 molar equivalent, with respect to 1 mol of compound (IIIa).

The reaction temperature is usually 0 to 100°C, preferably 20 to 60°C.

The reaction time is usually 0.5 to 100 hours, preferably 1 to 48 hours.

When Q^{a} is hydroxy, a condensing agent may be used.

Examples of the condensing agent include carbodiimide (e.g., dicyclohexylcarbodiimide (DCCD), water-soluble carbodiimide (WSCD), etc.), phosphoric acid ester (e.g., diethyl cyanophosphonate, diethyl chlorophosphonate, diphenylphosphoroazide, etc.), BOP reagent (1H-benzotriazole-1-yloxytripyrrolidinophosphonium hexafluorophosphate (PyBOP)), 2-ethoxy-1-ethoxycarbonyl-1,2-dihydroquinoline (EEDQ), carbonyldiimidazole, and the like. A preferable example of the condensing agent is WSCD.

Among the compounds represented as compound (I), compound (1b) in which X is -Xa-CO-Z^{b}-Xb- (wherein Z^{b} represents -NR^{8'}- or -O-, and the other letters each have the same significance as above) can be produced by, for example, Method D described below or a method conformed thereto.

### <Method D>

Compound (IId) is acylated with compound (1a-1) (optionally monoacylated by treatment with a base), and then subjected to deprotection reaction to obtain compound (IIIc). The resultant compound (IIIc) and compound (1a-3) are reacted, and then subjected to deprotection reaction, to produce compound (Ib). 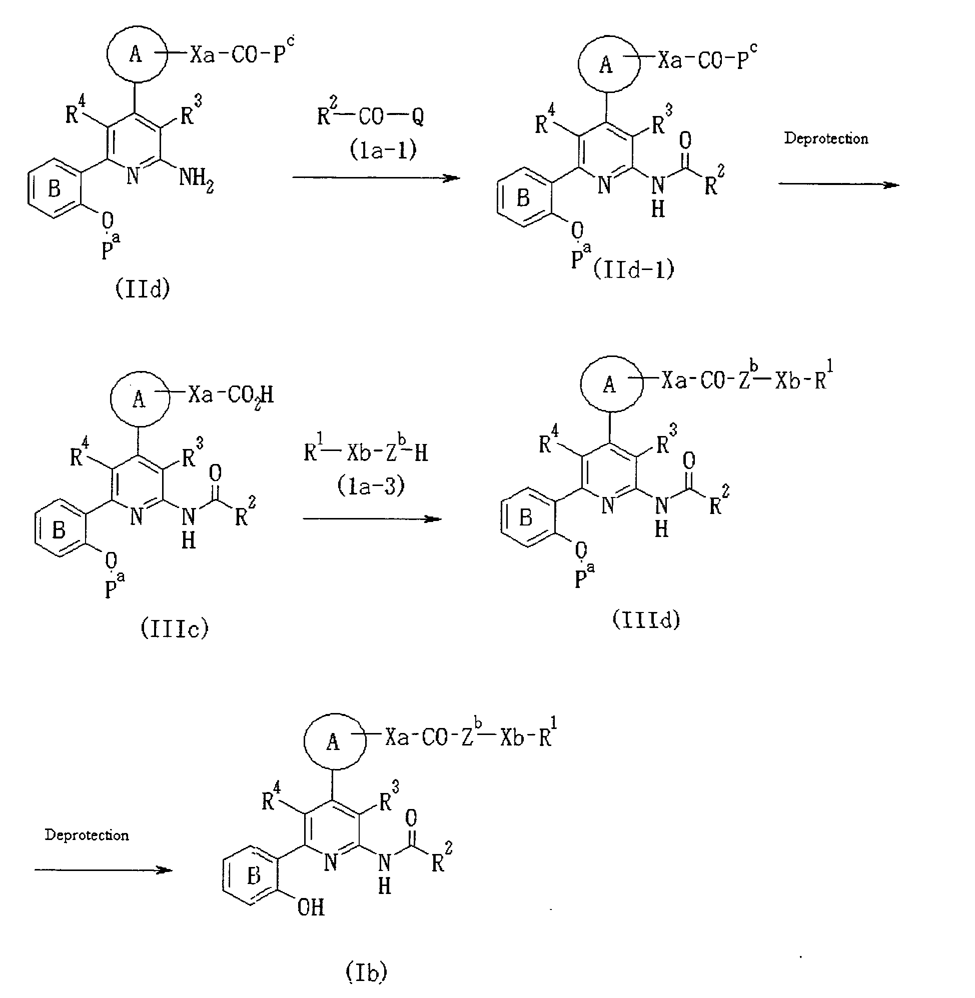 wherein P^{c} represents a protecting group, and the other letters each have the same significance as above.

Examples of P^{c} include alkoxy (e.g., methoxy, ethoxy, benzyloxy, t-butoxy, phenoxy, etc.), silyloxy (e.g., trimethylsilyloxy, tert-butyldimethylsilyloxy, etc.), and the like. P^{c} is preferably methoxy, ethoxy, tert-butoxy, or benzyloxy; and is more preferably benzyl, methoxy, or the like.

A commercially available compound may be used as compound (1a-3) as it is, or compound (1a-3) may be produced by a known method or a method conformed thereto.

The acylation reaction of compound (IId) is performed in a solvent having no adverse influence on the reaction by a method substantially the same as in Method B or a method conformed thereto.

The deprotection reaction performed after the acylation is preformed by a method substantially the same as in Method B or a method conformed thereto, and may be performed at the same time as the treatment with a base, which is necessary when a diacylated compound is generated.

The reaction of compound (IIIc) and compound (1a-3) may be performed using a condensing agent, in a solvent having no adverse influence on the reaction and optionally in the presence of a base.

As the condensing agent, substantially the same compounds as used in Method C may be used. Preferably, WSCD is used.

The amount of the condensing agent is usually 1 to 10 molar equivalent, preferably 1 to 5 molar equivalent, with respect to 1 mol of compound (IIIc).

As the solvent, the same compounds as used in the reaction of compound (IIIa) and compound (1a-2) in Method C are usable. Preferably, ethers, hydrocarbons, halogenated hydrocarbons, and amides are usable. Two or more of these solvents may be mixed at an appropriate ratio.

Examples of the base include triethylamine, diisopropylethylamine, and the like.

The amount of the base is usually 1 to 10 molar equivalent, preferably 1 to 5 molar equivalent, with respect to 1 mol of compound (IIIc).

When R¹ in compound (I) is protected primary or secondary amino, or a nitrogen-containing heterocyclic group having protected primary or secondary amino, an amino compound (Ic) removed the protecting group and a compound (Ie) obtained as a result of acylation or imination of the amino compound (Ic) can be produced by, for example, Method E described below or a method conformed thereto.

### <Method E>

Compound (Id) is subjected to deprotection reaction to obtain compound (Ic). The resultant compound (Ic) and compound (2a) are reacted, to produce compound (Ie). 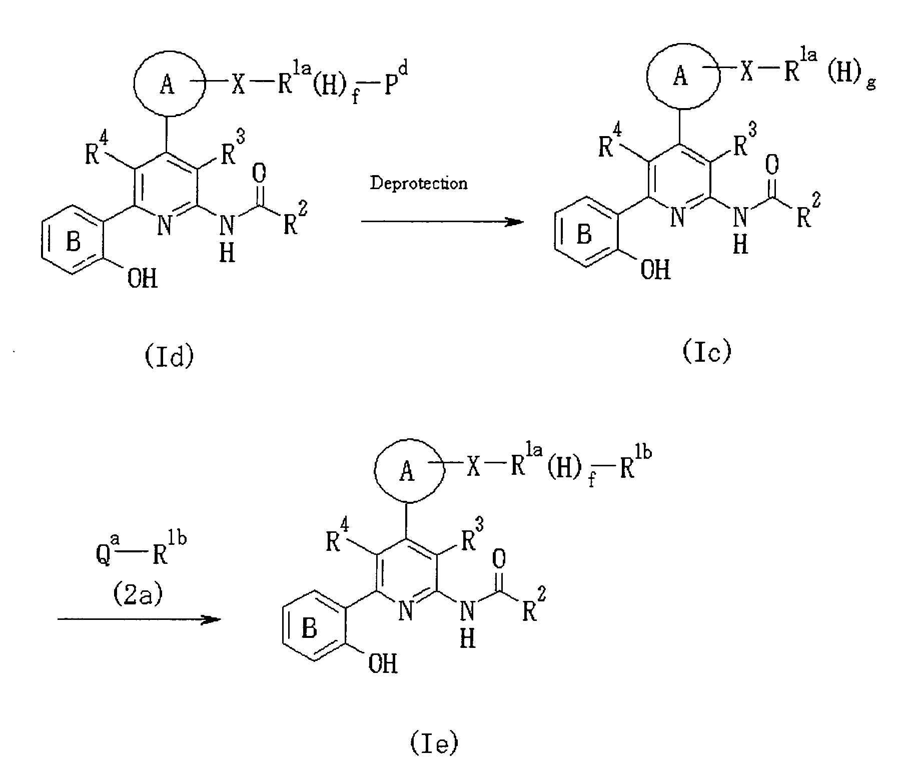 wherein P^{d} represents a protecting group; R^{1a} represents primary or secondary amino, or a nitrogen-containing heterocyclic group having protected primary or secondary amino; R^{1b} represents acyl or imino; f represents 0 or 1; g represents 1 or 2; and the other letters each have the same significance as above.

Examples of P^{d} include formyl, C₁₋₆ alkylcarbonyl (e.g., acetyl, propionyl, etc.), C₁₋₆ alkoxycarbonyl (e.g., methoxycarbonyl, ethoxycarbonyl, tert-butoxycarbonyl), C₆₋₁₂ arylcarbonyl (e.g., benzoyl), C₇₋₁₃ aralkylcarbonyl (e.g., benzylcarbonyl), C₇₋₁₃ aralkyloxy-carbonyl (e.g., benzyloxycarbonyl, 9-fluorenylmethoxycarbonyl, etc.), trityl, phthaloyl, N,N-dimethylaminomethylene, silyl (e.g., trimethylsilyl, triethylsilyl, dimethylphenylsilyl, tert-butyldimethylsilyl, tert-butyldiethylsilyl, etc.), C₇₋₁₃ aralkyl (e.g., benzyl), C₂₋₆ alkenyl (e.g., 1 -allyl), and the like. These groups may be substituted with 1 to 3 halogen atom(s) (e.g., fluorine, chlorine, bromine, iodine), C₁₋₆ alkoxy (e.g., methoxy, ethoxy, propoxy), nitro or the like. p^{a} is preferably C₁₋₆ alkoxycarbonyl or C₇₋₁₃ aralkyloxycarbonyl, and is more preferably benzyloxycarbonyl, or tert-butoxycarbonyl.

A commercially available compound may be used as compound (2a) as it is, or compound (2a) may be produced by a known method or a method conformed thereto.

The deprotection reaction is performed in a solvent having no adverse influence on the reaction.

Examples of such a solvent include alcohols, ethers, hydrocarbons, halogenated hydrocarbons, nitriles, amides, esters, aromatic amines, and the like. Preferable examples of such a solvent are ethers, hydrocarbons, halogenated hydrocarbons, and amides. Two or more of these solvents may be mixed at an appropriate ratio.

The reaction of compound (Ic) and compound (2a) may be performed in a solvent having no adverse influence on the reaction and optionally in the presence of a base.

Examples of such a solvent include ethers, hydrocarbons, halogenated hydrocarbons, ketones, nitriles, amides, esters, aromatic amines, and the like. Preferable examples of such a solvent are ethers, hydrocarbons, halogenated hydrocarbons, and amides. Two or more of these solvents may be mixed at an appropriate ratio.

Examples of the base include sodium hydride, potassium carbonate, sodium carbonate, potassium hydroxide, sodium hydroxide, triethylamine, diisopropylethylamine, and the like. The amount of the base is usually 1 to 10 molar equivalent, preferably 1 to 5 molar equivalent, with respect to 1 mol of compound (Ic).

The amount of compound (2a) is usually 1 to 20 molar equivalent, preferably 2 to 10 molar equivalent, with respect to 1 mol of compound (Ic).

The reaction temperature is usually 0 to 100°C, preferably 20 to 60°C.

The reaction time is usually 0.5 to 100 hours, preferably 1 to 48 hours.

When the leaving group represented by Q^{a} is hydroxy, a condensing agent may be used. As the condensing agent, the same compounds as used in Method C are usable. Preferably, WSCD is used.

Among the compounds represented as compound (Ic), compound (If) in which R^{1a} is amino can be produced by, for example, Method F described below or a method conformed thereto. Among the compounds represented as compound (IIIa), compound (IIIa-1) in which Y^{a} is -NH- can also be produced by, for example, Method F described below or a method conformed thereto.

### <Method F>

Compound (IIe) is acylated to obtain compound (IIIe). The resultant compound (IIIe) is subjected to reduction reaction to obtain compound (IIIa-1). The resultant compound (IIIa-1) is subjected to deprotection reaction, to produce compound (If).

Alternatively, the resultant compound (IIIe) is subjected to deprotection reaction to obtain compound (IIIe-1). The resultant compound (IIIe-1) is subjected to reduction reaction, to produce compound (If). 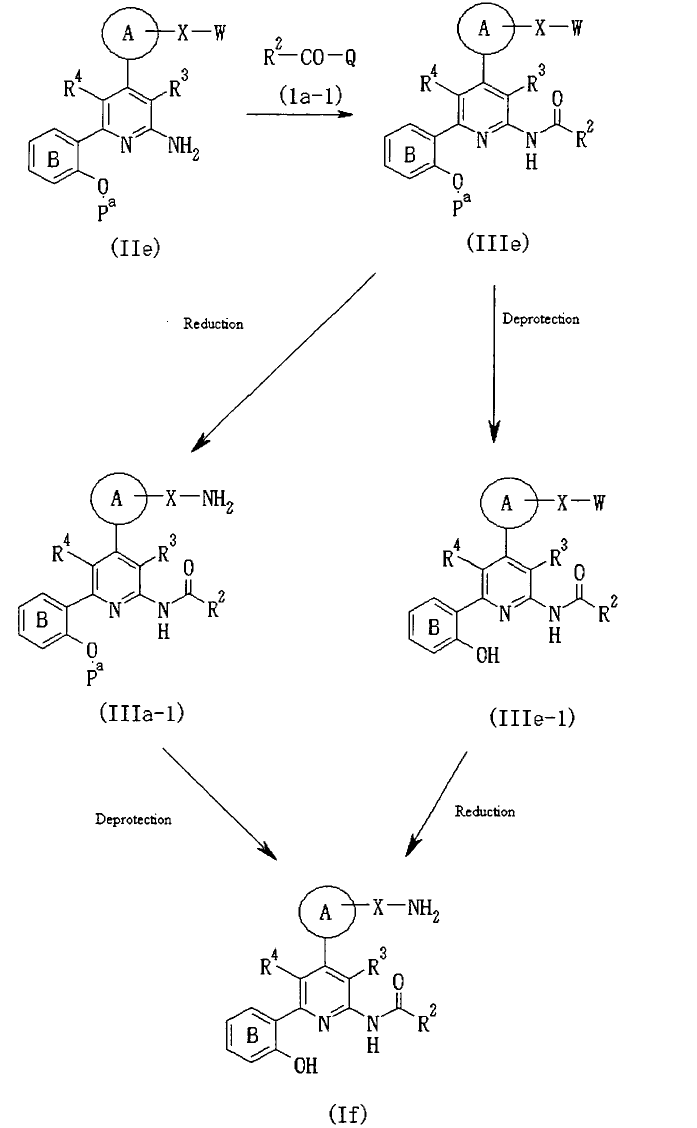 wherein W represents nitro or azide, and the other letters each have the same significance as above.

The acylation of compound (IIe) into compound (IIIe) is performed in accordance with the method described in Method B.

The reduction reaction of compound (IIIe) and compound (IIIe-1) is performed using catalytic reduction reaction or a metal hydride (e.g., lithium aluminum hydride, diisobutylaluminum hydride, lithium borate hydride, etc.).

Examples of the catalyst for the reduction reaction include platinum oxide; palladium, ruthenium, rhodium, iridium supported by activated carbon, barium sulfate, calcium carbonate, and Raney nickel, etc.; and the like. Examples of the source of hydrogen include hydrogen, cyclohexene, hydrazine, ammonium formate, and the like.

This reaction is performed in a solvent having no adverse influence on the reaction and optionally in the presence of an acid.

Examples of such a solvent include ethers, alcohols, hydrocarbons, ketones, nitriles, amides, esters, water, and the like. Preferable examples of such a solvent include alcohols and ethers. Two or more of these solvents may be mixed at an appropriate ratio.

Examples of the acid include organic carboxylic acid (e.g., formic acid, acetic, etc.), organic sulfonic acid (e.g., methanesulfonic acid, 4-toluenesulfonic acid, etc.), inorganic acid (e.g., hydrochloric acid, etc.), and the like.

The amount of the acid is usually 0.1 to 20 molar equivalent, preferably 0.1 to 10 molar equivalent, with respect to 1 mol of compound (IIIe) or compound (IIIe-1).

The amount of the catalyst is usually 0.01 to 5 grams, preferably 0.1 to 0.5 grams, with respect to 1 gram of compound (IIIe) or compound (IIIe-1).

When hydrogen is used, the pressure thereof is 1 to 10 atm, preferably 1 to 2 atm.

The reaction temperature is usually 0 to 100°C, preferably 20 to 60°C.

The reaction time is usually 0.5 to 100 hours, preferably 1 to 48 hours.

For the reduction reaction with a reducting agent such as a metal hydride (e.g., nickel borohydride, etc.) or the like, examples of the usable solvent include alcohols, ethers, hydrocarbons, halogenated hydrocarbons, nitriles, and amides. Two or more of these solvents may be mixed at an appropriate ratio.

The amount of the reducting agent such as a metal hydride is usually 1 to 50 molar equivalent, preferably 1 to 10 molar equivalent, with respect to 1 mol of compound (IIIe) or compound (IIIe-1).

The reaction temperature is usually -10 to 150°C, preferably 0 to 110°C.

The reaction time is usually 0.5 to 100 hours, preferably 1 to 48 hours.

When W is nitro, the reduction reaction may be performed in a solvent having no adverse influence on the reaction, with a combination of a metal material (e.g., iron, zinc, etc.) and an acidic reagent (e.g., hydrochloric acid, sulfuric acid, methanesulfonic acid, ammonium chloride, etc.).

Examples of such a solvent include alcohols, ethers, hydrocarbons, halogenated hydrocarbons, nitriles, amides, water, and the like. When a metal hydride is used, examples of the usable solvent include alcohols, ethers, hydrocarbons, halogenated hydrocarbons, nitriles, amides, and the like. Two or more of these solvents may be mixed at an appropriate ratio.

The amount of the metal or the acidic reagent is usually 1 to 50 molar equivalent, preferably 1 to 10 molar equivalent, with respect to 1 mol of compound (IIIe) or compound (IIIe-1).

The reaction temperature is usually -10 to 150°C, preferably 0 to 110°C.

The reaction time is usually 0.5 to 100 hours, preferably 1 to 48 hours.

The deprotection reaction is performed in accordance with the method described in Method B.

Among the compounds represented as compound (I), compound (1g) in which R¹ is NR^{1c}R^{1d} can also be produced by, for example, Method G described below or a method conformed thereto.

### <Method G>

Compound (IIIa-1) is subjected to alkylation reaction to obtain compound (IIIa-2). The resultant compound (IIIa-2) is subjected to deprotection reaction, to produce compound (Ig). 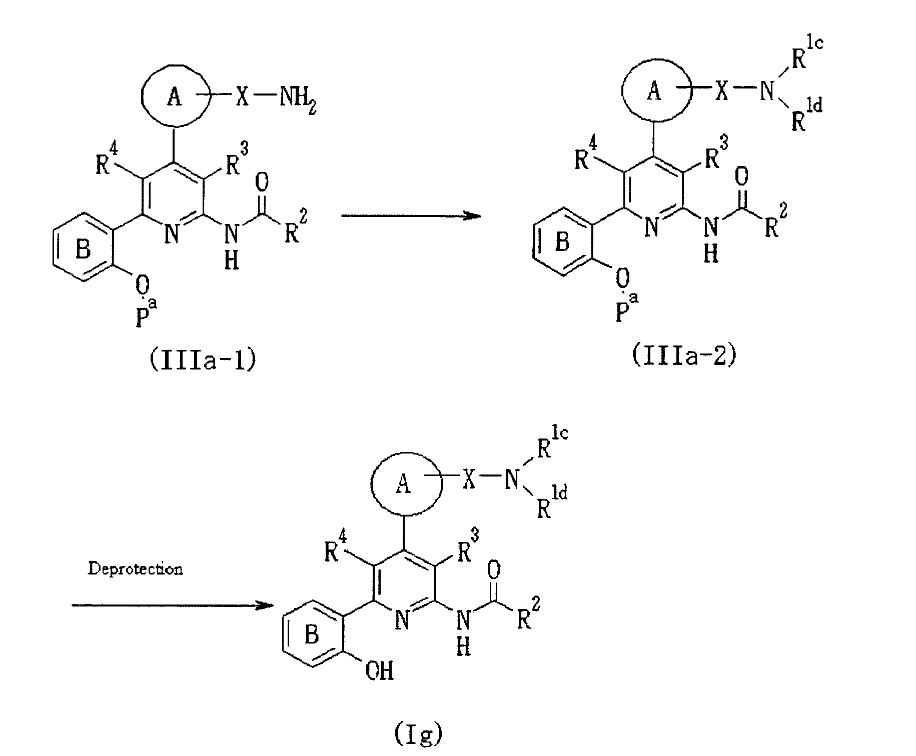 wherein R^{1c} represents optionally substituted alkyl, R^{1d} represents a hydrogen atom or optionally substituted alkyl, and the other letters each have the same significance as above.

The alkylation reaction of compound (IIIa-1) is performed in a solvent having no adverse influence on the reaction and optionally in the presence of an acid.

Examples of such a solvent include ethers, hydrocarbons, halogenated hydrocarbons, nitriles, and amides. Two or more of these solvents may be mixed at an appropriate ratio.

Examples of the reducting agent include sodium borohydride, sodium cyanobohydride, sodium triacetoxyborohydride, and the like.

The amount of the reductant is usually 1 to 5 molar equivalent, preferably 1 to 2 molar equivalent, with respect to compound (IIIa-1):

Examples of the acid include organic carboxylic acid (e.g., formic acid, acetic acid, etc.), inorganic protonic acid (e.g., hydrochloric acid, sulfuric acid, etc.), Lewis acid (e.g., zinc chloride, titanium chloride, etc.), and the like.

The amount of the acid is usually 0.1 to 10 molar equivalent, preferably 0.1 to 2 molar equivalent, with respect to compound (IIIa-1).

The amount of the carbonyl compound is usually 1 to 2 molar equivalent, preferably 1 to 1.2 molar equivalent, with respect to compound (IIIa-1).

The reaction temperature is usually -10 to 100°C, preferably 0 to 50°C.

The reaction time is usually 0.5 to 100 hours, preferably 1 to 48 hours.

The deprotection reaction may be performed in accordance with the method described in Method B.

Among the compounds represented as compound (IIIe) which are used as a starting material for Method F, compound (IIIe-2) in which W is azide can be synthesized by Method H.

### <Method H>

Compound (IIf) is subjected to azidation reaction to obtain compound (IIe-1). The resultant compound (IIe-1) is subjected to acylation reaction, to produce compound (IIIe-2). 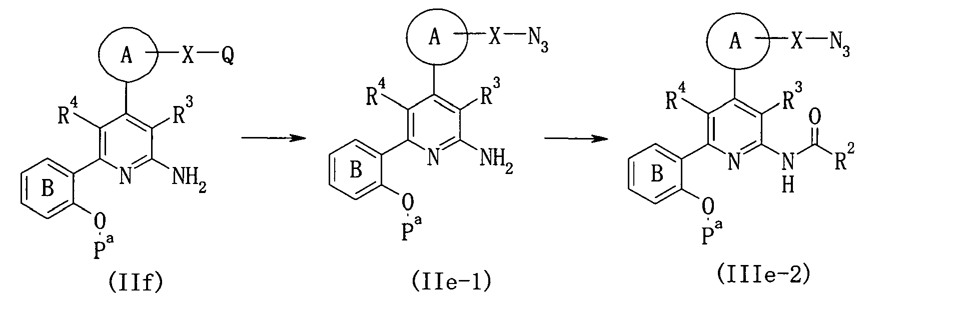 wherein each letter has the same significance as above.

The azidation reaction is performed in a solvent having no adverse influence on the reaction, by reacting compound (IIf) with an azide compound (e.g., sodium azide, trimethylsilyl azide, etc.).

Examples of such a solvent include ethers, hydrocarbons, halogenated hydrocarbons, nitriles, and amides. Two or more of these solvents may be mixed at an appropriate ratio.

The amount of the azide compound is usually 1 to 50 molar equivalent, preferably 1 to 10 molar equivalent, with respect to 1 mol of compound (IIf).

The reaction temperature is usually 0 to 150°C, preferably 20 to 110°C.

The reaction time is usually 0.5 to 100 hours, preferably 1 to 60 hours.

The acylation of compound (IIe-1) is performed in accordance with the method described in Method B.

Among the compounds represented as compound (IIc) which are used as a starting material for Method C, compound (IIc-2) in which Y^{a} is -NH- can also be produced by, for example, Method I described below or a method conformed thereto.

### <Method I>

Compound (IIe) is subjected to reduction reaction to obtain compound (IIg), to produce compound (IIg). Thus, compound (IIc-2) is produced. 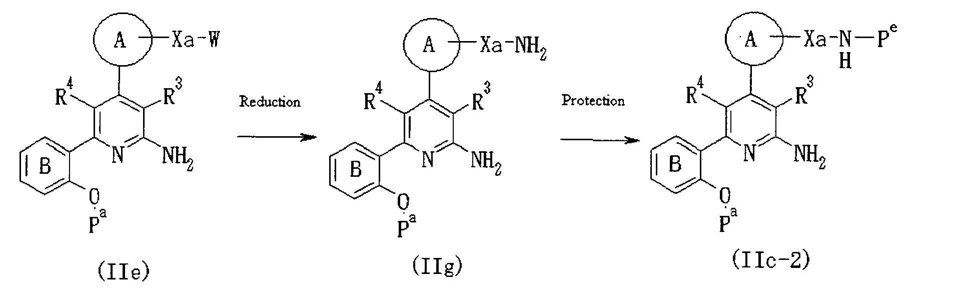 wherein P^{e} represents a protecting group, and the other letters each have the same significance as above.

Examples of the protecting group represented by P^{e} are the same as the examples of P^{b}. P^{e} is preferably trihalogenated acetyl, C₁₋₆ alkoxycarbonyl, or C₇₋₁₃ aralkyloxycarbonyl, and is more preferably trifluoroacetyl, tert-butoxycarbonyl, or the like.

The reduction reaction of compound (IIe) may be performed by the method described in Method F or a method conformed thereto.

The protection reaction of compound (IIg) may be performed using, for example, an acid anhydride such as trifluoroacetic anhydride, tert-butyl dicarbonate or the like, and optionally in a solvent having no adverse influence on the reaction.

Examples of such a solvent include ethers, hydrocarbons, halogenated hydrocarbons, nitriles, amides, and water. Two or more of these solvents may be mixed at an appropriate ratio.

Examples of the base include sodium carbonate, sodium hydrogencarbonate, sodium hydroxide, triethylamine, diisopropylethylamine, pyridine, 4-dimethylaminopyridine, and the like.

The amount of the base is usually 1 to 2 molar equivalent, preferably 1 to 1.5 molar equivalent, with respect to 1 mol of compound (IIg).

The amount of the acid anhydride used for the protection reaction is usually 1 to 2 molar equivalent, preferably 1 to 1.5 molar equivalent, with respect to 1 mol of compound (IIg).

The reaction temperature is usually -20 to 60°C, preferably 0 to 30°C.

The reaction time is usually 0.5 to 48 hours, preferably 1 to 24 hours.

Among the compounds represented as compound (IIc) which are used as a starting material for Method C, compound (IIc-3) in which Y^{a} is -NR^{8"'}- can also be produced by, for example, Method J described below or a method conformed thereto.

### <Method J>

Compound (IIc-2) and compound (3a) are reacted, to produce compound (IIc-3). 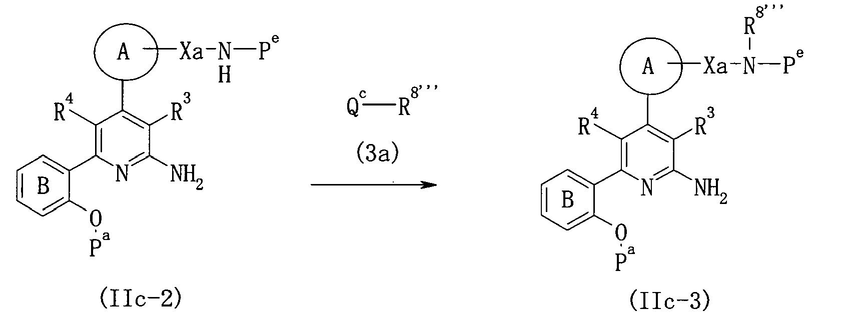 wherein Q^{c} represents a leaving group, R^{8"'} represents an optionally substituted hydrocarbon group or an optionally substituted heterocyclic group, and the other letters each have the same significance as above.

Examples of the leaving group represented by Q^{c} are the same as the examples of Q. Q^{c} is preferably a halogen atom.

A commercially available compound may be used as compound (3a) as it is, or compound (3 a) may be produced by a known method or a method conformed thereto.

The reaction of compound (IIc-2) and compound (3a) is performed in a solvent having no influence on the reaction and optionally in the presence of a base.

Examples of such a solvent include ethers, hydrocarbons, halogenated hydrocarbons, nitriles, and amides Two or more of these solvents may be mixed at an appropriate ratio.

Examples of the base include sodium carbonate, potassium carbonate, sodium hydride, and the like.

The amount of compound (3a) is usually 1 to 5 molar equivalent, preferably 1 to 2 molar equivalent, with respect to 1 mol of compound (IIc-2).

The reaction temperature is usually 0 to 100°C, preferably 20 to 80°C.

The reaction time is usually 0.5 to 100 hours, preferably 1 to 48 hours.

Among the compounds represented as compound (IIe) which are used as a starting material for Method H, compound (IIe-1) in which X is represented by formula -Xa-CH₂- can also be produced by Method K described below.

### <Method K>

Compound (IId-2) is subjected to reduction reaction to obtain compound (IIh). Hydroxy of the resultant compound (IIh) is substituted with a leaving group, to produce compound (IIe-1). 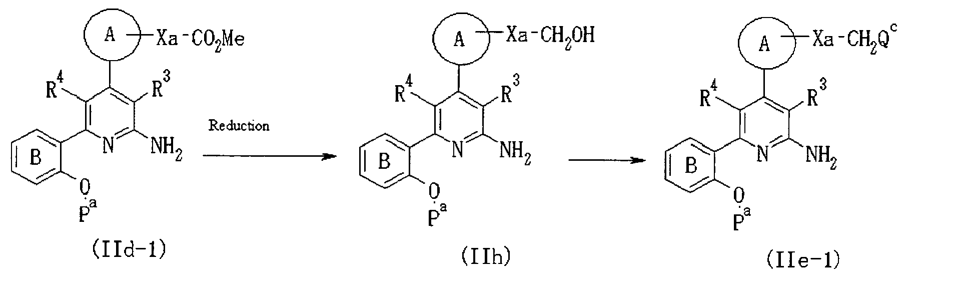 wherein Q^{c} represents a leaving group, and the other letters each have the same significance as above.

Examples of the leaving group represented by Q^{c} include a halogen atom (e.g., chlorine, bromine), and the like.

The reduction reaction of compound (IId-1) is performed in a solvent having no adverse on the reaction, using a metal hydride (e.g., lithium aluminum hydride, diisobutylaluminum hydride, lithium borohydride, etc.).

Examples of such a solvent include ethers, hydrocarbons, halogenated hydrocarbons, nitriles, and amides. Two or more of these solvents may be mixed at an appropriate ratio.

The amount of the metal hydride is usually 1 to 10 molar equivalent, preferably 1 to 5 molar equivalent, with respect to 1 mol of compound (IId-1).

The reaction temperature is usually -10 to 100°C, preferably 0 to 60°C.

The reaction time is usually 0.5 to 100 hours, preferably 1 to 24 hours.

The substitution reaction of hydroxy of compound (IIh) with a leaving group is performed in a solvent having no influence on the reaction, or with no solvent, using a halogenating reagent.

Examples of such a solvent include ethers, hydrocarbons, halogenated hydrocarbons, nitriles, and amides. Two or more of these solvents may be mixed at an appropriate ratio.

Examples of the halogenating reagent include thionyl chloride, phosphorus tribromide, phosphorus bromide, and the like. When the reaction is performed under a neutral condition, examples of the usable halogenating reagent include carbon tetrabromide-triphenylphosphine, N-bromosuccinimide-triphenylphosphine, and the like.

The amount of the halogenating reagent is usually 1 to 50 molar equivalent, preferably 1 to 20 molar equivalent, with respect to 1 mol of compound (IIh).

The reaction temperature is usually -10 to 120°C, preferably 20 to 100°C.

The reaction time is usually 0.5 to 100 hours, preferably 1 to 24 hours.

Among the compounds represented as compound (IIb), compound (IIf-1) in which R¹ is NR^{1c}R^{1d} can also be synthesized by Method L described below.

### <Method L>

Compound (IIf) and compound (4a) are reacted, to produce compound (IIf-1). 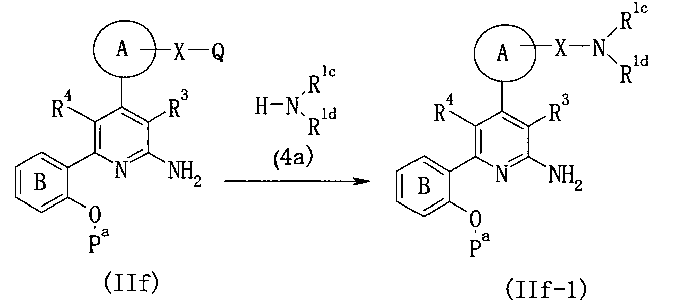 wherein each letter has the same significance as above.

A commercially available compound may be used as compound (4a) as it is, or compound (4a) may be produced by a known method or a method conformed thereto.

The reaction of compound (IIf) and compound (4a) is performed in a solvent having no influence on the reaction, or with no solvent.

Examples of such a solvent include alcohols, ethers, hydrocarbons, halogenated hydrocarbons, nitriles, and amides. Two or more of these solvents may be mixed at an appropriate ratio.

The amount of compound (4a) is usually 1 to 50 molar equivalent, preferably 1 to 2 molar equivalent, with respect to 1 mol of compound (IIf).

The reaction temperature is usually -10 to 120°C, preferably 20 to 100°C.

The reaction time is usually 0.5 to 100 hours, preferably 1 to 24 hours.

Among the compounds represented as compound (IIa), compound (IIb), compound (IIc), compound (IId), compound (IIe), compound (IIc-2), compound (IId-1) and compound (IIf) which are used as a starting material for Methods A through F and Methods H through L, a compound in which R³ is cyano, acyl or an optionally substituted hydrocarbon group can be produced by a known method, for example, the method described in WO 02/24679, WO 02/44153 or the like. Among the compounds represented as compound (IIc-1) which is an intermediate material of Method C, compound (IIc-1') in which R³ is hydrogen can be produced by, for example, Method M described below.

### <Method M>

Compound (IV) and compound (5a-1) are subjected to coupling reaction in the presence of a catalyst, to produce compound (IIc-1'). 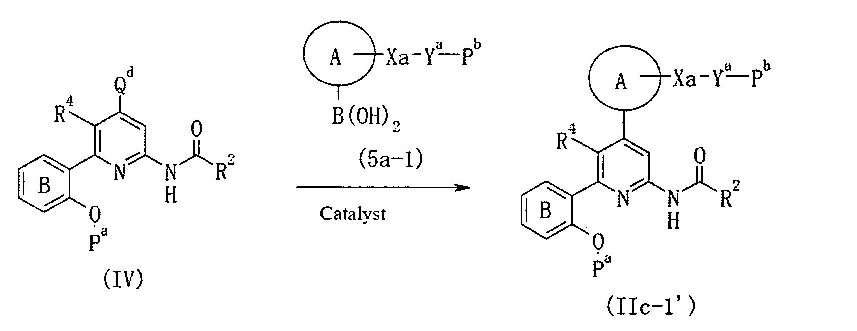 wherein Q^{d} represents a leaving group, and the other letters each have the same significance as above.

Examples of the leaving group represented by Q^{d} include a halogen atom (e.g., chlorine, bromine), trifluoromethanesulfonyloxy, and the like.

A commercially available compound may be used as compound (5a-1) as it is, or compound (5a-1) may be p 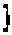 Catalyst a known method or a method conformed thereto.

The coupling reaction of compound (IV) and compound (5a-1) is performed in a solvent having no influence on the reaction and in the presence of a base, using a catalyst.

Examples of such a solvent include water, alcohols, ethers, hydrocarbons, halogenated hydrocarbons, amides, and the like. Two or more of these solvents may be mixed at an appropriate ratio.

The amount of compound (5a-1) is usually 1 to 5 molar equivalent, preferably 1 to 2 molar equivalent, with respect to 1 mol of compound (IV).

Examples of the catalyst include tetrakis(triphenylphosphine)palladium, palladium acetate, and the like. The amount thereof is usually 0.5 to 1 molar equivalent, preferably 0.05 to 0.5 molar equivalent, with respect to 1 mol of compound (IV).

Examples of the base include sodium carbonate, potassium carbonate, sodium phosphate, triethylamine, and the like.

The amount of the base is usually 2 to 10 molar equivalent, preferably 2 to 3 molar equivalent, with respect to 1 mol of compound (IV).

The reaction temperature is usually 20 to 180°C, preferably 80 to 130°C.

The reaction time is usually 0.5 to 100 hours, preferably 1 to 48 hours.

Among the compounds represented as compound (IId-1) which is an intermediate material of Method D, compound (IId-1') in which R³ is hydrogen can be produced by, for example, Method N described below.

### <Method N>

Compound (IV) and compound (5a-2) are subjected to coupling reaction in the presence of a catalyst, to produce compound (IId-1'). 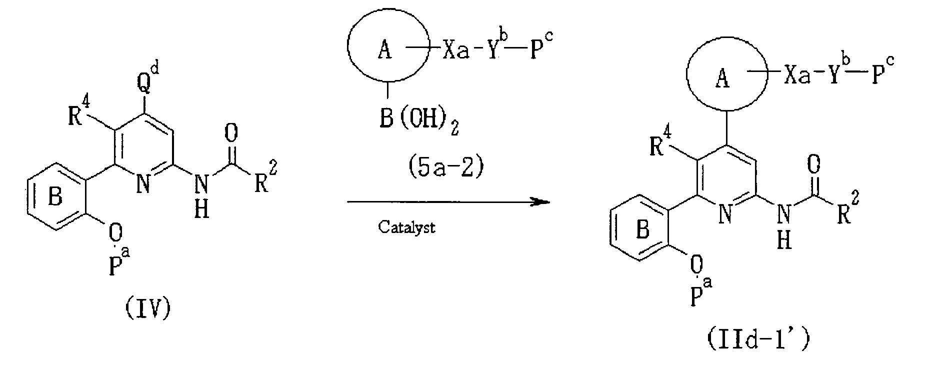 wherein each letter has the same significance as above.

A commercially available compound may be used as compound (5a-2) as it is, or compound (5a-2) may be produced by a known method or a method conformed thereto.

The coupling reaction of compound (IV) and compound (5a-2) may be performed in accordance with the method described in Method M.

Among the compounds represented as compound (IIIe) which is used as a starting material for Method F, compound (IIIe-3) in which R³ is hydrogen can be synthesized by, for example, Method O described below.

### <Method O>

Compound (IV) and compound (5a-3) are subjected to coupling reaction in the presence of a catalyst, to produce compound (IIIe-3). 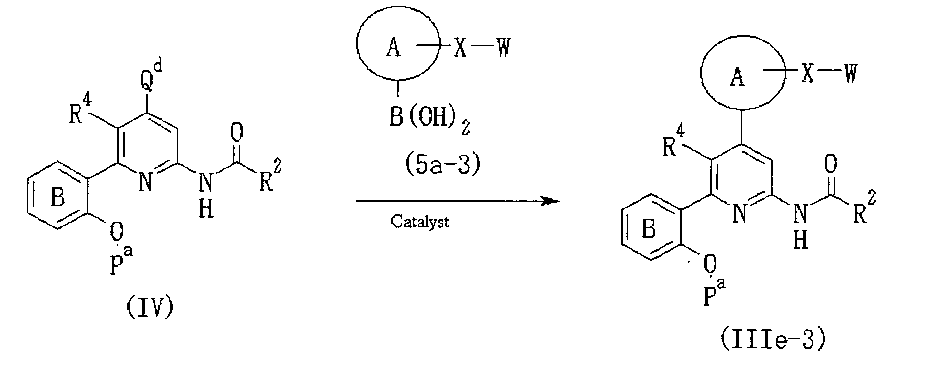 wherein each letter has the same significance as above.

A commercially available compound may be used as compound (5a-3) as it is, or compound (5a-3) may be produced by a known method or a method conformed thereto.

The coupling reaction of compound (IV) and compound (5a-3) may be performed in accordance with the method described in Method M.

Compound (IV) which is used as a starting material for Method M, Method N and Method O can be synthesized by, for example, Method P described below.

### <Method P>

Compound (V) and ammonia are reacted, and then hydrolyzed to obtain compound (VI). Compound (VI) is subjected to halogenation reaction and esterification reaction to obtain compound (VII). Compound (VII) is subjected to hydrolysis reaction, Curtius rearrangement reaction, and then acylation reaction, to produce compound (IV). 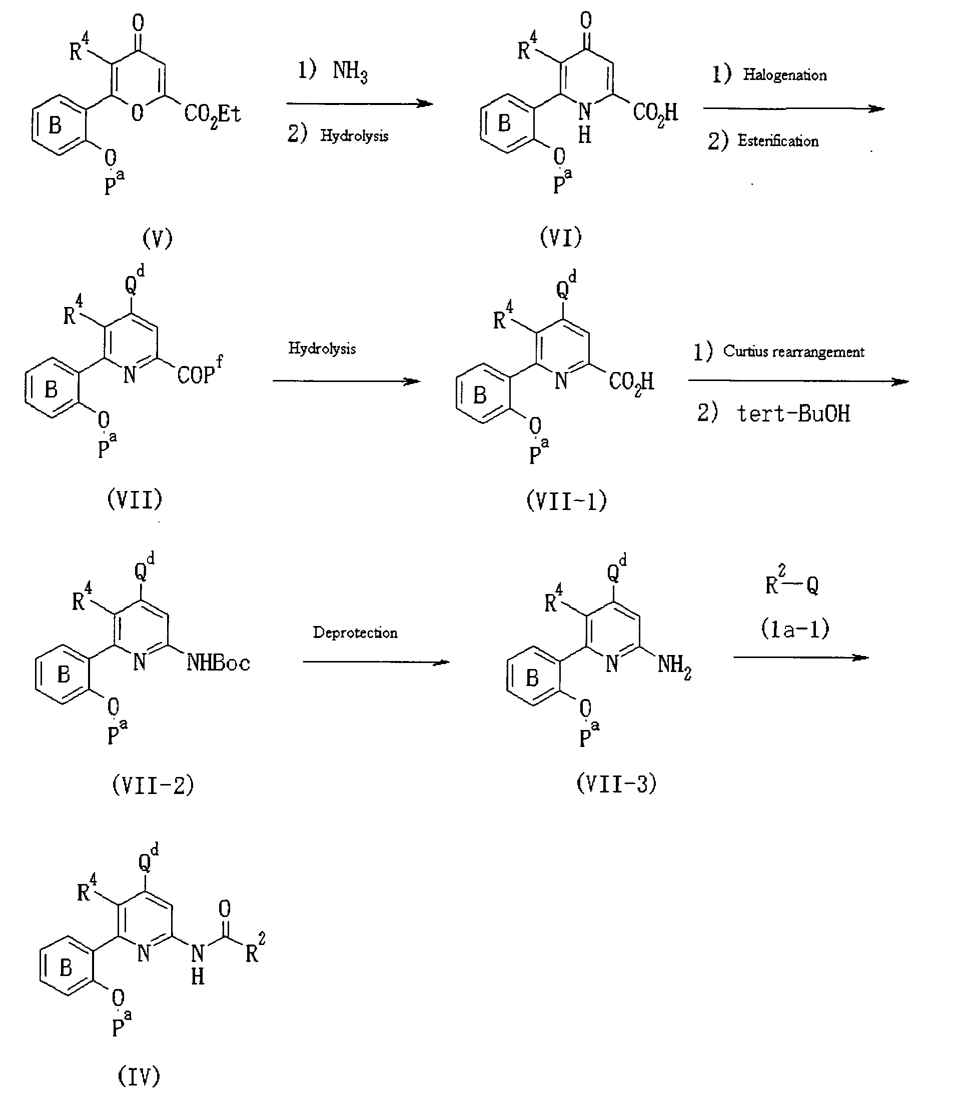 wherein P^{f} represents a protecting group, and the other letters each have the same significance as above.

Examples of the protecting group represented by P^{f} include alkoxy (e.g., methoxy, ethoxy, tert-butoxy, etc.), benzyloxy, and the like. The protecting group is preferably methoxy, or ethoxy.

The reaction of compound (V) and ammonia is performed in a solvent having no influence on the reaction.

Examples of such a solvent include alcohols, ethers, hydrocarbons, and the like. Two or more of these solvents may be mixed at an appropriate ratio.

The amount of ammonia is usually 1 to 50 molar equivalent, preferably 5 to 20 molar equivalent, with respect to 1 mol of compound (V).

The reaction temperature is usually 50 to 150°C, preferably 80 to 130°C.

The reaction time is usually 0.5 to 100 hours, preferably 1 to 48 hours.

The hydrolysis reaction may be performed in a solvent having no adverse influence on the reaction and in an acidic condition.

Examples of such a solvent include water, alcohols, aliphatic carboxylic acid (e.g., acetic acid), and the like. Two or more of these solvents may be mixed at an appropriate ratio.

As the acid, hydrochloric acid is usable, and the amount thereof is usually 1 to 50 molar equivalent, preferably 5 to 20 molar equivalent, with respect to 1 mol of compound (V).

The reaction temperature is usually 50 to 150°C, preferably 80 to 130°C.

The reaction time is usually 0.5 to 100 hours, preferably 1 to 48 hours.

The halogenation reaction of compound (VI) is performed in a solvent having no adverse influence on the reaction, or with no solvent.

Examples of such a solvent include ethers, hydrocarbons, halogenated hydrocarbons, esters, and the like. Two or more of these solvents may be mixed at an appropriate ratio.

Examples of the halogenating reagent include thionyl chloride, phosphoryl chloride, phosphorus pentachloride, phosphorus tribromide, and the like.

The amount of the halogenating reagent is usually 1 to 20 molar equivalent, preferably 2 to 10 molar equivalent, with respect to 1 mol of compound (VI).

The reaction temperature is usually 0 to 100°C, preferably 20 to 80°C.

The reaction time is usually 0.5 to 100 hours, preferably 1 to 48 hours.

The esterification reaction is performed in a solvent having no adverse influence on the reaction, or with no solvent, by reaction with an alcohol.

Examples of such a solvent include ethers, hydrocarbons, halogenated hydrocarbons, esters, and the like. Two or more of these solvents may be mixed at an appropriate ratio.

The amount of the alcohol is usually 1 to 20 molar equivalent, preferably 2 to 10 molar equivalent, with respect to 1 mol of compound (VI).

The reaction temperature is usually 0 to 100°C, preferably 20 to 80°C.

The reaction time is usually 0.5 to 100 hours, preferably 1 to 48 hours.

The hydrolysis reaction of compound (VII) is performed in a solvent having no adverse influence on the reaction and in a basic condition (e.g., using an aqueous solution of sodium hydroxide, or an aqueous solution of potassium hydroxide).

Examples of such a solvent include water, ethers, alcohols, hydrocarbons, halogenated hydrocarbons, and the like. Two or more of these solvents may be mixed at an appropriate ratio.

The amount of the base is usually 1 to 20 molar equivalent, preferably 2 to 10 molar equivalent, with respect to 1 mol of compound (VII).

The reaction temperature is usually 0 to 100°C, preferably 20 to 80°C.

The reaction time is usually 0.5 to 100 hours, preferably 1 to 48 hours.

The reaction of compound (VII-1) into compound (VII-2) is performed in a solvent having no adverse influence on the reaction, using, for example, diphenylphosphoryl azide and tert-butanol.

Examples of such a solvent include ethers, hydrocarbons, and the like. Such a solvent is preferably hydrocarbons. Two or more of these solvents may be mixed at an appropriate ratio.

The amount of diphenylphosphoryl azide is usually 1 to 2 molar equivalent, preferably 1 to 1.5 molar equivalent, with respect to 1 mol of compound (VII-1).

The amount of tert-butanol is usually 1 to 20 molar equivalent, preferably 1 to 10 molar equivalent, with respect to 1 mol of compound (VII-1).

The reaction temperature is usually 0 to 100°C, preferably 20 to 80°C.

The reaction time is usually 0.5 to 100 hours, preferably 1 to 48 hours.

The deprotection reaction of compound (VII-2) may be performed in a solvent having no adverse influence on the reaction and in, for example, an acidic condition.

Examples of such a solvent include ethers, hydrocarbons, halogenated hydrocarbons, and the like. Such a solvent is preferably halogenated hydrocarbons. Two or more of these solvents may be mixed at an appropriate ratio.

Examples of the acid include trifluoroacetic acid, hydrochloric acid, hydrogen bromide, and the like.

The amount of the acid is usually 10 to 100 molar equivalent, preferably 20 to 50 molar equivalent, with respect to 1 mol of compound (VII-2).

The reaction temperature is usually 0 to 100°C, preferably 20 to 80°C.

The reaction time is usually 0.5 to 100 hours, preferably 1 to 48 hours.

The reaction of compound (VII-3) and compound (1a-1) may be performed in accordance with the method described in Method A.

Among the compounds represented as compound (IIa) and compound (IIb) which are used as a starting material for Method A and Method B, compound (IIa') and compound (IIb') in which R³ is hydrogen are produced by, for example, Method Q described below.

### <Method Q>

Compound (VII-2) and compound (4a-4) are subjected to coupling reaction to obtain compound (VII-4). Compound (VII-4) is subjected to de-Boc reaction to obtain compound (IIb'). Compound (IIb') is subjected to deprotection reaction, to produce compound (IIa'). 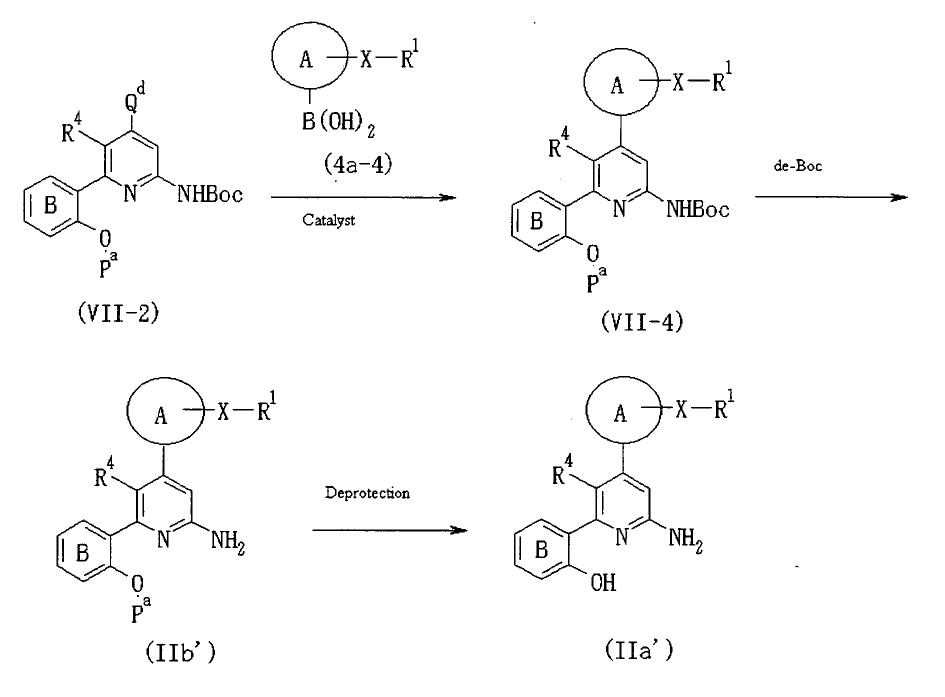 wherein each letter has the same significance as above.

The coupling reaction of compound (VII-2) and compound (4a-4) may be performed in accordance with the method described in Method M.

The de-Boc reaction of compound (VII-4) may be performed in accordance with the method described in Method P.

The deprotection reaction of compound (IIb') may be performed in accordance with the method described in Method B.

Compound (V) which is used as a starting material for Method P can be produced by a known method, for example, in accordance with the method described in Journal of Chemical Society, page 3663 (1956).

Among the compounds represented as compound (III), compound (IIIg) in which X is a bond can be produced by, for example, Method R described below.

### <Method R>

Compound (IIf-1) is subjected to acylation reaction to obtain compound (IIIf). Compound (IIIf) is subjected to coupling reaction, to produce compound (IIIg). 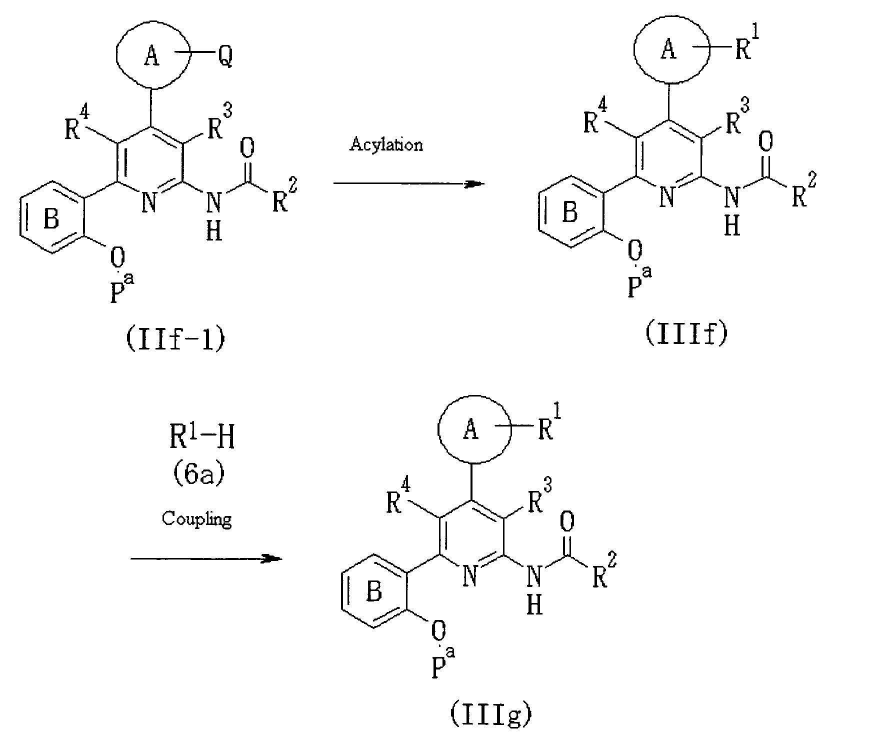 wherein each letter has the same significance as above.

If any, a commercially available compound may be used as compound (6a) as it is, or compound (6a) may be produced by a known method or a method conformed thereto.

The acylation reaction of compound (IIf-1) may be performed in conformity to the method described in Method B.

The coupling reaction of compound (IIIf) and compound (6a) is performed in a solvent having no influence on the reaction and in the presence of a base, using a catalyst.

Examples of such a solvent include water, alcohols, ethers, hydrocarbons, halogenated hydrocarbons, amides, and the like. Two or more of these solvents may be mixed at an appropriate ratio.

The amount of compound (6a) is usually 1 to 5 molar equivalent, preferably 1 to 2 molar equivalent, with respect to 1 mol of compound (IIIf).

Examples of the catalyst include nickel-based catalysts such as a combination of di(cyclooctadiene)nickel and 1,1'-bis(diphenylphosphino)ferrocene, a combination of di(cyclooctadiene)nickel and 1,10-phenanthrene, nickel(0)-2,2'-bipyridine, etc.,; palladium-based catalysts such as a combination of tris(dibenzylideneacetone)dipalladium and 2,2'-bis(diphenylphosphino)-1,1'-binaphthyl or 2-dicyclohexylphosphino-2'-4'-6'-triisopropyl-1,1'-biphenyl, etc.; copper-based catalysts such as copper 2-thiophenecarboxylate(I), etc.,; and the like. The amount thereof is usually 0.01 to 1 molar equivalent, preferably 0.05 to 0.5 molar equivalent, with respect to 1 mol of compound (IIIf).

Examples of the base include sodium carbonate, sodium tert-butoxide, cesium carbonate, potassium phosphate, and the like.

The amount of the base is usually 1 to 10 molar equivalent, preferably 1.5 to 3 molar equivalent, with respect to 1 mol of compound (IIIf).

The reaction temperature is usually -10 to 120°C, preferably 20 to 100°C.

The reaction time is usually 0.5 to 100 hours, preferably 1 to 24 hours.

When a substituent in compound (I) contains a convertible functional group (e.g., carboxy, amino, hydroxy, carbonyl, thiol, ester, sulfo, a halogen atom, etc.), the functional group can be converted by a known method or a method conformed thereto. Thus, various compounds can be produced.

For example, carboxy can be converted by a reaction such as esterification, reduction, amidation, conversion reaction into an optionally protected amino group, or the like.

Amino can be converted by a reaction such as amidation, sulfonylation, nitrosation, alkylation, arylation, imidation, or the like.

Hydroxy can be converted by a reaction such as esterification, carbamoylation, sulfonylation, alkylation, arylation, oxidation, halogenation, or the like

Carbonyl can be converted by a reaction such as reduction, oxidation, imination (including oximation and hydrazonation), (thio)ketalation, aklylidenation, thiocarbonylation, or the like.

Thiol can be converted by a reaction such as alkylation, oxidation, or the like.

Ester can be converted by a reaction such as reduction, hydrolysis, or the like.

Sulfo can be converted by a reaction such as sulfonamidation, reduction, or the like.

A halogen atom can be converted by a reaction such as, for example, various types of nucleophilic substitution reactions, various types of coupling reactions, or the like.

In each of the reactions according to the present invention, in the case where a compound can be in a free state, the compound may be converted into a salt in accordance with a usual method. In the case where a compound is obtained as a salt, such a salt may be converted into a free form or another salt in accordance with a usual method.

In each reaction during the production of compound (I) or in each reaction during the synthesis of a material compound, in the case where a material compound contains amino, carboxy or hydroxy as a substituent, such a substituent may contain a protecting group introduced thereto, which is generally used in peptide chemistry or the like. By optionally removing the protecting group after the reaction, a target compound can be obtained.

Examples of the protecting group for amino include formyl; and C₁₋₆ alkylcarbonyl (e.g., acetyl, ethylcarbonyl, etc.), phenylcarbonyl, C₁₋₆ alkyl-oxycarbonyl (e.g., methoxycarbonyl, ethoxycarbonyl, tert-butoxycarbonyl (Boc), etc.), allyloxycarbonyl (Aloc), phenyloxycarbonyl, fluorenylmethyloxycarbonyl (Fmoc), C₇₋₁₀ aralkyl-carbonyl (e.g., benzylcarbonyl, etc.), C₇₋₁₀ aralkyl-oxycarbonyl (e.g., benzyloxycarbonyl (Z), etc.), C₇₋₁₀ aralkyl (e.g., benzyl, etc.), trityl, phthaloyl, N,N-dimethylaminomethylene, and the like, each of which may be optionally substituted. Examples of the substituent usable for these protecting groups include phenyl, a halogen atom (e.g., fluorine, chlorine, bromine, iodine, etc.), C₁₋₆ alkyl-carbonyl (e.g., methylcarbonyl, ethylcarbonyl, butylcarbonyl, etc.), nitro, and the like. The number of the substituent(s) is about 1 to 3.

Examples of the protecting group for carboxy include C₁₋₆ alkyl (e.g., methyl, ethyl, n-propyl, isopropyl, n-butyl, tert-butyl, etc.), allyl, benzyl, phenyl, trityl, trialkylsilyl, and the like, each of which may be optionally substituted. Examples of the substituent usable for these protecting groups include a halogen atom (e.g., fluorine, chlorine, bromine, iodine, etc.), formyl, C₁₋₆ alkyl-carbonyl (e.g., acetyl, ethylcarbonyl, butylcarbonyl, etc.), nitro, and the like. The number of the substituent(s) is about 1 to 3.

Examples of the protecting group for hydroxy include C₁₋₆ alkyl (e.g., methyl, ethyl, n-propyl, isopropyl, n-butyl, tert-butyl, etc.), C₇₋₁₀ aralkyl (e.g., benzyl, etc.), a formyl group, C₁₋₆ alkyl-carbonyl (e.g., acetyl, ethylcarbonyl, etc.), a benzoyl group, C₇₋₁₀ aralkyl-carbonyl (e.g., benzylcarbonyl, etc.), tetrahydropyranyl, furanyl, silyl, and the like, each of which may be optionally substituted. Examples of the substituent usable for these protecting groups include a halogen atom (e.g., fluorine, chlorine, bromine, iodine, etc.), C₁₋₆ alkyl (e.g., methyl, ethyl, n-propyl, etc.), phenyl, C₇₋₁₀ aralkyl (e.g., benzyl, etc.), C₁₋₆ alkoxy (e.g., methoxy, ethoxy, n-propoxy, etc.), nitro, and the like. The number of the substituent(s) is about 1 to 4.

As a method for removing the protecting group, a known method or a method conformed thereto is usable. For example, a method of treating with acid, base, reduction, ultraviolet light, hydrazine, phenylhydrazine, sodium N-methyldithiocarbamate, tetrabutylammoniumfluoride, palladium acetate or the like is usable.

Compound (I') can be produced by, for example, a method for producing compound (I), a method conformed thereto, a known method (e.g., the method described in WO 04/58716, WO 02/44153, WO 02/24679, etc.), or a method conformed thereto.

In the case where a compound according to the present invention is present as a configurational isomer, a diastereomer, a conformer or the like, isolation can be optionally performed by any of the above-mentioned separation or purification means.

In the case where there are stereoisomers of a compound according to the present invention, such isomers which are independently present and a mixture thereof are both encompassed in the present invention.

A compound according to the present invention has superb metastin receptor (protein comprising an amino acid sequence represented by SEQ ID NO: 1, 3 or 5, a partial peptide thereof, or a salt thereof, etc.) antagonist activity, gonadotropic hormone (e.g., FSH, LH, etc.) secretion suppression activity, sex hormone [e.g., androgen (e.g., testosterone, androstenedione, etc.), estrogen (e.g., estradiol, estrone, etc.), progesterone, etc.] secretion suppression activity, and the like; is low in toxicity; and has very little side effects. Therefore, a compound according to the present invention or a prodrug thereof is useful as a safe pharmaceutical agent; for example, a metastin receptor antagonist (including an inverse agonist and a partial agonist), a gonadal function regulator, a gonadotropic hormone secretion suppressor, a sex hormone secretion suppressor, an ovulation inhibitor, an ovarium function regulator, or the like; more specifically, for example, a medicament such as a prophylactic or therapeutic agent for hormone-dependent cancer (e.g., prostate cancer, breast cancer, ovarian cancer, endometrial cancer, etc.), benign prostatomegaly (BPH), infertility, endometriosis, precocious puberty, uterine myoma or the like, a contraceptive, a follicle maturing inhibitor, a menstrual cycle-suspending agent or the like. A compound according to the present invention or a prodrug thereof is also useful for regulating ovulation, and is usable for infertility treatment such as, for example, IVF (in vitro fertilization), artificial insemination or the like.

When a compound according to the present invention is used as the above-described medicament, such a medicament can be administered both orally or parenterally in accordance with a known method. Usually, the medicament is mixed with a pharmaceutically acceptable carrier, and is orally administered in the form of a solid formulation such as tablets, capsules, granules, powder or the like, or parenterally, administered in the form of intravenously, subcutaneously or intramuscularly injectable preparations, suppositories, sublingual tablets or the like. The medicament may also be administered sublingually, subcutaneously, or intramuscularly as a sustained-release formulation such as sublingual tablets, microcapsules or the like.

The dose of the compound of the present invention varies depending on the administration target, administration route, symptom, or the like, and is not specifically limited. For treating the prostate cancer of an adult patient, for example, a usual dose of the compound is about 0.01 to about 20 mg/kg, preferably about 0.01 to about 10 mg/kg, and more preferably about 0.1 to about 2 mg/kg. It is desirable to administer such a dose about once to three times a day depending on the symptom.

The content of the compound of the present invention in each "pharmaceutical agent (pharmaceutical composition)" described above is about 0.01 to 100% by weight of the entirety pharmaceutical composition.

As the pharmaceutically acceptable carrier, various types of organic or inorganic carrier substances which are commonly used as a formulation material are usable. In a solid formulation, the carrier is contained as an excipient, a lubricant, a binder, a disintegrator, or the like. In a liquid formulation, the carrier is contained as a solvent, a dissolution aid, a suspending agent, an isotonizing agent, a buffering agent, a soothing agent or the like. Optionally, a formulation additive such as a preservative, an antioxidant, a colorant, a sweetener or the like may be used.

Preferable examples of the excipient include lactose, sucrose, D-mannitol, starch, crystalline cellulose, light anhydrous silicic acid, and the like. Preferable examples of the lubricant include magnesium stearate, calcium stearate, talc, colloidal silica, and the like. Preferable examples of the binder include crystalline cellulose, sucrose, D-mannitol, dextrin, hydroxypropylcellulose, hydroxypropylmethylcellulose, polyvinylpyrrolidone, and the like. Preferable examples of the disintegrator include starch, carboxymethylcellulose, carboxymethylcellulose calcium, croscarmellose sodium, carboxymethylstarch sodium, and the like. Preferable examples of the solvent include water for injection, alcohol, propyleneglycol, macrogol, sesame oil, corn oil, and the like. Preferable examples of the dissolution aid include polyethyleneglycol, propyleneglycol, D-mannitol, benzyl benzoate, ethanol, trisaminomethane, cholesterol, triethanolamine, sodium carbonate, sodium citrate, and the like. Preferable examples of the suspending agent include surfactants such as stearyl triethanolamine, sodium lauryl sulfate, laurylaminopropionic acid, lecithin, benzalkonium chloride, benzethonium chloride, glycerine monostearate, etc.; hydrophilic polymers such as polyvinyl alcohol, polyvinyl pyrrolidone, carboxymethylcellulose sodium, methylcellulose, hydroxymethylcellulose, hydroxyethylcellulose, hydroxypropylcellulose, etc.; and the like. Preferable examples of the isotonizing agent include sodium chloride, glycerin, D-mannitol, and the like. Preferable examples of the buffering agent include buffering solutions such as phosphate, acetate, carbonate, citrate, and the like. Preferable examples of the soothing agent include benzylalcohol and the like. Preferable examples of the preservative include paraoxybenzoic acid esters, chlorobutanol, benzylalcohol, phenethylalcohol, dehydroacetic acid, sorbic acid, and the like. Preferable examples of the antioxidant include sulfite, ascorbic acid, and the like.

The compound of the present invention may be formed into intravenously, subcutaneously or intramuscularly injectable preparations by a known method by being mixed with a suspending agent, a dissolution aid, a stabilizer, an isotonizing agent, a preservative or the like. Optionally, such an agent may be formed as a lyophilized agent by a known method.

For administration to, for example, humans, the compound of the present invention can be safely administered as a pharmaceutical composition orally or parenterally, independently or as a mixture with an appropriate pharmacologically acceptable carrier, excipient, or diluent.

Examples of the pharmaceutical composition include an oral agent (e.g., powder, granule, capsule, tablet), an injectable agent, a drip, an external preparation (e.g., transnasal formulation, subcutaneous formulation, etc.), a suppository (e.g., rectal suppository, vaginal suppository), and the like.

These formulations can be produced by a known method which is commonly used in formulation steps.

The compound of the present invention can be formed into an aqueous injectable agent together with a dispersant (e.g., Tween80 (Atlas Powder Company, U.S.A.), HCO60 (Nikko Chemicals Co., Ltd.), polyethyleneglycol, carboxymethylcellulose, sodium alginate, etc.), a preservative (e.g., methylparaben, propylparaben, benzylalcohol, etc.), an isotonizing agent (e.g., sodium chloride, mannitol, sorbitol, glucose, etc.) or the like. The compound of the present invention can be formed into an oleaginous injectable agent by being dissolved, suspended or emulsified in vegetable oil such as olive oil, sesame oil, cottonseed oil, corn oil, etc.; propyleneglycol; or the like.

The compound of the present invention can be formed into an oral formulation as follows in accordance with a known method. Any of the following additives is added to compound (I): an excipient (e.g., lactose, sucrose, starch, etc.), a disintegrator (e.g., starch, calcium carbonate, etc.), a binder (e.g., starch, gum arabic, carboxymethylcellulose, polyvinylpyrrolidone, hydroxypropylcellulose, etc.), a lubricant (e.g., talc, magnesium stearate, polyethyleneglycol 6000, etc.) or the like. Compound (I) having any of these additives incorporated thereto is compressed and molded, and then optionally treated with coating by a known method for the purpose of masking the taste, or making the formulation enteric or sustainable. Thus, an oral formulation is obtained. Examples of the coating agent include hydroxypropylmethylcellulose, ethylcellulose, hydroxymethylcellulose, hydroxypropylcellulose, polyoxyethyleneglycol, Tween80, pluronic F68, cellulose acetate phthalate, hydroxypropylmethylcellulose phthalate, hydroxymethylcellulose acetate succinate, eudragit (Rohm, Germany, methacrylic acid/acrylic acid copolymerization), and a colorant (e.g., rouge, titanium dioxide, etc.), and the like. For producing an enteric formulation, an intermediate phase may be provided between an enteric phase and an agent-containing phase by a known method for the purpose of separating the phases.

The compound of the present invention can be formed into a solid, semisolid or liquid external preparation as follows in accordance with a known method. For a solid preparation, the compound of the present invention is formed into a powdery composition, independently or after being mixed with an excipient (e.g., glycol, mannitol, starch, fine crystalline cellulose, etc.), a thickener (e.g., natural gums, cellulose derivative, acrylic acid polymer, etc.), or the like. For a liquid preparation, an oleaginous or aqueous suspension is formed in substantially the same manner as in the case of the injectable preparations. For a semisolid preparation, it is preferable to form an aqueous or oleaginous gel or ointment. To these preparations, a pH adjuster (e.g., carbonic acid, phosphoric acid, citric acid, hydrochloric acid, sodium hydroxide, etc.), a preservative (e.g., paraoxy benzoate esters, chlorobutanol, benzalkonium chloride, etc.) or the like may be added.

The compound of the present invention can be formed into, for example, an oleaginous or aqueous solid, semisolid or liquid suppository as follows in accordance with a known method. Examples of an oleaginous base usable for the above compositions include glyceride of higher fatty acid (e.g., cacao oil, Witepsols (Dynamite Noble, Germany), etc.), medium fatty acid (e.g., miglyol (Dynamite Noble, Germany), etc.), vegetable oil (e.g., sesame oil, soybean oil, cottonseed oil, etc.), and the like. Examples of an aqueous base usable for the above compositions include polyethyleneglycols, propyleneglycol, and the like. Examples of an aqueous gel base usable for the above compositions include natural gums, cellulose derivative, vinyl polymer, acrylic acid polymer, and the like.

The compound of the present invention may be used together with, for example, a hormone therapy agent, a carcinostatic agent (e.g., chemotherapy agent, immunotherapy agent, an agent for inhibiting the action of a cell proliferation factor or a receptor thereof) and the like (hereinafter, referred to simply as the "co-usable agent").

The compound of the present invention exhibits a superb anticancer action even when being used independently, but the effect thereof can be further improved by being used together with one or a plurality of the above-mentioned co-usable agents (polypharmacy).

Examples of the "hormone therapeutic agent" include fosfestrol, diethylstilbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogen (e.g., tamoxifen citrate, toremifene citrate, etc.), ER downregulator (e.g., fulvestrant, etc.), human menopausal gonadotropin, follicle-stimulating hormone, pill formulation, mepitiostane, testrolactone, aminoglutethimide, LH-RH agonist (e.g., goserelin acetate, buserelin, leuprorelin, etc.), droloxifene, epitiostanol, ethynylestradiol sulfonate, aromatase inhibitor (e.g., fadrozole hydrochloride, anastrozole, letrozole, exemestane, vorozole, formestan, etc.), anti-androgen (e.g., flutamide, bicalutamide, nilutamide, etc.), 5α-reductase inhibitor, (e.g., finasteride, dutasteride, epristeride, etc.), adrenocortical hormone-based agent (e.g., dexamethasone, prednisolone, betamethasone, triamcinolone, etc.), androgen synthesis inhibitor (e.g., abiraterone, etc.), agent for delaying metabolism of retinoid (e.g., liarozole, etc.), and the like. LH-RH agonist (e.g., goserelin acetate, buserelin, leuprorelin, etc.) is preferable.

Examples of the "chemotherapeutic agent" include alkylating agent, antimetabolite, anticancer antibiotic, plant-derived carcinostatic agent, and the like.

Examples of the "alkylating agent" include nitrogen mustard, nitrogen mustard-N-oxide hydrochloride, chlorambucil, cyclophosphamide, ifosfamide, thiotepa, carboquone, improsulfan tosilate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, predmustine, pumitepa, ribomustin, temozolomide, treosulfan, trofosfamide, zinostatin stimalamer, adozelesin, cystemustine, bizelesin, and the like.

Examples of the "antimetabolite " include methylmercaptopurine, 6-mercaptopurine riboside, thioinosine, methotrexate, enocitabine, cytarabine, cytarabine ocphosphate, ancitabine hydrochloride, 5-FU-based agent (e.g., fluorouracil, tegafur, UFT, doxifluridine, carmofur, galocitabine, emitefur, etc.), aminopterin, leucovorin calcium, tabloid, wybutosine, folinate calcium, levofolinate calcium, cladribine, emitefur, fludarabine, gemcitabine, hydroxycarbamide, pentostatin, piritrexim, idoxiuridine, mitoguazone, tiazofurine, ambamustine, and the like.

Examples of the "anticancer antibiotic" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mythramycin, sarcomycin, carzinophilin, mitotane, zorubicin hydrochloride, mitoxantrone hydrochloride, idarubicin hydrochloride, and the like.

Examples of the "plant-derived carcinostatic agent" include etoposide, etoposide phosphate, vinblastine sulfate, vincristine sulfate, vindesine sulfate, teniposide, paclitaxel, docetaxel, vinorelbine, and the like.

Examples of the "immunotherapeutic agent (BRM)" include picibanil, krestin, sizofiran, lentinan, ubenimex, interferon, interleukin, macrophage colony-stimulating factor, granulocyte colony-stimulating factor, erythropoietin, lymphotoxin, BCG vaccine, corynebacterium parvum, levamizole, polysaccharide K, procodazole, and the like.

The "cell proliferation factor" in the "agent for inhibiting the action of a cell proliferation factor or a receptor thereof" may be any substance which promotes the proliferation of cells. Usually, a peptide having a molecular weight of 20,000 or less and exhibiting an action thereof at a low concentration when being bonded with a receptor is used. Specific examples of such a substance include (1) EGF (epidermal growth factor) or a substance having substantially the same activity as that (e.g., EGF, herregrin (HER2 ligand), etc.); (2) insulin or a substance having substantially the same activity as that (e.g., insulin, IGF (insulin-like growth factor)-1, IGF-2, etc.); (3) FGF (fibroblast growth factor) or a substance having substantially the same activity as that (e.g., acidic FGF, basic FGF, KGF (keratinocyte growth factor), FGF-10, etc.); (4) other cell proliferation factors (e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), etc.); and the like.

The "receptor of the cell proliferation factor" may be any substance which has a bonding capability with the above-mentioned cell proliferation factor. Specific examples of such a substance include EGF receptor, herregrin receptor (HER2), insulin receptor, IGF receptor, FGF receptor-1, FGF receptor-2, and the like.

Examples of the "agent for inhibiting the action of a cell proliferation factor" include trastuzumab (Herceptin (trademark); HER2 antibody), imatinib mesylate, ZD1839 or cetuximab, antibody against VEGF (e.g., bevacizumab), antibody against VEGF receptor, gefitinib, erlotinib, and the like.

Other than the above-listed agents, the following agents are also usable: L-asparaginase, aceglatone, procarbazine hydrochloride, protoporphyrin-cobalt complex salt, mercury hematoporphine-sodium, topoisomerase I inhibitor (e.g., irinotecan, topotecan, etc.), topoisomerase II inhibitor (e.g., sobuzoxane, etc.), differentiation-inducing agent (e.g., retinoid, vitamin D's, etc.), neovascularization inhibitor (e.g., thalidomide, SU11248, etc.), α-blocker (e.g., tamsulosin hydrochloride, naftopidil, urapidil, alfuzosin, terazosin, prazosin, silodosin, etc.), serine-threoninekinase inhibitor, endothelin receptor antagonist (e.g., atrasentan, etc.), proteasome inhibitor (e.g., bortezomib, etc.), Hsp90 inhibitor (e.g., 17-AAG, etc.), spironolactone, minoxidil, 11α-hydroxyprogesterone, bone resportion

inhibitor-metastasis suppressor (e.g., zoledronic acid, alendronic acid, pamidronic acid, etidronic acid, ibandronic acid, clodronic acid), and the like.

Preferable examples of the co-usable agents, among the above-listed agents, are LH-RH agonist (e.g., goserelin acetate, buserelin, leuprorelin, etc.), trastuzumab (HER2 antibody), and the like.

By combining the compound of the present invention and the co-usable agent, the following superb effects are provided.
(1) As compared with the case where the compound of the present invention or the co-usable agent is independently used, the dose can be reduced.
(2) The agent to be co-used with the compound of the present invention can be selected according to the symptom (mild, serious, etc.) of the patient.
(3) By selecting an agent having a different acting mechanism from that of the compound of the present invention, the treating period can be set longer.
(4) By selecting an agent having a different acting mechanism from that of the compound of the present invention, the effect of the treatment can be sustained.
(5) By co-using the compound of the present invention and the co-usable agent, a combined effect is provided.

The compound of the present invention can decrease the testosterone level down to the castration level from immediately after being administered. By combining the compound of the present invention and a co-usable agent such as an LH-RH agonist (e.g., goserelin acetate, buserelin, leuprorelin, etc.; preferably leuprorelin), the period of hormone dependence can be kept longer, and so the combined agent can be used more advantageously.

The co-usable agent of the present invention is low in toxicity. For example, the compound of the present invention and/or any of above-listed co-usable agents can be mixed with a pharmacologically acceptable carrier in accordance with a known method; and safely administered orally or pareterally (e.g., topically, rectally, intravenously, etc.) as a pharmaceutical composition in the form of, for example, tablets (including sugar-coated tablet and film-coated tablet), powder, granules, capsules (including soft capsule), liquids, injectable preparations, suppositories, sustained-release agents or the like. An injectable preparation can be administered intravenously, intramuscularly, subcutaneously, intraorganically, or directly to the lesion.

Examples of the pharmacologically acceptable carrier usable for the production of the co-usable agent of the present invention include various types of organic and

inorganic carrier substances which are commonly used as a formulation material; for example, an excipient, a lubricant, a binder, and a disintegrator in a solid formulation; and a solvent, a dissolution aid, a suspending agent, an isotonizing agent, a buffering agent, and a soothing agent in a liquid formulation. Optionally, a formulation additive such as a common preservative, antioxidant, colorant, sweetener, adsorbent, moisturing agent or the like may be used.

Hereinafter, co-use of the compound of the present invention and the co-usable agent will be referred to as a "co-used agent of the present invention".

For using the co-used agent of the present invention, the time of administration of the compound of the present invention and the co-usable agent is not limited. The compound of the present invention or a pharmaceutical composition thereof, and the co-usable agent or a pharmaceutical composition thereof, may be administered to the target at the same time or at a certain interval. The dose of the co-usable agent may be determined in conformity to the clinically used dose, and may be appropriately selected according to the administration target, the administration route, the disease, the combination thereof, and the like.

The form of administration of the co-used agent of the present invention is not specifically limited. It is sufficient that the compound of the present invention and the co-usable agent are combined at the time of administration. Examples of such a form of administration include
(1) administering a single agent obtained by formulating the compound of the present invention and the co-usable agent at the same time;
(2) administering two types of agents, obtained by separately formulating the compound of the present invention and the co-usable agent, via the same route at the same time;
(3) administering two types of agents, obtained by separately formulating the compound of the present invention and the co-usable agent, via the same route at a certain interval;
(4) administering two types of agents, obtained by separately formulating the compound of the present invention and the co-usable agent, via different routes at the same time;
(5) administering two types of agents, obtained by separately formulating the compound of the present invention and the co-usable agent, via different routes at a certain interval apart (for example, first administering the compound of the present invention and then administering the co-usable agent; or the opposite order); and the like.

The ratio of the compound of the present invention and the co-usable agent in the co-used agent of the present invention may be appropriately selected according to the administration target, the administration route, the disease, and the like.

For example, the content of the compound of the present invention in the co-used agent of the present invention varies according to the form of the formulation, but is usually about 0.01 to 100% by weight, preferably about 0.1 to 50% by weight, more preferably about 0.5 to 20% by weight, with respect to the entire formulation.

The content of the co-usable agent in the co-used agent of the present invention varies according to the form of the formulation, but is usually about 0.01 to 100% by weight, preferably about 0.1 to 50% by weight, more preferably about 0.5 to 20% by weight, with respect to the entire formulation.

The content of the carrier or other additives in the co-used agent of the present invention varies according to the form of the formulation, but is usually about 1 to 99.99% by weight, preferably about 10 to 90% by weight, with respect to the entire formulation.

In the case where the compound of the present invention and the co-usable agent are separately formulated, the contents may be the same as above.

The sequence numbers of the sequence listing in the present specification respectively show the following.
[SEQ ID NO: 1]
   SEQ ID NO: 1 shows the amino acid sequence of human OT7T175.
[SEQ ID NO: 2]
   SEQ ID NO: 2 shows the base sequence of DNA encoding human OT7T175.
[SEQ ID NO: 3]
   SEQ ID NO: 3 shows the amino acid sequence of rat OT7T175.
[SEQ ID NO: 4]
   SEQ ID NO: 4 shows the base sequence of DNA encoding rat OT7T175.
[SEQ ID NO: 5]
   SEQ ID NO: 5 shows the amino acid sequence of mouse OT7T175.
[SEQ ID NO: 6]
   SEQ ID NO: 6 shows the base sequence of DNA encoding mouse OT7T175.

Hereinafter, the present invention will be described more specifically by way of reference examples, examples, formulation examples and test examples. The present invention is not limited to these examples.

In the reference examples and examples, the purity of the compounds was measured in the following HPLC conditions.
Device: LC-10Avp system, Shimadzu Corporation
Column: CAPSEL PAK C18UG120 S-3 µm, 2.0 x 50 mm
Solvent: Liquid A: 0.1% trifluoroacetic acid-containing water
Liquid B: 0.1 % trifluoroacetic acid-containing acetonitrile Gradient cycle: 0.00 minute (Liquid A/Liquid B = 90/10), 4.00 minutes (Liquid A/Liquid B = 5/95), 5.50 minutes (Liquid A/Liquid B = 5/95), 5.51 minutes (Liquid A/Liquid B = 90/10), 8.00 minutes (Liquid A/Liquid B = 90/10), Injection amount: 2 µl, Flow rate: 0.5 ml/min, Detection method: UV 220 nm

In the reference examples and examples, the purification by separation HPLC was performed in the following conditions.
Device: High throughput purification system, Gilson, Inc.
Column: YMC CombiPrep ODS-A, S-5 µm, 50 x 20 mm
Solvent: Liquid A: 0.1% trifluoroacetic acid-containing water
Liquid B: 0.1 % trifluoroacetic acid-containing acetonitrile Gradient cycle: 0.00 minute (Liquid A/Liquid B = 90/10), 1.00 minute (Liquid A/Liquid B = 90/10), 4.00 minutes (Liquid A/Liquid B = 10/95), 8.50 minutes (Liquid A/Liquid B = 10/95), 8.60 minutes (Liquid A/Liquid B = 90/10), 8.70 minutes (Liquid A/Liquid B = 90/10); or 0.00 minute (Liquid A/Liquid B = 95/5), 1.00 minute (Liquid A/Liquid B = 95/5), 5.20 minutes (Liquid A/Liquid B = 5/95), 6.40 minutes (Liquid A/Liquid B = 5/95), 6.50 minutes (Liquid A/Liquid B = 95/5), 6.60 minutes (Liquid A/Liquid B = 95/5);
Flow rate: 20 ml/min, Detection method: UV 220 nm

In the reference examples and examples, the mass spectrum was performed in the following conditions.

### Measuring device: Platform II by Micromass; or ZMD by Waters

Ionization method: Atmospheric pressure chemical ionization (APC), or electron spray ionization (ESI)

In the reference examples and examples, the HPLC mass spectrum (LC-MS) was performed in the following conditions.
Measuring device: ZMD by Micromass; HP1100 by Agilent Technologies
Column: CAPCELL PAK C18UG120, S-3 µm, 1.5 x 35 mm
Solvent: Liquid A: 0.05% trifluoroacetic acid-containing water
Liquid B: 0.04% trifluoroacetic acid-containing acetonitrile Gradient cycle: 0.00 minute (Liquid A/Liquid B = 90/10), 2.00 minutes (Liquid A/Liquid B = 5/95), 2.75 minutes (Liquid A/Liquid B = 5/95), 2.76 minutes (Liquid A/Liquid B = 90/10), 3.45 minutes (Liquid A/Liquid B = 90/10), or 0.00 minute (Liquid A/Liquid B = 90/10), 2.00 minutes (Liquid A/Liquid B = 5/95), 2.75 minutes (Liquid A/Liquid B = 5/95), 2.76 minutes (Liquid A/Liquid B = 90/10), 3.60 minutes (Liquid A/Liquid B = 90/10)
Injection amount: 2 µl, Flow rate: 0.5 ml/min., Detection method: UV 220 nm Ionization method: Electron spray ionization (ESI)
¹H-NMR spectrum was measured by AVANCE DPX-300 (300 MHz) or AV400M (400 MHz) produced by Bruker or JNR-AL400 produced by JEOL using tetramethylsilane as the internal reference. All the δ values are represented with ppm.

As the microwave reaction device, Emrys Optimizer produced by Biotage was used.

The values shown regarding the mixed solvents are the volumetric ratios of the solvents unless otherwise specified. "%" means % by weight unless otherwise specified. Herein, "room temperature" means about 10°C to about 35°C, but is not limited strictly.

The other letters or codes used herein means the following.
s : singlet
d : doublet
t : triplet
q : quartet
m : multiplet
br: broad
J : coupling constant
Hz: Hertz
CDCl₃: chloroform D
DMSO-d₆ : dimethylsulfoxide-d₆
CD₃OD : methanol D
¹H-NMR: proton nuclear magnetic resonance
DMF: N,N-dimethylformamide
DME: 1,2-dimethoxyethane
DPPA: diphenyl phosphorazidate
THF: tetrahydrofuran
TFA: trifluoroacetic acid
WSCD: water-soluble carbodiimide (1-ethyl-3-(3-dimethylaminopropyl)carbodiimide)
HOBt: 1-hydroxybenzotriazole
Boc: tert-butoxycarbonyl
Cbz: carbobenzoxy
H-Gly-OH: glycine
H-Ala-OH: alanine
H-Val-OH: varine
H-Leu-OH: leucine
H-Ile-OH: isoleucine
H-Ser-OH: serine
H-Thr-OH: threonine
H-Phe-OH: phenylalanine
H-Tyr-OH: tyrosine
H-Trp-OH: tryptophan
H-Asp-OH: aspartic acid
H-Asn-OH: asparagine
H-Glu-OH: glutamic acid
H-Gln-OH: glutamine
H-Cys-OH: cysteine
H-Met-OH: methionine
H-Lys-OH: lysine
H-Arg-OH: arginine
H-Pro-OH: proline
H-His-OH: histidine
H-Asp(OMe)-OH: β-methyl aspartate
H-Glu(OBzl)-OH: γ-benzyl glutamate

### Reference Example 1

### 2'-(methoxymethoxy)acetophenone

Sodium hydride (in oil: 60%, 7.3 g, 0.18 mol) was added to a solution of 2'-hydroxyacetophenone (25.0 g, 0.18 mol) in THF (300 mL) with ice-cooling, and stirred for 30 minutes. Chloromethyl methyl ether (29.4 g, 0.36 mol) was dropped to the resultant mixed solution with ice-cooling and stirred at room temperature for 1 hour. Saturated aqueous sodium hydrogencarbonate was added to the reaction mixture, and extracted with ethyl acetate. The extract was washed with saturated brine, dried over magnesium sulfate, and then concentrated under reduced pressure to give the title compound (33.1 g, 100%) as a colorless liquid.
¹H-NMR (300MHz, CDCl₃) δ: 2.64 (3H, s), 3.50 (3H, s), 5.29 (2H, s), 7.05 (1H, t, J = 7.5 Hz), 7.13 (1H, d, J = 8.4 Hz), 7.44 (1H, t, J = 7.8 Hz), 7.71 (1H, d, J = 7.8 Hz).

### Reference Example 2

### 2-amino-6-[2-(methoxymethoxy)phenyl]-4-(3-nitrophenyl)nicotinonitrile

A solution of 2'-(methoxymethoxy)acetophenone (10.0 g, 55.49 mmol) of Reference Example 1, 3-nitrobenzaldenyde (8.39 g, 55.49 mmol), malononitrile (3.67 g, 55.49 mmol) and ammonium acetate (6.41 g, 83.24 mmol) in toluene (60 mL) was refluxed for 19 hours. After cooling, saturated aqueous sodium hydrogencarbonate was added to the reaction mixture, and extracted twice with ethyl acetate. The organic layers were combined, washed with saturated brine, dried over magnesium sulfate, and then concentrated under reduced pressure. The residue was purified by silica gel chromatography (eluting solution: ethyl acetate/hexane/chloroform = 1/1/1), and then recrystallized from chloroform-ethyl acetate-hexane to give the title compound(5.93 g, 28%) as gray crystals.
¹H-NMR (300MHz, CDCl₃) δ: 3.46 (3H, s), 5.24 (2H, s), 5.39 (2H, br s), 7.15 (1H, t, J = 7.5 Hz), 7.23 (1H, d, J = 8.4 Hz), 7.38-7.44 (2H, m), 7.73 (1H, t, J = 8.0 Hz), 7.80 (1H, dd, J = 7.8, 1.8 Hz), 8.01 (1H, d, J = 7.8 Hz), 8.35-8.38 (1H, m), 8.47 (1H, d, J = 1.8 Hz).
Melting point: 169-171°C

### Reference Example 3

### N-[6-(2-{[tert-butyl(dimethyl)silyl]oxy}phenyl)-3-cyano-4-(3-nitrophenyl)pyridin-2-yl] thiophene-2-carboxamide

2-thenoyl chloride (0.6 mL, 5.60 mmol) was added to a solution of 2-amino-6-(2-{[tert-butyl(dimethyl)silyl]oxy}phenyl)-4-(3-nitrophenyl)- nicotinonitrile (1.0 g, 2.24 mmol) in pyridin (10 mL) described in WO 02/44153, and stirred at room temperature for 20 hours. The reaction mixture was diluted with ethyl acetate, washed with water and then with saturated brine, dried over magnesium sulfate, and then concentrated under reduced pressure. The residue was purified by amino silica gel chromatography (eluting solution: ethyl acetate/hexane = 5/95 to 1/1), and then recrystallized from ethyl acetate-hexane to give the title compound (590 mg, 47%) as white crystals.
¹H-NMR (300MHz, CDCl₃) δ: 0.12 (6H, s), 0.83 (9H, s), 6.95 (1H, dd, J = 0.9, 9.3 Hz), 7.13 (1H, t, J = 7.5 Hz), 7.18-7.21 (1H, m), 7.37 (1H, t, J = 7.8 Hz), 7.67 (1H, dd, J = 4.8, 0.9 Hz), 7.75 (1H, t, J = 8.1 Hz), 7.78-7.81 (1H, m), 7.85 (1H, dd, J = 7.5, 1.5 Hz), 7.97 (1H, s), 8.05 (1H, d, J = 7.5 Hz), 8.41 (1H, d, J = 7.5 Hz), 8.46 (1H, s), 8.49 (1H, s).
Melting point: 106-108°C

### Reference Example 4

### N-[4-(3-aminophenyl)-6-(2-{[tert-butyl(dimethyl)silyl]oxy}phenyl)-3-cyanopyridin-2-yl]thiophene-2-carboxamide

A solution of the compound of Reference Example 3 (200 mg, 0.36 mmol), iron powder (120 mg, 2.16 mmol) and ammonium chloride (125 mg, 2.34 mmol) in an ethanol (10 mL)-water (10 mL) was refluxed while heating for 20 minutes. After cooling, saturated aqueous sodium hydrogencarbonate was added to the reaction mixture, and extracted with ethyl acetate. The extract was washed with saturated brine, dried over magnesium sulfate, and then concentrated under reduced pressure to give the title compound (180 mg, 95%) as a yellow amorphous solid.
¹H-NMR (300MHz, DMSO-d₆) δ: 0.18 (6H, s), 0.76 (9H, s), 5.39-5.43 (2H, br s), 6.71-6.81 (2H, m), 6.88 (1H, s), 7.03 (1H, d, J = 8.1 Hz), 7.12-7.31 (3H, m), 7.44 (1H, t, J = 7.7 Hz), 7.79-7.92 (2H, m), 7.96 (1H, d, J = 4.8 Hz), 8.14 (1H, d, J = 3.0 Hz), 11.46 (1H, s).

### Reference Example 5

### Methyl 3-{2-amino-3-cyano-6-[2-(methoxymethoxy)phenyl]pyridin-4-yl}benzoate

A solution of 2'-(methoxymethoxy)acetophenone of Reference Example 1 (5.49 g, 30.46 mmol), methyl 3-formylbenzoate (5.0 g, 30.46 mmol), malononitrile (2.01 g, 30.46 mmol) and ammonium acetate (3.52 g, 45.69 mmol) in toluene (30 mL) was refluxed while heating for 16 hours. After cooling, the reaction mixture was concentrated under reduced pressure and diluted with ethyl acetate. The diluted mixture was washed with water and then with saturated brine, dried over magnesium sulfate, and then concentrated under reduced pressure. The residue was purified by silica gel chromatography (eluting solution: ethyl acetate/hexane = 1/2 to 1/1), and then recrystallized from ethyl acetate-hexane to give the title compound (3.37 g, 28%) as white crystals.
¹H-NMR (300MHz, CDCl₃) δ: 3.45 (3H, s), 3.95 (3H, s), 5.22 (2H, s), 5.35 (2H, br s), 7.14 (1H, t, J = 7.5 Hz), 7.22 (1H, d, J = 7.5 Hz), 7.37-7.42 (2H, m), 7.61 (1H, t, J = 7.8 Hz), 7.77 (1H, dd, J = 7.8, 1.8 Hz), 7.85-7.89 (1H, m), 8.15-8.19 (1H, m), 8.28 (1H, t, J = 1.7 Hz).
Melting point: 129-130°C

### Reference Example 6

### Methyl 3- {3-cyano-6-[2-(methoxymethoxy)phenyl]-2-[(2-thienylcarbonyl)amino] pyridin-4-yl}benzoate

2-thenoyl chloride (1.02 mL, 9.62 mmol) was added to a solution of the compound of Reference Example 5 (1.50 g, 3.85 mmol) in pyridin (15 mL), and stirred at room temperature for 17 hours. The reaction mixture was concentrated under reduced pressure, diluted with ethyl acetate, washed with water and then with saturated brine, dried over magnesium sulfate, and then concentrated under reduced pressure. The residue was dissolved in THF (20 mL), a 2M aqueous solution (20 mL) of sodium carbonate and methanol (15mL) were added, and stirred at room temperature for 20 hours. The reaction mixture was diluted with water, and extracted with ethyl acetate. The extract was washed with saturated brine, dried over magnesium sulfate, and then concentrated under reduced pressure. The residue was purified by silica gel chromatography (eluting solution: ethyl acetate/hexane =1/1), and then recrystallized from ethyl acetate-hexane to give the title compound (936 mg, 49%) as white crystals.
¹H-NMR (300MHz, CDCl₃) δ: 3.32 (3H, s), 3.92 (3H, s), 5.32 (2H, s), 7.19 (1H, t, J = 7.5 Hz), 7.27-7.31 (2H, m), 7.50 (1H, t, J = 7.8 Hz), 7.80 (1H, t, J = 7.8 Hz), 7.91 (1H, d, J = 7.8 Hz), 7.97-8.08 (3H, m), 8.16-8.19 (2H, m), 8.26 (1H, s), 11.49 (1H, s).
Melting point: 159-160°C

### Reference Example 7

### 3-{3-cyano-6-[2-(methoxymethoxy)phenyl]-2-[(2-thienylcarbonyl)amino]pyridin-4-yl} benzoic acid

A 4N aqueous sodium hydroxide solution (1.5 mL) was added to a solution of the compound of Reference Example 6 (1.04 g, 2.08 mmol) in THF (15 mL)-ethanol (8 mL) mixture, and stirred at room temperature for 16 hours. The reaction mixture was neutralized with 1N hydrochloric acid, and extracted with ethyl acetate. The extract was washed with saturated brine and dried over magnesium sulfate. Ethyl acetate was removed to give the title compound (1.01 g, 100%) as a white solid.
¹H-NMR (300MHz, DMSO-d₆) δ: 3.36 (3H, s), 5.32 (2H, s), 7.17 (1H, t, J = 7.5 Hz), 7.28-7.31 (2H, m), 7.47-7.53 (1H, m), 7.78 (1H, t, J = 7.8 Hz), 7.92 (1H, dd, J = 7.5, 1.5 Hz), 7.98-8.01 (2H, m), 8.09 (1H, s), 8.14-8.20 (2H, m), 8.29 (1H, s), 11.47 (1H, s), 13.15-13.35 (1H, br).
Melting point: 179-180°C

### Reference Example 8

### 2-amino-4-[3-(hydroxymethyl)phenyl]-6-[2-(methoxymethoxy)phenyl]nicotinonitrile

Lithium tetrahydroborate (56 mg, 2.57 mmol) was added to a solution of the compound of Reference Example 5 (1.0 g, 2.57 mmol) in THF (20 mL), and stirred at 50°C for 24 hours. The reaction mixture was diluted with water, and extracted with ethyl acetate. The extract was washed with saturated brine, dried over magnesium sulfate, and concentrated under reduced pressure. The residue was purified by silica gel chromatography (eluting solution: ethyl acetate/hexane = 2/1 to 1/1), and then recrystallized from ethyl acetate-hexane to give the title compound (830 mg, 89%) as a white amorphous solid.
¹H-NMR (300MHz, CDCl₃) δ: 1.87 (1H, t, J = 5.7 Hz), 3.43 (3H, s), 4.79 (2H, d, J = 5.7 Hz), 5.20 (2H, s), 5.37 (2H, s), 7.13 (1H, t, J = 7.2 Hz), 7.21 (1H, d, J = 8.4 Hz), 7.34-7.41 (2H, m), 7.47-7.58 (3H, m), 7.64 (1H, s), 7.75 (1H, d, J = 7.8 Hz).

### Reference Example 9

### 2-amino-4-[3-(bromomethyl)phenyl]-6-[2-(methoxymethoxy)phenyl]nicotinonitrile

Triphenylphosphine (550 mg, 2.09 mmol) and carbon tetrabromide (867 mg, 2.61 mmol) were added to a solution of the compound of Reference Example 8 (630 mg, 1.74 mmol) in dichloromethane (60 mL), and stirred at room temperature for 1 hour. The reaction mixture was diluted with saturated aqueous sodium hydrogencarbonate, and extracted with ethyl acetate. The extract was washed with saturated , dried over magnesium sulfate, and then concentrated under reduced pressure.

The residue was purified by silica gel chromatography (eluting solution: ethyl acetate/hexane = 1/2 to 3/2) to give the title compound (470 mg, 64%) as a white amorphous solid.
¹H-NMR (300MHz, CDCl₃) δ: 3.45 (3H, s), 4.56 (2H, s), 5.22 (2H, s), 5.32 (2H, s), 7.15 (1H, t, J = 7.5 Hz), 7.22 (1H, d, J = 7.5 Hz), 7.36-7.40 (2H, m), 7.49-7.58 (2H, m), 7.59-7.60 (1H, m), 7.66 (1H, s), 7.76 (1H, dd, J = 7.8, 1.8 Hz).

### Reference Example 10

### 2-amino-4-[3-(azidomethyl)phenyl]-6-[2-(methoxymethoxy)phenyl]nicotinonitrile

A solution of the compound of Reference Example 9 (200 mg, 0.47 mmol) and sodium azide (153 mg, 2.36 mmol) in DMF (10 mL) was stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/2) to give the title compound (170 mg, 94%) as a white solid.
¹H-NMR (300MHz, CDCl₃) δ: 3.44 (3H, s), 4.45 (2H, s), 5.21 (2H, s), 5.32 (2H, s), 7.14 (1H, t, J = 7.5 Hz), 7.21-7.26 (1H, m), 7.36-7.46 (3H, m), 7.52-7.64 (3H, m), 7.77 (1H, dd, J = 7.5, 1.8 Hz).
Melting point: 99-100°C

### Reference Example 11

### N- {4-[3-(azidomethyl)phenyl]-3-cyano-6-[2-(methoxymethoxy)phenyl]pyridin-2-yl} thiophene-2-carboxamide

From the compound of Reference Example 10 (160 mg, 0.47 mmol), the title compound (160 mg, 79%) was given as white crystasl in substantially the same manner as in Reference Example 3.
¹H-NMR (300MHz, CDCl₃) δ: 3.34 (3H, s), 4.62 (2H, s), 5.32 (2H, s), 7.19(1H, t, J = 7.5 Hz), 7.27-7.31 (2H, m), 7.47-7.52 (1H, m), 7.59-7.74 (4H, m), 7.89-7.92 (1H, m), 7.97-7.98 (1H, m), 8.04 (1H, s), 8.15 (1H, d, J = 3.6 Hz), 11.47 (1H, s).
Melting point: 139-140°C

### Reference Example 12

### N-{4-[3-(aminomethyl)phenyl]-3-cyano-6-[2-(methoxymethoxy)phenyl]pyridin-2-yl} thiophene-2-carboxamide

10% palladium-carbon (containing 50% of water, 50 mg) was added to a solution of the compound of Reference Example 11 (150 mg, 0.30 mmol) in ethyl acetate (10 mL)-ethanol (10 mL) mixture, and stirred under a hydrogen pressure of 3 atm for 4 hours. The catalyst was filtered out, and the filtrate was concentrated to give the title compound (140 mg, 100%) as a pale green amorphous solid.
¹H-NMR (300MHz, CDCl₃) δ: 3.44 (3H, s), 3.98 (2H, s), 5.25 (2H, s), 7.12-7.17 (2H, m), 7.25 (1H, d, J = 7.5 Hz), 7.38-7.63 (5H, m), 7.68 (1H, s), 7.79-7.98 (3H, m).

### Reference Example 13

### 2-amino-4-{3-[(dimethylamino)methyl]phenyl}-6-[2-(methoxymethoxy)phenyl] nicotinonitrile

A solution of the compound of Reference Example 9 (200 mg, 0.47 mmol) in THF (5 mL) was added to a 2.0 M solution of dimethylamine in THF (5 mL, 10.0 mmol), and stirred at room temperature for 16 hours. The reaction mixture was diluted with saturated aqueous sodium hydrogencarbonate, and extracted twice with ethyl acetate. The extract liquids were combined, washed with saturated brine, dried over magnesium sulfate, and then concentrated under reduced pressure. The residue was purified by silica gel chromatography (eluting solution: ethyl acetate/hexane = 1/1) to give the title compound (70 mg, 75%) as a white amorphous solid.
¹H-NMR (300MHz, CDCl₃) δ: 2.27 (6H, s), 3.43 (3H, s), 3.50 (2H, s), 5.20 (2H, s), 5.44 (2H, br s), 7.13 (1H, t, J = 7.5 Hz), 7.21 (1H, d, J = 8.1 Hz), 7.32 (1H, s), 7.35-7.49 (3H, m), 7.52-7.55 (1H, m), 7.58 (1H, s), 7.73 (1H, dd, J = 7.5, 1.5 Hz).

### Reference Example 14

### N-{3-cyano-4-{3-[(dimethylamino)methyl]phenyl}-6-[2-(methoxymethoxy)phenyl] pyridin-2-yl}thiophene-2-carboxamide

From the compound of Reference Example 13 (70 mg, 0.18 mmol), the title compound (90 mg, 100%) was given as a white amorphous solid in substantially the same manner as in Reference Example 3.
¹H-NMR (300MHz, CDCl₃) δ: 2.28 (6H, s), 3.46 (3H, s), 3.52 (2H, s), 5.26 (2H, s), 7.13-7.18 (2H, m), 7.25 (1H, d, J = 6.6 Hz), 7.39-7.53 (3H, m), 7.59-7.63 (2H, m), 7.67 (1H, s), 7.78-7.90 (3H, m), 8.40-8.80 (1H, br).

### Reference Example 15

### tert-butyl {2-[(3-{3-cyano-6-[2-(methoxymethoxy)phenyl]-2-[(2-thienylcarbonyl) amino]pyridin-4-yl}benzoyl)amino]ethyl}carbamate

WSCD (79 mg, 0.41 mmol) was added to a solution of the compound of Reference Example 7 (200 mg, 0.41 mmol), tert-butyl N-(2-aminoethyl)carbamate (66 mg, 0.41 mmol) and HOBt (56 mg, 0.41 mmol) in DMF (5 mL)with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate) to give the title compound (230 mg, 89%) as a white solid.
¹H-NMR (300MHz, CDCl₃) δ: 1.35 (9H, s), 3.09-3.16 (2H, m), 3.30-3.33 (5H, m), 5.31 (2H, s), 6.93 (1H, t, J = 5.7 Hz), 7.20 (1H, t, J = 7.5 Hz), 7.27-7.31 (2H, m), 7.47-7.53 (1H, m), 7.72 (1H, t, J = 7.8 Hz), 7.88 (2H, dd, J = 7.8, 1.8 Hz), 7.97-8.06 (3H, m), 8.15-8.17 (2H, m), 8.65 (1H, t, J = 5.7 Hz), 11.48 (1H, s).
Melting point: 200-201°C

### Reference Example 16

### tert-butyl {3-[(3-{3-cyano-6-[2-(methoxymethoxy)phenyl]-2-[(2-thienylcarbonyl) amino]pyridin-4-yl}benzoyl)amino]propyl}carbamate

From the compound of Reference Example 7 (100 mg, 0.21 mmol) and tert-butyl N-(2-aminopropyl)carbamate (36 mg, 0.21 mmol), the title compound (110 mg, 82%) was given as a pale green amorphous solid in substantially the same manner as in Reference Example 15.
¹H-NMR (300MHz, CDCl₃) δ: 1.45 (9H, s), 3.18-3.26 (2H, m), 3.43 (3H, s), 3.50 (2H, q, J = 5.8 Hz), 5.01-5.09 (1H, br), 5.23 (2H, s), 5.35 (2H, br s), 7.09-7.15 (2H, m), 7.22 (1H, d, J = 8.1 Hz), 7.37-7.50 (2H, m), 7.56-7.61 (2H, m), 7.79-7.87 (3H, m), 7.96-8.00 (2H, m), 8.13 (1H, br s), 8.79 (1H, s).

### Reference Example 17

### tert-butyl [1-(3-{3-cyano-6-[2-(methoxymethoxy)phenyl]-2-[(2-thienylcarbonyl) amino]pyridin-4-yl}benzoyl)piperidine-4-yl]carbamate

From the compound of Reference Example 7 (100 mg, 0.21 mmol) and 4-(N-Boc-amino)piperidine (41 mg, 0.21 mmol), the title compound (140 mg, 100%) was given as a green amorphous solid in substantially the same manner as in Reference Example 15.
¹H-NMR (300MHz, CDCl₃) δ: 1.52 (9H, s), 1.86-2.10 (3H, m), 2.90-3.05 (1H, br), 3.10-3.25 (1H, br), 3.43 (3H, s), 3.64-3.90 (2H, br), 4.45-4.65 (2H, br), 5.24 (2H, s), 7.14-7.19 (2H, m), 7.25 (1H, d, J = 7.8 Hz), 7.43 (1H, t, J = 7.8 Hz), 7.55-7.71 (5H, m), 7.80 (1H, d, J = 3.3 Hz), 7.87-7.91 (2H, m), 8.56 (1H, s).

### Reference Example 18

### tert-butyl 4-(3-{3-cyano-6-[2-(methoxymethoxy)phenyl]-2-[(2-thienylcarbonyl)amino] pyridin-4- yl}benzoyl)piperazine-1-carboxylate

From the compound of Reference Example 7 (200 mg, 0.41 mmol) and 1-Boc-piperazine (77 mg, 0.41 mmol), the title compound (260 mg, 97%) was given as a green amorphous solid in substantially the same manner as in Reference Example 15.
¹H-NMR (300MHz, CDCl₃) δ: 1.46 (9H, s), 3.43 (3H, s), 3.43-3.90 (8H, br), 5.24 (2H, s), 7.14-7.20 (2H, m), 7.24-7.26 (1H, m), 7.43 (1H, t, J = 8.0 Hz), 7.58-7.73 (5H, m), 7.79 (1H, d, J = 4.0 Hz), 7.87-7.92 (2H, m), 8.47 (1H, s).

### Reference Example 19

### tert-butyl 4-[(3-{3-cyano-6-[2-(methoxymethoxy)phenyl]-2-[(2-thienylcarbonyl)amino] pyridin-4-yl}benzoyl)amino]piperidine-1-carboxylate

From the compound of Reference Example 7 (200 mg, 0.41 mmol) and 1-Boc-4-aminopiperidine (83 mg, 0.41 mmol), the title compound (190 mg, 69%) was given as a white amorphous solid in substantially the same manner as in Reference Example 15.
¹H-NMR (300MHz, DMSO-d₆) δ: 1.40 (11H, s), 1.82 (2H, d, J = 10.8 Hz), 2.70-2.90 (2H, br), 3.32 (3H, s), 3.92-4.07 (3H, m), 5.31 (2H, s), 7.19 (1H, t, J = 7.5 Hz), 7.27-7.31 (2H, m), 7.47-7.53 (1H, m), 7.72 (1H, t, J = 7.7 Hz), 7.88-7.91 (2H, m), 7.96 (1H, d, J = 4.8 Hz), 8.03-8.06 (2H, m), 8.15 (2H, s), 8.45 (1H, d, J = 7.8 Hz), 11.49 (1H, s).

### Reference Example 20

### 3-{2-amino-3-cyano-6-[2-(methoxymethoxy)phenyl]pyridin-4-yl}benzoic acid

A 4N aqueous sodium hydroxide solution (2 mL) was added to a solution of the compound of Reference Example 5 (1.0 g, 2.57 mmol) in THF (15 mL)-methanol (8 mL) mixture, and stirred at room temperature for 18 hours. The reaction mixture was diluted with water and neutralized with 1N hydrochloric acid, and extracted twice with ethyl acetate. The extract liquids were combined, washed with saturated brine, dried over magnesium sulfate, and then concentrated under reduced pressure to give the title compound (960 mg, 100%) as a pale yellow solid.
¹H-NMR (300MHz, DMSO-d₆) δ: 3.36 (3H, s), 5.25 (2H, s), 7.04 (2H, s), 7.13 (1H, t, J = 6.5 Hz), 7.20-7.24 (2H, m), 7.38-7.45 (1H, m), 7.61-7.78 (2H, m), 7.89-7.91 (1H, m), 8.08-8.10 (1H, m), 8.19 (1H, t, J = 1.5 Hz), 13.15-13.30 (1H, br).

### Reference Example 21

### tert-butyl 3-[(3-{2-amino-3-cyano-6-[2-(methoxymethoxy)phenyl]pyridin-4-yl} benzoyl)amino] piperidine-1-carboxylate

From the compound of Reference Example 20 (960 mg, 2.56 mmol) and 3-amino-1-Boc-piperidine (563 mg, 2.81 mmol), the title compound (1.1 g, 78%) was given as a white amorphous solid in substantially the same manner as in Reference Example 15.
¹H-NMR (300MHz, CDCl₃) δ: 1.42 (9H, s), 1.62-1.81 (2H, br), 1.82-2.00 (1H, br), 3.43 (3H, s), 3.20-3.40 (1H, br), 3.43 (3H, s), 3.44-3.70 (2H, br), 4.13-4.25 (1H, br), 5.22 (2H, s), 5.35 (2H, br s), 7.14 (1H, t, J = 7.1 Hz), 7.21 (1H, d, J = 8.4 Hz), 7.37-7.42 (2H, m), 7.58 (1H, t, J = 7.7 Hz), 7.75-7.79 (2H, m), 7.86 (1H, t, J = 7.8 Hz), 8.01 (1H, s).

### Reference Example 22

### tert-butyl 3-[(3-{3-cyano-6-[2-(methoxymethoxy)phenyl]-2-[(2-thienylcarbonyl)amino] pyridin-4-yl}benzoyl)amino]piperidine-1-carboxylate

2-thenoyl chloride (197 mg, 1.34 mmol) was added to a solution of the compound of Reference Example 21 (300 mg, 0.54 mmol) in pyridin(5 mL), and stirred at room temperature for 46 hours. A 28% aqueous solution (0.5 mL) of ammonia was added to the resultant mixture and stirred at room temperature for 1 hour. The reaction mixture was diluted with water, and extracted twice with ethyl acetate. The extract liquids were combined, washed with saturated brine, dried over magnesium sulfate, and then concentrated under reduced pressure. The residue was purified by silica gel chromatography (eluting solution: ethyl acetate/hexane = 2/1), and then recrystallized from ethyl acetate-hexane to give the title compound (235 mg, 65%) as white needles.
¹H-NMR (300MHz, DMSO-d₆) δ: 1.38-1.59 (11H, m), 1.72-1.77 (1H, br), 1.91-1.99 (1H, br), 2.76-2.89 (2H, br), 3.32 (3H, s), 3.79-4.07 (3H, br), 5.31 (2H, s), 7.19 (1H, t, J = 7.5 Hz), 7.22-7.31 (2H, m), 7.47-7.53 (1H, m), 7.72 (1H, t, J = 7.8 Hz), 7.88-7.91 (2H, m), 7.97 (1H, d, J = 4.8 Hz), 8.04 (2H, d, J = 5.7 Hz), 8.15 (2H, s), 8.44 (1H, d, J = 7.2 Hz), 11.46 (1H, s).
Melting point: 167-168°C

### Reference Example 23

### tert-butyl 3-[(3-{2-[(1-benzothiophene-2-ylcarbonyl)amino]-3-cyano-6-[2-(methoxymethoxy) phenyl]pyridin-4-yl}benzoyl)amino]piperidine-1-carboxylate

From the compound of Reference Example 21 (300 mg, 0.54 mmol) and benzothiophene-2-carbonyl chloride (264 mg, 1.34 mmol), the title compound (174 mg, 45%) was given as a white needles in substantially the same manner as in Reference Example 22.
¹H-NMR (300MHz, DMSO-d₆) δ: 1.38-1.59 (11H, m), 1.72-1.76 (1H, br), 1.91-1.99 (1H, br), 2.76-2.84 (2H, m), 3.32 (3H, s), 3.70-4.04 (3H, br), 5.32 (2H, s), 7.20 (1H, t, J = 7.8 Hz), 7.30 (1H, d, J = 7.8 Hz), 7.48-7.57 (3H, m), 7.73 (1H, t, J = 7.8 Hz), 7.90-7.93 (2H, m), 8.04-8.12 (3H, m), 8.16 (1H, s), 8.44-8.49 (2H, m), 11.74 (1H, s).
Melting point: 196-197°C

### Reference Example 24

### tert-butyl 3-[(3-{3-cyano-6-[2-(methoxymethoxy)phenyl]-2-[(pyridin-2-ylcarbonyl) amino] pyridin-4-yl}benzoyl)amino]piperidine-1-carboxylate

From the compound of Reference Example 21 (230 mg, 0.41 mmol) and nicotinoyl chloride (587 mg, 3.30 mmol), the title compound (102 mg, 38%) was given as a white powder in substantially the same manner as in Reference Example 22.
¹H-NMR (300MHz, CDCl₃) δ: 1.43 (9H, s), 1.51-2.00 (5H, br), 3.46-3.62 (7H, br), 4.13 -4.19 (1H, br), 5.27 (2H, s), 7.16 (1H, t, J = 7.2 Hz), 7.40-7.55 (2H, m), 7.64 (1H, t, J = 8.1 Hz), 7.83-7.91 (3H, m), 8.02 (1H, s), 8.10 (1H, s), 8.30-8.34 (1H, br), 8.56 (1H, br s), 8.84 (1H, br s), 9.24 (1H, br s).

### Reference Example 25

### Methyl 2- {2-amino-3-cyano-6-[2-(methoxymethoxy)phenyl]pyridin-4-yl}benzoate

A solution of 2'-(methoxymethoxy)acetophenone of Reference Example 1 (2.19 g, 12.18 mmol), methyl 2-formylbenzoate (2.0 g, 12.18 mmol), malononitrile(805 mg, 12.18 mmol) and ammonium acetate (1.41 g, 18.27 mmol) in toluene (15 mL) was refluxed while heating for 18 hours. After cooling, the reaction mixture was concentrated under reduced pressure and diluted with ethyl acetate. The diluted mixture was washed with saturated aqueous sodium hydrogencarbonate and then with saturated brine, dried over magnesium sulfate, and then concentrated under reduced pressure. The residue was purified by silica gel chromatography (eluting solution: ethyl acetate/hexane = 2/1 to 1/1), and then recrystallized from ethyl acetate-hexane to give the title compound (900 mg, 19%) as orange crystals.
¹H-NMR (300MHz, DMSO-d₆) δ: 3.31 (3H, s), 3.68 (3H, s), 5.14 (2H, s), 6.93 (2H, s), 7.02 (1H, s), 7.13 (1H, t, J = 8.0 Hz), 7.20 (1H, d, J = 7.8 Hz), 7.37-7.49 (2H, m), 7.63 (1H, t, J = 8.2 Hz), 7.71-7.80 (2H, m), 7.95 (1H, dd, J = 7.8, 1.8 Hz).
Melting point: 109-110°C

### Reference Example 26

### Methyl 2-{3-cyano-6-[2-(methoxymethoxy)phenyl]-2-[(2-thienylcarbonyl)amino] pyridin-4-yl) benzoate

From the compound of Reference Example 25 (600 mg, 1.54 mmol), the title compound (693 mg, 90%) was given as white crystals in substantially the same manner as in Reference Example 3.
¹H-NMR (300MHz, CDCl₃) δ: 3.41 (3H, s), 3.77 (3H, s), 5.22 (2H, s), 7.14-7.26 (4H, m), 7.38-7.46 (2H, m), 7.57-7.70 (3H, m), 7.76 (1H, s), 7.95 (1H, dd, J = 8.1, 1.8 Hz), 8.12 (1H, d, J = 8.0 Hz), 8.38 (1H, s).
Melting point: 190-191°C

### Reference Example 27

### 2-{3-cyano-6-[2-(methoxymethoxy)phenyl]-2-[(2-thienylcarbonyl)amino]pyridin-4-yl} benzoic acid

Substantially the same method as in Reference Example 7 was performed on the compound of Reference Example 26 (590 mg, 1.18 mmol), and recrystallizated from ethyl acetate-diethylether to give the title compound (512 mg, 89%) as white crystals.
¹H-NMR (300MHz, DMSO-d₆) δ: 3.33 (3H, s), 5.26 (2H, s), 7.19 (1H, t, J = 7.7 Hz), 7.26-7.29 (2H, m), 7.45-7.51 (2H, m), 7.67 (1H, t, J = 7.7 Hz), 7.77 (1H, t, J = 7.5 Hz), 7.87 (1H, s), 7.91-7.97 (2H, m), 8.08 (1H, dd, J = 7.8, 1.2 Hz), 8.14-8.15 (1H, m), 11.35 (1H, s), 13.10 (1H, s).
Melting point: 182-183°C

### Reference Example 28

### tert-butyl [4-chloro-6-(2-methoxyphenyl)pyridin-2-yl]carbamate

Methyl 6-(2-methoxyphenyl)-4-oxo-4H-pyran-2-carboxylate (21.5 g, 78.39 mmol) which can be synthesized in accordance with the method described in Journal of Chemical Society page 3663 (1956) and a 10% ammonia-methanol solution (250 mL) were heated at 110°C for 24 hours in an autoclave. After cooling, concentrated hydrochloric acid (400 mL) was added and refluxed for 1 hour. Then, acetic acid (150 mL) was added and refluxed for another 24 hours. After cooling, the resultant substance was concentrated, and the residue was diluted with ethanol. The generated precipitate was filtered out, and the filtrate was concentrated. The residue was recrystallized from DMF-diethylether to give
4-hydroxy-6-(2-methoxyphenyl)pyridin-2-carboxylic acid (21.5 g) as a gray solid.

4-hydroxy-6-(2-methoxyphenyl)pyridin-2-carboxylic acid (9.0 g, 36.7 mmol) was added to a mixed solution of DMF (15 mL) and thionyl chloride (36 mL) with ice-cooling and refluxed for 3 hours. After cooling to 0°C, methanol (180 mL) was added, and stirred at room temperature for 17 hours. The reaction mixture was diluted with ethyl acetate, and washed with saturated aqueous sodium hydrogencarbonate and then with water, dried over magnesium sulfate, and then concentrated under reduced pressure. The residue was purified by silica gel chromatography (eluting solution: ethyl acetate/hexane = 1/4 to 1/3) to give methyl
4-chloro-6-(2-methoxyphenyl)pyridin-2-carboxylate (3.65 g,) as a white solid.

A 4N aqueous sodium hydroxide solution (50 mL) was added to a solution of methyl 4-chloro-6-(2-methoxyphenyl)pyridin-2-carboxylate (7.74 g, 27.87 mmol) in THF (100 mL)-methanol (100 mL) mixture, and stirred at room temperature for 1 hour. The resultant substance was neutralized with 5N hydrochloric acid (40 mL) and then concentrated under reduced pressure. The residue was diluted with ethyl acetate, washed with saturated brine, dried over magnesium sulfate, and then concentrated under reduced pressure to give 4-chloro-6-(2-methoxyphenyl)pyridin-2-carboxylic acid (6.73 g) as a pale yellow solid.

A solution of the obtained 4-chloro-6-(2-methoxyphenyl)pyridin-2-carboxylic acid (5.0 g, 18.96 mmol), DPPA (6.26 g, 22.76 mmol) and triethylamine (5.0 g, 49.30 mmol) in toluene (100 mL) was stirred at room temperature for 2 hours. Tert-butanol (10 mL) was added, and stirred at 90°C for 19 hours. After cooling, the reaction mixture was concentrated. The residue was purified by silica gel chromatography (eluting solution: ethyl acetate/hexane = 1/4) to give the title compound (5.61 g, 86%) as a pale orange amorphous solid.
¹H-NMR (300MHz, CDCl₃) δ: 1.53 (9H, s), 3.87 (3H, s), 6.98-7.07 (2H, m), 7.33-7.40 (1H, m), 7.55 (1H, d, J = 1.5 Hz), 7.71 (1H, dd, J = 7.5, 1.5 Hz), 7.93 (1H, d, J = 1.5 Hz).

### Reference Example 29

### 2-amino-4-chloro-6-(2-methoxyphenyl)pyridin trifluoroacetate

Trifluoroacetate (15 mL) was added to a solution of the compound of Reference Example 28 (5.60 g, 16.73 mmol) in dichloromethane (30 mL), and stirred at room temperature for 3 hours. Diisopropylether (200 mL) and hexane (200 mL) were added to the reaction mixture, and the precipitate was collected by filtration and dried to give 2-amino-4-chloro-6-(2-methoxyphenyl)pyridine trifluoroacetate (4.11 g, 70%) as a white powder.
¹H-NMR (300MHz, CDCl₃) δ: 3.87 (3H, s), 6.77 (1H, s), 6.82 (1H, s), 7.04-7.09 (2H, m), 7.40 (1H, dd, J = 7.5, 1.5 Hz), 7.48-7.53 (1H, m).

### Reference Example 30

### N-[4-chloro-6-(2-methoxyphenyl)pyridin-2-yl]thiophene-2-carboxamide

2-thenoyl chloride (93 mg, 0.64 mmol) was added to a solution of the compound of Reference Example 19 (185 mg, 0.53 mmol) and triethylamine (118 mg, 1.17 mmol) in dichloromethane(10 mL), and stirred at room temperature for 16 hours. The reaction mixture was diluted with saturated aqueous sodium hydrogencarbonate, and extracted twice with ethyl acetate. The extract liquids were combined, washed with saturated brine, dried over magnesium sulfate, and then concentrated under reduced pressure. The residue was purified by amino silica gel chromatography (eluting solution: ethyl acetate/hexane = 1/2) to give the title compound (180 mg, 100%) as a white amorphous solid.
¹H-NMR (300MHz, CDCl₃) δ: 3.87 (3H, s), 6.98-7.21 (3H, m), 7.40 (1H, t, J = 8.1 Hz), 7.58-7.76 (3H, m), 7.98 (1H, d, J = 4.8 Hz), 8.34 (1H, s), 8.69 (1H, br s).

### Reference Example 31

### N-[6-(2-methoxyphenyl)-4-(3-nitrophenyl)pyridin-2-yl]thiophene-2-carboxamide

A solution of the compound of Reference Example 30 (180 mg, 0.52 mmol), 3-nitrophenyl boric acid (105 mg, 0.63 mmol), tetrakis(triphenylphosphine)palladium(0) in DME (5 mL) and a 2M aqueous solutionof sodium carbonate (1 mL) was refluxed for 21 hours in an argon atmosphere. After cooling, the reaction mixture was diluted with saturated aqueous sodium hydrogencarbonate, and extracted twice with ethyl acetate. The extract liquids were combined, washed with saturated brine, dried over magnesium sulfate, and then concentrated under reduced pressure. The residue was purified by amino silica gel chromatography (eluting solution: ethyl acetate/hexane = 1/2 to 1/1) to give the title compound (1.34 g, 91 %) as a pale yellow amorphous solid.
¹H-NMR (300MHz, CDCl₃) δ: 3.86 (3H, s), 7.02-7.16 (3H, m), 7.41 (1H, t, J = 7.7 Hz), 7.55-7.72 (4H, m), 7.80 (1H, d, J = 1.2 Hz), 8.06 (1H, d, J = 8.1 Hz), 8.31 (1H, d, J = 8.1 Hz), 8.59-8.63 (2H, m), 8.71 (1H, br s).

### Reference Example 32

### N-[6-(2-hydroxyphenyl)-4-(3-nitrophenyl)pyridin-2-yl]thiophene-2-carboxamide

A 1.0M solution of boron tribromide in dichloromethane(1.0 mL, 1.0 mmol) was added to a solution of the compound of Reference Example 31 (90 mg, 0.21 mmol) in dichloromethane (5 mL) at -78°C, and stirred at room temperature for 2 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/2) to give the title compound (40 mg, 46%) as a yellow amorphous solid.
¹H-NMR (300MHz, CDCl₃) δ: 6.93-7.00 (2H, m), 7.20-7.22 (1H, m), 7.36-7.39 (1H, m), 7.60-7.88 (5H, m), 8.04 (1H, d, J = 8.7 Hz), 8.33 (1H, d, J = 8.8 Hz), 8.51 (1H, s), 8.61 (1H, s), 8.66 (1H, s), 12.87(1H, br s).

### Reference Example 33

### N-[6-(2-{[tert-butyl(dimethyl)silyl]oxy}phenyl)-3-cyano-4-(3-nitrophenyl)pyridin-2-yl]-5-chlorothiophene-2-carboxamide

From 2-amino-6-[2-((tert-butyl(dimethyl)silyl)oxy)phenyl]-4-(3-nitrophenyl)-nicotinonitrile (1.0 g, 2.24 mmol) described in WO 02/44153 and 5-chloro-2-thenoyl chloride (1.22 g, 6.72 mmol), the title compound (1.16 g, 88%) was given as a pale yellow solid in substantially the same manner as in Reference Example 3.
¹H-NMR (300MHz, DMSO-d₆) δ: 0.08 (6H, s), 0.70 (9H, s), 7.05 (1H, d, J = 8.3 Hz), 7.17 (1 H, t, J = 7.5 Hz), 7.34 (1H, d, J = 4.1 Hz), 7.40-7.50 (1H, m), 7.84 (1H, dd, J = 7.7, 1.7 Hz), 7.93 (1H, t, J = 8.1 Hz), 8.00-8.09 (2H, m), 8.17 (1H, d, J = 7.9 Hz), 8.46 (1H, d, J = 8.3 Hz), 8.52 (1H, s), 11.66 (1H, s).

### Reference Example 34

### N-[6-(2-{[tert-butyl(dimethyl)silyl]oxy}phenyl)-3-cyano-4-(3-nitrophenyl)pyridin-2-yl] thiophene-3-carboxamide

From 2-amino-6-[2-((tert-butyl(dimethyl)silyl)oxy)phenyl]-4-(3-nitrophenyl)-nicotinonitrile (1.0 g, 2.24 mmol) described in WO 02/44153 and 3-thenoyl chloride (0.985 g, 6.72 mmol), the title compound (780 mg, 63%) was given as pale yellow crystals in substantially the same manner as in Reference Example 3.
¹H-NMR (300MHz, DMSO-d₆) δ: 0.09 (6H, s), 0.71 (9H, s), 7.05 (1H, d, J = 8.3 Hz), 7.17 (1H, t, J = 7.5 Hz), 7.40-7.49 (1H, m), 7.68-7.75 (2H, m), 7.85 (1H, dd, J = 7.9, 1.5 Hz), 7.94 (1H, t, J = 8.1 Hz), 8.02 (1H, s), 8.17 (1H, d, J = 7.9 Hz), 8.46 (1H, d, J = 8.3 Hz), 8.54 (2H, d, J = 7.5 Hz), 11.33 (1H, s).
Melting point: 139-142°C

### Reference Example 35

### 2-amino-4-(2-chloro-5-nitrophenyl)-6-[2-(methoxymethoxy)phenyl]nicotinonitrile

From 2-chloro-5-nitrobenzaldehyde (5.0 g, 26.94 mmol), the title compound (3.1 g, 28%) was given as a pale yellow solid in substantially the same manner as in Reference Example 2.
¹H-NMR (300MHz, CDCl₃) δ: 3.42 (3H, s), 5.20 (2H, s), 5.39 (2H, br s), 7.13-7.22 (3H, m), 7.41 (1H, t, J = 5.0 Hz), 7.73 (1H, d, J = 9.6 Hz), 7.82 (1H, dd, J = 7.8, 1.8 Hz), 8.29-8.31 (2H, m).
Melting point: 187-188°C

### Reference Example 36

### N-{4-(2-chloro-5-nitrophenyl)-3-cyano-6-[2-(methoxymethoxy)phenyl]pyridin-2-yl} thiophene-2-carboxamide

From the compound of Reference Example 35 (1.50 g, 3.65 mmol), the title compound (1.40 g, 74%) was given as white needles in substantially the same manner as in Reference Example 3.
¹H-NMR (300MHz, CDCl₃) δ: 3.44 (3H, s), 5.25 (2H, s), 7.16-7.26 (2H, m), 7.45 (1H, t, J = 8.0 Hz), 7.66 (1H, d, J = 4.8 Hz), 7.77-7.81 (3H, m), 7.89-7.96 (2H, m), 8.50 (1H, br s).
Melting point: 208-209°C

### Reference Example 37

### N-{4-(5-amino-2-chlorophenyl)-3-cyano-6-[2-(methoxymethoxy)phenyl]pyridin-2-yl} thiophene-2-carboxamide

From the compound of Reference Example 36 (1.0 g, 1.92 mmol), the title compound (810 mg, 86%) was given as white needles in substantially the same manner as in Reference Example 4.
¹H-NMR (300MHz, CDCl₃) δ: 3.44 (3H, s), 5.25 (2H, s), 6.73-6.80 (2H, m), 7.13-7.18 (2H, m), 7.24-7.32 (4H, m), 7.42 (1H, t, J = 6.6 Hz), 7.63 (1H, d, J = 4.8 Hz), 7.79-7.91 (3H, m).
Melting point: 183-184°C

### Reference Example 38

### tert-butyl {3-[(4-chloro-3-{3-cyano-6-[2-(methoxymethoxy)phenyl]-2-[(2-thienylcarbonyl)amino]pyridin-4-yl}phenyl)amino]-3-oxopropyl}carbamate

WSCD (279 mg, 1.47 mmol) was added to a solution of the compound of Reference Example 37 (200 mg, 0.41 mmol), Boc-β-alanine (276 mg, 1.47 mmol) and HOBt (198 mg, 1.47 mmol) in DMF (5 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate) to give the title compound (250 mg, 92%) as a yellow amorphous solid.
¹H-NMR (300MHz, CDCl₃) δ: 1.42 (9H, s), 2.50-2.63 (2H, m), 3.43-3.47 (5H, m), 5.25-5.29 (3H, m), 7.12-7.17 (2H, m), 7.25 (1H, d, J = 7.8 Hz), 7.40-7.46 (2H, m), 7.62-7.66 (2H, m), 7.77-7.89 (4H, m), 8.59 (1H, br s).

### Reference Example 39

### 2-amino-4-[3-(aminomethyl)phenyl]-6-[2-(methoxymethoxy)phenyl]nicotinonitrile

An solution of the compound of Reference Example 10 (690 mg, 1.79 mmol) and Pd-C (200 mg) in ethanol (10 mL)-THF (10 mL) was stirred at room temperature for 1 hour in a hydrogen atmosphere. The catalyst was filtered out, and the filtrate was concentrated under reduced pressure to give the title compound (640 mg, 100%) as a yellow amorphous solid.
¹H-NMR (300MHz, CDCl₃) δ: 2.00-2.50 (2H, br), 3.44 (3H, s), 3.96 (2H, s), 5.26 (2H, s), 5.35 (2H, br s), 7.10-7.26 (3H, m), 7.35-7.41 (2H, m), 7.49-7.65 (3H, m), 7.70 (1H, s), 7.72 (1H, d, J = 6.6 Hz).

### Reference Example 40

### tert-butyl {2-[(3-{2-amino-3-cyano-6-[2-(methoxymethoxy)phenyl]pyridin-4-yl} benzyl)amino]-2-oxoethyl }carbamate

From the compound of Reference Example 39 (200 mg, 0.55 mmol) and Boc-glycine (97 mg, 0.55 mmol), the title compound (190 mg, 67%) was given as a colorless amorphous solid in substantially the same manner as in Reference Example 38.
¹H-NMR (300MHz, CDCl₃) δ: 1.45 (9H, s), 3.42 (3H, s), 3.80-3.90 (2H, br), 4.53 (2H, d, J = 6.0 Hz), 5.20 (1H, s), 5.40-5.44 (3H, m), 6.81 (1H, t, J = 5.7 Hz), 7.12 (1H, t, J = 7.5 Hz), 7.20 (1H, d, J = 8.4 Hz), 7.27-7.51 (4H, m), 7.58 (1H, s), 7.73 (1H, d, J = 7.5 Hz).

### Reference Example 41

### tert-butyl {3-[(3-{2-amino-3-cyano-6-[2-(methoxymethoxy)phenyl]pyridin-4-yl} benzyl)amino]-3-oxopropyl}carbamate

From the compound of Reference Example 39 (200 mg, 0.55 mmol) and Boc-β-alanine (105 mg, 0.55 mmol), the title compound (200 mg, 67%) was given as a colorless amorphous solid in substantially the same manner as in Reference Example 38.
¹H-NMR (300MHz, CDCl₃) δ: 1.39 (9H, s), 2.10 (1H, s), 2.45 (2H, t, J = 5.7 Hz), 3.37-3.42 (5H, m), 4.50 (2H, d, J = 5.7 Hz), 5.20 (1H, s), 5.22-5.35 (1H, br), 5.46 (2H, s), 6.46-6.55 (1H, br), 7.12 (1H, t, J = 7.5 Hz), 7.20 (1H, d, J = 8.4 Hz), 7.27-7.56 (6H, m), 7.73 (1H, dd, J = 7.8, 1.5 Hz).

### Reference Example 42

### di-tert-butyl {[(3-{2-amino-3-cyano-6-[2-(methoxymethoxy)phenyl]pyridin-4-yl} benzyl)amino]methylidene}biscarbamate

A solution of the compound of Reference Example 39 (200 mg, 0.55 mmol), 1,3-di-Boc-2-(trifluoromethylsulfonyl)guanidine (217 mg, 0.55 mmol) and triethylamine (72 mg, 0.55 mmol) in DMF (5 mL) was stirred at room temperature for 18 hours. The reaction mixture was diluted with saturated aqueous sodium hydrogencarbonate, and extracted twice with ethyl acetate. The extract liquids were combined, washed with saturated brine, dried over magnesium sulfate, and then concentrated under reduced pressure. The residue was purified by amino silica gel chromatography (eluting solution: ethyl acetate/hexane = 1/2) to give the title compound (280 mg, 84%) as a yellow oil.
¹H-NMR (300MHz, CDCl₃) δ: 1.45 (9H, s), 1.50 (9H, s), 3.44 (3H, s), 4.70-4.73 (2H, m), 5.20 (2H, s), 5.61 (2H, br s), 7.12 (1H, t, J = 7.5 Hz), 7.21 (1H, d, J = 7.8 Hz), 7.27-7.30 (1H, m), 7.35-7.58 (5H, m), 7.71 (1H, dd, J = 7.5, 1.5 Hz), 8.65-8.72 (1H, br).

### Reference Example 43

### tert-butyl {2-[(3-{3-cyano-6-[2-(methoxymethoxy)phenyl]-2-[(2-thienylcarbonyl) amino]pyridin-4-yl}benzyl)amino]-2-oxoethyl}carbamate

From the compound of Reference Example 40 (190 mg, 0.37 mmol), the title compound (122 mg, 53%) was given as white crystals in substantially the same manner as in Reference Example 6.
¹H-NMR (300MHz, CDCl₃) δ: 1.41 (9H, s), 3.46 (3H, s), 3.90 (2H, d, J = 6.0 Hz), 5.58 (2H, d, J = 6.0 Hz), 5.28 (2H, s), 6.64 (1H, t; J = 6.0 Hz), 7.13-7.19 (2H, m), 7.25-7.28 (1H, m), 7.41-7.68 (6H, m), 7.81-7.88 (3H, m), 8.40-8.65 (1H, br).
Melting point: 131-133°C

### Reference Example 44

### tert-butyl {3-[(3-{3-cyano-6-[2-(methoxymethoxy)phenyl]-2-[(2-thienylcarbonyl) amino]pyridin-4-yl}benzyl)amino]-3-oxopropyl}carbamate

From the compound of Reference Example 41 (200 mg, 0.38 mmol), the title compound (137 mg, 56%) was given as white crystals in substantially the same manner as in Reference Example 6.
¹H-NMR (300MHz, CDCl₃) δ: 1.42 (9H, s), 2.51 (2H, t, J = 6.0 Hz), 3.44-3.48 (5H, m), 4.57 (2H, d, J = 5.4 Hz), 5.20-5.29 (3H, m), 6.16-6.30 (1H, br), 7.16-7.21 (2H, m), 7.21-7.28 (1H, m), 7.43-7.68 (6H, m), 7.84-7.90 (3H, m), 8.40-8.70 (1H, br s).
Melting point: 123-124°C

### Reference Example 45

### di-tert-butyl {[(3-{3-cyano-6-[2-(methoxymethoxy)phenyl]-2-[(2-thienylcarbonyl) amino]pyridin-4-yl}benzyl)amino]methylylidene}biscarbamate

From the compound of Reference Example 42 (280 mg, 0.46 mmol), the title compound (80 mg, 24%) was given as a pale green amorphous solid in substantially the same manner as in Reference Example 6.
¹H-NMR (300MHz, CDCl₃) δ: 1.48 (9H, s), 1.53 (9H, s), 3.45 (3H, s), 4.75 (2H, d, J = 5.4 Hz), 5.25 (2H, s), 7.13-7.18 (2H, m), 7.26 (1H, d, J = 8.7 Hz), 7.38-7.69 (6H, m), 7.78 (1H, d, J = 2.4 Hz), 7.87 (1H, s), 7.91 (1H, s), 8.59 (1H, s), 8.66-8.75 (1H, br), 11.55 (1H, s).

### Reference Example 46

### 5'-chloro-2'-(methoxymethoxy)acetophenone

From 5'-chloro-2'-hydroxyacetophenone (5.0 g, 29.31 mmol), the title compound (5.58 g, 89%) was given as a colorless liquid in substantially the same manner as in Reference Example 1.
¹H-NMR (300MHz, CDCl₃) δ: 2.63 (3H, s), 3.51 (3H, s), 5.26 (2H, s), 7.15 (1H, d, J = 9.0 Hz), 7.37 (1H, dd, J = 9.0, 2.7 Hz), 7.67 (1H, d, J = 2.7 Hz).

### Reference Example 47

### 2-amino-6-[5-chloro-2-(methoxymethoxy)phenyl]-4-(3-nitrophenyl)nicotinonitrile

From the compound of Reference Example 46 (5.58 g, 26.04 mmol), the title compound (1.14 g, 11%) was given as a pale brown solid in substantially the same manner as in Reference Example 2.
¹H-NMR (300MHz, CDCl₃) δ: 3.46 (3H, s), 5.22 (2H, s), 5.42 (2H, br s), 7.18 (1H, d, J = 9.0 Hz), 7.35 (1H, dd, J = 9.0, 2.4 Hz), 7.41 (1H, s), 7.74 (1H, t, J = 7.1 Hz), 7.83 (1H, d, J = 2.4 Hz), 8.02 (1H, d, J = 8.7 Hz), 8.36 (1H, d, J = 8.4 Hz), 8.46 (1H, s).
Melting point: 192-193°C

### Reference Example 48

### N- {6-[5-chloro-2-(methoxymethoxy)phenyl]-3-cyano-4-(3-nitrophenyl)pyridin-2-yl} thiophene-2-carboxamide

From the compound of Reference Example 47 (500 mg, 1.22 mmol), the title compound (360 mg, 57%) was given as white crystals in substantially the same manner as in Reference Example 6.
¹H-NMR (300MHz, DMSO-d₆) δ: 3.48 (3H, s), 5.27 (2H, s), 7.19-7.26 (3H, m), 7.39-7.42 (1H, m), 7.68 (1H, d, J = 4.8 Hz), 7.75-7.83 (2H, m), 7.95 (1H, s), 7.99 (1H, s), 8.13-8.52 (3H, m).
Melting point: 161-162°C

### Reference Example 49

### N-{4-(3-aminophenyl)-6-[5-chloro-2-(methoxymethoxy)phenyl]-3-cyanopyridin-2-yl} thiophene-2-carboxamide

From the compound of Reference Example 48 (300 mg, 0.58 mmol), the title compound (212 mg, 74%) was given as pale brown crystals in substantially the same manner as in Reference Example 4.
¹H-NMR (300MHz, CDCl₃) δ: 3.45 (3H, s), 5.23 (2H, s), 6.69-6.91 (1H, m), 7.04-7.08 (2H, m), 7.19-7.38 (4H, m), 7.64 (1H, d, J = 7.5 Hz), 7.81-7.88 (3H, m), 8.30-8.60 (1H, br).
Melting point: 180-182°C

### Reference Example 50

### tert-butyl {3-[(3-{6-[5-chloro-2-(methoxymethoxy)phenyl]-3-cyano-2-[(2-thienylcarbonyl) amino]pyridin-4-yl}phenyl)amino]-3-oxopropyl}carbamate

From the compound of Reference Example 49 (100 mg, 0.20 mmol) and Boc-β-alanine (77 mg, 0.41 mmol), the title compound (91 mg, 69%) was given as white crystals in substantially the same manner as in Reference Example 38.
¹H-NMR (300MHz, DMSO-d₆) δ: 1.38 (9H, s), 3.21-3.28 (2H, m), 3.78 (3H, s), 5.37 (2H, s), 6.80-6.90 (1H, br), 7.27-7.40 (3H, m), 7.52-7.58 (2H, m), 7.73 (1H, d, J = 7.8 Hz), 7.98 (2H, d, J = 3.0 Hz), 8.06-8.16 (3H, m), 10.24 (1H, s), 11.41-11.50 (1H, br). Melting point: 230-231 °C

### Reference Example 51

### N-[6-[5-chloro-2-(methoxymethoxy)phenyl]-3-cyano-4-(3-nitrophenyl)pyridin-2-yl] benzamide

From the compound of Reference Example 47 (500 mg, 1.22 mmol) and benzoyl chloride, the title compound (390 mg, 62%) was given as pale yellow crystals in substantially the same manner as in Reference Example 6.
¹H-NMR (300MHz, DMSO-d₆) δ: 3.49 (3H, s), 5.27 (2H, s), 7.21 (1H, d, J = 8.7 Hz), 7.38 (1H, dd, J = 8.7, 2.7 Hz), 7.53-7.68 (3H, m), 7.78 (1H, t, J = 8.1 Hz), 7.91 (1H, d, J = 2.7 Hz), 8.01-8.05 (3H, m), 8.15 (1H, d, J = 8.4 Hz), 8.41 (1H, d, J = 7.8 Hz), 8.40-8.43 (1H, m), 8.50-8.64 (1H, br).
Melting point: 205-206°C

### Reference Example 52

### N- {4-(3-aminophenyl)-6-[5-chloro-2-(methoxymethoxy)phenyl]-3-cyanopyridin-2-yl} benzamide

From the compound of Reference Example 51 (300 mg, 0.59 mmol), the title compound (203 mg, 71 %) was given as pale brown crystals in substantially the same manner as in Reference Example 4.
¹H-NMR (300MHz, CDCl₃) δ: 3.44 (3H, s), 5.22 (2H, s), 6.84 (1H, dd, J = 8.1, 1.8 Hz), 7.03-7.05 (2H, m), 7.20 (1H, d, J = 9.0 Hz), 7.26-7.37 (3H, m), 7.51-7.65 (3H, m), 7.84 (1H, d, J = 2.7 Hz), 7.90 (1H, s), 8.01 (1H, s), 8.03 (1H, s), 8.50-8.60 (1H, br).
Melting point: 174-175°C

### Reference Example 53

### tert-butyl {3-[(3-{2-(benzoylamino)-6-[5-chloro-2-(methoxymethoxy)phenyl]-3-cyanopyridin-4-yl}phenyl)amino]-3-oxopropyl}carbamate

From the compound of Reference Example 52 (100 mg, 0.21 mmol) and Boc-β-alanine (78 mg, 0.41 mmol), the title compound (96 mg, 70%) was given as white crystals in substantially the same manner as in Reference Example 38.
¹H-NMR (300MHz, DMSO-d₆) δ:1.37 (9H, s), 2.50-2.55 (2H, m), 3.21-3.27 (2H, m), 3.38 (3H, s), 5.37 (2H, s), 6.83-6.91 (1H, br), 7.32-7.40 (2H, m), 7.52-7.74 (6H, m), 7.96 (1H, d, J = 2.7 Hz), 8.07-8.11 (4H, m), 10.24 (1H, s), 11.49 (1H, br s).
Melting point: 233-234°C

### Reference Example 54

### N-(3-cyano-6-[2-(methoxymethoxy)phenyl]-4-{3-[(propylamino)carbonyl]phenyl} pyridin-2-yl)thiophene-2-carboxamide

From the compound of Reference Example 7 (100 mg, 0.21 mmol) and propylamine (12 mg, 0.21 mmol), the title compound (90 mg, 81 %) was given as a white amorphous solid in substantially the same manner as in Reference Example 15.
¹H-NMR (300MHz, CDCl₃) δ: 1.01 (3H, t, J = 7.5 Hz), 1.61-1.73 (2H, m), 3.46 (2H, q, J = 6.7 Hz), 5.27 (2H, s), 6.19-6.30 (1H, br), 7.14-7.26 (3H, m), 7.41-7.46 (1H, m), 7.60-7.66 (2H, m), 7.77-7.98 (5H, m), 8.07 (1H, s), 8.45 (1H, s).

### Reference Example 55

### 3-{2-(benzoylamino)-3-cyano-6-[2-(methoxymethoxy)phenyl]pyridin-4-yl}-N-(2-hydroxyethyl)benzamide

From the compound of Reference Example 7 (100 mg, 0.21 mmol) and 2-ethanolamine (13 mg, 0.21 mmol), the title compound (84 mg, 76%) was given as a white solid in substantially the same manner as in Reference Example 15.
¹H-NMR (300MHz, CDCl₃) δ: 3.34-3.39 (2H, m), 3.53 (2H, q, J = 5.9 Hz), 4.74 (1H, t, J = 5.6 Hz), 5.31 (2H, s), 7.20 (1H, t, J = 7.5 Hz), 7.22-7.31 (2H, m), 7.50 (1H, t, J = 7.8 Hz), 7.72 (1H, t, J = 7.7 Hz), 7.89 (2H, d, J = 7.8 Hz), 7.97 (1H, d, J = 4.8 Hz), 8.06 (2H, d, J = 5.1 Hz), 8.16 (2H, d, J = 6.0 Hz), 8.63 (1H, t, J = 5.4 Hz), 11.49 (1H, s).

### Reference Example 56

### N-{3-cyano-6-[2-(methoxymethoxy)phenyl]-4-[3-(morpholine-4-ylcarbonyl)phenyl] pyridin-2-yl}benzamide

From the compound of Reference Example 7 (200 mg, 0.41 mmol) and morpholine (36 mg, 0.41 mmol), the title compound (190 mg, 84%) was given as a green amorphous solid in substantially the same manner as in Reference Example 15.
¹H-NMR (300MHz, CDCl₃) δ: 3.35 (3H, s), 3.43-3.90 (8H, br), 5.24 (2H, s), 7.14-7.20 (2H, m), 7.24-7.26 (1H, m), 7.40-7.46 (1H, m), 7.62-7.74 (5H, m), 7.79 (1H, d, J = 7.1 Hz), 7.87-7.96 (2H, m), 8.49 (1H, s).

### Reference Example 57

### N- {3-[2-amino-6-(2- {[tert-butyl(dimethyl)silyl]oxy}phenyl)-3-cyanopyridin-4-yl] phenyl} -2,2,2-trifluoroacetamide

From 2-amino-6-[2-((tert-butyl(dimethyl)silyl)oxy)phenyl]-4-(3-nitrophenyl)-nicotinonitrile (500 mg, 1.12 mmol) described in WO 02/44153, 2-amino-4-(3-aminophenyl)-6-(2-{[tert-butyl(dimethyl)silyl]oxy}phenyl)nicotinonitrile (470 mg, 100%) was given as a pale yellow amorphous solid in substantially the same manner as in Reference Example 4.

Trifluoroacetic anhydride (249 mg, 1.18 mmol) was added to a solution of the resultant solid (470 mg, 1.12 mmol) and triethylamine(114 mg, 1.13 mmol) in dichloromethane (10 mL) with ice-cooling, and stirred for 1 hour. The reaction mixture was diluted with saturated aqueous sodium hydrogencarbonate, and extracted twice with ethyl acetate. The extract liquids were combined, washed with saturated brine, dried over magnesium sulfate, and then concentrated under reduced pressure.
The residue was purified by amino silica gel chromatography (eluting solution: ethyl acetate/hexane = 1/2) to give the title compound (410 mg, 71%) as a white solid.
¹H-NMR (300MHz, CDCl₃) δ: 0.06 (6H, s), 0.78 (9H, s), 5.34 (2H, br s), 6.90 (1H, d, J = 8.1 Hz), 7.08 (1H, t, J = 7.5 Hz), 7.28-7.34 (2H, m), 7.46-7.57 (2H, m), 7.67-7.76 (3H, m), 7.94 (1H, br s).

### Reference Example 58

### 2-amino-6-(2-{[tert-butyl(dimethyl)silyl]oxy}phenyl)-4-(4-nitrophenyl)nicotinonitrile

A solution of 2'-{[tert-butyl(dimethyl)silyl]oxy}acetophenone (4.0 g, 16.0 mmol), 4-nitrobenzaldehyde (2.4 g, 16.0 mmol), malononitrile (1.2 g, 17.5 mmol) and ammonium acetate (1.9 g, 23.9 mmol) in toluene (80 mL) was refluxed while heating for 6 hours. After cooling, saturated aqueous sodium hydrogencarbonate was added to the reaction mixture, and extracted twice with ethyl acetate. The organic layers were combined, washed with saturated brine, dried over magnesium sulfate, and then concentrated under reduced pressure. The residue was purified by silica gel chromatography (eluting solution: ethyl acetate/hexane = 1/1). Thus, yellow crystals were obtained. Recrystallization was performed from ethyl acetate-hexane to give the title compound (1.0g, 14%) as pale yellow crystals.
¹H-NMR (300MHz, CDCl₃) δ: 0.08 (6H, s), 0.78 (9H, s), 5.39 (2H, br s), 6.93 (1H, dd, J = 1.5, 0.6 Hz), 7.11 (1H, td, J = 7.8, 0.9 Hz), 7.21-7.30 (2H, m), 7.63-7.74 (3H, m), 8.38 (2H, d, J = 8.7 Hz).
Melting point: 168-169°C

### Reference Example 59

### N-[6-(2- [tert-butyl(dimethyl)silyl]oxylphenyl)-3-cyano-4-(4-nitrophenyl)pyridin-2-yl] thiophene-2-carboxamide

2-thenoyl chloride (535 µL, 5.3 mmol) was added to a solution of the compound of Reference Example 58 (900 mg, 2.0 mmol) in pyridin (7 mL), and stirred at room temperature for 20 hours. The reaction mixture was diluted ethyl acetate, washed with water and then with saturated brine, dried over magnesium sulfate, and then concentrated under reduced pressure. The residue was purified by amino silica gel chromatography (eluting solution: ethyl acetate/hexane = 5/95 to 1/1), and then recrystallized from tetrahydrofuran-hexane to give the title compound (663 mg, 60%) as white crystals.
¹H-NMR (300MHz, CDCl₃) δ: 0.10 (6H, s), 0.77 (9H, s), 6.95 (1H, dd, J = 8.1, 0.6 Hz), 7.09-7.21 (2H, m), 7.37 (1H, td, J = 7.5, 1.8 Hz), 7.66 (1H, dd, J = 5.1, 1.2 Hz), 7.78-7.90 (4H, m), 7.95 (1H, s), 8.40 (2H, d, J = 9.0 Hz), 8.45 (1H, br s).
Melting point: 168-169°C
Element analysis: Calculated (%) for C₂₉H₂₈N₄O₄SSi: C, 62.57; H, 5.07; N, 10.06. Found (%): C, 62.21; H, 4.97; N, 10.06.

### Reference Example 60

### N-[4-(4-aminophenyl)-6-(2-{[tert-butyl(dimethyl)silyl]oxy}phenyl)-3-cyanopyridin-2-yl]thiophene-2-carboxamide

From the compound of Reference Example 59 (470 mg, 0.84 mmol), the title compound (400 mg, 90%) was given as an amorphous solid in substantially the same manner as in Reference Example 4.
¹H-NMR (300MHz, CDCl₃) δ: 0.07 (6H, s), 0.80 (9H, s), 3.95 (2H, br s), 6.78 (2H, d, J = 8.4 Hz), 6.93 (1H, d, J = 8.1 Hz), 7.07-7.21 (2H, m), 7.28-7.37 (1H, m), 7.55 (2H, d, J = 8.4 Hz), 7.63 (1H, dd, J = 4.8, 0.9 Hz), 7.73-7.81 (2H, m), 7.84 (1H, s), 8.39 (1H, s).

### Reference Example 61

### Benzyl [3-(3-formylphenoxy)propyl]carbamate

Diethyl azodicarboxylate (2.5 mL, 16.0 mmol) was added to a solution of 3-hydroxybenzaldehyde (2.0 g, 16.0 mmol), benzyl (3-hydroxypropyl)carbamate (3.4 g, 16.0 mmol) and triphenylphosphine (1.2 g, 16.0 mmol) in THF (40 mL) with ice-cooling, and stirred at room temperature overnight. The solvent was filtered out under reduced pressure. Then, the residue was diluted with ethyl acetate, washed with water and then with saturated brine, dried over magnesium sulfate, and then concentrated under reduced pressure. The residue was purified by silica gel chromatography (eluting solution: ethyl acetate/hexane = 3/7) to give the title compound (3.7 g, 73%) as an oily substance.
¹H-NMR (300MHz, CDCl₃) δ: 1.95-2.15 (2H, m), 3.43 (2H, td, J = 6.3, 6.0 Hz), 4.09 (2H, t, J = 6.0 Hz), 4.95 (1H, br s), 5.11 (2H, s), 7.17 (1H, m), 7.28-7.40 (6H, m), 7.41-7.49 (2H, m), 9.97 (1H, s).

### Reference Example 62

### Benzyl (3-{3-[2-amino-6-(2-{[tert-butyl(dimethyl)silyl]oxy}phenyl)-3-cyanopyridin-4-yl]phenoxy}propyl)carbamate

Using 1-(2-{[tert-butyl(dimethyl)silyl]oxy}phenyl)ethanone (3.0 g, 11.8 mmol) and benzyl [3-(3-formylphenoxy)propyl]carbamate (3.7 g, 11.8 mmol), the title compound (590 mg, 8%) was given as an amorphous solid in substantially the same manner as in Reference Example 2.
¹H-NMR (300MHz, CDCl₃) δ: 0.06 (6H, s), 0.78 (9H, s), 1.95-2.11 (2H, m), 3.33-3.48 (2H, m), 4.09 (2H, t, J = 6.6 Hz), 5.10 (2H, s), 5.30 (2H, s), 6.90 (1H, dd, J = 8.1, 0.6 Hz), 6.99 (1H, d, J = 8.4 Hz), 7.04-7.11 (2H, m), 7.16 (1H, d, J = 8.1 Hz), 7.25-7.41 (9H, m), 7.66 (1H, dd, J = 7.8, 1.8 Hz).

### Reference Example 63

### Benzyl [3-(3-{6-(2-{[tert-butyl(dimethyl)silyl]oxy}phenyl)-3-cyano-2-[(2-thienylcarbonyl)amino]pyridin-4-yl}phenoxy)propyl]carbamate

From the compound of Reference Example 62 (590 mg, 0.97 mmol), the title compound (123 mg, 18%) was given as an oily substance in substantially the same manner as in Reference Example 3.
¹H-NMR (300MHz, CDCl₃) δ: 0.07 (6H, s), 0.78 (9H, s), 1.98-2.18 (2H, m), 3.38-3.50 (2H, m), 4.11 (2H, t, J = 7.2 Hz), 5.02 (1H, br s), 5.11 (2H, s), 6.89-6.96 (1H, m), 6.98-7.08 (1H, m), 7.09-7.45 (11H, m), 7.64 (1H, d, J = 6.0 Hz), 7.69 (1H, d, J = 5.4 Hz), 7.82 (1H, dd, J = 7.8, 1.8 Hz), 7.89 (1H, s), 8.42 (1H, s).

### Reference Example 64

### Methyl 5-{2-amino-3-cyano-6-[2-(methoxymethoxy)phenyl]pyridin-4-yl}-2-methoxybenzoate

From methyl 5-formyl-2-methoxybenzoate (2.0 g, 10.3 mmol), the title compound (1.3 g, 30%) was given as an oily substance in substantially the same manner as in Reference Example 2.
¹H-NMR (300MHz, CDCl₃) δ: 3.45 (3H, s), 3.90 (3H, s), 3.98 (3H, s), 5.21 (2H, s), 5.33 (2H, br s), 7.10-7.18 (2H, m), 7.21 (1H, dd, J = 8.1, 0.6 Hz), 7.33 (1H, s), 7.35-7.43 (1H, m), 7.75 (1H, dd, J = 7.5, 1.8 Hz), 7.84 (1H, dd, J = 8.7, 2.4 Hz), 8.08 (1H, d, J = 2.4 Hz).

### Reference Example 65

### Methyl 5-{3-cyano-6-[2-(methoxymethoxy)phenyl]-2-[(2-thienylcarbonyl)amino] pyridin-4-yl}-2-methoxybenzoate

From the compound of Reference Example 64 (310 mg, 0.74 mmol), the title compound (200 mg, 51 %) was given as a yellow powder in substantially the same manner as in Reference Example 3. ¹H-NMR (300MHz, CDCl₃) δ: 3.47 (3H, s), 3.93 (3H, s), 4.00 (3H, s), 5.27 (2H, s), 7.05-7.25 (4H, m), 7.43-7.50 (1H, m), 7.64 (1H, br s), 7.79 (1H, br s), 7.80-8.00 (3H, m), 8.14 (1H, d, J = 2.4 Hz), 8.45 (1H, br s).
Melting point: 105-106°C

### Reference Example 66

### 5- {3-cyano-6-[2-(methoxymethoxy)phenyl]-2-[(2-thienylcarbonyl)amino]pyridin-4-yl}-2-methoxybenzoic acid

A 4N aqueous sodium hydroxide solution (0.7 mL) was added to a solution of the compound of Reference Example 65 (500 mg, 0.94 mmol) in THF (1.5 mL)-ethanol (5 mL), and stirred at 50°C for 35 minutes. The reaction mixture was cooled and then neutralized with a 4N hydrogen chloride-ethyl acetate solution, and the solvent was removed under reduced pressure. The residue was dissolved in chloroform and washed with saturated brine. The organic layer was dried over magnesium sulfate, and then concentrated under reduced pressure to give the title compound (360 mg, 74%) as a pale yellow solid.
¹H-NMR (300MHz CDCl₃) δ: 3.50 (3H, s), 4.18 (3H, s), 5.30 (2H, s), 7.10-7.20 (3H, m), 7.21-7.30 (1H, m), 7.38-7.50 (1H, m), 7.65 (1H, d, J = 4.5 Hz), 7.80 (1H, d, J = 3.0 Hz), 7.88 (1H, d, J = 7.5 Hz), 7.99 (1H, s), 8.15 (1H, dd, J = 8.7, 2.7 Hz), 8.47 (1H, d, J = 2.4 Hz), 8.55 (1H, br s).
Melting point: 187-189°C

### Reference Example 67

### tert-butyl {2-[(5-{3-cyano-6-[2-(methoxymethoxy)phenyl]-2-[(2-thienylcarbonyl) amino]pyridin-4-yl}-2-methoxybenzoyl)amino]ethyl}carbamate

From the compound of Reference Example 66 (340 mg, 0.66 mmol), the title compound (235 mg, 54%) was given as a yellow powder in substantially the same manner as in Reference Example 15.
¹H-NMR (300MHz, CDCl₃) δ: 1.43 (9H, s), 3.35-3.43 (2H, m), 3.47 (3H, s), 3.54-3.68 (2H, m), 4.08 (3H, s), 5.00 (1H, br s), 5.29 (2H, s), 7.09-7.25 (3H, m), 7.35-7.48 (1H, m), 7.64 (1H, d, J = 4.8 Hz), 7.79 (1H, d, J = 3.3 Hz), 7.86 (1H, d, J = 7.5 Hz), 7.99 (1H, s), 8.01 (1H, dd, J = 8.7, 2.4 Hz), 8.16 (1H, br s), 8.48 (1H, d, J = 2.4 Hz), 8.51 (1H, s).
Melting point: 116-117°C

### Reference Example 68

### Methyl 5-{3-cyano-6-(2-hydroxyphenyl)-2-[(2-thienylcarbonyl)amino]pyridin-4-yl}-2-methoxybenzoate

From the compound of Reference Example 65 (190 mg, 0.36 mmol), the title compound (150 mg, 86%) was given as a yellow powder in substantially the same manner as in Example 3
¹H-NMR (300MHz, DMSO-d₆) δ: 3.85 (3H, s), 3.95 (3H, s), 6.94-7.03 (2H, m), 7.31 (1H, dd, J = 4.8,3.9 Hz), 7.37-7.48 (2H, m), 7.96 (1H, dd, J = 8.7, 2.4 Hz), 8.01 (1H, d, J = 4.2 Hz), 8.05 (1H, d, J = 2.4 Hz), 8.18 (1H, dd, J = 7.8, 1.2 Hz), 8.21 (1H, s), 11.43 (1H, s), 11.95 (1H, br s).
Melting point: 271-273°C

### Reference Example 69

### 4'-methoxy-2'-(methoxymethoxy)acetophenone

From 2'-hydroxy-4'-methoxyacetophenone (5.0 g, 30.09 mmol), the title compound (4.4 g, 70%) was given as a colorless liquid in substantially the same manner as in Reference Example 1.
¹H-NMR (300MHz, CDCl₃) δ: 2.60 (3H, s), 3.52 (3H, s), 3.84 (3H, s), 5.28 (2H, s), 6.58 (1H, dd, J = 8.7, 1.8 Hz), 6.70 (1H, d, J = 1.8 Hz), 7.81 (1H, d, J = 8.7 Hz).

### Reference Example 70

### 2-amino-6-[4-methoxy-2-(methoxymethoxy)phenyl]-4-(3-nitrophenyl)nicotinonitrile

From the compound of Reference Example 69 (2.0 g, 9.53 mmol), the title compound (850 mg, 22%) was given as yellow crystals in substantially the same manner as in Reference Example 2.
¹H-NMR (300MHz, CDCl₃) δ: 3.47 (3H, s), 3.86 (3H, s), 5.24 (2H, s), 5.37 (2H, br s), 6.69 (1H, dd, J = 8.7, 2.4 Hz), 6.78 (1H, d, J = 2.4 Hz), 7.44 (1H, s), 7.72 (1H, t, J = 8.0 Hz), 7.86 (1H, d, J = 8.7 Hz), 8.01 (1H, dd, J = 6.6, 1.2 Hz), 8.36 (1H, d, J = 8.4 Hz), 8.47 (1H, d, J = 2.1 Hz).
Melting point: 171-172°C

### Reference Example 71

### N-[3-cyano-6-[4-methoxy-2-(methoxymethoxy)phenyl]-4-(3-nitrophenyl)pyridin-2-yl] benzamide

Benzoyl chloride (311 mg, 2.21 mmol) was added to a solution of the compound of Reference Example 70 (300 mg, 0.74 mmol) in pyridin(5 mL), and stirred at room temperature for 18 hours. A 28% aqueous solution of ammonia (1 mL) was added to the resultant mixture and stirred at room temperature for 1 hour. The reaction mixture was diluted with water, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1) to give the title compound (360 mg, 95%) as a yellow amorphous solid.
¹H-NMR (300MHz, CDCl₃) δ: 3.49 (3H, s), 3.83 (3H, s), 5.27 (2H, s), 6.64 (1H, dd, J = 8.7, 2.1 Hz), 6.77 (1H, d, J = 2.1 Hz), 7.48-7.62 (3H, m), 7.73 (1H, t, J = 8.1 Hz), 7.94 (1H, d, J = 8.7 Hz), 8.00-8.02 (3H, m), 8.11 (1H, d, J = 7.2 Hz), 8.36 (1H, d, J = 7.2 Hz), 8.49 (1H, d, J = 1.8 Hz), 8.86 (1H, s).

### Reference Example 72

### N-{4-(3-aminophenyl)-3-cyano-6-[4-methoxy-2-(methoxymethoxy)phenyl]pyridin-2-yl}benzamide

From the compound of Reference Example 71 (360 mg, 0.71 mmol), the title compound (340 mg, 100%) was given as a yellow amorphous solid in substantially the same manner as in Reference Example 4.
¹H-NMR (300MHz, CDCl₃) δ: 3.42 (3H, s), 3.80 (3H, s), 5.20 (2H, s), 6.62 (1H, dd, J = 9.0, 2.1 Hz), 6.75-6.77 (2H, m), 6.96-7.00 (2H, m), 7.25 (1H, t, J = 7.8 Hz), 7.46 (2H, t, J = 7.4 Hz), 7.53 (1H, d, J = 7.2 Hz), 7.85-7.98 (4H, m), 8.85 (1H, s).

### Reference Example 73

### tert-butyl {3-[(3-{2-(benzoylamino)-3-cyano-6-[4-methoxy-2-(methoxymethoxy) phenyl]pyridin-4-yl}phenyl)amino]-3-oxopropyl} carbamate

From the compound of Reference Example 72 (340 mg, 0.71 mmol) and Boc-β-alanine (268 mg, 1.42 mmol), the title compound (151 mg, 32%) was given as a white amorphous solid in substantially the same manner as in Reference Example 38.
¹H-NMR (300MHz, CDCl₃) δ: 1.44 (9H, s), 2.64 (2H, t, J = 6.0 Hz), 3.48-3.55 (5H, m), 3.86 (3H, s), 5.10-5.20 (1H, br), 5.31 (2H, s), 6.69 (1H, dd, J=8.7, 2.1 Hz), 6.82 (1H, s), 7.48-7.63 (6H, m), 7.91-8.04 (6H, m).

### Reference Example 74

### 4'-chloro-2'-(methoxymethoxy)acetophenone

From 4'-chloro-2'-hydroxyacetophenone (1.0 g, 5.86 mmol), the title compound (860 mg, 68%) was given as a colorless liquid in substantially the same manner as in Reference Example 1.
¹H-NMR (300MHz, CDCl₃) δ: 2.62 (3H, s), 3.53 (3H, s), 5.28 (2H, s), 7.03 (1H, dd, J = 8.4, 2.1 Hz), 7.22 (1H, d, J = 2.1 Hz), 7.68 (1H, d, J = 8.7 Hz).

### Reference Example 75

### 2-amino-6-[4-chloro-2-(methoxymethoxy)phenyl]-4-(3-nitrophenyl)nicotinonitrile

From the compound of Reference Example 74 (860 mg, 4.01 mmol), the title compound (413 mg, 25%) was given as a yellow crystal in substantially the same manner as in Reference Example 2.
¹H-NMR (300MHz, CDCl₃) δ: 3.48 (3H, s), 5.24 (2H, s), 5.40 (2H, br s), 7.13 (1H, dd, J = 8.4, 2.1 Hz), 7.26 (1H, d, J = 2.1 Hz), 7.39 (1H, s), 7.71-7.81 (2H, m), 8.01 (1H, dt, J = 8.1, 0.9 Hz), 8.37 (1H, dq, J = 8.1, 0.9 Hz), 8.46 (1H, t, J = 1.8 Hz). Melting point: 196-198°C

### Reference Example 76

### N-[6-[4-chloro-2-(methoxymethoxy)phenyl]-3-cyano-4-(3-nitrophenyl)pyridin-2-yl] benzamide

From the compound of Reference Example 75 (200 mg, 0.49 mmol), the title compound (240 mg, 95%) was given as a yellow amorphous solid in substantially the same manner as in Reference Example 71.
¹H-NMR (300MHz, CDCl₃) δ: 3.51 (3H, s), 5.29 (2H, s), 7.15 (1H, dd, J = 8.4, 1.8 Hz), 7.29 (1H, d, J = 1.8 Hz), 7.55 (2H, t, J = 7.5 Hz), 7.63 (1H, d, J = 7.2 Hz), 7.78 (1H, t, J = 8.1 Hz), 7.90 (1H, d, J = 8.4 Hz), 7.99-8.17 (3H, m), 8.15 (1H, dt, J = 7.5, 0.9 Hz), 8.41 (1H, dq, J = 8.4, 0.9 Hz), 8.52 (1H, t, J = 1.8 Hz), 8.58 (1H, s).

### Reference Example 77

### tert-butyl {3-[(3-{2-(benzoylamino)-6-[4-chloro-2-(methoxymethoxy)phenyl]-3-cyanopyridin-4-yl}phenyl)amino]-3-oxopropyl carbamate

A solution of the compound of Reference Example 76 (240 mg, 0.47 mmol), iron powder (155 mg, 2.79 mmol) and ammonium chloride (162 mg, 3.03 mmol) in ethanol (10 mL)-water (10 mL) was refluxed while heating for 2 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give N-{4-(3-aminophenyl)-6-[4-chloro-2-(methoxymethoxy)phenyl]-3-cyanopyridin-2-yl} benzamide (110 mg) as a yellow amorphous solid.

WSCD (87 mg, 0.45 mmol) was added to a solution of the resultant N- {4-(3-aminophenyl)-6-[4-chloro-2-(methoxymethoxy)phenyl]-3-cyanopyridin-2-yl} benzamide (110 mg), Boc-β-alanine (86 mg, 0.45 mmol) and HOBt (61 mg, 0.45 mmol) in DMF (3 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1 to 2/1) to give the title compound (61 mg, 25%) as a white amorphous solid.
¹H-NMR (300MHz, CDCl₃) δ: 1.44 (9H, s), 2.64 (2H, t, J = 5.6 Hz), 3.48-3.53 (5H, m), 5.05-5.16 (1H, br), 5.30 (2H, s), 7.13 (1H, dd, J = 8.4, 1.8 Hz), 7.26-7.30 (1H, m), 7.48-7.65 (6H, m), 7.87 (1H, d, J = 8.4 Hz), 7.95-8.03 (5H, m), 8.40-8.70 (1H, br).

### Reference Example 78

### N-[6-[4-chloro-2-(methoxymethoxy)phenyl]-3-cyano-4-(3-nitrophenyl)pyridin-2-yl] thiophene-2-carboxamide

From the compound of Reference Example 74 (200 mg, 0.49 mmol), the title compound (188 mg, 74%) was given as a yellow amorphous solid in substantially the same manner as in Reference Example 22.
¹H-NMR (300MHz, CDCl₃) δ: 3.51 (3H, s), 5.29 (2H, s), 7.14-7.21 (2H, m), 7.30 (1H, d, J = 1.8 Hz), 7.67 (1H, d, J = 5.1 Hz), 7.75-7.82 (2H, m), 7.92-7.98 (2H, m), 8.13 (1H, d, J = 7.8 Hz), 8.41 (1H, d, J = 7.5 Hz), 8.43-8.52 (2H, m).

### Reference Example 79

### N-{4-(3-aminophenyl)-6-[4-chloro-2-(methoxymethoxy)phenyl]-3-cyanopyridin-2-yl} thiophene-2-carboxamide

From the compound of Reference Example 78 (150 mg, 0.29 mmol), the title compound (140 mg, 100%) was given as a green amorphous solid in substantially the same manner as in Reference Example 4.
¹H-NMR (300MHz, CDCl₃) δ: 3.39 (3H, s), 3.70-4.00 (2H, br), 5.15 (2H, s), 6.72 (1H, d, J = 7.2 Hz), 6.90-7.20 (5H, m), 7.54 (1H, br s), 7.65-7.80 (4H, m), 8.54 (1H, br s).

### Reference Example 80

### tert-butyl {3-[(3-{6-[4-chloro-2-(methoxymethoxy)phenyl]-3-cyano-2-[(2-thienylcarbonyl)amino]pyridin-4-yl}phenyl)amino]-3-oxopropyl}carbamate

From the compound of Reference Example 79 (140 mg, 0.29 mmol) and Boc-β-alanine (108 mg, 0.58 mmol), the title compound (103 mg, 54%) was given as a white crystal in substantially the same manner as in Reference Example 38.
¹H-NMR (300MHz, DMSO-d₆) δ: 1.37 (9H, s), 2.50-2.55 (2H, m), 3.22-3.30 (2H, m), 3.36 (3H, s), 5.41 (2H, s), 6.80-6.95 (1H, br), 7.25-7.29 (2H, m), 7.3.7-7.39 (2H, m), 7.54 (1H, td, J = 7.8, 3.0 Hz), 7.72 (1H, d, J = 7.5 Hz), 7.94-7.98 (2H, m), 8.02 (1H, s), 8.15 (1H, s), 8.16 (1H, s), 10.24 (1H, s), 11.46 (1H, s).
Melting point: 194-195°C

### Reference Example 81

### 2'-methoxy-6'-(methoxymethoxy)acetophenone

From 2'-hydroxy-6'-methoxyacetophenone (5.0 g, 30.09 mmol), the title compound (4.2 g, 67%) was given as a colorless liquid in substantially the same manner as in Reference Example 1.
¹H-NMR (300MHz, CDCl₃) δ: 2.50 (3H, s), 3.46 (3H, s), 3.81 (3H, s), 5.16 (2H, s), 6.60 (1H, d, J = 8.4 Hz), 6.75 (1H, d, J = 8.4 Hz), 7.22 (1H, d, J = 8.4 Hz).

### Reference Example 82

### 2-amino-6-[2-methoxy-6-(methoxymethoxy)phenyl]-4-(3-nitrophenyl)nicotinonitrile

From the compound of Reference Example 81 (2.0 g, 9.53 mmol), the title compound (1.33 g, 34%) was given as a yellow amorphous solid in substantially the same manner as in Reference Example 2.
¹H-NMR (300MHz, CDCl₃) δ: 3.39 (3H, s), 3.77 (3H, s), 5.12 (2H, s), 5.43 (2H, br s), 6.68 (1H, d, J = 8.4 Hz), 6.83-6.86 (2H, m), 7.32 (1H, t, J = 8.4 Hz), 7.71 (1H, t, J = 8.1 Hz), 8.02 (1H, d, J = 8.1 Hz), 8.35 (1H, d, J = 8.1 Hz), 8.46 (1H, d, J = 1.8 Hz).

### Reference Example 83

### N-[3-cyano-6-[2-methoxy-6-(methoxymethoxy)phenyl]-4-(3-nitrophenyl)pyridin-2-yl] benzamide

From the compound of Reference Example 82 (300 mg, 0.74 mmol), the title compound (360 mg, 95%) was given as a yellow amorphous solid in substantially the same manner as in Reference Example 76.
¹H-NMR (300MHz, CDCl₃) δ: 3.39 (3H, s), 3.78 (3H, s), 5.13 (2H, s), 6.70 (1H, d, J = 8.4 Hz), 6.88 (1H, d, J = 8.4 Hz), 7.33-7.40 (2H, m), 7.52 (2H, t, J = 7.2 Hz), 7.60 (1H, d, J = 7.2 Hz), 7.76 (1H, t, J = 8.1 Hz), 8.00 (2H, d, J = 7.8 Hz), 8.16 (1H, d, J = 7.8 Hz), 8.39 (1H, d, J = 8.1 Hz), 8.51 (1H, t, J = 2.1 Hz), 8.70 (1H, s).

### Reference Example 84

### N-{4-(3-aminophenyl)-3-cyano-6-[2-methoxy-6-(methoxymethoxy)phenyl]pyridin-2-yl}benzamide

From the compound of Reference Example 83 (300 mg, 0.59 mmol), the title compound (300 mg, 100%) was given as a yellow amorphous solid in substantially the same manner as in Reference Example 4.
¹H-NMR (300MHz, CDCl₃) δ: 3.35 (3H, s), 3.75 (3H, s), 5.10 (2H, s), 6.69 (1H, d, J = 8.4 Hz), 6.80-6.88 (2H, m), 7.04-7.07 (2H, m), 7.26-7.35 (3H, m), 7.47-7.61 (3H, m), 7.99 (2H, d, J = 6.6 Hz), 8.68 (1H, br s).

### Reference Example 85

### tert-butyl {3-[(3-{2-(benzoylamino)-3-cyano-6-[2-methoxy-6-(methoxymethoxy) phenyl]pyridin-4-yl}phenyl)amino]-3-oxopropyl}carbamate

From the compound of Reference Example 84 (300 mg, 0.62 mmol) and Boc-β-alanine (268 mg, 1.42 mmol), the title compound (236 mg, 58%) was given as a white crystal in substantially the same manner as in Reference Example 38.
¹H-NMR (300MHz, CDCl₃) δ: 1.43 (9H, s), 2.62 (2H, t, J = 5.7 Hz), 3.36 (3H, s), 3.40-3.51 (2H, m), 3.75 (3H, s), 5.11 (2H, s), 5.12-5.20 (1H, br), 6.68 (1H, d, J = 8.4 Hz), 6.87 (1H, d, J = 8.4 Hz), 7.34 (2H, t, J = 8.4 Hz), 7.45-7.60 (5H, m), 7.61-7.74 (1H, br), 7.92-8.05 (4H, m), 8.68-8.78 (1H, br).
Melting point: 220-221°C

### Reference Example 86

### Methyl 4-{2-amino-3-cyano-6-[2-(methoxymethoxy)phenyl]pyridin-4-yl}benzoate

From the compound of Reference Example 1 (5.48 g, 30.46 mmol) and methyl p-formylbenzoate (5.0 g, 30.46 mmol), the title compound (4.11 g, 35%) was given as a yellow crystal in substantially the same manner as in Reference Example 5.
¹H-NMR (300MHz, CDCl₃) δ: 3.43 (3H, s), 3.96 (3H, s), 5.21 (2H, s), 5.36 (2H, br s), 7.14 (1H, td, J = 7.5, 0.9 Hz), 7.21 (1H, dd, J = 8.4, 0.9 Hz), 7.35-7.42 (2H, m), 7.69 (2H, d, J = 8.4 Hz), 7.77 (1H, dd, J = 7.2, 1.8 Hz), 8.18 (2H, d, J = 8.4 Hz).
Melting point: 170-171°C

### Reference Example 87

### 4-{3-cyano-6-[2-(methoxymethoxy)phenyl]-2-[(2-thienylcarbonyl)amino]pyridin-4-yl} benzoic acid

2-thenoyl chloride (282 mg, 1.93 mmol) was added to a solution of the compound of Reference Example 86 (300 mg, 0.77 mmol) in pyridin(10 mL), and stirred at room temperature for 18 hours. A 4N aqueous sodium hydroxide solution (2.5 mL) was added to the resultant mixture and stirred at room temperature for 1 hour. The resultant mixture was diluted with water and neutralized with 5N hydrochloric acid, and then extracted with ethyl acetate. The extract was washed with saturated brine, dried over magnesium sulfate, and then concentrated under reduced pressure. The residue was recrystallized from ethyl acetate to give the title compound (301 mg, 81%) as a yellow crystal.
¹H-NMR (300MHz, DMSO-d₆) δ: 3.34 (3H, s), 5.32 (2H, s), 7.19 (1H, t, J = 8.4 Hz), 7.27-7.31 (2H, m), 7.50 (1H, t, J = 7.8 Hz), 7.84-8.00 (4H, m), 8.06 (1H, d, J = 2.4 Hz), 8.14-8.18 (3H, m), 11.47 (1H, s), 13.10-13.40 (1H, br).
Melting point: 205-206°C

### Reference Example 88

### tert-butyl {2-[(4-{3-cyano-6-[2-(methoxymethoxy)phenyl]-2-[(2-thienylcarbonyl) amino]pyridin-4-yl}benzoyl)amino]ethyl}carbamate

From the compound of Reference Example 87 (200 mg, 0.41 mmol), the title compound (301 mg, 81 %) was given as a white powder in substantially the same manner as in Reference Example 15.
¹H-NMR (300MHz, DMSO-d₆) δ: 1.38 (9H, s), 2.49-2.51 (2H, m), 3.10-3.17 (2H, m), 3.34 (3H, s), 5.32 (2H, s), 6.93 (1H, t, J = 5.4 Hz), 7.19 (1H, t, J = 7.5 Hz), 7.27-7.31 (2H, m), 7.49 (1H, td, J = 7.8, 1.8 Hz), 7.81 (2H, d, J = 8.1 Hz), 7.91 (1H, dd, J = 7.5, 1.8 Hz), 7.97 (1H, d, J = 5.1 Hz), 8.04 (3H, d, J = 6.3 Hz), 8.16 (1H, d, J = 3.6 Hz), 8.62-8.65 (1H, br), 11.47 (1H, s).

### Reference Example 89

### 2'-(ethoxymethoxy)-4'-methylacetophenone

From 2'-hydroxy-4'-methylacetophenone (10.0 g, 66.59 mmol) and chloromethyl ethyl ether (8.0 g, 84.94 mol), the title compound (9.7 g, 75%) was given as a colorless liquid in substantially the same manner as in Reference Example 1.
¹H-NMR (300MHz, CDCl₃) δ: 1.26 (3H, t, J = 7.2 Hz), 2.37 (3H, s), 2.61 (3H, s), 3.76 (2H, q, J = 7.2 Hz), 5.32 (2H, s), 6.84 (1H, d, J = 7.8 Hz), 7.01 (1H, s), 7.65 (1H, d, J = 7.8 Hz).

### Reference Example 90

### 2-amino-6-[2-(ethoxymethoxy)-4-methylphenyl]-4-(3-nitrophenyl)nicotinonitrile

From the compound of Reference Example 89 (5.0 g, 25.79 mmol), the title compound (3.20 g, 31 %) was given as yellow needles in substantially the same manner as in Reference Example 2.
¹H-NMR (300MHz, CDCl₃) δ: 1.20 (3H, t, J = 7.2 Hz), 2.40 (3H, s), 3.70 (2H, q, J = 7.2 Hz), 5.27 (2H, s), 5.38 (2H, br s), 6.95 (1H, dd, J = 8.4, 0.9 Hz), 7.05 (1H, s), 7.42 (1H, s), 7.69-7.75 (2H, m), 8.02 (1H, d, J = 7.8 Hz), 8.36 (1H, dq, J = 8.4, 0.9 Hz), 8.46 (1H, t,J=1.8Hz).
Melting point: 148-149°C

### Reference Example 91

### N-[3-cyano-6-[2-(ethoxymethoxy)-4-methylphenyl]-4-(3-nitrophenyl)pyridin-2-yl] thiophene-2-carboxamide

From the compound of Reference Example 90 (1.0 g, 2.47 mmol), the title compound (630 mg, 50%) was given as a yellow crystal in substantially the same manner as in Reference Example 22.
¹H-NMR (300MHz, CDCl₃) δ: 1.21 (3H, t, J = 6.8 Hz), 2.42 (3H, s), 3.73 (2H, q, J = 6.8 Hz), 5.33 (2H, s), 6.98 (1H, d, J = 7.8 Hz), 7.10 (1H, s), 7.19 (1H, t, J = 4.5 Hz), 7.65-7.87 (4H, m), 8.01 (1H, br s), 8.14 (1H, d, J = 7.8 Hz), 8.40 (1H, dt, J = 7.5, 0.9 Hz), 8.49-8.52 (2H, m).
Melting point: 121-123°C

### Reference Example 92

### 4'-fluoro-2'-(ethoxymethoxy)acetophenone

From 4'-fluoro-2'-hydroxyacetophenone (5.0 g, 32.43 mmol), the title compound (5.8 g, 90%) was given as a colorless liquid in substantially the same manner as in Reference Example 89.
¹H-NMR (300MHz, CDCl₃) δ: 1.25 (3H, t, J = 7.2 Hz), 2.61 (3H, s), 3.76 (2H, q, J = 7.2 Hz), 5.32 (2H, s), 6.74 (1H, tt, J = 8.1, 2.4 Hz), 6.96 (1H, dd, J = 10.8, 2.4 Hz), 7.78 (1H, t,J=8.1Hz).

### Reference Example 93

### 2-amino-6-[2-(ethoxymethoxy)-4-fluorophenyl]-4-(3-nitrophenyl)nicotinonitrile

From the compound of Reference Example 92 (5.0 g, 23.60 mmol), the title compound (1.80 g, 19%) was given as a yellow powder in substantially the same manner as in Reference Example 2.
¹H-NMR (300MHz, CDCl₃) δ: 1.21 (3H, t, J = 7.2 Hz), 3.70 (2H, q, J = 7.2 Hz), 5.28 (2H, s), 5.39 (2H, br s), 6.85 (1H, t, J = 8.4 Hz), 7.01 (1H, dd, J = 10.8,2.4 Hz), 7.38 (1H, s), 7.73 (1H, t, J = 8.1 Hz), 7.84 (1H, t, J = 7.8 Hz), 8.00 (1H, d, J = 7.8 Hz), 8.37 (1H, dq, J = 8.1, 1.2 Hz), 8.46 (1H, t, J = 2.4 Hz).

### Reference Example 94

### N-[3-cyano-6-[2-(ethoxymethoxy)-4-fluorophenyl]-4-(3-nitrophenyl)pyridin-2-yl] thiophene-2-carboxamide

From the compound of Reference Example 93 (1.0 g, 2.50 mmol), the title compound (910 mg, 70%) was given as a yellow crystal in substantially the same manner as in Reference Example 22.
¹H-NMR (300MHz, CDCl₃) δ: 1.22 (3H, t, J = 6.9 Hz), 3.73 (2H, q, J = 6.9 Hz), 5.33 (2H, s), 6.88 (1H, t, J = 7.2 Hz), 7.05 (1H, d, J = 10.8 Hz), 7.20 (1H, t, J = 4.4 Hz), 7.63-7.84 (3H, m), 7.97-8.02 (2H, m), 8.13 (1H, d, J = 7.8 Hz), 8.39-8.51 (3H, m). Melting point: 166-167°C

### Reference Example 95

### tert-butyl {3-[(3-{3-cyano-6-[2-(ethoxymethoxy)-4-fluorophenyl]-2-[(2-thienylcarbonyl)amino]pyridin-4-yl}phenyl)amino]-3-oxopropyl} carbamate

A solution of the compound of Reference Example 94 (200 mg, 0.39 mmol), iron powder (129 mg, 2.31 mmol) and ammonium chloride (134 mg, 2.50 mmol) in ethanol (10 mL)-water (10 mL) was refluxed while heating for 2 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give N-{4-(3-aminophenyl)-3-cyano-6-[2-(ethoxymethoxy)-4-fluorophenyl]pyridin-2-yl} thiophene-2-carboxamide (150 mg) as a brown amorphous solid.

WSCD (117 mg, 0.61 mmol) was added to a solution of the resultant N-{4-(3-aminophenyl)-3-cyano-6-[2-(ethoxymethoxy)-4-fluorophenyl]pyridin-2-yl} thiophene-2-carboxamide (150 mg), Boc-β-alanine (116 mg, 0.61 mmol) and HOBt (83 mg, 0.61 mmol) in DMF (10 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1) to give the title compound (66 mg, 32%) as a yellow powder.
¹H-NMR (300MHz, CDCl₃) δ: 1.21 (3H, t, J = 7.2 Hz), 1.44 (9H, s), 2.63 (2H, t, J = 6.0 Hz), 3.49 (2H, q, J = 6.0 Hz), 3.71 (2H, q, J = 7.2 Hz), 5.12-5.20 (1H, br), 5.34 (2H, s), 6.85 (1H, td, J = 8.4, 2.4 Hz), 7.05 (1H, d, J = 10.5 Hz), 7.17 (1H, t, J = 4.5 Hz), 7.42-7.50 (2H, m), 7.60-7.65 (2H, m), 7.75-7.80 (1H, br), 7.93-7.98 (3H, m), 8.08-8.20 (1H, br), 8.52 (1H, br s).

### Reference Example 96

### 5'-methoxy-2'-(methoxymethoxy)acetophenone

From 2'-hydroxy-5'-methoxyacetophenone (5.0 g, 30.09 mmol), the title compound (5.0 g, 79%) was given as a colorless liquid in substantially the same manner as in Reference Example 1.
¹H-NMR (300MHz, CDCl₃) δ: 2.64 (3H, s), 3.51 (3H, s), 3.80 (3H, s), 5.21 (2H, s), 7.00 (1H, dd, J = 9.0, 3.3 Hz), 7.14 (1H, d, J = 9.0 Hz), 7.25 (1H, d, J = 3.3 Hz).

### Reference Example 97

### 2-amino-6-[5-methoxy-2-(methoxymethoxy)phenyl]-4-(3-nitrophenyl)nicotinonitrile

A solution of the compound of Reference Example 96 (4.9 g, 23.35 mmol), 3-nitrobenzaldehyde (3.53 g, 23.35 mmol), malononitrile(1.54 g, 23.35 mmol) and ammonium acetate (2.70 g, 35.02 mmol) in toluene (50 mL) was refluxed while heating for 19 hours. After cooling, the precipitate was collected by filtration, washed with ethanol and then with diethylether, and then dried under reduced pressure to give the title compound (3.72 g, 39%) as a yellow powder.
¹H-NMR (300MHz, DMSO-d₆) δ: 3.31 (3H, s), 3.77 (3H, s), 5.14 (2H, s), 7.02 (1H, d, J = 9.0, 3.3 Hz), 7.11 (2H, br s), 7.17 (1H, d, J = 9.0 Hz), 7.29 (1H, s), 7.33 (1H, d, J = 3.3 Hz), 7.87 (1H, t, J = 8.1 Hz), 8.12 (1H, d, J = 7.8 Hz), 8.39 (1H, dd, J = 7.8, 1.8 Hz), 8.44 (1H,d,J=1.8Hz).

### Reference Example 98

### N-[3-cyano-6-[5-methoxy-2-(methoxymethoxy)phenyl]-4-(3-nitrophenyl)pyridin-2-yl] thiophene-2-carboxamide

From the compound of Reference Example 97 (500 mg, 1.23 mmol), the title compound (150 mg, 24%) was given as a yellow amorphous solid in substantially the same manner as in Reference Example 22.
¹H-NMR (300MHz, CDCl₃) δ: 3.47 (3H, s), 3.86 (3H, s), 5.20 (2H, s), 7.00 (1H, dd, J = 9.0, 3.3 Hz), 7.19-7.26 (3H, m), 7.47 (1H, d, J = 3.3 Hz), 7.67 (1H, d, J = 4.5 Hz), 7.45-7.83 (2H, m), 8.00 (1H, s), 8.14 (1H, d, J = 7.8 Hz), 8.40 (1H, d, J = 7.8 Hz), 8.53 (1H, t, J = 2.1 Hz).

### Reference Example 99

### N-{4-(3-aminophenyl)-3-cyano-6-[5-methoxy-2-(methoxymethoxy)phenyl]pyridin-2-yl} thiophene-2-carboxamide

From the compound of Reference Example 98 (150 mg, 0.29 mmol), the title compound (140 mg, 99%) was given as a yellow amorphous solid in substantially the same manner as in Reference Example 4.
¹H-NMR (300MHz, CDCl₃) δ: 3.42 (3H, s), 3.85 (3H, s), 5.16 (2H, s), 6.84 (1H, d, J = 7.8 Hz), 6.97 (1H, dd, J = 9.0, 3.3 Hz), 7.03-7.06 (2H, m), 7.16-7.34 (4H, m), 7.40 (1H, br s), 7.63 (1H, d, J = 4.8 Hz), 7.80 (1H, d, J = 3.6 Hz), 7.85-8.00 (1H, br).

### Reference Example 100

### tert-butyl {3-[(3-{3-cyano-6-[5-methoxy-2-(methoxymethoxy)phenyl]-2-[(2-thienylcarbonyl)amino]pyridin-4-yl}phenyl)arnko]-3-oxopropyl} carbamate

From the compound of Reference Example 99 (140 mg, 0.29 mmol) and Boc-β-alanine (117 mg, 0.62 mmol), the title compound (128 mg, 63%) was given as a yellow crystal in substantially the same manner as in Reference Example 38.
¹H-NMR (300MHz, CDCl₃) δ: 1.43 (9H, s), 2.62 (2H, t, J = 5.7 Hz), 3.43 (3H, s), 3.49 (2H, q, J = 5.9 Hz), 3.84 (3H, s), 5.12 (2H, s), 6.97 (1H, dd, J = 9.0, 3.3 Hz), 7.15-7.22 (2H, m), 7.43-7.50 (3H, m), 7.62-7.68 (2H, m), 7.81 (1H, d, J = 3.3 Hz), 7.95 (2H, br s), 7.98-8.10 (1H, br), 8.45-8.64 (1H, br).
Melting point: 167-168°C

### Reference Example 101

### 4'-bromo-2'-hydroxyacetophenone

Aluminum chloride (5.78 g, 43.35 mmol) was added to a solution of 3-bromophenol (5.0 g, 28.90 mmol), and then acetyl chloride (2.27 g, 28.90 mmol) in 1,2-dichloroethane (25 mL) was added. The resulting mixture was refluxed for 2 hours. After cooling, the reaction mixture was added to 1N hydrochloric acid (100 mL), and extracted with dichloromethane. The extract was washed with saturated brine, dried over magnesium sulfate, and then concentrated under reduced pressure. The residue was purified by silica gel chromatography (eluting solution: ethyl acetate/hexane = 1/10) to give the title compound (2.2 g, 35%) as a colorless liquid.
¹H-NMR (300MHz, CDCl₃) δ: 2.61 (3H, s), 7.04 (1H, dd, J = 8.4, 1.8 Hz), 7.18 (1H, d, J = 1.8 Hz), 7.59 (1H, d, J = 8.4 Hz), 12.30 (1H, s).

### Reference Example 102

### 4'-bromo-2'-(methoxymethoxy)acetophenone

From the compound of Reference Example 101 (2.18 g, 10.13 mmol), the title compound (2.07 g, 79%) was given as a colorless liquid in substantially the same manner as in Reference Example 1.
¹H-NMR (300MHz, CDCl₃) δ: 2.61 (3H, s), 3.52 (3H, s), 5.28 (2H, s), 7.19 (1H, dd, J = 8.4, 1.8 Hz), 7.37 (1H, d, J = 1.8 Hz), 7.60 (1H, d, J = 8.4 Hz).

### Reference Example 103

### 2-amino-6-[4-bromo-2-(methoxymethoxy)phenyl]-4-(3-nitrophenyl)nicotinonitrile

From the compound of Reference Example 102 (2.0 g, 7.73 mmol), the title compound (960 mg, 27%) was given as a yellow crystal in substantially the same manner as in Reference Example 2.
¹H-NMR (300MHz, CDCl₃) δ: 3.48 (3H, s), 5.23 (2H, s), 5.40 (2H, br s), 7.29 (1H, dd, J = 8.4, 1.8 Hz), 7.40 (2H, d, J = 7.5 Hz), 7.73 (2H, t, J = 8.1 Hz), 8.00 (1H, dt, J = 7.5, 1.2 Hz), 8.38 (1H, dt, J = 8.1, 1.2 Hz), 8.46 (1H, t, J = 1.8 Hz).

Melting point: 201-202°C

### Reference Example 104

### N-{6-[4-bromo-2-(methoxymethoxy)phenyl]-3-cyano-4-(3-nitrophenyl)pyridin-2-yl} thiophene-2-carboxamide

From the compound of Reference Example 103 (150 mg, 0.33 mmol), the title compound (162 mg, 87%) was given as a yellow crystal in substantially the same manner as in Reference Example 22.
¹H-NMR (300MHz, CDCl₃) δ: 3.51 (3H, s), 5.29 (2H, s), 7.20 (1H, t, J = 4.4 Hz), 7.32 (1H, dd, J = 8.1, 1.5 Hz), 7.45 (1H, s), 7.67 (1H, d, J = 4.4 Hz), 7.75-7.87 (3H, m), 7.98 (1H, s), 8.13 (1H, d, J = 7.5 Hz), 8.41 (1H, dd, J = 8.1, 1.5 Hz), 8.47 (1H, s), 8.51 (1H, t, J = 1.5 Hz).
Melting point: 166-167°C

### Reference Example 105

### tert-butyl {3-[(3-{6-[4-bromo-2-(methoxymethoxy)phenyl]-3-cyano-2-[(2-thienylcarbonyl)amino]pyridin-4-yl}phenyl)amino]-3-oxopropyl}carbamate

A solution of the compound of Reference Example 104 (150 mg, 0.27 mmol), iron powder (88 mg, 1.59 mmol) and ammonium chloride (92 mg, 1.72 mmol) in ethanol (5 mL)-water (5 mL) was refluxed while heating for 2 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give N-{4-(3-aminophenyl)-6-[4-bromo-2-(methoxymethoxy)phenyl]-3-cyanopyridin-2-yl} thiophene-2-carboxamide (140 mg) as a brown amorphous solid.
WSCD (100 mg, 0.52 mmol) was added to a solution of the resultant N-{4-(3-aminophenyl)-6-[4-bromo-2-(methoxymethoxy)phenyl]-3-cyanopyridin-2-yl} thiophene-2-carboxamide (140 mg), Boc-β-alanine (99 mg, 0.52 mmol) and HOBt (71 mg, 0.52 mmol) in DMF (10 mL) with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1), and recrystallized from diethylether to give the title compound (44 mg, 24%) as a yellow crystal.
¹H-NMR (300MHz, CDCl₃) δ: 1.44 (9H, t, J = 7.2 Hz), 2.64 (2H, t, J = 6.0 Hz), 3.45-3.54 (5H, m), 5.10-5.18 (1H, br), 5.30 (2H, s), 7.18 (1H, t, J = 4.4 Hz), 7.26-7.32 (2H, m), 7.45-7.58 (3H, m), 7.61-7.66 (2H, m), 7.79-7.81 (2H, m), 7.91-8.02 (2H, m), 8.40-8.50 (1H, br).
Melting point: 144-145°C

### Reference Example 106

### tert-butyl 2-(benzoylamino)-6-[2-(benzyloxy)phenyl]-4-(3-nitrophenyl)nicotinate

A solution of 2'-(benzyloxy)acetophenone (10.0 g, 44.20 mmol) described in WO 02/44153, tert-butyl cyanoacetate (6.24 g, 44.20 mmol), 3-nitrobenzaldehyde (6.68 g, 44.20 mmol) and ammonium acetate (5.11 g, 66.31 mmol) in toluene (100 mL) was refluxed while heating for 3 hours. After cooling, saturated aqueous sodium hydrogencarbonate was added to the reaction mixture, and extracted twice with ethyl acetate. The organic layers were combined, washed with saturated brine, dried over magnesium sulfate, and then concentrated under reduced pressure. The residue was purified by silica gel chromatography (eluting solution: ethyl acetate/hexane = 1/3) to give tert-butyl 2-amino-6-[2-(benzyloxy)phenyl]-4-(3-nitrophenyl)nicotinate (10.65 g) as a yellow oily substance.

Benzoyl chloride (7.51 g, 53.51 mmol) was added to a solution of the resultant tert-butyl 2-amino-6-[2-(benzyloxy)phenyl]-4-(3-nitrophenyl)nicotinate (10.65 g) in pyridin(100 mL), and stirred at room temperature for 20 hours. The reaction mixture was diluted with water, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/3)to give a mixture (8.11 g) of tert-butyl 2-(benzoylamino)-6-[2-(benzyloxy)phenyl]-4-(3-nitrophenyl)nicotinate and tert-butyl 6-[2-(benzyloxy)phenyl]-2-(dibenzoylamino)-4-(3-nitrophenyl)nicotinate as a yellow solid.

The resultant mixture (8.11 g) was dissolved in a THF (60 mL)-ethanol (40 mL) mixed solvent. A 4N aqueous sodium hydroxide solution (25 mL) of was added, and stirred at room temperature for 12 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/2) to give the title compound (3.06 g, 32%) as a yellow amorphous solid.
¹H-NMR (300MHz, CDCl₃) δ: 1.15 (9H, s), 5.09 (2H, s), 7.07 (1H, d, J = 8.1 Hz), 7.16 (1H, t, J = 7.8 Hz), 7.18-7.31 (5H, m), 7.39-7.44 (2H, m), 7.49-7.59 (4H, m), 7.80 (1H, s), 8.02 (2H, d, J = 8.4 Hz), 8.08 (1H, d, J = 1.8 Hz), 8.20-8.26 (2H, m), 10.30 (1H, s).

### Reference Example 107

### N-[6-[4-chloro-2-(methoxymethoxy)phenyl]-3-cyano-4-(3-nitrophenyl)pyridin-2-yl]-2-furamide

2-furoyl chloride (2.38 g, 18.26 mmol) was added to a solution of the compound of Reference Example 75 (3.0 g, 7.30 mmol) in pyridin (50 mL), and stirred at room temperature for 24 hours. A 28% aqueous solution of ammonia (10 mL) was added to the resultant mixture and stirred at room temperature for 30 minutes. The reaction mixture was diluted with water, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1) to give the title compound (360 mg, 95%) as a yellow crystal.
¹H-NMR (300MHz, CDCl₃) δ: 3.50 (3H, s), 5.29 (2H, s), 6.63 (1H, q, J = 1.8 Hz), 7.17 (1H, dd, J = 8.4, 1.8 Hz), 7.29 (1H, d, J = 1.8 Hz), 7.42 (1H, d, J = 3.3 Hz), 7.61 (1H, t, J = 0.9 Hz), 7.78 (1H, t, J = 8.1 Hz), 7.99 (2H, d, J = 9.9 Hz), 8.13 (1H, d, J = 8.1 Hz), 8.41 (1H, dd, J = 8.1, 1.8 Hz), 8.52 (1H, t, J = 1.8 Hz), 8.84 (1H, s).
Melting point: 154-155°C

### Reference Example 108

### N-{4-(3-aminophenyl)-6-[4-chloro-2-(methoxymethoxy)phenyl]-3-cyanopyridin-2-yl}-2-furamide

From the compound of Reference Example 107 (3.30 g, 6.54 mmol), the title compound (3.35, 100%) was given as a yellow amorphous solid in substantially the same manner as in Reference Example 4.
¹H-NMR (300MHz, CDCl₃) δ: 3.46 (3H, s), 5.25 (2H, s), 6.60-6.62 (1H, m), 6.83 (1H, d, J = 8.4 Hz), 7.00-7.03 (3H, m), 7.15 (1H, dd, J = 8.4, 1.8 Hz), 7.26-7.39 (2H, m), 7.63 (1H, s), 7.90-7.95 (2H, m), 8.70-8.90 (1H, br).

### Reference Example 109

### tert-butyl [3-({3-[6-[4-chloro-2-(methoxymethoxy)phenyl]-3-cyano-2-(2-furoylamino) pyridin-4-yl]phenyl}amino)-3-oxopropyl]carbamate

From the compound of Reference Example 108 (400 mg, 0.84 mmol) and Boc-β-alanine(319 mg, 1.68 mmol), the title compound (368 mg, 68%) was given as a white crystal in substantially the same manner as in Reference Example 38.
¹H-NMR (300MHz, DMSO-d₆) δ: 1.37 (9H, s), 2.50-2.54 (2H, m), 3.23 (2H, q, J = 6.5 Hz), 3.37 (3H, s), 5.41 (2H, s), 6.76 (1H, q, J = 1.8 Hz), 6.80-6.90 (1H, br), 7.36-7.38 (2H, m), 7.51-7.57 (2H, m), 7.71 (1H, d, J = 8.4 Hz), 7.95 (1H, d, J = 8.7 Hz), 8.02 (2H, s), 8.11 (1H, s), 10.24 (1H, s), 11.29 (1H, br s).
Melting point: 225-226°C

### Reference Example 110

### tert-butyl {1-[({3-[6-[4-chloro-2-(methoxymethoxy)phenyl]-3-cyano-2-(2-furoylamino) pyridin-4-yl]phenyl}amino)carbonyl]cyclobutyl}carbamate

From the compound of Reference Example 108 (150 mg, 0.32 mmol) and N-Boc-1-aminocyclobutane carboxylic acid (135 mg, 0.63 mmol), the title compound (50 mg, 23%) was given as a white crystal in substantially the same manner as in Reference Example 38.
¹H-NMR (300MHz, DMSO-d₆) δ: 1.39 (9H, s), 1.72-2.00 (2H, m), 2.05-2.19 (2H, m), 2.50-2.61 (2H, m), 3.38 (3H, s), 5.42 (2H, s), 6.76 (1H, t, J = 1.5 Hz), 7.28 (1H, d, J = 8.4 Hz), 7.38-7.56 (5H, m), 7.75 (1H, br s), 7.96 (1H, d, J = 8.4 Hz), 8.03 (2H, d, J = 2.7 Hz), 8.17 (1H, s), 9.63 (1H, s), 10.30 (1H, s).
Melting point: 211-212°C

### Reference Example 111

### tert-butyl {1-[({3-[6-[4-chloro-2-(methoxymethoxy)phenyl]-3-cyano-2-(2-furoylamino) pyridin-4-yl]phenyl}amino)carbonyl]cyclopropyl}carbamate

From the compound of Reference Example 108 (150 mg, 0.32 mmol) and N-Boc-1-aminocyclopropane carboxylic acid (127 mg, 0.63 mmol), the title compound (131 mg, 62%) was given as a white crystal in substantially the same manner as in Reference Example 38.
¹H-NMR (300MHz, CDCl₃) δ: 1.10-1.15 (2H, m), 1.50 (9H, s), 1.64-1.69 (2H, m), 3.47 (3H, s), 5.15 (1H, br s), 5.30 (2H, s), 6.61 (1H, q, J = 1.8 Hz), 7.14 (1H, dd, J = 8.4, 1.8 Hz), 7.30 (1H, d, J = 1.8 Hz), 7.38 (1H, t, J = 1.8 Hz), 7.49 (3H, br s), 7.59 (1H, d, J = 0.9 Hz), 7.96 (1H, d, J = 8.7 Hz), 7.99 (1H, s), 8.05 (1H, s), 8.60-8.72 (1H, br), 8.79-8.85 (1H, br).
Melting point: 225-226°C

### Reference Example 112

### 2-hydroxy-N-methoxy-N-methyl-4-(trifluoromethyl)benzamide

WSCD (4.63 g, 24.26 mmol) was added to a solution of 4-(trifluoromethyl) salicylic acid (5.0 g, 24.26 mmol), N,O-dimethylhydroxylamine hydrochloride (2.37 g, 24.26 mmol), HOBt (3.28 g, 24.26 mmol) and diisopropylethylamine (3.14 g, 24.26 mmol) in DMF (50 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate = 1/3) to give the title compound (4.72 g, 78%) as a white crystal.
¹H-NMR (300MHz, CDCl₃) δ: 3.43 (3H, s), 3.65 (3H, s), 7.08 (1H, d, J = 8.4 Hz), 7.25 (1H, d, J = 5.4 Hz), 8.09 (1H, d, J = 8.4 Hz), 11.29 (1H, s).
Melting point: 69-70°C

### Reference Example 113

### N-methoxy-2-(methoxymethoxy)-N-methyl-4-(trifluoromethyl)benzamide

From the compound of Reference Example 112 (4.50 g, 18.06 mmol), the title compound (4.33 g, 82%) was given as a colorless liquid in substantially the same manner as in Reference Example 1.
¹H-NMR (300MHz, CDCl₃) δ: 3.37 (3H, br s), 3.49 (6H, s), 5.24 (2H, s), 7.31 (1H, d, J = 7.8 Hz), 7.40 (2H, d, J = 11.4 Hz).

### Reference Example 114

### 2'-(methoxymethoxy)-4'-(trifluoromethyl)acetophenone

A 3M diethylether solution of methyl magnesium bromide(5.42 mL, 16.26 mmol) was added to a solution of the compound of Reference Example 113 (4.33 g, 14.78 mmol) in THF (50 mL) with ice-cooling, and stirred for 2 hours. The reaction mixture was diluted with saturated ammonium chloride, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/5 to 1/1) to give the title compound (1.46 g, 40%) as a colorless oil.
¹H-NMR (300MHz, CDCl₃) δ: 2.66 (3H, s), 3.55 (3H, s), 5.34 (2H, s), 7.32 (1H, d, J = 7.8 Hz), 7.47 (1H, s), 7.78 (1H, d, J = 7.8 Hz).

### Reference Example 115

### 2-amino-6-[2-(methoxymethoxy)-4-(trifluoromethyl)phenyl]-4-(3-nitrophenyl) nicotinonitrile

From the compound of Reference Example 114 (1.46 g, 5.89 mmol), the title compound (800 mg, 31 %) was given as a yellow crystal in substantially the same manner as in Reference Example 2.
¹H-NMR (300MHz, CDCl₃) δ: 3.49 (3H, s), 5.28 (2H, s), 5.43 (2H, br s), 7.40 (2H, d, J = 9.0 Hz), 7.49 (1H, s), 7.74 (1H, t, J = 8.1 Hz), 7.91 (1H, d, J = 8.1 Hz), 8.02 (1H, dt, J = 7.8, 1.2 Hz), 8.38 (1H, dq, J = 8.4, 1.2 Hz), 8.47 (1H, t, J = 2.1 Hz). Melting point: 144-145°C

### Reference Example 116

### N-[3-cyano-6-[2-(methoxymethoxy)-4-(trifluoromethyl)phenyl]-4-(3-nitrophenyl) pyridin-2-yl]thiophene-2-carboxamide

From the compound of Reference Example 115 (300 mg, 0.68 mmol), the title compound (290 mg, 77%) was given as a white crystal in substantially the same manner as in Reference Example 22.
¹H-NMR (300MHz, CDCl₃) δ: 3.51 (3H, s), 5.33 (2H, s), 7.21 (1H, t, J = 4.5 Hz), 7.43 (1H, d, J = 8.4 Hz), 7.53 (1H, s), 7.68 (1H, d, J = 4.8 Hz), 7.76-7.83 (2H, m), 7.98 (1H, s), 8.05 (1H, d, J = 8.1 Hz), 8.15 (1H, d, J = 7.8 Hz), 8.42 (1H, dd, J = 7.2, 1.8 Hz), 8.44-8.52 (2H, m).
Melting point: 139-140°C

### Reference Example 117

### N-{4-(3-aminophenyl)-3-cyano-6-[2-(methoxymethoxy)-4-(trifluoromethyl)phenyl] pyridin-2-yl}thiophene-2-carboxamide

From the compound of Reference Example 116 (250 mg, 0.45 mmol), the title compound (182 mg, 77%) was given as a yellow amorphous solid in substantially the same manner as in Reference Example 4.
¹H-NMR (300MHz, CDCl₃) δ: 3.46 (3H, s), 5.29 (2H, s), 6.84 (1H, d, J = 9.0 Hz), 7.01 (2H, br s), 7.18 (1H, t, J = 4.4 Hz), 7.29-7.42 (2H, m), 7.50 (1H, s), 7.64 (1H, d, J = 4.4 Hz), 7.79 (1H, d, J = 3.9 Hz), 7.88 (1H, s), 8.00 (1H, d, J = 8.1 Hz), 8.45 (1H, br s).

### Reference Example 118

### tert-butyl {3-[(3-{3-cyano-6-[2-(methoxymethoxy)-4-(trifluoromethyl)phenyl]-2-[(2-thienylcarbonyl)amino]pyridin-4-yl}phenyl)amino]-3-oxopropyl}carbamate

From the compound of Reference Example 117 (150 mg, 0.29 mmol) and Boc-β-alanine(108 mg, 0.57 mmol), the title compound (128 mg, 63%) was givenas a yellow crystal in substantially the same manner as in Reference Example 38.
¹H-NMR (300MHz, DMSO-d₆) δ: 1.37 (9H, s), 2.50-2.55 (2H, m), 3.24 (2H, q, J = 6.6 Hz), 3.38 (3H, s), 5.46 (2H, s), 6.88 (1H, t, J = 5.7 Hz), 7.28 (1H, q, J = 0.9 Hz), 7.39 (1H, d, J = 8.1 Hz), 7.52-7.61 (3H, m), 7.73 (1H, d, J = 8.7 Hz), 7.99 (1H, t, J = 2.4 Hz), 8.06-8.17 (4H, m), 10.25 (1H, s), 11.50 (1H, s).
Melting point: 186-187°C

### Reference Example 119

### Methyl 3-{2-amino-6-[4-chloro-2-(methoxymethoxy)phenyl]-3-cyanopyridin-4-yl} benzoate

From the compound of Reference Example 74 (19.57 g, 91.37 mmol), the title compound (14.16 g, 37%) was given as a yellow crystal in substantially the same manner as in Reference Example 5.
¹H-NMR (300MHz, CDCl₃) δ: 3.46 (3H, s), 3.95 (3H, s), 5.22 (2H, s), 5.36 (2H, br s), 7.12 (1H, dd, J = 8.1, 2.1 Hz), 7.24-7.26 (1H, m), 7.35 (1H, s), 7.61 (1H, t, J = 7.8 Hz), 7.76 (1H, d, J = 8.4 Hz), 7.86 (1H, d, J = 7.8 Hz), 8.17 (1H, dd, J = 7.8, 1.5 Hz), 8.27 (1H, t, J=1.8 Hz).
Melting point: 159-160°C

### Reference Example 120

### 3- {6-[4-chloro-2-(methoxymethoxy)phenyl]-3-cyano-2-(2-furoylamino)pyridin-4-yl} benzoic acid

2-furoyl chloride (1.54 g, 11.80 mmol) was added to a solution of the compound of Reference Example 119 (2.0 g, 4.72 mmol) in pyridin(30 mL), and stirred at room temperature for 44 hours. The reaction mixture was diluted with water, and extracted with ethyl acetate. The extract was washed with saturated brine, dried over magnesium sulfate, and then concentrated under reduced pressure. The residue was dissolved in a THF (15 mL)-methanol (8 mL) mixed solvent, an aqueous solution of 4N sodium hydroxide (5 mL) was added, and stirred at room temperature for 16 hours. The reaction mixture diluted with water and neutralized with 1N hydrochloric acid, and then extracted with ethyl acetate. The extract was washed with saturated brine, dried over magnesium sulfate, and then concentrated under reduced pressure. The residue was recrystallized from ethyl acetate to give the title compound (1.50 g, 63%) as a pink crystal.
¹H-NMR (300MHz, DMSO-d₆) δ: 3.77 (3H, s), 5.37 (2H, s), 6.77 (1H, dd, J = 3.6, 1.5 Hz), 7.28 (1H, dd, J = 8.4, 1.8 Hz), 7.37 (1H, d, J = 1.8 Hz), 7.55 (1H, d, J = 3.6 Hz), 7.77 (1H, t, J = 7.8 Hz), 7.93-8.09 (4H, m), 8.15 (1H, d, J = 7.8 Hz), 8.27 (1H, s), 11.33 (1H, s), 13.29 (1H, s).
Melting point: 214-216°C

### Reference Example 121

### 3-{6-[4-chloro-2-(methoxymethoxy)phenyl]-3-cyano-2-[(2-methoxybenzoyl)amino] pyridin-4-yl}benzoic acid

From the compound of Reference Example 119 (2.0 g, 4.72 mmol) and 2-methoxybenzoyl chloride (5.40 g, 31.65 mmol), the title compound (1.27 g, 50%) was given as an orange crystal in substantially the same manner as in Reference Example 121.
¹H-NMR (300MHz, DMSO-d₆) δ: 3.37 (3H, s), 3.94 (3H, s), 5.37 (2H, s), 7.15 (1H, t, J = 7.5 Hz), 7.22-7.27 (2H, m), 7.35 (1H, d, J = 1.8 Hz), 7.60 (1H, t, J = 8.4 Hz), 7.75-7.90 (3H, m), 7.98 (1H, d, J = 7.8 Hz), 8.06 (1H, s), 8.15 (1H, d, J = 7.8 Hz), 8.27 (1H, s), 10.92 (1H, s), 13.20-13.40 (1H, br).
Melting point: 213-215°C

### Reference Example 122

### tert-butyl [2-({3-[6-[4-chloro-2-(methoxymethoxy)phenyl]-3-cyano-2-(2-furoylamino) pyridin-4-yl]benzoyl}amino)ethyl]carbamate

From the compound of Reference Example 120 (300 mg, 0.60 mmol), the title compound (347 mg, 90%) was givenas a white crystal in substantially the same manner as in Reference Example 15.
¹H-NMR (300MHz, DMSO-d₆) δ: 1.38 (9H, s), 3.42-3.47 (5H, m), 3.57-3.62 (2H, m), 4.92-5.05 (1H, br), 5.27 (2H, s), 6.62 (1H, q, J = 1.8 Hz), 7.15 (1H, dd, J = 8.4, 1.8 Hz), 7.26-7.28 (1H, m), 7.40 (2H, dd, J = 3.6, 0.9 Hz), 7.60 (1H, t, J = 0.9 Hz), 7.64 (1H, d, J = 7.8 Hz), 7.88 (1H, d, J = 8.1 Hz), 7.94-7.97 (3H, m), 8.13 (1H, s), 8.82 (1H, br s). Melting point: 194-195°C

### Reference Example 123

### N-{6-[4-chloro-2-(methoxymethoxy)phenyl]-3-cyano-4-[3-({[3-(1H-imidazole-1-yl) propyl]amino}carbonyl)phenyl]pyridin-2-yl}-2-furamide

From the compound of Reference Example 120 (300 mg, 0.60 mmol) and 1-(3-aminopropyl)imidazole (89 mg, 0.71 mmol), the title compound (279 mg, 76%) was givenas a light green crystal in substantially the same manner as in Reference Example 15.
¹H-NMR (300MHz, DMSO-d₆) δ: 2.10-2.20 (2H, m), 3.45-3.54 (5H, m), 4.08 (2H, t, J = 7.1 Hz), 5.26 (2H, s), 6.61-6.65 (2H, m), 7.00 (1H, s), 7.06 (1H, s), 7.15 (1H, dd, J = 8.4, 1.8 Hz), 7.26-7.28 (1H, m), 7.40 (1H, d, J = 3.3 Hz), 7.58-7.65 (3H, m), 7.80 (1H, d, J = 7.8 Hz), 7.91-7.98 (3H, m), 8.10 (1H, s), 8.85 (1H, br s).
Melting point: 121-122°C

### Reference Example 124

### tert-butyl 2-[({3-[6-[4-chloro-2-(methoxymethoxy)phenyl]-3-cyano-2-(2-furoylamino) pyridin-4-yl]benzoyl} amino)methyl]piperidine-1-carboxylate

From the compound of Reference Example 120 (300 mg, 0.60 mmol) and 2-aminomethyl-1-N-Boc-piperidine (153 mg, 0.71 mmol), the title compound (178 mg, 42%) was givenas a light green crystal in substantially the same manner as in Reference Example 15.
¹H-NMR (300MHz, CDCl₃) δ:1.33 (9H, s), 1.58-1.80 (8H, m), 3.10-3.40 (2H, br), 3.45 (3H, s), 3.80-3.95 (1H, br), 4.90-5.00 (1H, br), 5.25 (2H, s), 6.62 (1H, q, J = 1.8 Hz), 7.15 (1H, dd, J = 8.4, 2.1 Hz), 7.29 (1H, d, J = 2.1 Hz), 7.39 (1H, dd, J = 3.3, 0.9 Hz), 7.57-7.67 (5H, m), 7.91-7.96 (2H, m), 8.81 (1H, s).
Melting point: 133-136°C

### Reference Example 125

### tert-butyl [3-({3-[6-[4-chloro-2-(methoxymethoxy)phenyl]-3-cyano-2-(2-furoylamino) pyridin-4-yl]benzoyl}amino)-2,2-dimethylpropyl]carbamate

From the compound of Reference Example 120 (300 mg, 0.60 mmol) and tert-butyl (3-amino-2,2-dimethylpropyl)carbamate (145 mg, 0.71 mmol), the title compound (292 mg, 71 %) was givenas a light green crystal in substantially the same manner as in Reference Example 15.
¹H-NMR (300MHz, CDCl₃) δ: 0.93 (6H, s), 1.37 (9H, s), 2.97 (2H, d, J = 7.2 Hz), 3.27 (2H, d, J = 6.6 Hz), 3.46 (3H, s), 5.07 (1H, t, J = 6.6 Hz), 5.26 (2H, s), 6.62 (1H, q, J = 1.8 Hz), 7.15 (1H, dd, J = 8.4, 1.8 Hz), 7.26-7.28 (1H, m), 7.39 (1H, t, J = 1.8 Hz), 7.59 (1H, t, J = 0.9 Hz), 7.66 (1H, t, J = 7.8 Hz), 7.91-8.00 (4H, m), 8.07 (1H, d, J = 7.5 Hz), 8.21 (1H, s), 8.82 (1H, s).
Melting point: 129-130°C

### Reference Example 126

### tert-butyl {2-[(3-{6-[4-chloro-2-(methoxymethoxy)phenyl]-3-cyano-2-[(2-methoxybenzoyl)amino]pyridin-4-yl}benzoyl)amino]ethyl}carbamate

From the compound of Reference Example 121 (300 mg, 0.55 mmol), the title compound (251 mg, 67%) was givenas a white crystal in substantially the same manner as in Reference Example 15.
¹H-NMR (300MHz, CDCl₃) δ:1.40 (9H, s), 3.44-3.48 (5H, m), 3.59-3.64 (2H, m), 4.15 (3H, s), 5.03 (1H, br s), 5.28 (2H, s), 7.10 (1H, d, J = 8.4 Hz), 7.18 (2H, t, J = 8.1 Hz), 7.28 (1H, s), 7.42 (1H, br s), 7.55-7.67 (2H, m), 7.88 (1H, d, J = 7.8 Hz), 7.95-8.00 (2H, m), 8.10-8.15 (2H, m), 8.40 (1H, d, J = 7.8 Hz), 10.90 (1H, s).
Melting point: 178-180°C

### Reference Example 127

### N-{(6-[4-chloro-2-(methoxymethoxy)phenyl]-3-cyano-4-[3-({[3-(1H-imidazole-1-yl) propyl] amino }carbonyl)phenyl]pyridin-2-yl}-2-methoxybenzamide

From the compound of Reference Example 121 (300 mg, 0.55 mmol) and 1-(3-aminopropyl)imidazole (83 mg, 0.66 mmol), the title compound (199 mg, 56%) was givenas a white crystal in substantially the same manner as in Reference Example 15.
¹H-NMR (300MHz, CDCl₃) δ: 2.12-2.18 (2H, m), 3.45-3.54 (5H, m), 4.06-4.15 (5H, m), 5.24 (2H, s), 6.60 (1H, t, J = 5.7 Hz), 7.06 (1H, s), 7.09-7.18 (4H, m), 7.24 (1H, d, J = 1.8 Hz), 7.53-7.65 (3H, m), 7.80 (1H, dd, J = 6.6, 1.5 Hz), 7.91-7.94 (2H, m), 8.08 (1H, d, J = 10.2 Hz), 8.10 (1H, s), 8.35 (1H, dd, J = 7.8, 1.8 Hz), 10.79 (1H, s).
Melting point: 100-101°C

### Reference Example 128

### tert-butyl 2-{[(3-{6-[4-chloro-2-(methoxymethoxy)phenyl]-3-cyano-2-[(2-methoxybenzoyl)amino]pyridin-4-yl}benzoyl)amino]methyl}piperidine-1-carboxylate

From the compound of Reference Example 121 (300 mg, 0.55 mmol) and 2-aminomethyl-1-N-Boc-piperidine (142 mg, 0.66 mmol), the title compound (244 mg, 60%) was givenas a light green amorphous solid in substantially the same manner as in Reference Example 15.
¹H-NMR (300MHz, CDCl₃) δ: 1.38 (9H, s), 1.60-1.85 (6H, m), 2.90-3.40 (3H, br), 3.45 (3H, s), 3.50-3.70 (1H, br), 3.80-3.95 (1H, br), 4.13 (3H, s), 4.97 (1H, br s), 5.24 (2H, s), 7.09 (1H, d, J = 8.4 Hz), 7.14-7.20 (2H, m), 7.26-7.28 (1H, m), 7.53-7.65 (5H, m), 7.86 (1H, s), 8.09 (1H, d, J = 8.4 Hz), 8.37 (1H, dd, J = 7.8, 1.8 Hz), 10. 86 (1H, s).

### Reference Example 129

### 2-amino-4-(3-aminophenyl)-6-[4-chloro-2-(methoxymethoxy)phenyl]nicotinonitrile

From the compound of Reference Example 75 (2.0 g, 4.87 mmol), the title compound (1.76, 95%) was givenas a brown amorphous solid in substantially the same manner as in Reference Example 4.
¹H-NMR (300MHz, CDCl₃) δ: 3.47 (3H, s), 3.60-4.10 (2H, br), 5.20 (2H, s), 5.31 (2H, br s), 6.79 (1H, dd, J = 7.8, 2.1 Hz), 6.91 (1H, t, J = 2.1 Hz), 6.97 (1H, d, J = 7.5 Hz), 7.11 (1H, dd, J = 8.4, 2.1 Hz), 7.24-7.31 (3H, m), 7.72 (1H, d, J = 8.1 Hz).

### Reference Example 130

### tertbutyl {3-[(3-{2-amino-6-[4-chloro-2-(methoxymethoxy)phenyl]-3-cyanopyridin-4-yl} phenyl)amino]-3-oxopropyl}carbamate

WSCD (1.70 g, 8.93 mmol) was added to a solution of the compound of Reference Example 129 (1.70 g, 4.46 mmol), Boc-β-alanine (1.69 g, 8.93 mmol) and HOBt (1.21 g, 8.93 mmol) in DMF (50 mL) with ice-cooling, and stirred at room temperature for 40 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate = 1/1), and then recrystallized from diethylether-diisopropylether to give the title compound (1.60 g, 65%) as a white crystal.
¹H-NMR (300MHz, CDCl₃) δ: 1.43 (9H, s), 2.63 (2H, t, J = 6.0 Hz), 3.45-3.54 (5H, m), 5.08-5.18 (1H, br), 5.23 (2H, s), 5.32 (2H, br s), 7.11 (1H, dd, J = 8.4, 2.1 Hz), 7.25 (1H, s), 7.35 (1H, s), 7.37 (1H, d, J = 10.2 Hz), 7.46 (1H, t, J = 7.8 Hz), 7.56 (1H, d, J = 8.4 Hz), 7.75 (1H, d, J = 8.4 Hz), 7.96 (2H, br s).
Melting point: 141-142°C

### Reference Example 131

### tert-butyl 2-(benzoylamino)-4-[3-({N-[(benzyloxy)carbonyl]-β-alanyl}amino) phenyl]-6-[2- (benzyloxy)phenyl]nicotinate

From the compound of Reference Example 106 (1.0 g, 1.66 mmol), tert-butyl 4-(3-aminophenyl)-2-(benzoylamino)-6-[2-(benzyloxy)phenyl]nicotinate (0.95 g, 100%) was given as a yellow amorphous solid in substantially the same manner as in Reference Example 4.

WSCD (634 mg, 3.33 mmol) was added to a solution of the resultant tert-butyl 4-(3-aminophenyl)-2-(benzoylamino)-6-[2-(benzyloxy)-phenyl]nicotinate (0.95 g, 1.66 mmol), Cbz-β-alanine (742 mg, 3.33 mmol), and HOBt (449 mg, 3.33 mmol) in DMF (10 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1) to give the title compound (1.04 g, 81 %) as a white amorphous solid.
¹H-NMR (300MHz, CDCl₃) δ: 1.16 (9H, s), 2.62 (2H, br s), 3.52-3.59 (2H, m), 5.09 (4H, s), 5.43 (1H, br s), 6.91 (1H, d, J = 7.5 Hz), 7.04 (1H, d, J = 8.4 Hz), 7.10-7.15 (2H, m), 7.21-7.41 (13H, m), 7.48-7.59 (3H, m), 7.75 (1H, d, J = 8.7 Hz), 7.81 (1H, s), 8.00 (2H, d, J = 8.4 Hz), 8.13 (1H, dd, J = 7.8, 1.8 Hz), 10.21 (1H, br s).

### Reference Example 132

### 4'-fluoro-2'-(methoxymethoxy)acetophenone

Sodium hydride (in oil: 60%, 7.1 g, 0.18 mol) was added to a solution of 4'-fluoro-2'-hydroxyacetophenone (26.0 g, 0.17 mol) in THF (300 mL) with ice-cooling, and stirred for 30 minutes. Chloromethyl methyl ether (14.3 g, 0.18 mol) was dropped to the resultant mixture with ice-cooling and stirred at room temperature for 16 hours. An aqueous solution of saturated ammonium chloride was added to the reaction mixture and concentrated under reduced pressure, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/6 to 1/1) to give the title compound (31.0g, 92%) as a colorless liquid.
¹H-NMR (300MHz, CDCl₃) δ: 2.62 (3H, s), 3.53 (3H, s), 5.28 (2H, s), 6.70-6.80 (1H, m), 6.93 (1H, dd, J = 10.8,2.4 Hz), 7.79 (1H, dd, J = 8.7, 6.9 Hz).

### Reference Example 133

### 2-amino-6-[4-fluoro-2-(methoxymethoxy)phenyl]-4-(3-nitrophenyl)nicotinonitrile

A mixed solution of the compound of Reference Example 132 (33.4 g, 168 mmol), 3-nitrobenzaldehyde (25.5 g, 168 mmol), malononitrile (11.1 g, 168 mmol) and ammonium acetate (19.5 g, 253 mmol) in toluene (200 mL) was refluxed while heating for 16 hours. After cooling, the precipitated crystals were collected by filtration and washed with cold methanol to give the title compound (22.5 g, 34%) as a light yellow crystal.
¹H-NMR (300MHz, CDCl₃) δ: 3.48 (3H, s), 5.23 (2H, s), 5.39 (2H, s), 6.80-6.90 (1H, m), 6.99 (1H, dd, J = 10.8, 2.4 Hz), 7.38 (1H, s), 7.73 (1H, t, J = 7.8 Hz), 7.84 (1H, dd, J = 8.7, 6.9 Hz), 7.96-8.05 (1H, m), 8.35-8.40 (1H, m), 8.45-8.50 (1H, m).
Melting point: 202-203°C

### Reference Example 134

### N-[3-cyano-6-[4-fluoro-2-(methoxymethoxy)phenyl]-4-(3-nitrophenyl)pyridin-2-yl]-2-furamide

2-furoyl chloride (3.75 g, 38.0 mmol) was added to a solution of the compound of Reference Example 133 (3.0 g, 7.61 mmol) in pyridin(40 mL) with ice-cooling, and stirred at room temperature for 16 hours. Saturated aqueous sodium hydrogencarbonate was added to the reaction mixture, and extracted with ethyl acetate. The extract was washed with saturated brine, dried over magnesium sulfate, and then concentrated under reduced pressure. Thus, a yellow amorphous solid was obtained. This solid was purified by amino silica gel chromatography (eluting solution: ethyl acetate/hexane = 1/4 to 2/1) to give the title compound (3.33 g, 90%) as a white powder. A part thereof is recrystallized from ethyl acetate-hexane to give the title compound (117 mg) as colorless plates.
¹H NMR (300 MHz, DMSO-d₆) δ: 3.37 (3H, s), 5.36 (2H, s), 6.77 (1H, dd, J = 3.4, 1.9 Hz), 7.01-7.10 (1H, m), 7.18 (1H, dd, J = 11.3, 2.3 Hz), 7.55 (1H, d, J = 3.8 Hz), 7.90-8.06 (3H, m), 8.11 (1H, s), 8.17-8.23 (1H, m, J = 8.3 Hz), 8.43-8.48 (1H, m), 8.55 (1H, t, J = 1.9 Hz), 11.37 (1H, br s).
Melting point: 177-179°C
Element analysis: Calculated (%) for C₂₅H₁₇N₄O₆F: C,61.48; H, 3.51; N, 11.47. Found (%): C, 61.58; H, 3.69; N, 11.43.

### Reference Example 135

### N-{4-(3-aminophenyl)-3-cyano-6-[4-fluoro-2-(methoxymethoxy)phenyl]pyridin-2-yl}-2-furamide

An solution of the compound of Reference Example 134 (3.20 g, 6.55 mmol), iron powder (2.19 g, 39.3 mmol) and ammonium chloride (2.28 g, 42.6 mmol) in ethanol (160 mL)-water (160 mL) was refluxed while heating for 1.5 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give a yellow amorphous solid. This solid was purified by amino silica gel chromatography (eluting solution: ethyl acetate/hexane = 1/4 to 2/1) to give the title compound (3.33 g, 90%) as a white powder. A part thereof is recrystallized from ethyl acetate-hexane to give the title compound (117 mg) as a colorless plate-like crystal.
¹H NMR (300 MHz, DMSO-d₆) δ: 3.36 (3H, s), 5.38 (2H, s), 5.45 (2H, s), 6.71-6.81 (3H, m), 6.85 (1H, d, J = 1.9 Hz), 6.99-7.09 (1H, m), 7.13-7.27 (2H, m), 7.52 (1H, d, J = 3.4 Hz), 7.92-7.99 (2H, m), 8.02 (1H, s), 11.09 (1H, br s).
Melting point: 204-206°C
Element analysis: Calculated (%) for C₂₅H₁₉N₄O₄F·H₂O: C,63.02; H, 4.44; N, 11.75. Found (%): C, 63.28; H, 4.07; N, 11.59.

### Reference Example 136

### tert-butyl [3-({3-[3-cyano-6-[4-fluoro-2-(methoxymethoxy)phenyl]-2-(2-furoylamino) pyridin-4-yl]phenyl}amino)-3-oxopropyl]carbamate

WSCD (150 mg, 0.785 mmol) was added to a solution of the compound of Reference Example 135 (300 mg, 0.654 mmol), Boc-β-alanine (149 mg, 0.785 mmol) and HOBt (106 mg, 0.785 mmol) in DMF (3 mL), and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/50 to 100/0), and then recrystallized from ethyl acetate/hexane to give the title compound (76.8 mg, 19%) as a colorless solid.
¹H NMR (300 MHz, DMSO-d₆) δ: 1.38 (9H, s), 2.48-2.55 (2H, m), 3.20-3.28 (2H, m), 3.37 (3H, s), 5.40 (2H, s), 6.77 (1H, dd, J = 3.4, 1.9 Hz), 6.88 (1H, t, J = 5.7 Hz), 7.01-7.10 (1H, m), 7.18 (1H, dd, J = 11.3, 2.6 Hz), 7.38 (1H, d, J = 7.5 Hz), 7.50-7.59 (2H, m), 7.71 (1H, d, J = 8.3 Hz), 7.95-8.06 (3H, m), 8.11 (1H, br s), 10.24 (1H, br s), 11.28 (1H, br s).
Melting point: 198-200°C
Element analysis: Calculated (%) for C₃₃H₃₂N₅O₇F: C,62.95; H, 5.12; N, 11.12. Found (%): C, 62.76; H, 5.10; N, 11.10.

### Reference Example 137

### Methyl 3-{2-amino-3-cyano-6-[4-fluoro-2-(methoxymethoxy)phenyl]pyridin-4-yl} benzoate

A solution of 4'-fluoro-2'-(methoxymethoxy)acetophenone (22.4 g, 113 mmol) of Reference Example 135, methyl 3-formylbenzoate (18.6 g, 113 mmol), malononitrile (7.47 g, 113 mmol) and ammonium acetate (13.1 g, 169 mmol) in toluene (140 mL) was refluxed while heating for 16 hours. After cooling, an aqueous solution of saturated ammonium chloride was added to the reaction mixture, and extracted with ethyl acetate. The extract was washed with water and then with saturated brine, dried over magnesium sulfate, and then concentrated under reduced pressure. The residue was recrystallized from ethyl acetate-hexane to give the title compound (10.1 g, 35%) as a yellow crystal.
¹H-NMR (300MHz, CDCl₃) δ: 3.46 (3H, s), 3.95 (3H, s), 5.22 (2H, s), 5.36 (2H, br s), 6.85 (1H, td, J = 8.4, 2.4 Hz), 6.98 (1H, dd, J = 10.8, 2.4 Hz), 7.34 (1H, s), 7.61 (1H, t, J = 7.8 Hz), 7.75-7.90 (2H, m), 8.10-8.20 (1H, m), 8.27 (1H, s).
Melting point: 155-158°C

### Reference Example 138

### Methyl 3 - {3-cyano-6-[4-fluoro-2-(methoxymethoxy)phenyl]-2-(2-furoylamino) pyridin-4-yl}benzoate

From the compound of Reference Example 137 (4.0 g, 9.82 mmol) and 2-furoyl chloride (3.20 g, 24.5 mmol), the title compound (3.13 g, 64%) as a white crystal in substantially the same manner as in Reference Example 6.
¹H-NMR (300MHz, CDCl₃) δ: 3.48 (3H, s), 3.97 (3H, s), 5.27 (2H, s), 6.60-6.65 (1H, m), 6.85-6.95 (1H, m), 7.01 (1H, dd, J = 10.8, 2.4 Hz), 7.05 (1H, d, J = 3.3 Hz), 7.60 (1H, s), 7.66 (1H, t, J = 7.5 Hz), 7.90-8.00 (2H, m), 8.02 (1H, dd, J = 8.7, 6.9 Hz), 8.18-8.25 (1H, m), 8.30-8.35 (1H, m), 8.83 (1H, s).
Melting point: 160-162°C

### Reference Example 139

### 3-[3-cyano-6-[4-fluoro-2-(methoxymethoxy)phenyl]-2-(2-furoylamino)pyridin-4-yl] benzoic acid

From the compound of Reference Example 138 (2.95 g, 5.88 mmol), the title compound (2.64 g, 92%) was givenas a light yellow crystal in substantially the same manner as in Reference Example 7.
¹H-NMR (300MHz, DMSO-d₆) δ: 3.38 (3H, s), 5.36 (2H, s), 6.77 (1H, dd, J = 3.3, 1.8 Hz), 7.05 (1H, td, J = 8.4, 2.4 Hz), 7.17 (1H, dd, J = 11.1, 2.4 Hz), 7.55 (1H, d, J =3.3 Hz), 7.78 (1H, t, J = 7.8 Hz), 7.95-8.05 (3H, m), 8.08 (1H, s), 8.12-8.20 (1H, m), 8.27 (1H, s), 11.35 (1H, s).
Melting point: 254°C (decomposes)

### Reference Example 140

### 3-{3-cyano-6-[4-fluoro-2-(methoxymethoxy)phenyl]-2-[(2-thienylcarbonyl)amino] pyridin-4-yl}benzoic acid

2-thenoyl chloride (5.24 mL, 49.0 mmol) was added to a solution of the compound of Reference Example 137 (4.00 g, 9.82 mmol) in pyridin (30 mL), and stirred at room temperature for 17 hours. The reaction mixture was concentrated under reduced pressure, diluted with ethyl acetate, washed with water and then with saturated brine, dried over magnesium sulfate, and then concentrated under reduced pressure.
The residue was dissolved in THF (20 mL), a 2M aqueous solution (20 mL) of sodium carbonate and methanol (15 mL) were added, and stirred at room temperature for 20 hours. The reaction mixture was diluted with water, and extracted with ethyl acetate. The extract was washed with saturated brine, dried over magnesium sulfate, and then concentrated under reduced pressure. The residue was recrystallized from methanol-diethylether to give a mixture of methyl 3-{3-cyano-6-[4-fluoro-2-(methoxymethoxy)phenyl]-2-[(2-thienylcarbonyl)amino] pyridin-4-yl}benzoate and methyl 3-{3-cyano-6-(4-fluoro-2-hydroxyphenyl)-2-[(2-thienylcarbonyl)amino]pyridin-4-yl} benzoate was obtained. A 4N aqueous sodium hydroxide solution (4.5 mL) was added to a solution of the resultant mixture in THF (45 mL)-ethanol (24 mL), and stirred at room temperature for 16 hours. The reaction mixture was neutralized with 1N hydrochloric acid, and extracted with ethyl acetate. The extract was washed with saturated brine, and dried over magnesium sulfate. Ethyl acetate was evaporated outto give a mixture of the title compound and

### 3- {3-cyano-6-(4-fluoro-2-hydroxyphenyl)-2-[(2-thienylcarbonyl)amino]pyridin-4-yl} benzoic acid as a white solid.

### Reference Example 141

### tert-butyl {2-[(3-{3-cyano-6-[4-fluoro-2-(methoxymethoxy)phenyl]-2-(2-furoylamino) pyridin-4-yl}benzoyl)amino]ethyl} carbamate

From the compound of Reference Example 139 (300 mg, 0.62 mmol) and tert-butyl N-(2-aminoethyl)carbamate (197 mg, 1.23 mmol), the title compound (310 mg, 80%) was given as a white crystal in substantially the same manner as in Reference Example 15.
¹H-NMR (300MHz, CDCl₃) δ: 1.38 (9H, s), 3.30-3.50 (2H, m), 3.47 (3H, s), 3.50-3.65 (2H, m), 5.04 (1H, br s), 5.27 (2H, s), 6.60-6.65 (1H, m), 6.80-6.95 (1H, m), 7.01 (1H, dd, J = 10.8, 2.4 Hz), 7.35-7.45 (2H, m), 7.60 (1H, s), 7.62 (1H, t, J = 7.8 Hz), 7.88 (1H, d, J = 7.8 Hz), 7.95-8.05 (3H, m), 8.13 (1H, s), 8.83 (1H, s).
Melting point: 172-173°C

### Reference Example 142

### N-{3-cyano-6-[4-fluoro-2-(methoxymethoxy)phenyl]-4-[3-({[3-(1H-imidazole-1-yl) propyl]amino}carbonyl)phenyl]pyridin-2-yl}-2-furamide

From the compound of Reference Example 139 (300 mg, 0.62 mmol) and 3-(1H-imidazole-1-yl)propane-1-amine (249 mg, 1.23 mmol), the title compound (368 mg, 100%) was given as a yellow liquid in substantially the same manner as in Reference Example 15.
¹H-NMR (300MHz, CDCl₃) δ: 2.10-2.30 (2H, m), 3.46 (3H, s), 3.40-3.60 (2H, m), 4.00-4.10 (2H, m), 5.26 (2H, s), 6.60-6.65 (1H, m), 6.65-6.75 (1H, m), 6.85-6.95 (1H, m), 6.95-7.10 (3H, m), 7.40 (1H, dd, J = 3.6, 0.6 Hz), 7.55-7.70 (3H, m), 7.75-7.85 (1H, m), 7.90-8.05 (3H, m), 8.10 (1H, s), 8.87 (1H, s).

### Reference Example 143

### tert-butyl 3-({3-[3-cyano-6-[4-fluoro-2-(methoxymethoxy)phenyl]-2-(2-furoylamino) pyridin-4-yl] benzoyl}amino)pyrrolidine-1-carboxylate

From the compound of Reference Example 139 (300 mg, 0.62 mmol) and tert-butyl 3-aminopyrrolidine-1-carboxylate (229 mg, 1.23 mmol), the title compound (351 mg, 87%) was given as a yellow liquid in substantially the same manner as in Reference Example 15.
¹H-NMR (300MHz, CDCl₃) δ: 1.44 (9H, s), 1.90-2.40 (2H, m), 3.20-3.55 (6H, m), 3.65-3.80 (1H, m), 4.60-4.70 (1H, m), 5.24 (2H, s), 6.55-6.65 (1H, m), 6.75-6.90 (1H, m), 6.90-7.00 (2H, m), 7.35 (1H, d, J = 3.6 Hz), 7.55-7.65 (2H, m), 7.80 (1H, d, J = 7.8 Hz), 7.90-8.05 (3H, m), 8.09 (1H, s), 8.88 (1H, br s).

### Reference Example 144

### tert-butyl [3-({3-[3-cyano-6-[4-fluoro-2-(methoxymethoxy)phenyl]-2-(2-furoylamino) pyridin-4-yl]benzoyl}amino)-2,2-dimethylpropyl]carboxylate

From the compound of Reference Example 139 (300 mg, 0.62 mmol) and tert-butyl (3-amino-2,2-dimethylpropyl)carboxylate (249 mg, 1.23 mmol), the title compound (344 mg, 83%) was given as a yellow liquid in substantially the same manner as in Reference Example 15.
¹H-NMR (300MHz, CDCl₃) δ: 0.92 (6H, s), 1.40 (9H, s), 2.96 (2H, d, J = 5.9 Hz), 3.26 (2H, d, J = 6.9 Hz), 3.45 (3H, s), 5.20-5.40 (3H, m), 6.55-6.65 (1H, m), 6.80-6.90 (1H, m), 6.95-7.05 (1H, m), 7.38 (1H, dd, J = 3.6, 0.6 Hz), 7.55-7.70 (2H, m), 7.85-8.10 (5H, m), 8.18 (1H, s), 8.92 (1H, br s).

### Reference Example 145

### 3-{2-(benzoylamino)-3-cyano-6-[2-(methoxymethoxy)phenyl]pyridin-4-yl}benzoic acid

The compound of Reference Example 5 (780 mg, 2.0 mmol) and benzoyl chloride (850 mg, 6.0 mmol) were dissolved in pyridin (8 mL), and stirred at room temperature for 20 hours. Ethyl acetate (30 mL) and water (20 mL) were added to the reaction mixture, extracted, and the separated organic layer was concentrated underreduced pressure. THF (5 mL), methanol 5 mL) and a 4N aqueous sodium hydroxide solution (5 mL) were added to the resultant residue, and stirred at room temperature for 20 hours. The resultant substance was neutralized with 1N hydrochloric acid (20 mL). The resultant precipitate was collected by filtration and washed with a small amount of water. The resultant substance was dried under reduced pressure to give the title compound (810 mg) was obtained. As a result of LC-MS analysis, the purity was 45% (net yield amount: 360 mg; yield: 38%). The resultant compound was subject to the next reaction for synthesize a library without further treatment.

### Reference Example 146 to Reference Example 158

Using substantially the same conditions as those in Reference Example 145 while varying the carboxylic chloride subjected to the condensation reaction, the compounds of Reference Example 146 to Reference Example 158 shown in Table 1 were obtained. Table 1 shows the gross yield amount, purity, and net yield amount.

**Table 1**

| | | | | | |
|---|---|---|---|---|---|
| 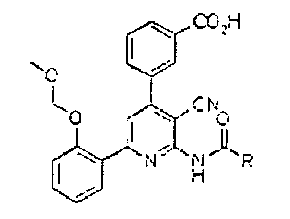 | | | | | |

| | R | Gross yield amount (mg) | Purity (%) | Net yield amount (mg) | Yield (%) |
|---|---|---|---|---|---|
| Reference Ex. 146 | 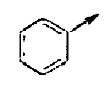 | 810 | 45 | 360 | 38 |
| Reference Ex. 147 | 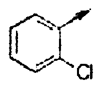 | 1210 | 70 | 850 | 82 |
| Reference Ex. 148 | 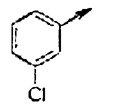 | 720 | 80 | 570 | 45 |
| Reference Ex.149 | 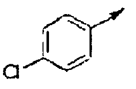 | 1660 | 50 | 830 | 53 |
| Reference Ex. 150 | 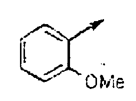 | 1140 | 65 | 740 | 73 |
| Reference Ex 151 | 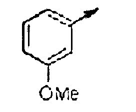 | 750 | 90 | 670 | 66 |
| Reference Ex 152 | 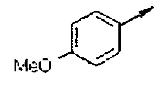 | 1380 | 63 | 870 | 85 |
| Reference Ex. 153 | 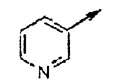 | 400 | 55 | 220 | 28 |
| Reference Ex. 154 | 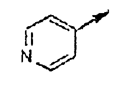 | 960 | 55 | 450 | 47 |
| Reference Ex. 155 | 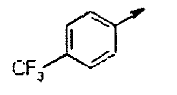 | 750 | 60 | 450 | 41 |
| Reference Ex. 156 | 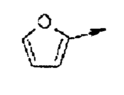 | 770 | 87 | 670 | 71 |
| Reference Ex. 157 | 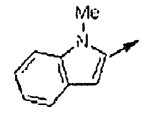 | 560 | 85 | 480 | 46 |
| Reference Ex. 158 | 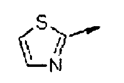 | 550 | 65 | 360 | 37 |

### Reference Example 159

### tert-butyl {3-[(3-{6-[4-chloro-2-(methoxymethoxy)phenyl]-3-cyano-2-[(3-methyl-2-furoyl)amino]pyridin-4-yl}phenyl)amino]-3-oxopropyl}carbamate

From the compound of Reference Example 130 (150 mg, 0.27 mmol) and 3-methyl-2-furoyl chloride (98 mg, 0.68 mmol), the title compound (78 mg, 44%) was given as a white crystal in substantially the same manner as in Reference Example 3.
¹H-NMR (300MHz, DMSO-d₆) δ: 1.44 (9H, s), 2.49 (3H, s), 2.64 (2H, t, J = 6.0 Hz), 3.44-3.53 (5H, m), 5.08-5.19 (1H, br), 5.29 (2H, s), 6.45 (1H, d, J = 1.5 Hz), 7.15 (1H, dd, J = 8.4, 1.8 Hz), 7.29 (1H, s), 7.45-7.50 (3H, m), 7.62 (1H, br s), 7.88 (1H, br s), 7.94-8.01 (3H, m), 9.79 (1H, s).
Melting point: 161-162°C

### Reference Example 160

### tert-butyl {3-[(3-{6-[4-chloro-2-(methoxymethoxy)phenyl]-3-cyano-2-[(2-methoxybenzoyl)amino]pyridin-4-yl}phenyl)amino]-3-oxopropyl}carbamate

From the compound of Reference Example 130 (150 mg, 0.27 mmol) and 2-anisoyl chloride (350 mg, 2.04 mmol), the title compound (80 mg, 43%) was given as a pale yellow crystal in substantially the same manner as in Reference Example 3.
¹H-NMR (300MHz, CDCl₃) δ: 1.44 (9H, s), 2.64 (2H, t, J = 5.7 Hz), 3.47-3.52 (5H, m), 4.13 (3H, s), 5.10-5.20 (1H, br), 5.29 (2H, s), 7.08 (1H, d, J = 8.4 Hz), 7.16 (2H, t, J = 8.0 Hz), 7.27 (1H, d, J = 1.5 Hz), 7.46-7.61 (4H, m), 7.92 (2H, s), 8.02 (1H, s), 8.09 (1H, d, J = 8.4 Hz), 8.37 (1H, dd, J = 7.8,1.5 Hz), 10.85 (1H, s).
Melting point: 172-173°C

### Reference Example 161

### tert-butyl [2-({3-[6-[4-chloro-2-(methoxymethoxy)phenyl]-3-cyano-2-(2-furoylamino) pyridin-4-yl]phenyl}amino)-1-methyl-2-oxoethyl]carbamate

From the compound of Reference Example 108 (300 mg, 0.63 mmol) and Boc-D-alanine (239 mg, 1.26 mmol), the title compound (250 mg, 61%) was given as a yellow crystal in substantially the same manner as in Reference Example 38.
¹H-NMR (300MHz, CDCl₃) δ: 1.45 (3H, d, J = 6.9 Hz), 1.48 (9H, s), 3.48 (3H, s), 4.30-4.39 (1H, m), 4.95 (1H, d, J = 6.0 Hz), 5.31 (2H, s), 6.61 (1H, dd, J = 3.6, 1.8 Hz), 7.14 (1H, dd, J = 8.4, 1.8 Hz), 7.30 (1H, d, J = 2.1 Hz), 7.39 (1H, dd, J = 3.6, 0.6 Hz), 7.45-7.53 (3H, m), 7.58-7.59 (1H, m), 7.96 (1H, d, J = 8.1 Hz), 7.99 (1H, s), 8.07 (1H, s), 8.09-8.18 (1H, br), 8.72(1H, s).
Melting point: 170-172°C

### Reference Example 162

### tert-butyl [3-({3-[6-[4-chloro-2-(methoxymethoxy)phenyl]-3-cyano-2-(2-furoylamino) pyridin-4-yl]phenyl}amino)-1-methyl-3-oxopropyl]carbamate

From the compound of Reference Example 108 (300 mg, 0.63 mmol) and 3-N-Boc-aminobutyric acid (257 mg, 1.26 mmol), the title compound (136 mg, 33%) was given as a white crystal in substantially the same manner as in Reference Example 38.
¹H-NMR (300MHz, DMSO-d₆) δ: 1.09 (3H, d, J = 6.6 Hz), 1.36 (9H, s), 2.37-2.44 (1H, m), 2.50-2.58 (1H, m), 3.39 (3H, s), 3.80-3.95 (1H, m), 5.41 (2H, s), 6.76 (1H, dd, J = 3.3, 1.5 Hz), 6.83 (1H, d, J = 7.8 Hz), 7.27 (1H, dd, J = 8.4, 1.5 Hz), 7.35-7.40 (2H, m), 7.55 (2H, t, J = 7.8 Hz), 7.69 (1H, d, J = 8.7 Hz), 7.95 (1H, d, J = 8.4 Hz), 8.04 (2H, s), 8.12 (1H, s), 10.26 (1H, s), 11.32 (1H, s).
Melting point: 184-185°C

### Reference Example 163

### N-(6-[4-chloro-2-(methoxymethoxy)phenyl]-3-cyano-4-{3-[(1H-imidazole-1-ylacetyl) amino]phenyl}pyridin-2-yl)-2-furamide

From the compound of Reference Example 108 (100 mg, 0.21 mmol) and imidazole-lyl-acetic acid (27 mg, 0.21 mmol) described in Chem. Pharm. Bull., page 1213 (1983), the title compound (73 mg, 60%) was given as a pale yellow crystal in substantially the same manner as in Reference Example 38.
¹H-NMR (300MHz, CDCl₃) δ: 3.43 (3H, s), 4.92 (2H, s), 5.25 (2H, s), 6.59 (1H, q, J = 1.8 Hz), 7.10-7.17 (3H, m), 7.28 (1H, s), 7.36-7.45 (3H, m), 7.58-7.62 (2H, m), 7.80 (1H, s), 7.90-7.93 (3H, m), 8.86 (2H, br s).
Melting point: 157-160°C

### Reference Example 164

### tert-butyl [(1S)-2-({3-[6-[4-chloro-2-(methoxymethoxy)phenyl]-3-cyano-2-(2-furoylamino)pyridin-4-yl]phenyl}amino)-1-methyl-2-oxoethyl]carbamate

From the compound of Reference Example 108 (300 mg, 0.63 mmol) and Boc-L-alanine (239 mg, 1.26 mmol), the title compound (256 mg, 63%) was given as a white crystal in substantially the same manner as in Reference Example 38.
¹H-NMR (300MHz, CDCl₃) δ:1.44 (3H, d, J = 6.9 Hz), 1.48 (9H, s), 3.48 (3H, s), 4.29-4.44 (1H, m), 4.95 (1H, d, J = 6.3 Hz), 5.31 (2H, s), 6.61 (1H, dd, J = 3.6, 1.8 Hz), 7.14 (1H, dd, J = 8.4, 1.8 Hz), 7.30 (1H, d, J = 2.7 Hz), 7.38 (1H, d, J = 3.6 Hz), 7.47-7.53 (3H, m), 7.59 (1H, d, J = 1.5 Hz), 7.97 (1H, d, J = 8.4 Hz), 7.99 (1H, s), 8.07 (1H, s), 8.16-8.22 (1H, br), 8.83 (1H, s).
Melting point: 171-173°C

### Reference Example 165

### tert-butyl 3-[({3-[6-[4-chloro-2-(methoxymethoxy)phenyl]-3-cyano-2-(2-furoylamino) pyridin-4-yl]phenyl}amino)carbonyl]pyrrolidine-1-carboxylate

From the compound of Reference Example 108 (300 mg, 0.63 mmol) and 1-tert-butyl pyrrolidine-1,3-dicarboxylate (272 mg, 1.26 mmol), the title compound (313 mg, 74%) was given as a white crystal in substantially the same manner as in Reference Example 38.
¹H-NMR (300MHz, CDCl₃) δ: 1.47 (9H, s), 2.14-2.30 (2H, m), 2.98-3.10 (1H, m), 3.36-3.44 (1H, m), 3.45 (3H, s), 3.50-3.70 (3H, m), 5.30 (2H, s), 6.62 (1H, t, J = 1.8 Hz), 7.15 (1H, dd, J = 8.4, 1.8 Hz), 7.30 (1H, d, J = 1.8 Hz), 7.39 (1H, d, J = 2.7 Hz), 7.45-7.53 (3H, m), 7.60 (2H, br s), 7.94-7.99 (3H, m), 8.80 (1H, s).
Melting point: 180-181°C

### Reference Example 166

### N-[6-[4-chloro-2-(methoxymethoxy)phenyl]-3-cyano-4-(3-{[4-(1H-imidazole-1-yl) butanoyl]amino}phenyl)pyridin-2-yl]-2-furamide

From the compound of Reference Example 108 (300 mg, 0.63 mmol) and 4-imidazole-1-yl butyric acid (195 mg, 1.26 mmol) described in Tetrahedron Lett., page 7499 (1997), the title compound (212 mg, 55%) was given as a pale yellow powder in substantially the same manner as in Reference Example 38.
¹H-NMR (300MHz, CDCl₃) δ: 2.19-2.27 (2H, m), 2.37 (2H, t, J = 6.9 Hz), 3.46 (3H, s), 4.11 (2H, t, J = 6.6 Hz), 5.27 (2H, s), 6.60 (1H, q, J = 1.8 Hz), 6.94 (1H, s), 7.04 (1H, s), 7.13 (1H, dd, J = 8.1, 1.5 Hz), 7.28 (1H, d, J = 4.8 Hz), 7.36-7.48 (3H, m), 7.56 (2H, d, J = 8.7 Hz), 7.73 (1H, d, J = 7.8 Hz), 7.91-7.97 (3H, m), 8.83 (1H, s), 9.29 (1H, s).

### Reference Example 167

### N-(tert-butoxycarbonyl)-N- {3-[6-[4-chloro-2-(methoxymethoxy)phenyl]-3-cyano-2-(2-furoylamino)pyridin-4-yl]phenyl}-L-histidineamide

From the compound of Reference Example 108 (300 mg, 0.63 mmol) and Boc-histidine (323 mg, 1.26 mmol), the title compound (203 mg, 45%) was given as a pale yellow crystal in substantially the same manner as in Reference Example 38.
¹H-NMR (300MHz, CDCl₃) δ: 1.48 (9H, s), 2.95-3.03 (1H, m), 3.20-3.39 (1H, m), 3.45-3.49 (3H, m), 4.62 (1H, br s), 5.28 (2H, s), 6.20-6.40 (1H, br), 6.64 (1H, q, J = 1.8 Hz), 6.96 (1H, s), 7.15 (1H, dd, J = 8.4, 1.8 Hz), 7.26-7.50 (6H, m), 7.59-7.63 (2H, m), 7.74 (1H, s), 7.92 (2H, d, J = 7.5 Hz), 8.70-8.80 (1H, br), 8.85 (1H, s).
Melting point: 149-150°C

### Reference Example 168

### 3-{3-cyano-6-[4-fluoro-2-(methoxymethoxy)phenyl]-2-[(2-methoxybenzoyl)amino] pyridin-4-yl}benzoic acid

### 3-{3-cyano-6-(4-fluoro-2-hydroxyphenyl)-2-[(2-methoxybenzoyl)amino]pyridin-4-yl} benzoic acid

2-methoxybenzoyl chloride (7.29 mL, 49.0 mmol) was added to a solution of the compound of Reference Example 137 (4.00 g, 9.82 mmol) in pyridin(30 mL), and stirred at room temperature for 17 hours. The reaction mixture was concentrated under reduced pressure, diluted with ethyl acetate, washed with water and then with saturated brine, dried over magnesium sulfate, and then concentrated under reduced pressure.
The residue was dissolved in THF (20 mL), a 2M aqueous solution (20 mL) of sodium carbonate and methanol (15mL) were added, and stirred at room temperature for 20 hours. The reaction mixture was diluted with water, and extracted with ethyl acetate. The extract was washed with saturated brine, dried over magnesium sulfate, and then concentrated under reduced pressure. The residue was purified by silica gel column chromatography (eluting solution: hexane/ethyl acetate = 4/1 to hexane/ethyl acetate = 4/1). Thus, a mixture of methyl 3-{3-cyano-6-[4-fluoro-2-(methoxymethoxy)phenyl]-2-[(2-methoxybenzoyl)amino] pyridin-4-yl}benzoate and methyl 3-{3-cyano-6-(4-fluoro-2-hydroxyphenyl)-2-[(2-methoxybenzoyl)amino]pyridin-4-yl} benzoate was obtained. A 4N aqueous sodium hydroxide solution (4 mL) was added to a solution of the resultant mixture in THF (20 mL)-ethanol (10 mL), and stirred at room temperature for 16 hours. The reaction mixture was neutralized with 1N hydrochloric acid, and extracted with ethyl acetate. The extract was washed with saturated brine and dried over magnesium sulfate. Ethyl acetate was evaporated outto give a mixture of 3-{3-cyano-6-[4-fluoro-2-(methoxymethoxy)phenyl]-2-[(2-methoxybenzoyl)amino] pyridin-4-yl}benzoic acid and 3- {3-cyano-6-(4-fluoro-2-hydroxyphenyl)-2-[(2-methoxybenzoyl)amino]pyridin-4-yl} benzoic acid as a white solid. This mixture was used to the next reaction without being purified.

### Reference Example 169

### N-[3-cyano-6-(4-fluoro-2-(methoxymethoxy)phenyl)-4-{3-[(pyrrolidine-1-ylacetyl) amino]phenyl}pyridin-2-yl]-2-furamide

A solution of the compound of Reference Example 254 (0.25 g, 0.47 mmol), pyrrolidine (37 mg, 0.52 mmol) and potassium carbonate (78 mg, 0.56 mmol) in DMF (2 mL) was stirred at room temperature for 11 hours. Pyrrolidine (20 µl, 0.33 mmol) was added and stirred at room temperature for 7 hours. The reaction mixture was diluted with ethyl acetate, washed twice with water and once with saturated brine, dried over magnesium sulfate, and then concentrated under reduced pressure. The residue was purified by basic silica gel column chromatography (eluting solution: hexane to ethyl acetate), and then recrystallized from hexane-ethyl acetate to give the title compound (0.18 g, 67%) as a colorless crystal.
¹H-NMR (300MHz, CDCl₃) δ: 1.87-1.91 (4H, m), 2.73 (4H, brs), 3.32 (2H, s), 3.47 (3H, s), 5.30 (2H, s), 6.62 (1H, dd, J = 1.8 Hz, 3.6 Hz), 6.84-6.91 (1H, m), 7.02 (1H, dd, J = 2.4 Hz, 10.8 Hz), 7.39-7.40 (1H, m), 7.45-7.54 (2H, m), 7.59-7.60 (1H, m), 7.69-7.73 (1H, m), 7.98-8.04 (3H, m), 8.82 (1H, brs), 9.28 (1H, brs).
Melting point: 152-154°C

### Reference Example 170

### Methyl 5-(dibromomethyl)-2-fluorobenzoate

A carbon tetrachloride (200 mL) mixture of methyl 2-fluoro-5-methylbenzoate (9.13 g, 54.29 mmol), N-bromosuccinimide (21.26 g, 119.44 mmol) and 1,1'-azobisisobutyronitrile (892 mg, 5.43 mmol) was refluxed while heating for 10 hours. The reaction mixture was cooled, and insoluble components were filtered out. The filtrate was washed with water and then with saturated brine, dried over magnesium sulfate, and then concentrated under reduced pressure to give the title compound (16.84 g, 95%) as a red oily substance.
¹H-NMR (300MHz, CDCl₃) δ: 3.96 (3H, s), 6.64 (1H, s), 7.18 (1H, t, J = 9.6 Hz), 7.78-7.83 (1H, m), 8.10 (1H, dd, J = 6.6, 2.7 Hz).

### Reference Example 171

### Methyl 2-(dimethylamino)-5-formylbenzoate

A mixture of the compound of Reference Example 170 (16.84 g, 51.66 mmol) and a 40% aqueous solution of dimethylamine (250 mL) was stirred at 60°C for 15 minutes. The reaction mixture was added to dichloromethane (200 mL) and stirred for 30 minutes. The organic phase was washed with saturated brine, dried over magnesium sulfate, and then concentrated under reduced pressure. The residue was purified by silica gel column chromatography (eluting solution: ethyl acetate/hexane = 1/1) to give the title compound (4.73 g, 44%) as a red liquid.
¹H-NMR (300MHz, CDCl₃) δ: 3.01 (6H, s), 3.92 (3H, s), 6.93 (1H, d, J = 8.7 Hz), 7.82 (1H, dd, J = 8.7, 2.1 Hz), 8.13 (1H, d, J = 2.1 Hz), 9.78 (1H, s).

### Reference Example 172

### 5-{3-cyano-6-[2-(methoxymethoxy)phenyl]-2-[(2-thienylcarbonyl)amino]pyridin-4-yl}-2-(dimethylamino)benzoic acid

A toluene (200 mL) mixture of the compound of Reference Example 171 (2.73 g, 13.13 mmol), the compound of Reference Example 1 (2.37 g, 13.17 mmol), malononitrile (870 mg, 13.17 mmol) and ammonium acetate (1.52 g, 19.76 mmol) was refluxed while heating for 16 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure. The residue was purified by silica gel column chromatography (eluting solution: hexane/ethyl acetate = 4/1 to hexane/ethyl acetate = 1/1)to give methyl 5-{2-amino-3-cyano-6-[2-(methoxymethoxy)phenyl]pyridin-4-yl}-2-(dimethylamino) benzoate (2.16 g) as a red amorphous solid. A pyridin (15 mL) mixture of the resultant methyl 5-{2-amino-3-cyano-6-[2-(methoxymethoxy)phenyl]pyridin-4-yl}-2-(dimethylamino) benzoate (500 mg, 1.16 mmol) and 2-thenoyl chloride (424 mg, 2.89 mmol) was stirred at room temperature for 20 hours. Water was added to the reaction mixture, and extracted twice with ethyl acetate. The organic layers were combined, washed with saturated brine, dried over magnesium sulfate, and then concentrated under reduced pressure. A 4N sodium hydroxide (3 mL) was added to a solution of the residue in THF (8 mL)-methanol (5 mL), and stirred at room temperature for 16 hours. Water was added to the reaction mixture, the resultant substance was neutralized with 1N hydrochloric acid, and then extracted with twice with ethyl acetate. The organic layers were combined, washed with saturated brine, dried over magnesium sulfate, and then concentrated under reduced pressure. The resultant solid was washed with an ethyl acetate-diethylether mixed solvent and dried under reduced pressure to give the title compound (335 mg, 55%) as a brown powder.
¹H-NMR (300MHz, DMSO-d₆) δ: 2.50 (6H, s), 3.32 (3H, s), 5.43 (2H, s), 6.23 (2H, s), 6.62 (1H, dd, J = 8.1, 2.1 Hz), 6.75 (1H, dd, J = 7.8, 1.8 Hz), 6.85 (1H, t, J = 2.1 Hz), 7.11-7.28 (4H, m), 7.36-7.42 (1H, m), 7.88 (1H, d, J = 7.8 Hz), 10.75 (1H, s).

### Reference Example 173

### tert-butyl (2-{[5-{3-cyano-6-[2-(methoxymethoxy)phenyl]-2-[(2-thienylcarbonyl) amino]pyridin-4-yl}-2-(dimethylamino)benzoyl]amino ethyl)carbamate

From the compound of Reference Example 172 (200 mg, 0.38 mmol) and tert-butyl N-(2-aminoethyl)carbamate (67 mg, 0.42 mmol), the title compound (62 mg, 24%) was given as a pale yellow crystal in substantially the same manner as in Reference Example 15.
¹H-NMR (300MHz, CDCl₃) δ:1.42 (9H, s), 2.85 (6H, s), 3.37-3.42 (2H, m), 3.48 (3H, s), 3.60 (2H, q, J = 5.9 Hz), 4.92-5.01 (1H, br), 5.29 (2H, s), 7.16 (2H, d, J = 6.9 Hz), 7.26-7.32 (2H, m), 7.42 (1H, t, J = 7.5 Hz), 7.63 (1H, t, J = 6.3 Hz), 7.73-7.97 (4H, m), 8.27 (1H, d, J = 1.8 Hz), 8.46 (1H, br s), 9.08 (1H, br s).
Melting point: 139-140°C

### Reference Example 174

### 3-{6-[4-chloro-2-(methoxymethoxy)phenyl]-3-cyano-2-[(1,3-thiazole-2-ylcarbonyl) amino]pyridin-4-yl}benzoic acid

From the compound of Reference Example 119 (300 mg, 0.71 mmol) and 1,3-thiazole-2-carbonyl chloride (261 mg, 1.77 mmol), the title compound (225 mg, 61%) was given as a pale yellow powder in substantially the same manner as in Reference Example 120.
¹H-NMR (300MHz, CDCl₃) δ: 3.39 (3H, s), 5.38 (2H, s), 7.28 (1H, dd, J = 8.4, 1.8 Hz), 7.37 (1H, d, J = 1.8 Hz), 7.78 (1H, t, J = 7.8 Hz), 7.94-8.01 (2H, m), 8.15-8.28 (5H, m), 11.56 (1H, s), 13.28 (1H, br s).

### Reference Example 175

### tert-butyl {2-[(3-{6-[4-chloro-2-(methoxymethoxy)phenyl]-3-cyano-2-[(1,3-thiazole-2-ylcarbonyl)amino]pyridin-4-yl}benzoyl)amino]ethyl}carbamate

From the compound of Reference Example 174 (250 mg, 0.48 mmol) and tert-butyl N-(2-aminoethyl)carbamate (85 mg, 0.53 mmol), the title compound (264 mg, 83%) was given as a pale yellow powder in substantially the same manner as in Reference Example 15.
¹H-NMR (300MHz, CDCl₃) δ: 1.38 (9H, s), 3.42-3.46 (2H, m), 3.47 (3H, s), 3.57-3.62 (2H, m), 4.94-5.03 (1H, br), 5.28 (2H, s), 7.17 (1H, dd, J = 8.4, 2.1 Hz), 7.27 (1H, d, J = 2.1 Hz), 7.33-7.42 (1H, br), 7.64 (1H, t, J = 7.8 Hz), 7.73 (1H, d, J = 3.0 Hz), 7.88 (1H, d, J = 8.1 Hz), 7.96-8.05 (4H, m), 8.13 (1H, s), 9.92 (1H, s).

### Reference Example 176

### N-{6-[4-chloro-2-(methoxymethoxy)phenyl]-3-cyano-4-[3-({[3-(1H-imidazole-1-yl) propyl]amino}carbonyl)phenyl]pyridin-2-yl}-1,3-thiazole-2-carboxamide

From the compound of Reference Example 174 (200 mg, 0.38 mmol) and 1-(3-aminopropyl)imidazole (53 mg, 0.42 mmol), the title compound (171 mg, 72%) was given as a pale yellow powder in substantially the same manner as in Reference Example 120.
¹H-NMR (300MHz, CDCl₃) δ: 2.12-2.21 (2H, m), 3.46 (3H, s), 3.52 (2H, q, J = 6.3 Hz), 4.11 (2H, t, J = 6.8 Hz), 5.27 (2H, s), 6.84 (1H, t, J = 5.7 Hz), 7.01 (1H, t, J = 0.9 Hz), 7.08 (1H, s), 7.15 (1H, dd, J = 8.4, 1.5 Hz), 7.27 (1H, s), 7.62 (1H, t, J = 7.8 Hz), 7.73 (1H, d, J = 3.0 Hz), 7.80-7.82 (2H, m), 7.96-8.02 (4H, m), 8.13 (1H, t, J = 1.5 Hz), 9.80-10.00 (1H, br).

### Reference Example 177

### N-[6-[4-chloro-2-(methoxymethoxy)phenyl]-3-cyano-4-(3-nitrophenyl)pyridin-2-yl] isoxazole-5-carboxamide

From the compound of Reference Example 75 (300 mg, 0.73 mmol) and 5-isoxazolecarbonyl chloride (240 mg, 1.83 mmol), the title compound (140 mg, 38%) was given as a pale green amorphous solid in substantially the same manner as in Reference Example 107.
¹H-NMR (300MHz, CDCl₃) δ: 3.51 (3H, s), 5.30 (2H, s), 7.18 (1H, dd, J = 7.5, 1.8 Hz), 7.20 (1H, s), 7.30 (1H, d, J = 1.8 Hz), 7.80 (1H, t, J = 8.1 Hz), 7.97 (1H, d, J = 8.4 Hz), 8.09-8.14 (2H, m), 8.43 (1H, dq, J = 8.1, 0.9 Hz), 8.46 (1H, d, J = 1.8 Hz), 8.52 (1H, t, J = 1.8 Hz), 8.97 (1H, s).

### Reference Example 178

### N-{4-(3-aminophenyl)-6-[4-chloro-2-(methoxymethoxy)phenyl]-3-cyanopyridin-2-yl} isoxazole-5-carboxamide

From the compound of Reference Example 177 (120 mg, 0.24 mmol), the title compound (40 mg, 35%) was given as a pale yellow amorphous solid in substantially the same manner as in Reference Example 4.
¹H-NMR (300MHz, CDCl₃) δ: 3.46 (3H, s), 3.90 (2H, br s), 5.25 (2H, s), 6.83 (1H, dd, J = 8.1, 1.5 Hz), 6.99-7.02 (2H, m), 7.14-7.17 (2H, m), 7.26-7.35 (2H, m), 7.91 (1H, d, J = 8.4 Hz), 7.98 (1H, s), 8.44 (1H, d, J = 1. 5 Hz), 8.96 (1H, s).

### Reference Example 179

### tert-butyl {3-[(3-{6-[4-chloro-2-(methoxymethoxy)phenyl]-3-cyano-2-[(isoxazole-5-ylcarbonyl)amino]pyridin-4-yl}phenyl)amino]-3-oxopropyl}carbamate

From the compound of Reference Example 178 (40 mg, 0.085 mmol), the title compound (40 mg, 73%) was given as a white amorphous solid in substantially the same manner as in Reference Example 38.
¹H-NMR (300MHz, CDCl₃) δ: 1.44 (9H, s), 2.65 (2H, t, J = 5.7 Hz), 3.45-3.55 (5H, m), 5.05-5.15 (1H, br), 5.31 (2H, s), 7.14-7.18 (2H, m), 7.30 (1H, s), 7.48-7.57 (3H, m), 7.95 (2H, d, J = 8.4 Hz), 8.06-8.08 (2H, m), 8.44 (1H, d, J = 1.8 Hz), 8.94 (1H, s).

### Reference Example 180

### N-[6-[4-chloro-2-(methoxymethoxy)phenyl]-3-cyano-4-(3-nitrophenyl)pyridin-2-yl]-4-methoxybenzamide

From the compound of Reference Example 75 (300 mg, 0.73 mmol) and 4-methoxybenzoyl chloride (872 mg, 5.11 mmol), the title compound (340 mg, 85%) was given as a pale yellow powder in substantially the same manner as in Reference Example 107.
¹H-NMR (300MHz, CDCl₃) δ: 3.50 (3H, s), 3.91 (3H, s), 5.29 (2H, s), 7.02 (2H, d, J = 9.0 Hz), 7.15 (1H, dd, J = 8.7, 2.1 Hz), 7.29 (1H, d, J = 1.8 Hz), 7.77 (1H, t, J = 8.0 Hz), 7.91 (1H, d, J = 8.4 Hz), 7.96-8.00 (3H, m), 8.15 (1H, dt, J = 8.0, 1.2 Hz), 8.41 (1H, dq, J = 8.0, 1.2 Hz), 8.50-8.52 (2H, m).

### Reference Example 181

### N-{4-(3-aminophenyl)-6-[4-chloro-2-(methoxymethoxy)phenyl]-3-cyanopyridin-2-yl}-4-methoxybenzamide

From the compound of Reference Example 180 (300 mg, 0.55 mmol), the title compound (230 mg, 81 %) was given as a pale yellow amorphous solid in substantially the same manner as in Reference Example 4.
¹H-NMR (300MHz, CDCl₃) δ: 3.46 (3H, s), 3.86 (2H, br s), 3.90 (3H, s), 5.24 (2H, s), 6.82 (1H, dd, J = 9.0,1.5 Hz), 7.00-7.03 (4H, m), 7.13 (1H, dd, J = 8.4, 1.8 Hz), 7.26-7.34 (2H, m), 7.85-7.87 (2H, m), 7.97 (2H, dd, J = 6.9, 1.8 Hz), 9.45 (1H, s).

### Reference Example 182

### tert-butyl {3-[(3-{6-[4-chloro-2-(methoxymethoxy)phenyl]-3-cyano-2-[(4-methoxybenzoyl) amino]pyridin-4-yl}phenyl)amino]-3-oxopropyl}carbamate

From the compound of Reference Example 181 (200 mg, 0.39 mmol), the title compound (250 mg, 94%) was given as a pale green amorphous solid in substantially the same manner as in Reference Example 38.
¹H-NMR (300MHz, CDCl₃) δ: 1.44 (9H, s), 2.63 (2H, t, J = 5.9 Hz), 3.47-3.53 (5H, m), 3.89 (3H, s), 5.17 (1H, br s), 5.29 (2H, s), 7.00 (2H, dd, J = 9.9, 3.0 Hz), 7.12 (1H, dd, J = 8.4, 1.8 Hz), 7.26-7.29 (1H, m), 7.47 (2H, q, J = 1.8 Hz), 7.55-7.63 (1H, m), 7.86-8.00 (6H, m), 8.52 (1H, s).

### Reference Example 183

### N-(tert-butoxycarbonyl)-N-{3-[6-[4-chloro-2-(methoxymethoxy)phenyl]-3-cyano-2-(2-furoylamino)pyridin-4-yl]phenyl} -D-histidineamide

From the compound of Reference Example 108 (300 mg, 0.63 mmol) and Boc-D-histidine(323 mg, 1.26 mmol), the title compound (88 mg, 20%) was given as a pale yellow crystal in substantially the same manner as in Reference Example 38.
¹H-NMR (300MHz, CDCl₃) δ: 1.50 (9H, s), 2.98-3.05 (1H, m), 3.31 (1H, d, J = 13.5 Hz), 3.49 (3H, s), 4.64 (1H, br s), 5.30 (2H, s), 6.20-6.40 (1H, br), 6.66 (1H, q, J = 1.8 Hz), 6.97 (1H, s), 7.17 (1H, dd, J = 8.4, 2.1 Hz), 7.28-7.52 (6H, m), 7.60-7.64 (2H, m), 7.75 (1H, s), 7.94 (1H, d, J = 7.8 Hz), 7.96 (1H, s), 8.70-8.80 (1H, br), 8.85 (1H, br s). Melting point: 148-149°C

### Reference Example 184

### N-[6-[4-chloro-2-(methoxymethoxy)phenyl]-3-cyano-4-(3-nitrophenyl)pyridin-2-yl]-2,4-dimethoxybenzamide

From the compound of Reference Example 75 (300 mg, 0.73 mmol) and 2,4-dimethoxybenzoyl chloride (1.03 g, 5.11 mmol), the title compound (103 mg, 24%) was given as a white powder in substantially the same manner as in Reference Example 107.
¹H-NMR (300MHz, CDCl₃) δ: 3.49 (3H, s), 3.90 (3H, s), 4.10 (3H, s), 5.28 (2H, s), 6.58 (1H, d, J = 2.4 Hz), 6.69 (1H, dd, J = 8.7, 2.1 Hz), 7.19 (1H, dd, J = 8.4, 2.1 Hz), 7.26 (1H, s), 7.77 (1H, t, J =8.1 Hz), 7.92 (1H, s), 8.09-8.13 (2H, m), 8.35 (1H, d, J = 8.7 Hz), 8.40-8.43 (1H, m), 8.52 (1H, t, J = 1.8 Hz), 10.76 (1H, s).

### Reference Example 185

### N-{4-(3-aminophenyl)-6-[4-chloro-2-(methoxymethoxy)phenyl]-3-cyanopyridin-2-yl}-2,4-dimethoxybenzamide

From the compound of Reference Example 184 (100 mg, 0.17 mmol), the title compound (95 mg, 100%) was given as a colorless amorphous solid in substantially the same manner as in Reference Example 4.
¹H-NMR (300MHz, CDCl₃) δ: 3.44 (3H, s), 3.88 (3H, s), 3.92 (2H, br s), 4.11 (3H, s), 5.23 (2H, s), 6.55 (1H, t, J = 2.1 Hz), 6.67-6.68 (1H, m), 6.79-6.83 (1H, m), 6.99-7.01 (2H, m), 7.15 (1H, dd, J = 8.4, 1.8 Hz), 7.24-7.32 (2H, m), 7.84 (1H, s), 8.08 (1H, d, J = 8.4 Hz), 8.33 (1H, d, J = 8.7 Hz), 10.71 (1H, s).

### Reference Example 186

### tert-butyl {3-[(3-{6-[4-chloro-2-(methoxymethoxy)phenyl]-3-cyano-2-[(2,4-dimethoxybenzoyl)amino]pyridin-4-yl}phenyl)amino]-3-oxopropyl}carbamate

From the compound of Reference Example 185 (95 mg, 0.17 mmol), the title compound (100 mg, 78%) was given as a colorless oily substance in substantially the same manner as in Reference Example 38.
¹H-NMR (300MHz, CDCl₃) δ:1.43 (9H, s), 2.61 (2H, t, J = 5.7 Hz), 3.45-3.50 (5H, m), 3.88 (3H, s), 4.08 (3H, s), 5.20-5.30 (3H, br s), 6.55 (1H, dd, J = 8.7, 2.4 Hz), 6.65 (1H, dd, J = 8.7, 2.4 Hz), 7.13 (1H, dd, J = 8.4, 1.8 Hz), 7.25 (1H, d, J = 1.8 Hz), 7.39-7.44 (2H, m), 7.59 (1H, d, J = 7.8 Hz), 7.88 (1H, s), 7.98 (1H, br s), 8.06 (1H, d, J = 8.4 Hz), 8.31 (1H, d, J = 8.7 Hz), 8.40 (1H, br s), 10.70 (1H, s).

### Reference Example 187

### 2-(benzoylamino)-4-[3-({N-[(benzyloxy)carbonyl]-beta-alanyl}amino)phenyl]-6-[2-(benzyloxy)phenyl]nicotinic acid

A solution of the compound of Reference Example 131 (590 mg, 0.76 mmol) in formic acid (20 mL) was stirred at room temperature for 6 days. The reaction mixture was concentrated under reduced pressure, and the resultant solid was washed with ethyl acetate and dried under reduced pressure to give the title compound (444 mg, 81%) as a pale yellow powder.
¹H-NMR (300MHz, DMSO-d₆) δ: 2.51-2.55 (2H, m), 3.27-3.33 (2H, m), 5.01 (2H, s), 5.20 (2H, s), 6.84 (1H, d, J = 7.8 Hz), 7.12 (1H, t, J = 7.2 Hz), 7.23-7.38 (12H, m), 7.44-7.68 (6H, m), 7.84 (1H, s), 7.92 (1H, dd, J = 9.3, 1.5 Hz), 8.01 (1H, d, J = 8.4 Hz), 8.13 (1H, s), 10.08 (1H, s), 10.80 (1H, s), 12.65-12.90 (1H, br).

### Reference Example 188

### N-[3-cyano-6-[4-fluoro-2-(methoxymethoxy)phenyl]-4-(3-nitrophenyl)pyridin-2-yl] isoxazole-5-carboxamide

From the compound of Reference Example 133 (1.0 g, 2.54 mmol) and 5-isoxazolecarbonyl chloride (834 mg, 6.34 mmol), the title compound (891 mg, 72%) was given as a white crystal in substantially the same manner as in Reference Example 134.
¹H-NMR (300MHz, CDCl₃) δ: 3.51 (3H, s), 5.30 (2H, s), 6.91 (1H, td, J = 9.0, 2.1 Hz), 7.04 (1H, dd, J = 10.8, 2.4 Hz), 7.19 (1H, d, J = 2.1 Hz), 7.80 (1H, t, J = 8.1 Hz), 8.03 (1H, dd, J = 7.8, 6.9 Hz), 8.08 (1H, s), 8.13 (1H, dt, J = 7.8, 1.2 Hz), 8.43 (1H, dq, J = 8.1, 1.2 Hz), 8.46 (1H, d, J = 1.8 Hz), 8.53 (1H, t, J = 1.8 Hz), 8.97 (1H, s).
Melting point: 161-162°C

### Reference Example 189

### tert-butyl {(1S)-3-[(3-{3-cyano-6-[4-fluoro-2-(methoxymethoxy)phenyl]-2-[(isoxazole-5-ylcarbonyl)amino]pyridin-4-yl}phenyl)amino]-1-methyl-3-oxopropyl} carbamate

An ethanol (20 mL)-water (20 mL) mixture of the compound of Reference Example 188 (500 mg, 1.02 mmol), iron powder (341 mg, 6.13 mmol) and ammonium chloride (388 mg, 6.64 mmol) was refluxed while heating for 2 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give N-{4-(3-aminophenyl)-3-cyano-6-[4-fluoro-2-(methoxymethoxy)phenyl]pyridin-2-yl} isoxazole-5-carboxamide (100 mg) as a yellow amorphous solid.

WSCD (66 mg, 0.35 mmol) was added to a solution of the resultant N- {4-(3-aminophenyl)-3-cyano-6-[4-fluoro-2-(methoxymethoxy)phenyl]pyridin-2-yl} isoxazole-5-carboxamide (80 mg, 0.17 mmol), (S)-3-(N-Boc-amino)butyric acid (71 mg, 0.35 mmol) and HOBt (47 mg, 0.35 mmol) in DMF (5 mL) with ice-cooling, and stirred at room temperature for 17 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: hexane/ethyl acetate = 1/1 to hexane/ethyl acetate = 1/2), and then recrystallized from ethyl acetate-hexane to give the title compound (39 mg, 89%) as a pale yellow crystal.
¹H-NMR (300MHz, CDCl₃) δ: 1.31 (3H, d, J = 6.6 Hz), 1.43 (9H, s), 2.61-2.64 (2H, m), 3.49 (3H, s), 4.07-4.14 (1H, m), 4.95-5.04 (1H, br), 5.32 (2H, s), 6.85-6.91 (1H, m), 7.02-7.05 (1H, m), 7.16 (1H, d, J = 1.8 Hz), 7.47-7.56 (3H, m), 7.97-8.12 (3H, m), 8.20-8.30 (1H, br), 8.44 (1H, s), 9.96 (1H, s).
Melting point: 171-174°C

### Reference Example 190

### 2-amino-6-[2-(benzyloxy)phenyl]-4-(methylthio)nicotinonitrile

A mixture of 6-[2-(benzyloxy)phenyl]-4-(methylthio)-2-oxo-1,2-dihydronicotinonitrile (2.5 g, 7.1 mmol) described in WO 02/24679, page 240, bromoacetamide (1.1 g, 7.8 mmol), potassium carbonate (1.2 g, 8.6 mmol) and DMF (35 mL) was stirred at 60°C for 1.5 hours. Water (5 mL) was added to the reaction mixture, and extracted with ethyl acetate. The extract was washed with water and then with saturated brine, dried over magnesium sulfate, and then concentrated under reduced pressure. Among 2.6 g of the resultant residue, 1.6 g (4.0 mmol) was dissolved in DMF (7 mL), potassium carbonate (1.1 g, 8.0 mmol) was added, and stirred at 130°C for 5 hours. The reaction solution was concentrated under reduced pressure. The residue was diluted with chloroform, washed with water and then with saturated brine, dried over magnesium sulfate, and then concentrated under reduced pressure.
The residue was purified by silica gel chromatography (eluting solution: ethyl acetate/hexane = 95/5 to ethyl acetate/hexane = 1/1) to give the title compound (315 mg, 22%) as a white crystal.
¹H-NMR (300MHz, CDCl₃) δ: 2.05 (3H, s), 5.09 (2H, s), 5.14 (2H, br s), 7.08-7.13 (2H, m), 7.20 (2H, s), 7.30-7.46 (5H, m), 7.91 (1H, dd, J = 7.8, 1.8 Hz).
Melting point: 145-146°C

### Reference Example 191

### N-[6-[2-(benzyloxy)phenyl]-3-cyano-4-(methylsulfinyl)pyridin-2-yl]-2-furamide

2-furoyl chloride (517 mg, 4.0 mmol) was added to a solution of the compound of Reference Example 190 (459 mg, 1.3 mmol) in pyridin(2.5 mL) at room temperature and then stirred at room temperature for 12 hours. Water (5 mL) was added to the reaction solution, and the precipitated crystals were collected by filtration and dried.
The resultant difuroyl derivatve (670 mg, 1.3 mmol) was dissolved in dichloromethane(10 mL). mCPBA (70%, 370 mg, 1.5 mmol) was added with ice-cooling and stirred with ice-cooling for 1.5 hours. The reaction solution was concentrated under reduced pressure, and then the residue was purified by silica gel chromatography using basic silica gel (eluting solution: ethyl acetate/hexane = 9/1 to ethyl acetate/hexane = 2/3) to give the title compound (412 mg, 68%) as a white crystal.
¹H-NMR (300MHz, CDCl₃) δ: 2.81 (3H, s), 5.14 (1H, d, J = 11.4 Hz), 5.21 (1H, d, J = 11.4 Hz), 6.63 (1H, dd, J = 3.6, 1.5 Hz), 7.08-7.18 (2H, m), 7.28-7.52 (7H, m), 7.60 (1H, s), 7.91 (1H, dd, J = 8.1, 1.8 Hz), 8.56 (1H, s), 8.81 (1H, s). Melting point: 172-173°C

### Reference Example 192

### ethyl 1-[6-[2-(benzyloxy)phenyl]-3-cyano-2-(2-furoylamino)pyridin-4-yl]piperidine-3-carboxylate

A solution of the compound of Reference Example 191 (410 mg, 0.89 mmol) and ethyl piperidine-3-carboxylate (353 mg, 2.2 mmol) in DMF (4 mL) was stirred at 130°C for 5 hours. After the reaction solution was concentrated under reduced pressure, the residue was purified by silica gel chromatography (eluting solution: ethyl acetate/hexane = 9/1 to ethyl acetate/hexane = 2/3) to give the title compound (324 mg, 66%) as an amorphous solid.
¹H-NMR (300MHz, CDCl₃) δ: 1.23 (3H, t, J = 7.2 Hz), 1.50-1.78 (3H, m), 1.95-2.11 (1H, m), 2.51-2.68 (1H, m), 2.80-2.95 (1H, m), 3.10 (1H, dd, J = 12.9, 9.9 Hz), 3.54 (1H, d, J = 12.9 Hz), 3.88 (1H, d, J = 9.6 Hz), 4.12 (2H, q, J = 7.2 Hz), 5.10 (2H, s), 6.58 (1H, dd, J = 3.6, 1.8 Hz), 7.03-7.20 (2H, m), 7.30-7.48 (8H, m), 7.55 (1H, d, J = 1.8 Hz), 7.96 (1H, dd, J = 8.1, 1.8 Hz), 8.60 (1H, s).

### Reference Example 193

### 1-[6-[2-(benzyloxy)phenyl]-3-cyano-2-(2-furoylamino)pyridin-4-yl]piperidine-3-carboxylic acid

A 4N aqueous sodium hydroxide solution (0.4 mL, 1.6 mmol) was added to a solution of the compound of Reference Example 192 (300 mg, 0.55 mmol) in ethanol (3 mL) and stirred at 60°C for 40 minutes. The reaction solution was ice-cooled. Then, 2N hydrochloric acid (0.8 ml, 1.6 mmol) was added and concentrated under reduced pressure. The residue was diluted with dichloromethane (10 mL), washed with water and then with saturated brine, dried over magnesium sulfate, and then concentrated under reduced pressure to give the title compound (210 mg, 74%) as an amorphous solid.
¹H-NMR (300MHz, CDCl₃) δ: 1.50-1.82 (3H, m), 1.95-2.15 (1H, m), 2.52-2.71 (1H, m), 2.91 (1H, br t, J = 10.2 Hz), 2.99-3.15 (1H, m), 3.57 (1H, d, J = 12.6 Hz), 3.86 (1H, d, J = 13.2 Hz), 5.09 (2H, s), 6.57 (1H, dd, J = 3.6, 1.8 Hz), 7.00-7.18 (2H, m), 7.20-7.48 (8H, m), 7.56 (1H, s), 7.91 (1H, d, J = 8.1 Hz).

### Reference Example 194

### N-[6-[4-chloro-2-(methoxymethoxy)phenyl]-3-cyano-4-(3-nitrophenyl)pyridin-2-yl] isoxazole-5-carboxamide

From the compound of Reference Example 75 (1.0 g, 2.43 mmol) and 5-isoxazsolecarbonyl chloride (800 mg, 6.09 mmol), the title compound (877 mg, 79%) was given as a pale orange crystal in substantially the same manner as in Reference Example 134.
¹H-NMR (300MHz, CDCl₃) δ: 3.51 (3H, s), 5.30 (2H, s), 7.18 (1H, dd, J = 7.5, 1.8 Hz), 7.20 (1H, s), 7.30 (1H, d, J = 1.8 Hz), 7.80 (1H, t, J = 8.1 Hz), 7.97 (1H, d, J = 8.4 Hz), 8.09-8.14 (2H, m), 8.43 (1H, dq, J = 8.1, 0.9 Hz), 8.46 (1H, d, J = 1.8 Hz), 8.52 (1H, t, J = 1.8 Hz), 8.97 (1H, s). Melting point: 194-195°C

### Reference Example 195

### tert-butyl {(1S)-3-[(3-{6-[4-chloro-2-(methoxymethoxy)phenyl]-3-cyano-2-[(isoxazole-5-ylcarbonyl)amino]pyridin-4-yl}phenyl)amino]-1-methyl-3-oxopropyl} carbamate

From the compound of Reference Example 194 (500 mg, 0.99 mmol), the title compound (10 mg, 5%) was given as a pale yellow crystal in substantially the same manner as in Reference Example 189.
¹H-NMR (300MHz, CDCl₃) δ: 1.33 (3H, d, J = 6.9 Hz), 1.43 (9H, s), 2.62-2.65 (2H, m), 3.49 (3H, s), 4.04-4.13 (1H, m), 4.92-5.04 (1H, br), 5.32 (2H, s), 7.14-7.17 (2H, m), 7.31 (1H, d, J = 2.1 Hz), 7.48-7.55 (3H, m), 7.95 (1H, d, J = 8.4 Hz), 8.07 (1H, s), 8.12 (1H, br s), 8.20-8.30 (1H, br), 8.44 (1H, d, J = 1.8 Hz), 8.94 (1H, s). Melting point: 193-194°C

### Reference Example 196

### N-[6-[4-chloro-2-(methoxymethoxy)phenyl]-3-cyano-4-(3-nitrophenyl)pyridin-2-yl]-1,3-thiazole-2-carboxamide

From the compound of Reference Example 75 (600 mg, 1.46 mmol) and thiazole-2-carbonyl chloride (539 mg, 3.65 mmol), the title compound (607 mg, 90%) was given as a white crystal in substantially the same manner as in Reference Example 134.
¹H-NMR (300MHz, CDCl₃) δ: 3.50 (3H, s), 5.30 (2H, s), 7.18 (1H, dd, J = 8.4, 1.8 Hz), 7.29 (1H, d, J = 1.8 Hz), 7.74-7.82 (2H, m), 8.02-8.14 (4H, m), 8.42 (1H, dt, J = 8.4, 1.2 Hz), 8.52 (1H, d, J = 1.8 Hz), 9.94 (1H, s). Melting point: 194-195°C

### Reference Example 197

### N-{4-(3-aminophenyl)-6-[4-chloro-2-(methoxymethoxy)phenyl]-3-cyanopyridin-2-yl}-1,3-thiazole-2-carboxamide

From the compound of Reference Example 196 (300 mg, 0.57 mmol), the title compound (80 mg, 29%) was given as a pale orange amorphous solid in substantially the same manner as in Reference Example 4.
¹H-NMR (300MHz, CDCl₃) δ: 3.46 (3H, s), 3.89 (2H, br s), 5.25 (2H, s), 6.83 (1H, dd, J = 7.5, 1.5 Hz), 6.98-7.00 (2H, m), 7.15 (1H, dd, J = 8.4, 1.2 Hz), 7.26-7.34 (2H, m), 7.71 (1H, s), 7.95 (1H, s), 7.99-8.03 (2H, m), 9.91 (1H, s).

### Reference Example 198

### tert-butyl {(1S)-3-[(3-{6-[4-chloro-2-(methoxymethoxy)phenyl]-3-cyano-2-[(1,3-thiazole-2-ylcarbonyl)amino]pyridin-4-yl}phenyl)amino]-1-methyl-3-oxopropyl} carbamate

From the compound of Reference Example 197 (40 mg, 0.081 mmol) and (S)-3-(N-Boc-amino)butyric acid, the title compound (18 mg, 33%) was given as a white crystal in substantially the same manner as in Reference Example 38.
¹H-NMR (300MHz, CDCl₃) δ:1.31 (3H, d, J = 6.6 Hz), 1.43 (9H, s), 2.55-2.72 (2H, m), 3.47 (3H, s), 4.06-4.18 (1H, m), 4.90-5.02 (1H, br), 5.32 (2H, s), 7.16 (1H, dd, J = 8.4, 1.8 Hz), 7.30 (1H, d, J = 1.8 Hz), 7.48-7.56 (3H, m), 7.72 (1H, d, J = 2.7 Hz), 8.00-8.10 (4H, m), 8.15-8.30 (1H, br), 9.92 (1H, s). Melting point: 153-155°C

### Reference Example 199

### tert-butyl {(1R)-3-[(3-{6-[4-chloro-2-(methoxymethoxy)phenyl]-3-cyano-2-[(1,3-thiazole-2-ylcarbonyl)amino]pyridin-4-yl}phenyl)amino]-1-methyl-3-oxopropyl} carbamate

From the compound of Reference Example 197 (40 mg, 0.081 mmol) and (R)-3-(N-Boc-amino)butyric acid, the title compound (21 mg, 33%) was given as a white crystal in substantially the same manner as in Reference Example 38.
¹H-NMR (300MHz, CDCl₃) δ: 1.31 (3H, d, J = 6.6 Hz), 1.43 (9H, s), 2.55-2.69 (2H, m), 3.48 (3H, s), 4.06-4.18 (1H, m), 4.92-5.00 (1H, br), 5.32 (2H, s), 7.16 (1H, dd, J = 8.4, 1.8 Hz), 7.30 (1H, d, J = 2.1 Hz), 7.48-7.56 (3H, m), 7.72 (1H, d, J = 3.0 Hz), 8.00-8.10 (4H, m), 8.16-8.30 (1H, br), 9.92 (1H, s). Melting point: 155-156°C

### Reference Example 200

### tert-butyl {3-[(3-{2-amino-6-[4-chloro-2-(methoxymethoxy)phenyl]-3-cyanopyridin-4-yl}phenyl)amino]-1-methyl-3-oxopropyl}carbamate

From the compound of Reference Example 129 (1.74 g, 4.57 mmol) and N-Boc-3-aminobutyric acid (1.86 g, 9.14 mmol), the title compound (1.90 g, 73%) was given as a colorless amorphous solid in substantially the same manner as in Reference Example 130.
¹H-NMR (300MHz, CDCl₃) δ:1.32 (3H, d, J = 6.6 Hz), 1.48 (9H, s), 2.59-2.70 (2H, m), 3.50 (3H, s), 4.05-4.18 (1H, m), 5.04 (1H, d, J = 8.1 Hz), 5.26 (2H, s), 5.37 (2H, br s), 7.12 (1H, dd, J =8.4, 1.8 Hz), 7.28 (1H, d, J = 2.4 Hz), 7.37-7.57 (4H, m), 7.76 (1H, d, J = 8.4 Hz), 8.01 (1H, s), 8.20-8.30 (1H, br).

### Reference Example 201

### tert-butyl {3-[(3-{6-[4-chloro-2-(methoxymethoxy)phenyl]-3-cyano-2-[(5-methyl-2-furoyl)amino]pyridin-4-yl}phenyl)amino]-1-methyl-3-oxopropyl}carbamate

From the compound of Reference Example 200 (120 mg, 0.21 mmol) and 5-methylfuran-2-carbonyl chloride (77 mg, 0.53 mmol), the title compound (40 mg, 28%) was given as a white amorphous solid in substantially the same manner as in Reference Example 59.
¹H-NMR (300MHz, CDCl₃) δ: 1.31 (3H, d, J = 6.6 Hz), 1.43 (9H, s), 2.43 (3H, s), 2.62 (2H, d, J = 5.4 Hz), 3.48 (3H, s), 4.00-4.15 (1H, m), 4.90-5.05 (1H, br), 5.31 (2H, s), 6.22 (1H, d, J = 3.3 Hz), 7.13-7.30 (5H, m), 7.49-7.61 (3H, m), 7.95 (1H, s), 8.08 (1H, s), 8.72 (1H, s).

### Reference Example 202

### Ethyl 2'-amino-3'-cyano-6'-[2-(methoxymethoxy)phenyl]-4,4'-bipyridin-2-carboxylate

From the compound of Reference Example 1 (1.51 g, 8.37 mmol) and ethyl 4-formylpyridin-2-carboxylate (1.50 g, 8.37 mmol), the title compound (640 mg, 19%) was given as a pale yellow amorphous solid in substantially the same manner as in Reference Example 2.
¹H-NMR (300MHz, CDCl₃) δ: 1.47 (3H, t, J = 7.2 Hz), 3.45 (3H, s), 4.51 (2H, q, J = 7.2 Hz), 5.23 (2H, s), 5.40 (2H, br s), 7.15 (1H, t, J = 7.5 Hz), 7.23 (1H, d, J = 8.4 Hz), 7.39-7.46 (2H, m), 7.76 (1H, dd, J = 4.8, 1.8 Hz), 7.80 (1H, dd, J = 7.5, 1.8 Hz), 8.34 (1H, d, J = 0.9 Hz), 8.92 (1H, d, J = 4.8 Hz).

### Reference Example 203

### Ethyl 3'-cyano-2'-(2-furoylamino)-6'-[2-(methoxymethoxy)phenyl]-4,4'-bipyridin-2-carboxylate

From the compound of Reference Example 202 (300 mg, 0.74 mmol) and 2-furoyl chloride (242 mg, 1.85 mmol), the title compound (292 mg, 79%) was given as a white powder in substantially the same manner as in Reference Example 59.
¹H-NMR (300MHz, CDCl₃) δ: 1.48 (3H, t, J = 7.2 Hz), 3.47 (3H, s), 4.53 (2H, q, J = 7.2 Hz), 5.28 (2H, s), 6.63 (1H, q, J = 1.8 Hz), 7.19 (1H, t, J = 7.5 Hz), 7.24-7.27 (1H, m), 7.41-7.48 (2H, m), 7.61 (1H, q, J = 0.9 Hz), 7.86 (1H, q, J = 1.8 Hz), 7.98 (1H, dd, J = 7.8, 1.8 Hz), 8.02 (1H, s), 8.40 (1H, dd, J = 1.8, 0.6 Hz), 8.85 (1H, s), 8.98 (1H, dd, J = 5.1, 0.6 Hz).

### Reference Example 204

### 3'-cyano-2'-(2-furoylamino)-6'-[2-(methoxymethoxy)phenyl]-4,4'-bipyridin-2-carboxylic acid

From the compound of Reference Example 203 (200 mg, 0.40 mmol), the title compound (160 mg, 85%) was given as a white amorphous solid in substantially the same manner as in Reference Example 7.
¹H-NMR (300MHz, CDCl₃) δ: 3.49 (3H, s), 5.29 (2H, s), 6.64 (1H, t, J = 1.8 Hz), 7.16-7.28 (2H, m), 7.41-7.46 (2H, m), 7.61 (1H, s), 7.97 (1H, d, J = 7.8 Hz), 8.05 (2H, s), 8.46 (1H, s), 8.84 (1H, s), 8.86 (1H, s).

### Reference Example 205

### tert-butyl {2-[({3'-cyano-2'-(2-furoylamino)-6'-[2-(methoxymethoxy)phenyl]-4,4'-bipyridin-2-yl}carbonyl)amino]ethyl}carbamate

From the compound of Reference Example 204 (90 mg, 0.19 mmol), the title compound (85 mg, 73%) was given as a white amorphous solid in substantially the same manner as in Reference Example 15.
¹H-NMR (300MHz, CDCl₃) δ: 1.44 (9H, s), 3.42-3.47 (5H, m), 3.60-3.66 (2H, m), 4.95 (1H, br s), 5.29 (2H, s), 6.62 (1H, q, J = 1.8 Hz), 7.18 (1H, t, J = 7.5 Hz), 7.26-7.28 (1H, m), 7.41-7.48 (2H, m), 7.61 (1H, t, J = 0.9 Hz), 7.88 (1H, dd, J = 5.1, 1.8 Hz), 7.96 (1H, dd, J = 7.5, 1.8 Hz), 8.03 (1H, s), 8.30-8.42 (2H, m), 8.76 (1H, d, J = 4.8 Hz), 8.84 (1H, s).

### Reference Example 206

### Methyl 3 3-{2-amino-3-cyano-6-[2-(methoxymethoxy)phenyl]pyridin-4-yl}-2-methoxy benzoate

From the compound of Reference Example 1 (933 mg, 5.18 mmol) and methyl 3-formyl-2-methoxybenzoate (1.0 g, 5.18 mmol), the title compound (1.01 g, 46%) was given as a yellow oily substance in substantially the same manner as in Reference

### Example 2.

¹H-NMR (300MHz, CDCl₃) δ: 3.43 (3H, s), 3.68 (3H, s), 3.94 (3H, s), 5.18 (2H, s), 5.34 (2H, br s), 7.13 (1H, td, J = 7.5, 0.9 Hz), 7.20 (1H, dd, J = 8.4, 0.9 Hz), 7.25-7.30 (1H, m), 7.35-7.41 (2H, m), 7.53 (1H, dd, J = 7.5, 1.8 Hz), 7.77 (1H, dd, J = 7.5, 1.8 Hz), 7.89 (1H, dd, J = 7.8, 1.8 Hz).

### Reference Example 207

### Methyl 3-{3-cyano-2-(2-furoylamino)-6-[2-(methoxymethoxy)phenyl]pyridin-4-yl}-2-methoxybenzoate

From the compound of Reference Example 206 (300 mg, 0.72 mmol) and 2-furoyl chloride (233 mg, 1.79 mmol), the title compound (223 mg, 60%) was given as a white crystal in substantially the same manner as in Reference Example 59.
¹H-NMR (300MHz, CDCl₃) δ: 3.42 (3H, s), 3.72 (3H, s), 3.96 (3H, s), 5.23 (2H, s), 6.61 (1H, q, J = 1.8 Hz), 7.14-7.42 (5H, m), 7.59 (1H, s), 7.63 (1H, dd, J = 7.5, 1.8 Hz), 7.92-7.97 (3H, m), 8.81 (1H, s). Melting point: 140-141°C

### Reference Example 208

### 3-{3-cyano-2-(2-furoylamino)-6-[2-(methoxymethoxy)phenyl]pyridin-4-yl}-2-methoxy benzoic acid

From the compound of Reference Example 207 (200 mg, 0.39 mmol), the title compound (190 mg, 100%) was given as a pale green amorphous solid in substantially the same manner as in Reference Example 7.
¹H-NMR (300MHz, DMSO-d₆) δ: 3.30 (3H, s), 3.65 (3H, s), 5.28 (2H, s), 6.76 (1H, t, J = 1.8 Hz), 7.18 (1H, t, J = 7.8 Hz), 7.28 (1H, d, J = 8.4 Hz), 7.38 (1H, t, J = 7.8 Hz), 7.46-7.54 (2H, m), 7.62 (1H, d, J = 7.8 Hz), 7.88 (2H, t, J = 8.4 Hz), 7.98 (1H, s), 8.02 (1H, s), 11.29 (1H, s), 13.26 (1H, br s).

### Reference Example 209

### tert-butyl {2-[(3-{3-cyano-2-(2-furoylamino)-6-[2-(methoxymethoxy)phenyl] pyridin-4-yl}-2-methoxybenzoyl)amino]ethyl}carbamate

From the compound of Reference Example 208 (90 mg, 0.18 mmol), the title compound (47 mg, 41 %) was given as a white crystal in substantially the same manner as in Reference Example 15.
¹H-NMR (300MHz, CDCl₃) δ: 1.40 (9H, s), 3.39-3.43 (5H, m), 3.57-3.65 (5H, m), 4.98 (1H, br s), 5.24 (2H, s), 6.62 (1H, q, J = 1.8 Hz), 7.16-7.26 (2H, m), 7.35-7.47 (3H, m), 7.55-7.60 (2H, m), 7.97-8.03 (3H, m), 8.22 (1H, dd, J = 7.8, 1.8 Hz), 8.82 (1H, s). Melting point: 143-144°C

### Reference Example 210

### tert-butyl (3-{[3-(6-[4-chloro-2-(methoxymethoxy)phenyl]-3-cyano-2-{[(1-methyl-1H-pyrrole-2-yl)carbonyl]amino }pyridin-4-yl)phenyl]amino}-1-methyl-3-oxopropyl) carbamate

From the compound of Reference Example 200 (120 mg, 0.21 mmol) and 1-methyl-pyrrole-2-carbonyl chloride (76 mg, 0.53 mmol), the title compound (14 mg, 10%) was given as a yellow amorphous solid in substantially the same manner as in Reference Example 59.
¹H-NMR (300MHz, CDCl₃) δ:1.32 (3H, d, J = 6.6 Hz), 1.45 (9H, s), 2.64 (2H, d, J = 5.7 Hz), 3.50 (3H, s), 4.04-4.14 (4H, m), 4.96-5.08 (1H, br), 5.32 (2H, s), 6.21 (1H, dd, J = 4.2, 2.4 Hz), 6.88 (1H, d, J = 2.4 Hz), 6.92 (1H, dd, J = 4.2, 1.5 Hz), 7.13-7.18 (1H, m), 7.28-7.31 (2H, m), 7.46-7.50 (2H, m), 7.50-7.59 (1H, m), 7.91-7.94 (1H, m), 8.07 (1H, s), 8.16-8.30 (1H, br), 8.37 (1H, s).

### Reference Example 211

### tert-butyl (3-{3-cyano-2-(2-furoylamino)-6-[2-(methoxymethoxy)phenyl]pyridin-4-yl}-2-methoxyphenyl)carbamate, and

### N-{4-(3-amino-2-methoxyphenyl)-3-cyano-6-[2-(methoxymethoxy)phenyl]pyridin-2-yl}-2-furamide

A mixture of the compound of Reference Example 208 (80 mg, 0.16 mmol), diphenylphosphonyl azide (53 mg, 0.19 mmol) and triethylamine (39 mg, 0.38 mmol) in toluene (5 mL) was stirred at room temperature for 2 hours, and then tert-butanol (2 mL) was added and stirred at 90°C for 8 hours. The reaction mixture was cooled and then diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 2/3 to 3/2). Thus, tert-butyl (3-{3-cyano-2-(2-furoylamino)-6-[2-(methoxymethoxy)phenyl]pyridin-4-yl}-2-methoxyphenyl)carbamate (25 mg, 27%) was given as a green oil. Also, N-{4-(3-amino-2-methoxyphenyl)-3-cyano-6-[2-(methoxymethoxy)phenyl]pyridin-2-yl}-2-furamide (60 mg, 71%) was given as a pale yellow amorphous solid.
tert-butyl (3-{3-cyano-2-(2-furoylamino)-6-[2-(methoxymethoxy)phenyl]pyridin-4-yl}-2-methoxyphenyl)carbamate
¹H-NMR (300MHz, DMSO-d₆) δ: 1.55 (9H, s), 3.43 (3H, s), 3.54 (3H, s), 5.25 (2H, s), 6.61 (1H, q, J = 1.8 Hz), 7.08 (1H, dd, J = 7.5, 1.5 Hz), 7.15-7.26 (4H, m), 7.38-7.45 (2H, m), 7.58 (1H, t, J = 0.9 Hz), 7.98-8.09 (2H, m), 8.27 (1H, d, J = 8.1 Hz), 8.82 (1H, s).
N-{4-(3-amino-2-methoxyphenyl)-3-cyano-6-[2-(methoxymethoxy)phenyl]pyridin-2-yl}-2-furamide
¹H-NMR (300MHz, DMSO-d₆) δ: 3.43 (3H, s), 3.52 (3H, s), 3.90-4.10 (1H, br), 5.24 (2H, s), 6.58-6.60 (1H, m), 6.80-6.89 (1H, m), 7.09-7.29 (4H, m), 7.37-7.45 (2H, m), 7.57 (1H, t, J = 0.9 Hz), 7.84 (0.5H, s), 7.98-8.09 (2H, m), 8.40-8.44 (0.5H, m), 8.85 (0.5H, s), 8.93 (0.5H, s).

### Reference Example 212

### tert-butyl {3-[(3-{3-cyano-2-(2-furoylamino)-6-[2-(methoxymethoxy)phenyl] pyridin-4-yl}-2-methoxyphenyl)amino]-3-oxopropyl}carbamate

From N-{4-(3-amino-2-methoxyphenyl)-3-cyano-6-[2-(methoxymethoxy) phenyl]pyridin-2-yl}-2-furamide (60 mg, 0.13 mmol) of Reference Example 211, the title compound (40 mg, 48%) was given as a white crystal in substantially the same manner as in Reference Example 15.
¹H-NMR (300MHz, CDCl₃) δ: 1.44 (9H, s), 2.67 (2H, t, J = 5.7 Hz), 3.43 (3H, s), 3.50-3.55 (5H, m), 5.10-5.20 (1H, br), 5.25 (2H, s), 6.61 (1H, q, J = 1.8 Hz), 7.16-7.30 (4H, m), 7.39-7.46 (2H, m), 7.59 (1H, d, J = 0.6 Hz), 7.98-8.00 (3H, m), 8.51 (1H, d, J = 7.8 Hz), 8.82 (1H, s).
Melting point: 194-195°C
Reference Example 213

### Methyl 2-fluoro-5-formylbenzoate

A mixture of the compound of Reference Example 170 (15.0 g, 46.0 mmol) and DMF (30 mL) was stirred at 140°C for 5 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate/hexane = 1/5) to give the title compound (1.37 g, 16%) as a pale yellow powder.
¹H-NMR (300MHz, CDCl₃) δ: 3.98 (3H, s), 7.32 (1H, t, J = 9.3 Hz), 8.07-8.12 (1H, m), 8.49 (1H, dd, J = 6.9, 2.1 Hz), 10.01 (1H, s).

### Reference Example 214

### Methyl 5-{2-amino-3-cyano-6-[4-fluoro-2-(methoxymethoxy)phenyl]pyridin-4-yl}-2-fluorobenzoate

From the compound of Reference Example 132 (1.49 g, 7.52 mmol) and the title compound of Reference Example 213 (1.37 g, 7.52 mmol), the compound (970 mg, 30%) was givenas a white amorphous solid in substantially the same manner as in Reference Example 2.
¹H-NMR (300MHz, CDCl₃) δ: 3.46 (3H, s), 3.97 (3H, s), 5.22 (2H, s), 5.33 (2H, br s), 6.85 (1H, td, J = 8.4, 2.1 Hz), 6.98 (1H, dd, J = 10.8, 2.4 Hz), 7.26-7.34 (2H, m), 7.78-7.86 (2H, m), 8.19 (1H, dd, J = 6.6, 2.4 Hz).

### Reference Example 215

### Methyl 5-[3-cyano-6-[4-fluoro-2-(methoxymethoxy)phenyl]-2-(2-furoylamino) pyridin-4-yl]-2-fluorobenzoate

From the compound of Reference Example 214 (500 mg, 1.18 mmol) and 2-furoyl chloride (384 mg, 2.94 mmol), the title compound (490 mg, 80%) was given as a white amorphous solid in substantially the same manner as in Reference Example 59.
¹H-NMR (300MHz, CDCl₃) δ: 3.48 (3H, s), 3.98 (3H, s), 5.27 (2H, s), 6.23 (1H, q, J = 1.8 Hz), 6.88 (1H, td, J = 8.4, 2.4 Hz), 7.01 (1H, dd, J = 10.8, 2.4 Hz), 7.32-7.41 (2H, m), 7.60 (1H, d, J = 1.8 Hz), 7.91-8.04 (3H, m), 8.24-8.27 (1H, m), 8.80 (1H, s).

### Reference Example 216

### 5-[3-cyano-6-[4-fluoro-2-(methoxymethoxy)phenyl]-2-(2-furoylamino)pyridin-4-yl]-2-fluorobenzoic acid

From the compound of Reference Example 215 (490 mg, 0.94 mmol), the title compound (450 mg, 95%) was given as a pale green amorphous solid in substantially the same manner as in Reference Example 7.
¹H-NMR (300MHz, DMSO-d₆) δ: 3.37 (3H, s), 5.36 (2H, s), 6.77 (1H, q, J = 1.8 Hz), 7.05 (1H, td, J = 8.4, 1.8 Hz), 7.17 (1H, dd, J = 11.4, 2.4 Hz), 7.55 (1H, d, J=2.4 Hz), 7.60 (1H, d, J = 10.2 Hz), 7.95-8.05 (4H, m), 8.19 (1H, dd, J = 6.6, 2.4 Hz), 11.33 (1H, s), 13.55-13.66 (1H, br).

### Reference Example 217

### tert-butyl [2-({5-[3-cyano-6-[4-fluoro-2-(methoxymethoxy)phenyl]-2-(2-furoylamino) pyridin-4-yl]-2-fluorobenzoyl}amino)ethyl]carbamate

From the compound of Reference Example 216 (150 mg, 0.30 mmol), the title compound (155 mg, 80%) was given as a white crystal in substantially the same manner as in Reference Example 15.
¹H-NMR (300MHz, DMSO-d₆) δ: 1.34 (9H, s), 3.10-3.15 (2H, m), 3.29-3.60 (5H, m), 5.35 (2H, s), 6.75 (1H, q, J = 1.8 Hz), 6.89 (1H, t, J = 5.4 Hz), 7.05 (1H, td, J = 8.4, 2.4 Hz), 7.17 (1H, dd, J = 11.4,2.4 Hz), 7.51-7.60 (2H, m), 7.87-8.02 (5H, m), 8.49 (1H, br s), 11.33 (1H, br s).
Melting point: 179-181°C

### Reference Example 218

### tert-butyl {5-[3-cyano-6-[4-fluoro-2-(methoxymethoxy)phenyl]-2-(2-furoylamino) pyridin-4-yl]-2-fluorophenyl}carbamate

A mixture of the compound of Reference Example 216 (300 mg, 0.59 mmol), diphenylphosphoryl azide (196 mg, 0.71 mmol) and triethylamine (144 mg, 1.42 mmol) in toluene (20 mL) was stirred at room temperature for 5 hours, and then tert-butanol (8 mL) was added and stirred at 110°C for 2 hours. The reaction mixture was cooled and then diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/2) to give the title compound (230 mg, 68%) as a white amorphous solid.
¹H-NMR (300MHz, CDCl₃) δ: 1.55 (9H, s), 3.52 (3H, s), 5.36 (2H, s), 6.61 (1H, q, J = 1.8 Hz), 6.84-6.90 (2H, m), 7.04 (1H, dd, J = 10.8, 2.4 Hz), 7.21-7.27 (1H, m), 7.39 (1H, d, J = 3.6 Hz), 7.44-7.49 (1H, m), 7.59 (1H, t, J = 0.9 Hz), 8.02-8.08 (2H, m), 8.51 (1H, dd, J = 7.5, 2.1 Hz), 8.80 (1H, s).

### Reference Example 219

### tert-butyl 2-[2-((3-(3-cyano-6-(4-fluoro-2-(methoxymethoxy)phenyl)-2-(2-furoylamino) pyridin-4-yl)phenyl)amino)-2-oxoethyl]piperidine-1-carboxylate

WSCD (100 mg, 0.52 mmol) was added to a solution of the compound of Reference Example 135 (190 mg, 0.41 mmol),
[1-(tert-butoxycarbonyl)piperidine-2-yl]acetic acid (130 mg, 0.53 mmol) and HOBt (71 mg, 0.53 mmol) in DMF (3 mL) at room temperature, and stirred at room temperature for 3 days. The resultant solution was diluted with ethyl acetate, and washed with water and then with saturated brine. The organic layer was dried over magnesium sulfate and filtered, and then the solvent was removed under reduced pressure. The residue was purified by silica gel column chromatography (eluting solution: hexane to ethyl acetate) and by basic silica gel column chromatography (eluting solution: hexane to ethyl acetate). The residue was recrystallized from hexane-ethyl acetate to give the title compound (230 mg, 82%) as a pale yellow powder.
¹H-NMR (300MHz, CDCl₃) δ:1.43 (9H, s), 1.52-1.76 (6H, m), 2.57 (1H, dd, J = 5.4 Hz, 16.2 Hz), 2.83-2.96 (2H, m), 3.47 (3H, s), 3.98 (1H, d, J = 14.7 Hz), 4.89 (1H, br s), 5.31 (2H, s), 6.61 (1H, dd, J = 3.3, 1.8 Hz), 6.84-6.91 (1H, m), 7.03 (1H, dd, J = 10.8, 2.4 Hz), 7.38 (1H, dd, J = 3.6, 0.9 Hz), 7.43-7.50 (2H, m), 7.53-7.61 (2H, m), 7.97 (1H, s), 8.02 (1H, dd, J = 9.0, 6.9 Hz), 8.16 (1H, br s), 8.81 (1H, br s), 9.01 (1H, br s).

### Reference Example 220

### 2-amino-4-(3-aminophenyl)-6-[4-fluoro-2-(methoxymethoxy)phenyl]nicotinonitrile

From the compound of Reference Example 133 (3.0 g, 7.61 mmol), the title compound (2.55 g, 92%) was given as a yellow amorphous solid in substantially the same manner as in Reference Example 4.
¹H-NMR (300MHz, CDCl₃) δ: 3.44 (3H, s), 3.82 (2H, br s), 5.20 (2H, s), 5.28 (2H, br s), 6.77-7.00 (5H, m), 7.26-7.31 (2H, m), 7.77 (1H, dd, J = 8.7, 6.9 Hz).

### Reference Example 221

### tert-butyl (2R)-2-{[(3-{2-amino-3-cyano-6-[4-fluoro-2-(methoxymethoxy)phenyl] pyridin-4-yl}phenyl)amino]carbonyl}pyrrolidine-1-carboxylate

From the compound of Reference Example 220 (1.0 g, 2.77 mmol) and Boc-D-proline (1.18 g, 5.49 mmol), the title compound (1.38 g, 89%) was given as a white amorphous solid in substantially the same manner as in Reference Example 130.
¹H-NMR (300MHz, CDCl₃) δ: 1.50 (10H, s), 1.80-2.05 (2H, br), 2.45-2.60 (1H, br), 3.30-3.50 (5H, m), 4.40-4.55 (1H, br), 5.24 (2H, s), 5.30 (2H, br s), 6.80-6.86 (1H, m), 6.99 (1H, dd, J = 10.8, 2.4 Hz), 7.35-7.45 (4H, m), 7.78 (1H, dd, J = 8.7, 6.9 Hz), 8.02 (1H, s), 9.65-9.80 (1H, br).

### Reference Example 222

### tert-butyl (2R)-2-[({3-[3-cyano-6-[4-fluoro-2-(methoxymethoxy)phenyl]-2-(2-furoylamino)pyridin-4-yl]phenyl }amino)carbonyl]pyrrolidine-1-carboxylate

From the compound of Reference Example 221 (200 mg, 0.36 mmol) and 2-furoyl chloride (116 mg, 0.89 mmol), the title compound (190 mg, 80%) was given as a colorless amorphous solid in substantially the same manner as in Reference Example 59.
¹H-NMR (300MHz, CDCl₃) δ: 1.51 (10H, s), 1.85-2.00 (2H, br), 2.45-2.65 (1H, br), 3.30-3.50 (5H, m), 4.40-4.55 (1H, br), 5.32 (2H, s), 6.61 (1H, q, J = 1.8 Hz), 6.84-6.91 (1H, m), 7.03 (1H, dd, J = 10.8,2.4 Hz), 7.39 (1H, dd, J = 6.6, 0.6 Hz), 7.48 (3H, br s), 7.59-7.61 (1H, m), 7.98 (1H, s), 8.03 (1H, dd, J = 8.7, 6.9 Hz), 8.12 (1H, s), 8.81 (1H, s), 9.65-9.85 (1H, br).

### Reference Example 223

### tert-butyl (2R)-2- {[(3-{3-cyano-6-[4-fluoro-2-(methoxymethoxy)phenyl]-2-[(1,3-thiazole-2-ylcarbonyl)amino]pyridin-4-yl}phenyl)amino]carbonyl}pyrrolidine-1-carboxylate

From the compound of Reference Example 221 (200 mg, 0.36 mmol) and 1,3-thiazole-2-carbonyl chloride (131 mg, 0.89 mmol), the title compound (210 mg, 87%) was given as a colorless amorphous solid in substantially the same manner as in Reference Example 59.
¹H-NMR (300MHz, CDCL₃) δ: 1.51 (10H, s), 1.85-2.00 (2H, br), 2.45-2.65 (1H, br), 3.30-3.50 (5H, m), 4.40-4.55 (1H, br), 5.32 (2H, s), 6.86-6.92 (1H, m), 7.03 (1H, dd, J = 10.8, 2.4 Hz), 7.50 (3H, br s), 7.71 (1H, d, J = 3.0 Hz), 8.00-8.03 (2H, m), 8.08-8.14 (2H, m), 9.65-9.80 (1H, br), 9.92 (1H, s).

### Reference Example 224

### tert-butyl (2R)-2-{[(3-{3-cyano-6-[4-fluoro-2-(methoxymethoxy)phenyl]-2-[(isoxazole-5-ylcarbonyl)amino]pyridin-4-yl}phenyl)amino]carbonyl}pyrrolidine-1-carboxylate

From the compound of Reference Example 221 (200 mg, 0.36 mmol) and 5-isoxazole carbonyl chloride (117 mg, 0.89 mmol), the title compound (170 mg, 72%) was given as a pale green amorphous solid in substantially the same manner as in Reference Example 59.
¹H-NMR (300MHz, CDCl₃) δ: 1.50 (10H, s), 1.85-2.00 (2H, br), 2.40-2.60 (1H, br), 3.30-3.50 (5H, m), 4.40-4.55 (1H, br), 5.33 (2H, s), 6.83-6.90 (1H, m), 7.04 (1H, dd, J = 10.8,2.1 Hz), 7.18 (1H, d, J = 1.8 Hz), 7.44 (3H, br s), 7.97-8.05 (2H, m), 8.14 (1H, s), 8.43 (1H, d, J = 1.5 Hz), 9.06 (1H, s), 9.70-9.80 (1H, br).

### Reference Example 225

### tert-butyl {3-[(3-{6-[4-chloro-2-(methoxymethoxy)phenyl]-3-cyano-2-[(3-methyl-2-furoyl)amino]pyridin-4-yl}phenyl)amino]-3-oxopropyl}carbamate

From the compound of Reference Example 129 (1.05 g, 2.77 mmol) and Boc-D-proline (1.18 g, 5.49 mmol), the title compound (1.58 g, 99%) was given as a white amorphous solid in substantially the same manner as in Reference Example 130.
¹H-NMR (300MHz, CDCl₃) δ: 1.50 (10H, s), 1.85-2.00 (2H, br), 2.45-2.65 (1H, br), 3.30-3.50 (5H, m), 4.40-4.55 (1H, br), 5.24 (2H, s), 5.31 (2H, br s), 7.10 (1H, dd, J = 8.4, 1.8 Hz), 7.26-7.45 (5H, m), 7.749 (1H, d, J = 8.4 Hz), 8.02 (1H, s), 9.65-9.80 (1H, br).

### Reference Example 226

### tert-butyl (2R)-2-{[(3-{6-[4-chloro-2-(methoxymethoxy)phenyl]-3-cyano-2-[(1,3-thiazole-2-ylcarbonyl)amino]pyridin-4-yl}phenyl)amino]carbonyl}pyrrolidine-1-carboxylate

From the compound of Reference Example 225 (200 mg, 0.35 mmol) and 1,3-thiazole-2-carbonyl chloride (128 mg, 0.86 mmol), the title compound (160 mg, 66%) was given as a pale yellow amorphous solid in substantially the same manner as in Reference Example 59.
¹H-NMR (300MHz, CDCl₃) δ: 1.50 (10H, s), 1.85-2.10 (2H, br), 2.45-2.65 (1H, br), 3.30-3.50 (5H, m), 4.40-4.55 (1H, br), 5.33 (2H, s), 7.15 (1H, dd, J = 8.4, 1.8 Hz), 7.30 (1H, d, J = 2.1 Hz), 7.46 (3H, br s), 7.72 (1H, d, J = 3.3 Hz), 7.99-8.09 (3H, m), 8.13 (1H, s), 9.65-9.80 (1H, br), 9.93 (1H, s).

### Reference Example 227

### tert-butyl (2R)-2-{[(3-{6-[4-chloro-2-(methoxymethoxy)phenyl]-3-cyano-2-[(isoxazole-5-ylcarbonyl)amino]pyridin-4-yl}phenyl)amino]carbonyl}pyrrolidine-1-carboxylate

From the compound of Reference Example 225 (200 mg, 0.35 mmol) and 5-isoxazolecarbonyl chloride (114 mg, 0.86 mmol), the title compound (170 mg, 72%) was given as a pale yellow amorphous solid in substantially the same manner as in Reference Example 59.
¹H-NMR (300MHz, CDCl₃) δ: 1.50 (10H, s), 1.85-2.05 (2H, br), 2.40-2.60 (1H, br), 3.30-3.55 (5H, m), 4.40-4.60 (1H, br), 5.33 (2H, s), 7.04-7.05 (2H, m), 7.15 (1H, d, J = 1.8 Hz), 7.44 (3H, br s), 7.94 (1H, d, J = 8.4 Hz), 8.06 (1H, s), 8.14 (1H, s), 8.43 (1H, d, J = 1.5 Hz), 9.09 (1H, s), 9.70-9.80 (1H, br).

### Reference Example 228

### tert-butyl (2R)-2-[({3-[6-[4-chloro-2-(methoxymethoxy)phenyl]-3-cyano-2-(2-furoylamino)pyridin-4-yl]phenyl}amino)carbonyl]pyrrolidine-1-carboxylate

From the compound of Reference Example 225 (200 mg, 0.35 mmol) and 2-furoyl chloride (113 mg, 0.86 mmol), the title compound (170 mg, 72%) was given as a colorless amorphous solid in substantially the same manner as in Reference Example 59.
¹H-NMR (300MHz, CDCl₃) δ:1.50 (10H, s), 1.85-2.00 (2H, br), 2.40-2.60 (1H, br), 3.30-3.50 (5H, m), 4.40-4.55 (1H, br), 5.32 (2H, s), 6.06 (1H, q, J = 1.8 Hz), 7.14 (1H, dd, J = 8.4, 1.8 Hz), 7.30 (1H, d, J = 1.8 Hz), 7.35-7.45 (4H, m), 7.58 (1H, d, J = 0.9 Hz), 7.96-8.02 (2H, m), 8.12 (1H, s), 8.84 (1H, s), 9.65-9.85 (1H, br).

### Reference Example 229

### 1-[2-(methoxymethoxy)phenyl]propane-1-one

From o-hydroxypropiophenone (28.74 g, 0.19 mol), the title compound (30.15 g, 82%) was given as a colorless oil in substantially the same manner as in Reference Example 1.
¹H-NMR (300MHz, CDCl₃) δ: 1.18 (3H, t, J = 7.2 Hz), 3.01 (2H, q, J = 7.2 Hz), 3.51 (3H, s), 5.27 (2H, s), 7.05 (1H, td, J = 7.5, 0.6 Hz), 7.18 (1H, dd, J = 8.4,0.6 Hz), 7.42 (1H, td, J = 7.5, 1.8 Hz), 7.65 (1H, dd, J = 7.5, 1.8 Hz).

### Reference Example 230

### 2-amino-6-[2-(methoxymethoxy)phenyl]-5-methyl-4-(3-nitrophenyl)nicotinonitrile

From the compound of Reference Example 229 (3.0 g, 15.45 mmol), the title compound (2.26 g, 37%) was given as a pale yellow crystal in substantially the same manner as in Reference Example 2.
¹H-NMR (300MHz, CDCl₃) δ: 1.82 (3H, s), 3.42 (3H, s), 5.17 (2H, s), 5.25 (2H, s), 7.13 (1H, t, J = 6.6 Hz), 7.23-7.28 (2H, m), 7.37-7.42 (1H, m), 7.72-7.74 (2H, m), 8.25 (1H, br s), 8.34-8.37 (1H, m).
Melting point: 154-155°C

### Reference Example 231

### N-[3-cyano-6-[2-(methoxymethoxy)phenyl]-5-methyl-4-(3-nitrophenyl)pyridin-2-yl]-2-furamide

From the compound of Reference Example 230 (500 mg, 1.28 mmol) and 2-furoyl chloride (418 mg, 3.20 mmol), the title compound (620 mg, 100%) was given as a white amorphous solid in substantially the same manner as in Reference Example 134.
¹H-NMR (300MHz, DMSO-d₆) δ: 2.00 (3H, s), 3.42 (3H, s), 5.17 (2H, s), 6.59 (1H, q, J = 1.8 Hz), 7.16 (1H, td, J = 7.5, 0.9 Hz), 7.26-7.37 (3H, m), 7.41-7.47 (1H, m), 7.55 (1H, q, J = 0.9 Hz), 7.74-7.82 (2H, m), 8.32 (1H, s), 8.37-8.41 (1H, m), 8.71 (1H, s).

### Reference Example 232

### N-{4-(3-aminophenyl)-3-cyano-6-[2-(methoxymethoxy)phenyl]-5-methylpyridin-2-yl}-2-furamide

From the compound of Reference Example 231 (600 mg, 1.24 mmol), the title compound (520 mg, 92%) was given as a colorless amorphous solid in substantially the same manner as in Reference Example 4.
¹H-NMR (300MHz, CDCl₃) δ: 2.00 (3H, s), 3.38 (3H, s), 3.89 (2H, br s), 5.13 (2H, s), 6.53 (1H, q, J = 1.8 Hz), 6.69-6.77 (3H, m), 7.12 (1H, td, J = 7.8, 0.9 Hz), 7.22-7.42 (5H, m), 7.50 (1H, s), 8.82 (1H, s).

### Reference Example 233

### tert-butyl {3-[(3-{3-cyano-2-(2-furoylamino)-6-[2-(methoxymethoxy)phenyl]-5-methylpyridin-4-yl}phenyl)amino]-3-oxopropyl}carbamate

From the compound of Reference Example 232 (250 mg, 0.55 mmol) and Boc-β-alanine(208 mg, 1.10 mmol), the title compound (250 mg, 73%) was given as a white amorphous solid in substantially the same manner as in Reference Example 38.
¹H-NMR (300MHz, CDCl₃) δ: 1.44 (9H, s), 2.01 (3H, s), 2.61 (2H, t, J = 5.7 Hz), 3.41 (3H, s), 3.49 (2H, q, J = 5.9 Hz), 5.19 (2H, s), 5.20-5.30 (1H, br), 6.57 (1H, q, J = 1.8 Hz), 7.12-7.16 (2H, m), 7.24-7.35 (3H, m), 7.38-7.53 (4H, m), 7.65-7.85 (1H, m), 7.97 (1H, br s), 8.70 (1H, s).

### Reference Example 234

### tert-butyl [(1S)-2-({3-[3-cyano-6-[4-fluoro-2-(methoxymethoxy)phenyl]-2-(2-furoylamino)pyridin-4-yl]phenyl}amino)-1-(methoxymethyl)-2-oxoethyl]carbamate

From the compound of Reference Example 135 (300 mg, 0.63 mmol) and N-Boc-O-methylserine dicyclohexylammonium (524 mg, 1.31 mmol), the title compound (268 mg, 63%) was given as a white crystal in substantially the same manner as in Reference Example 38.
¹H-NMR (300MHz, CDCl₃) δ: 1.49 (9H, s), 3.44 (3H, s), 3.47 (3H, s), 3.57 (1H, dd, J = 9.3, 6.6 Hz), 3.92 (1H, dd, J = 9.3, 2.4 Hz), 4.30-4.45 (1H, br), 5.31 (2H, s), 5.40-5.50 (1H, br), 6.61 (1H, q, J = 1.8 Hz), 6.85-6.91 (1H, m), 7.03 (1H, dd, J = 10.8, 2.1 Hz), 7.39 (1H, dd, J = 3.6, 0.9 Hz), 7.50-7.61 (4H, m), 7.98-8.05 (3H, m), 8.50-8.60 (1H, br), 8.81 (1H, s).
Melting point: 152-153°C

### Reference Example 235

### tert-butyl [(2S)-2-[(tert-butoxycarbonyl)amino]-3-({3-[3-cyano-6-[4-fluoro-2-(methoxymethoxy)phenyl]-2-(2-furoylamino)pyridin-4-yl]phenyl}amino)-3-oxopropyl] carbamate

From the compound of Reference Example 135 (300 mg, 0.63 mmol) and N-(tert-butoxycarbonyl)-3-[(tert-butoxycarbonyl)amino]-L-alanine dicyclohexylammonium (636 mg, 1.31 mmol), the title compound (246 mg, 68%) was given as a white crystal in substantially the same manner as in Reference Example 38.
¹H-NMR (300MHz, CDCl₃) δ:1.46 (9H, s), 1.56 (9H, s), 3.47 (3H, s), 3.52-3.69 (2H, m), 4.16-4.32 (1H, m), 5.15-5.25 (1H, br), 5.30 (2H, s), 5.85-5.95 (1H, br), 6.62 (1H, q, J = 1.8 Hz), 6.85-6.91 (1H, m), 7.02 (1H, dd, J = 10.8, 2.4 Hz), 7.39 (1H, dd, J = 3.6, 0.6 Hz), 7.48-7.52 (3H, m), 7.59-7.61 (1H, m), 7.97 (1H, s), 8.05 (1H, dd, J = 8.7, 6.9 Hz), 8.10 (1H, s), 8.81 (1H, s), 9.15-9.30 (1H, br).
Melting point: 159-161°C

### Reference Example 236

### 2-amino-4-(3-bromophenyl)-6-[4-fluoro-2-(methoxymethoxy)phenyl]nicotinonitrile

From the compound of Reference Example 132 (5.0 g, 25.27 mmol) and 3-bromobenzaldehyde(4.68 g, 25.27 mmol), the title compound (3.28 g, 30%) was given as a yellow crystal in substantially the same manner as in Reference Example 133.
¹H-NMR (300MHz, CDCl₃) δ: 3.46 (3H, s), 5.22 (2H, s), 5.34 (2H, br s), 6.81-6.88 (1H, m), 6.98 (1H, dd, J = 10.8,2.4 Hz), 7.29 (1H, s), 7.39 (1H, t, J = 7.8 Hz), 7.57-7.65 (2H, m), 7.73 (1H, t, J = 1.8 Hz), 7.79 (1H, dd, J = 8.7, 6.9 Hz).
Melting point: 165-166°C

### Reference Example 237

### N- {4-(3-bromophenyl)-3-cyano-6-[4-fluoro-2-(methoxymethoxy)phenyl]pyridin-2-yl}-2-furamide

From the compound of Reference Example 236 (2.20 g, 5.14 mmol) and 2-furoyl chloride (1.68 g, 12.84 mmol), the title compound (2.41 g, 90%) was given as a white amorphous solid in substantially the same manner as in Reference Example 134.
¹H-NMR (300MHz, CDCl₃) δ: 3.49 (3H, s), 5.27 (2H, s), 6.62 (1H, q, J = 1.8 Hz), 6.85-6.91 (1H, m), 7.01 (1H, dd, J = 10.8, 1.8 Hz), 7.39-7.46 (2H, m), 7.60 (1H, d, J = 0.9 Hz), 7.66-7.69 (2H, m), 7.80 (1H, t, J = 1.8 Hz), 7.90 (1H, s), 8.01 (1H, t, J = 7.8 Hz), 8.81 (1H, s).

### Reference Example 238

### tert-butyl 4-{3-[3-cyano-6-[4-fluoro-2-(methoxymethoxy)phenyl]-2-(2-furoylamino) pyridin-4-yl]phenyl}piperazine-1-carboxylate

Tris(dibenzylideneacetone)dipalladium(0) (53 mg, 0.057 mmol) was added to a mixture of the compound of Reference Example 237 (300 mg, 0.57 mmol), 1-Boc-piperazine(128 mg, 0.69 mmol), 2-dicyclohexylphosphino-2',4',6'-tri-isopropyl-1,1'-biphenyl (55 mg, 0.11 mmol) and sodium tert-butoxide (83 mg, 0.86 mmol) in toluene (15 mL), and refluxed while heating for 2 hours. After cooling, the reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: hexane/ethyl acetate = 1/1) to give the title compound (300 mg, 84%) as pale brown amorphous solid.
¹H-NMR (300MHz, CDCl₃) δ: 1.49 (9H, s), 3.24 (4H, t, J = 5.1 Hz), 3.45 (3H, s), 3.61 (4H, t, J = 5.1 Hz), 5.20 (2H, s), 6.61 (1H, q, J = 1.8 Hz), 6.84-6.90 (1H, m), 6.98-7.14 (3H, m), 7.27 (1H, s), 7.39-7.46 (2H, m), 7.58 (1H, t, J = 0.9 Hz), 7.93 (1H, s), 8.00 (1H, dd, J = 8.7, 6.9 Hz), 8.84(1H, s).

### Reference Example 239

### N-{3-cyano-6-[4-fluoro-2-(methoxymethoxy)phenyl]-4-[3-(4-methylpiperazine-1-yl) phenyl]pyridin-2-yl}-2-furamide

From the compound of Reference Example 237 (300 mg, 0.57 mmol) and N-methylpiperazine (69 mg, 0.69 mmol), the title compound (103 mg, 53%) was given as a pale yellow powder in substantially the same manner as in Reference Example 238.
¹H-NMR (300MHz, CDCl₃) δ: 2.37 (3H, s), 2.60 (4H, t, J = 5.1 Hz), 3.32 (4H, t, J = 5.1 Hz), 3.46 (3H, s), 5.24 (2H, s), 6.61 (1H, q, J = 1.8 Hz), 6.85-6.91 (1H, m), 7.01 (1H, dd, J = 10.8, 2.4 Hz), 7.06-7.12 (2H, m), 7.24-7.26 (1H, m), 7.39-7.45 (2H, m), 7.59 (1H, s), 7.93 (1H, s), 8.00 (1H, dd, J = 8.7, 7.2 Hz), 8.81 (1H, s).

### Reference Example 240

### N-{3-cyano-4-[3-(4-ethylpiperazine-1-yl)phenyl]-6-[4-fluoro-2-(methoxymethoxy) phenyl]pyridin-2-yl}-2-furamide

From the compound of Reference Example 237 (300 mg, 0.57 mmol) and N-ethylpiperazine (79 mg, 0.69 mmol), the title compound (94 mg, 30%) was given as a pale yellow powder in substantially the same manner as in Reference Example 238.
¹H-NMR (300MHz, CDCl₃) δ: 1.14 (3H, t, J = 7.2 Hz), 2.49 (2H, q, J = 7.2 Hz), 2.63 (4H, t, J = 5.1 Hz), 3.30 (4H, t, J = 5.1 Hz), 3.46 (3H, s), 5.24 (2H, s), 6.61 (1H, q, J = 1.8 Hz), 6.71-6.91 (1H, m), 7.00 (1H, dd, J = 10.8,2.4 Hz), 7.06-7.12 (2H, m), 7.23-7.26 (1H, m), 7.38-7.45 (2H, m), 7.59 (1H, q, J = 0.9 Hz), 7.93 (1H, s), 8.00 (1H, dd, J = 8.7, 6.6 Hz), 8.81 (1H, s).

### Reference Example 241

### N-[3-cyano-6-[4-fluoro-2-(methoxymethoxy)phenyl]-4-(3-morpholine-4-ylphenyl) pyridin-2-yl]-2-furamide

From the compound of Reference Example 237 (300 mg, 0.57 mmol) and morpholine (60 mg, 0.69 mmol), the title compound (250 mg, 83%) was given as a pale yellow amorphous solid in substantially the same manner as in Reference Example 238.
¹H-NMR (300MHz, CDCl₃) δ: 3.25 (4H, t, J = 4.7 Hz), 3.45 (3H, s), 3.88 (4H, t, J = 4.7 Hz), 5.23 (2H, s), 6.58-6.60 (1H, m), 6.82-6.88 (1H, m), 6.97-7.07 (2H, m), 7.12 (1H, d, J = 7.2 Hz), 7.25 (1H, s), 7.37-7.45 (2H, m), 7.57 (1H, s), 7.94-8.02 (2H, m), 8.88 (1H, s).

### Reference Example 242

### N-[3-cyano-6-[4-fluoro-2-(methoxymethoxy)phenyl]-4-(3-piperidine-1-ylphenyl) pyridin-2-yl]-2-furamide

From the compound of Reference Example 237 (300 mg, 0.57 mmol) and piperidine (59 mg, 0.69 mmol), the title compound (230 mg, 77%) was given as a pale yellow amorphous solid in substantially the same manner as in Reference Example 238.
¹H-NMR (300MHz, CDCl₃) δ: 1.56-1.65 (2H, m), 1.66-1.76 (4H, m), 3.26 (4H, t, J = 5.4 Hz), 3.45 (3H, s), 5.23 (2H, s), 6.59 (1H, q, J = 1.8 Hz), 6.83-6.89 (1H, m), 6.98-7.08 (3H, m), 7.23 (1H, d, J = 1.8 Hz), 7.36-7.41 (2H, m), 7.57 (1H, s), 7.93 (1H, s), 8.00 (1H, dd, J = 8.7, 6.9 Hz), 8.85 (1H, s).

### Reference Example 243

### tert-butyl 4-{3-[3-cyano-6-[4-fluoro-2-(methoxymethoxy)phenyl]-2-(2-furoylamino) pyridin-4-yl]phenyl}-1,4-diazepan-1-carboxylate

From the compound of Reference Example 237 (300 mg, 0.57 mmol) and N-Boc-homopiperazine (138 mg, 0.69 mmol), the title compound (155 mg, 42%) was given as a pale yellow powder in substantially the same manner as in Reference Example 238.
¹H-NMR (300MHz, CDCl₃) δ: 1.39 (4H, s), 1.44 (5H, s), 2.00-2.15 (2H, m), 3.20-3.40 (2H, m), 3.46 (3H, s), 3.58-3.70 (6H, br s), 5.25 (2H, s), 6.61 (1H, q, J = 1.8 Hz), 6.83-6.95 (3H, m), 6.99-7.03 (2H, m), 7.34-7.39 (2H, m), 7.59 (1H, t, J = 0.9 Hz), 7.94 (1H, s), 8.03 (1H, t, J = 7.8 Hz), 8.81 (1H, s).

### Reference Example 244

### tert-butyl [2-({3-[3-cyano-6-[4-fluoro-2-(methoxymethoxy)phenyl]-2-(2-furoylamino) pyridin-4-yl]phenyl}amino)ethyl]carbamate

Sodium triacetoxycyanoborate (462 mg, 2.18 mmol) was added to a mixture of the compound of Reference Example 135 (500 mg, 1.09 mmol), N-2-Boc-acetaminoaldehyde (347 mg, 2.18 mmol) and acetic acid (1 mL) in THF (10 mL), and stirred for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: hexane/ethyl acetate = 1/1), and then recrystallized from diethylether to give the title compound (395 mg, 60%) as a yellow crystal.
¹H-NMR (300MHz, CDCl₃) δ: 1.45 (9H, s), 3.33-3.52 (7H, m), 4.11 (1H, br s), 4.34 (1H, br s), 5.25 (2H, s), 6.61 (1H, q, J = 1.8 Hz), 6.75 (1H, dd, J = 8.1, 1.8 Hz), 6.85-7.03 (4H, m), 7.26 (1H, s), 7.32 (1H, t, J = 7.8 Hz), 7.38 (1H, dd, J = 3.0, 0.6 Hz), 7.92 (1H, s), 8.01 (1H, dd, J = 8.4, 6.6 Hz), 8.80 (1H, s).
Melting point: 145-146°C

### Reference Example 245

### tert-butyl {2-[{3-[3-cyano-6-[4-fluoro-2-(methoxymethoxy)phenyl]-2-(2-furoylamino) pyridin-4-yl]phenyl}(trifluoroacetyl)amino]ethyl}carbamate

A solution of trifluoroacetic anhydride (146 mg, 0.69 mmol) in THF (5 mL) was added to a solution of the compound of Reference Example 244 (380 mg, 0.63 mmol) and triethylamine (70 mg, 0.69 mmol) in THF (20 mL) with ice-cooling, and stirred for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: hexane/ethyl acetate = 1/1) to give the title compound (360 mg, 82%) as a white amorphous solid.
¹H-NMR (300MHz, CDCl₃) δ: 1.37 (9H, s), 3.35-3.50 (5H, m), 3.95 (2H, t, J = 5.4 Hz), 4.80-4.95 (1H, br), 5.25 (2H, s), 6.63 (1H, q, J = 1.8 Hz), 6.85-6.92 (1H, m), 7.03 (1H, dd, J = 10.8, 2.4 Hz), 7.40 (1H, d, J = 3.6 Hz), 7.53-7.68 (4H, m), 7.79 (1H, d, J = 7.5 Hz), 7.97 (1H, s), 8.03 (1H, t, J = 7.5 Hz), 8.82 (1H, s).

### Reference Example 246

### 2-[4-{3-[[2-(acetylamino)ethyl](trifluoroacetyl)amino]phenyl}-5-cyano-6-(2-furoylamino)pyridin-2-yl]-5-fluorophenyl acetate

Acetic anhydride (26 mg, 0.25 mmol) was added to a solution of the compound of Example 340 (60 mg, 0.10 mmol) and triethylamine (36 mg, 0.36 mmol) in THF (5 mL) at room temperature and stirred for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate) to give the title compound (60 mg, 94%) as a white amorphous solid.
¹H-NMR (300MHz, CDCl₃) δ: 1.78 (3H, s), 2.19 (3H, s), 3.50-3.60 (2H, m), 3.95-4.15 (2H, m), 6.14 (1H, br s), 6.62-6.64 (1H, m), 6.98 (1H, dd, J = 9.0, 2.4 Hz), 7.12 (1H, td, J = 8.1, 2.4 Hz), 7.40-7.78 (7H, m), 7.88-7.92 (1H, m), 8.90 (1H, s).

### Reference Example 247

### N-{3-cyano-4-[3-({[2-(dimethylamino)ethyl]amino}carbonyl)phenyl]-6-[4-fluoro-2-(methoxymethoxy)phenyl]pyridin-2-yl}-2-furamide

From the mixture of Reference Example 135 (200 mg, 0.41 mmol) and N,N-dimethylethylenediamine (43 mg, 0.49 mmol), the title compound (90 mg, 39%) was given as a pale brown amorphous solid in substantially the same manner as in Reference Example 78.
¹H-NMR (300MHz, CDCl₃) δ: 2.29 (6H, s), 2.56 (2H, t, J = 5.7 Hz), 3.47 (3H, s), 3.56 (2H, q, J = 5.5 Hz), 5.27 (2H, s), 6.61 (1H, q, J = 1.8 Hz), 6.84-6.90 (1H, m), 7.01 (1H, dd, J = 10.8, 2.4 Hz), 7.13 (1H, br s), 7.39 (1H, dd, J = 3.6, 0.6 Hz), 7.59-7.64 (2H, m), 7.83-7.86 (1H, m), 7.95-8.01 (3H, m), 8.15 (1H, d, J = 1.5 Hz), 8.70-9.10 (1H, br).

### Reference Example 248

### N-{ 3-cyano-4-[3-({[2-(diethylamino)ethyl]amino}carbonyl)phenyl]-6-[4-fluoro-2-(methoxymethoxy)phenyl]pyridin-2-yl}-2-furamide

From the mixture of Reference Example 135 (200 mg, 0.41 mmol) and N,N,-diethylethylenediamine (57 mg, 0.49 mmol), the title compound (100 mg, 42%) was given as a pale yellow amorphous solid in substantially the same manner as in Reference Example 78.
¹H-NMR (300MHz, CDCl₃) δ: 1.05 (6H, t, J = 7.2 Hz), 2.59 (4H, q, J = 7.2 Hz), 2.68 (2H, t, J = 5.7 Hz), 3.46 (3H, s), 3.52 (2H, q, J = 5.7 Hz), 5.26 (2H, s), 6.60 (1H, q, J = 1.8 Hz), 6.82-6.87 (1H, m), 7.00 (1H, dd, J = 10.8, 2.4 Hz), 7.26 (1H, br s), 7.38 (1H, dd, J = 4.2, 0.9 Hz), 7.58-7.64 (2H, m), 7.83-8.01 (4H, m), 8.16 (1H, d, J = 1.8 Hz).

### Reference Example 249

### N-{3-cyano-6-[4-fluoro-2-(methoxymethoxy)phenyl]-4-[3-(hydroxymethyl)phenyl] pyridin-2-yl} -2-furamide

From the compound of Reference Example 138 (200 mg, 0.41 mmol), the title compound (120 mg, 63%) was given as a pale yellow amorphous solid in substantially the same manner as in Reference Example 8.
¹H-NMR (300MHz, CDCl₃) δ: 3.45 (3H, s), 4.80 (2H, s), 5.24 (2H, s), 6.60 (1H, q, J = 1.8 Hz), 6.83-6.90 (1H, m), 7.00 (1H, dd, J = 10.5,2.4 Hz), 7.38 (1H, dd, J = 3.3, 0.6 Hz), 7.51-7.62 (4H, m), 7.70 (1H, s), 7.92 (1H, s), 7.96 (1H, dd, J = 8.7, 6.9 Hz), 8.84 (1H, br s).

### Reference Example 250

### 3-[3-cyano-6-[4-fluoro-2-(methoxymethoxy)phenyl]-2-(2-furoylamino)pyridin-4-yl] benzyl methanesulfonate

Methanesulfonyl chloride (44 mg, 0.38 mmol) was added to a solution of the compound of Reference Example 249 (90 mg, 0.19 mmol) and triethylamine (38 mg, 0.38 mmol) in dichloromethane (5 mL) at room temperature and stirred for 1 hour.
The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate/hexane = 1/1) to give the title compound (60 mg, 57%) as a white amorphous solid.
¹H-NMR (300MHz, CDCl₃) δ: 3.02 (3H, s), 3.47 (3H, s), 5.27 (2H, s), 5.34 (2H, s), 6.62 (1H, q, J = 1.8 Hz), 6.85-6.92 (1H, m), 7.02 (1H, dd, J = 10.5,2.4 Hz), 7.40 (1H, dd, J = 3.6, 0.6 Hz), 7.60-7.61 (3H, m), 7.72-7.76 (2H, m), 7.94 (1H, s), 8.02 (1H, dd, J = 8.7, 6.9 Hz), 8.81 (1H, s).

### Reference Example 251

### tert-butyl 4-{3-[3-cyano-6-[4-fluoro-2-(methoxymethoxy)phenyl]-2-(2-furoylamino) pyridin-4-yl]benzyl}piperazine-1-carboxylate

A solution of the compound of Reference Example 250 (60 mg, 0.11 mmol), 1-Boc-piperazine (24 mg, 0.13 mmol) and triethylamine(14 mg, 0.13 mmol) in THF (5 mL) was stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate/hexane = 1/1 to 2/1) to give the title compound (40 mg, 57%) as a pale yellow amorphous solid.
¹H-NMR (300MHz, CDCl₃) δ: 1.45 (9H, s), 2.44 (4H, s), 3.46 (7H, s), 3.61 (2H, s), 5.25 (2H, s), 6.62 (1H, q, J = 1.8 Hz), 6.89-6.91 (1H, m), 7.01 (1H, dd, J = 10.8, 2.4 Hz), 7.40 (1H, q, J = 2.4 Hz), 7.49-7.60 (4H, m), 7.72 (1H, s), 7.92 (1H, s), 8.00 (1H, dd, J = 8.7, 6.6 Hz), 8.82 (1H, s).

### Reference Example 252

### tert-butyl [2-((3-(3-cyano-6-(4-fluoro-2-(methoxymethoxy)phenyl)-2-(2-furoylamino) pyridin-4-yl)phenyl)amino)-2-oxoethyl]methylcarbamate

WSCD (420 mg, 2.2 mmol) was added to a solution of the compound of Reference Example 135 (500 mg, 1.1 mmol), N-(tert-butoxycarbonyl)-N-methylglycine (410 mg, 2.2 mmol) and HOBt (290 mg, 2.1 mmol) in DMF (5 mL) at room temperature, and stirred at room temperature for 3 days. The resultant solution was diluted with ethyl acetate, and washed with saturated sodium hydrogencarbonate solution, water, and then saturated brine. The organic layer was dried over magnesium sulfate and filtered, and then the solvent was removed under reduced pressure. The residue was purified by silica gel column chromatography (eluting solution: hexane to ethyl acetate/hexane = 1/1) and by amino silica gel column chromatography (eluting solution: hexane to ethyl acetate), and concentrated. The residue was recrystallized from hexane-ethyl acetate to give the title compound (540 mg, 78%) as a colorless powder.
¹H-NMR (300MHz, CDCl₃) δ: 1.51 (9H, s), 3.03 (3H, s), 3.48 (3H, s), 4.00 (2H, s), 5.31 (2H, s), 6.61 (1H, dd, J = 3.6, 1.8 Hz), 6.84-6.90 (1H, m), 7.03 (1H, dd, J = 10.8, 2.4 Hz), 7.39 (1H, dd, J = 3.3, 0.6 Hz), 7.45 (3H, br s), 7.59-7.61 (1H, m), 7.97-8.05 (3H, m), 8.60 (1H, br s), 8.82 (1H, br s).
Melting point: 162-164°C

### Reference Example 253

### tert-butyl [3-((3-(3-cyano-6-(4-fluoro-2-(methoxymethoxy)phenyl)-2-(2-furoylamino) pyridin-4-yl)phenyl)amino)-3-oxopropyl]methylcarbamate

WSC (420 mg, 2.2 mmol) was added to a solution of the compound of Reference Example 135 (500 mg, 1.1 mmol), N-(tert-butoxycarbonyl)-N-methyl-β-alanine (440 mg, 2.2 mmol) and HOBt (290 mg, 2.1 mmol) DMF (5 mL) at room temperature, and stirred at room temperature for 3 days. The resultant solution was diluted with ethyl acetate, and washed with saturated sodium hydrogencarbonate solution, water, and then saturated brine. The organic layer was dried over magnesium sulfate and filtered, and then the solvent was removed under reduced pressure. The residue was purified by silica gel column chromatography (eluting solution: hexane to ethyl acetate/hexane = 1/1) and by basic silica gel column chromatography (eluting solution: hexane to ethyl acetate), and concentrated. The residue was recrystallized from hexane-ethyl acetate to give the title compound (530 mg, 75%) as a colorless powder.
¹H-NMR (300MHz, CDCl₃) δ: 1.46 (9H, s), 2.69 (2H, br s), 2.91 (3H, s), 3.47 (3H, s), 3.64 (2H, t, J = 6.3 Hz), 5.31 (2H, s), 6.61 (1H, dd, J = 3.3,1.5 Hz), 6.84-6.91 (1H, m), 7.03 (1H, dd, J = 10.8, 2.4 Hz), 7.38 (1H, dd, J = 3.6, 0.6 Hz), 7.44-7.51 (2H, m), 7.59-7.60 (2H, m), 7.96 (1H, s), 8.02 (1H, dd, J = 8.4, 6.9 Hz), 8.14 (1H, br s), 8.82 (1H, br s), 9.07 (1H, br s).
Melting point: 172-174°C

### Reference Example 254

### N-[4-(3-((chloroacetyl)amino)phenyl)-3-cyano-6-(4-fluoro-2-(methoxymethoxy)phenyl) pyridin-2-yl]-2-furamide

Chloroacetyl chloride (0.10 ml, 1.3 mmol) was added to a solution of the compound of Reference Example 135 (0.50 g, 1.1 mmol) and N,N-diisopropylethylamine (0.17 g, 1.3 mmol) in THF (5 mL) with ice-cooling, and stirred with ice-cooling for 3 hours. The reaction mixture was diluted with ethyl acetate, washed with water and then saturated brine, dried over magnesium sulfate, and then concentrated. The residue was purified by silica gel column chromatography (eluting solution: ethyl acetate) and then recrystallized from hexane-ethyl acetate to give the title compound (0.55 g, 93%) as a pale yellow crystal.
¹H-NMR (300MHz, CDCl₃) δ: 3.47 (3H, s), 4.23 (2H, d, J = 2.1 Hz), 5.30 (2H, s), 6.62 (1H, dd, J = 3.3,1.5 Hz), 6.84-6.91 (1H, m), 7.03 (1H, dd, J = 10.8,2.4 Hz), 7.40 (1H, dd, J = 3.6, 0.9 Hz), 7.53-7.66 (4H, m), 7.97 (1H, s), 8.00-8.05 (2H, m), 8.38 (1H, br s), 8.82 (1H, br s).
Melting point: 174-176°C

### Reference Example 255

### N-[3-cyano-4-(3-((N,N-diethylglycyl)amino)phenyl)-6-(4-fluoro-2-(methoxymethoxy) phenyl)pyridin-2-yl]-2-furamide

Diethylamine (0.12 ml, 1.2 mmol) was added to a solution of the compound of Reference Example 254 (0.30 g, 0.56 mmol) and potassium carbonate (0.15 g, 1.1 mmol) in DMF (2 mL) at room temperature, and stirred at room temperature for 16 hours. The reaction mixture was diluted with ethyl acetate, washed twice with water and then once with saturated brine, dried over magnesium sulfate, and then concentrated under reduced pressure. The residue was purified by amino silica gel column chromatography (eluting solution: hexane to ethyl acetate) and then recrystallized from hexane-ethyl acetate to give the title compound (0.23 g, 72%) as a colorless crystal.
¹H-NMR (300MHz, CDCl₃) δ: 1.11 (6H, t, J = 7.2 Hz), 2.68 (4H, q, J = 7.2 Hz), 3.17 (2H, s), 3.47 (3H, s), 5.30 (2H, s), 6.62 (1H, dd, J = 3.6, 1.8 Hz), 6.84-6.91 (1H, m), 7.03 (1H, dd, J = 10.8, 2.4 Hz), 7.39 (1H, dd, J = 3.6, 0.9 Hz), 7.45-7.54 (2H, m), 7.59-7.60 (1H, m), 7.67-7.70 (1H, m), 7.98-8.05 (3H, m), 8.82 (1H, s), 9.58 (1H, br s). Melting point: 126-128°C

### Reference Example 256

### N-(3-{2-amino-3-cyano-6-[4-fluoro-2-(methoxymethoxy)phenyl]pyridin-4-yl}phenyl)-N²,N²-dimethylglycinamide

From the compound of Reference Example 220 (450 mg, 1.23 mmol) and N,N-dimethylglycine (255 mg, 2.47 mmol), the title compound (430 mg, 78%) was given as a colorless amorphous solid in substantially the same manner as in Reference Example 130.
¹H-NMR (300MHz, CDCl₃) δ: 2.39 (6H, s), 3.10 (2H, s), 3.45 (3H, s), 5.24 (2H, s), 5.29 (2H, br s), 6.81-6.87 (1H, m), 6.99 (1H, dd, J = 10.8, 1.8 Hz), 7.36-7.40 (2H, m), 7.48 (1H, t, J = 7.8 Hz), 7.63-7.66 (1H, m), 7.78 (1H, dd, J = 8.7, 6.9 Hz), 7.96 (1H, t, J = 1.8 Hz), 9.24 (1H, br s).

### Reference Example 257

### N-{3-cyano-4-{3-[(N,N-dimethylglycyl)amino]phenyl}-6-[4-fluoro-2-(methoxyrnethoxy)phenyl]pyridin-2-yl}-2-furamide

From the compound of Reference Example 256 (210 mg, 0.47 mmol) and 2-furoyl chloride (152 mg, 1.17 mmol), the title compound (118 mg, 46%) was given as a white crystal in substantially the same manner as in Reference Example 59.
¹H-NMR (300MHz, DMSO-d₆) δ: 2.41 (6H, s), 3.11 (2H, s), 3.47 (3H, s), 5.30 (2H, s), 6.62 (1H, q, J = 1.8 Hz), 6.84-6.91 (1H, m), 7.02 (1H, dd, J = 10.8, 2.4 Hz), 7.39 (1H, dd, J = 3.6, 0.9 Hz), 7.45-7.59 (2H, m), 7.60 (1H, s), 7.73-7.77 (1H, m), 7.98-8.04 (3H, m), 8.81 (1H, s), 9.30 (1H, s).
Melting point: 168-169°C

### Reference Example 258

### tert-butyl 4-{3-[3-cyano-6-[4-fluoro-2-(methoxymethoxy)phenyl]-2-(2-furoylamino) pyridin-4-yl]benzyl}-1,4-diazepan-1-carboxylate

From the compound of Reference Example 250 (100 mg, 0.18 mmol) and N-Boc-homopiperazine (54 mg, 0.27 mmol), the title compound (80 mg, 68%) was given as a pale yellow amorphous solid in substantially the same manner as in Reference Example 251.
¹H-NMR (300MHz, DMSO-d₆) δ: 1.45 (9H, s), 1.75-1.94 (2H, br), 2.62-2.78 (4H, m), 3.43-3.55 (7H, m), 3.72 (2H, s), 5.25 (2H, s), 6.61 (1H, q, J = 1.8 Hz), 6.84-6.90 (1H, m), 7.00 (1H, dd, J = 10.8,2.4 Hz), 7.39 (1H, d, J = 3.6 Hz), 7.49-7.59 (4H, m), 7.69 (1H, s), 7.92 (1H, s), 8.00 (1H, dd, J = 8.7, 6.9 Hz), 8.85 (1H, s).

### Reference Example 259

### tert-butyl 6-[4-fluoro-2-(methoxymethoxy)phenyl]-2-(2-furoylamino)-4-(3-nitrophenyl)nicotinate

From the compound of Reference Example 132 (20.7 g, 0.10 mol), tert-butyl cyanoacetate (14.77 g, 0.10 mol), tert-butyl 2-amino-6-[4-fluoro-2-(methoxymethoxy)phenyl]-4-(3-nitrophenyl)nicotinate (5.12 g, 11 %) was given as a pale yellow amorphous solid in substantially the same manner as in Reference Example 2.

From the resultant tert-butyl 2-amino-6-[4-fluoro-2-(methoxymethoxy)phenyl]-4-(3-nitrophenyl)nicotinate (1.50 g, 3.20 mmol) and 2-furoyl chloride (1.04 g, 7.99 mmol), the title compound (1.64 g, 91%) was given as a white amorphous solid in substantially the same manner as in Reference Example 134.
¹H-NMR (300MHz, CDCl₃) δ: 1.22 (9H, s), 3.43 (3H, s), 5.21 (2H, s), 6.59 (1H, q, J = 1.8 Hz), 6.86-6.98 (2H, m), 7.31 (1H, dd, J = 3.3, 0.9 Hz), 7.60 (1H, q, J = 0.9 Hz), 7.65-7.72 (3H, m), 8.18 (1H, dd, J = 8.7, 6.9 Hz), 8.26 (1H, t, J = 1.5 Hz), 8.30-8.34 (1H, m), 10.42 (1H, s).

### Reference Example 260

### N- {3-[(dimethylamino)methyl]-6-[4-fluoro-2-(methoxymethoxy)phenyl]-4-(3-nitrophenyl)pyridin-2-yl}-2-furamide

Lithium tetrahydroborate (77 mg, 3.55 mmol) was added to a solution of the compound of Reference Example 259 (1.0 g, 1.77 mmol) in THF (30 mL), and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate/hexane = 3/2 to 2/1) to give N-[6-[4-fluoro-2-(methoxymethoxy)phenyl]-3-(hydroxymethyl)-4-(3-nitrophenyl) pyridin-2-yl]-2-furamide (240 mg, 27%) as a yellow amorphous solid.

Methanesulfonyl chloride (168 mg, 1.46 mmol) was added to a solution of the resultant N-[6-[4-fluoro-2-(methoxymethoxy)phenyl]-3-(hydroxymethyl)-4-(3-nitrophenyl) pyridin-2-yl]-2-furamide(240 mg, 0.49 mmol) and triethylamine(148 mg, 1.46 mmol) in dichloromethane (15 mL) with ice-cooling, and stirred at room temperature for 24 hours. The reaction mixture was diluted with saturated aqueous sodium hydrogencarbonate, and extracted with ethyl acetate. The extract was washed with saturated brine, dried over magnesium sulfate, and then concentrated under reduced pressure to give [6-[4-fluoro-2-(methoxymethoxy)phenyl]-2-(2-furoylamino)-4-(3-nitrophenyl) pyridin-3-yl]methyl methanesulfonate (270 mg, 100%) as a pale yellow amorphous solid.

A solution of the resultant [6-[4-fluoro-2-(methoxymethoxy)phenyl]-2-(2-furoylamino)-4-(3-nitrophenyl) pyridin-3-yl]methyl methanesulfonate (130 mg, 0.25 mmol), dimethylammonium chloride (31 mg, 0.38 mmol) and triethylamine (76 mg, 1.00 mmol) in THF (10 mL) was stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel column chromatography (eluting solution: ethyl acetate/hexane = 1/1) to give the title compound (100 mg, 77%) as a pale yellow amorphous solid.
¹H-NMR (300MHz, CDCl₃) δ: 2.32 (6H, s), 3.44 (3H, s), 3.48 (2H, s), 5.21 (2H, s), 6.57 (1H, q, J = 1.8 Hz), 6.84-6.96 (2H, m), 7.26 (1H, t, J = 1.8 Hz), 7.51 (1H, t, J = 0.9 Hz), 7.59-7.71 (3H, m), 8.19-8.34 (3H, m), 12.28 (1H, s).

### Reference Example 261

### N-[3-cyano-6-[4-fluoro-2-(methoxymethoxy)phenyl]-4-{3-[(piperidine-1-ylacetyl) amino]phenyl}pyridin-2-yl]-2-furamide

Piperidine (0.095 ml, 0.96 mmol) was added to a solution of the compound of Reference Example 254 (0.25 g, 0.47 mmol) and potassium carbonate (0.13 g, 0.94 mmol) in DMF (2 mL) at room temperature, and stirred at room temperature for 17 hours. The reaction mixture was diluted with ethyl acetate, washed twice with water and once with saturated brine, dried over magnesium sulfate, and then concentrated under reduced pressure. The residue was purified by basic silica gel column chromatography (eluting solution: hexane to ethyl acetate), and then recrystallized from hexane-ethyl acetate to give the title compound (0.19 g, 69%) as a colorless crystal.
¹H-NMR (300MHz, CDCl₃) δ: 1.51 (2H, brs), 1.69 (4H, brs), 2.59 (4H, brs), 3.13 (2H, brs), 3.47 (3H, s), 5.31 (2H, s), 6.62 (1H, dd, J = 1.5 Hz, 3.6 Hz), 6.84-6.91 (1H, m), 7.03 (1H, dd, J = 2.4 Hz, 10.8 Hz), 7.39 (1H, dd, J = 0.6 Hz, 3.3 Hz), 7.47-7.54 (2H, m), 7.59 (1H, dd, J = 0.6 Hz, 1.5 Hz), 7.66 (1H, d, J = 6.9 Hz), 7.98-8.05 (3H, m), 8.82 (1H, brs), 9.46 (1H, brs).
Melting point: 140-142°C

### Example 1

### N-[4-[3-(aminomethyl)phenyl]-3-cyano-6-(2-hydroxyphenyl)pyridin-2-yl]thiophene-2-carboxamide

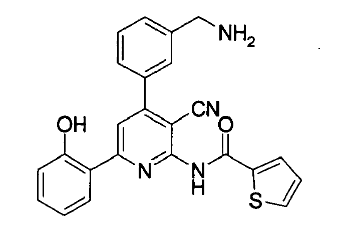

Concentrated hydrochloric acid (0.5 mL) was added to a solution of the compound of Reference Example 12 (140 mg, 0.30 mmol) in THF (10 mL), and stirred at room temperature for 1 hour. The reaction mixture was diluted with saturated aqueous sodium hydrogencarbonate, and extracted with ethyl acetate twice. The extracts were combined, washed with saturated brine, dried over magnesium sulfate, and then concentrated under reduced pressure. The residue was purified by amino silica gel chromatography (eluting solution: ethyl acetate/methanol = 50/1 to 20/1), and then recrystallized from ethyl acetate to give the title compound (26 mg, 20%) as pale yellow crystals.
¹H-NMR (300MHz, DMSO-d₆) δ : 4.04 (2H, s), 6.89 (2H, t, J = 8.9 Hz), 7.15 (1H, t, J = 4.4 Hz), 7.33 (1H, t, J = 7.1 Hz), 7.49-7.86 (7H, m), 7.52-7.55 (1H, m), 8.00 (1H, d, J = 6.6 Hz).
Melting point: 203-205°C
Element analysis: Calculated (%) for C₂₄H₁₈N₄O₂S·0.1H₂O: C, 67.31; H, 4.28; N. 13.08. Found (%): C, 67.23; H, 4.21; N, 12.90.

### Example 2

### N-[3-cyano-4-{3-[(dimethylamino)methyl]phenyl}-6-(2-hydroxyphenyl)pyridin-2-yl] thiophene-2-carboxamide

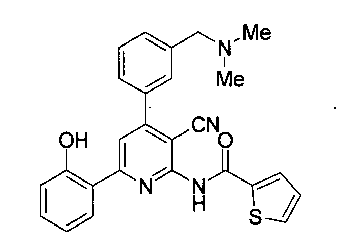

From the compound of Reference Example 14 (90 mg, 0.18 mmol), the title compound (29 mg, 35%) was given as a pale yellow crystal in substantially the same manner as in Example 1.
¹H-NMR (300MHz, DMSO-d₆) δ : 2.23 (6H, s), 3.55 (2H, s), 6.95-7.01 (2H, m), 7.29-7.32 (1H, m), 7.40 (1H, t, J = 7.7 Hz), 7.51-7.68 (4H, m), 7.99 (1H, d, J = 4.8 Hz), 8.11-8.17 (3H, m), 11.20-11.55 (1H, br), 11.90-12.20 (1H, br).
Melting point: 225-226°C
Element analysis: Calculated (%) for C₂₆H₂₂N₄O₂S·0.1H₂: C, 68.43; H, 4.90; N. 12.28. Found (%): C, 68.31; H, 4.93; N, 12.07.

### Example 3

### N-[4-(3-{[(2-aminoethyl)amino]carbonyl}phenyl)-3-cyano-6-(2-hydroxyphenyl) pyridin-2-yl]thiophene-2-carboxamide hydrochloride

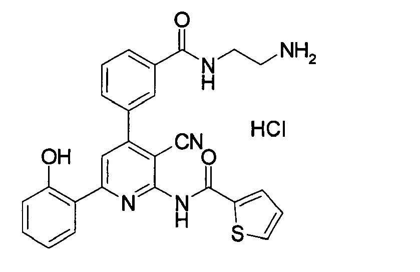

A 4N hydrogen chloride-ethyl acetate solution (5 mL) was added to a solution of the compound of Reference Example 15 (100 mg, 0.17 mmol) in ethyl acetate (10 mL), and stirred at room temperature for 3 hours. The reaction mixture was concentrated, and the residue was recrystallized from methanol-diethylether to give the title compound (83 mg, 88%) as a pale green crystal.
¹H-NMR (300MHz, DMSO-d₆) δ:3.03 (2H, t, J = 6.0 Hz), 3.54-3.60 (2H, m), 6.96-7.04 (2H, m), 7.30-7.33 (1H, m), 7.40 (1H, t, J = 7.7 Hz), 7.75 (1H, t, J = 7.7 Hz), 7.91-8.03 (5H, m), 8.11-8.28 (5H, m), 8.96 (1H, t, J = 5.4 Hz), 11.40-11.60 (1H, br), 11.90 (1H, br s).
Melting point: 203-210°C
Element analysis: Calculated (%) for C₂₆H₂₁N₅O₃S·1.2HCl·0.9H₂O: C, 57.45; H, 4.45; N. 12.89. Found (%): C, 57.49; H, 4.81; N, 12.89.

### Example 4

### N-[4-(3-{[(3-aminopropyl)amino]carbonyl}phenyl)-3-cyano-6-(2-hydroxyphenyl) pyridin-2-yl]thiophene-2-carboxamide hydrochloride

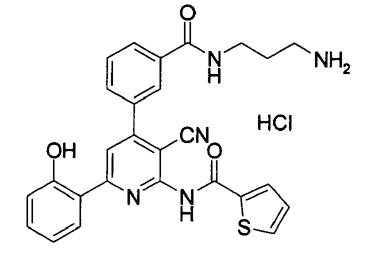

From the compound of Reference Example 16 (110 mg, 0.17 mmol), the title compound (91 mg, 94%) was given as a yellow powder in substantially the same manner as in Example 3.
¹H-NMR (300MHz, DMSO-d₆) δ : 1.80-1.91 (2H, m), 2.83-2.89 (2H, m), 3.39 (2H, q, J = 6.3 Hz), 6.96-7.04 (2H, m), 7.31 (1H, dd, J = 5.1, 3.9 Hz), 7.41 (1H, t, J = 7.7 Hz), 7.74 (1H, t, J = 7.7 Hz), 7.87-7.96 (4H, m), 8.02 (1H, dd, J = 5.1, 0.9 Hz), 8.09 (1H, d, J = 7.8 Hz), 8.15-8.28 (3H, m), 8.90 (1H, s), 8.92 (1H, t, J = 5.6 Hz), 11.50 (1H, s), 11.85-12.00 (1H, br).
Melting point: 192-198°C
Element analysis: Calculated (%) for C₂₇H₂₃N₅O₃S·1.0HCl·1.1H₂O: C, 58.55; H, 4.77; N, 12.64; Cl, 6.40. Found (%): C, 58.71; H, 5.11; N, 12.44; Cl, 6.40.

### Example 5

### N-[4-{3-[(4-aminopiperidine-1-yl)carbonyl]phenyl}-3-cyano-6-(2-hydroxyphenyl) pyridin-2-yl]thiophene-2-carboxamide hydrochloride

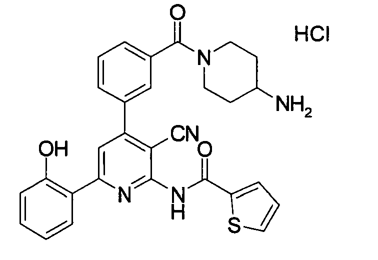

From the compound of Reference Example 17 (140 mg, 0.21 mmol), the title compound (104 mg, 83%) was given as a yellow powder in substantially the same manner as in Example 3.
¹H-NMR (300MHz, DMSO-d₆) δ:1.38-1.60 (2H, m), 1.85-2.08 (2H, m), 2.73-3.75 (4H, m), 4.40-4.60 (1H, br), 6.96-7.04 (2H, m), 7.32 (1H, t, J = 5.1 Hz), 7.40 (1H, t, J = 7.7 Hz), 7.61 (1H, d, J = 7.8 Hz), 7.70-7.76 (2H, m), 7.85 (1H, d, J = 7.8 Hz), 8.01-8.18 (6H, m), 8.25 (1H, s), 11.50 (1H, s), 11.80-11.90 (1H, br s).
Melting point: 215-218°C
Element analysis: Calculated (%) for C₂₉H₂₅N₅O₃S·1.0HCl·1.3H₂O: C, 59.70; H, 4.94; N, 12.00; Cl, 6.08. Found (%): C, 59.88; H, 5.32; N, 12.12; Cl, 5.87.

### Example 6

### N- {3-cyano-6-(2-hydroxyphenyl)-4-[3-(piperazine-1-ylcarbonyl)phenyl]pyridin-2-yl} thiophene-2-carboxamide hydrochloride

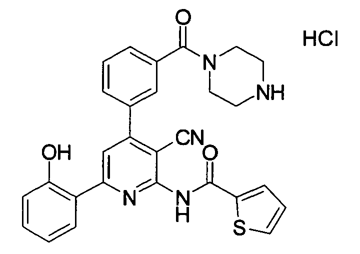

From the compound of Reference Example 18 (260 mg, 0.40 mmol), the title compound (175 mg, 75%) was given as a yellow powder in substantially the same manner as in Example 3.
¹H-NMR (300MHz, DMSO-d₆) δ:3.10-3.30 (4H, br), 3.60-3.90 (4H, br), 6.97-7.04 (2H, m), 7.30-7.33 (1H, m), 7.38-7.46 (1H, m), 7.69-7.77 (2H, m), 7.83-7.88 (2H, m), 8.01 (1H, d, J = 4.2 Hz), 8.15-8.18 (2H, m), 8.24 (1H, s), 9.10-9.30 (2H, br), 11.45-11.60 (1H, br), 11.80 (1H, s).
Melting point: 232-236°C
Element analysis: Calculated (%) for C₂₈H₂₃N₅O₃S·1.0HC1·1.8H₂O: C, 58.13; H, 4.81; N, 12.11; Cl, 6.13. Found (%): C, 58.02; H, 5.08; N, 12.00; Cl, 6.01.

### Example 7

### N-(3-cyano-6-(2-hydroxyphenyl)-4-{3-[(piperidin-4-ylamino)carbonyl]phenyl} pyridin-2-yl)thiophene-2-carboxamide hydrochloride

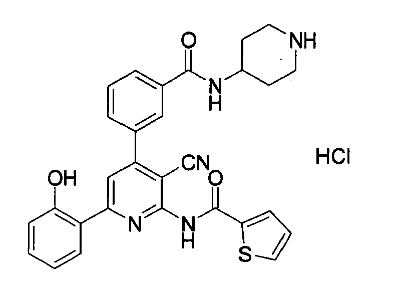

From the compound of Reference Example 19 (190 mg, 0.28 mmol), the title compound (157 mg, 94%) was given as a yellow powder in substantially the same manner as in Example 3.
¹H-NMR (300MHz, DMSO-d₆) δ : 1.77 (2H, q, J = 10.9 Hz), 2.01 (2H, d, J = 12.3 Hz), 3.03 (2H, q, J = 10.9 Hz), 3.24-3.41 (2H, m), 4.05-4.20 (1H, m), 6.96-7.03 (2H, m), 7.30-7.33 (1H, m), 7.41 (1H, t, J = 7.7 Hz), 7.73 (1H, d, J = 7.8 Hz), 7.90 (1H, d, J = 8.1 Hz), 8.01-8.19 (5H, m), 8.28 (1H, s), 8.60-8.80 (3H, m), 11.50 (1H, s), 11.89 (1H, s).
Melting point: 254-256°C
Element analysis: Calculated (%) for C₂₉H₂₅N₅O₃S·1.0HCl·1.8H₂O: C, 58.79; H, 5.04; N, 11.82; Cl, 5.98. Found (%): C, 58.51; H, 5.03; N, 12.09; Cl, 6.35.

### Example 8

### N-(3-cyano-6-(2-hydroxyphenyl)-4-{3-[(piperidin-3-ylamino)carbonyl]phenyl}pyridin-2-yl)thiophene-2-carboxamide hydrochloride

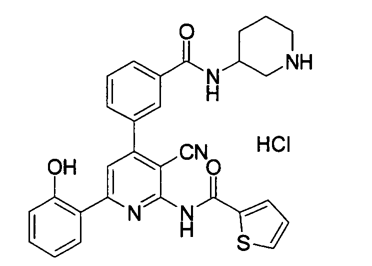

A 4N hydrogen chloride-ethyl acetate solution (5 mL) was added to a solution of the compound of Reference Example 22 (200 mg, 0.30 mmol) in dichloromethane (5 mL), and stirred at room temperature for 1 hour. The precipitated crystals were collected by filtration and dried under reduced pressure to give the title compound (172 mg, 100%) as a yellow crystal.
¹H-NMR (300MHz, DMSO-d₆) δ:1.55-1.75 (2H, m), 1.91-1.99 (2H, m), 2.85-2.90 (2H, m), 3.16-3.21 (1H, br), 3.30-3.50 (1H, m), 4.15-4.30 (1H, br), 6.96-7.03 (2H, m), 7.30-7.33 (1H, m), 7.38-7.43 (1H, m), 7.75 (1H, t, J = 7.8 Hz), 7.92 (1H, d, J = 7.8 Hz), 8.01 (1H, dd, J = 5.1, 1.8 Hz), 8.09-8.22 (4H, m), 8.29 (1H, s), 8.75-8.84 (2H, m), 8.90-9.00 (1H,br), 11.50 (1H, s), 11.91 (1H, br s).
Melting point: 232-233°C
Element analysis: Calculated (%) for C₂₉H₂₅N₅O₃S·1.0HCl·1.4H₂O: C, 59.51; H, 4.96; N. 11.97; Cl, 6.06. Found (%): C, 59.41; H, 5.10; N, 11.67; Cl, 6.09.

### Example 9

### N-(3-cyano-6-(2-hydroxyphenyl)-4-{3-[(piperidin-3-ylainino)carbonyl]phenyl} pyridin-2-yl)-1-benzothiophene-2-carboxamide hydrochloride

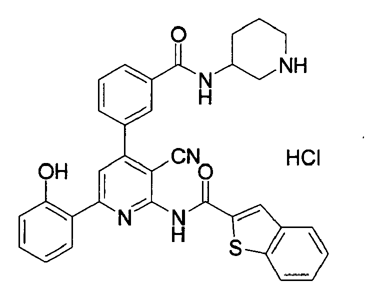

From the compound of Reference Example 23 (150 mg, 0.21 mmol), the title compound (130 mg, 100%) was given as a yellow crystal in substantially the same manner as in Example 8.
¹H-NMR (300MHz, DMSO-d₆) δ:1.60-1.75 (2H, m), 1.91-1.99 (2H, m), 2.80-2.95 (2H, m), 3.16-3.19 (1H, br), 3.33-3.45 (1H, br), 4.20-4.30 (1H, br), 6.97-7.04 (2H, m), 7.39-7.44 (1H, m), 7.50-7.59 (2H, m), 7.76 (1H, t, J = 7.8 Hz), 7.93 (1H, d, J = 7.8 Hz), 8.08-8.14 (3H, m), 8.21 (1H, s), 8.23 (1H, s), 8.32 (1H, s), 8.51 (1H, s), 8.78-8.90 (2H, m), 8.95-9.10 (1H, br), 11.79 (1H, s), 11.85-11.95 (1H, br s).
Melting point: 225-229°C
Element analysis: Calculated (%) for C₃₃H₂₇N₅O₃S·1.0HCl·1.8H₂O: C, 61.68; H, 4.96; N, 10.90; Cl, 5.52. Found (%): C, 61.73; H, 4.97; N, 10.71; Cl, 5.60.

### Example 10

### N-(3-cyano-6-(2-hydroxyphenyl)-4-{3-[(piperidin-3-ylamino)carbonyl]phenyl} pyridin-2-yl)pyridin-2-carboxamide dihydrochloride

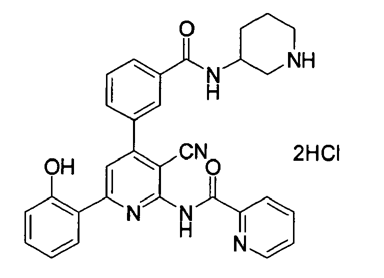

From the compound of Reference Example 24 (80 mg, 0.13 mmol), the title compound (70 mg, 97%) was given as a yellow powder in substantially the same manner as in Example 8.
¹H-NMR (300MHz, DMSO-d₆) δ:1.62-1.75 (2H, m), 1.91-1.95 (2H, m), 2.85-2.95 (2H, m), 3.14-3.19 (1H, br), 3.32-3.40(1H, m), 4.15-4.30 (1H, br), 6.95-7.03 (2H, m), 7.41 (1H, t, J = 7.7 Hz), 7.68-7.77 (2H, m), 7.92 (1H, d, J = 7.8 Hz), 8.13 (1H, d, J = 7.8 Hz), 8.23 (1H, s), 8.24 (1H, s), 8.34 (1H, s), 8.50 (1H, d, J = 8.1 Hz), 8.85-9.00 (3H, m), 9.19-9.25 (2H, m), 11.79 (1H, s), 11.80-12.10 (1H, br).
Melting point: 204-210°C
Element analysis: Calculated (%) for C₃₀H₂₆N₆O₃·1.8HCl·3.2H₂O: C, 56.14; H, 5.40; N, 13.09; Cl, 9.94. Found (%): C, 55.89; H, 5.30; N, 13.12; Cl, 10.02.

### Example 11

### tert-butyl {2-[(3-{3-cyano-6-(2-hydroxyphenyl)-2-[(2-thienylcarbonyl)amino]pyridin-4-yl}phenyl)amino]-2-oxoethyl}carbamate

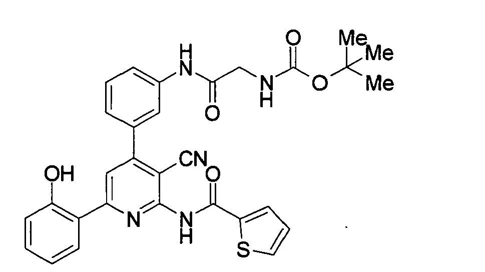

WSCD (94 mg, 0.50 mmol) was added to a solution of the compound of Reference Example 4 (130 mg, 0.25 mmol), Boc-glycine (86 mg, 0.50 mmol) and HOBt (66 mg, 0.50 mmol) in DMF (4 mL) with ice-cooling, and stirred at room temperature for 17 hours. The reaction mixture was diluted with saturated aqueous sodium hydrogencarbonate, and extracted twice with ethyl acetate. The extract liquids were combined, washed with saturated , dried over magnesium sulfate, and then concentrated under reduced pressure. The residue was purified by silica gel chromatography (eluting solution: ethyl acetate/hexane = 1/1 to 3/2) to give a mixture (100 mg) of tert-butyl {2-[(3-{6-(2-{[tert-butyl(dimethyl)silyl]oxy}phenyl)-3-cyano-2-[(2-thienylcarbonyl) amino]pyridin-4-yl}phenyl)amino]-2-oxoethyl}carbamate and the title compound as a pale yellow solid.

A 1M solution of tetrabutylammonium fluoride in THF(1 mL) was added to a solution of the resultant mixture (100 mg) in THF (5 mL)with ice-cooling, and stirred for 1 hour. The reaction mixture was diluted with water and concentrated under reduced pressure. The residue was diluted with ethyl acetate, washed with water and then with saturated brine, dried over magnesium sulfate, and then concentrated under reduced pressure. The residue was purified by silica gel chromatography (eluting solution: ethyl acetate/hexane = 1/1 to 2/1), and then recrystallized from ethyl acetate-diethylether to give the title compound (70 mg, 40%) as a pale yellow crystal.
¹H-NMR (300MHz, CDCl₃) δ:1.56 (9H, s), 3.98 (2H, d, J = 6.0 Hz), 5.20-5.30 (1H, br), 6.90 (1H, t, J = 7.5 Hz), 7.10 (1H, d, J = 7.5 Hz), 7.19 (1H, dd, J = 4.8, 3.9 Hz), 7.35-7.40 (2H, m), 7.50 (1H, t, J = 7.8 Hz), 7.60 (1H, d, J = 8.1 Hz), 7.67-7.69 (2H, m), 7.77-7.81 (2H, m), 7.95 (1H, s), 8.45-8.50 (1H, br), 8.80 (1H, s), 13.15-13.25 (1H, br s).
Melting point: 206-215°C

### Example 12

### N-[3-cyano-4-[3-(glycylamino)phenyl]-6-(2-hydroxyphenyl)pyridin-2-yl]thiophene-2-carboxamide dihydrochloride

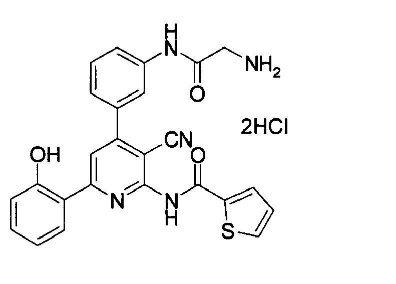

From the compound of Example 11 (60 mg, 0.11 mmol), the title compound (53 mg, 95%) was given as a yellow powder in substantially the same manner as in Example 3.
¹H-NMR (300MHz, DMSO-d₆) δ: 3.85 (2H, t, J = 5.7 Hz), 6.96-7.05 (2H, m), 7.31 (1H, t, J = 2.6 Hz), 7.37-7.49 (2H, m), 7.62 (1H, t, J = 8.0 Hz), 7.83 (1H, d, J = 9.0 Hz), 8.00-8.04 (2H, m), 8.13-8.25 (6H, m), 10.97 (1H, s), 11.09 (1H, s).
Melting point: 210-217°C
Element analysis: Calculated (%) for C₂₅H₁₉N₅O₃S·2.0HCl·0.3H₂O: C, 54.81; H, 3.97; N, 12.78. Found (%): C, 54.65; H, 4.01; N, 12.61.

### Example 13

### tert-butyl {3-[(3-{3-cyano-6-(2-hydroxyphenyl)-2-[(2-thienylcarbonyl)amino]pyridin-4-yl}phenyl)amino]-3-oxopropyl}carbamate

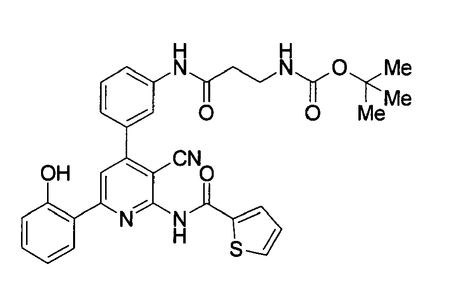

WSCD (109 mg, 0.57 mmol) was added to a solution of the compound of the compound of Reference Example 4 (300 mg, 0.57 mmol), Boc-β-alanine (108 mg, 0.57 mmol) and HOBt (77 mg, 0.57 mmol) in DMF (8 mL) with ice-cooling, and stirred at room temperature for 12 hours. The reaction mixture was diluted with saturated aqueous sodium hydrogencarbonate, and extracted with ethyl acetate. The extract liquids were combined, washed with saturated brine, dried over magnesium sulfate, and then concentrated under reduced pressure. The residue was purified by silica gel chromatography (eluting solution: ethyl acetate/hexane = 1/1 to 2/1), and then recrystallized from chloroform-diethylether to give a mixture of the title compound (132 mg, 40%) as a pale yellow crystal.
¹H-NMR (300MHz, DMSO-d₆) δ : 1.39 (9H, s), 2.40-2.54 (2H, m), 3.22-3.27 (2H, m), 6.89-7.01 (3H, m), 7.28-7.42 (3H, m), 7.54 (1H, t, J = 8.0 Hz), 7.80 (1H, d, J = 8.1 Hz), 7.99 (2H, s), 8.12-8.14 (3H, m), 10.24 (1H, s),11.44 (1H, s), 11.75-11.85 (1H, br s).
Melting point: 230-231°C

### Example 14

### N-{4-[3-(β-alanylamino)phenyl]-3-cyano-6-(2-hydroxyphenyl)pyridin-2-yl} thiophene-2-carboxamide hydrochloride

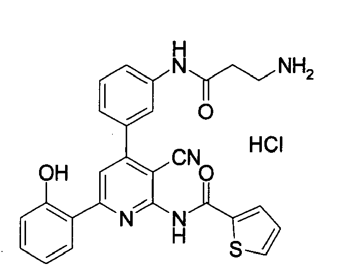

From the compound of Example 13 (100 mg, 0.17 mmol), the title compound (83 mg, 88%) was given as a yellow powder in substantially the same manner as in Example 3.
¹H-NMR (300MHz, DMSO-d₆) δ:2.78 (2H, t, J = 6.8 Hz), 3.10-3.17 (2H, m), 6.95-7.04 (2H, m), 7.30-7.33 (1H, m), 7.40 (1H, t, J = 7.8 Hz), 7.58 (1H, t, J = 7.8 Hz), 7.80-8.00 (4H, br), 8.00-8.02 (1H, m), 8.12-8.18 (3H, m), 10.54 (1H, s), 11.50 (1H, s), 11.79 (1H, s).
Melting point: 223-228°C
Element analysis: Calculated (%) for C₂₆H₂₁N₅O₃S·1.OHCl·1.SH₂O: C, 57.19; H, 4.43; N, 12.83; Cl, 6.49. Found (%): C, 57.36; H, 4.58; N, 12.85; Cl, 6.70.

### Example 15

### tert-butyl{2-[(3-{3-cyano-6-(2-hydroxyphenyl)-2-[(2-thienylcarbonyl)amino]pyridin-4-yl}phenyl)amino]-2-oxoethyl}methylcarbamate

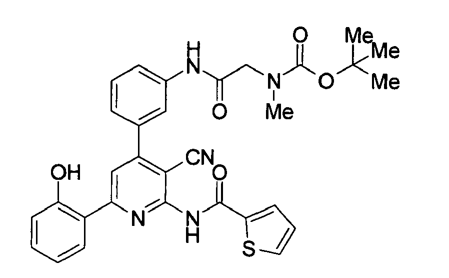

From the compound of Reference Example 4 (300 mg, 0.57 mmol) and Boc-sarcosine (108 mg, 0.57 mmol), the title compound (72 mg, 22%) was given as a yellow powder in substantially the same manner as in Example 13.
¹H-NMR (300MHz, DMSO-d₆) δ: 1.33 (5H, s), 1.41 (4H, s), 2.88 (3H, d, J = 6.3 Hz), 4.00 (2H, d, J = 11.7 Hz), 6.95-7.02 (2H, m), 7.30-7.42 (3H, m), 7.57 (1H, t, J = 7.7 Hz), 7.78 (1H, t, J = 7.4 Hz), 7.81-7.99 (2H, m), 8.02-8.18 (3H, m), 10.27 (0.6H, s), 10.30 (0.4H, s), 11.45 (1H, s), 11.80-11.90 (1H, m).
Melting point: 143-149°C

### Example 16

### N-(3-cyano-6-(2-hydroxyphenyl)-4-{3-[(N-methylglycyl)amino]phenyl}pyridin-2-yl) thiophene-2-carboxamide hydrochloride

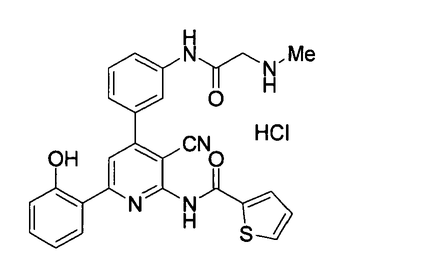

From the compound of Example 15 (60 mg, 0.10 mmol), the title compound (53 mg, 95%) was given as a yellow crystal in substantially the same manner as in Example 3.
¹H-NMR (300MHz, DMSO-d₆) δ : 2.64 (3H, s), 4.00 (2H, s), 6.95-7.04 (2H, m), 7.28-7.33 (1H, m), 7.40 (1H, t, J = 7.8 Hz), 7.48 (1H, d, J = 7.8 Hz), 7.63 (1H, t, J = 8.0 Hz), 7.79-7.85 (1H, m), 8.02-8.05 (2H, m), 8.13-8.20 (3H, m), 9.02 (2H,br s), 11.00 (1H, s), 11.52 (1H, s), 11.80 (1H, s).
Melting point: 248-254°C
Element analysis: Calculated (%) for C₂₆H₂₁N₅O₃S·1.1HCl·0.9H₂O: C, 57.84; H, 4.46; N, 12.97; Cl, 7.22. Found (%): C, 58.01; H, 4.39; N, 13.03; Cl, 7.07.

### Example 17

### N-[3-cyano-4-{3-[(N,N-dimethylglycyl)amino]phenyl}-6-(2-hydroxyphenyl)pyridin-2-yl]thiophene-2-carboxamide

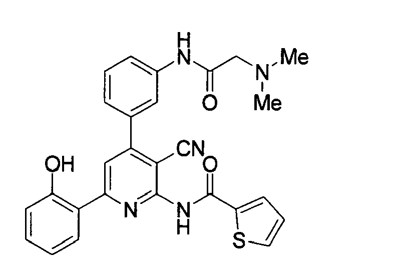

From the compound of Reference Example 4 (300 mg, 0.57 mmol) and N,N-dimethylglycine (59 mg, 0.57 mmol), the title compound (160 mg, 56%) was given as a yellow powder in substantially the same manner as in Example 13.
¹H-NMR (300MHz, DMSO-d₆) δ : 2.31 (6H, s), 3.14 (2H, s), 6.95-7.01 (2H, m), 7.29-7.32 (1H, m), 7.37-7.43 (2H, m), 7.55 (1H, t, J = 8.0 Hz), 7.91 (1H, d, J = 8.4 Hz), 7.99 (1H, d, J = 4.5 Hz), 8.06 (1H, s), 8.13-8.15 (3H, m), 10.02 (1H, s), 11.45 (1H, br s), 11.80-12.10 (1H, br).
Melting point: 220-221°C
Element analysis: Calculated (%) for C₂₇H₂₃N₅O₃S·0.1H₂O: C, 64.94; H, 4.68; N, 14.02. Found (%): C, 64.78; H, 4.67; N, 13.91.

### Example 18

### tert-butyl 2-{[(3-{3-cyano-6-(2-hydroxyphenyl)-2-[(2-thienylcarbonyl)amino]pyridin-4-yl}phenyl)amino]carbonyl}pyrrolidine-1-carboxylate

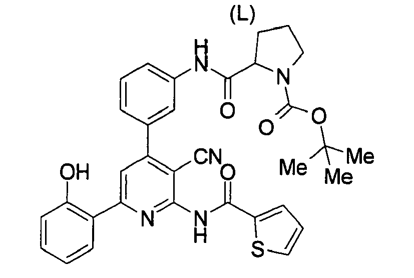

From the compound of Reference Example 4 (600 mg, 1.14 mmol) and N-Boc-L-proline (490 mg, 2.28 mmol), the title compound (280 mg, 40%) was given as a yellow crystal in substantially the same manner as in Example 13.
¹H-NMR (300MHz, CDCl₃) δ : 1.37-1.65 (10H, m), 1.90-2.10 (2H, br), 2.40-2.61 (1H, br), 3.30-3.55 (2H, br), 4.40-4.55 (1H, br), 6.91 (1H, t, J = 7.5 Hz), 7.10 (1H, d, J = 8.1 Hz), 7.18-7.21 (1H, m), 7.35-7.47 (3H, m), 7.57 (1H, d, J = 7.2 Hz), 7.67-7.72 (2H, m), 7.77-7.79 (1H, m), 7.85 (1H, d, J = 8.1 Hz), 7.97 (1H, d, J = 1.5Hz), 8.80 (1H, s), 9.80-10.00 (1H, br), 13.00-13.35 (1H, br).
Melting point: 169-171°C
Element analysis: Calculated (%) for C₃₃H₃₁N₅O₃S·0.4H₂O: C, 64.25; H, 5.20; N, 11.35. Found (%): C, 64.25; H, 5.23; N, 11.39.

### Example 19

### N-(3-{3-cyano-6-(2-hydroxyphenyl)-2-[(2-thienylcarbonyl)amino]pyridin-4-yl}phenyl) -L-prolinamide hydrochloride

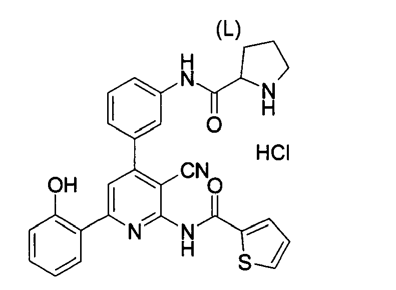

From the compound of Example 18 (200 mg, 0.33 mmol), the title compound (184 mg, 96%) was given as a yellow crystal in substantially the same manner as in Example 3.
¹H-NMR (300MHz, DMSO-d₆) δ:1.91-2.05 (3H, m), 2.42-2.50 (1H, m), 3.24-3.32 (2H, m), 4.39-4.43 (1H, m), 6.96-7.04 (2H, m), 7.31 (1H, t, J = 4.4 Hz), 7.40 (1H, t, J = 7.7 Hz), 7.49 (1H, d, J = 8.1 Hz), 7.63 (1H, t, J = 8.0 Hz), 7.84 (1H, d, J = 9.0 Hz), 8.00-8.04 (2H, m), 8.12-8.20 (3H, m), 8.15-8.30 (1H, br), 9.55-9.70 (1H, br), 11.00 (1H, s), 11.50 (1H, s), 11.70-11.85 (1H, br).
Melting point: 210-218°C
Element analysis: Calculated (%) for C₂₈H₂₃N₅O₃S·1.0HCl·2.1H₂O: C, 57.60; H, 4.86; N, 11.99. Found (%): C, 57.30; H, 4.65; N, 11.79.

### Example 20

### tert-butyl 2-{[(3-{3-cyano-6-(2-hydroxyphenyl)-2-[(2-thienylcarbonyl)amino]pyridin-4-yl}phenyl)amino]carbonyl}pyrrolidine-1-carboxylate

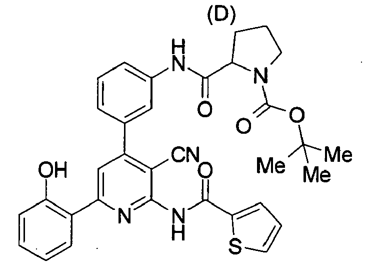

From the compound of Reference Example 4 (500 mg, 0.95 mmol) and N-Boc-D-proline (409 mg, 1.90 mmol), the title compound (252 mg, 44%) was given as a yellow crystal in substantially the same manner as in Example 13.
¹H-NMR (300MHz, CDCl₃) δ : 1.45-1.65 (10H, m), 1.90-2.10 (2H, br), 2.40-2.60 (1H, br), 3.30-3.55 (2H, br), 4.40-4.5 (1H, br), 6.88 (1H, t, J = 7.1 Hz), 7.10 (1H, d, J = 7.5 Hz), 7.18-7.21 (1H, m), 7.35-7.46 (3H, m), 7.57 (1H, d, J = 7.2 Hz), 7.77-7.79 (1H, m), 7.83 (1H, d, J = 8.1 Hz), 7.97 (1H, s), 8.80 (1H, s), 9.80-10.00 (1H, br), 13.00-13.30 (1H, br).
Melting point: 167-169°C
Element analysis: Calculated (%) for C₃₃H₃₁N₅O₃S·0.3H₂O: C, 64.43; H, 5.19; N, 11.39. Found (%): C, 84.46; H, 5.34; N, 11.33.

### Example 21

### N-(3-{3-cyano-6-(2-hydroxyphenyl)-2-[(2-thienylcarbonyl)amino]pyridin-4-yl}phenyl) -D-prolinamide hydrochloride

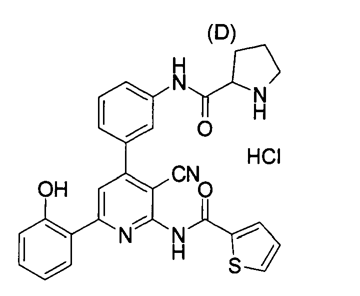

From the compound of Example 18 (200 mg, 0.33 mmol), the title compound (181 mg, 94%) was given as a yellow crystal in substantially the same manner as in Example 3.
¹H-NMR (300MHz, DMSO-d₆) δ : 1.91-2.08 (3H, m), 2.40-2.50 (1H, m), 3.25-3.32 (2H, m), 4.37-4.42 (1H, m), 6.96-7.04 (2H, m), 7.30-7.33 (1H, m), 7.38-7.43 (1H, m), 7.49 (1H, t, J = 7.8 Hz), 7.63 (1H, t, J = 7.8 Hz), 7.84 (1H, d, J = 8.4 Hz), 8.00-8.05 (2H, m), 8.13-8.20 (3H, m), 8.70-8.80 (1H, m), 9.62-9.70 (1H, br), 10.98 (1H, s), 11.50 (1H, s), 11.70-11.80 (1H, br).
Melting point: 213-218°C
Element analysis: Calculated (%) for C₂₈H₂₃N₅O₃S·1.1HCl·2.0H₂O: C, 57.42; H, 4.84; N, 11.96; Cl, 6.66. Found (%): C, 57.59; H, 4.91; N, 11.87; Cl, 6.74.

### Example 22

### tert-butyl {4-[(3-{3-cyano-6-(2-hydroxyphenyl)-2-[(2-thienylcarbonyl)amino]pyridin-4-yl}phenyl)amino]-4-oxobutyl}carbamate

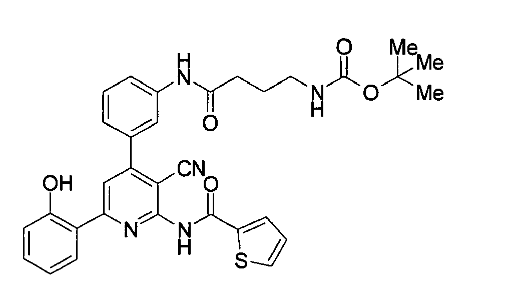

From the compound of Reference Example 4 (300 mg, 0.57 mmol) and Boc-γ-aminobutyric acid (232 mg, 1.14 mmol), the title compound (310 mg, 91 %) was given as a white amorphous solid in substantially the same manner as in Example 13.
¹H-NMR (300MHz, DMSO-d₆) δ : 1.56 (9H, s), 1.66-1.75 (2H, m), 2.35 (2H, t, J = 7.4 Hz), 2.89-3.01 (2H, m), 6.80-6.90 (1H, br), 6.95-7.01 (2H, m), 7.31-7.59 (4H, m), 7.70-7.80 (1H, m), 7.90-8.05 (3H, m), 8.14 (2H, d, J = 7.8 Hz), 10.21 (1H, s), 11.45 (1H, s), 11.80-11.90 (1H, br).

### Example 23

### N-[4-{3-[(4-aminobutanoyl)amino]phenyl}-3-cyano-6-(2-hydroxyphenyl)pyridin-2-yl] thiophene-2-carboxamide dihydrochloride

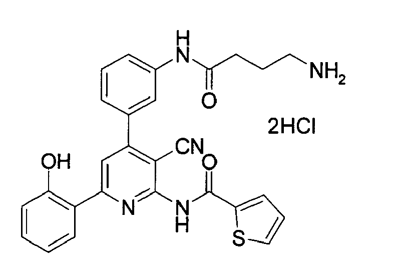

From the compound of Example 22 (310 mg, 0.52 mmol), the title compound (121 mg, 41%) was given as a pale yellow crystal in substantially the same manner as in Example 3.
¹H-NMR (300MHz, DMSO-d₆) δ : 1.83-1.93 (2H, m), 2.47-2.51 (2H, m), 2.87 (2H, t, J = 7.8 Hz), 6.95-7.03 (2H, m), 7.30 (1H, t, J = 4.4 Hz), 7.38-7.41 (2H, m), 7.56 (1H, t, J = 8.0 Hz), 7.60-7.70 (3H, m), 7.99-8.02 (2H, m), 8.13-8.15 (3H, m), 10.35 (1H, s), 11.40-11.90 (2H, br).
Melting point: 236-238°C

### Example 24

### N-[4-(2-{[(2-aminoethyl)amino]carbonyl}phenyl)-3-cyano-6-(2-hydroxyphenyl) pyridin-2-yl]thiophene-2-carboxamide dihydrochloride

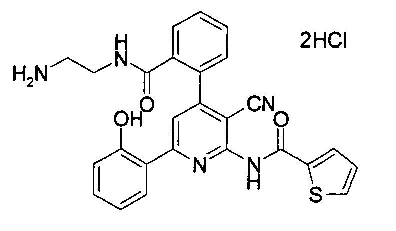

From the compound of Reference Example 27 (120 mg, 0.25 mmol) and tert-butyl N-(2-aminoethyl)carbamate (50 mg, 0.30 mmol), tert-butyl {2-[(2-{3-cyano-6-[2-(methoxymethoxy)phenyl]-2-[(2-thienylcarbonyl)amino]pyridin-4 -yl}benzoyl)amino]ethyl}carbamate (130 mg, 83%) was given as a green amorphous solid in substantially the same manner as in Reference Example 15. From the resultant compound (130 mg, 0.21 mmol), the title compound (93 mg, 80%) was given as a yellow crystal in substantially the same manner as in Example 3.
¹H-NMR (300MHz, CDCl₃) δ : 2.87-2.93 (2H, m), 3.37-3.43 (2H, m), 5.70-6.50 (3H, br), 6.93-7.03 (2H, m), 7.30 (1H, t, J = 4.4 Hz), 7.39 (1H, t, J = 7.7 Hz), 7.52 (1H, dd, J = 7.5, 2.1 Hz), 7.65-7.74 (2H, m), 7.90-8.01 (3H, m), 8.07-8.10 (2H, m), 8.15 (1H, d, J = 3.0 Hz), 8.84 (1H, t, J = 5.6 Hz), 11.40 (1H, s).
Melting point: 196-201°C
Element analysis: Calculated (%) for C₂₆H₂₁N₅O₃S·1.7HCl·1.5H₂O: C, 54.54; H, 4.52; N, 12.23; Cl, 10.53. Found (%): C, 54.63; H, 4.49; N, 12.38; Cl, 10.64.

### Example 25

### N-[4-(3-aminophenyl)-6-(2-hydroxyphenyl)pyridin-2-yl]thiophene-2-carboxamide

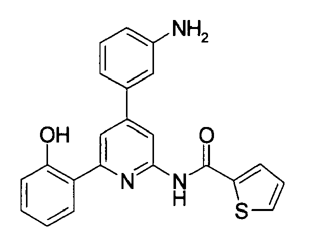

From the compound of Reference Example 32 (40 mg, 0.096 mmol), the title compound (12 mg, 32%) was given as a white crystal in substantially the same manner as in Reference Example 4.
¹H-NMR (300MHz, CDCl₃) δ : 5.33 (2H, m), 6.71 (1H, t, J = 7.5 Hz), 6.91-7.04 (4H, m), 7.18-7.35 (3H, m), 7.95 (1H, s), 8.00 (1H, s), 8.11-8.14 (2H, m), 8.21 (1H, s), 10.10 (1H, s), 12.76 (1H, s).
Melting point: 161-162°C
Element analysis: Calculated (%) for C₂₂H₁₇N₃O₂S·0.3H₂O: C, 67.26; H, 4.52; N, 10.70. Found (%): C, 67.25; H, 4.50; N, 10.77.

### Example 26

### tert-butyl {3-[(3-{2-(2-hydroxyphenyl)-6-[(2-thienylcarbonyl)amino]pyridin-4-yl} phenyl)amino]-3-oxopropyl}carbamate

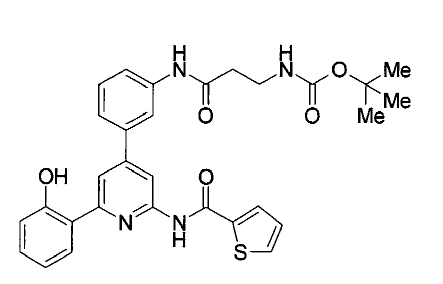

From the compound of Example 25 (60 mg, 0.15 mmol), the title compound (41 mg, 49%) was given as a white crystal in substantially the same manner as in Reference Example 38.
¹H-NMR (300MHz, CDCl₃) δ :1.59 (9H, s), 2.68 (2H, t, J = 5.7 Hz), 3.55 (2H, q, J = 6.0 Hz), 5.33-5.45 (1H, br), 6.91 (1H, t, J = 7.7 Hz), 7.03 (1H, d, J = 8.1 Hz), 7.18 (1H, t, J = 4.4 Hz), 7.26-7.34 (1H, m), 7.39-7.45 (2H, m), 7.63-7.90 (6H, s), 7.92-8.05 (1H, br), 8.40 (1H, s), 8.50-8.60 (1H, br s), 12.84-13.09 (1H, br).
Melting point: 183-184°C

### Example 27

### N-(4-[3-(β-alanylamino)phenyl]-6-(2-hydroxyphenyl)pyridin-2-yl)thiophene-2-carboxamide dihydrochloride

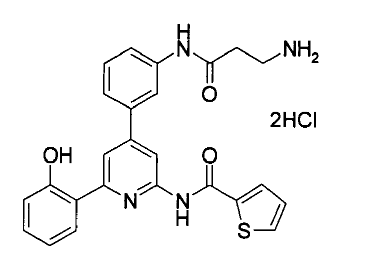

From the compound of Example 26 (40 mg, 0.072 mmol), the title compound (23 mg, 60%) was given as a white crystal in substantially the same manner as in Example 8.
¹H-NMR (300MHz, DMSO-d₆) δ: 2.79 (2H, t, J = 6.8 Hz), 3.10 (2H, q, J = 6.2 Hz), 6.96 (2H, t, J = 7.5 Hz), 7.28-7.37 (2H, m), 7.53 (1H, t, J = 7.8 Hz), 7.63 (1H, d, J = 7.8 Hz), 7.76 (1H, d, J = 8.4 Hz), 7.80-7.98 (4H, m), 7.07 (1H, s), 7.12-7.15 (2H, m), 7.24-7.27 (2H, m), 10.49 (1H, s), 11.34 (1H, s).
Melting point: 214-220°C
Element analysis: Calculated (%) for C₂₅H₂₂N₄O₃S·1.5HCl·2.0H₂O: C, 54.67; H, 5.05; N, 10.20. Found (%): C, 54.66; H, 5.10; N, 10.10.

### Example 28

### N-[4-(3-aminophenyl)-3-cyano-6-(2-hydroxyphenyl)pyridin-2-yl]benzamide

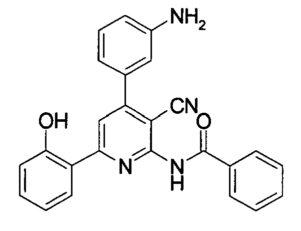

Benzoyl chloride (0.65 mL, 5.6 mmol) was added to a solution of 2-amino-6-(2-{[tert-butyl(dimethyl)silyl]oxy}phenyl)-4-(3-nitrophenyl) nicotinonitrile (1.0 g, 2.24 mmol) in pyridin(10 mL) described in WO 02/44153, and was stirred at room temperature for 23 hours. The resultant substance was concentrated. The residue was diluted with ethyl acetate, washed with water (3 times) and then with saturated brine, dried over magnesium sulfate, and then concentrated under reduced pressure. The residue was purified by amino silica gel chromatography (eluting solution: hexane to ethyl acetate/hexane = 1/1), and the solvent was removed under reduced pressure. The resultant solid was washed with diisopropylether to give N-[6-(2-((tert-butyl(dimethyl)silyl)oxy))phenyl]-3-cyano-4-(3-nitrophenyl)pyridin-2-yl] benzamide (920 mg, 77%). Among the resultant substance, 0.80 g (1.5 mmol) was dissolved in a mixed solvent of ethanol (10 ml) and water (5 ml), ammonium chloride (0.40 g, 7.5 mmol) and iron powder (0.41 g, 7.3 mmol) were added, and refluxed while heating for 2 hours. The resultant mixture was diluted with ethyl acetate, and washed with water and then with saturated brine. The organic layer was dried over magnesium sulfate and concentrated under reduced pressure. The residue was purified by silica gel chromatography (eluting solution: hexane to ethyl acetate/hexane = 1/2), and the solvent was removed under reduced pressure to give N-[4-(3-aminophenyl)-6-(2-((tert-butyl(dimethyl)silyl)oxy)phenyl)-3-cyanopyridin-2-yl]benzamide(0.55 g, 72%) as an amorphous solid.

The resultant solid was entirely dissolved in THF(10 ml), a 1M solution of tetrabutylammonium fluoride in THF(2 ml) was dropped at 0°C, and stirred for another 15 minutes. The resultant solution was diluted with ethyl acetate, washed with water (twice) and then with saturated brine, and dried over magnesium sulfate. The solvent was removed under reduced pressure. The residue was recrystallized from acetonitrile to give the title compound(0.29 g, 65%) as a yellow crystal.
¹H-NMR (300MHz, DMSO-d₆) δ: 5.45 (2H, br s), 6.76-6.79 (1H, m), 6.82-6.85 (1H, m), 6.90 (1H, t, J = 2.1 Hz), 6.94-7.01 (2H, m), 7.25 (1H, t, J = 7.8 Hz), 7.36-7.42 (1H, m), 7.59-7.64 (2H, m), 7.67-7.72 (1H, m), 8.05-8.08 (2H, m), 8.12 (1H, s), 8.15 (1H, dd, J = 8.1,1.5 Hz), 11.41 (1H, s), 11.99 (1H, s).
Melting point: 259-261°C
Element analysis: Calculated (%) for C₂₅H₁₈N₄O₂: C, 73.88; H, 4.46; N, 13.78. Found (%): C, 73.60; H, 4.42; N, 13.73.

### Example 29

### N-[4-(3-aminophenyl)-3-cyano-6-(2-hydroxyphenyl)pyridin-2-yl] cyclohexanecarboxamide

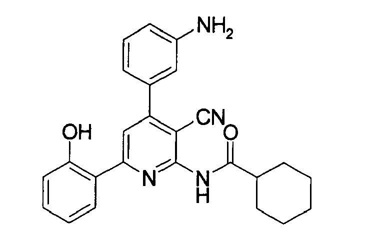

From 2-amino-6-[2-((tert-butyl(dimethyl)silyl)oxy)phenyl]-4-(3-nitrophenyl) nicotinonitrile (1.0 g, 2.24 mmol) and cyclohexanecarbonyl chloride (0.74 ml, 5.5 mmol), the title compound (0.23 g, 25%, recrystallized from ethyl acetate-ethanol) was given as a pale yellow crystal in substantially the same manner as in Example 28.
¹H-NMR (300MHz, DMSO-d₆) δ:1.15-1.52 (5H, m), 1.65-1.91 (5H, m), 2.53-2.58 (1H, m), 5.42 (2H, brs), 6.73-6.80 (2H, m), 6.84 (1H, t, J = 1.8 Hz), 6.92-6.98 (2H, m), 7.22 (1H, t, J = 7.8 Hz), 7.35-7.41 (1H, m), 8.00 (1H, s), 8.10 (1H, dd, J = 7.8, 1.2 Hz), 10.80 (1H, br s), 12.14 (1H, s).
Melting point: 236-238°C
Element analysis: Calculated (%) for C₂₅H₂₄N₄O₂·0.25 H₂O: C, 72.01; H, 5.92; N, 13.44. Found (%): C, 72.10; H, 5.94; N, 13.22.

### Example 30

### tert-butyl [3-({3-[2-(benzoylamino)-3-cyano-6-(2-hydroxyphenyl)pyridin-4-yl]phenyl} amino)-3-oxopropyl]carbamate

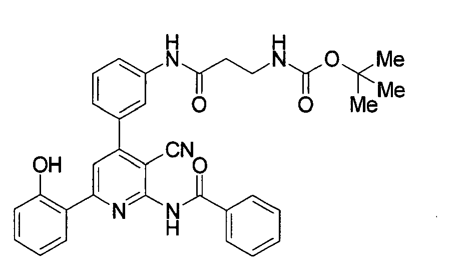

From the compound of Example 28 (0.20 g, 0.49 mmol) and Boc-β-alanine (0.11 g, 0.58 mmol), the title compound (0.21 g, 74%, recrystallized from chloroform-ether) was given as a pale yellow crystal in substantially the same manner as in Reference Example 38.
¹H-NMR (300MHz, DMSO-d₆) δ : 1.38 (9 H, s), 2.53-2.55 (2H, m), 3.21-3.27 (2H, m), 6.88 (1H, br s), 6.95-7.01 (2H, m), 7.37-7.43 (2H, m), 7.52-7.73 (4H, m), 7.82 (1H, br d, J = 8.4 Hz), 8.00 (1H, br s), 8.06-8.08 (2H, m), 8.13-8.17 (2H, m), 10.24 (1H, br s), 11.45 (1H, br s), 11.92 (1H, br s).
Melting point: 220-221°C
Element analysis: Calculated (%) for C₃₃H₃₁N₅O₅: C, 68.62; H, 5.41; N, 12.12. Found (%): C, 68.37; H, 5.36; N, 12.11.

### Example 31

### N-[4-(3-(β-alanylamino)phenyl)-3-cyano-6-(2-hydroxyphenyl)pyridin-2-yl]benzamide trifluoroacetate

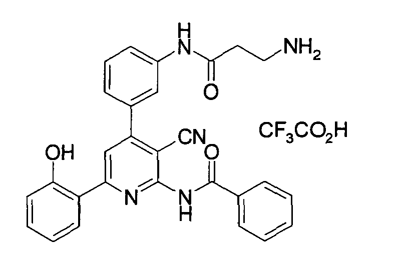

The compound of Example 30 (0.15 g, 0.26 mmol) was dissolved in trifluoroacetic acid (5 mL), and stirred at room temperature for 90 minutes. The solvent was removed under reduced pressure, and the residue was recrystallized from ethanol-diethylether to give the title compound (0.12 g, 78%) as a pale yellow crystal.
¹H-NMR (300MHz, DMSO-d₆) δ : 2.76 (2H, t, J = 6.9 Hz), 3.12 (2H, br s), 6.95-7.02 (2H, m), 7.37-7.45 (2H, m), 7.56-7.64 (3H, m), 7.68-7.85 (5H, m), 8.00 (1H, br s), 8.05-8.08 (2H, m), 8.14 (1H, dd, J = 8.1, 1.5 Hz), 8.17 (1H, s), 10.46 (1H, br s), 11.49 (1H, br s), 11.87 (1H, br s).
Melting point: 225-226°C
Element analysis: Calculated (%) for C₃₀H₂₄N₅O₅F₃: C, 60.91; H, 4.09; N, 11.84. Found (%): C, 60.75; H, 4.17; N, 11.90.

### Example 32

### N-[4-(3-aminophenyl)-3-cyano-6-(2-hydroxyphenyl)pyridin-2-yl]-5-chlorothiophene-2-carboxamide

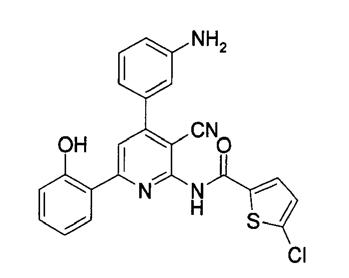

A solution of the compound of Reference Example 33 (1.16 g, 1.96 mmol), iron powder (657 mg, 11.8 mmol) and ammonium chloride (682 mg, 12.8 mmol) in ethanol (50 mL)-water (50 mL) was refluxed while heating for 20 minutes. After cooling, saturated aqueous sodium hydrogencarbonate was added to the reaction mixture, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1 to 2/1) to give N-[4-(3-aminophenyl)-6-(2-{[tert-butyl (dimethyl)silyl]oxy}phenyl)-3-cyanopyridin-2-yl]-5-chlorothiophene-2-carboxamide (328 mg) as a pale yellow solid.

A 1M solution of tetrabutyl ammonium fluoride in THF(1.75 mL) was added to a solution of the resultant N-[4-(3-aminophenyl)-6-(2-{[tert-butyl(dimethyl) silyl]oxy}phenyl)-3-cyanopyridin-2-yl]-5-chlorothiophene-2-carboxamide (328 mg, 0.584 mmol) in THF (7 mL) with ice-cooling, and stirred for 1 hour. The reaction mixture was diluted with water and concentrated under reduced pressure. The residue was diluted with ethyl acetate, washed with water and then with saturated brine, dried over magnesium sulfate, and then concentrated under reduced pressure. The residue was recrystallized from ethyl acetate-hexane, washed with dichloromethane, and then dried to give the title compound (138 mg, 16%) as a pale yellow crystal.
¹H-NMR (300MHz, DMSO-d₆) δ : 5.44 (2H, s), 6.70-6.90 (3H, m), 6.92-7.06 (2H, m), 7.24 (1H, t, J = 7.7 Hz), 7.32-7.45 (2H, m), 8.02 (1H, d, J = 4.1 Hz), 8.09-8.21 (2H, m), 11.51 (1H, s), 11.75 (1H, s).
Melting point: 259-260°C
Element analysis: Calculated (%) for C₂₃H₁₅N₄O₂SCl: C, 61.81; H, 3.38; N. 12.54. Found (%): C, 61.95; H, 3.57; N, 12.25.

### Example 33

### N-[4-(3-aminophenyl)-3-cyano-6-(2-hydroxyphenyl)pyridin-2-yl]thiophene-3-carboxamide

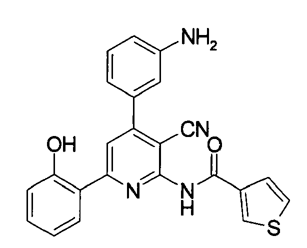

From the compound of Reference Example 34 (780 mg, 1.40 mmol), the title compound (139 mg, 24%) was given as a pale yellow crystal in substantially the same manner as in Example 32.
¹H-NMR (300MHz, DMSO-d₆) δ : 5.44 (2H, s), 6.68-7.08 (5H, m), 7.18-7.52 (2H, m), 7.66-7.81 (2H, m), 8.08-8.20 (2H, m), 8.48-8.59 (1H, m, J = 1.5 Hz), 11.23 (1H, s), 11.96 (1H, s).
Melting point: 257-260°C
Element analysis: Calculated (%) for C₂₃H₁₆N₄O₂S·0.75H₂O: C, 64.85; H, 4.14; N. 13.15. Found (%): C, 65.09; H, 3.88; N, 12.95.

### Example 34

### N-(3-cyano-6-(2-hydroxyphenyl)-4-{3-[(propylamino)carbonyl]phenyl}pyridin-2-yl) thiophene-2-carboxamide

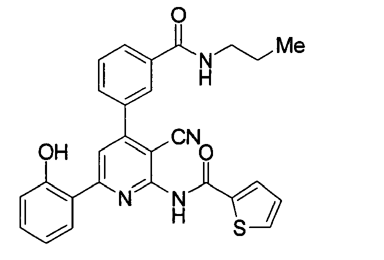

Concentrated hydrochloric acid (0.5 mL) was added to a solution of the compound of Reference Example 54 (90 mg, 0.17 mmol) in THF (5 mL), and stirred at room temperature for 1 hour. The reaction mixture was diluted with saturated aqueous sodium hydrogencarbonate, and extracted with ethyl acetate. The extract was washed with saturated brine, dried over magnesium sulfate, and then concentrated under reduced pressure. The residue was recrystallized from ethyl acetate-hexane to give the title compound (61 mg, 74%) as a pale yellow crystal.
¹H-NMR (300MHz, DMSO-d₆) δ:1.04 (3H, t, J = 7.5 Hz), 1.68-1.90 (2H, m), 3.52 (2H, q, J = 6.7 Hz), 6.70 (1H, t, J = 5.4 Hz), 6.81 (1H, t, J = 7.5 Hz), 6.99 (1H, d, J = 8.1 Hz), 7.19 (1H, t, J = 4.4 Hz), 7.34 (1H, t, J = 7.8 Hz), 7.60-7.77 (6H, m), 8.00 (1H, d, J = 7.8 Hz), 8.14 (1H, s), 8.71 (1H, s), 13.20 (1H, s).
Melting point: 245-246°C
Element analysis: Calculated (%) for C₂₇H₂₂N₄O₃S: C, 67.20; H, 4.60; N. 11.61. Found (%): C, 67.07; H, 4.60; N, 11.66.

### Example 35

### N-[3-cyano-4-(3-{[(2-hydroxyethyl)amino]carbonyl}phenyl)-6-(2-hydroxyphenyl) pyridin-2-yl]thiophene-2-carboxamide

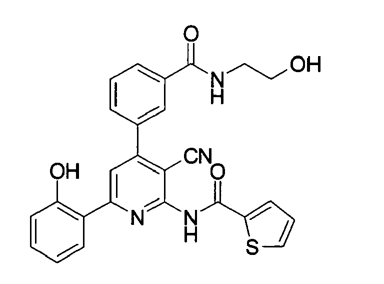

From the compound of Reference Example 55 (70 mg, 0.13 mmol), the title compound (55 mg, 87%) was given as a white crystal in substantially the same manner as in Example 34.
¹H-NMR (300MHz, DMSO-d₆) δ : 3.34-3.41 (2H, m), 3.51-3.57 (2H, m), 4.76 (1H, t, J = 5.6 Hz), 6.96-7.02 (2H, m), 7.30-7.33 (1H, m), 7.41 (1H, t, J = 7.7 Hz), 7.72 (1H, t, J = 7.7 Hz), 7.89 (1H, d, J = 7.8 Hz), 8.00-8.25 (6H, m), 8.63 (1H, br s), 11.46 (1H, s), 11.80-12.00 (1H, br).
Melting point: 218-219°C
Element analysis: Calculated (%) for C₂₆H₂₀N₄O₄S·0.4H₂O: C, 63.51; H, 4.26; N. 11.39.
Found (%): C, 63.52; H, 4.55; N, 11.29.

### Example 36

### N-{3-cyano-6-(2-hydroxyphenyl)-4-[3-(morpholin-4-ylcarbonyl)phenyl]pyridin-2-yl} thiophene-2-carboxamide

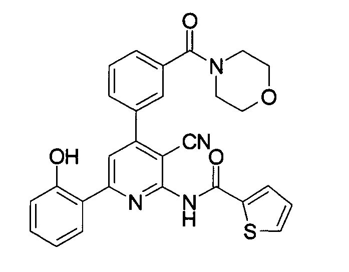

From the compound of Reference Example 56 (190 mg, 0.34 mmol), the title compound (110 mg, 59%) was given as a pale yellow crystal in substantially the same manner as in Example 3.
¹H-NMR (300MHz, DMSO-d₆) δ: 3.50-3.70 (8H, m), 6.96-7.02 (2H, m), 7.30-7.43 (2H, m), 7.64-7.76 (3H, m), 7.85 (1H, d, J = 7.5 Hz), 8.01 (1H, d, J = 4.8 Hz), 8.14-8.24 (8H, m), 11.46 (1H, s), 11.92 (1H, s).
Melting point: 253-254°C
Element analysis: Calculated (%) for C₂₈H₂₂N₄O₄S: C, 65.87; H, 4.34; N. 10.97. Found (%): C, 65.82; H, 4.28; N,11.04.

### Example 37

### Benzyl {4-[(3-{3-cyano-6-(2-hydroxyphenyl)-2-[(2-thienylcarbonyl)amino]-pyridin-4-yl} phenyl)amino]-4-oxobutyl}carbamate

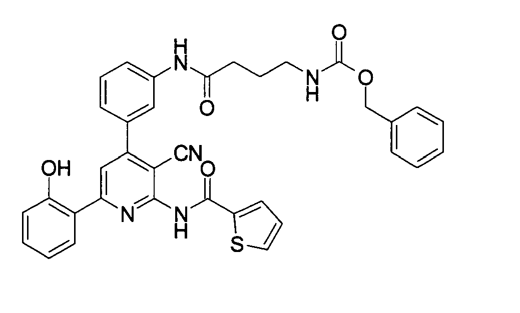

From the compound of Reference Example 4 (300 mg, 0.57 mmol) and N-Cbz-γ-aminobutyric acid (270 mg, 1.14 mmol), the compound specified in the title section title (105 mg, 29%) was given as a pale yellow crystal in substantially the same manner as in Reference Example 38.
¹H-NMR (300MHz, DMSO-d₆) δ: 1.70-1.80 (2H, m), 2.38 (2H, t, J = 7.4 Hz), 3.07 (2H, q, J = 6.5 Hz), 5.01 (2H, s), 6.95-7.02 (2H, m), 7.30-7.43 (9H, m), 7.55 (1H, t, J = 8.0 Hz), 7.80 (1H, d, J = 8.1 Hz), 8.00 (2H, s), 8.13-8.17 (3H, m), 10.21 (1H, s), 11.45 (1H, s), 11.80 (1H, br s).
Melting point: 156-157°C
Element analysis: Calculated (%) for C₃₅H₂₉N₅O₅S.0.3H₂O: C, 65.98; H, 4.68; N. 10.99. Found (%): C, 65.99; H, 4.63; N, 11.07.

### Example 38

### N-{3-cyano-4-[3-(dimethylamino)phenyl]-6-(2-hydroxyphenyl)pyridin-2-yl}thiophene-2-carboxamide

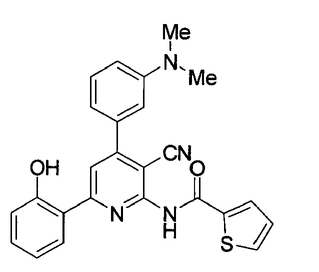

A suspension of the compound of Reference Example 4 (180 mg, 0.34 mmol) and sodium borohydride (270 mg, 9.56 mmol) in THF(2 mL) was added to a solution of 37% formalin solution (0.66 mL) and 3M aqueous solution of sulfuric acid (0.96 mL) in DMF (0.5 mL) at a temperature of 20°C or lower, and stirred at room temperature for 30 minutes. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate), and then recrystallized from ethyl acetate-hexane to give the title compound (77 mg, 51 %) as a yellow crystal.
¹H-NMR (300MHz, DMSO-d₆) δ: 3.00 (6H, m), 6.92-7.01 (5H, m), 7.29-7.43 (3H, m), 8.00 (1H, d, J = 4.8 Hz), 8.12-8.18 (3H, m), 11.40 (1H, s), 11.80-12.00 (1H, br).
Melting point: 230-231°C
Element analysis: Calculated (%) for C₂₅H₂₀N₄O₂S: C, 68.16; H, 4.58; N. 12.72. Found (%): C, 68.09; H, 4.59; N, 12.66.

### Example 39

### N- {3-cyano-6-(2-hydroxyphenyl)-4-[3-(methylamino)phenyl]pyridin-2-yl}thiophene-2-carboxamide

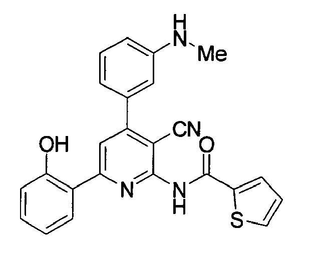

Methyl iodide (24 µL, 0.40 mmol) was added to a suspension of the compound of Reference Example 57 (200 mg, 0.39 mmol) and potassium carbonate (54 mg, 0.39 mmol) in DMF (2 mL) with ice-cooling, and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure to give a mixture of N-{3-[2-amino-6-(2-{[tert-butyl(dimethyl)silyl]oxy}phenyl)-3-cyanopyridin-4-yl]phenyl}-2,2,2-trifluoro-N-methylacetamide and N-{3-[2-amino-3-cyano-6-(2-hydroxyphenyl)pyridin-4-yl]phenyl}-2,2,2-trifluoro-N- methylacetamide.

2-thenoyl chloride (200 mg, 1.37 mmol) was added to a solution of the mixture resudue in pyridin(5 mL), and stirred at room temperature for 16 hours. The reaction mixture was concentrated under reduced pressure, and extracted with ethyl acetate. The extract was washed with water and then with saturated brine, dried over magnesium sulfate, and then concentrated under reduced pressure.

A 4N aqueous sodium hydroxide solution (1 mL) was added to a solution of the residue in THF (10 mL)-ethanol (5 mL), and stirred at room temperature for 2 hours.

The reaction mixture was neutralized with 1N hydrochloric acid, and extracted with ethyl acetate. The extract was washed with saturated brine, dried over magnesium sulfate, and then concentrated under reduced pressure.

The residue was purified by silica gel chromatography (eluting solution: ethyl acetate/hexane = 1/1 to ethyl acetate), and then recrystallized from acetonitrileto give the title conpound (71 mg, 44%) as yellow needles.
¹H-NMR (300MHz, DMSO-d₆) δ: 2.75 (3H, d, J = 5.1 Hz), 6.02 81H, q, J = 5.1 Hz), 6.75 (1H, dd, J = 8.1, 1.5 Hz), 6.83-6.87 (2H, m), 6.95-7.01 (2H, m), 7.28-7.33 (2H, m), 8.00 (1H, d, J = 4.8 Hz), 8.12-8.15 (3H, m), 11.39 (1H, s), 11.88 (1H, s).
Melting point: 227-230°C
Element analysis: Calculated (%) for C₂₄H₁₈N₄O₂S·0.1 H₂O: C, 67.31; H, 4.28; N. 13.08. Found (%): C, 67.27; H, 4.25; N, 13.30.

### Example 40

### tert-butyl{2-[(3-{3-cyano-6-(2-hydroxyphenyl)-2-[(2-thienylcarbonyl)amino]pyridin-4-yl }phenyl)(methyl)amino]-2-oxoethyl }carbamate

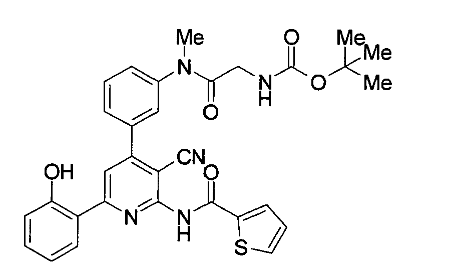

From the compound of Example 39 (80 mg, 0.19 mmol) and Boc-glycine (49 mg, 0.28 mmol), the title compound (30 mg, 27%) was given as a yellow amorphous solid in substantially the same manner as in Reference Example 38.
¹H-NMR (300MHz, DMSO-d₆) δ: 1.49 (9H, s), 3.39 (3H, s), 3.69-3.78 (2H, br), 5.42 (1H, br s), 6.87-6.92 (1H, m), 7.08 (1H, d, J = 8.4 Hz), 7.16-7.21 (1H, m), 7.36-7.46 (2H, m), 7.55 (1H, s), 7.65-7.83 (6H, m), 8.79 (1H, s), 13.12 (1H, s).

### Example 41

### N-[3-cyano-4-{3-[glycyl(methyl)amino]phenyl}-6-(2-hydroxyphenyl)pyridin-2-yl] thiophene-2-carboxamide dihydrochloride

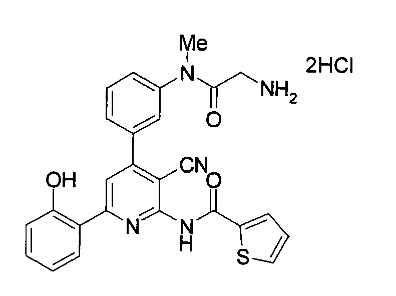

From the compound of Example 40 (30 mg, 0.05 mmol), the title compound (22 mg, 78%) was given as a yellow powder in substantially the same manner as in Example 3.
¹H-NMR (300MHz, DMSO-d₆) δ: 3.32 (3H, s), 3.43 (2H, br s), 6.97-7.04 (2H, m), 7.32 (1H, t, J = 4.4 Hz), 7.41 (1H, t, J = 7.8 Hz), 7.60-7.90 (3H, m), 8.01-8.27 (5H, m), 11.52 (1H, s), 11.70-11.80 (1H, br s).
Melting point: 198-205°C
Element analysis: Calculated (%) for C₂₆H₂₂N₅O₃S·2.0HCl·0.3H₂O: C, 55.58; H, 4.23; N, 12.46. Found (%): C, 55.51; H, 4.40; N, 12.39.

### Example 42

### N- {4-[5-(β-alanylamino)-2-chlorophenyl]-3-cyano-6-(2-hydroxyphenyl)pyridin-2-yl} thiophene-2-carboxamide dihydrochloride

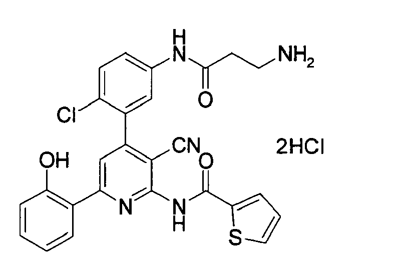

From the compound of Reference Example 38 (250 mg, 0.38 mmol), the title compound (189 mg, 84%) was given as a yellow powder in substantially the same manner as in Example 8.
¹H-NMR (300MHz, DMSO-d₆) δ: 2.78 (2H, t, J = 6.8 Hz), 3.08 (2H, q, J = 6.0 Hz), 6.94-7.04 (2H, m), 7.29-7.31 (1H, m), 7.40 (1H, t, J = 7.7 Hz), 7.66 (1H, d, J = 8.7 Hz), 7.76-8.02 (6H, m), 8.11-8.20 (3H, m), 10.70 (1H, s), 11.51 (1H, s), 11.64-11.81 (1H, br).
Melting point: 208-214°C
Element analysis: Calculated (%) for C₂₆H₂₁N₅O₃S·1.5HCl·1.2H₂O: C, 52.55; H, 4.05; N, 11.78. Found (%): C, 52.53; H, 4.12; N, 11.96.

### Example 43

### N-[3-cyano-4-{3-[(glycylamino)methyl]phenyl }-6-(2-hydroxyphenyl)pyridin-2-yl] thiophene-2-carboxamide hydrochloride

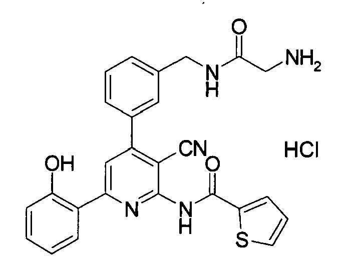

From the compound of Reference Example 43 (100 mg, 0.16 mmol), the title compound (85 mg, 100%) was given as a yellow powder in substantially the same manner as in Example 8.
¹H-NMR (300MHz, DMSO-d₆) δ: 3.63 (2H, d, J = 5.7 Hz), 4.49 (2H, d, J = 5.7 Hz), 6.94-7.04 (2H, m), 7.31 (1H, t, J = 4.4 Hz), 7.41 (1H, t, J = 7.7 Hz), 7.53-7.68 (4H, m), 8.00-8.19 (7H, m), 9.00 (1H, t, J = 5.9 Hz), 11.49 (1H, s), 11.80 (1H, s).
Melting point: 181-186°C
Element analysis: Calculated (%) for C₂₆H₂₁N₅O₃S·1.1HCl·1.4H₂O: C, 56.89; H, 4.57; N, 12.76; Cl, 7.11. Found (%): C, 56.96; H, 4.71; N, 12.82; Cl, 6.90.

### Example 44

### N-[4-{3-[(β-alanylamino)methyl]phenyl}-3-cyano-6-(2-hydroxyphenyl)pyridin-2-yl] thiophene-2-carboxamide hydrochloride

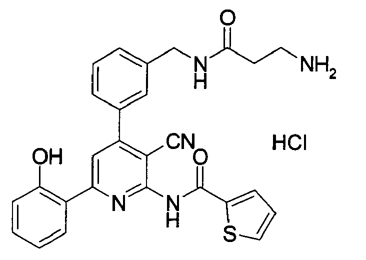

From the compound of Reference Example 44 (100 mg, 0.16 mmol), the title compound (76 mg, 78%) was given as a yellow powder in substantially the same manner as in Example 8.
¹H-NMR (300MHz, DMSO-d₆) δ: 2.56 (2H, t, J = 6.9 Hz), 3.03 (2H, q, J = 6.0 Hz), 4.43 (2H, d, J = 5.1 Hz), 6.96-7.03 (2H, m), 7.31 (1H, t, J = 4.4 Hz), 7.40 (1H, t, J = 7.8 Hz), 7.51 (1H, d, J = 7.5 Hz), 7.57-7.76 (6H, m), 8.01 (1H, d, J = 4.2 Hz), 8.13-8.19 (5H, m), 8.74 (1H, t, J = 5.1 Hz), 11.49 (1H, s), 11.82 (1H, s).
Melting point: 173-180°C
Element analysis: Calculated (%) for C₂₇H₂₃N₅O₃S·1.0HCl·1.1H₂O: C, 58.55; H, 4.76; N, 12.64. Found (%): C, 58.51; H, 4.82; N, 12.51.

### Example 45

### N-{4-[3-({[amino(imino)methyl]amino}methyl)phenyl]-3-cyano-6-(2-hydroxyphenyl) pyridin-2-yl}thiophene-2-carboxamide trifluoroacetate

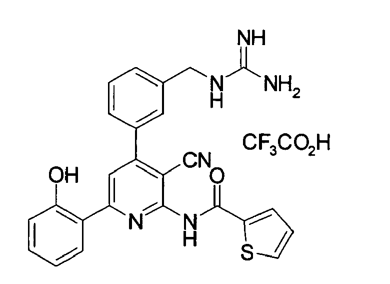

Trifluoroacetic acid (2 mL) was added to a solution of the compound of Reference Example 45 (80 mg, 0.11 mmol) in dichloromethane (5 mL), and stirred at room temperature for 1 hour. Diethylether was added to the reaction mixture. The generated precipitate was collected by filtration and dried under reduced pressure to give the title compound (32 mg, 50%) as a white powder.
¹H-NMR (300MHz, DMSO-d₆) δ: 4.51 (2H, d, J = 6.0 Hz), 6.97-7.03 (2H, m), 7.10-7.44 (5H, m), 7.54 (1H, d, J = 7.5 Hz), 7.64-7.73 (3H, m), 8.01-8.06 (2H, m), 8.12-8.18 (3H, m), 11.48 (1H, s), 11.77(1H, s).
Melting point: 144-151°C
Element analysis: Calculated (%) for C₂₅H₂₀N₆O₂S·1.0CF₃CO₂H·0.5H₂O: C, 54.82; H, 3.75; N, 14.21. Found (%): C, 54.70; H, 3.82; N, 14.08.

### Example 46

### N-{4-[3-(β-alanylamino)phenyl]-6-(5-chloro-2-hydroxyphenyl)-3-cyanopyridin-2-yl} thiophene-2-carboxamide trifluoroacetate

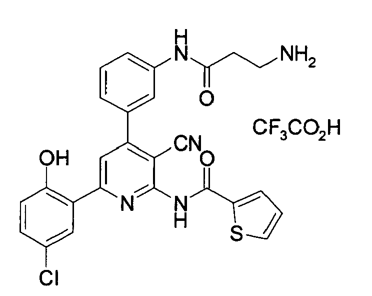

From the compound of Reference Example 50 (80 mg, 0.12 mmol), the title compound (72 mg, 95%) was given as a yellow powder in substantially the same manner as in Example 45.
¹H-NMR (300MHz, DMSO-d₆) δ: 2.76 (2H, t, J = 6.6 Hz), 3.10-3.13 (2H, m), 7.05 (1H, d, J = 8.7 Hz), 7.31 (1H, t, J = 4.4 Hz), 7.41-7.45 (2H, m), 7.58 (1H, t, J = 8.0 Hz), 7.60-7.85 (4H, m), 8.01 (2H, d, J = 5.1 Hz), 8.14 (1H, d, J = 3.6 Hz), 8.21 (1H, d, J = 2.7 Hz), 8.26 (1H, s), 10.46 (1H, s), 11.44 (1H, s), 11.26 (1H, s).
Melting point: 197-204°C
Element analysis: Calculated (%) for C₂₆H₂₀N₅O₃SCl·1.0CF₃CO₂H·1.0H₂O: C, 51.74; H, 3.56; N, 10.77. Found (%): C, 51.74; H, 3.57; N, 10.77.

### Example 47

### N-{4-[3-(β-alanylamino)phenyl]-6-(5-chloro-2-hydroxyphenyl)-3-cyanopyridin-2-yl} benzamide trifluoroacetate

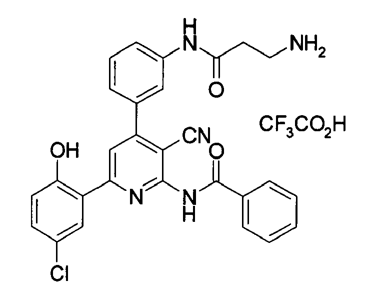

From the compound of Reference Example 53 (80 mg, 0.12 mmol), the title compound (75 mg, 100%) was given as a yellow powder in substantially the same manner as in Example 45.
¹H-NMR (300MHz, DMSO-d₆) δ: 2.76 (2H, t, J = 6.6 Hz), 3.12 (2H, q, J = 6.1 Hz), 7.04 (1H, d, J = 8.7 Hz), 7.43 (2H, dd, J = 8.7, 2.7 Hz), 7.56-7.85 (8H, m), 7.99 (1H, d, J = 1.5 Hz), 8.05 (2H, d, J = 1.2 Hz), 8.08 (1H, s), 8.22 (1H, d, J = 2.7 Hz), 8.27 (1H, s), 10.46 (1H, s), 11.48 (1H, s), 11.86(1H, s).
Melting point: 205-210°C
Element analysis: Calculated (%) for C₂₈H₂₂N₅O₃Cl·1.0CF₃CO₂H·1.0H₂O: C, 55.95; H, 3.91; N, 10.87. Found (%): C, 55.94; H, 3.93; N, 10.80.

### Example 48

### N-[4-(4-aminophenyl)-3-cyano-6-(2-hydroxyphenyl)pyridin-2-yl]thiophene-2-carboxamide

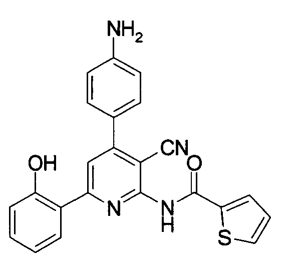

A 1M solution of tetrabutylammonium fluoride in THF (2.5 mL) was added to a solution of the compound of Reference Example 60 (213 mg, 0.40 mmol) in THF (5 mL) with ice-cooling, and stirred at the same temperature for 1.5 hours. The reaction mixture was diluted with water (20 mL) and stirred for 30 minutes. The precipitated crystals were collected by filtration, dried under reduced pressure, and recrystallized from DMF-water to give the title compound (35 mg, 21 %) as a yellow crystal.
¹H-NMR (300MHz, DMSO-d₆) δ: 5.82 (2H, br s), 6.74 (2H, d, J = 8.7 Hz), 6.92-7.06 (2H, m), 7.27-7.47 (2H, m), 7.54 (2H, d, J = 8.7 Hz), 7.94-8.21 (3H, m), 11.31 (1H, br s), 12.11 (1H, br s).
Melting point: 292-293°C
Element analysis: Calculated (%) for C₂₃H₁₆N₄O₂S: C, 66.97; H, 3.91; N, 13.58. Found (%): C, 66.91; H, 3.80; N, 13.33.

### Example 49

### Benzyl [3-(3-{3-cyano-6-(2-hydroxyphenyl)-2-[(2-thienylcarbonyl)amino]pyridin-4-yl} phenoxy)propyl]carbamate

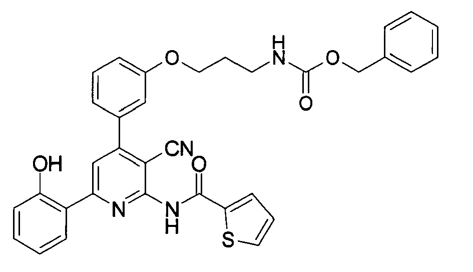

A 1M solution of tetrabutylammonium fluoride in THF (0.5 mL) was added to a solution of the compound of Reference Example 63 (123 mg, 0.17 mmol) in THF (2.5 mL) with ice-cooling, and stirred for 1 hour. The reaction mixture was diluted with ethyl acetate, washed with water and then with saturated brine, dried over magnesium sulfate, and then concentrated under reduced pressure. The residue was purified by silica gel chromatography (eluting solution: ethyl acetate/hexane = 1/9 to 1/1) to give the title compound (49 mg, 47%) as an amorphous solid.
¹H-NMR (300MHz, CDCl₃) δ: 2.02-2.20 (2H, m), 3.40-3.60 (2H, m), 4.10-4.58 (2H, m), 5.02 (1H, br s), 5.13 (2H, s), 6.94 (1H, t, J = 7.2 Hz), 7.08-7.47 (11H, m), 7.51 (1H, t, J = 8.1 Hz), 7.70 (1H, s), 7.78 (1H, s), 7.80 (1H, d, J = 3.6 Hz), 7.84 (1H, d, J = 7.8 Hz), 8.81 (1H, s), 13.25 (1H, s).

### Example 50

### N-{4-[3-(β-alanylamino)phenyl]-3-cyano-6-(2-hydroxy-4-methoxyphenyl)pyridin-2-yl} benzamide trifluoroacetate

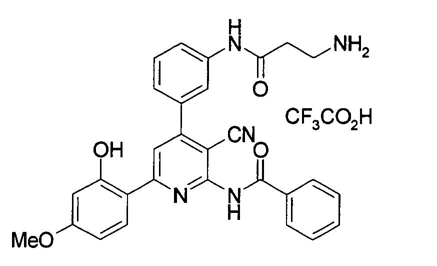

From the compound of Reference Example 73 (100 mg, 0.15 mmol), the title compound (80 mg, 86%) was given as a yellow powder in substantially the same manner as in Example 45.
¹H-NMR (300MHz, DMSO-d₆) δ: 2.76 (2H, t, J = 7.1 Hz), 3.12 (2H, q, J = 5.9 Hz), 3.81 (3H, s), 6.53-6.58 (2H, m), 7.42 (1H, d, J = 7.5 Hz), 7.55-7.85 (8H, m), 7.97 (1H, s), 8.05-8.14 (4H, m), 10.45 (1H, s), 11.45 (1H, s), 12.40(1H, s).
Melting point: 164-170°C
Element analysis: Calculated (%) for C₂₉H₂₅N₅O₄·1.0CF₃CO₂H·1.0H₂O: C, 58.22; H, 4.41; N, 10.95. Found (%): C, 58.45; H, 4.48; N, 10.92.

### Example 51

### N-{4-[3-(β-alanylamino)phenyl]-6-(4-chloro-2-hydroxyphenyl)-3-cyanopyridin-2-yl} benzamide trifluoroacetate

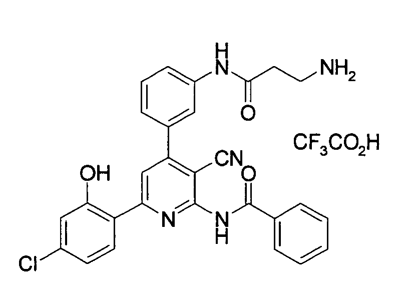

From the compound of Reference Example 77 (55 mg, 0.084 mmol), the title compound (37 mg, 70%) was given as a white powder in substantially the same manner as in Example 45.
¹H-NMR (300MHz, DMSO-d₆) δ: 2.76 (2H, t, J = 6.8 Hz), 3.00-3.20 (2H, br), 7.03-7.10 (2H, m), 7.43 (1H, d, J = 7.5 Hz), 7.56-7.85 (8H, m), 8.02-8.20 (5H, m), 10.47 (1H, s), 11.50 (1H, s), 12.22(1H, s).
Melting point: 160-167°C
Element analysis: Calculated (%) for C₂₈H₂₂N₅O₃Cl·1.0CF₃CO₂H·1.0H₂O: C, 55.18; H, 4.01; N, 10.72. Found (%): C, 55.43; H, 4.10; N, 10.83.

### Example 52

### N-{4-[3-(β-alanylamino)phenyl]-3-cyano-6-(2-hydroxy-6-methoxyphenyl)pyridin-2-yl} benzamide trifluoroacetate

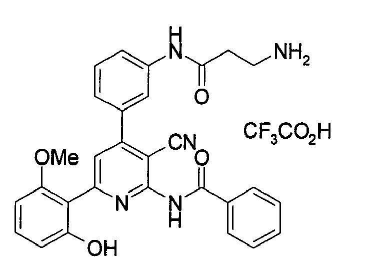

From the compound of Reference Example 85 (200 mg, 0.31 mmol), the title compound (180 mg, 93%) was given as a yellow powder in substantially the same manner as in Example 45.
¹H-NMR (300MHz, DMSO-d₆) δ: 2.76 (2H, t, J = 6.5 Hz), 3.04-3.19 (2H, m), 3.78 (3H, s), 6.63 (2H, t, J = 7.2 Hz), 7.29 (1H, t, J = 8.3 Hz), 7.39 (1H, d, J = 7.2 Hz), 7.54-7.79 (9H, m), 8.03-8.08 (3H, m), 10.46 (1H, s), 10.71 (1H, s), 11.44(1H, s).
Melting point: 160-167°C
Element analysis: Calculated (%) for C₂₉H₂₅N₅O₄·1.0CF₃CO₂H·0.8H₂O: C, 58.55; H, 4.37; N, 11.01. Found (%): C, 58.58; H, 4.36; N, 10.97.

### Example 53

Di-tert-butyl [({2-[(3-{3-cyano-6-(2-hydroxyphenyl)-2-[(2-thienylcarbonyl)-amino]pyridin-4-yl}benzoyl)amino]ethyl}amino)methylylidene]biscarbamate

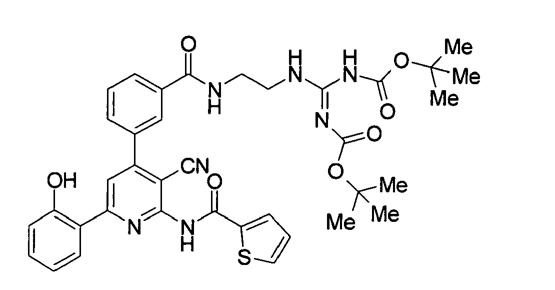

A solution of the compound of Example 3 (100 mg, 0.19 mmol), 1,3-di-Boc-2-(trifluoromethylsulfonyl)guanidine (75 mg, 0.19 mmol) and diisopropylethylamine (50 mg, 0.38 mmol) in DMF (5 mL) was stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1) to give the title compound (60 mg, 43%) as a pale green amorphous solid.
¹H-NMR (300MHz, CDCl₃) δ: 1.47 (9H, s), 1.49 (9H, s), 3.65-3.82 (4H, m), 6.85 (1H, t, J = 7.5 Hz), 7.02 (1H, d, J = 8.4 Hz), 7.17 (1H, t, J = 4.2 Hz), 7.33 (1H, t, J = 7.5 Hz), 7.58-7.68 (3H, m), 7.73-7.82 (3H, m), 7.97 (1H, d, J = 7.5 Hz), 8.21-8.22 (2H, m), 8.76 (1H, t, J = 5.6 Hz), 8.86 (1H, s), 11.48 (1H, s), 13.12 (1H, s).

### Example 54

### N-[4-(3- {[(2-{ [amino(imino)methyl]amino}ethyl)amino]carbonyl}phenyl)-3-cyano-6-(2-hydroxyphenyl)pyridin-2-yl]thiophene-2-carboxamide trifluoroacetate

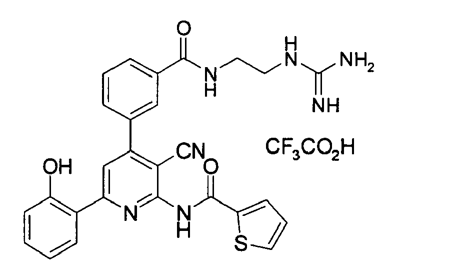

From the compound of Example 53 (60 mg, 0.083 mmol), the title compound (50 mg, 100%) was given as a yellow powder in substantially the same manner as in

### Example 45.

¹H-NMR (300MHz, DMSO-d₆) δ: 3.30-3.50 (4H, m), 6.97-7.44 (8H, m), 7.55 (1H, t, J = 5.4 Hz), 7.75 (1H, t, J = 7.8 Hz), 7.92 (1H, d, J = 7.8 Hz), 8.01 (1H, d, J = 4.8 Hz), 8.08 (1H, d, J = 7.8 Hz), 8.14-8.18 (3H, m), 8.24 (1H, s), 8.80 (1H, t, J = 5.4 Hz), 11.49 (1H, s), 11.83 (1H, s).
Melting point: 220-224°C
Element analysis: Calculated (%) for C₂₇H₂₃N₇O₃S·1.0CF₃CO₂H·0.8H₂O: C, 53.26; H, 3.79; N, 14.99. Found (%): C, 53.30; H, 3.90; N, 14.82.

### Example 55

### N-[4-(4-{[(2-aminoethyl)amino]carbonyl}phenyl)-3-cyano-6-(2-hydroxyphenyl) pyridin-2-yl]thiophene-2-carboxamide hydrochloride

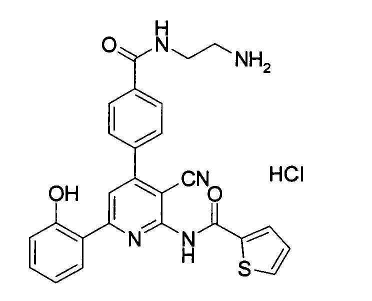

From the compound of Reference Example 88 (120 mg, 0.19 mmol), the title compound (104 mg, 100%) was given as a yellow crystal in substantially the same manner as in Example 8.
¹H-NMR (300MHz, DMSO-d₆) δ: 3.03 (2H, q, J = 5.8 Hz), 3.56-3.65 (2H, m), 6.96-7.04 (2H, m), 7.32 (1H, t, J = 4.4 Hz), 7.41 (1H, t, J = 7.8 Hz), 7.86 (2H, d, J = 8.1 Hz), 8.01-8.18 (8H, m), 8.25 (1H, s), 8.93 (1H, t, J = 5.4 Hz), 11.50 (1H, s), 11.55-11.90 (1H, br).
Melting point: 202-208°C
Element analysis: Calculated (%) for C₂₆H₂₁N₅O₃S·1.0HCl·1.5H₂O: C, 57.09; H, 4.61; N, 12.80. Found (%): C, 57.15; H, 4.64; N, 12.57.

### Example 56

### N-{4-[3-(β-alanylamino)phenyl]-6-(4-chloro-2-hydroxyphenyl)-3-cyanopyridin-2-yl} thiophene-2-carboxamide trifluoroacetate

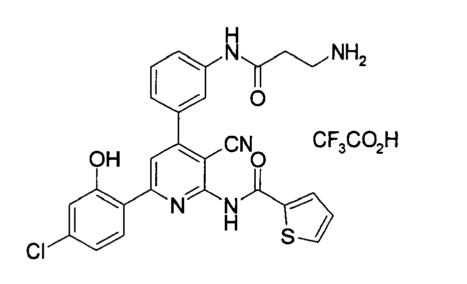

From the compound of Reference Example 80 (100 mg, 0.15 mmol), the title compound (55 mg, 58%) was given as a green powder in substantially the same manner as in Example 45.
¹H-NMR (300MHz, DMSO-d₆) δ: 2.76 (2H, q, J = 6.6 Hz), 3.09-3.15 (2H, m), 7.03-7.10 (2H, m), 7.31 (1H, t, J = 4.4 Hz), 7.41 (1H, t, J = 7.5 Hz), 7.58 (1H, t, J = 8.0 Hz), 7.65-7.80 (4H, m), 8.01 (2H, d, J = 4.4 Hz), 8.13-8.19 (3H, m), 10.47 (1H, s), 11.48 (1H, s), 12.09 (1H, s).
Melting point: 206-209°C
Element analysis: Calculated (%) for C₂₆H₂₀N₅O₃SCl·1.0CF₃CO₂H·2.0H₂O: C, 50.34; H, 3.77; N, 10.48. Found (%): C, 50.19; H, 3.76; N, 10.51.

### Example 57

### N-{4-[3-(β-alanylamino)phenyl]-3-cyano-6-(2-hydroxy-4-methylphenyl)pyridin-2-yl} thiophene-2-carboxamide hydrochloride

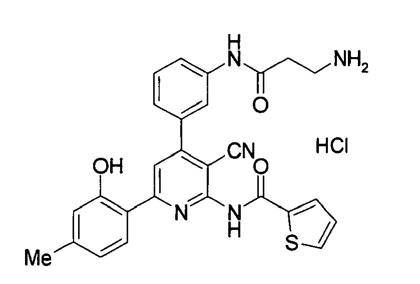

An solution of the compound of Reference Example 91 (200 mg, 0.39 mmol), iron powder (130 mg, 2.33 mmol) and ammonium chloride (135 mg, 2.53 mmol) in ethanol (10 mL)-water (10 mL) was refluxed while heating for 2 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give N-{4-(3-aminophenyl)-3-cyano-6-[2-(ethoxymethoxy)-4-methylphenyl]pyridin-2-yl}thiophene-2-carboxamide(140 mg) as a brown amorphous solid.
WSCD (110 mg, 0.58 mmol) was added to a solution of the resultant N-{4-(3-aminophenyl)-3-cyano-6-[2-(ethoxymethoxy)-4-methylphenyl] pyridin-2-yl}thiophene-2-carboxamide (140 mg), Boc-β-alanine (109 mg, 0.58 mmol) and HOBt (78 mg, 0.58 mmol) in DMF (8 mL) with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/1 to 3/2), and then recrystallized from ethyl acetate-diethylether to give tert-butyl {3-[(3-{3-cyano-6-[2-(ethoxymethoxy)-4-methylphenyl]-2-[(2-thienylcarbonyl)amino]pyridin-4-yl}phenyl)amino]-3-oxopropyl} carbamate (55 mg, 29%) as a yellow solid.

From the resultant tert-butyl {3-[(3-{3-cyano-6-[2-(ethoxymethoxy)-4-methylphenyl]-2-[(2-thienylcarbonyl)amino]pyridin-4-yl}phenyl)amino]-3-oxopropyl} carbamate (50 mg, 0.076 mmol), the title compound (41 mg, 100%) was given as a yellow powder in substantially the same manner as in Example 8.
¹H-NMR (300MHz, DMSO-d₆) δ: 2.31 (3H, s), 2.79 (2H, t, J = 6.8 Hz), 3.10 (2H, t, J = 6.0 Hz), 6.79-6.84 (2H, m), 7.31 (1H, t, J = 4.4 Hz), 7.41 (1H, t, J = 7.8 Hz), 7.57 (1H, t, J = 7.8 Hz), 7.82-7.96 (4H, m), 8.00-8.07 (3H, m), 8.14 (2H, d, J = 3.9 Hz), 10.54 (1H, s), 11.43 (1H, s), 11.91 (1H, s).
Melting point: 204-210°C
Element analysis: Calculated (%) for C₂₇H₂₃N₅O₃S·1.0HCl·1.2H₂O: C, 58.36; H, 4.79; N, 12.60. Found (%): C, 58.42; H, 4.90; N, 12.30.

### Example 58

### N-{4-[3-(β-alanylamino)phenyl]-3-cyano-6-(4-fluoro-2-hydroxyphenyl)pyridin-2-yl} thiophene-2-carboxamide hydrochloride

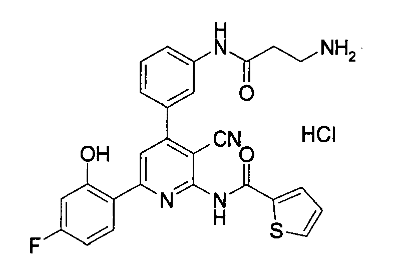

From the compound of Reference Example 95 (55 mg, 0.083 mmol), the title compound (46 mg, 100%) was given as a yellow powder in substantially the same manner as in Example 8.
¹H-NMR (300MHz, DMSO-d₆) δ: 2.80 (2H, t, J = 6.8 Hz), 3.05-3.15 (2H, m), 6.81-6.90 (2H, m), 7.31 (1H, t, J = 4.4 Hz), 7.41 (1H, t, J = 7.8 Hz), 7.57 (1H, t, J = 7.8 Hz), 7.83 (1H, d, J = 8.4 Hz), 7.85-8.02 (5H, m), 8.15-8.25 (3H, m), 10.58 (1H, s), 11.47 (1H, s), 12.25 (1H, s).
Melting point: 217-220°C
Element analysis: Calculated (%) for C₂₆H₂₀N₅O₃SF·1.0HCl·1.5H₂O: C, 55.27; H, 4.28; N, 12.40. Found (%): C, 55.20; H, 4.31; N, 12.39.

### Example 59

### N-[4-{3-[(N-acetyl-β-alanyl)amino]phenyl}-6-(4-chloro-2-hydroxyphenyl)-3-cyanopyridin-2-yl]thiophene-2-carboxamide

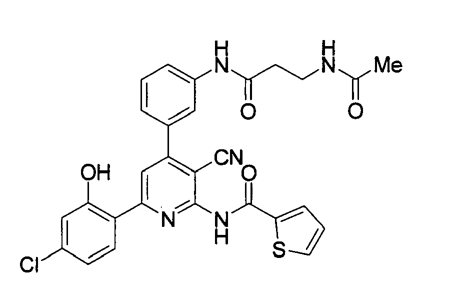

Anhydrous acetic acid (4.4 mg, 0.043 mmol) was added to a solution of the compound of Example 56 (27 mg, 0.043 mmol) and triethylamine (9 mg, 0.085 mmol) in dichloromethane (5 mL) with ice-cooling, and stirred at room temperature for 1 hour. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/methanol = 100/1), and then recrystallized from ethyl acetate-hexane to give the title compound (15 mg, 62%) as a white crystal.
¹H-NMR (300MHz, DMSO-d₆) δ: 1.80 (3H, s), 2.50-2.55 (2H, m), 3.31-3.36 (2H, m), 7.02-7.08 (2H, m), 7.30 (1H, t, J = 4.2 Hz), 7.3 (1H, t, J = 7.2 Hz), 7.55 (1H, t, J = 8.0 Hz), 7.81 (1H, d, J = 7.8 Hz), 7.95-8.01 (3H, m), 8.12-8.19 (3H, m), 10.24 (1H, s), 11.45 (1H, s), 12.00-12.25 (1H, br).
Melting point: 209-210°C
Element analysis: Calculated (%) for C₂₈H₂₂N₅O₄SCl·1.0H₂O: C, 58.18; H, 4.18; N, 12.12. Found (%): C, 58.26; H, 4.25; N, 12.01.

### Example 60

### N- {4-[3-(β-alanylamino)phenyl]-3-cyano-6-(2-hydroxy-5-methoxyphenyl)pyridin-2-yl} thiophene-2-carboxamide hydrochloride

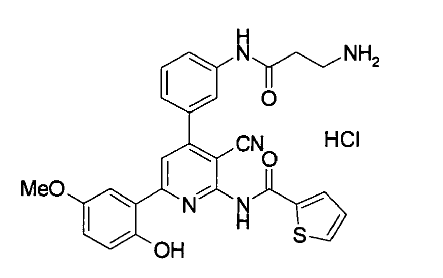

From the compound of Reference Example 100 (100 mg, 0.15 mmol), the title compound (90 mg, 100%) was given as an orange crystal in substantially the same manner as in Example 8.
¹H-NMR (300MHz, DMSO-d₆) δ: 2.79 (2H, t, J = 6.6 Hz), 3.10 (2H, q, J = 6.0 Hz), 3.78 (3H, s), 6.95-7.05 (2H, m), 7.31 (1H, t, J = 4.4 Hz), 7.40 (1H, d, J = 7.5 Hz), 7.55-7.63 (2H, m), 7.82-8.00 (4H, m), 8.01 (2H, d, J = 4.0 Hz), 8.15 (1H, d, J = 4.0 Hz), 8.22 (1H, s), 10.55 (1H, s), 11.20-11.45 (1H, br), 11.49 (1H, s).
Melting point: 197-201°C
Element analysis: Calculated (%) for C₂₇H₂₃N₅O₄S·1.0HCl·2.5H₂O: C, 54.50; H, 4.91; N, 11.77. Found (%): C, 54.46; H, 4.78; N, 11.75.

### Example 61

### N-{4-[3-(β-alanylamino)phenyl]-6-(4-bromo-2-hydroxyphenyl)-3-cyanopyridin-2-yl} thiophene-2-carboxamide hydrochloride

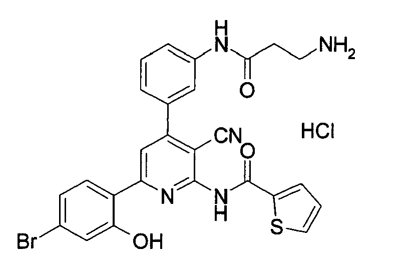

From the compound of Reference Example 105 (40 mg, 0.057 mmol), the title compound (35 mg, 100%) was given as an orange crystal in substantially the same manner as in Example 8.
¹H-NMR (300MHz, DMSO-d₆) δ: 2.78 (2H, t, J = 6.6 Hz), 3.05-3.16 (2H, m), 7.17 (1H, t, J = 8.7 Hz), 7.26-7.33 (2H, m), 7.40 (1H, d, J = 7.2 Hz), 7.57 (1H, t, J = 8.1 Hz), 7.80-7.98 (4H, m), 8.00-8.18 (5H, m), 10.53 (1H, s), 11.44 (1H, s), 11.90-12.05 (1H, br).
Melting point: 203-211 °C
Element analysis: Calculated (%) for C₂₆H₂₀N₅O₃SBr·1.0HCl·1.0H₂O: C, 48.50; H, 4.07; N, 10.88. Found (%): C, 48.40; H, 4.35; N, 11.06.

### Example 62

### tert-butyl 4-(3-aminophenyl)-2-(benzoylamino)-6-(2-hydroxyphenyl)nicotinate

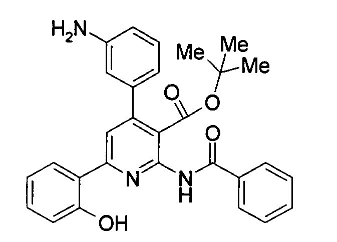

An ethanol (20 mL) mixture of the compound of Reference Example 106 (200 mg, 0.33 mmol) and palladium-carbon (100 mg) was stirred at room temperature under 3 atm of hydrogen atmosphere for 40 hours. The catalyst was filtered out, and the filtrate was concentrated. The residue was recrystallized from diethylether-diisopropylether to give the title compound (21 mg, 13%) as a yellow crystal.
¹H-NMR (300MHz, CDCl₃) δ: 1.26 (9H, s), 6.63 (1H, t, J = 2.1 Hz), 6.70-6.78 (2H, m), 6.87 (1H, t, J = 7.8 Hz), 7.11 (1H, dd, J = 8.4, 1.2 Hz), 7.21-7.26 (1H, m), 7.34 (1H, t, J = 7.8 Hz), 7.51-7.60 (4H, m), 7.80 (1H, d, J = 8.1 Hz), 8.10 (2H, dd, J = 7.8, 1.2 Hz), 11.59 (1H, s), 13.80 (1H, s).
Melting point: 162-164°C
Element analysis: Calculated (%) for C₂₉H₂₇N₃O₄·1.0H₂O: C, 69.72; H, 5.85; N, 8.41. Found (%): C, 69.70; H, 5.98; N, 8.04.

### Example 63

### N-{4-[3-(β-alanylamino)phenyl]-6-(4-chloro-2-hydroxyphenyl)-3-cyanopyridin-2-yl}-2-furamide hydrochloride

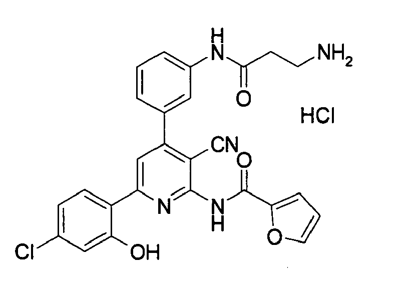

From the compound of Reference Example 109 (350 mg, 0.54 mmol), the title compound (308 mg, 100%) was given as a yellow crystal in substantially the same manner as in Example 8.
¹H-NMR (300MHz, DMSO-d₆) δ: 2.78 (2H, t, J = 6.8 Hz), 3.10 (2H, q, J = 6.3 Hz), 6.80 (1H, q, J = 1.6 Hz), 7.03 (1H, dd, J = 8.4, 1.8 Hz), 7.10 (1H, d, J = 1.8 Hz), 7.40 (1H, d, J = 7.5 Hz), 7.54 (1H, d, J = 3.6 Hz), 7.58 (1H, t, J = 7.8 Hz), 7.75-7.90 (4H, m), 8.00 (1H, s), 8.07 (1H, s), 8.18 (2H, d, J = 6.9 Hz), 10.52 (1H, s), 11.28 (1H, s), 12.14 (1H, br s).
Melting point: 206-212°C
Element analysis: Calculated (%) for C₂₆H₂₀N₅O₄Cl·1.0HCl·2.4H₂O: C, 53.69; H, 4.47; N, 12.01. Found (%): C, 53.70; H, 4.53; N, 11.91.

### Example 64

### N-{4-[3-(β-alanylamino)phenyl]-3-cyano-6-[2-hydroxy-4-(trifluoromethyl)phenyl] pyridin-2-yl}thiophene-2-carboxamide hydrochloride

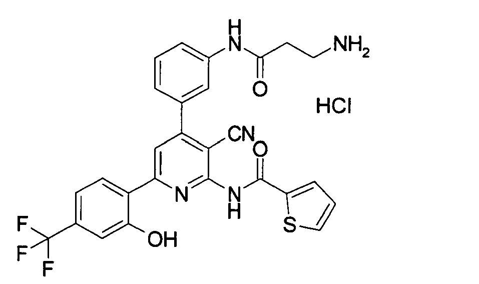

From the compound of Reference Example 118 (120 mg, 0.17 mmol), the title compound (101 mg, 100%) was given as a yellow crystal in substantially the same manner as in Example 8.
¹H-NMR (300MHz, DMSO-d₆) δ: 2.77-2.82 (2H, m), 3.06-3.14 (2H, m), 7.23-7.33 (2H, m), 7.40-7.43 (2H, m), 7.58 (1H, t, J = 8.1 Hz), 7.82-7.99 (4H, m), 8.00-8.04 (2H, m), 8.17 (1H, d, J = 3.6 Hz), 8.25 (1H, s), 8.31 (1H, d, J = 8.1 Hz), 10.56 (1H, s), 11.54 (1H, s), 11.98 (1H, s).
Melting point: 209-216°C
Element analysis: Calculated (%) for C₂₇H₂₀N₅O₃SF₃·1.0HCl·1.3H₂O: C, 53.03; H, 3.89; N, 11.45. Found (%): C, 53.11; H, 4.01; N, 11.34.

### Example 65

### N-[4-(3-{[(1-aminocyclobutyl)carbonyl]amino }phenyl)-6-(4-chloro-2-hydroxyphenyl)-3-cyanopyridin-2-yl]-2-furamide hydrochloride

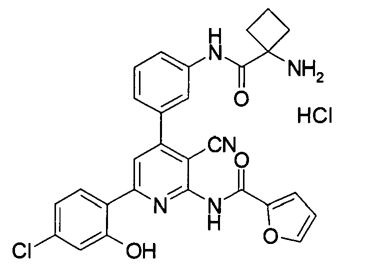

From the compound of Reference Example 110 (50 mg, 0.074 mmol), the title compound (39 mg, 93%) was given as a yellow crystal in substantially the same manner as in Example 8.
¹H-NMR (300MHz, DMSO-d₆) δ: 1.91-2.10 (1H, br), 2.20-2.40 (3H, m), 2.80-2.94 (2H, m), 6.80 (1H, q, J = 1.8 Hz), 7.04 (1H, dd, J = 8.7, 2.1 Hz), 7.10 (1H, d, J = 2.1 Hz), 7.49-7.54 (2H, m), 7.64 (1H, t, J = 8.1 Hz), 7.92 (1H, d, J = 7.5 Hz), 8.08 (2H, d, J = 7.8 Hz), 8.18 (1H, d, J = 7.5 Hz), 8.19 (1H, s), 8.73 (3H, br s), 10.55 (1H, s), 11.32 (1H, s), 12.20 (1H, s).
Melting point: 195-200°C
Element analysis: Calculated (%) for C₂₈H₂₂N₅O₄Cl·1.0HCl·2.0H₂O: C, 56.01; H, 4.53; N, 11.66. Found (%): C, 56.00; H, 4.60; N, 11.67.

### Example 66

### N-[4-(3-{[(2-aminoethyl)amino]carbonyl}phenyl)-3-cyano-6-(4-fluoro-2-hydroxyphenyl)pyridin-2-yl]-2-furamide hydrochloride

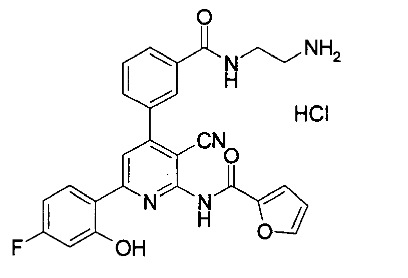

From the compound of Reference Example 141 (250 mg, 0.40 mmol), the title compound (114 mg, 55%) was given as a yellow powder in substantially the same manner as in Example 8.
¹H-NMR (300MHz, DMSO-d₆) δ: 2.95-3.05 (2H, m), 3.55-3.65 (2H, m), 6.80-6.90 (3H, m), 7.54 (1H, d, J = 3.6 Hz), 7.75 (1H, t, J = 7.5 Hz), 7.92 (1H, d, J = 7.8 Hz), 8.01 (3 H, br s), 8.05-8.15 (2H, m), 8.26 (2H, s), 8.32 (1H, t, J = 7.5 Hz), 8.98 (1H, t, J = 5.4 Hz), 11.31 (1H, s), 12.45 (1H, s).
Melting point: 205-207°C

### Example 67

### N-{3-cyano-6-(4-fluoro-2-hydroxyphenyl)-4-[3-({[3-(1H-imidazole-1-yl)propyl]amino} carbonyl)phenyl]pyridin-2-yl}-2-furamide hydrochloride

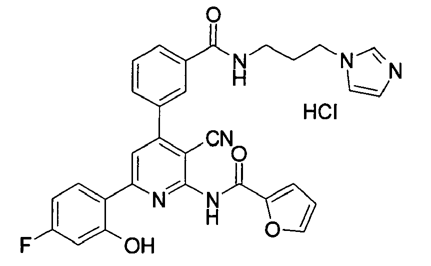

From the compound of Reference Example 142 (300 mg, 0.50 mmol), the title compound (241 mg, 81 %) was given as a yellow powder in substantially the same manner as in Example 3.
¹H-NMR (300MHz, DMSO-d₆) δ: 2.00-2.20 (2H, m), 3.30-3.50 (2H, m), 4.20-4.40 (2H, m), 6.75-6.90 (3H, m), 7.55 (1H, s), 7.69 (1H, s), 7.74 (1H, t, J = 7.8 Hz), 7.80-7.95 (2H, m), 8.08 (2H, s), 8.20-8.35 (3H, m), 8.80-9.00 (1H, m), 9.17 (1H, d, J = 17.1 Hz), 11.30 (1H, s), 12.50 (1H, s), 14.2-14.8 (1H, br).
Melting point: 173-174°C
Element analysis: Calculated (%) for C₃₀H₂₃N₆O₄F·1.0HCl·4.0H₂O: C, 54.67; H, 4.89; N. 12.75. Found (%): C, 54.87; H, 4.88; N, 12.73.

### Example 68

### N-{4-[3-(β-alanylamino)phenyl]-3-cyano-6-(4-fluoro-2-hydroxyphenyl)pyridin-2-yl}-2-furamide hydrochloride

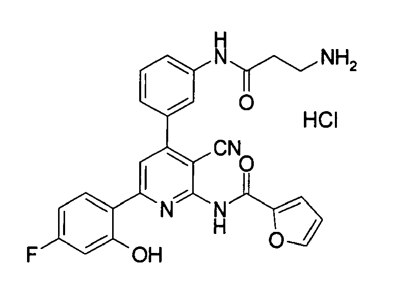

The compound of Reference Example 136 (97 mg, 0.154 mmol) was dissolved in a 10% methanol solution of hydrogen chloride (6 mL) and stirred at room temperature for 3.5 hours. The excessive hydrochloric acid and solvent were removed under reduced pressure. The resultant solid was washed with hot ethyl acetate and cold ethyl acetate, and recrystallized from hot ethanol/diethylether to give the title compound (25.6 mg, 32%) as a yellow crystal.
¹H NMR (300 MHz, DMSO-d₆) δ: 2.70-2.80 (2H, m), 3.11 (2H, t, J = 6.8 Hz), 6.76-6.89 (3H, m), 7.41 (1H, d, J = 7.5 Hz), 7.50 (1H, br s), 7.58 (1H, t, J = 8.1 Hz), 7.60-7.95 (4H, m), 7.99 (1H, s), 8.03-8.14 (2H, m), 8.22 (1H, dd, J = 9.8, 6.8 Hz), 10.47 (1H, br s), 12.40 (2H, br s).
Melting point: 269-270°C
Element analysis: Calculated (%) for C₂₆H₂₀N₅O₄F·1.0HCl·2.0H₂O: C,55.97; H, 4.52; N, 12.55. Found (%): C, 55.85; H, 4.42; N, 12.31.

### Example 69

### N-[4-(3-{ [(1-aminocyclopropyl)carbonyl]amino}phenyl)-6-(4-chloro-2-hydroxyphenyl)-3-cyanopyridin-2-yl]-2-furamide hydrochloride

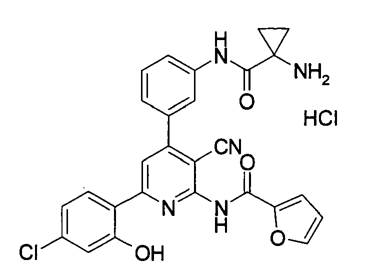

From the compound of Reference Example 111 (120 mg, 0.18 mmol), the title compound (102 mg, 100%) was given as a yellow crystal in substantially the same manner as in Example 8.
¹H-NMR (300MHz, DMSO-d₆) δ: 1.44 (2H, t, J = 6.9 Hz), 1.71 (2H, t, J = 6.9 Hz), 6.80 (1H, q, J = 1.7 Hz), 7.03 (1H, dd, J = 8.4, 2.1 Hz), 7.12 (1H, d, J = 2.1 Hz), 7.46 (1H, d, J = 7.8 Hz), 7.54-7.63 (2H, m), 7.86 (1H, d, J = 8.4 Hz), 8.06 (1H, s), 8.07 (1H, t, J = 1.2 Hz), 8.18 (2H, t, J = 4.4 Hz), 8.87 (3H, br s), 9.87 (1H, s), 11.33 (1H, s), 11.90-12.30 (1H, br).
Melting point: 190-196°C
Element analysis: Calculated (%) for C₂₇H₂₀N₅O₄Cl·1.0HCl·1.0H₂O: C, 57.05; H, 4.08; N, 12.32. Found (%): C, 57.03; H, 4.26; N, 12.08.

### Example 70

### N-{6-(4-chloro-2-hydroxyphenyl)-3-cyano-4-[3-({[3-(1H-imidazole-1-yl)propyl] amino}carbonyl)phenyl]pyridin-2-yl} -2-methoxybenzamide hydrochloride

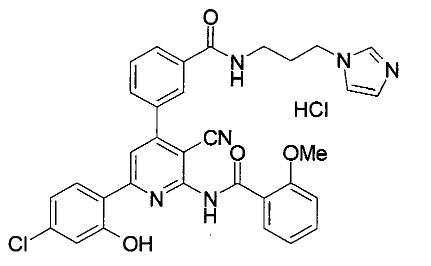

A 4N hydrogen chloride-ethyl acetate solution (2 mL) was added to a solution of the compound of Reference Example 127 (190 mg, 0.29 mmol) in dichloromethane (10 mL), and stirred at room temperature for 2 hours. The precipitate was collected by filtration, and recrystallized from methanol-diethylether to give the title compound (168 mg, 90%) as a yellow crystal.
¹H-NMR (300MHz, DMSO-d₆) δ: 2.09-2.15 (2H, m), 3.30-3.35 (2H, m), 4.10 (3H, s), 4.29 (2H, t, J = 6.8 Hz), 7.00 (1H, dd, J = 8.4, 2.1 Hz), 7.08 (1H, d, J = 3.2 Hz), 7.22 (1H, t, J = 7.5 Hz), 7.35 (1H, d, J = 8.4 Hz), 7.67-7.77 (3H, m), 7.84 (1H, s), 7.96 (1H, d, J = 7.8 Hz), 8.10 (2H, t, J = 6.9 Hz), 8.20 (1H, s), 8.25 (1H, s), 8.31 (1H, d, J = 8.7 Hz), 8.99 (1H, br s), 9.18 (1H, s), 11.38 (1H, s), 13.30 (1H, s).
Melting point: 234-237°C
Element analysis: Calculated (%) for C₃₃H₂₇N₆O₄Cl·1.0HCl·1.3H₂O: C, 59.43; H, 4.62; N, 12.60. Found (%): C, 59.52; H, 4.61; N, 12.67.

### Example 71

### N-[6-(4-chloro-2-hydroxyphenyl)-3-cyano-4-(3-{[(piperidine-2-ylmethyl)amino] carbonyl}phenyl)pyridin-2-yl]-2-methoxybenzamide hydrochloride

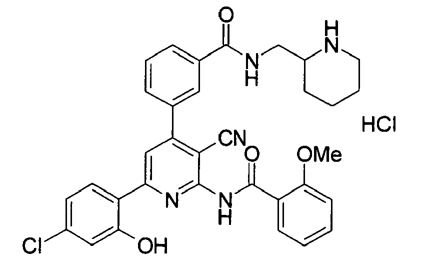

A 4N hydrogen chloride-ethyl acetate solution (2 mL) was added to a solution of the compound of Reference Example 128 (220 mg, 0.24 mmol) in dichloromethane(10 mL), and stirred at room temperature for 2 hours. The precipitate was collected by filtration, and recrystallized from methanol-diethylether to give the title compound (155 mg, 100%) as a yellow crystal.
¹H-NMR (300MHz, DMSO-d₆) δ: 1.40-1.80 (7H, m), 3.05 (1H, dd, J = 13.2, 5.4 Hz), 3.31-3.42 (1H, m), 3.50-3.61 (1H, m), 4.10 (3H, s), 4.95 (1H, br s), 7.00 (1H, dd, J = 8.4, 2.1 Hz), 7.08 (1H, d, J = 2.1 Hz), 7.22 (1H, t, J = 7.1 Hz), 7.35 (1H, d, J = 8.1 Hz), 7.67-7.74 (3H, m), 7.85-8.05 (5H, m), 8.11 (2H, dd, J = 8.7, 2.4 Hz), 8.26 (1H, d, J = 9.0 Hz), 11.38 (1H, s), 13.20-13.50 (1H, br).
Melting point: 210-217°C
Element analysis: Calculated (%) for C₃₃H₃₀N₅O₄Cl·1.0HCl·1.0H₂O: C, 60.93; H, 5.11; N, 10.77. Found (%): C, 61.03; H, 5.01; N, 10.67.

### Example 72

### N-[4-(3-{ [(2-aminoethyl)amino]carbonyl}phenyl)-6-(4-chloro-2-hydroxyphenyl)-3-cyanopyridin-2-yl]-2-methoxybenzamide hydrochloride

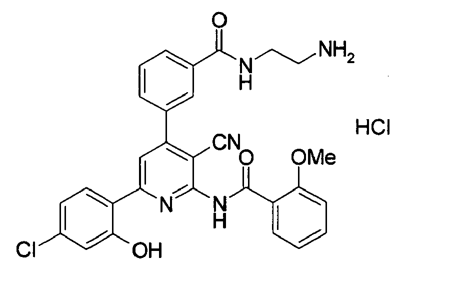

From the compound of Reference Example 126 (240 mg, 0.35 mmol), the title compound (194 mg, 96%) was given as a yellow crystal in substantially the same manner as in Example 71.
¹H-NMR (300MHz, DMSO-d₆) δ: 3.03 (2H, t, J = 6.2 Hz), 3.57 (2H, q, J = 5.9 Hz), 4.10 (3H, s), 7.00 (1H, dd, J = 8.4, 2.1 Hz), 7.08 (1H, d, J = 2.1 Hz), 7.22 (1H, t, J = 7.4 Hz), 7.35 (1H, d, J = 8.1 Hz), 7.67-7.79 (2H, m), 7.83-7.99 (4H, m), 8.11 (2H, dd, J = 7.8, 1.5 Hz), 8.28 (1H, s), 8.30 (1H, s), 8.35 (1H, d, J = 8.4 Hz), 8.93 (1H, t, J = 5.4 Hz), 11.40 (1H, s), 13.32 (1H,s).
Melting point: 227-229°C
Element analysis: Calculated (%) for C₂₉H₂₄N₅O₄Cl·1.0HCl·2.0H₂O: C, 56.69; H, 4.76; N, 11.40. Found (%): C, 56.77; H, 4.79; N, 11.37.

### Example 73

### N-[4-(3-{[(2-aminoethyl)amino]carbonyl}-4-methoxyphenyl)-3-cyano-6-(2-hydroxyphenyl)pyridin-2-yl]thiophene-2-carboxamide hydrochloride

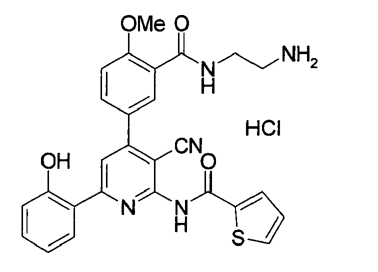

From the compound of Reference Example 67 (200 mg, 0.30 mmol), the title compound (140 mg, 84%) was given as a yellow powder in substantially the same manner as in Example 3.
¹H-NMR (300MHz, DMSO-d₆) δ: 3.00 (2H, t, J = 6.3 Hz), 3.58 (2H, dd, J = 12.0, 6.3 Hz), 4.01 (3H, s), 6.94-7.06 (2H, m), 7.31 (1H, dd, J = 4.8, 3.6 Hz), 7.35-7.46 (2H, m), 7.91 (1H, dd, J = 8.7, 2.4 Hz), 7.93-8.10 (3H, m), 8.12-8.25 (4H, m), 8.57 (1H, t, J = 5.7 Hz), 11.45 (1H, br s), 11.89 (1H, br s).
Melting point: 227-228°C

### Example 74

### N-[4-[3-(β-alanylamino)phenyl]-6-(4-chloro-2-hydroxyphenyl)-3-cyanopyridin-2-yl]-2-ethoxybenzamide hydrochloride

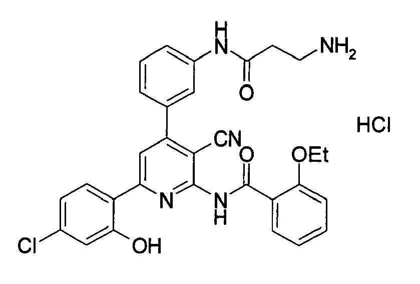

2-ethoxybenzoyl chloride (375 mg, 2.04 mmol) was added to a solution of the compound of Reference Example 130 (150 mg, 0.27 mmol) in pyridin (5 mL), and stirred at room temperature for 42 hours. A 25% ammonia water (1 mL) was added to the resultant mixture, and stirred for another hour. The reaction mixture was diluted with water, and extracted with ethyl acetate. The extract was washed with saturated brine, dried over magnesium sulfate, and then concentrated under reduced pressure.
The residue was purified by silica gel chromatography (eluting solution: ethyl acetate/hexane = 2/1), and then recrystallized from ethyl acetate-hexane to give tert-butyl {2-[(3-{6-[4-chloro-2-(methoxymethoxy)phenyl]-3-cyano-2-[(2-ethoxybenzoyl)amino]pyridin-4-yl}benzoyl)amino]ethyl}carbamate (226 mg) as a white powder.

A 4N hydrogen chloride-ethyl acetate solution (2 mL) was added to a solution of the resultant tert-butyl {2-[(3-{6-[4-chloro-2-(methoxymethoxy)phenyl]-3-cyano-2-[(2-ethoxybenzoyl)amino]pyridin-4-yl}benzoyl) amino]ethyl}carbamate (200 mg) in dichloromethane(10 mL), and stirred at room temperature for 2 hours. The precipitate was collected by filtration, and recrystallized from methanol-diethylether to give the title compound (62 mg, 42%) as a yellow crystal.
¹H-NMR (300MHz, DMSO-d₆) δ: 1.44 (3H, t, J = 6.9 Hz), 2.80 (2H, t, J = 6.8 Hz), 3.04-3.15 (2H, m), 4.43 (2H, q, J = 6.9 Hz), 7.00 (1H, d, J = 8.7 Hz), 7.08 (1H, d, J = 1.8 Hz), 7.18 (1H, t, J = 7.5 Hz), 7.32 (1H, d, J = 8.4 Hz), 7.44 (1H, d, J = 7.2 Hz), 7.55-7.67 (2H, m), 7.84-8.04 (6H, m), 8.08 (1H, s), 8.19 (1H, d, J = 8.7 Hz), 10.55 (1H, s), 11.22 (1H, s), 12.99 (1H, s).
Melting point: 240-244°C
Element analysis: Calculated (%) for C₃₀H₂₆N₅O₄Cl·1.0HCl·1.5H₂O: C, 58.16; H, 4.88; N, 11.30. Found (%): C, 57.90; H, 4.75; N, 11.05.

### Example 75

### N-[4-[3-(β-alanylamino)phenyl]-6-(4-chloro-2-hydroxyphenyl)-3-cyanopyridin-2-yl]-2-(trifluoromethoxy)benzamide hydrochloride

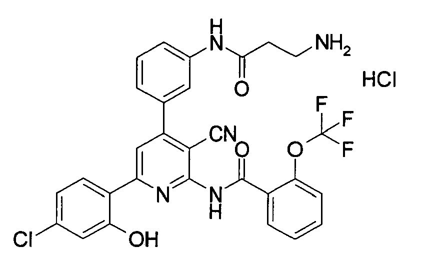

2-(trifluoromethoxy)benzoyl chloride (459 mg, 2.04 mmol) was added to a solution of the compound of Reference Example 130 (150 mg, 0.27 mmol) in pyridin (5 mL), and stirred at room temperature for 42 hours. A 25% ammonia water (1 mL) was added to the resultant mixture, and stirred for another hour. The reaction mixture was diluted with water, and extracted with ethyl acetate. The extract was washed with saturated brine, dried over magnesium sulfate, and then concentrated under reduced pressure. The residue was purified by silica gel chromatography (eluting solution: ethyl acetate/hexane = 2/1), and then recrystallized from ethyl acetate-hexane to give tert-butyl (2-{ [3-(6-[4-chloro-2-(methoxymethoxy) phenyl]-3 -cyano-2- {[2-(trifluoromethoxy)benzoyl]amino}pyridin-4-yl)benzoyl]amino} ethyl)carbamate (197 mg) as a white powder.

A 4N hydrogen chloride-ethyl acetate solution (2 mL) was added to a solution of the resultant tert-butyl (2-{[3-(6-[4-chloro-2-(methoxymethoxy)phenyl] -3 -cyano-2-{[2-(trifluoromethoxy)benzoyl]amino}pyridin-4-yl)benzoyl]amino}ethyl)carbamate (180 mg) in dichloromethane(10 mL), and stirred at room temperature for 2 hours. The precipitate was collected by filtration, and recrystallized from methanol-diethylether to give the title compound (55 mg, 35%) as a yellow crystal.
¹H-NMR (300MHz, DMSO-d₆) δ: 2.79 (2H, t, J = 6.9 Hz), 3.10 (2H, q, J = 6.1 Hz), 7.01 (1H, dd, J = 8.7, 2.1 Hz), 7.11 (1H, d, J = 2.1 Hz), 7.40 (1H, d, J = 8.4 Hz), 7.55-7.63 (3H, m), 7.71-8.08 (8H, m), 8.18 (1H, s), 10.57 (1H, s), 11.66 (1H, s), 12.15 (1H, s).
Melting point: 218-223°C
Element analysis: Calculated (%) for C₂₉H₂₁N₅O₄ClF₃·1.0HCl·0.5H₂O: C, 54.30; H, 3.61; N, 10.92. Found (%): C, 54.14; H, 3.91; N, 10.62.

### Example 76

### N-(3-cyano-6-(4-fluoro-2-hydroxyphenyl)-4-{3-[(pyrrolidin-3-ylamino)carbonyl] phenyl}pyridin-2-yl)-2-furamide hydrochloride

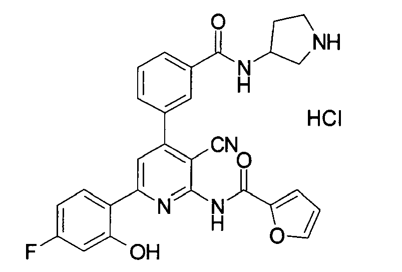

From the compound of Reference Example 143 (250 mg, 0.38 mmol), the title compound (139 mg, 65%) was given as a yellow powder in substantially the same manner as in Example 3.
¹H-NMR (300MHz, DMSO-d₆) δ: 2.00-2.15 (1H, m), 2.15-2.30 (1H, m), 3.20-3.35 (2H, m), 3.35-3.50 (2H, m), 4.50-4.70 (1H, m), 6.75-6.90 (3H, m), 7.54 (1H, d, J = 3.6 Hz), 7.74 (1H, t, J = 7.8 Hz), 7.92 (1H, d, J = 7.8 Hz), 8.05-8.20 (2H, m), 8.25-8.30 (2H, m), 8.37 (1H, t, J = 7.8 Hz), 9.05 (1H, d, J = 6.3 Hz), 9.17 (1H, br), 9.32 (1H, br), 11.30 (1H, s), 12.48 (1H, s).
Melting point: 225-226°C
Element analysis: Calculated (%) for C₂₈H₂₂N₅O₄F·1.0HCl·1.5H₂O: C, 58.49; H, 4.56; N. 12.18. Found (%): C, 58.29; H, 4.70; N, 12.14.

### Example 77

### N-[4-(3-{[(3-amino-2,2-dimethylpropyl)amino]carbonyl}phenyl)-3-cyano-6-(4-fluoro-2-hydroxyphenyl)pyridin-2-yl]-2-furamide hydrochloride

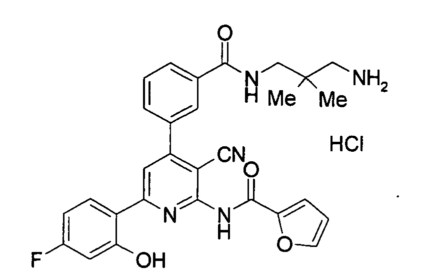

From the compound of Reference Example 144 (250 mg, 0.37 mmol), the title compound (122 mg, 58%) was given as a yellow powder in substantially the same manner as in Example 3.
¹H-NMR (300MHz, DMSO-d₆) δ: 0.99 (6H, s), 2..60-2.75 (2H, m), 3.20-3.35 (2H, m), 6.75-6.90 (3H, m), 7.54 (1H, d, J = 3.6 Hz), 7.70-8.00 (5H, m), 8.05-8.15 (2H, m), 8.20-8.35 (3H, m), 9.02 (1H, t, J = 3.3 Hz), 11.31 (1H, s), 12.40 (1H, s).
Melting point: 256-257°C
Element analysis: Calculated (%) for C₂₉H₂₆N₅O₄F·1.0HC1·0.5H₂O: C, 60.79; H, 4.93; N. 12.22. Found (%): C, 60.53; H, 5.04; N, 12.22.

### Example 78

### N-{3-cyano-6-(4-fluoro-2-hydroxyphenyl)-4-[3-({[3-(1H-imidazole-1-yl)propyl]amino} carbonyl)phenyl]pyridin-2-yl}thiophene-2-carboxamide hydrochloride

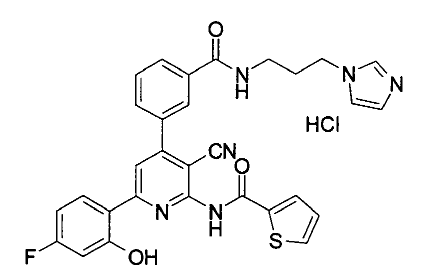

WSCD (236 mg, 1.23 mmol) was added to a solution of the mixture of Reference Example 140 (250 mg), 3-(1H-imidazole-1-yl)propane-1-amine (154 mg, 1.23 mmol) and HOBt (166 mg, 1.23 mmol) in DMF (25 mL) with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate to ethyl acetate/methanol = 10/1) to give a mixture of N-{3-cyano-6-[4-fluoro-2-(methoxymethoxy)phenyl]-4-[3-({[3-(1H-imidazole-1-yl)propyl]amino}carbonyl)phenyl]pyridin-2-yl}thiophene-2-carboxamide and N-{3-cyano-6-(4-fluoro-2-hydroxyphenyl)-4-[3-({[3-(1H-imidazole-1-yl)propyl]amino}carbonyl)phenyl]pyridin-2-yl}thiophene-2-carboxamide as a white solid.

A 4N hydrogen chloride-ethyl acetate solution (4 mL) was added to a solution of the resultant mixture in ethyl acetate (6 mL), and stirred at room temperature for 2 hours. The reaction mixture was concentrated, and the residue was recrystallized from methanol-diethylether to give the title compound (175 mg) as a pale yellow crystal.
¹H-NMR (300MHz, DMSO-d₆) δ: 2.00-2.20 (2H, m), 3.20-3.50 (2H, m), 4.20-4.40 (2H, m), 6.80-6.95 (2H, m), 7.32 (1H, t, J = 3.9 Hz), 7.70 (1H, s), 7.74 (1H, t, J = 7.8 Hz), 7.85-7.95 (2H, m), 8.01 (1H, d, J =5.1 Hz), 8.11 (1H, d, J = 7.5 Hz), 8.19 (1H, d, J = 3.6 Hz), 8.25-8.40 (3H, m), 9.00-9.10 (1H, m), 9.27 (1H, s), 11.58 (1H, s), 12.40 (1H, s), 14.3-14.8 (1H, br).
Melting point: 195-196°C
Element analysis: Calculated (%) for C₃₀H₂₃N₆O₃SF·1.0HCl·0.5Et₂O·0.5H₂O: C, 58.62; H, 4.44; N. 13.23. Found (%): C, 58.87; H, 4.77; N, 12.79.

### Example 79

### N-(3-cyano-6-(4-fluoro-2-hydroxyphenyl)-4-{3-[(pyrrolidine-3-ylamino)carbonyl] phenyl}pyridin-2-yl)thiophene-2-carboxamide hydrochloride

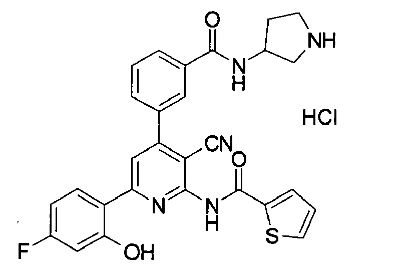

From the mixture of Reference Example 140 (250 mg) and tert-butyl 3-aminopyrrolidine-1-carbamate (229 mg, 1.23 mmol), the title compound (60 mg) was given as a pale yellow crystal in substantially the same manner as in Example 78.
¹H-NMR (300MHz, DMSO-d₆) δ: 2.00-2.15 (1H, m), 2.15-2.30 (1H, m), 3.20-3.50 (4H, m), 4.50-4.70 (1H, m), 6.80-6.90 (2H, m), 7.32 (1H, dd, J = 5.1, 3.6 Hz), 7.75 (1H, t, J = 7.8 Hz), 7.92 (1H, d, J = 8.1 Hz), 8.02 (1H, dd, J = 5.1, 0.9 Hz), 8.10-8.20 (2H, m), 8.20-8.30 (2H, m), 8.35 (1H, t, J = 8.1 Hz), 9.01 (1H, d, J = 6.6 Hz), 9.09 (1H, br s), 9.25 (1H, br s), 11.51 (1H, s), 12.40 (1H, s).
Melting point: 240-241 °C
Element analysis: Calculated (%) for C₂₈H₂₂N₅O₃SF·1.0HCl·1.0H₂O: C, 57.78; H, 4.33; N. 12.03. Found (%): C, 57.30; H, 4.30; N, 12.10.

### Example 80

### N-[4-(3-{[(3-amino-2,2-dimethylpropyl)amino]carbonyl}phenyl)-3-cyano-6-(4-fluoro-2-hydroxyphenyl)pyridin-2-yl]thiophene-2-carboxamide hydrochloride

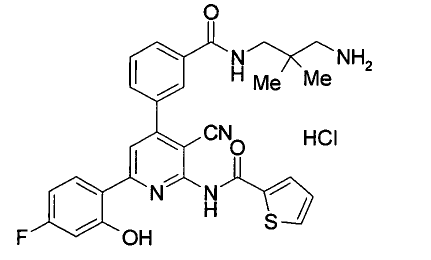

From the mixture of Reference Example 140 (250 mg) and tert-butyl (3-amino-2,2-dimethylpropyl)carbamate (249 mg, 1.23 mmol), the title compound (262 mg) was given as a pale yellow crystal in substantially the same manner as in Example 78.
¹H-NMR (300MHz, DMSO-d₆) δ: 1.00 (6H, s), 2.60-2.80 (2H, m), 3.20-3.40 (2H, m), 6.80-6.95 (2H, m), 7.32 (1H, dd, J = 5.1, 3.9 Hz), 7.75 (1H, t, J = 7.8 Hz), 7.90-8.05 (5H, m), 8.13 (1H, d, J = 7.8 Hz), 8.17 (1H, dd, J = 3.6, 0.9 Hz), 8.25-8.35 (3H, m), 8.97 (1H, s), 9.08 (1H, t, J = 6.3 Hz), 11.53 (1H, s), 12.35 (1H, s).
Melting point: 265-268°C
Element analysis: Calculated (%) for C₂₉H₂₆N₅O₃SF·1.0HCl·0.5H₂O: C, 59.13; H, 4.79; N. 11.89. Found (%): C, 59.15; H, 4.69; N, 11.76.

### Example 81

### N-[4-{3-[(2-aminoethyl)amino]carbonyl}phenyl)-3-cyano-6-(4-fluoro-2-hydroxyphenyl)pyridin-2-yl]thiophene-2-carboxamide hydrochloride

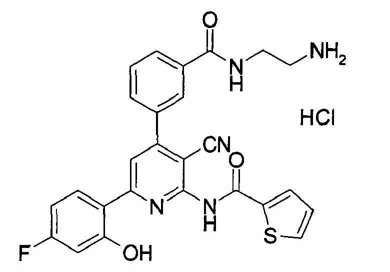

WSCD (236 mg, 1.23 mmol) was added to a solution of the mixture of Reference Example 140 (250 mg), tert-butyl (2-aminoethyl)carbamate (197 mg, 1.23 mmol) and HOBt (166 mg, 1.23 mmol) in DMF (25 mL) with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate) to give a mixture of tert-butyl {2-[(3-{3-cyano-6-[4-fluoro-2-(methoxymethoxy)phenyl]-2-[(2-thienylcarbonyl)amino] pyridin-4-yl}benzoyl)amino]ethyl}carbamate and tert-butyl {2-[(3-{3-cyano-6-(4-fluoro-2-hydroxyphenyl)-2-[(2-thienylcarbonyl)amino]pyridin-4-yl}benzoyl)amino] ethyl}carbamate as a white solid.

A 4N hydrogen chloride-ethyl acetate solution (4 mL) was added to a solution of the resultant mixture in ethyl acetate (6 mL), and stirred at room temperature for 2 hours. The reaction mixture was concentrated, and the residue was recrystallized from methanol-diethylether to give the title compound (184 mg) as a pale yellow crystal.
¹H-NMR (300MHz, DMSO-d₆) δ:2.90-3.10 (2H, m), 3.50-3.70 (2H, m), 6.80-6.90 (2H, m), 7.32 (1H, dd, J = 4.8, 3.9 Hz), 7.75 (1H, t, J = 7.8 Hz), 7.90-8.05 (5H, m), 8.10-8.20 (2H, m), 8.25-8.35 (3H, m), 11.50 (1H, s), 12.35 (1H, s).
Melting point: 228-229°C
Element analysis: Calculated (%) for C₂₆H₂₀N₅O₃SF·1.0HCl·2.0H₂O: C, 54.40; H, 4.39; N. 12.20. Found (%): C, 54.90; H, 4.35; N, 12.26.

### Example 82

### tert-butyl 4-[3-(β-alanylamino)phenyl]-2-(benzoylamino)-6-(2-hydroxyphenyl) nicotinate

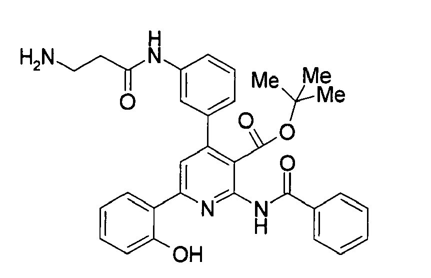

An ethanol (20 mL) mixture of the compound of Reference Example 131 (200 mg, 0.26 mmol) and palladium-carbon (100 mg) was stirred at room temperature under3 atom of hydrogen atmosphere for 72 hours. The catalyst was filtered out, and the filtrate was concentrated. Then, the residue was recrystallized from diisopropylether to give the title compound (123 mg, 86%) as a yellow crystal.
¹H-NMR (300MHz, DMSO-d₆) δ: 1.14 (9H, s), 2.45-2.50 (2H, m), 2.86-2.90 (2H, m), 6.89-6.97 (2H, m), 7.11 (1H, d, J = 7.5 Hz), 7.33-7.45 (2H, m), 7.58-7.72 (5H, m), 7.91 (1H, s), 8.00 (2H, d, J = 6.9 Hz), 8.10 (1H, d, J = 8.1 Hz), 10.20-10.3 (1H, br).
Melting point: 247-249°C
Element analysis: Calculated (%) for C₃₂H₃₂N₄O₅·1.3H₂O: C, 66.72; H, 6.05; N, 9.72. Found (%): C, 66.94; H, 6.08; N, 9.32.

### Example 83

### N-{6-(4-chloro-2-hydroxyphenyl)-3-cyano-4-[3-({[3-(1H-imidazol-1-yl)propyl] amino}carbonyl)phenyl]pyridin-2-yl}-2-furamide hydrochloride

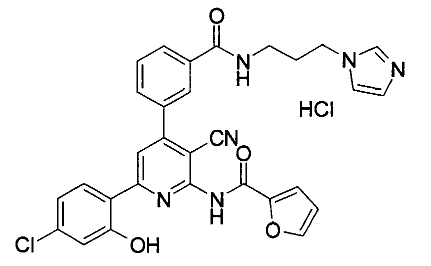

From the compound of Reference Example 123 (250 mg, 0.41 mmol), the title compound (228 mg, 92%) was given as a yellow crystal in substantially the same manner as in Example 70.
¹H-NMR (300MHz, DMSO-d₆) δ: 2.07-2.15 (2H, m), 3.32-3.37 (2H, m), 4.28 (2H, t, J = 6.8 Hz), 6.80 (1H, q, J = 1.8 Hz), 7.04 (1H, dd, J = 8.7, 2.1 Hz), 7.11 (1H, d, J = 2.1 Hz), 7.55 (1H, t, J = 2.1 Hz), 7.69-7.77 (2H, m), 7.84 (1H, t, J = 1.5 Hz), 7.90 (1H, d, J = 8.1 Hz), 8.07-8.09 (2H, m), 8.21-8.28 (3H, m), 8.88 (1H, t, J = 5.6 Hz), 9.17 (1H, s), 11.31 (1H, s), 12.25 (1H, s), 14.30-14.50 (1H, br).
Melting point: 182-184°C
Element analysis: Calculated (%) for C₃₀H₂₃N₆O₄Cl·1.0HCl·1.0H₂O: C, 57.98; H, 4.22; N, 13.52. Found (%): C, 58.18; H, 4.46; N, 13.34.

### Example 84

### N-[6-(4-chloro-2-hydroxyphenyl)-3-cyano-4-(3-{[(piperidin-2-ylmethyl)amino] carbonyl}phenyl)pyridin-2-yl]-2-furamide hydrochloride

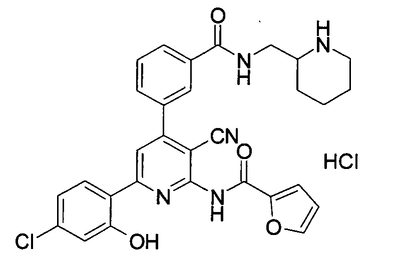

From the compound of Reference Example 124 (160 mg, 0.23 mmol), the title compound (123 mg, 90%) was given as a yellow crystal in substantially the same manner as in Example 70.
¹H-NMR (300MHz, DMSO-d₆) δ: 1.30-1.81 (6H, m), 2.95-3.10 (1H, m), 3.20-3.40.(2H, m), 3.45-3.60 (1H, m), 4.84-5.00 (1H, br), 6.80 (1H, q, J = 1.8 Hz), 7.04 (1H, dd, J = 8.7, 2.1 Hz), 7.12 (1H, d, J = 2.1 Hz), 7.55 (1H, d, J = 3.6 Hz), 7.69-7.73 (2H, m), 7.81-7.83 (2H, m), 7.90-8.05 (3H, br), 8.08 (1H, d, J = 1.2 Hz), 8.20-8.23 (2H, m), 11.20-11.30 (1H, br), 12.20-12.30 (1H, br).
Melting point: 222-226°C
Element analysis: Calculated (%) for C₃₀H₂₆N₅O₄Cl·1.0HCl·0.7H₂O: C, 59.55; H, 4.73; N, 11.57. Found (%): C, 59.58; H, 4.78; N, 11.33.

### Example 85

### N-[4-(3-{[(3-amino-2,2-dimethylpropyl)amino]carbonyl}phenyl)-6-(4-chloro-2-hydroxyphenyl)-3-cyanopyridin-2-yl]-2-furamide hydrochloride

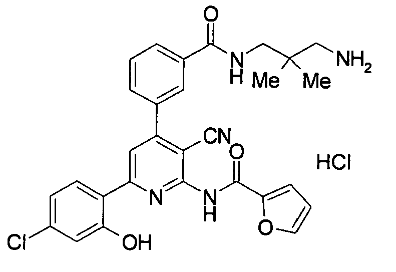

From the compound of Reference Example 125 (250 mg, 0.36 mmol), the title compound (206 mg, 99%) was given as a yellow crystal in substantially the same manner as in Example 70.
¹H-NMR (300MHz, DMSO-d₆) δ: 0.99 (6H, s), 2.60-2.70 (2H, br), 3.22-3.34 (2H, m), 6.80 (1H, q, J = 1.8 Hz), 7.04 (1H, dd, J = 8.7, 2.1 Hz), 7.10 (1H, d, J = 2.1 Hz), 7.54 (1H, d, J = 3.6 Hz), 7.73-7.90 (4H, m), 7.92 (1H, d, J = 8.1 Hz), 8.08-8.13 (2H, m), 8.22-8.28 (3H, m), 8.99 (1H, t, J = 6.3 Hz), 11.34 (1H, br s), 12.22 (1H, br s).
Melting point: 241-244°C
Element analysis: Calculated (%) for C₂₉H₂₆N₅O₄Cl·1.0HCl·1.0H₂O: C, 59.09; H, 4.79; N, 11.88. Found (%): C, 59.14; H, 4.92; N, 11.60.

### Example 86

### N-[4-(3-{[(2-aminoethyl)amino]carbonyl}phenyl)-6-(4-chloro-2-hydroxyphenyl)-3-cyanopyridin-2-yl]-2-furamide hydrochloride

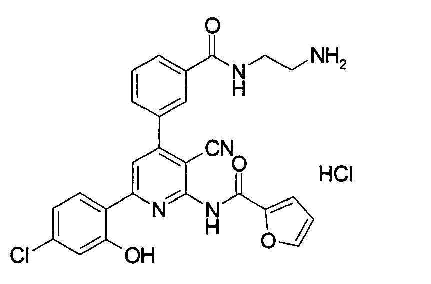

From the compound of Reference Example 122 (320 mg, 0.50 mmol), the title compound (239 mg, 89%) was given as a yellow crystal in substantially the same manner as in Example 70.
¹H-NMR (300MHz, DMSO-d₆) δ: 3.02 (2H, t, J = 6.0 Hz), 3.57 (2H, q, J = 5.8 Hz), 6.80 (1H, q, J = 1.8 Hz), 7.04 (1H, dd, J = 8.4, 2.1 Hz), 7.11 (1H, d, J = 2.1 Hz), 7.53 (1H, d, J = 3.3 Hz), 7.75 (1H, t, J = 7.8 Hz), 7.80-8.00 (4H, m), 8.08-8.13 (2H, m), 8.23-8.27 (3H, m), 8.92 (1H, t, J = 5.4 Hz), 11.30-11.50 (1H, br), 12.25-12.40 (1H, br).
Melting point: 252-255°C
Element analysis: Calculated (%) for C₂₆H₂₀N₅O₄Cl·1.0HCl·0.1H₂O: C, 57.81; H, 3.96; N, 12.96. Found (%): C, 57.67; H, 4.12; N, 12.80.

### Example 87

### N-(2-aminoethyl)-3-[2-(benzoylamino)-3-cyano-6-(2-hydroxyphenyl)pyridin-4-yl] benzamide ditrifluoroacetate

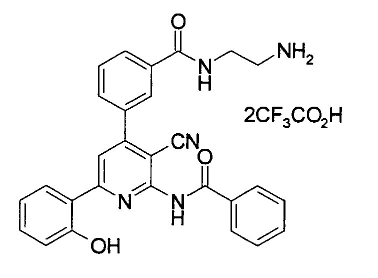

Triethylamine (14.1 µL) was added to a 0.12M solution of the compound of Reference Example 145 in DMF (830 µL, 100 µmol). A 0.24 M solution of tert-butyl N-(2-aminoethyl)carbamate in dichloromethane (600 µL, 1.44 µmol), a 0.20 M dichloromethane solution (720 µL, 1.44 µmol) of HOBt and WSCD, and a 2.0 M solution of N,N-dimethylaminopyridin in dichloromethane (7.2 µL, 14.4 µmol) were added thereto, and stirred at 50°C for 24 hours. The temperature of the reaction solution was returned to room temperature, and dichloromethane (3 mL) and a 5% aqueous solution of sodium hydrogencarbonate (2 mL) were added for extraction. The organic layer was separated by a phase-separation tube (Whatman). The solvent was removed under reduced pressure. The residue was dissolved in DMSO (1 mL) and purified by separation HPLC to give tert-butyl {2-[(3-{2-(benzoylamino)-3-cyano-6-[2-(methoxymethoxy)phenyl]pyridin-4-yl} benzoyl)amino]ethyl}carbamate. The solvent was concentrated, trifluoroacetic acid (3 mL) was added, and the resultant substance was stirred at room temperature for 20 hours. TFA was removed, and the residue was dissolved in DMSO (1 mL) and purified again by separation HPLC to give the title compound (13.8 mg, purity: 100%).
¹H-NMR (300MHz, DMSO-d₆) δ: 3.05 (2H, t, J = 6.4 Hz), 3.58 (2H, t, J = 6.4 Hz), 6.95-7.02 (2H, m), 7.42 (1H, t, J = 4.8 Hz), 7.61-7.99 (8H, m), 8.01-8.29 (6H, m), 8.81 (1H, t, J = 4.8 Hz).

### Example 88 - Example 234

Using 12 types of amine shown below on the compounds given in Reference Example 7 and Reference Examples 145 through 158, the compounds shown in Tables 2 through 26 were given in substantially the same manner as in Example 87.

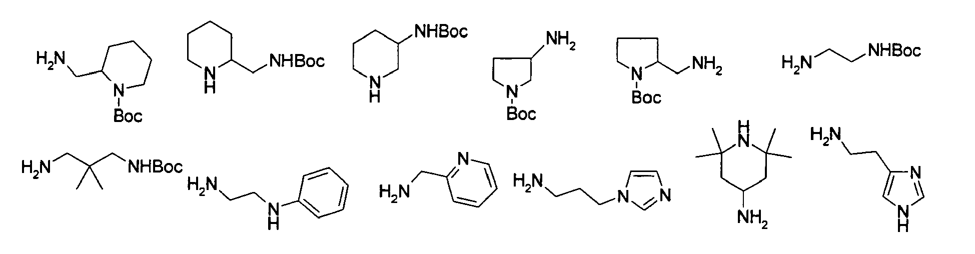

Tables 2 through 26 show the formula, yield amount and purity of the compounds of Examples 88 through 234.

**Table 2**

| Example | | | Yield amount (mg) | Purity (%) |
|---|---|---|---|---|
| Example 88 | 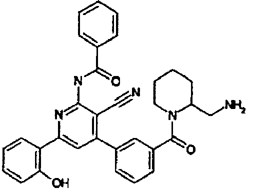 | 2CF₃CO₂H | 21 | 100 |
| Example 89 | 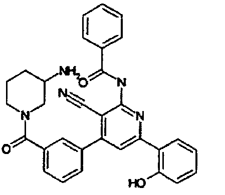 | 2CF₃CO₂H | 23 | 100 |
| Example 90 | 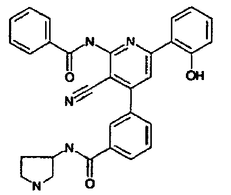 | 2CF₃CO₂H | 10 | 100 |
| Example 91 | 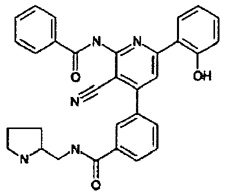 | 2CF₃CO₂H | 22 | 100 |

**Table 3**

| Example No. | | | Yield amount (mg) | Purity (%) |
|---|---|---|---|---|
| Example 92 | 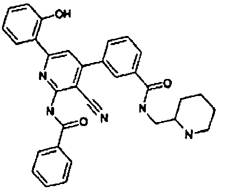 | 2CF₃CO₂H | 14 | 100 |
| Example 93 | 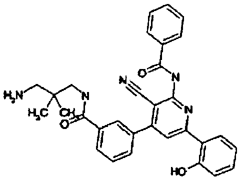 | 2CF₃CO₂H | 25 | 100 |
| Example 94 | 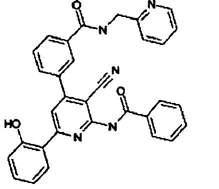 | 2CF₃CO₂H | 20 | 100 |
| Example 95 | 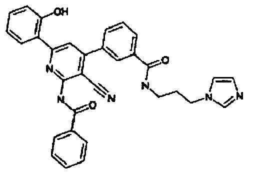 | 2CF₃CO₂H | 25 | 100 |
| Example 96 | 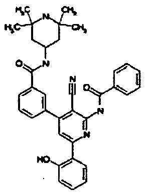 | 2CF₃CO₂H | 24 | 100 |
| Example 97 | 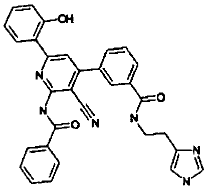 | 2CF₃CO₂H | 12 | 100 |

**Table 4**

| Example No. | | | Yield amount (mg) | Punty (%) |
|---|---|---|---|---|
| Example 98 | 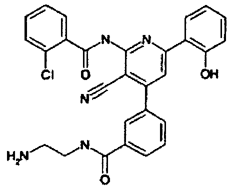 | 2CF₃CO₂H | 35 | 100 |
| Example 99 | 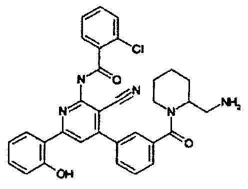 | 2CF₃CO₂H | 18 | 100 |
| Example 100 | 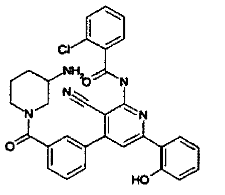 | 2CF₃CO₂H | 40 | 100 |
| Example 101 | 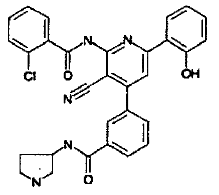 | 2CF₃CO₂H | 36 | 100 |
| Example 102 | 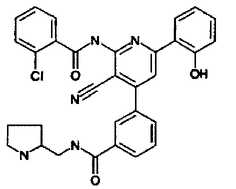 | 2CF₃CO₂H | 40 | 100 |
| Example 103 | 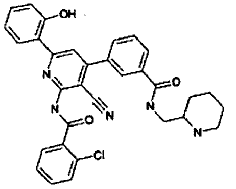 | 2CF₃CO₂H | 34 | 99 |

**Table 5**

| Example No. | | | Yield amount (mg) | Purity (%) |
|---|---|---|---|---|
| Example 104 | 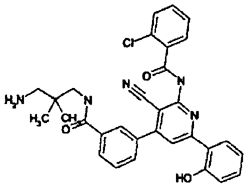 | 2CF₃CO₂H | 41 | 88 |
| Example 105 | 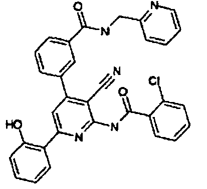 | 2CF₃CO₂H | 33 | 100 |
| Example 106 | 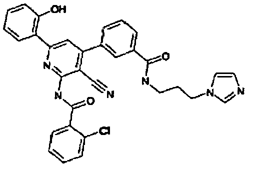 | 2CF₃CO₂H | 41 | 100 |
| Example 107 | 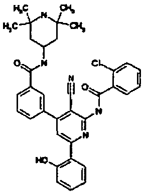 | 2CF₃CO₂H | 40 | 100 |
| Example 108 | 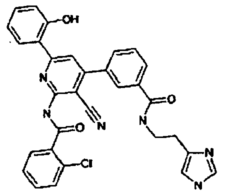 | 2CF₃CO₂H | 24 | 100 |
| Example 1 09 | 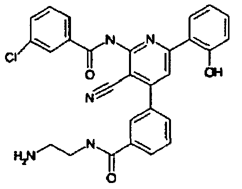 | 2CF₃CO₂H | 17 | 100 |

**Table 6**

| Example No. | | | Yield amount (mg) | Purity (%) |
|---|---|---|---|---|
| Example 110 | 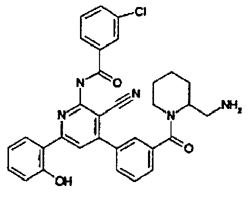 | 2CF₃CO₂H | 25 | 100 |
| Example 111 | 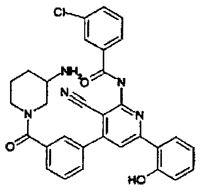 | 2CF₃CO₂H | 23 | 100 |
| Example 112 | 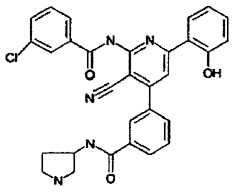 | 2CF₃CO₂H | 28 | 100 |
| Example 113 | 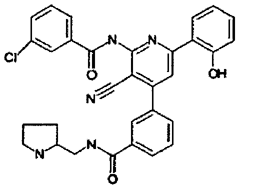 | 2CF₃CO₂H | 26 | 100 |
| Example 114 | 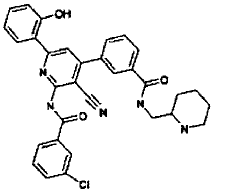 | 2CF₃CO₂H | 21 | 100 |
| Example 115 | 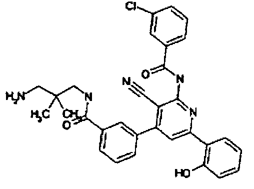 | 2CF₃CO₂H | 34 | 100 |

**Table 7**

| Example No. | | | Yield amount (mg) | Purity (%) |
|---|---|---|---|---|
| Example 116 | 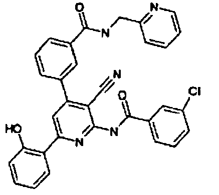 | 2CF₃CO₂H | 27 | 100 |
| Example 117 | 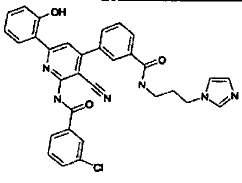 | 2CF₃CO₂H | 31 | 100 |
| Example 118 | 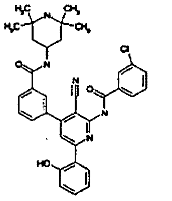 | 2CF₃CO₂H | 26 | 100 |
| Example 119 | 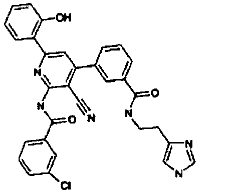 | 2CF₃CO₂H | 13 | 99 |
| Example 120 | 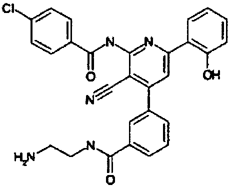 | 2CF₃CO₂H | 10 | 100 |
| Example 121 | 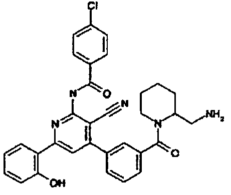 | 2CF₃CO₂H | 11 | 100 |

**Table 8**

| Example No. | | | | Punty(%) |
|---|---|---|---|---|
| Example 122 Example | 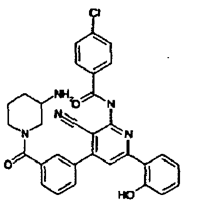 | 2CF₃CO₂H | 10 | 100 |
| Example 123 | 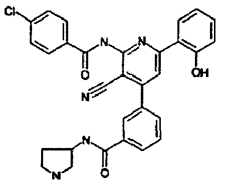 | 2CF₃CO₂H | 12 | 100 |
| Example 124 | 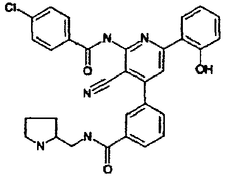 | 2CF₃CO₂H | 11 | 100 |
| Example 125 | 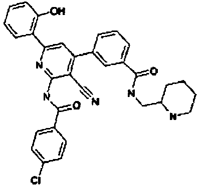 | 2CF₃CO₂H | 11 | 100 |
| Example 126 | 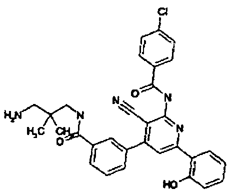 | 2CF₃CO₂H | 11 | 87 |
| Example 127 | 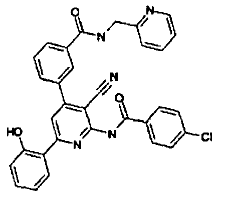 | 2CF₃CO₂H | 7 | 100 |

**Table 9**

| Example No. | | | Yield amount (mg) | Purity (%) |
|---|---|---|---|---|
| Example 128 | 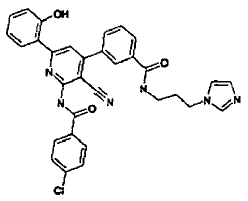 | 2CF₃CO₂H | 17 | 98 |
| Example 129 | 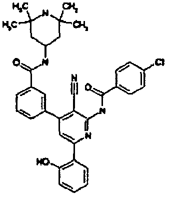 | 2CF₃CO₂H | 12 | 87 |
| Example 130 | 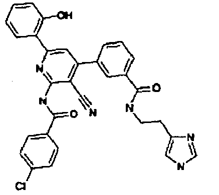 | 2CF₃CO₂H | 7 | 80 |
| Example 131 | 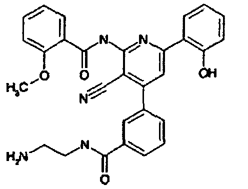 | 2CF₃CO₂H | 11 | 100 |
| Example 132 | 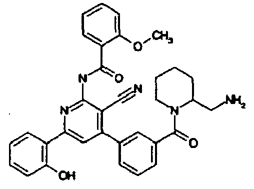 | 2CF₃CO₂H | 4 | 100 |
| Example 133 | 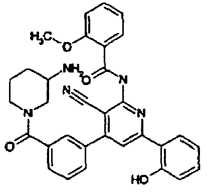 | 2CF₃CO₂H | 19 | 100 |

**Table 10**

| Example No. | | | Yield amount (mg) | Purity (%) |
|---|---|---|---|---|
| Example 134 | 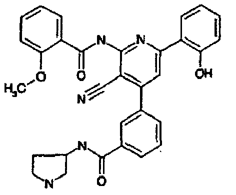 | 2CF₃CO₂H | 13 | 100 |
| Example 135 | 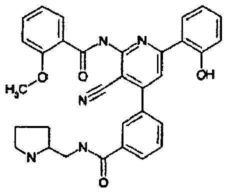 | 2CF₃CO₂H | 15 | 98 |
| Example 136 | 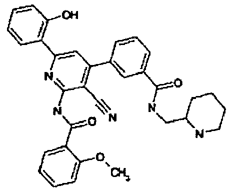 | 2CF₃CO₂H | 6 | 98 |
| Example 137 | 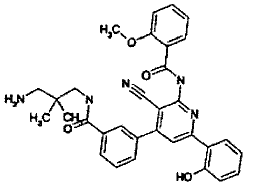 | 2CF₃CO₂H | 17 | 100 |
| Example 138 | 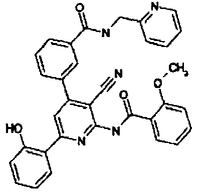 | 2CF₃CO₂H | 15 | 95 |
| Example 139 | 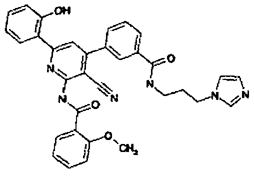 | 2CF₃CO₂H | 17 | 100 |

**Table 11**

| Example No. | | | Yield amount (mg) | Purity (%) |
|---|---|---|---|---|
| Example 140 | 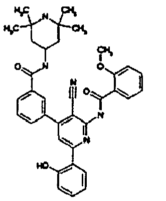 | 2CF₃CO₂H | 18 | 93 |
| Example 141 | 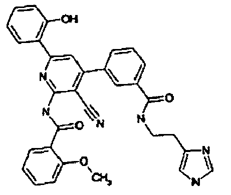 | 2CF₃CO₂H | 6 | 100 |
| Example 142 | 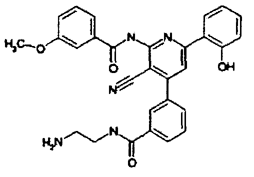 | 2CF₃CO₂H | 27 | 100 |
| Example 143 | 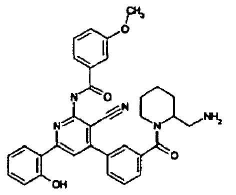 | 2CF₃CO₂H | 29 | 100 |
| Example 144 | 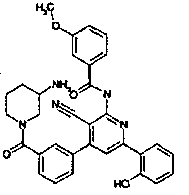 | 2CF₃CO₂H | 34 | 100 |
| Example 14 5 | 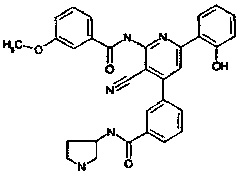 | 2CF₃CO₂H | 36 | 100 |

**Table 12**

| Example No. | | | Yield amount (mg) | Purity (%) |
|---|---|---|---|---|
| Example 146 | 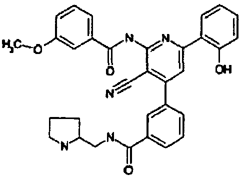 | 2CF₃CO₂H | 32 | 100 |
| Example 147 | 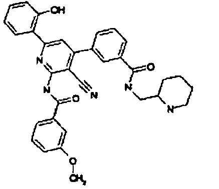 | 2CF₃CO₂H | 33 | 98 |
| Example 148 | 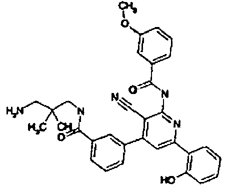 | 2CF₃CO₂H | 38 | 86 |
| Example 149 | 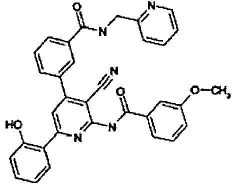 | 2CF₃CO₂H | 28 | 100 |
| Example 150 | 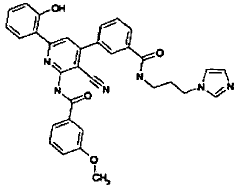 | 2CF₃CO₂H | 23 | 100 |
| Example 151 | 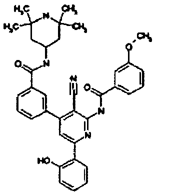 | 2CF₃CO₂H | 26 | 100 |

**Table 13**

| Example No. | | | Yield amount (mg) | Purity (%) |
|---|---|---|---|---|
| Example 152 | 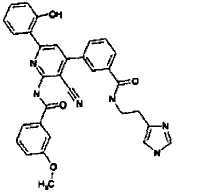 | 2CF₃CO₂H | 19 | 100 |
| Example 153 | 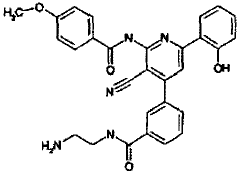 | 2CF₃CO₂H | 6 | 100 |
| Example 154 | 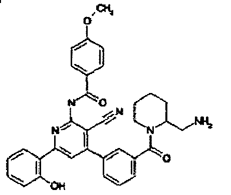 | 2CF₃CO₂H | 20 | 100 |
| Example 155 | 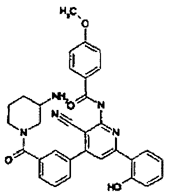 | 2CF₃CO₂H | 29 | 100 |
| Example 156 | 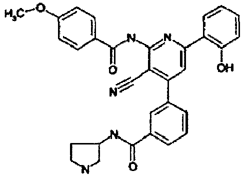 | 2CF₃CO₂H | 23 | 100 |
| Example 157 | 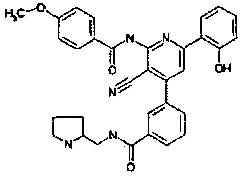 | 2CF₃CO₂H | 28 | 98 |

**Table 14**

| Example No. | | | Yield amount (mg) | Purity (%) |
|---|---|---|---|---|
| Example 158 | 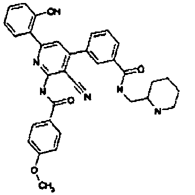 | 2CF₃CO₂H | 21 | 98 |
| Example 159 | 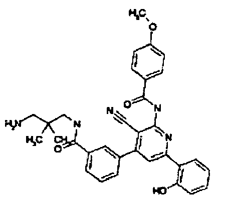 | 2CF₃CO₂H | 31 | 93 |
| Example 160 | 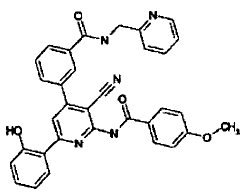 | 2CF₃CO₂H | 20 | 100 |
| Example 161 | 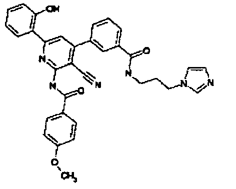 | 2CF₃CO₂H | 24 | 100 |
| Example 162 | 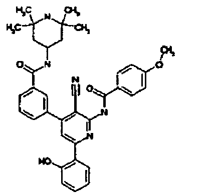 | 2CF₃CO₂H | 25 | 100 |
| Example 163 | 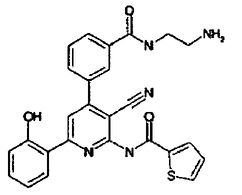 | 2CF₃CO₂H | 25 | 100 |

**Table 15**

| Example No. | | | Yield amount (mg) | Purity (%) |
|---|---|---|---|---|
| Example 164 | 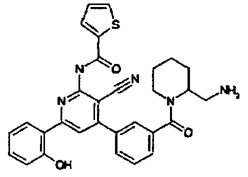 | 2CF₃CO₂H | 24 | 100 Example |
| Example 165 | 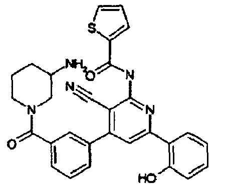 | 2CF₃CO₂H | 31 | 100 |
| Example 166 | 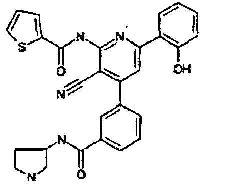 | 2CF₃CO₂H | 33 | 100 |
| Example 1 67 | 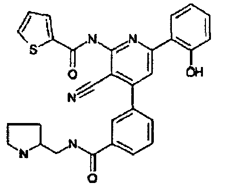 | 2CF₃CO₂H | 28 | 100 |
| Example 168 | 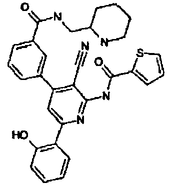 | 2OF₃CO₂H | 22 | 100 |
| Example 169 | 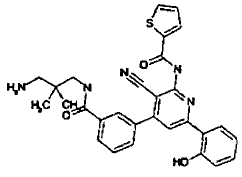 | 2CF₃CO₂H | 32 | 90 |

**Table 16**

| Example No. | | | Yield amount (mg) | Purity (%) |
|---|---|---|---|---|
| Example 170 | 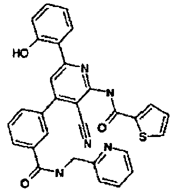 | 2CF₃CO₂H | 26 | 100 |
| Example 171 | 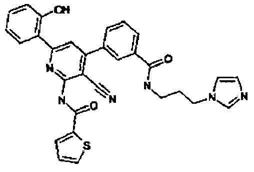 | 2CF₃CO₂H | 26 | 100 |
| Example 172 | 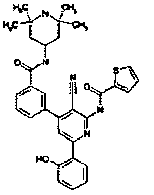 | 2CF₃CO₂H | 30 | 100 |
| Example 173 | 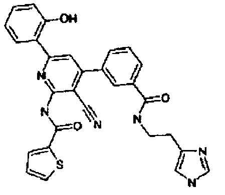 | 2CF₃CO₂H | 16 | 100 |
| Example 174 | 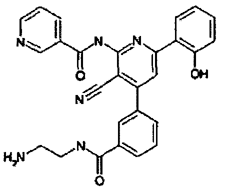 | 3CF₃CO₂H | 14 | 100 |
| Example 175 | 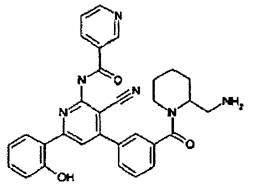 | 3CF₃CO₂H | 15 | 100 |

**Table 17**

| Example No. | | | Yield amount (mg) | Purity(%) |
|---|---|---|---|---|
| Example 176 | 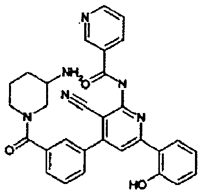 | 3CF₃CO₂H | 15 | 100 |
| Example 177 | 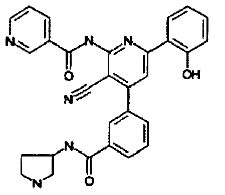 | 3CF₃CO₂H | 13 | 100 |
| Example 178 | 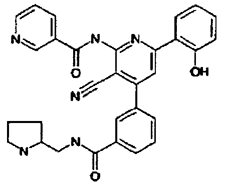 | 3CF₃CO₂H | 13 | 100 |
| Example 179 | 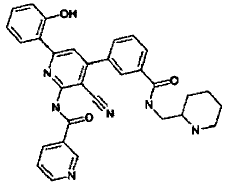 | 3CF₃CO₂H | 16 | 97 |
| Example 180 | 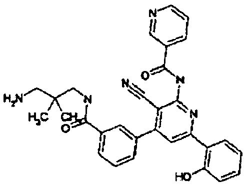 | 3CF₃CO₂H | 14 | 100 |
| Example 181 | 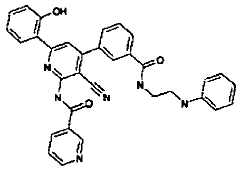 | 2CF₃CO₂H | 8 | 98 |

**Table 18**

| Example No. | | | Yield amount (mg) | Purity (%) |
|---|---|---|---|---|
| Example 182 | 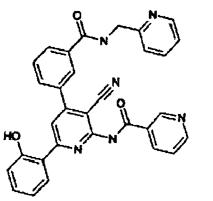 | 3CF₃CO₂H | 13 | 97 |
| Example 183 | 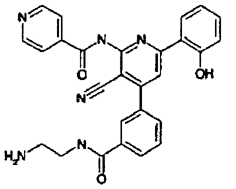 | 3CF₃CO₂H | 6 | 100 |
| Example 184 | 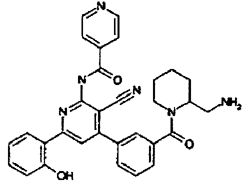 | 3CF₃CO₂H | 20 | 100 |
| Example 185 | 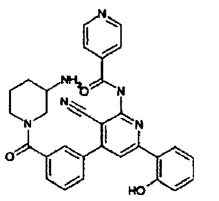 | 3CF₃CO₂H | 21 | 100 |
| Example 186 | 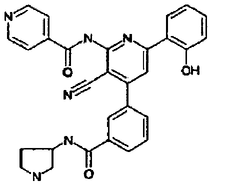 | 3CF₃CO₂H | 21 | 100 |
| Example 187 | 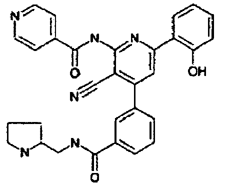 | 3CF₃CO₂H | 14 | 99 |

**Table 19**

| Example No. | | | Yield amount (mg) | Purity (%) |
|---|---|---|---|---|
| Example 188 | 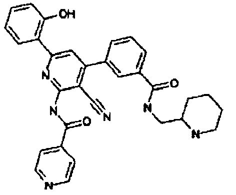 | 3CF₃CO₂H | 19 | 98 |
| Example 189 | 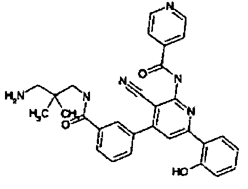 | 3CF₃CO₂H | 22 | 100 |
| Example 190 | 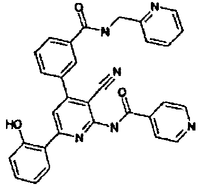 | 3CF₃CO₂H | 19 | 98 |
| Example 191 | 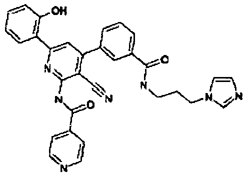 | 3CF₃CO₂H | 19 | 100 |
| Example 192 | 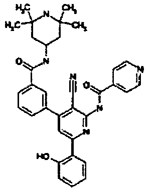 | 3CF₃CO₂H | 19 | 100 |
| Example 193 | 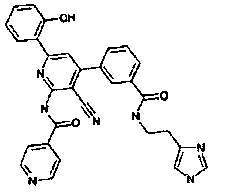 | 3CF₃CO₂H | 12 | 100 |

**Table 20**

| Example No. | | | Yield amount (mg) | Purity (%) |
|---|---|---|---|---|
| Example 194 | 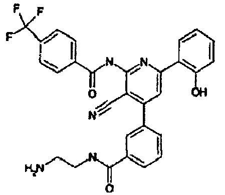 | 2CF₃CO₂H | 12 | 100 |
| Example 195 | 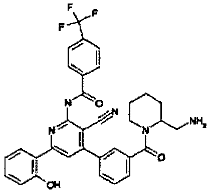 | 2CF₃CO₂H | 27 | 100 |
| Example 196 | 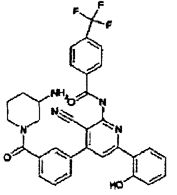 | 2CF₃CO₂H | 31 | 100 |
| Example 197 | 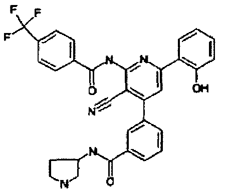 | 2CF₃CO₂H | 28 | 100 |
| Example 198 | 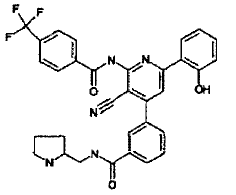 | 2CF₃CO₂H | 23 | 96 |
| Example 199 | 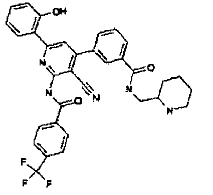 | 2CF₃CO₂H | 19 | 100 |

**Table 21**

| Example No. | | | Yield amount (mg) | Purity (%) |
|---|---|---|---|---|
| Example 200 | 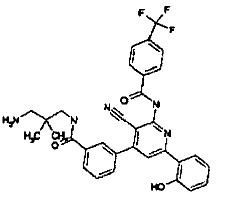 | 2CF₃CO₂H | 23 | 96 |
| Example 201 | 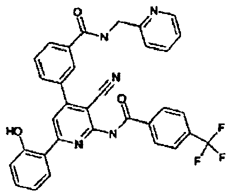 | 2CF₃CO₂H | 27 | 99 |
| Example 202 | 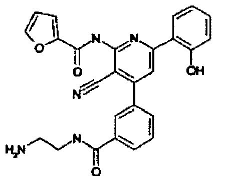 | 2CF₃CO₂H | 25 | 100 |
| Example 203 | 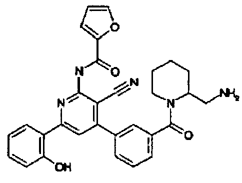 | 2CF₃CO₂H | 24 | 100 |
| Example 204 | 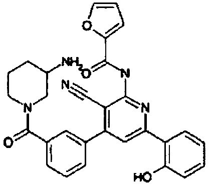 | 2CF₃CO₂H | 25 | 100 |
| Example 205 | 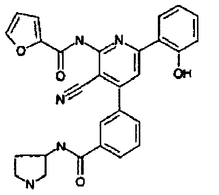 | 2CF₃CO₂H | 31 | 100 |

**Table 22**

| Example No. | | | Yield amount (mg) | Purity (%) |
|---|---|---|---|---|
| Example 206 | 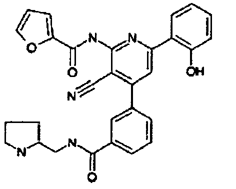 | 2CF₃CO₂H | 26 | 98 |
| Example 207 | 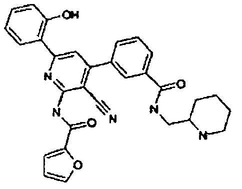 | 2CF₃CO₂H | 24 | 100 |
| Example 208 | 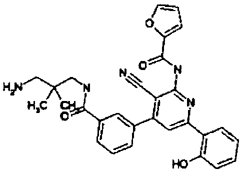 | 2CF₃CO₂H | 20 | 100 |
| Example 209 | 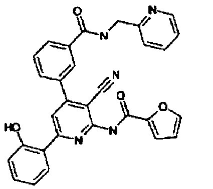 | 2CF₃CO₂H | 18 | 100 |
| Example 210 | 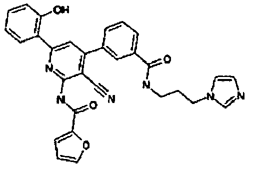 | 2CF₃CO₂H | 18 | 100 |
| Example 21 | 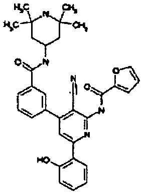 | 2CF₃CO₂H | 11 | 100 |

**Table 23**

| Example No. | | | Yield amount (mg) | Purity (%) |
|---|---|---|---|---|
| Example 212 | 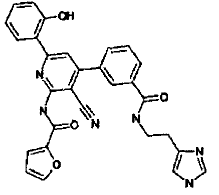 | 2CF₃CO₂H | 13 | 100 |
| Example 213 | 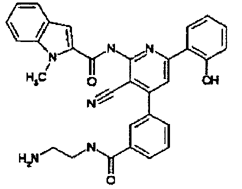 | 2CF₃CO₂H | 22 | 99 |
| Example 214 | 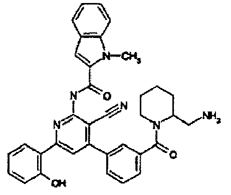 | 2CF₃CO₂H | 27 | 95 |
| Example 215 | 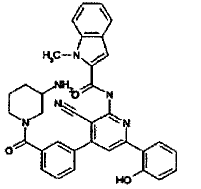 | 2CF₃CO₂H | 26 | 100 |
| Example 216 | 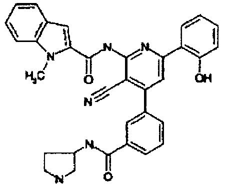 | 2CF₃CO₂H | 22 | 100 |
| Example 217 | 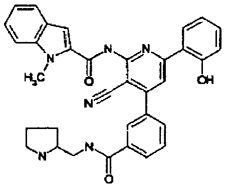 | 2CF₃CO₂H | 30 | 98 |

**Table 24**

| Example No. | | | Yield amount (mg) | Purity (%) |
|---|---|---|---|---|
| Example 218 | 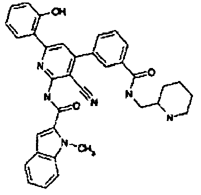 | 2CF₃CO₂H | 24 | 96 |
| Example 219 | 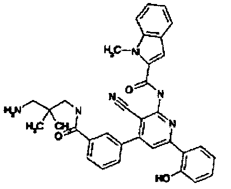 | 2CF₃CO₂H | 29 | 100 |
| Example 220 | 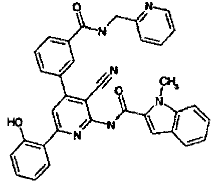 | 2CF₃CO₂H | 34 | 100 |
| Example 221 | 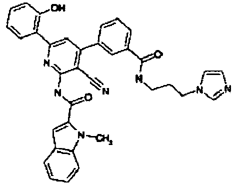 | 2CF₃CO₂H | 26 | 100 |
| Example 222 | 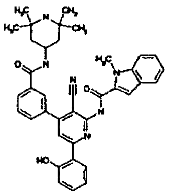 | 2CF₃CO₂H | 15 | 100 |
| Example 223 | 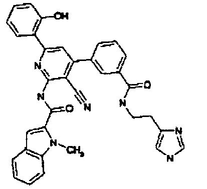 | 2CF₃CO₂H | 14 | 100 |

**Table 25**

| Example No. | | | Yield amount (mg) | Purity (%) |
|---|---|---|---|---|
| Example 224 | 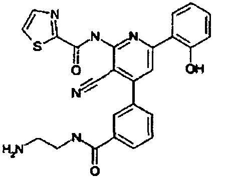 | 2CF₃CO₂H | 14 | 100 |
| Example 225 | 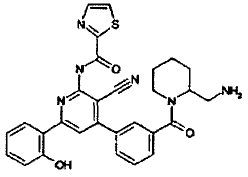 | 2CF₃CO₂H | 19 | 100 |
| Example 226 | 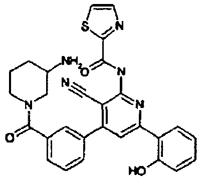 | 2CF₃CO₂H | 17 | 100 |
| Example 227 | 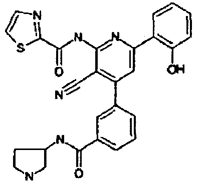 | 2CF₃CO₂H | 18 | 100 |
| Example 228 | 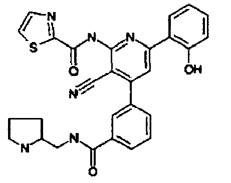 | 2CF₃CO₂H | 23 | 100 |
| Example 229 | 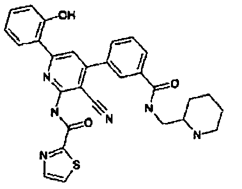 | 2CF₃CO₂H | 19 | 100 |

**Table 26**

| Example No. | | | Yield amount (mg) | Purity (%) |
|---|---|---|---|---|
| Example 230 | 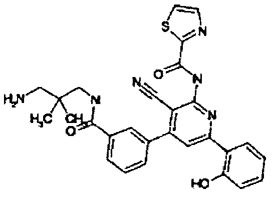 | 2CF₃CO₂H | 11 | 100 |
| Example 231 | 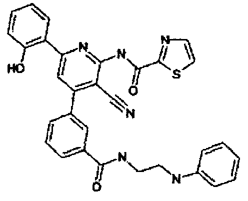 | OF₃CO₂H | 8 | 100 |
| Example 232 | 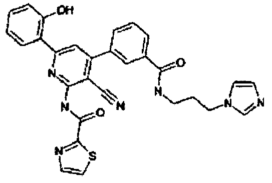 | 2CF₃CO₂H | 20 | 100 |
| Example 233 | 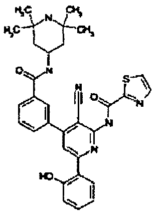 | 2CF₃CO₂H | 12 | 100 |
| Example 234 | 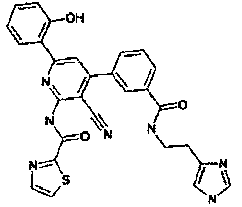 | 2CF₃CO₂H | 17 | 100 |

### Example 235

### N-[4-[3-(β-alanylamino)phenyl]-6-(4-chloro-2-hydroxyphenyl)-3-cyanopyridin-2-yl]-3-methyl-2-furamide hydrochloride

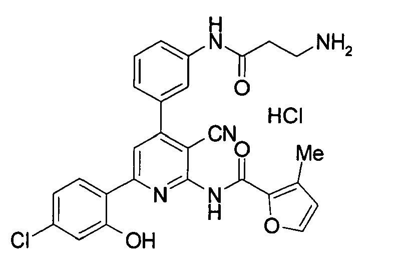

From the compound of Reference Example 159 (70 mg, 0.11 mmol), the title compound (56 mg, 92%) was given as a yellow powder in substantially the same manner as in Example 8.
¹H-NMR (300MHz, DMSO-d₆) δ: 2.39 (3H, s), 2.78 (2H, t, J = 6.9 Hz), 3.11 (2H, t, J = 6.6 Hz), 6.69 (1H, s), 7.02 (1H, dd, J = 8.7, 1.8 Hz), 7.10 (1H, d, J = 1.8 Hz), 7.41 (1H, d, J = 8.1 Hz), 7.57 (1H, t, J = 7.8 Hz), 7.80-7.98 (7H, m), 8.14 (1H, s), 10.54 (1H, s), 10.80-11.00 (1H, br), 12.36 (1H, br s).
Melting point: 218-225°C
Element analysis: Calculated (%) for C₂₇H₂₂N₅O₄Cl·1.0HCl·2.0H₂O: C, 55.11; H, 4.62; N, 11.90. Found (%): C, 55.28; H, 4.59; N, 11.91.

### Example 236

### N-[4-[3-(β-alanylamino)phenyl]-6-(4-chloro-2-hydroxyphenyl)-3-cyanopyridin-2-yl]-2-methoxybenzamide hydrochloride

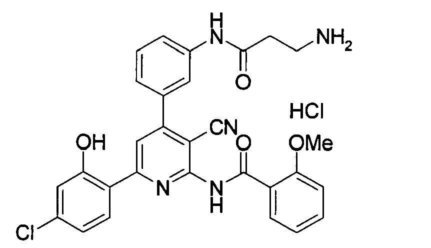

From the compound of Reference Example 160 (75 mg, 0.11 mmol), the title compound (56 mg, 88%) was given as a yellow powder in substantially the same manner as in Example 8.
¹H-NMR (300MHz, DMSO-d₆) δ: 2.79 (2H, t, J = 6.9 Hz), 3.10 (2H, q, J = 6.1 Hz), 4.09 (3H, s), 6.99 (1H, dd, J = 8.7, 2.1 Hz), 7.08 (1H, d, J = 2.1 Hz), 7.21 (1H, t, J = 7.5 Hz), 7.34 (1H, d, J = 8.4 Hz), 7.45 (1H, d, J = 8.1 Hz), 7.58 (1H, t, J = 7.8 Hz), 7.70 (1H, td, J = 7.8, 1.5 Hz), 7.82-7.96 (4H, m), 8.00 (1H, s), 8.06-8.12 (2H, m), 8.22 (1H, d, J = 9.0 Hz), 10.53 (1H, s), 11.34 (1H, s), 13.28 (1H, s).
Melting point: 233-238°C
Element analysis: Calculated (%) for C₂₉H₂₄N₅O₄Cl·1.0HCl·2.0H₂O: C, 56.68; H, 4.76; N, 11.40. Found (%): C, 56.55; H, 4.74; N, 11.33.

### Example 237

### N-[4-[3-(alanylamino)phenyl]-6-(4-chloro-2-hydroxyphenyl)-3-cyanopyridin-2-yl]-2-furamide hydrochloride

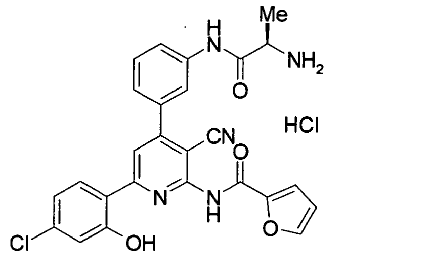

From the compound of Reference Example 161 (200 mg, 0.31 mmol), the title compound (181 mg, 99%) was given as a yellow powder in substantially the same manner as in Example 8.
¹H-NMR (300MHz, DMSO-d₆) δ: 1.50 (3H, d, J = 6.9 Hz), 4.00-4.14 (1H, m), 6.80 (1H, q, J = 1.8 Hz), 7.04 (1H, dd, J = 8.7, 2.1 Hz), 7.12 (1H, d, J = 2.1 Hz), 7.47 (1H, d, J = 7.8 Hz), 7.54 (1H, d, J = 3.6 Hz), 7.63 (1H, t, J = 7.8 Hz), 7.85 (1H, d, J = 9.3 Hz), 8.01 (1H, d, J = 1.5 Hz), 8.07 (1H, t, J = 0.9 Hz), 8.17-8.20 (2H, m), 8.29 (3H, br s), 10.90 (1H, s), 11.32 (1H, s), 12.16 (1H, s).
Melting point: 210-216°C
Element analysis: Calculated (%) for C₂₆H₂₀N₅O₄Cl·1.0HCl·1.0H₂O: C, 56.13; H, 4.17; N, 12.59. Found (%): C, 56.41; H, 4.44; N, 12.50.

### Example 238

### N-[4-{3-[(3-aminobutanoyl)amino]phenyl}-6-(4-chloro-2-hydroxyphenyl)-3-cyanopyridin-2-yl]-2-furamide hydrochloride

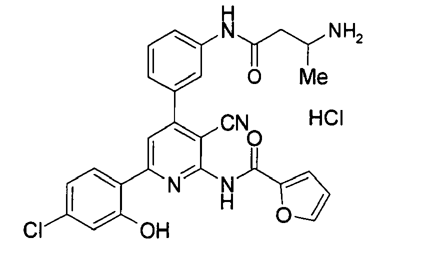

From the compound of Reference Example 162 (120 mg, 0.18 mmol), the title compound (96 mg, 97%) was given as a pale yellow crystal in substantially the same manner as in Example 8.
¹H-NMR (300MHz, DMSO-d₆) δ: 1.26 (3H, d, J = 6.6 Hz), 2.67-2.82 (2H, m), 3.57-3.72 (1H, br), 6.80 (1H, q, J = 1.5 Hz), 7.04 (1H, dd, J = 8.7, 2.4 Hz), 7.11 (1H, d, J = 2.4 Hz), 7.41 (1H, d, J = 7.8 Hz), 7.54-7.60 (2H, m), 7.83 (1H, d, J = 8.1 Hz), 7.90-8.00 (4H, m), 8.07 (1H, d, J = 0.9 Hz), 8.16-8.19 (2H, m), 10.58 (1H, s), 11.30 (1H, s), 12.14 (1H, s).
Melting point: 206-214°C
Element analysis: Calculated (%) for C₂₇H₂₂N₅O₄Cl·1.0HCl·1.5H₂O: C, 55.97; H, 4.52; N, 12.09. Found (%): C, 56.26; H, 4.60; N, 12.06.

### Example 239

### N-(6-(4-chloro-2-hydroxyphenyl)-3-cyano-4- {3-[(1H-imidazole-1-ylacetyl)amino] phenyl}pyridin-2-yl)-2-furamide hydrochloride

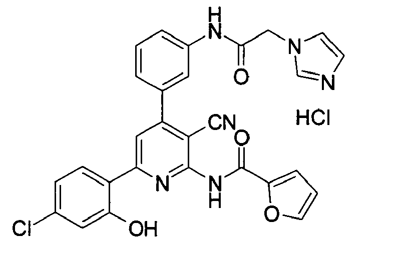

From the compound of Reference Example 163 (60 mg, 0.10 mmol), the title compound (59 mg, 100%) was given as a yellow crystal in substantially the same manner as in Example 70.
¹H-NMR (300MHz, DMSO-d₆) δ: 5.29 (2H, s), 6.80 (1H, q, J = 1.8 Hz), 7.03 (1H, dd, J = 8.7, 2.1 Hz), 7.10 (1H, d, J = 2.1 Hz), 7.45 (1H, d, J = 7.8 Hz), 7.54 (1H, d, J = 3.3 Hz), 7.61 (1H, t, J = 7.8 Hz), 7.69 (1H, t, J = 1.5 Hz), 7.76 (1H, t, J = 1.5 Hz), 7.81 (1H, d, J = 7.8 Hz), 8.00 (1H, s), 8.07 (1H, s), 8.16-8.19 (2H, m), 9.10 (1H, s), 10.96 (1H, s), 11.28 (1H, s), 12.12 (1H, s), 14.30-14.60 (1H, br).
Melting point: 188-194°C
Element analysis: Calculated (%) for C₂₈H₁₉N₆O₄Cl·1.0HCl·1.5H₂O: C, 55.82; H, 3.85; N, 13.95; Cl, 11.77. Found (%): C, 55.56; H, 4.05; N, 13.61; Cl, 11.44.

### Example 240

### N-[4-[3-(L-alanylamino)phenyl]-6-(4-chloro-2-hydroxyphenyl)-3-cyanopyridin-2-yl]-2-furamide hydrochloride

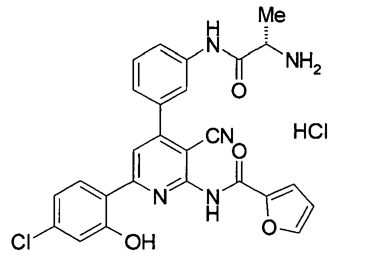

From the compound of Reference Example 164 (220 mg, 0.34 mmol), the title compound (169 mg, 92%) was given as a pale yellow crystal in substantially the same manner as in Example 8.
¹H-NMR (300MHz, DMSO-d₆) δ: 1.50 (3H, d, J = 6.9 Hz), 4.00-4.14 (1H, br), 6.80 (1H, q, J = 1.5 Hz), 7.04 (1H, dd, J = 8.4, 2.1 Hz), 7.10 (1H, d, J = 2.1 Hz), 7.47 (1H, d, J = 8.1 Hz), 7.54 (1H, d, J = 3.9 Hz), 7.63 (1H, t, J = 8.1 Hz), 7.85 (1H, d, J = 8.1 Hz), 8.01 (1H, s), 8.07 (1H, d, J = 1.2 Hz), 8.17-8.20 (2H, m), 8.20-8.35 (3H, br), 10.86 (1H, s), 11.32 (1H, s), 12.15 (1H, s).
Melting point: 207-210°C
Element analysis: Calculated (%) for C₂₆H₂₀N₅O₄Cl·1.0HCl·1.5H₂O: C, 55.23; H, 4.28; N, 12.39. Found (%): C, 55.38; H, 4.51; N, 12.35.

### Example 241

### N-{3-[6-(4-chloro-2-hydroxyphenyl)-3-cyano-2-(2-furoylamino)pyridin-4-yl]phenyl} pyrrolidine-3-carboxamide hydrochloride

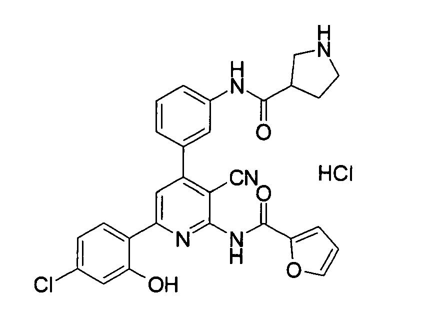

From the compound of Reference Example 165 (300 mg, 0.45 mmol), the title compound (227 mg, 89%) was given as a pale yellow crystal in substantially the same manner as in Example 8.
¹H-NMR (300MHz, DMSO-d₆) δ: 2.05-2.12 (1H, m), 2.25-2.32 (1H, m), 3.24 (2H, t, J = 7.2 Hz), 3.30-3.44 (3H, m), 6.79 (1H, q, J = 1.5 Hz), 7.04 (1H, dd, J = 8.4, 2.1 Hz), 7.10 (1H, d, J = 2.1 Hz), 7.42 (1H, d, J = 8.1 Hz), 7.53-7.61 (2H, m), 7.83 (1H, d, J = 9.0 Hz), 8.02 (1H, s), 8.07 (1H, t, J = 0.9 Hz), 8.16 (1H, s), 8.18 (1H, d, J = 8.7 Hz), 8.95-9.15 (2H, br), 10.62 (1H, s), 11.20-11.40 (1H, br), 12.10-12.25 (1H, br).
Melting point: 226-235°C
Element analysis: Calculated (%) for C₂₈H₂₂N₅O₄Cl·1.0HCl·1.0H₂O: C, 57.74; H, 4.33; N, 12.02. Found (%): C, 57.57; H, 4.55; N, 11.87.

### Example 242

### N-[6-(4-chloro-2-hydroxyphenyl)-3-cyano-4-(3-{ [4-(1H-imidazole-1-yl)butanoyl] amino}phenyl)pyridin-2-yl]-2-furamide hydrochloride

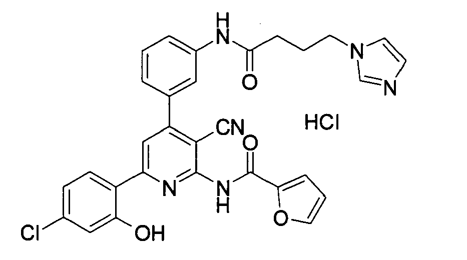

From the compound of Reference Example 166 (200 mg, 0.33 mmol), the title compound (189 mg, 95%) was given as a pale yellow powder in substantially the same manner as in Example 70.
¹H-NMR (300MHz, DMSO-d₆) δ: 2.10-2.20 (2H, m), 2.42 (2H, t, J = 7.2 Hz), 4.27 (2H, t, J = 7.2 Hz), 6.80 (1H, q, J = 1.5 Hz), 7.03 (1H, dd, J = 8.4, 2.1 Hz), 7.10 (1H, d, J = 2.1 Hz), 7.38 (1H, d, J = 7.8 Hz), 7.53-7.58 (2H, m), 7.70 (1H, s), 7.78-7.82 (2H, m), 7.98 (1H, s), 8.07 (1H, t, J = 0.9 Hz), 8.16 (1H, s), 8.18 (1H, d, J = 8.4 Hz), 9.15 (1H, s), 10.31 (1H, s), 11.25 (1H, s), 12.11(1H, s), 14.20-14.40 (1H, br).
Melting point: 166-170°C
Element analysis: Calculated (%) for C₃₀H₂₃N₆O₄Cl·1.0HCl·1.5H₂O: C, 57.15; H, 4.32; N, 13.33; Cl, 11.25. Found (%): C, 57.34; H, 4.52; N, 13.35; Cl, 11.34.

### Example 243

### N-{3-[6-(4-chloro-2-hydroxyphenyl)-3-cyano-2-(2-furoylamino)pyridin-4-yl]phenyl}-L-histidinamide dihydrochloride

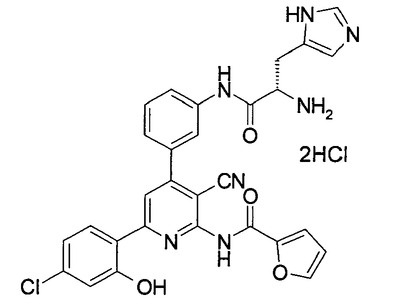

From the compound of Reference Example 167 (180 mg, 0.25 mmol), the title compound (155 mg, 97%) was given as a pale yellow powder in substantially the same manner as in Example 70.
¹H-NMR (300MHz, DMSO-d₆) δ: 3.26-3.32 (2H, m), 4.40-4.50 (1H, br), 6.80 (1H, q, J = 1.5 Hz), 7.05 (1H, dd, J = 8.7, 2.1 Hz), 7.12 (1H, d, J = 2.1 Hz), 7.47-7.55 (3H, m), 7.62 (1H, t, J = 7.8 Hz), 7.82 (1H, d, J = 8.1 Hz), 8.03 (1H, s), 8.07 (1H, d, J = 1.2 Hz), 8.16-8.19 (2H, m), 8.50-8.75 (3H, br), 8.99 (1H, s), 11.20 (1H, s), 11.30 (1H, s), 12.15 (1H, s), 13.80-14.80 (2H, br).
Melting point: 206-214°C
Element analysis: Calculated (%) for C₂₉H₂₂N₇O₄Cl·2.0HCl.2.0H₂O: C, 51.45; H, 4.17; N, 14.48; Cl, 15.71. Found (%): C, 51.70; H, 4.50; N, 14.55; Cl, 15.97.

### Example 244

### N-[4-(3-{[(2-aminoethyl)amino]carbonyl}phenyl)-3-cyano-6-(4-fluoro-2-hydroxyphenyl)pyridin-2-yl]-2-methoxybenzamide hydrochloride

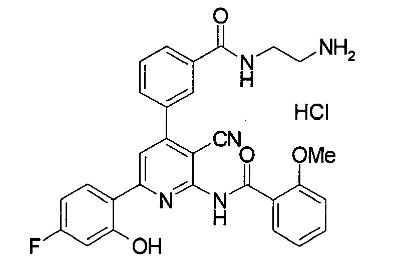

From the mixture of Reference Example 168 (250 mg) and tert-butyl N-(2-aminoethyl)carbamate (152 mg, 0.95 mmol), the compound specified in the title section title (50 mg) was given as a pale yellow crystal in substantially the same manner as in Example 78.
¹H-NMR (300MHz, DMSO-d₆) δ: 2.95-3.05 (2H, m), 3.50-3.60 (2H, m), 4.10 (3H, s), 6.70-6.85 (2H, m), 7.22 (1H, t, J = 7.5 Hz), 7.34 (1H, d, J = 8.4 Hz), 7.65-7.80 (2H, m), 7.98 (1H, d, J = 8.1 Hz), 8.03 (3H, br s), 8.10-8.20 (3H, m), 8.36 (1H, s), 8.35-8.45 (1H, m), 9.02 (1H, t, J=5.4 Hz), 11.36 (1H, br s), 13.45 (1H, br s).
Melting point: 227-229°C
Element analysis: Calculated (%) for C₂₉H₂₄N₅O₄F·1.0HCl·1.5H₂O: C, 59.13; H, 4.79; N. 11.89. Found (%): C, 59.22; H, 4.76; N, 11.90.

### Example 245

### N-{3-cyano-6-(4-fluoro-2-hydroxyphenyl)-4-[3-({[3-(1H-imidazol-1-yl)propyl]amino}c arbonyl)phenyl]pyridin-2-yl}-2-methoxybenzamide hydrochloride

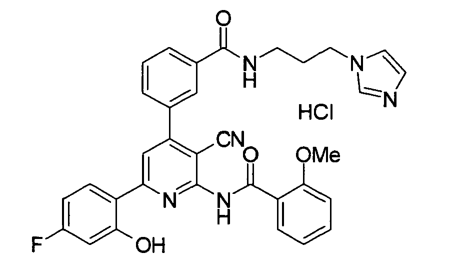

From the mixture of Reference Example 168 (250 mg) and 3-(1H-imidazol-1-yl)propane-1-amine (119 mg, 0.95 mmol), the title compound (55 mg) was given as a pale yellow crystal in substantially the same manner as in Example 78.
¹H-NMR (300MHz, DMSO-d₆) δ: 2.00-2.20 (2H, m), 3.20-3.40 (2H, m), 4.10 (3H, s), 4.25-4.40 (2H, m), 6.75-6.90 (2H, m), 7.22 (1H, t, J = 7.8 Hz), 7.35 (1H, d, J = 8.7 Hz), 7.60-7.80 (3H, m), 7.85 (1H, s), 7.96 (1H, d, J = 7.5 Hz), 8.05-8.15 (2H, m), 8.17 (1H, s), 8.26 (1H, s), 8.30-8.45 (1H, m), 8.90-9.10 (1H, m), 9.18 (1H, s), 11.40 (1H, s), 13.45 (1H, s), 14.50 (1H, br s).
Melting point: 234-235°C
Element analysis: Calculated (%) for C₃₃H₂₇N₆O₄F·1.0HCl·1.5H₂O: C, 60.60; H, 4.78; N. 12.85. Found (%): C, 60.63; H, 4.93; N, 12.89.

### Example 246

### N-[4-(3-{[(3-amino-2,2-dimethylpropyl)amino]carbonyl}phenyl)-3-cyano-6-(4-fluoro-2-hydroxyphenyl)pyridin-2-yl]-2-methoxybenzamide hydrochloride

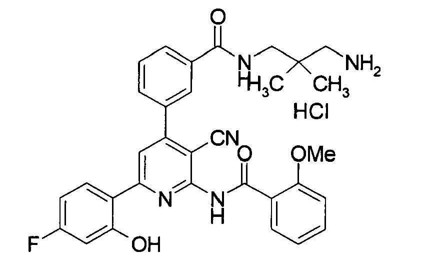

From the mixture of Reference Example 168 (250 mg) and tert-butyl (3-amino-2,2-dimethylpropyl)carboxylate (192 mg, 0.95 mmol), the title compound (31 mg) was given as a pale yellow crystal in substantially the same manner as in Example 78.
¹H-NMR (300MHz, DMSO-d₆) δ: 1.00 (6H, s), 2.60-2.75 (2H, m), 3.20-3.40 (2H, m), 4.10 (3H, s), 6.75-6.85 (2H, m), 7.22 (1H, t, J = 7.5 Hz), 7.35 (1H, d, J = 8.4 Hz), 7.65-7.80 (2H, m), 7.93 (3H, br s), 7.98 (1H, d, J = 7.8 Hz), 8.05-8.20 (2H, m), 8.18 (1H, s), 8.36 (1H, s), 8.38 (1H, t, J = 8.7 Hz), 9.08 (1H, br s), 11.38 (1H, s), 13.45 (1H, s).
Melting point: 244-246°C
Element analysis: Calculated (%) for C₃₂H₃₀N₅O₄F·1.0HCl·0.5H₂O: C, 62.69; H, 5.26; N. 11.42. Found (%): C, 62.59; H, 5.18; N, 11.44.

### Example 247

### N-[4-[3-(β-alanylamino)phenyl]-3-cyano-6-(4-fluoro-2-hydroxyphenyl)pyridin-2-yl]-2-methoxybenzamide hydrochloride

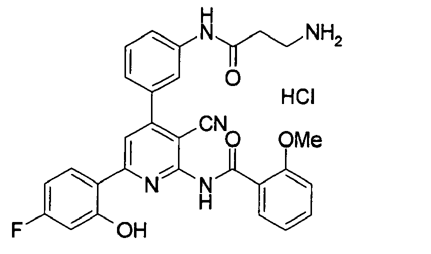

2-methoxybenzoyl chloride (8.49 mL, 57.0 mmol) was added to a solution of the compound of Reference Example 133 (3.0 g, 7.61 mmol) in pyridine (30 mL) with ice-cooling, and stirred at room temperature overnight. Saturated aqueous sodium hydrogencarbonate was added to the reaction mixture, and extracted with ethyl acetate. The extract was washed with saturated brine, dried over magnesium sulfate, and then concentrated under reduced pressure to give a crude product. The crude product was purified by amino silica gel chromatography (eluting solution: ethyl acetate/hexane = 1/2 to 1/1) to give a yellow solid (3.69 g). A solution of the resultant solid (3.50 g, 6.62 mmol), iron powder (2.22 g, 39.7 mmol) and ammonium chloride (2.30 g, 43.0 mmol) in ethanol (175 mL)-water (175 mL) was refluxed while heating for 4.5 hours. After cooling, saturated aqueous sodium hydrogencarbonate was added to the reaction mixture, and extracted with ethyl acetate. The extract was washed with saturated brine, dried over magnesium sulfate, and then concentrated under reduced pressure to give a crude product. The crude product was purified by silica gel chromatography (eluting solution: methanol/chloroform = 1/100 to 1/20) to give a yellow solid (2.60 g). A part of the resultant solid was washed with ethyl acetate to give a yellow solid (300 mg). WSCD (138 mg, 0.722 mmol) was added to a solution of the resultant solid (300 mg, 0.602 mmol), Boc-β-alanine (137 mg, 0.722 mmol) and HOBt (97.6 mg, 0.722 mmol) in DMF (3 mL), and stirred at room temperature for 2 days. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate/hexane = 1/5 to 3/5) to give a pale yellow amorphous solid (433 mg). The resultant solid (228 mg) was dissolved in a mixed solution of a 10% methanol solution of hydrogen chloride (6 mL) and dichloromethane (2 mL), and stirred at room temperature for 3 hours. The excessive hydrochloric acid and solvent were removed under reduced pressure. The resultant solid was recrystallized from DMF/diethylether to give the title compound (96 mg) as a yellow crystal. ¹H-NMR (300 MHz, DMSO-d₆) δ: 2.79 (2 H, t, J = 6.8 Hz), 3.11 (2 H, t, J = 6.8 Hz), 4.09 (3 H, s), 6.74-6.87 (2 H, m), 7.22 (1 H, t, J = 7.5 Hz), 7.35 (1 H, d, J = 7.9 Hz), 7.46 (1 H, d, J = 7.5 Hz), 7.58 (1 H, t, J = 7.9 Hz), 7.65 - 7.73 (1 H, m), 7.80-7.88 (3 H, m, J = 8.3 Hz), 8.00 (1 H, s), 8.02 (1 H, s), 8.11 (1 H, dd, J = 7.7, 1.7 Hz), 8.27 (1 H, dd, J = 8.9, 7.0 Hz), 10.52 (1 H, br s.), 11.36 (1 H, br s.), 13.38 (1 H, br s.).
Melting point: 232-233°C
Element analysis: Calculated (%) for C₂₉H₂₄N₅O₄F·1.0HCl·1.0H₂O: C,60.05; H, 4.69; N, 12.07. Found (%): C, 59.76; H, 4.81; N, 12.12.

### Example 248

### N-[4-[3-{[(2-aminoethyl)amino]carbonyl}-4-(dimethylamino)phenyl]-3-cyano-6-(2-hydroxyphenyl)pyridin-2-yl]thiophene-2-carboxamide dihydrochloride

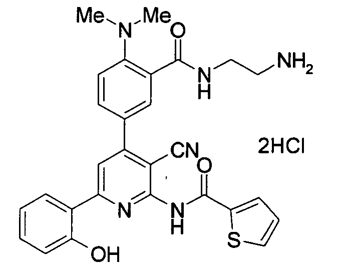

From the compound of Reference Example 173 (55 mg, 0.082 mmol), the title compound (42 mg, 85%) was given as an orange crystal in substantially the same manner as in Example 8.
¹H-NMR (300MHz, DMSO-d₆) δ: 2.95-3.02(8H, m), 3.55 (2H, q, J = 6.0 Hz), 6.95-7.02 (2H, m), 7.30-7.33 (2H, m), 7.40 (1H, t, J = 7.2 Hz), 7.81 (1H, dd, J = 8.7, 2.4 Hz), 7.94-8.10 (5H, m), 8.14 (1H, d, J = 3.6 Hz), 8.20-8.23 (2H, m), 8.93 (1H, s), 11.39 (1H, s).
Melting point: 210-216°C
Element analysis: Calculated (%) for C₂₈H₂₆N₆O₃S·2.0HCl·1.0H₂O: C, 54.46; H, 4.90; N, 13.61; Cl, 11.48. Found (%): C, 54.62; H, 5.12; N, 13.57; Cl, 11.44.

### Example 249

### N-[4-(3-{[(2-aminoethyl)amino]carbonyl}phenyl)-6-(4-chloro-2-hydroxyphenyl)-3-cyanopyridin-2-yl]-1,3-thiazole-2-carboxamide hydrochloride

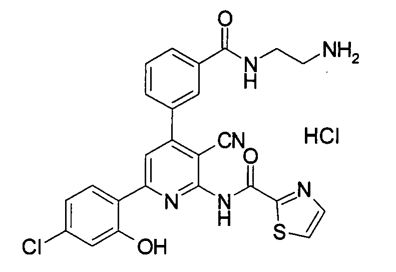

From the compound of Reference Example 175 (250 mg, 0.38 mmol), the title compound (205 mg, 97%) was given as a pale yellow crystal in substantially the same manner as in Example 8.
¹H-NMR (300MHz, DMSO-d₆) δ: 3.02-3.06 (2H, br), 3.55-3.59 (2H, br), 7.03 (1H, dd, J=8.7, 2.1 Hz), 7.10 (1H, d, J = 2.1 Hz), 7.76 (1H, t, J = 7.8 Hz), 7.93-8.10 (4H, m), 8.13 (1H, d, J = 7.5 Hz), 8.23-8.32 (5H, m), 9.00 (1H, br s), 11.41 (1H, s), 12.50 (1H, s).
Melting point: 242-244°C
Element analysis: Calculated (%) for C₂₅H₁₉N₆O₃SCl·1.0HCl·0.5H₂O: C, 53.20; H, 3.75; N, 14.89. Found (%): C, 53.36; H, 4.16; N, 14.68.

### Example 250

### N-{6-(4-chloro-2-hydroxyphenyl)-3-cyano-4-[3-({[3-(1H-imidazol-l-yl)propyl] amino}carbonyl)phenyl]pyridin-2-yl}-1,3-thiazole-2-carboxamide hydrochloride

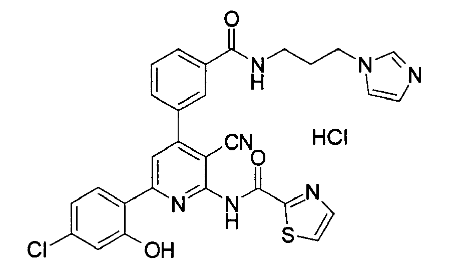

From the compound of Reference Example 176 (160 mg, 0.25 mmol), the title compound (149 mg, 96%) was given as a pale yellow crystal in substantially the same manner as in Example 8.
¹H-NMR (300MHz, DMSO-d₆) δ: 2.12 (2H, t, J = 6.9 Hz), 3.30-3.34 (2H, m), 4.29 (2H, t, J = 6.9 Hz), 7.04 (1H, dd, J = 8.4, 2.4 Hz), 7.10 (1H, d, J = 2.4 Hz), 7.69 (1H, d, J = 1.2 Hz), 7.75 (1H, t, J = 7.8 Hz), 7.84 (1H, d, J = 1.5 Hz), 7.93 (1H, d, J = 8.1 Hz), 8.09 (1H, d, J = 7.8 Hz), 8.23-8.31 (5H, m), 8.88 (1H, t, J = 5.4 Hz), 9.17 (1H, s), 11.42 (1H, s), 12.45 (1H, s), 14.30-14.60 (1H, br).
Melting point: 208-212°C
Element analysis: Calculated (%) for C₂₉H₂₂N₇O₃SCl·1.0HCl·0.5H₂O: C, 55.33; H, 3.84; N, 15.57; Cl, 11.26. Found (%): C, 55.23; H, 3.81; N, 15.55; Cl, 11.32.

### Example 251

### N-[4-[3-(β-alanylamino)phenyl]-6-(4-chloro-2-hydroxyphenyl)-3-cyanopyridin-2-yl] isoxazole-5-carboxamide hydrochloride

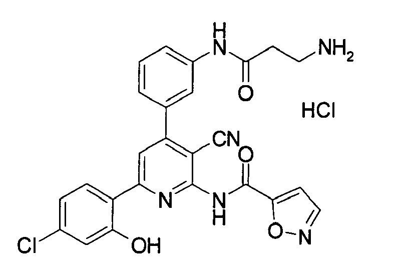

From the compound of Reference Example 179 (40 mg, 0.062 mmol), the title compound (16 mg, 48%) was given as a pale yellow crystal in substantially the same manner as in Example 8.
¹H-NMR (300MHz, DMSO-d₆) δ: 2.78 (2H, t, J = 6.9 Hz), 3.10 (2H, q, J = 6.3 Hz), 7.05 (1H, dd, J = 8.7, 2.1 Hz), 7.12 (1H, d, J = 2.1 Hz), 7.41 (1H, d, J = 7.5 Hz), 7.46 (1H, d, J = 2.1 Hz), 7.58 (1H, t, J = 8.1 Hz), 7.75-7.90 (4H, m), 8.02 (1H, s), 8.15 (1H, d, J = 8.4 Hz), 8.24 (1H, s), 8.92 (1H, d, J = 1.8 Hz), 10.54 (1H, s), 11.89 (1H, s), 11.98 (1H, s).
Melting point: 250°C or higher (decomposes)

### Example 252

### N-[4-[3-(β-alanylamino)phenyl]-6-(4-chloro-2-hydroxyphenyl)-3-cyanopyridin-2-yl]-4-methoxybenzamide hydrochloride

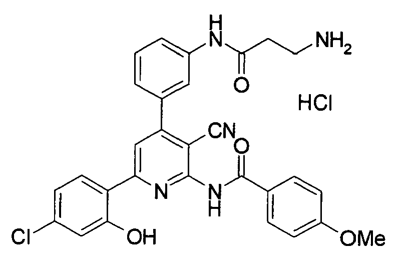

From the compound of Reference Example 182 (230 mg, 0.34 mmol), the title compound (171 mg, 87%) was given as a pale yellow crystal in substantially the same manner as in Example 8.
¹H-NMR (300MHz, DMSO-d₆) δ: 2.78 (2H, t, J = 6.6 Hz), 3.10 (2H, q, J = 6.0 Hz), 3.88 (3H, s), 7.04 (1H, dd, J = 8.7, 2.1 Hz), 7.10-7.16 (3H, m), 7.41 (1H, d, J = 7.8 Hz), 7.58 (1H, t, J = 7.8 Hz), 7.75-7.90 (4H, m), 8.00 (1H, s), 8.06 (2H, d, J = 9.0 Hz), 8.16-8.20 (2H, m), 10.53 (1H, s), 11.34 (1H, s), 12.26 (1H, s).
Melting point: 193-197°C
Element analysis: Calculated (%) for C₂₉H₂₄N₅O₄Cl·1.0HCl·3.0H₂O: C, 55.07; H, 4.94; N, 11.07. Found (%): C, 54.81; H, 4.91; N, 10.98.

### Example 253

### N-{3-[6-(4-chloro-2-hydroxyphenyl)-3-cyano-2-(2-furoylamino)pyridin-4-yl]phenyl}-D-histidinamide dihydrochloride

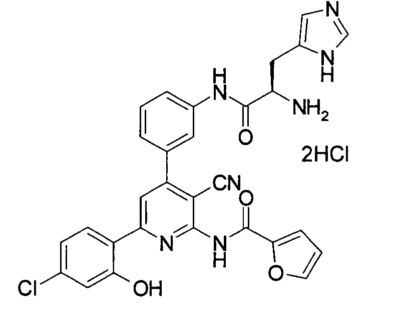

From the compound of Reference Example 183 (80 mg, 0.11 mmol), the title compound (77 mg, 100%) was given as a pale yellow crystal in substantially the same manner as in Example 8.
¹H-NMR (300MHz, DMSO-d₆) δ: 3.30-3.40 (2H, m), 4.50 (1H, br), 6.80 (1H, q, J = 1.8 Hz), 7.04 (1H, dd, J = 8.7, 2.1 Hz), 7.14 (1H, d, J = 2.1 Hz), 7.47 (1H, d, J = 7.8 Hz), 7.55 (2H, d, J = 3.0 Hz), 7.62 (1H, d, J = 7.8 Hz), 7.83 (1H, d, J = 8.1 Hz), 8.05 (2H, d, J = 8.7 Hz), 8.17 (2H, d, J = 7.2 Hz), 8.50-8.80 (3H, br), 9.02 (1H, s), 11.32 (2H, s), 12.17 (1H, s), 14.00-14.40 (2H, br).
Melting point: 200-206°C
Element analysis: Calculated (%) for C₂₉H₂₂N₇O₄Cl·2.0HCl·2.0H₂O: C, 51.45; H, 4.17; N, 14.48. Found (%): C, 51.34; H, 4.44; N, 14.48.

### Example 254

### N-[4-[3-(β-alanylamino)phenyl]-6-(4-chloro-2-hydroxyphenyl)-3-cyanopyridin-2-yl]-3-furamide hydrochloride

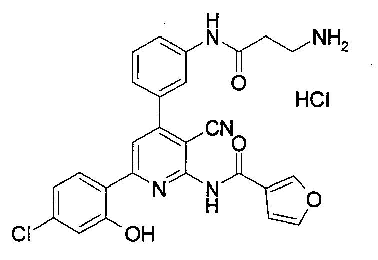

A mixture of the compound of Reference Example 130 (150 mg, 0.27 mmol) and 3-furoyl chloride (441 mg, 3.38 mmol) in pyridine (5 mL) was stirred at room temperature for 24 hours. Water was added to the reaction mixture, and extracted with ethyl acetate. The organic layer was washed with saturated aqueous sodium hydrogencarbonate and then with saturated brine, dried over magnesium sulfate, and then concentrated under reduced pressure. The residue was purified by silica gel column chromatography (eluting solution: hexane/ethyl acetate = 1/1) to give tert-butyl [3-({3-[6-[4-chloro-2-(methoxymethoxy)phenyl]-3-cyano-2-(3-furoylamino)pyridin-4-yl]phenyl}amino)-3-oxopropyl]carbamate (12 mg, 7%) as a white powder.

From the resultant tert-butyl [3-({3-[6-[4-chloro-2-(methoxymethoxy)phenyl]-3-cyano-2-(3-furoylamino)pyridin-4-yl]phenyl}amino)-3-oxopropyl]carbamate (10 mg, 0.015 mmol), the title compound (8 mg, 95%) was given as a pale yellow crystal in substantially the same manner as in Example 8.
¹H-NMR (300MHz, DMSO-d₆) δ: 2.78 (2H, t, J = 6.6 Hz), 3.05-3.18 (2H, m), 7.03-7.05 (2H, m), 7.11 (1H, d, J = 2.1 Hz), 7.40 (1H, d, J = 7.8 Hz), 7.57 (1H, t, J = 7.8 Hz), 7.81-7.90 (5H, m), 8.01 (1H, s), 8.15-8.18 (2H, m), 8.55 (1H, s), 10.52 (1H, s), 11.21 (1H, s), 12.08 (1H, s).
Melting point: 207-214°C

### Example 255

### N-[4-[3-(β-alanylamino)phenyl]-6-(4-chloro-2-hydroxyphenyl)-3-cyanopyridin-2-yl]-2,4-dimethoxybenzamide dihydrochloride

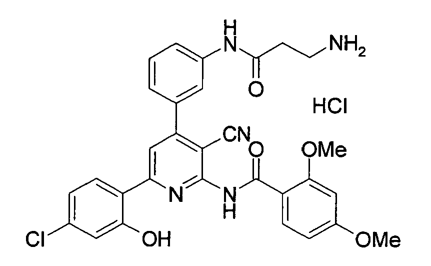

From the compound of Reference Example 186 (100 mg, 0.14 mmol), the title compound (71 mg, 83%) was given as a pale yellow crystal in substantially the same manner as in Example 8.
¹H-NMR (300MHz, DMSO-d₆) δ: 2.79 (2H, t, J = 6.6 Hz), 3.10 (2H, q, J = 6.1 Hz), 3.89 (3H, s), 4.10 (3H, s), 6.80 (1H, d, J = 7.2 Hz), 6.81 (1H, s), 6.98 (1H, d, J = 10.2 Hz), 7.06 (1H, d, J = 2.1 Hz), 7.45 (1H, d, J = 7.8 Hz), 7.58 (1H, t, J = 7.8 Hz), 7.82-7.95 (4H, m), 8.00 (2H, s), 8.10 (1H, d, J = 9.3 Hz), 8.22 (1H, d, J = 8.7 Hz), 10.54 (1H, s), 11.22 (1H, s), 13.40 (1H, br s).
Melting point: 225-230°C

### Example 256

### 4-[3-(β-alanylamino)phenyl]-2-(benzoylamino)-6-(2-hydroxyphenyl)nicotinic acid hydrochloride

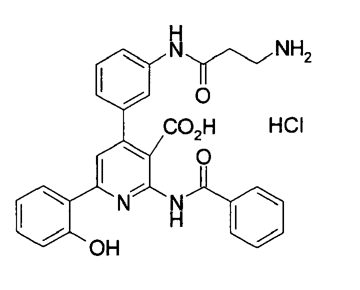

A mixture of the compound of Reference Example 187 (150 mg, 0.21 mmol) and palladium-carbon (100 mg) in ethanol (10 mL)-ethyl acetate (10 mL) was stirred under 3 atm of hydrogen at room temperature for 72 hours. A 4N hydrogen chloride-ethyl acetate solution (0.5 mL) was added to the reaction mixture. The precipitate was collected by filtration, and the filtrate was concentrated under reduced pressure. The residue was recrystallized from methanol-diethylether to give the title compound (96 mg, 86%) as a yellow crystal.
¹H-NMR (300MHz, DMSO-d₆) δ: 2.76 (2H, t, J = 6.3 Hz), 3.05-3.12 (2H, m), 6.89-6.98 (2H, m), 7.15 (1H, d, J = 7.5 Hz), 7.34-7.46 (2H, m), 7.59-7.71 (5H, m), 7.76 (1H, s), 7.86 (3H, br s), 8.00 (2H, d, J = 8.1 Hz), 8.09 (1H, d, J = 7.8 Hz), 10.41 (1H, s), 11.34 (1H, s), 12.70-13.10(1H, br), 13.30-13.90 (1H, br).
Melting point: 250-253°C
Element analysis: Calculated (%) for C₂₈H₂₄N₄O₅Cl·1.0HCl·2.5H₂O: C, 58.18; H, 5.23; N, 9.69. Found (%): C, 58.50; H, 5.24; N, 9.66.

### Example 257

### N-[4-[3-({[(2S)-2-aminopropyl]amino}carbonyl)phenyl]-3-cyano-6-(4-fluoro-2-hydroxyphenyl)pyridin-2-yl] isoxazole-5-carboxamide hydrochloride

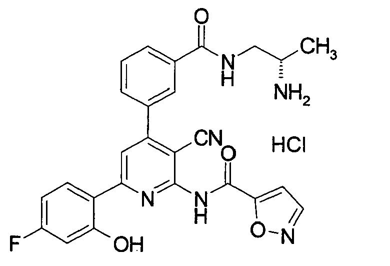

From the compound of Reference Example 189 (35 mg, 0.054 mmol), the title compound (27 mg, 93%) was given as a yellow crystal in substantially the same manner as in Example 8.
¹H-NMR (300MHz, DMSO-d₆) δ: 1.26 (3H, d, J = 6.3 Hz), 2.73 (2H, t, J = 5.4 Hz), 3.50-3.70 (1H, br), 6.83-6.88 (2H, m), 7.12-7.46 (2H, m), 7.59 (1H, t, J = 8.1 Hz), 7.82 (1H, d, J = 8.7 Hz), 7.93 (3H, br s), 8.02 (1H, s), 8.20-8.24 (2H, m), 8.93 (1H, s), 10.56 (1H, s), 12.00 (1H, s), 12.12 (1H, s).
Melting point: 250°C or higher (decomposes)

### Example 258

### N-[4-(3-{[amino(phenyl)acetyl]amino}phenyl)-3-cyano-6-(4-fluoro-2-hydroxyphenyl) pyridin-2-yl]-2-furamide hydrochloride

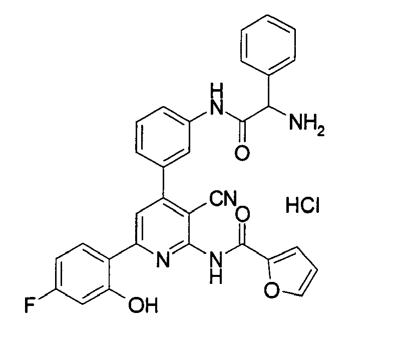

From the compound of Reference Example 135 (120 mg, 0.26 mmol) and Boc-DL-phenylglycine (131 mg, 0.52 mmol), the title compound (65 mg, 43%) was given as a yellow powder in substantially the same manner as in Example 78.
¹H-NMR (400 MHz, DMSO-d₆) δ: 5.22 (1H, s), 6.75-6.85 (3H, m), 7.30-7.70 (8H, m), 7.84 (1H, d, J = 7.2 Hz), 7.98 (1H, s), 8.08 (1H, s), 8.14 (1H, s), 8.22 (1H, t, J = 8.0 Hz), 8.83 (3H, br s), 11.15 (1H, br s), 11.32 (1H, s), 12.34 (1H, s).
Melting point: 224-225°C
Element analysis: Calculated (%) for C₃₁H₂₂N₅O₄F·1.0HCl·1.0H₂O: C, 61.85; H, 4.19; N. 11.63. Found (%): C, 61.80; H, 4.00; N, 11.35.

### Example 259

### N-(3-cyano-6-(4-fluoro-2-hydroxyphenyl)-4-{3-[(pyrrolidin-1-ylacetyl)amino]phenyl}p yridin-2-yl)-2-furamide hydrochloride

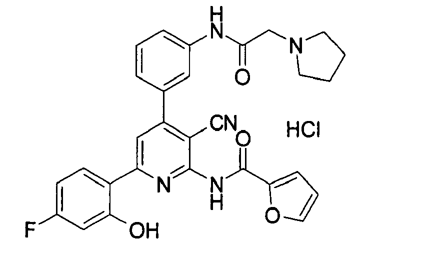

A 4N hydrogen chloride-ethyl acetate solution (5 mL) was added to a solution of the compound of Reference Example 169 (150 mg, 0.26 mmol) in ethyl acetate (5 mL)-methanol (2 ml), and stirred at room temperature for 14 hours. The solvent was removed under reduced pressure, and the residue was recrystallized from methanol-diethylether to give the title compound (140 mg, 96%) as a yellow powder.
¹H-NMR (300MHz, DMSO-d₆) δ: 1.94-2.00 (4H, m), 3.17 (2H, brs), 3.63 (2H, br s), 4.32 (2H, s), 6.79-6.90 (3H, m), 7.47 (1H, d, J = 8.1 Hz), 7.55 (1H, dd, J = 0.9 Hz, 3.6 Hz), 7.63 (1H, t, J = 8.1 Hz), 7.82-7.85 (1H, m), 8.03 (1H, brs), 8.08 (1H, dd, J = 0.6 Hz, 1.5 Hz), 8.16 (1H, s), 8.24 (1H, dd, J = 6.9 Hz, 8.7 Hz), 10.33 (1H, br s), 11.10 (1H, s), 11.35 (1H, s), 12.33 (1H, s).
Melting point: 203-205°C
Element analysis: Calculated (%) for C₂₉H₂₄N₅O₄F·1.0HCl·1.0H₂O: C, 60.05; H, 4.69; N, 12.07. Found (%): C, 59.90; H, 4.69; N, 12.02.

### Example 260

### N-[3-cyano-6-(4-fluoro-2-hydroxyphenyl)-4-{3-[(piperidin-1-ylacetyl]amino}phenyl) pyridin-2-yl]-2-furamide hydrochloride

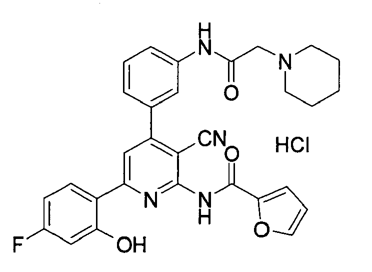

A 4N hydrogen chloride-ethyl acetate solution (5 mL) was added to a solution of the compound of Reference Example 261 (160 mg, 0.27 mmol) in ethyl acetate (5 mL)-methanol (2 ml), and stirred at room temperature for 14 hours. The solvent was removed under reduced pressure, and the residue was recrystallized from methanol to give the title compound (100 mg, 64%) as a pale yellow powder.
¹H-NMR (300MHz, DMSO-d₆) δ: 1.42 (1H, br s), 1.68-1.80 (5H, m), 3.10 (2H, br s), 3.50 (2H, d, J = 11.7 Hz), 4.20 (2H, br s), 6.79-6.90 (3H, m), 7.48 (1H, d, J = 7.8 Hz), 7.55 (1H, d, J = 3.6 Hz), 7.63 (1H, t, J = 7.8 Hz), 7.83 (1H, d, J = 7.8 Hz), 8.05-8.08 (2H, m), 8.16 (1H, s), 8.23 (1H, dd, J = 6.9, 8.7 Hz), 9.92 (1H, br s), 11.17 (1H, s), 11.35 (1H, s), 12.32 (1H, s).
Melting point: 249-251°C
Element analysis: Calculated (%) for C₃₀H₂₆N₅O₄F·1.0HCl·1.0H₂O: C, 60.66; H, 4.92; N, 11.79. Found (%): C, 60.45; H, 5.06; N, 12.00.

### Example 261

### N-{3-[3-cyano-6-(4-fluoro-2-hydroxyphenyl)-2-(2-furoylamino)pyridin-4-yl]phenyl}-D-histidinamide hydrochloride

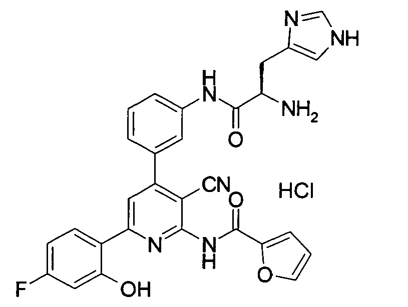

From the compound of Reference Example 135 (120 mg, 0.26 mmol) and Boc-D-histidine (134 mg, 0.52 mmol), the title compound (44 mg, 24%) was given as a yellow powder in substantially the same manner as in Example 78.
¹H-NMR (300MHz, DMSO-d₆) δ: 3.20-3.50 (2H, m), 4.43 (1H, br s), 6.75-6.95 (3H, m), 7.45-7.60 (3H, m), 7.63 (1H, t, J = 7.8 Hz), 7.81 (1H, d, J = 7.8 Hz), 8.01 (1H, s), 8.08 (1H, s), 8.15 (1H, s), 8.20-8.30 (1H, m), 8.56 (3H, br s), 9.00 (1H, br s), 11.15 (1H, br s), 11.36 (1H, s), 12.28 (1H, s).
Melting point: 218-219°C

### Example 262

### tert-butyl {2-[({1-[3-cyano-2-(2-furoylamino)-6-(2-hydroxyphenyl)pyridin-4-yl] piperidine-3-yl}carbonyl)amino]ethyl}carbamate

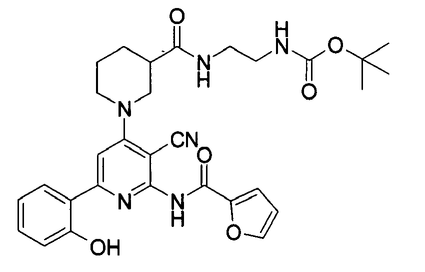

A solution of the compound of Reference Example 193 (210 mg, 0.40 mmol), tert-butyl (2-aminoethyl)carbamate (77 mg, 0,48 mmol), WSCD (93 mg, 0,48 mmol) and HOBt (65 mg, 0,48 mmol) in DMF (3 mL) was stirred at room temperature for 12 hours. The reaction mixture was concentrated under reduced pressure. Then, the residue was dissolved in ethyl acetate (10 ml), washed with water and then with saturated brine, dried over magnesium sulfate, and then concentrated under reduced pressure. The residue was purified by silica gel chromatography (eluting solution: ethyl acetate/hexane = 9/1 to ethyl acetate/hexane = 2/3) to give tert-butyl {2-[({1-[6-[2-(benzyloxy)phenyl]-3-cyano-2-(2-furoylamino)pyridin-4-yl]piperidine-3-yl}carbonyl)amino]ethyl}carbamate (109 mg, 41%) as an oily substance.

A mixed solution of tert-butyl {2-[({1-[6-[2-(benzyloxy)phenyl]-3-cyano-2-(2-furoylamino)pyridin-4-yl]piperidin-3-yl}carbonyl)amino]ethyl}carbamate (95 mg, 0.14 mmol), 10% Pd-C (50% wet, 20 mg), ethyl acetate(5 ml) and THF (5 ml) was stirred under 3 atm of hydrogen atmosphere at room temperature for 3 hours. The catalyst was filtered out, and the reaction solution was concentrated under reduced pressure. The residue was purified by silica gel chromatography (eluting solution: ethyl acetate/hexane = 9/1 to ethyl acetate/hexane = 2/3) to give the title compound (30 mg, 37%) as a white powder.
¹H-NMR (300MHz, CDCl₃) δ : 1.42 (9H, s), 1.70-2.00 (3H, m), 2.02-2.18 (1H, m), 2.51-2.70 (1H, m), 3.12 (1H, t, J = 10.8 Hz), 3.20-3.48 (5H, m), 4.00 (1H, d, J = 11.7 Hz), 4.13 (1H, d, J = 11.7 Hz), 4.97 (1H, br s), 6.55-6.70 (2H, m), 6.86 (1H, td, J = 6.9, 1.2 Hz), 7.01 (1H, s), 7.04 (1H, dd, J = 8.4, 1.2 Hz), 7.29-7.36 (1H, m), 7.40 (1H, dd, J = 3.6, 0.6 Hz), 7.60 (1H, dd, J = 1.5, 0.6 Hz), 7.65 (1H, d, J = 7.2 Hz), 8.93 (1H, s), 13.37 (1H, s).

### Example 263

### N-{3-[3-cyano-6-(4-fluoro-2-hydroxyphenyl)-2-(2-furoylamino)pyridin-4-yl]phenyl}-D-phenylalaninamide hydrochloride

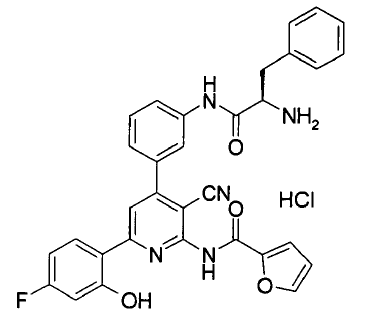

From the compound of Reference Example 135 (120 mg, 0.26 mmol) and Boc-D-phenylalanine (139 mg, 0.52 mmol), the title compound (66 mg, 36%) was given as a yellow powder in substantially the same manner as in Example 78.
¹H-NMR (400 MHz, DMSO-d₆) δ : 2.30-2.50 (2H, m), 4.26 (1H, s), 6.70-6.90 (3H, m), 7.00-8.60 (16H, m).
Melting point: 186-188°C
Element analysis: Calculated (%) for C₃₂H₂₄N₅O₄F·1.0HC1·4.0H₂O: C, 57.36; H, 4.96; N. 10.45. Found (%): C, 57.06; H, 4.60; N, 10.22.

### Example 264

### N-[4-{3-[(3-aminopentanoyl)amino]phenyl}-3-cyano-6-(4-fluoro-2-hydroxyphenyl) pyridin-2-yl]-2-furamide hydrochloride

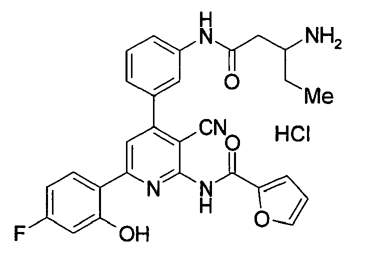

From the compound of Reference Example 135 (120 mg, 0.26 mmol) and N-Boc-3-aminopentanoic acid (114 mg, 0.52 mmol), the title compound (105 mg, 73%) was given as a yellow powder in substantially the same manner as in Example 78.
¹H-NMR (400 MHz, DMSO-d₆) δ : 0.95 (3H, t, J = 7.6 Hz), 1.55-1.75 (2H, m), 2.65-2.90 (2H, m), 3.46 (1H, br s), 6.75-6.90 (3H, m), 7.42 (1H, d, J = 7.6 Hz), 7.54 (1H, d, J = 3.6 Hz), 7.58 (1H, t, J = 8.0 Hz), 7.83 (1H, d, J = 8.8 Hz), 8.01 (4H, br s), 8.08 (1H, s), 8.14 (1H, s), 8.23 (1H, t, J = 7.6 Hz), 10.63 (1H, s), 11.31 (1H, s), 12.33 (1H, s).
Melting point: 212-214°C
Element analysis: Calculated (%) for C₂₈H₂₄N₅O₄F·1.0HCl·1.5H₂O: C, 58.28; H, 4.89; N. 12.14. Found (%): C, 58.36; H, 4.77; N, 12.13.

### Example 265

### N-[4-{3-[(3-aminohexanoyl)amino]phenyl}-3-cyano-6-(4-fluoro-2-hydroxyphenyl) pyridin-2-yl]-2-furamide hydrochloride

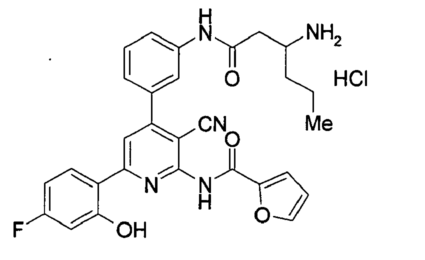

From the compound of Reference Example 135 (120 mg, 0.26 mmol) and N-Boc-3-aminohexanoic acid (121 mg, 0.52 mmol), the title compound (107 mg, 73%) was given as a yellow powder in substantially the same manner as in Example 78.
¹H-NMR (400 MHz, DMSO-d₆) δ : 0.90 (3H, t, J = 6.8 Hz), 1.25-1.45 (2H, m), 1.45-1.70 (2H, m), 2.50-2.80 (2H, m), 3.53 (1H, br s), 6.75-6.90 (3H, m), 7.43 (1H, d, J = 8.0 Hz), 7.53 (1H, d, J = 3.6 Hz), 7.58 (1H, t, J = 7.6 Hz), 7.82 (1H, d, J = 8.0 Hz), 7.90 (3H, br s), 7.99 (1H, s), 8.08 (1H, s), 8.13 (1H, s), 8.15-8.30 (1H, m), 10.54 (1H, s), 11.31 (1H, s), 12.32 (1H, s).
Melting point: 214-217°C
Element analysis: Calculated (%) for C₂₉H₂₆N₅O₄F·1.0HCl·1.0H₂O: C, 59.84; H, 5.02; N. 12.03. Found (%): C, 60.09; H, 4.92; N, 12.07.

### Example 266

### N-[4-{3-[(3-amino-4-methylpentanoyl)amino]phenyl}-3-cyano-6-(4-fluoro-2-hydroxyphenyl)pyridin-2-yl]-2-furamide hydrochloride

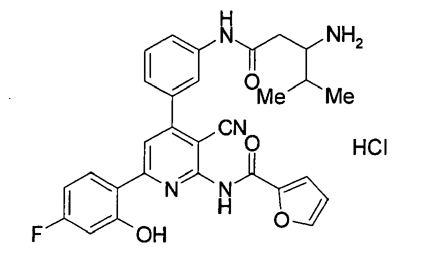

From the compound of Reference Example 135 (120 mg, 0.26 mmol) and Boc-β-leucine(121 mg, 0.52 mmol), the title compound (72 mg, 49%) was given as a yellow powder in substantially the same manner as in Example 78.
1H-NMR (400 MHz, DMSO-d₆) δ:0.95 (6H, d, J=6.8 Hz), 1.90-2.00 (1H, m), 2.50-2.90 (2H, m), 3.30-3.50 (1H, m), 6.75-6.90 (3H, m), 7.41 (1H, d, J=8.0 Hz), 7.53 (1H, d, J=3.6 Hz), 7.58 (1H, t, J=8.0 Hz), 7.82 (1H, d, J=8.8 Hz), 7.92 (1H, brs), 8.00 (1H, s), 8.07 (1H, s), 8.13 (1H, s), 8.22 (1H, t, J=7.2 Hz), 10.58 (1H, s), 11.31 (1H, s), 12.31 (1H, s).
Melting point: 214-217°C
Element analysis: Calculated (%) for C₂₉H₂₆N₅O₄F·1.0HCl·1.0H₂O: C, 59.84; H, 5.02; N. 12.03. Found (%): C, 59.70; H, 5.06; N, 11.96.

### Example 267

### N-{4-[3-({[(1SR,2RS)-2-aminocyclohexyl]carbonyl}amino)phenyl]-3-cyano-6-(4-fluoro-2-hydroxyphenyl)pyridin-2-yl}-2-furamide hydrochloride

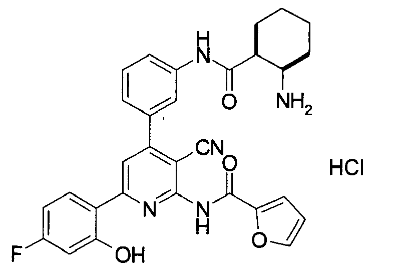

From the compound of Reference Example 135 (120 mg, 0.26 mmol) and Boc-cis-2-aminocyclohexane-1-carboxylic acid (128 mg, 0.52 mmol), the title compound (33 mg, 18%) was given as a yellow powder in substantially the same manner as in Example 78.
¹H-NMR (400 MHz, DMSO-d₆) δ : 1.40-3.50 (10H, m), 6.80-6.90 (3H, m), 7.43 (1H, d, J = 8.0 Hz), 7.54 (1H, d, J = 3.2 Hz), 7.60 (1H, t, J = 8.0 Hz), 7.84 (1H, d, J = 7.6 Hz), 7.90 (3H, br s), 8.02 (1H, s), 8.09 (1H, s), 8.14 (1H, s), 8.24 (1H, t, J = 7.2 Hz), 10.51 (1H, s), 11.33 (1H, s), 12.32 (1H, s). Melting point: 224-225°C
Element analysis: Calculated (%) for C₃₀H₂₆N₅O₄F·1.0HCl·1.5H₂O: C, 59.75; H, 5.01; N. 11.61. Found (%): C, 59.63; H, 5.07; N, 11.42.

### Example 268

### N-[4-[3-({[(2S)-2-aminopropyl]amino}carbonyl)phenyl]-6-(4-chloro-2-hydroxyphenyl) -3-cyanopyridin-2-yl] isoxazole-5-carboxamide hydrochloride

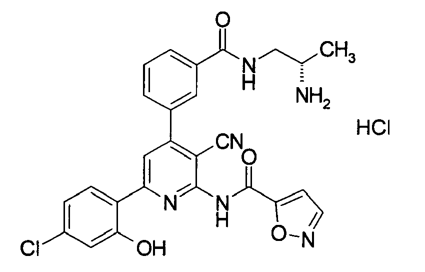

A 4N hydrogen chloride-ethyl acetate solution (0.2 mL) was added to a solution of the compound of Reference Example 195 (10 mg, 0.015 mmol) in dichloromethane (1 mL), and stirred at room temperature for 2 hours. The precipitated crystals were collected by filtration, and recrystallized from methanol-diethylether to give the title compound (2 mg, 24%) as a yellow crystal.
¹H-NMR (300MHz, DMSO-d₆) δ : 1.25 (3H, d, J = 6.6 Hz), 2.71 (2H, d, J = 5.7 Hz), 3.58-3.69 (1H, br), 7.04-7.11 (2H, m), 7.43 (1H, d, J = 9.0 Hz), 7.44 (1H, s), 7.59 (1H, t, J = 8.1 Hz), 7.75-7.90 (4H, m), 8.00 (1H, s), 8.16 (1H, d, J = 8.7 Hz), 8.23 (1H, s), 8.92 (1H, s), 10.51 (1H, s), 11.89 (1H, s), 11.99 (1H, s).
Melting point: 250°C or higher (decomposes)

### Example 269

### N-[4-(3-{[(3S)-3-aminobutanoyl]amino}phenyl)-6-(4-chloro-2-hydroxyphenyl)-3-cyanopyridin-2-yl]-1,3-thiazole-2-carboxamide hydrochloride

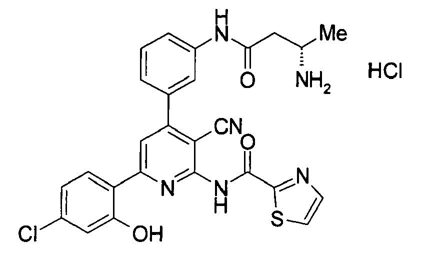

From the compound of Reference Example 198 (18 mg, 0.027 mmol), the title compound (13 mg, 85%) was given as a yellow crystal in substantially the same manner as in Example 8.
¹H-NMR (300MHz, DMSO-d₆) δ : 1.26 (3H, d, J = 6.3 Hz), 2.63-2.80 (2H, m), 3.51-3.68 (1H, m), 7.05 (1H, d, J=8.4 Hz), 7.10 (1H, d, J = 1.8 Hz), 7.44 (1H, d, J = 7.8 Hz), 7.59 (1H, t, J = 7.8 Hz), 7.81-8.01 (5H, m), 8.20-8.24 (3H, m), 8.30 (1H, d, J = 3.0 Hz), 10.56 (1H, s), 11.42 (1H, s), 12.42 (1H, s). Melting point: 218-221°C
Element analysis: Calculated (%) for C₂₆H₂₁N₆O₃SCl·1.0HCl·1.5H₂O: C, 52.35; H, 4.22; N, 14.09. Found (%): C, 52.62; H, 4.19; N, 14.11.

### Example 270

### N-[4-(3-{[(3R)-3-aminobutanoyl]amino}phenyl)-6-(4-chloro-2-hydroxyphenyl)-3-cyanopyridin-2-yl]-1,3-thiazole-2-carboxamide hydrochloride

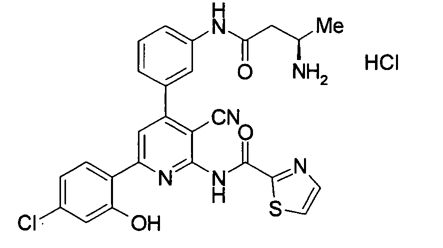

From the compound of Reference Example 199 (21 mg, 0.031 mmol), the title compound (12 mg, 68%) was given as a yellow crystal in substantially the same manner as in Example 8.
¹H-NMR (300MHz, DMSO-d₆) δ : 1.26 (3H, d, J = 6.6 Hz), 2.66-2.81 (2H, m), 3.55-3.70 (1H, m), 7.04 (1H, dd, J = 8.4, 2.1 Hz), 7.10 (1H, d, J = 2.1 Hz), 7.44 (1H, d, J = 7.5 Hz), 7.59 (1H, t, J = 7.8 Hz), 7.83 (1H, d, J = 8.4 Hz), 7.93 (3H, br s), 8.01 (1H, s), 8.20-8.24 (3H, m), 8.30 (1H, d, J = 3.0 Hz), 10.56 (1H, s), 11.44 (1H, s), 12.40 (1H, s).
Melting point: 218-221°C
Element analysis: Calculated (%) for C₂₆H₂₁N₆O₃SCl·1.0HCl·1.5H₂O: C, 52.35; H, 4.22; N, 14.09. Found (%): C, 52.53; H, 4.05; N, 13.94.

### Example 271

### N-[4-{3-[(3-aminobutanoyl)amino]phenyl}-6-(4-chloro-2-hydroxyphenyl)-3-cyanopyridin-2-yl]-5-methyl-2-furamide hydrochloride

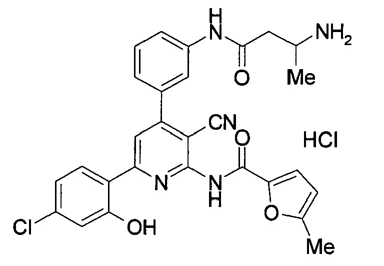

A 4N hydrogen chloride-ethyl acetate solution (0.75 mL) was added to a solution of the compound of Reference Example 201 (35 mg, 0.052 mmol) in ethyl acetate (3 mL), and stirred at room temperature for 6 hours. The precipitated crystals were collected by filtration, and recrystallized from methanol-diethylether to give the title compound (28 mg, 95%) as a pale yellow crystal.
¹H-NMR (300MHz, DMSO-d₆) δ : 1.25 (3H, d, J = 6.3 Hz), 2.43 (3H, s), 2.65-2.80 (2H, m), 3.55-3.69 (1H, m), 6.43 (1H, d, J = 3.3 Hz), 7.03 (1H, dd, J = 8.7, 1.8 Hz), 7.09 (1H, d, J = 1.8 Hz), 7.40-7.44 (2H, m), 7.58 (1H, t, J = 7.8 Hz), 7.80-7.99 (5H, m), 8.15-8.19 (2H, m), 10.52 (1H, s), 10.90-11.30 (1H, br), 12.00-12.30 (1H, br).
Melting point: 206-212°C
Element analysis: Calculated (%) for C₂₈H₂₄N₅O₄Cl·1.0HCl.2.2H₂O: C, 55.49; H, 4.89; N, 11.56. Found (%): C, 55.47; H, 5.03; N, 11.48.

### Example 272

### N-(2-aminoethyl)-3'-cyano-2'-(2-furoylamino)-6'-(2-hydroxyphenyl)-4,4'-bipyridine-2-carboxamide dihydrochloride

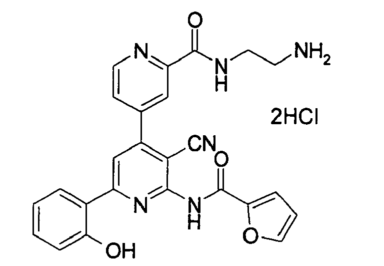

From the compound of Reference Example 205 (80 mg, 0.13 mmol), the title compound (65 mg, 99%) was given as a pale yellow crystal in substantially the same manner as in Example 271.
¹H-NMR (300MHz, DMSO-d₆) δ: 3.04 (2H, t, J = 6.0 Hz), 3.55-3.65 (2H, m), 6.80 (1H, s), 6.96-7.03 (2H, m), 7.42 (1H, t, J = 7.5 Hz), 7.55 (1H, d, J = 2.7 Hz), 7.60-8.17 (6H, m), 8.30 (1H, s), 8.37 (1H, s), 8.95 (1H, d, J = 4.8 Hz), 9.22 (1H, t, J = 6.0 Hz), 10.85 (1H, br s).
Melting point: 204-208°C

### Example 273

### N-[4-(3-{[(2-aminoethyl)amino]carbonyl}-2-methoxyphenyl)-3-cyano-6-(2-hydroxyphenyl)pyridin-2-yl]-2-furamide hydrochloride

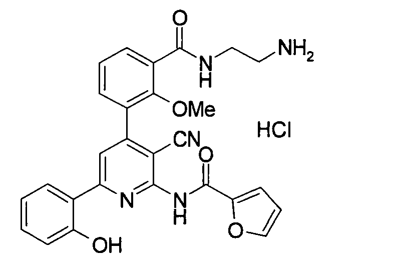

From the compound of Reference Example 209 (40 mg, 0.062 mmol), the title compound (28 mg, 85%) was given as a pale yellow crystal in substantially the same manner as in Example 271.
¹H-NMR (300MHz, DMSO-d₆) δ : 3.00 (2H, t, J = 6.3 Hz), 3.56 (2H, q, J = 6.0 Hz), 3.65 (3H, s), 6.79 (1H, q, J = 1.5 Hz), 6.94-7.03 (2H, m), 7.40 (2H, t, J = 7.5 Hz), 7.53 (1H, d, J = 3.3 Hz), 7.60 (1H, dd, J = 7.5, 1.5 Hz), 7.70-8.20 (7H, m), 8.64 (1H, t, J = 5.7 Hz), 10.80-11.70 (1H, br), 11.83 (1H, br s).
Melting point: 182-188°C
Element analysis: Calculated (%) for C₂₇H₂₃N₅O₅·1.0HCl·1.5H₂O: C, 57.81; H, 4.85; N, 12.48. Found (%): C, 58.05; H, 4.60; N, 12.62.

### Example 274

### N-[4-{3-[(3-aminobutanoyl)amino]phenyl}-6-(4-chloro-2-hydroxyphenyl)-3-cyanopyridin-2-yl]-1-methyl-1H-pyrrole-2-carboxamide hydrochloride

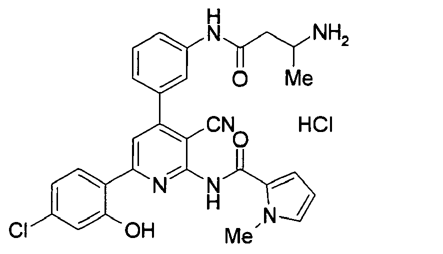

From the compound of Reference Example 210 (14 mg, 0.021 mmol), the title compound (10 mg, 81%) was given as a pale yellow crystal in substantially the same manner as in Example 271.
¹H-NMR (300MHz, DMSO-d₆) δ : 1.25 (3H, d, J = 6.6 Hz), 2.73 (2H, t, J = 6.0 Hz), 3.55-3.70 (1H, m), 3.91 (3H, s), 6.18-6.20 (1H, m), 7.03 (1H, dd, J = 8.7, 1.8 Hz), 7.10 (1H, s), 7.16-7.19 (2H, m), 7.40 (1H, d, J = 7.8 Hz), 7.57 (1H, t, J = 7.8 Hz), 7.81-8.00 (5H, m), 8.09 (1H, s), 8.19 (1H, d, J = 8.7 Hz), 10.54 (1H, s), 10.95 (1H, br s), 12.20-12.50 (1H, br).
Melting point: 209-213°C

### Example 275

### tert-butyl {3-[3-cyano-2-(2-furoylamino)-6-(2-hydroxyphenyl)pyridin-4-yl]-2-methoxyphenyl} carbamate

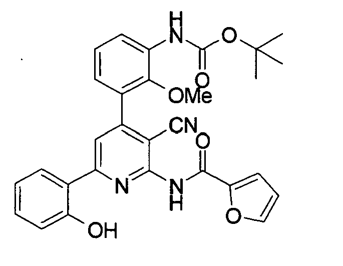

From tert-butyl (3-{3-cyano-2-(2-furoylamino)-6-[2-(methoxymethoxy)phenyl] pyridin-4-yl}-2-methoxyphenyl)carbamate of Reference Example 211 (25 mg, 0.044 mmol), the title compound (15 mg, 65%) was given as a white crystal in substantially the same manner as in Example 271.
¹H-NMR (300MHz, DMSO-d₆) δ : 1.49 (9H, s), 3.50 (3H, s), 6.79 (1H, q, J = 1.5 Hz), 6.92-7.00 (2H, m), 7.16 (1H, dd, J = 7.8, 1.5 Hz), 7.26 (1H, t, J = 6.6 Hz), 7.36-7.41(1H, m), 7.50 (1H, d, J = 3.0 Hz), 7.90 (1H, dd, J = 8.4, 1.2 Hz), 8.07 (1H, s), 8.10-8.15 (2H, m), 8.60 (1H, s), 11.35 (1H, s), 11.90 (1H, br s).
Melting point: 245-246°C

### Example 276

### N-[4-[3-(β-alanylamino)-2-methoxyphenyl]-3-cyano-6-(2-hydroxyphenyl)pyridin-2-yl]-2-furamide hydrochloride

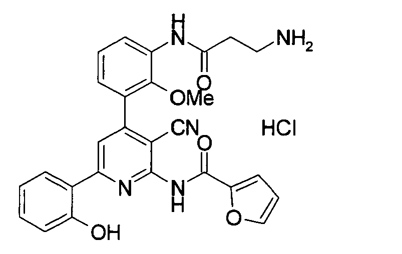

From the compound of Reference Example 212 (40 mg, 0.062 mmol), the title compound (12 mg, 39%) was given as a white crystal in substantially the same manner as in Example 271.
¹H-NMR (300MHz, DMSO-d₆) δ : 2.86 (2H, t, J = 6.3 Hz), 3.12 (2H, t, J = 6.3 Hz), 3.51 (3H, s), 6.80 (1H, q, J = 1.8 Hz), 6.94-7.02 (2H, m), 7.24 (1H, d, J = 7.5 Hz), 7.32 (1H, t, J = 7.8 Hz), 7.40 (1H, t, J = 7.5 Hz), 7.52 (1H, d, J = 1.8 Hz), 7.60-7.90 (3H, br), 8.07 (1H, s), 8.12 (1H, d, J = 8.1 Hz), 8.19-8.20 (2H, m), 8.90 (1H, s), 11.10-11.50 (1H, br), 11.89 (1H, s).
Melting point: 219-220°C

### Example 277

### N-{4-[3-({[(1SR,2SR)-2-aminocyclohexyl]carbonyl}amino)phenyl]-3-cyano-6-(4-fluoro-2-hydroxyphenyl)pyridin-2-yl}-2-furamide hydrochloride

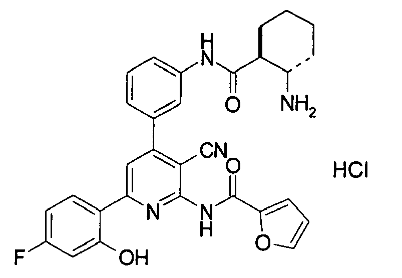

From the compound of Reference Example 135 (120 mg, 0.26 mmol) and Boc-trans-2-aminocyclohexane-1-carboxylic acid (128 mg, 0.52 mmol), the title compound (35 mg, 23%) was given as a yellow powder in substantially the same manner as in Example 78.
¹H-NMR (300MHz, DMSO-d₆) δ : 1.30-1.50 (4H, m), 1.70-1.80 (2H, m), 1.90-2.20 (2H, m), 2.50-2.70 (1H, m), 3.20-3.50 (1H, m), 6.70-6.90 (3H, m), 6.86 (1H, d, J = 9.6 Hz), 7.50-7.65 (2H, m), 7.70-8.00 (1H, m), 7.93 (3H, br s), 8.01 (1H, s), 8.08 (1H, s), 8.13 (1H, s), 8.22 (1H, t, J = 7.5 Hz), 10.58 (1H, s), 11.32 (1H, s), 12.30 (1H, s).
Melting point: 250-252°C

### Example 278

### N-{4-[3-({[(1SR,2RS)-2-aminocyclopentyl]carbonyl}amino)phenyl]-3-cyano-6-(4-fluoro-2-hydroxyphenyl)pyridin-2-yl}-2-furamide hydrochloride

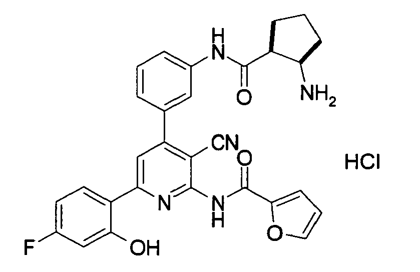

From the compound of Reference Example 135 (120 mg, 0.26 mmol) and Boc-cis-2-aminocyclopentane-1-carboxylic acid (120 mg, 0.52 mmol), the title compound (66 mg, 45%) was given as a yellow powder in substantially the same manner as in Example 78.
¹H-NMR (300MHz, DMSO-d₆) δ : 1.60-2.20 (6H, m), 3.05-3.20 (1H, m), 3.70-3.85 (1H, m), 6.75-6.90 (3H, m), 7.42 (1H, d, J = 8.1 Hz), 7.54 (1H, d, J = 3.0 Hz), 7.58 (1H, t, J = 7.8 Hz), 7.80-7.90 (1H, m), 7.93 (3H, br s), 8.00-8.10 (2H, m), 8.13 (1H, s), 8.23 (1H, dd, J = 8.7, 6.9 Hz), 10.68 (1H, s), 11.33 (1H, s), 12.31 (1H, s).
Melting point: 224-226°C
Element analysis: Calculated (%) for C₂₉H₂₄N₅O₄F·1.0HCl·1.5H₂O: C, 59.13; H, 4.79; N. 11.89. Found (%): C, 59.37; H, 4.76; N, 11.93.

### Example 279

### N-(3-cyano-6-(4-fluoro-2-hydroxyphenyl)-4-{3-[(piperidin-2-ylacetyl)amino]phenyl} pyridin-2-yl)-2-furamide hydrochloride

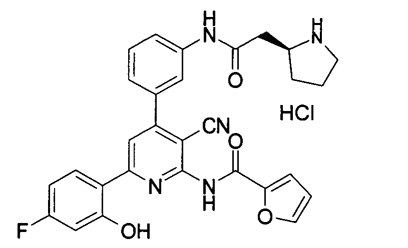

WSC (250 mg, 1.3 mmol) was added to a solution of the compound of Reference Example 134 (500 mg, 1.1 mmol), [(2S)-1-(tert-butoxycarbonyl)pyrrolidine-2-yl]acetic acid (300 mg, 1.3 mmol) and HOBt (180 mg, 1.3 mmol) in DMF (5 mL) at room temperature, and stirred at room temperature for 5 days. The resultant solution was diluted with ethyl acetate, washed twice with water and once with saturated brine. The organic layer was dried over magnesium sulfate and filtered, and then the solvent was removed under reduced pressure. The residue was purified by silica gel column chromatography (eluting solution: hexane to ethyl acetate/hexane = 1/1) and by basic silica gel column chromatography (eluting solution: hexane to ethyl acetate), and concentrated. The residue was dissolved in ethyl acetate (5 mL)-methanol (2 ml). A 4N hydrogen chloride-ethyl acetate solution (5 mL) was added and stirred at room temperature overnight. The solvent was removed under reduced pressure, and the residue was recrystallized from methanol-diethylether to give the title compound (390 mg, 63%) was given as a yellow powder.
¹H-NMR (300MHz, DMSO-d₆) δ : 1.55-1.68 (1H, m), 1.81-1.97 (2H, m), 2.10-2.20 (1H, m), 2.93 (2H, d, J = 6.9 Hz), 3.13-3.21 (2H, m), 3.76-3.85 (1H, m), 6.79-6.89 (3H, m), 7.41 (1H, d, J = 7.8 Hz), 7.54-7.61 (2H, m), 7.81-7.84 (1H, m), 8.04 (1H, br s), 8.08-8.09 (1H, m), 8.15 (1H, s), 8.23 (1H, dd, J = 8.4, 6.6 Hz), 8.94 (1H, br s), 9.18 (1H, br s), 10.64 (1H, s), 11.33 (1H, s), 12.33 (1H, s).
Melting point: 268-269°C
Element analysis: Calculated (%) for C₂₉H₂₄N₅O₄F·1.0HCl·0.25H₂O: C, 61.49; H, 4.54; N, 12.36. Found (%): C, 61.33; H, 4.52; N, 12.41.

### Example 280

### N-{4-[3-({[(1SR,2RS)-2-aminocyclohexyl]amino}carbonyl)phenyl]-3-cyano-6-(4-fluoro-2-hydroxyphenyl)pyridin-2-yl}-2-furamide hydrochloride

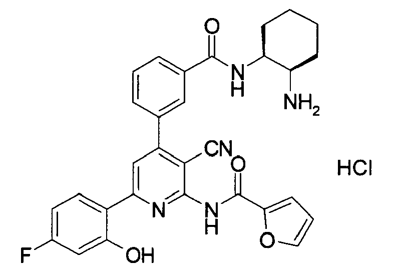

From the compound of Reference Example 135 (200 mg, 0.41 mmol) and 1-N-Boc-1,2-cis-cyclohexyldiamine (176 mg, 0.82 mmol), the title compound (110 mg, 47%) was given as a yellow powder in substantially the same manner as in Example 78.
¹H-NMR (300MHz, DMSO-d₆) δ : 1.30-1.55 (2H, m), 1.60-2.00 (6H, m), 3.47 (1H, s), 4.35 (1H, s), 6.75-6.90 (3H, m), 7.55 (1H, d, J = 3.0 Hz), 7.73 (1H, t, J = 7.8 Hz), 7.91 (1H, d, J = 7.8 Hz), 8.08 (3H, br s), 8.00-8.10 (1H, m), 8.17 (1H, d, J = 7.8 Hz), 8.25-8.40 (3H, m), 8.46 (1H, d, J = 7.2 Hz), 11.32 (1H, s), 12.46 (1H, s).
Melting point: 250-252°C
Element analysis: Calculated (%) for C₃₀H₂₆N₅O₄F·1.0HCl·1.0H₂O: C, 60.66; H, 4.92; N. 11.79. Found (%): C, 60.88; H, 4.88; N, 11.82.

### Example 281

### N-[4-(3-{[(2-aminoethyl)amino]carbonyl}-4-fluorophenyl)-3-cyano-6-(4-fluoro-2-hydroxyphenyl)pyridin-2-yl]-2-furamide hydrochloride

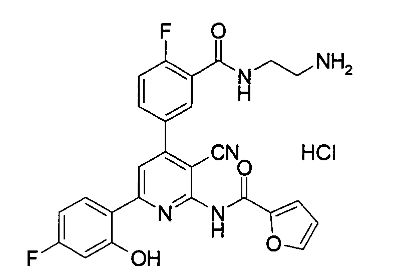

From the compound of Reference Example 217 (150 mg, 0.23 mmol), the title compound (117 mg, 94%) was given as a white crystal in substantially the same manner as in Example 271.
¹H-NMR (300MHz, DMSO-d₆) δ : 3.01 (2H, t, J = 6.0 Hz), 3.56 (2H, q, J = 5.8 Hz), 6.80-6.87 (3H, m), 7.54-7.64 (2H, m), 7.90-8.05 (4H, br), 8.08-8.12 (2H, m), 8.25 (1H, s), 8.31 (1H, t, J = 8.1 Hz), 8.75 (1H, br s), 11.20-11.40 (1H, br), 12.40 (1H, br s).
Melting point: 255-264°C
Element analysis: Calculated (%) for C₂₆H₁₉N₅O₄F₂·1.0HCl·0.4H₂O: C, 57.08; H, 3.83; N, 12.80. Found (%): C, 56.95; H, 3.82; N, 12.79.

### Example 282

### N-[4-(3-amino-4-fluorophenyl)-3-cyano-6-(4-fluoro-2-hydroxyphenyl)pyridin-2-yl]-2-furamide hydrochloride

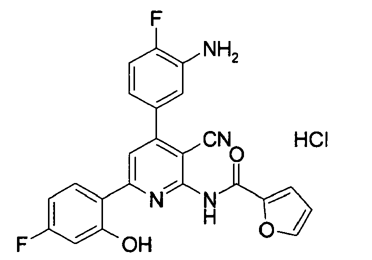

A mixture of the compound of Reference Example 218 (100 mg, 0.17 mmol) and a 4N hydrogen chloride-ethyl acetate solution (0.75 mL) was stirred at room temperature for 16 hours. The precipitated crystals were collected by filtration, and recrystallized from methanol-diethylether to give the title compound (62 mg, 78%) as a yellow crystal.
¹H-NMR (300MHz, DMSO-d₆) δ : 5.50-5.80 (3H, br), 6.79-6.91 (4H, m), 7.13 (1H, d, J = 8.4 Hz), 7.25 (1H, t, J = 9.8 Hz), 7.52 (1H, s), 8.07-8.09 (2H, m), 8.24 (1H, t, J = 7.8 Hz), 11.23 (1H, s), 12.00-12.70 (1H, br).
Melting point: 284-285°C
Element analysis: Calculated (%) for C₂₃H₁₄N₄O₃F₂·1.0HCl·1.5H₂O: C, 57.57; H, 3.78; N, 11.68. Found (%): C, 57.83; H, 3.60; N,11.41.

### Example 283

### N-{4-[3-({[(1S,2R)-2-aminocyclohexyl]carbonyl}amino)phenyl]-3-cyano-6-(4-fluoro-2-hydroxyphenyl)pyridin-2-yl}-2-furamide hydrochloride

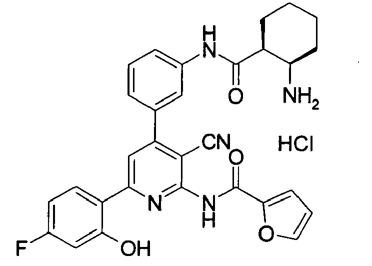

From the compound of Reference Example 135 (240 mg, 0.52 mmol) and (1S,2R)-Boc-2-aminocyclohexane-1-carboxylic acid (256 mg, 1.05 mmol), the title compound (172 mg, 57%) was given as a yellow powder in substantially the same manner as in Example 78.
¹H-NMR (300MHz, DMSO-d₆) δ : 1.30-1.90 (6H, m), 1.90-2.10 (2H, m), 2.96 (1H, s), 3.52 (1H, s), 6.75-6.90 (3H, m), 7.41 (1H, d, J = 7.8 Hz), 7.55 (1H, d, J = 3.9 Hz), 7.59 (1H, d, J = 8.1 Hz), 7.83 (1H, d, J = 9.0 Hz), 7.98 (3H, br s), 8.03 (1H, s), 8.08 (1H, s), 8.14 (1H, s), 8.20-8.30 (1H, m), 10.45-10.60 (1H, m), 11.34 (1H, s), 12.32 (1H, s).
Melting point: 237-239°C
Element analysis: Calculated (%) for C₃₀H₂₆N₅O₄F·1.0HCl·2.0H₂O: C, 58.87; H, 5.11; N. 11.44. Found (%): C, 58.58; H, 4.90; N, 11.46.

### Example 284

### N-{4-[3-({[(1R,2S)-2-aminocyclohexyl]carbonyl}amino)phenyl]-3-cyano-6-(4-fluoro-2-hydroxyphenyl)pyridin-2-yl}-2-furamide hydrochloride

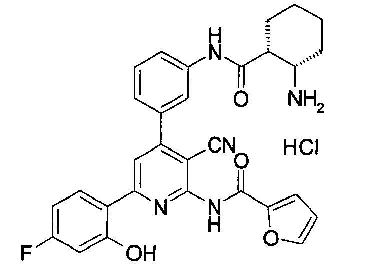

From the compound of Reference Example 135 (240 mg, 0.52 mmol) and (1R, 2S)-Boc-2-aminocyclohexane-1-carboxylic acid (256 mg, 1.05 mmol), the title compound (187 mg, 62%) was given as a yellow powder in substantially the same manner as in Example 78.
¹H-NMR (300MHz, DMSO-d₆) δ: 1.30-1.90 (6H, m), 1.90-2.10 (2H, m), 2.95 (1H, s), 3.52 (1H, s), 6.75-6.90 (3H, m), 7.41 (1H, d, J = 6.9 Hz), 7.50-7.65 (2H, m), 7.70-7.83 (5H, m), 7.95 (3H, br s), 10.45-10.60 (1H, m), 11.33 (1H, br s), 12.32 (1H, s).
Melting point: 220-223°C
Element analysis: Calculated (%) for C₃₀H₂₆N₅O₄F·1.0HCl·2.0H₂O: C, 58.87; H, 5.11; N. 11.44. Found (%): C, 58.67; H, 5.13; N, 11.48.

### Example 285

### N-[3-cyano-4-(3-{[3-(dimethylamino)hexanoyl]amino}phenyl)-6-(4-fluoro-2-hydroxyphenyl)pyridin-2-yl]-2-furamide hydrochloride

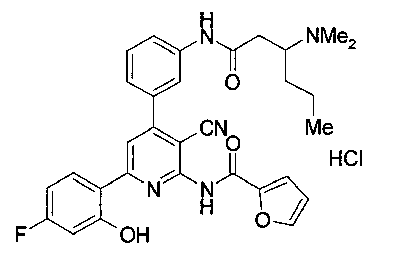

From the compound of Example 265 (50 mg, 0.089 mmol), the title compound (28 mg, 53%) was given as a yellow powder in substantially the same manner as in Example 305.
¹H-NMR (300MHz, DMSO-d₆) δ : 0.80-0.95 (3H, m), 1.20-1.50 (2H, m), 1.50-1.70 (1H, m), 1.70-1.90 (1H, m), 2.65-2.80 (7H, m), 2.90-3.05 (1H, m), 3.60-3.80 (1H, m), 6.75-6.90 (3H, m), 7.43 (1H, d, J = 8.1 Hz), 7.50-7.65 (2H, m), 7.82 (1H, d, J = 8.1 Hz), 8.01 (1H, s), 8.08 (1H, s), 8.14 (1H, s), 8.24 (1H, t, J = 6.9 Hz), 9.68 (1H, br s), 10.67 (1H, s), 11.32 (1H, s), 12.32 (1H, s).
Melting point: 180-182°C
Element analysis: Calculated (%) for C₃₁H₃₀N₅O₄F·1.0HCl·1.5H₂O: C, 60.14; H, 5.54; N. 11.31. Found (%): C, 60.32; H, 5.35; N, 11.38.

### Example 286

### N-{3-cyano-4-[3-({[(1S,2R)-2-(dimethylamino)cyclohexyl]carbonyl}amino)phenyl]-6-(4-fluoro-2-hydroxyphenyl)pyridin-2-yl}-2-furamide hydrochloride

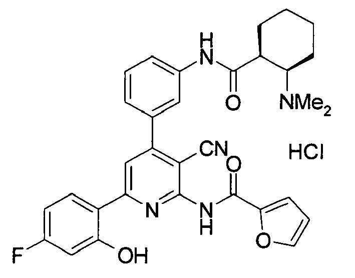

From the compound of Example 283 (80 mg, 0.14 mmol), the title compound (52 mg, 62%) was given as a yellow powder in substantially the same manner as in Example 305.
¹H-NMR (300MHz, DMSO-d₆) δ:1.20-1.80 (6H, m), 2.20-2.40 (2H, m), 2.70-2.85 (6H, m), 3.20-3.50 (2H, m), 6.75-6.90 (3H, m), 7.43 (1H, d, J = 7.8 Hz), 7.54 (1H, d, J = 3.3 Hz), 7.59 (1H, t, J = 7.8 Hz), 7.83 (1H, d, J = 8.1 Hz), 8.04 (1H, s), 8.08 (1H, s), 8.14 (1H, s), 8.23 (1H, t, J = 8.4 Hz), 9.29 (1H, br s), 10.61 (1H, s), 11.32 (1H, s), 12.33 (1H, s).
Melting point: 205-207°C
Element analysis: Calculated (%) for C₃₂H₃₀N₅O₄F·1.0HC1-1.5H₂O: C, 60.90; H, 5.43; N. 11.10. Found (%): C, 61.04; H, 5.23; N, 11.32.

### Example 287

### N-{3-cyano-4-[3-({[(1R,2S)-2-(dimethylamino)cyclohexyl]carbonyl}amino)phenyl]-6-(4-fluoro-2-hydroxyphenyl)pyridin-2-yl}-2-furamide hydrochloride

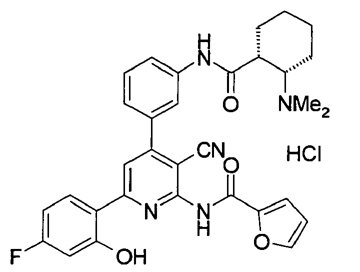

From the compound of Example 284 (80 mg, 0.14 mmol), the title compound (55 mg, 64%) was given as a yellow powder in substantially the same manner as in Example 305.
¹H-NMR (300MHz, DMSO-d₆) δ : 1.25-1.80 (4H, m), 1.80-1.95 (1H, m), 2.00-2.10 (1H, m), 2.10-2.35 (2H, m), 2.76 (3H, d, J = 4.8 Hz), 2.80 (3H, d, J = 4.8 Hz), 3.25-3.45 (2H, m), 6.75-6.90 (3H, m), 7.43 (1H, d, J = 8.1 Hz), 7.54 (1H, d, J = 3.0 Hz), 7.59 (1H, t, J = 7.8 Hz), 7.83 (1H, d, J = 8.1 Hz), 8.07 (1H, s), 8.08 (1H, s), 8.14 (1H, s), 8.20-8.30 (1H, m), 9.41 (1H, s), 10.67 (1H, s), 11.32 (1H, s), 12.33 (1H, s).
Melting point: 217-218°C
Element analysis: Calculated (%) for C₃₂H₃₀N₅O₄F·1.0HCl·1.5H₂O: C, 60.90; H, 5.43; N. 11.10. Found (%): C, 61.06; H, 5.38; N, 11.08.

### Example 288

### N-(2-aminoethyl)-1-[3-cyano-2-(2-furoylamino)-6-(2-hydroxyphenyl)pyridin-4-yl] piperidine-3-carboxamide hydrochloride

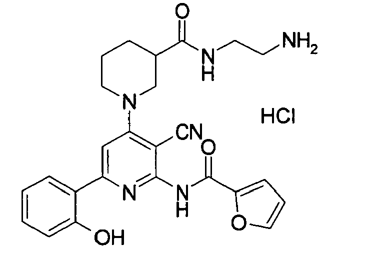

A 4N hydrogen chloride-ethyl acetate solution (1 mL) was added to a solution of the compound of Example 262 (29 mg, 0.050 mmol) in chloroform (2 mL) at room temperature. The reaction solution was stirred at room temperature for 30 minutes, and the solvent was removed under reduced pressure. The residue was dissolved in chloroform, washed with saturated sodium hydrogencarbonate solution, water and then saturated brine, dried over magnesium sulfate, and then concentrated under reduced pressure to give N-(2-aminoethyl)-1-[3-cyano-2-(2-furoylamino)-6-(2-hydroxyphenyl)pyridin-4-yl] piperidine-3-carboxamide (10.3 mg, 43%) as a white powder.
¹H-NMR (300MHz, CDCl₃) δ: 1.56-2.20 (4H, m), 2.55-2.71 (1H, m), 2.75-3.01 (2H. m), 3.11 (1H, t, J = 11.4 Hz), 3.26 (1H, t, J = 12.9 Hz), 3.30-3.50 (5H, m), 3.96 (1H, d, J = 12.9 Hz), 4.15 (1H, d, J = 12.9 Hz), 6.51 (1H, br s), 6.61 (1H, dd, J = 3.6, 1.8 Hz), 6.83 (1H, t, J = 7.8 Hz), 6.95 (1H, s), 7.02 (1H, d, J = 8.1 Hz), 7.27-7.35 (1H, m), 7.39 (1H, d, J = 3.6 Hz), 7.52-7.65 (2H, m), 13.38 (1H, br s).

A 4N hydrogen chloride-ethyl acetate solution (5 µL) was added to a solution of N-(2-aminoethyl)-1-[3-cyano-2-(2-furoylamino)-6-(2-hydroxyphenyl)pyridin-4-yl]piperidine-3-carboxamide (10.3 mg, 0.02 mmol) in chloroform (2 mL) at room temperature, and stirred at room temperature for 5 minutes. Then, the solvent was removed under reduced pressure to give the title compound (10 mg) as a yellow powder.
Melting point: 178-179°C

### Example 289

### N-{4-[3-({[(1SR,2SR)-2-aminocyclohexyl] amino} carbonyl)phenyl] -3 -cyano-6-(4-fluoro-2-hydroxyphenyl)pyridin-2-yl}-2-furamide hydrochloride

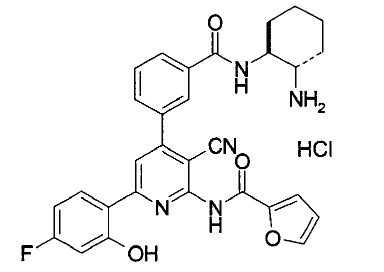

From the compound of Reference Example 135 (200 mg, 0.41 mmol) and 1-N-Boc-1,2-trans-cyclohexyldiamine (176 mg, 0.82 mmol), the title compound (18 mg, 8%) was given as a yellow powder in substantially the same manner as in Example 78.
¹H-NMR (400 MHz, DMSO-d₆) δ:1.20-2.70 (8H, m), 3.00-3.20 (1H, m), 3.90-4.00 (1H, m), 6.75-6.90 (3H, m), 7.54 (1H, s), 7.75 (1H, t, J = 8.0 Hz), 7.91 (1H, d, J = 7.6 Hz), 7.97 (3H, br s), 8.08 (1H, s), 8.15 (1H, d, J = 7.6 Hz), 8.25 (1H, s), 8.31 (1H, t, J = 8.0 Hz), 8.68 (1H, d, J = 8.8 Hz), 11.32 (1H, s), 12.40 (1H, s).
Melting point: 285-288°C
Element analysis: Calculated (%) for C₃₀H₂₆N₅O₄F·1.0HCl·1.5H₂O: C, 59.75; H, 5.01; N. 11.61. Found (%): C, 59.82; H, 4.83; N, 11.61.

### Example 290

### N-[3-cyano-6-(4-fluoro-2-hydroxyphenyl)-4-(3-{[(2S)-pyrrolidin-2-ylacetyl]amino} phenyl)pyridin-2-yl]-2-furamide hydrochloride

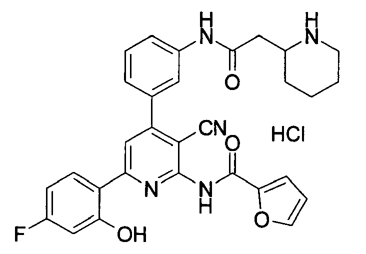

A 4N hydrogen chloride-ethyl acetate solution (5 mL) was added to a solution of the compound of Reference Example 129 (150 mg, 0.22 mmol) in ethyl acetate (5 mL)-methanol (2 ml), and stirred at room temperature for 23 hours. The solvent was removed under reduced pressure, and the residue was recrystallized from methanol-diethylether to give the title compound (97 mg, 77%) (10 mg) as a yellow powder.
¹H-NMR (300MHz, DMSO-d₆) δ : 1.51-1.88 (6H, m), 2.73-2.98 (3H, m), 3.26 (1H, d, J = 12.6 Hz), 6.79-6.88 (3H, m), 7.42 (1H, d, J = 7.8 Hz), 7.54-7.61 (2H, m), 7.82 (1H, d, J = 9.0 Hz), 8.02 (1H, br s), 8.08 (1H, d, J = 0.9 Hz), 8.14 (1H, s), 8.23-8.26 (1H, m), 8.78-8.91 (2H, m), 10.65 (1H, s), 11.32 (1H, s), 12.34 (1H, s).
Melting point: 277-278°C (decomposes
Element analysis: Calculated (%) for C₃₀H₂₆N₅O₄F·1.0HCl·0.5H₂O: C, 61.59; H, 4.82; N, 11.97. Found (%): C, 61.28; H, 4.90; N, 12.05.

### Example 291

### N- {3-[3-cyano-6-(4-fluoro-2-hydroxyphenyl)-2-(2-furoylamino)pyridin-4-yl]phenyl}-D-prolinamide hydrochloride

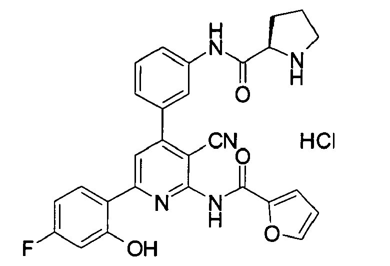

From the compound of Reference Example 222 (190 mg, 0.29 mmol), the title compound (122 mg, 77%) was given as a pale yellow crystal in substantially the same manner as in Example 271.
¹H-NMR (300MHz, DMSO-d₆) δ:1.91-2.07 (3H, m), 2.39-2.51 (1H, br), 3.22-3.29 (2H, m), 4.41 (1H, t, J = 7.2 Hz), 6.79-6.87 (3H, m), 7.47-7.55 (2H, m), 7.63 (1H, t, J = 7.8 Hz), 7.84 (1H, d, J = 8.4 Hz), 8.02 (1H, s), 8.08 (1H, d, J = 0.9 Hz), 8.16 (1H, s), 8.23 (1H, t, J = 7.8 Hz), 9.00-9.60 (2H, br), 11.02 (1H, s), 11.30-11.40 (1H, br), 12.33 (1H, s).
Melting point: 216-220°C
Element analysis: Calculated (%) for C₂₈H₂₂N₅O₄F·1.0HCl·1.0H₂O: C, 59.42; H, 4.45; N, 12.37. Found (%): C, 59.17; H, 4.52; N, 12.29.

### Example 292

### N-(3-{3-cyano-6-(4-fluoro-2-hydroxyphenyl)-2-[(1,3-thiazol-2-ylcarbonyl)amino] pyridin-4-yl}phenyl)-D-prolinamide hydrochloride

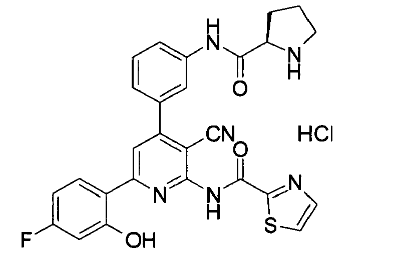

From the compound of Reference Example 223 (210 mg, 0.31 mmol), the title compound (140 mg, 79%) was given as a pale yellow crystal in substantially the same manner as in Example 271.
¹H-NMR (300MHz, DMSO-d₆) δ : 1.91-2.04 (3H, m), 2.42-2.51 (1H, m), 3.25-3.33 (2H, m), 4.42 (1H, t, J = 6.9 Hz), 6.81-6.89 (2H, m), 7.50 (1H, d, J = 7.8 Hz), 7.64 (1H, t, J = 7.8 Hz), 7.85 (1H, d, J = 8.4 Hz), 8.03 (1H, s), 8.18 (1H, s), 8.23-8.31 (3H, m), 9.00-9.70 (2H, br), 11.08 (1H, s), 11.30-11.70 (1H, br), 12.56 (1H, br s).
Melting point: 241-244°C
Element analysis: Calculated (%) for C₂₇H₂₁N₆O₃SF·1.0HCl·1.0H₂O: C, 55.62; H, 4.15; N, 14.41. Found (%): C, 55.38; H, 4.23; N, 14.33.

### Example 293

### N-(3-{3-cyano-6-(4-fluoro-2-hydroxyphenyl)-2-[(isoxazol-5-ylcarbonyl)amino] pyridin-4-yl}phenyl)-D-prolinamide hydrochloride

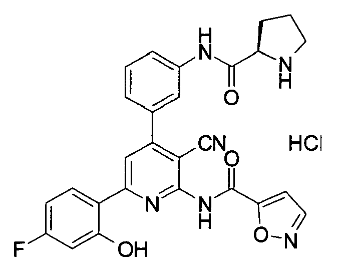

From the compound of Reference Example 224 (170 mg, 0.26 mmol), the title compound (132 mg, 92%) was given as a pale yellow crystal in substantially the same manner as in Example 271.
¹H-NMR (300MHz, DMSO-d₆) δ : 1.91-2.05 (3H, m), 2.41-2.50 (1H, m), 3.24-3.33 (2H, m), 4.40 (1H, t, J = 7.2 Hz), 6.82-6.88 (2H, m), 7.45 (1H, s), 7.49 (1H, d, J = 7.5 Hz), 7.64 (1H, t, J = 7.8 Hz), 7.84 (1H, d, J = 7.8 Hz), 8.02 (1H, s), 8.19-8.24 (2H, m), 8.91 (1H, d, J = 1.5 Hz), 10.98 (1H, s), 11.60-11.90 (1H, br), 12.14 (1H, br s).
Melting point: 250°C or higher (decomposes)
Element analysis: Calculated (%) for C₂₇H₂₁N₆O₄F·1.0HCl·1.5H₂O: C, 56.30; H, 4.37; N, 14.59. Found (%): C, 56.37; H, 4.41; N, 14.46.

### Example 294

### N-(3-{6-(4-chloro-2-hydroxyphenyl)-3-cyano-2-[(1,3-thiazol-2-ylcarbonyl)amino] pyridin-4-yl}phenyl)-D-prolinamide hydrochloride

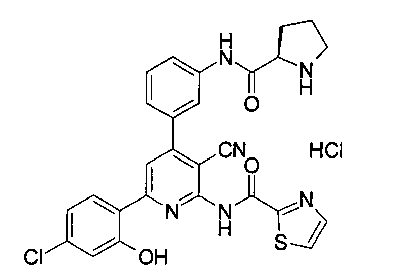

From the compound of Reference Example 226 (160 mg, 0.23 mmol), the title compound (121 mg, 90%) was given as a pale yellow crystal in substantially the same manner as in Example 271.
¹H-NMR (300MHz, DMSO-d₆) δ : 1.91-2.04 (3H, m), 2.44-2.50 (1H, m), 3.20-3.40 (2H, br), 4.40-4.50 (1H, br), 7.03 (1H, dd, J = 8.4, 2.1 Hz), 7.10 (1H, d, J = 2.1 Hz), 7.49 (1H, d, J = 7.8 Hz), 7.63 (1H, t, J = 7.8 Hz), 7.86 (1H, d, J = 8.4 Hz), 8.04 (1H, s), 8.18-8.24 (3H, m), 8.30 (1H, s), 8.70-8.85 (1H, br), 9.80-9.95 (1H, br), 11.14 (1H, s), 11.45 (1H, s), 12.43 (1H, s).
Melting point: 222-228°C
Element analysis: Calculated (%) for C₂₇H₂₁N₆O₃SCl·1.0HCl·1.5H₂O: C, 54.09; H, 4.04; N, 14.02. Found (%): C, 53.97; H, 4.08; N, 13.99.

### Example 295

### N-(3-{6-(4-chloro-2-hydroxyphenyl)-3-cyano-2-[(isoxazol-5-ylcarbonyl)amino] pyridin-4-yl}phenyl)-D-prolinamide hydrochloride

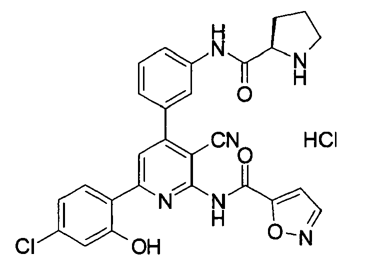

From the compound of Reference Example 227 (170 mg, 0.25 mmol), the title compound (104 mg, 74%) was given as a pale yellow crystal in substantially the same manner as in Example 271.
¹H-NMR (300MHz, DMSO-d₆) δ:1.91-2.05 (3H, m), 2.40-2.50 (1H, m), 3.20-3.35 (2H, br), 4.43 (1H, br s), 7.05 (1H, dd, J = 8.4, 1.2 Hz), 7.14 (1H, d, J = 1.5 Hz), 7.48 (2H, d, J = 6.9 Hz), 7.63 (1H, t, J = 7.8 Hz), 7.85 (1H, d, J = 8.1 Hz), 8.04 (1H, s), 8.16 (1H, d, J = 8.4 Hz), 8.26 (1H, s), 8.65-8.80 (1H, br), 8.92 (1H, t, J = 0.9 Hz), 9.70-9.85 (1H, br), 11.09 (1H, s), 11.94 (1H, s), 12.04 (1H, s).
Melting point: 250°C or higher (decomposes)
Element analysis: Calculated (%) for C₂₇H₂₁N₆O₄Cl·1.0HCl·1.2H₂O: C, 55.24; H, 4.19; N, 14.32. Found (%): C, 55.27; H, 4.28; N, 14.27.

### Example 296

### N-{3-[6-(4-chloro-2-hydroxyphenyl)-3-cyano-2-(2-furoylamino)pyridin-4-yl]phenyl}-D-prolinamide hydrochloride

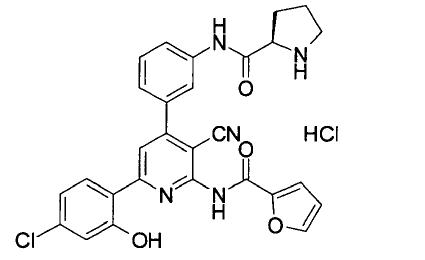

From the compound of Reference Example 228 (140 mg, 0.21 mmol), the title compound (75 mg, 63%) was given as a pale yellow crystal in substantially the same manner as in Example 271.
¹H-NMR (300MHz, DMSO-d₆) δ : 1.91-2.04 (3H, m), 2.40-2.50 (1H, m), 3.20-3.35 (2H, br), 4.35-4.49 (1H, br), 6.80 (1H, q, J = 1.8 Hz), 7.04 (1H, dd, J = 8.4, 2.1 Hz), 7.12 (1H, d, J = 2.1 Hz), 7.47 (1H, d, J = 7.8 Hz), 7.55 (1H, d, J = 3.6 Hz), 7.63 (1H, t, J = 7.8 Hz), 7.85 (1H, d, J = 8.7 Hz), 8.03 (1H, s), 8.07 (1H, d, J = 0.9 Hz), 8.17 (2H, d, J = 8.1 Hz), 8.70-8.85 (1H, br), 9.70-9.85 (1H, br), 11.09 (1H, s), 11.34 (1H, s), 12.18 (1H, s).
Melting point: 208-213°C
Element analysis: Calculated (%) for C₂₈H₂₂N₅O₄Cl·1.0HCl·1.0H₂O: C, 56.86; H, 4.43; N, 11.84. Found (%): C, 56.58; H, 4.50; N, 11.85.

### Example 297

### N-[4-[3-(β-alanylamino)phenyl]-3-cyano-6-(2-hydroxyphenyl)-5-methylpyridin-2-yl]-2-furamide hydrochloride

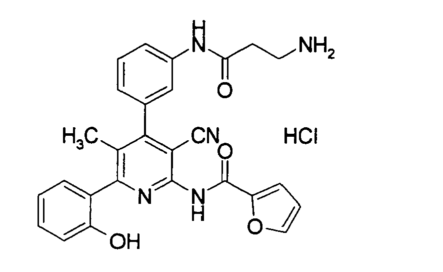

A 4N hydrogen chloride-ethyl acetate solution (2.5 mL) was added to a solution of the compound of Reference Example 233 (250 mg, 0.40 mmol) in ethyl acetate (10 mL), and stirred at room temperature for 3 hours. The precipitate was collected by filtration, and recrystallized from methanol-diethylether to give the title compound (160 mg, 77%) as a pale yellow amorphous solid.
¹H-NMR (300MHz, DMSO-d₆) δ : 1.95 (3H, s), 2.78 (2H, t, J = 3.3 Hz), 3.09 (2H, q, J = 3.3 Hz), 6.73 (1H, q, J = 1.8 Hz), 6.90-7.02 (2H, m), 7.10 (1H, d, J = 7.5 Hz), 7.26-7.32 (2H, m), 7.51-7.56 (2H, m), 7.71 (1H, d, J = 8.4 Hz), 7.78 (1H, s), 7.80-8.00 (4H, m), 9.89 (1H, br s), 10.56 (1H, s), 11.22 (1H, s).
Element analysis: Calculated (%) for C₂₇H₂₃N₅O₄·1.0HCl·1.4H₂O: C, 59.70; H, 4.97; N, 12.89. Found (%): C, 59.86; H, 5.10; N, 12.55.

### Example 298

### (4R)-N- {3-[3-cyano-6-(4-fluoro-2-hydroxyphenyl)-2-(2-furoylamino)pyridin-4-yl] phenyl}-4-hydroxy-L-prolinamide hydrochloride

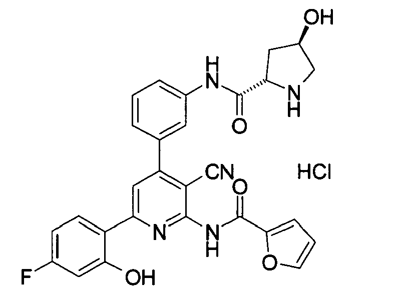

From the compound of Reference Example 135 (200 mg, 0.44 mmol) and (4R)-1-(tert-butoxycarbonyl)-4-hydroxy-L-proline (203 mg, 0.88 mmol), the title compound (148 mg, 60%) was given as a yellow powder in substantially the same manner as in Example 78.
¹H-NMR (300MHz, DMSO-d₆) δ:2.00-2.10 (1H, m), 2.40-2.60 (1H, m), 3.30-3.50 (2H, m), 4.51 (1H, s), 4.55 (1H, br s), 5.61 (1H, br s), 6.75-6.90 (3H, m), 7.49 (1H, d, J = 8.1 Hz), 7.54 (1H, d, J = 3.3 Hz), 7.64 (1H, t, J = 8.1 Hz), 7.84 (1H, d, J = 8.1 Hz), 8.02 (1H, s), 8.05-8.10 (1H, m), 8.16 (1H, s), 8.20-8.30 (1H, m), 8.87 (1H, s), 9.87 (1H, br s), 10.98 (1H, s), 11.33 (1H, s), 12.33 (1H, s).
Melting point: 260-262°C
Element analysis: Calculated (%) for C₂₈H₂₂N₅O₅F·1.0HCl: C, 57.79; H, 4.33; N. 12.03. Found (%): C, 57.67; H, 4.31; N, 12.11.

### Example 299

### (4R)-N-{3-[3-cyano-6-(4-fluoro-2-hydroxyphenyl)-2-(2-furoylamino)pyridin-4-yl] phenyl}-4-hydroxy-1-methyl-L-prolinamide hydrochloride

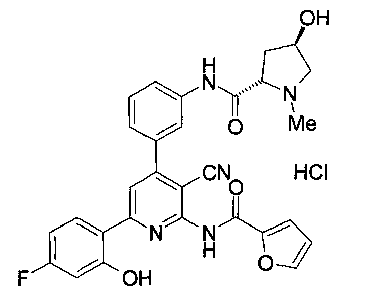

From the compound of Example 298 (80 mg, 0.14 mmol), the title compound (62 mg, 76%) was given as a yellow powder in substantially the same manner as in Example 305.
¹H-NMR (300MHz, DMSO-d₆) δ : 2.10-2.30 (1H, m), 2.40-2.60 (1H, m), 2.97 (3H, s), 3.10-3.20 (1H, m), 3.80-3.90 (1H, m), 4.40-4.60 (2H, m), 5.78 (1H, s), 6.75-6.85 (2H, m), 6.86 (1H, d, J = 9.6 Hz), 7.50 (1H, d, J = 8.1 Hz), 7.54 (1H, d, J = 3.6 Hz), 7.64 (1H, t, J = 7.8 Hz), 7.86 (1H, d, J = 8.1 Hz), 8.02 (1H, s), 8.05-8.10 (1H, m), 8.16 (1H, s), 8.20-8.30 (1H, m), 9.96 (1H, s), 11.04 (1H, s), 11.35 (1H, s), 12.31 (1H, s).
Melting point: 215-217°C
Element analysis: Calculated (%) for C₂₉H₂₄N₅O₅F·1.0HCl·1.5H₂O: C, 57.57; H, 4.66; N. 11.58. Found (%): C, 57.91; H, 4.67; N, 11.15.

### Example 300

### N-[3-[(dimethylamino)methyl]-4-{3-[(N,N-dimethylglycyl)amino]phenyl}-6-(4-fluoro-2-hydroxyphenyl)pyridin-2-yl]-2-furamide dihydrochloride

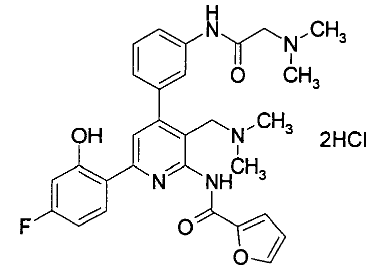

From the compound of Reference Example 260 (100 mg, 0.19 mmol), N-{4-(3-aminophenyl)-3-[(dimethylamino)methyl]-6-[4-fluoro-2-(methoxymethoxy) phenyl]pyridin-2-yl}-2-furamide (70 mg, 83%) was given as a pale brown amorphous solid in substantially the same manner as in Reference Example 4.

WSCD (60 mg, 0.31 mmol) was added to a solution of the resultant N-{4-(3-aminophenyl)-3-[(dimethylamino)methyl]-6-[4-fluoro-2-(methoxymethoxy)phenyl]pyridin-2-yl}-2-furamide (70 mg, 0.16 mmol), N,N-dimethylglycine (32 mg, 0.31 mmol) and HOBt (42 mg, 0.31 mmol) in DMF (5 mL) with ice-cooling, and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure. The residue was purified by silica gel chromatography (eluting solution: ethyl acetate to ethyl acetate/methanol = 20/1) to give N-[3-[(dimethylamino)methyl]-4-{3-[(N,N-dimethylglycyl)amino]phenyl}-6-(4-fluoro-2-hydroxyphenyl)pyridin-2-yl]-2-furamide (50 mg) as a colorless amorphous solid.

A 4N hydrogen chloride-ethyl acetate solution (1 mL) was added to a of the resultant N-[3-[(dimethylamino)methyl]-4-{3-[(N,N-dimethylglycyl)amino]phenyl}-6-(4-fluoro-2-hydroxyphenyl)pyridin-2-yl]-2-furamide (50 mg) in ethyl acetate (5 mL), and concentrated under reduced pressure. The residue was recrystallized from methanol-diethylether to give the title compound (41 mg, 42%) as a white crystal.
¹H-NMR (300MHz, DMSO-d₆) δ : 2.33 (6H, s), 2.41 (6H, s), 3.11 (2H, s), 3.57 (2H, s), 6.49-6.57 (2H, m), 6.75 (1H, dd, J = 10.8, 2.7 Hz), 6.99 (1H, d, J = 7.5 Hz), 7.27-7.33 (2H, m), 7.41-7.49 (2H, m), 7.62-7.70 (3H, m), 9.27 (1H, s), 13.00 (1H, s), 14.24 (1H, s).
Melting point: 186-194°C
Element analysis: Calculated (%) for C₂₉H₃₀N₅O₄F·2.0HCl·1.1H₂O: C, 55.79; H, 5.52; N, 11.21. Found (%): C, 55.79; H, 5.82; N, 10.80.

### Example 301

### N-(3-cyano-6-(4-fluoro-2-hydroxyphenyl)-4-{3-[(O-methyl-L-seryl)amino]phenyl} pyridin-2-yl)-2-furamide hydrochloride

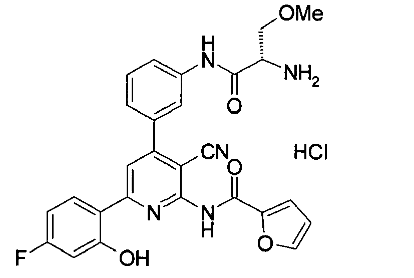

From the compound of Reference Example 234 (260 mg, 0.39 mmol), the title compound (156 mg, 72%) was given as a pale yellow crystal in substantially the same manner as in Example 271.
¹H-NMR (300MHz, DMSO-d₆) δ : 3.33 (3H, s), 3.77-3.87 (2H, m), 4.26 (1H, t, J = 4.5 Hz), 6.79-6.87 (3H, m), 7.47 (1H, d, J = 7.5 Hz), 7.54 (1H, d, J = 3.6 Hz), 7.63 (1H, t, J = 7.8 Hz), 7.86 (1H, d, J = 8.4 Hz), 8.00 (1H, s), 8.08 (1H, d, J = 0.6 Hz), 8.16 (1H, s), 8.23 (1H, t, J = 7.8 Hz), 8.25-8.50 (3H, br), 11.04 (1H, br s), 11.10-11.50 (1H, br), 12.33 (1H, s).
Melting point: 224-230°C
Element analysis: Calculated (%) for C₂₇H₂₂N₅O₅F·1.0HCl·1.5H₂O: C, 56.01; H, 4.53; N, 12.10. Found (%): C, 56.00; H, 4.52; N, 12.18.

### Example 302

### N-[4-{3-[(3-amino-L-alanyl)amino]phenyl}-3-cyano-6-(4-fluoro-2-hydroxyphenyl) pyridin-2-yl]-2-furamide dihydrochloride

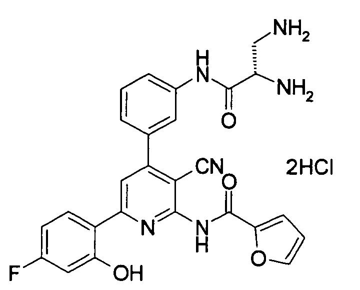

From the compound of Reference Example 235 (230 mg, 0.31 mmol), the title compound (167 mg, 94%) was given as a pale yellow crystal in substantially the same manner as in Example 271.
¹H-NMR (300MHz, DMSO-d₆) δ : 3.35-3.42 (2H, m), 4.46 (1H, br s), 6.80-6.88 (3H, m), 7.49 (1H, d, J = 7.8 Hz), 7.55 (1H, d, J = 3.3 Hz), 7.65 (1H, t, J = 7.8 Hz), 7.88 (1H, d, J = 8.1 Hz), 8.07 (2H, d, J = 8.1 Hz), 8.14 (1H, s), 8.22 (1H, t, J = 7.8 Hz), 8.30-8.70 (6H, br), 11.34 (2H, br s), 12.29 (1H, s).
Melting point: 231-240°C
Element analysis: Calculated (%) for C₂₆H₂₁N₆O₄F.2.0HCl·1.5H₂O: C, 52.01; H, 4.36; N, 14.00. Found (%): C, 52.03; H, 4.40; N, 14.03.

### Example 303

### N-[3-cyano-6-(4-fluoro-2-hydroxyphenyl)-4-(3-piperazin-1-ylphenyl)pyridin-2-yl]-2-furamide hydrochloride

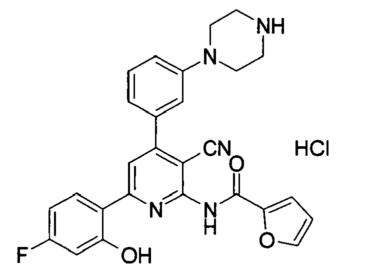

From the compound of Reference Example 238 (300 mg, 0.48 mmol), the title compound (194 mg, 73%) was given as a pale yellow crystal in substantially the same manner as in Example 271.
¹H-NMR (300MHz, DMSO-d₆) δ: 3.26 (4H, s), 3.50 (4H, s), 6.79-6.87 (3H, m), 7.22 (2H, t, J = 9.0 Hz), 7.30 (1H, s), 7.48-7.54 (2H, m), 8.08 (1H, s), 8.16 (1H, s), 8.25 (1H, t, J = 8.1 Hz), 8.80-9.20 (2H, br), 11.10-11.50 (1H, br), 12.43 (1H, br s).
Melting point: 266-273°C
Element analysis: Calculated (%) for C₂₇H₂₂N₅O₃F·1.0HCl·1.0H₂O: C, 59.29; H, 4.79; N, 12.80; Cl, 6.48. Found (%): C, 59.61; H, 4.97; N, 12.76; Cl, 6.25.

### Example 304

### N-[3-cyano-4-{3-[[2-(diethylamino)ethyl](ethyl)amino]phenyl}-6-(4-fluoro-2-hydroxyphenyl)pyridin-2-yl]-2-furamide dihydrochloride

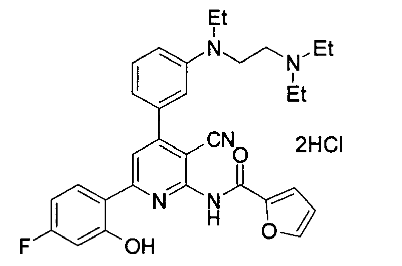

Sodium cyanoborate (38 mg, 0.60 mmol) was added to a solution of the compound of Example 340 (90 mg, 0.15 mmol) in methanol (10 mL), a 90% acetaldehyde (44 mg, 0.92 mmol) and acetic acid (0.1 mL), and stirred at room temperature for 24 hours. The reaction mixture was diluted with saturated aqueous sodium hydrogencarbonate, and extracted with ethyl acetate. The extract was washed with saturated brine, dried over magnesium sulfate, and then concentrated under reduced pressure to give
N-[3-cyano-4-{3-[[2-(diethylamino)ethyl](ethyl)amino]phenyl}-6-(4-fluoro-2-hydroxyphenyl)pyridin-2-yl]-2-furamide.

A 4N hydrogen chloride-ethyl acetate solution (0.5 mL) was added to a solution of the entire amount of the resultant N-[3-cyano-4-{3-[[2-(diethylamino) ethyl](ethyl)amino]phenyl}-6-(4-fluoro-2-hydroxyphenyl)pyridin-2-yl]-2-furamide in ethyl acetate (5 mL), and concentrated under reduced pressure. The residue was recrystallized from methanol-diethylether to give the title compound (84 mg, 91 %) as a pale yellow crystal.
¹H-NMR (300MHz, DMSO-d₆) δ : 1.11-1.26 (9H, m), 3.17-3.30 (6H, m), 3.43-3.50 (2H, m), 3.80-3.85 (2H, m), 6.79-6.87 (3H, m), 7.00-7.05 (3H, m), 7.42 (1H, t, J = 7.5 Hz), 7.53-7.54 (1H, m), 8.07 (1H, s), 8.16 (1H, s), 8.26 (1H, t, J = 7.8 Hz), 10.48 (1H, br s), 11.25 (1H, s), 12.00-12.70 (1H, br).
Melting point: 138-144°C
Element analysis: Calculated (%) for C₃₁H₃₂N₅O₃F·2.0HCl·1.0H₂O: C, 58.86; H, 5.74; N, 11.07. Found (%): C, 59.19; H, 5.44; N, 11.04.

### Example 305

### N-[3-cyano-4-(3-{[3-(dimethylamino)-N,N-dimethyl-L-alanyl]amino} phenyl)-6-(4-fluoro-2-hydroxyphenyl)pyridin-2-yl]-2-furamide dihydrochloride

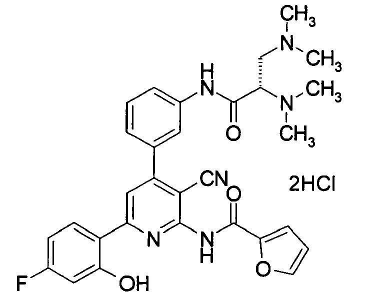

Sodium cyanoborate (41 mg, 0.68 mmol) was added to a solution of the compound of Example 302 (100 mg, 0.17 mmol) in methanol (10 mL), a 37% aqueous solution of formaldehyde (55 µL, 0.68 mmol) and acetic acid (0.1 mL), and stirred at room temperature for 18 hours. The reaction mixture was diluted with saturated aqueous sodium hydrogencarbonate, and extracted with ethyl acetate. The extract was washed with saturated brine, dried over magnesium sulfate, and then concentrated under reduced pressure to give N-[3-cyano-4-(3-{[3-(dimethylamino)-N,N-dimethyl-L-alanyl]amino}phenyl)-6-(4-fluoro-2-hydroxyphenyl)pyridin-2-yl]-2-furamide.

A 4N hydrogen chloride-ethyl acetate solution (0.5 mL) was added to a solution of the entire amount of the resultant N-[3-cyano-4-(3-{[3-(dimethylamino)-N,N-dimethyl-L-alanyl] amino }phenyl)-6-(4-fluoro-2-hydroxyphenyl) pyridin-2-yl]-2-furamide in ethyl acetate (5 mL), and concentrated under reduced pressure. The residue was recrystallized from methanol-diethylether to give the title compound (96 mg, 90%) as a white crystal.
¹H-NMR (300MHz, DMSO-d₆) δ : 2.75 (6H, s), 2.89 (6H, s), 3.60-3.80 (2H, m), 4.40-4.70 (1H, br), 6.79-6.89 (3H, m), 7.49-7.55 (2H, m), 7.64 (1H, t, J = 7.8 Hz), 7.88 (1H, d, J = 8.1 Hz), 8.08 (2H, t, J = 0.9 Hz), 8.15 (1H, s), 8.22 (1H, dd, J = 8.7, 7.2 Hz), 11.33 (1H, s), 12.32 (1H, br s).
Melting point: 195-200°C
Element analysis: Calculated (%) for C₃₀H₂₉N₆O₄F·2.0HCl·0.5H₂O: C, 56.43; H, 5.05; N, 13.16. Found (%): C, 56.32; H, 5.35; N, 13.08.

### Example 306

### N-[3-cyano-4-{3-[(N,N-diethyl-O-methyl-L-seryl)amino]phenyl}-6-(4-fluoro-2-hydroxyphenyl)pyridin-2-yl]-2-furamide hydrochloride

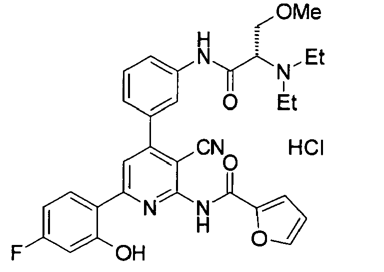

From the compound of Example 301 (100 mg, 0.18 mmol) and 97% acetaldehyde (27 mg, 0.54 mmol), the title compound (115 mg, 100%) was given as a white crystal in substantially the same manner as in Example 305.
¹H-NMR (300MHz, DMSO-d₆) δ : 1.25-1.29 (6H, m), 3.19-3.33 (4H, m), 3.35 (3H, s), 3.93-4.07 (2H, m), 4.50 (1H, br s), 6.79-6.88 (3H, m), 7.49-7.55 (2H, m), 7.64 (1H, t, J = 7.8 Hz), 7.87 (1H, d, J = 8.7 Hz), 8.02 (1H, s), 8.08 (1H, t, J = 0.9 Hz), 8.16 (1H, s), 8.23 (1H, t, J = 7.8 Hz), 9.99 (1H, br s), 11.35 (2H, s), 12.3 (1H, s).
Melting point: 192-196°C
Element analysis: Calculated (%) for C₃₁H₃₀N₅O₅F·1.0HCl·0.5H₂O: C, 60.34; H, 5.23; N, 11.35. Found (%): C, 60.07; H, 5.15; N, 11.29.

### Example 307

### N- [4-(3- {[2-(acetylamino)ethyl]amino}phenyl)-3 -cyano-6-(4-fluoro-2-hydroxyphenyl) pyridin-2-yl]-2-furamide

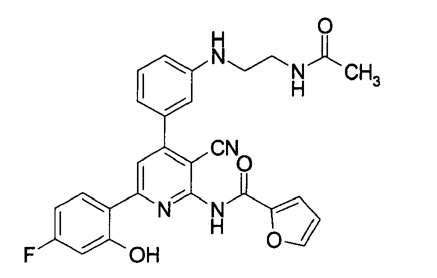

A 4N aqueous sodium hydroxide solution (1 mL) was added to a solution of the compound of Reference Example 246 (60 mg, 0.094 mmol) in methanol (2 mL)-THF (2 mL), and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure. The resultant crystal was washed with ethyl acetate and then dried to give the title compound (35 mg, 75%) as a white crystal.
¹H-NMR (300MHz, DMSO-d₆) δ : 1.82 (3H, s), 3.15-3.19 (2H, m), 3.22-3.28 (2H, m), 6.04 (1H, t, J = 5.4 Hz), 6.78-6.88 (6H, m), 7.30 (1H, t, J = 7.8 Hz), 7.51 (1H, d, J = 3.3 Hz), 7.99 (1H, t, J = 5.4 Hz), 8.07-8.10 (2H, m), 8.26 (1H, dd, J = 9.6, 6.9 Hz), 11.19 (1H, br s), 12.52 (1H, br s).
Melting point: 158-160°C
Element analysis: Calculated (%) for C₂₇H₂₂N₅O₄F·0.5H₂O: C, 63.78; H, 4.56; N, 13.77. Found (%): C, 63.86; H, 4.68; N, 13.32.

### Example 308

### N-[3-cyano-4-(3-{[3-(ethylamino)pentanoyl]amino}phenyl)-6-(4-fluoro-2-hydroxyphenyl)pyridin-2-yl]-2-furamide hydrochloride

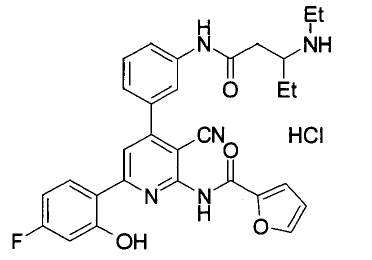

Sodium cyanoborate (38 mg, 0.60 mmol) was added to a solution of the compound of Example 264 (150 mg, 0.27 mmol) in methanol (10 mL), 97% acetaldehyde (26 mg, 0.60 mmol) and acetic acid (0.1 mL), and stirred at room temperature for 4 hours. The reaction mixture was diluted with saturated aqueous sodium hydrogencarbonate, and extracted with ethyl acetate. The extract was washed with saturated brine, dried over magnesium sulfate, and then concentrated under reduced pressure to give N-[3-cyano-4-(3-{ [3-(ethylamino)pentanoyl]amino}phenyl)-6-(4-fluoro-2-hydroxyphenyl)pyridin-2-yl]-2-furamide.

A 4N hydrogen chloride-ethyl acetate solution (0.5 mL) was added to a solution of the entire amount of the resultant N-[3-cyano-4-(3-{[3-(ethylamino) pentanoyl]amino}phenyl)-6-(4-fluoro-2-hydroxyphenyl)pyridin-2-yl]-2-furamide in ethyl acetate (5 mL), and concentrated under reduced pressure. The residue was recrystallized from methanol-diethylether to give the title compound (137 mg, 88%) as a pale yellow crystal.
¹H-NMR (300MHz, DMSO-d₆) δ : 0.96 (3H, t, J = 7.5 Hz), 1.23 (3H, t, J = 7.2 Hz), 1.61-1.84 (2H, m), 2.80-2.95 (2H, m), 3.02 (2H, q, J = 7.2 Hz), 3.48 (1H, br s), 6.79-6.88 (3H, m), 7.42 (1H, d, J = 7.8 Hz), 7.53-7.61 (2H, m), 7.83 (1H, d, J = 9.0 Hz), 8.01 (1H, d, J = 1.5 Hz), 8.07 (1H, t, J = 0.9 Hz), 8.21 (1H, s), 8.26 (1H, t, J = 7.8 Hz), 8.50-8.85 (2H, br), 10.70 (1H, s), 11.25-11.40 (1H, br), 12.34 (1H, br s).
Melting point: 233-238°C
Element analysis: Calculated (%) for C₃₀H₂₈N₅O₄F·1.0HCl·0.5H₂O: C, 61.38; H, 5.15; N, 11.93. Found (%): C, 61.21; H, 5.18; N, 11.92.

### Example 309

### N-[3-cyano-4-[3-({[2-(dimethylamino)ethyl]amino}carbonyl)phenyl]-6-(4-fluoro-2-hydroxyphenyl)pyridin-2-yl]-2-furamide hydrochloride

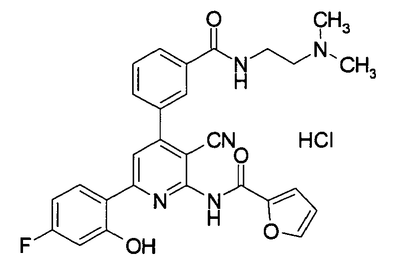

From the compound of Reference Example 247 (80 mg, 0.14 mmol), the title compound (60 mg, 76%) was given as a white crystal in substantially the same manner as in Example 271.
¹H-NMR (300MHz, DMSO-d₆) δ : 2.84 (6H, s), 3.26-3.33 (2H, m), 3.65-3.69 (2H, m), 6.80-6.87 (3H, m), 7.54 (1H, d, J = 3 .6 Hz), 7.76 (1H, t, J = 7.8 Hz), 7.93 (1H, d, J = 7.8 Hz), 8.08-8.14 (2H, m), 8.26-8.35 (3H, m), 9.03 (1H, t, J = 5.4 Hz), 9.89 (1H, br s), 11.32 (1H, s), 12.43 (1H, s).
Melting point: 200-204°C
Element analysis: Calculated (%) for C₂₈H₂₄N₅O₄F·1.0HCl·1.5H₂O: C, 58.28; H, 4.89; N, 12.14. Found (%): C, 58.49; H, 4.69; N, 12.12.

### Example 310

### N-[3-cyano-4-[3-({[2-(diethylamino)ethyl]amino}carbonyl)phenyl]-6-(4-fluoro-2-hydroxyphenyl)pyridin-2-yl]-2-furamide hydrochloride

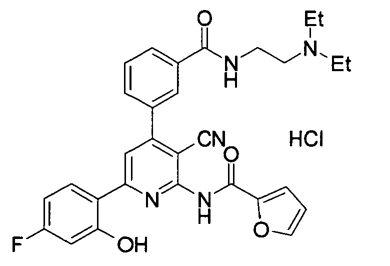

From the compound of Reference Example 248 (90 mg, 0.15 mmol), the title compound (61 mg, 70%) was given as a white crystal in substantially the same manner as in Example 271.
¹H-NMR (300MHz, DMSO-d₆) δ: 1.24 (6H, t, J = 7.2 Hz), 3.19-3.30 (6H, m), 3.64-3.75 (2H, m), 6.80-6.87 (3H, m), 7.54 (1H, d, J = 3.0 Hz), 7.76 (1H, t, J = 7.5 Hz), 7.93 (1H, d, J = 8.1 Hz), 8.08-8.14 (2H, m), 8.26-8.35 (3H, m), 9.12 (1H, br s), 9.96 (1H, br s), 11.32 (1H, s), 12.44 (1H, s).
Melting point: 200-204°C
Element analysis: Calculated (%) for C₃₀H₂₈N₅O₄F·1.0HCl·0.5H₂O: C, 61.38; H, 5.15; N, 11.93. Found (%): C, 61.28; H, 5.04; N, 12.02.

### Example 311

### tert-butyl 4-{3-[3-cyano-6-(4-fluoro-2-hydroxyphenyl)-2-(2-furoylamino)pyridin-4-yl] benzyl}piperazine-1-carboxylate hydrochloride

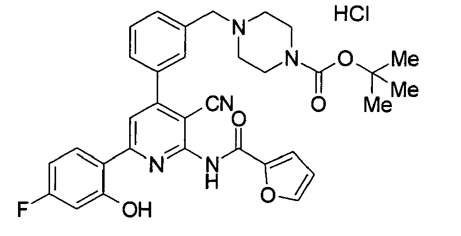

A 4N hydrogen chloride-ethyl acetate solution (1 mL) was added to a solution of the compound of Reference Example 251 (40 mg, 0.062 mmol) in ethyl acetate (5 mL), and stirred at room temperature for 6 hours. The precipitated crystals were collected by filtration, and recrystallized from methanol-diethylether to give the title compound (13 mg, 35%) as a pale yellow crystal.
¹H-NMR (300MHz, DMSO-d₆) δ : 1.41 (9H, s), 3.00-3.40 (6H, m), 4.03 (2H, d, J = 13.8 Hz), 4.48 (2H, s), 6.80-6.87 (3H, m), 7.54 (1H, d, J = 3.6 Hz), 7.74-7.79 (2H, m), 7.87 (1H, d, J = 7.5 Hz), 7.99 (1H, s), 8.08 (1H, d, J = 0.9 Hz), 8.30-8.35 (2H, m), 10.80-11.00 (1H, br), 11.34 (1H, s), 12.45 (1H, s).
Melting point: 179-183°C

### Example 312

### N-(3-cyano-6-(4-fluoro-2-hydroxyphenyl)-4-{3-[(N-methylglycyl)amino]phenyl} pyridin-2-yl)-2-furamide hydrochloride

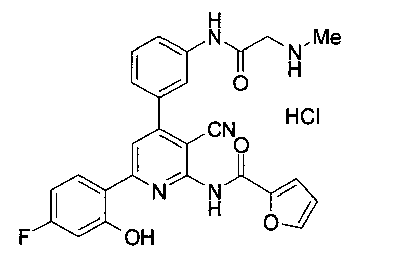

A 4N hydrogen chloride-ethyl acetate solution (5 mL) was added to a solution of the compound of Reference Example 252 (500 mg, 0.79 mmol) in ethyl acetate (5 mL)-methanol (2 mL), and stirred at room temperature overnight. The solvent was removed under reduced pressure, and the residue was recrystallized from methanol to give the title compound (380 mg, 92%) as a pale yellow powder.
¹H-NMR (300MHz, DMSO-d₆) δ : 2.64 (3H, s), 4.00 (2H, s), 6.79-6.89 (3H, m), 7.47 (1H, d, J = 8.1 Hz), 7.55 (1H, dd, J = 3.6, 0.6 Hz), 7.62 (1H, t, J = 7.8 Hz), 7.83 (1H, d, J = 8.1 Hz), 8.02 (1H, s), 8.08 (1H, dd, J = 1.8, 0.6 Hz), 8.16 (1H, s), 8.24 (1H, dd, J = 8.7, 6.9 Hz), 9.03 (1H, br s), 11.04 (1H, s), 11.33 (1H, s), 12.34 (1H, s).
Melting point: 294-296°C (decomposes)
Element analysis: Calculated (%) for C₂₆H₂₀N₅O₄F·1.0HCl: C, 59.72; H, 3.86; N, 13.34. Found (%): C, 59.83; H, 4.06; N, 13.42.

### Example 313

### N-(3-cyano-6-(4-fluoro-2-hydroxyphenyl)-4-{3-[(N-methyl-β-alanyl)amino]phenyl } pyridin-2-yl)-2-furamide hydrochloride

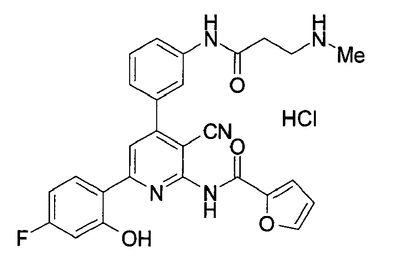

A 4N hydrogen chloride-ethyl acetate solution (5 mL) was added to a solution of the compound of Reference Example 253 (500 mg, 0.78 mmol) in ethyl acetate (5 mL)-methanol (2 mL), and stirred at room temperature overnight. The solvent was removed under reduced pressure, and the residue was recrystallized from methanol to give the title compound (380 mg, 91%) as a pale yellow powder.
¹H-NMR (300MHz, DMSO-d₆) δ : 2.58 (3H, t, J = 5.4 Hz), 2.86 (2H, t, J = 6.9 Hz), 3.16-3.22 (2H, m), 6.79-6.88 (3H, m), 7.41 (1H, d, J = 7.8 Hz), 7.53-7.60 (2H, m), 7.83 (1H, d, J = 8.1 Hz), 8.01 (1H, s), 8.08 (1H, dd, J = 1.5, 0.6 Hz), 8.14 (1H, s), 8.23 (1H, dd, J = 8.1, 6.6 Hz), 8.74 (2H, br s), 10.61 (1H, s), 11.31 (1H, s), 12.33 (1H, s).
Melting point: 253-255°C (decomposes)
Element analysis: Calculated (%) for C₂₇H₂₂N₅O₄F·1.0HCl·1.0H₂O: C, 58.54; H, 4.55; N, 12.64. Found (%): C, 58.80; H, 4.71; N, 12.70.

### Example 314

### N-[3-cyano-6-(4-fluoro-2-hydroxyphenyl)-4-(3-{[(2R)-pyrrolidin-2-ylacetyl]amino} phenyl)pyridin-2-yl]-2-furamide hydrochloride

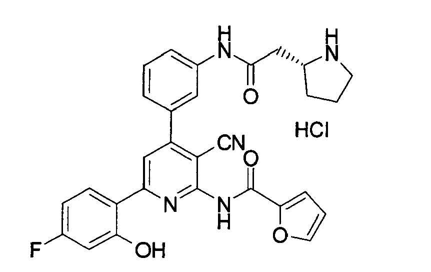

WSCD (250 mg, 1.30 mmol) was added to a solution of the compound of Reference Example 135 (400 mg, 0.87 mmol), [(2R)-1-(tert-butoxycarbonyl)pyrrolidin-2-yl]acetic acid (300 mg, 1.31 mmol) and HOBt (180 mg, 1.33 mmol) in DMF (4 mL) at room temperature, and stirred at room temperature for 4 days. The resultant solution was diluted with ethyl acetate, and washed twice with water and once with saturated brine. The organic layer was dried over magnesium sulfate and filtered, and the solvent was removed under reduced pressure. The residue was purified by basic silica gel column chromatography (eluting solution: hexane to ethyl acetate/hexane =1/1) and concentrated. The residue was dissolved in ethyl acetate (5 ml)-methanol (2 mL). A 4N hydrogen chloride-ethyl acetate solution (5 mL) was added, and stirred at room temperature overnight. The solvent was removed under reduced pressure, and the residue was recrystallized from methanol to give the title compound (340 mg, 70%) as a yellow powder.
¹H-NMR (300MHz, DMSO-d₆) δ: 1.55-1.68 (1H, m), 1.81-1.97 (2H, m), 2.10-2.20 (1H, m), 2.93 (2H, d, J = 6.9 Hz), 3.13-3.21 (2H, m), 3.76-3.85 (1H, m), 6.79-6.89 (3H, m), 7.41 (1H, d, J = 7.8 Hz), 7.54-7.61 (2H, m), 7.81-7.84 (1H, m), 8.04 (1H, br s), 8.08-8.09 (1H, m), 8.15 (1H, s), 8.23 (1H, dd, J = 8.4, 6.6 Hz), 8.94 (1H, br s), 9.18 (1H, br s), 10.64 (1H, s), 11.33 (1H, s), 12.33 (1H, s).
Melting point: 269-270°C
Element analysis: Calculated (%) for C₂₉H₂₄N₅O₄F·1.0HCl: C, 61.98; H, 4.48; N, 12.46. Found (%): C, 61.77; H, 4.53; N, 12.34.

### Example 315

### N-[3-cyano-4-{3-[(N,N-diethylglycyl)amino]phenyl}-6-(4-fluoro-2-hydroxyphenyl) pyridin-2-yl]-2-furamide hydrochloride

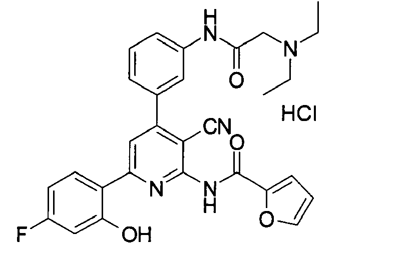

A 4N hydrogen chloride-ethyl acetate solution (5 mL) was added to a solution of the compound of Reference Example 255 (150 mg, 0.26 mmol) in ethyl acetate (5 mL)-methanol (2 mL), and stirred at room temperature overnight. The solvent was removed under reduced pressure, and the residue was recrystallized from methanol-diethylether to give the title compound (130 mg, 89%) as a pale yellow powder.
¹H-NMR (300MHz, DMSO-d₆) δ : 1.25 (6H, t, J = 7.2 Hz), 3.26 (4H, br s), 4.20 (2H, d, J = 3.9 Hz), 6.79-6.89 (3H, m), 7.49 (1H, d, J = 8.1 Hz), 7.54 (1H, dd, J = 3.6, 0.6 Hz), 7.63 (1H, t, J = 8.1 Hz), 7.82-7.86 (1H, m), 8.03-8,04 (1H, m), 8.08 (1H, dd, J = 1.5, 0.6 Hz), 8.16 (1H, s), 8.23 (1H, dd, J = 8.4, 6.9 Hz), 9.75 (1H, br s), 11.18 (1H, s), 11.34 (1H, s), 12.31 (1H, s).
Melting point: 262-263°C
Element analysis: Calculated (%) for C₂₉H₂₆N₅O₄F·1.0HCl·0.25H₂O: C, 61.27; H, 4.88; N, 12.32. Found (%): C, 61.22; H, 4.85; N, 12.30.

### Example 316

### N-[3-cyano-4-{3-[(N-ethyl-N-methyl-β-alanyl)amino]phenyl}-6-(4-fluoro-2-hydroxyphenyl)pyridin-2-yl]-2-furamide hydrochloride

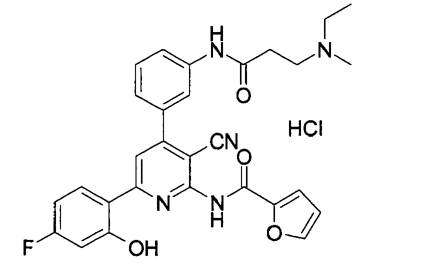

Sodium cyanoborohydride (53 mg, 0.84 mmol) was added to a solution of the compound of Example 313 (0.15 g, 0.28 mmol) in methanol (5 mL), acetaldehyde (90%, 41 mg, 0.84 mmol), triethylamine (0.060 mL, 0.43 mmol) and acetic acid (0.3 mL) at room temperature, and stirred at room temperature for 4 hours. THF (5 mL) was added to dissolve insoluble components, and stirred at room temperature for 90 minutes. Acetaldehyde (0.10 mL) and sodium cyanoborohydride (53 mg, 0.84 mmol) were added, and stirred at room temperature for 17 hours. The reaction mixture was diluted with ethyl acetate, washed with saturated sodium hydrogencarbonate solution, water and saturated brine, dried over magnesium sulfate, and then concentrated under reduced pressure. The residue was purified by basic silica gel column chromatography (eluting solution: ethyl acetate to ethyl acetate/methanol = 9/1), and concentrated. An excessive amount of 4N hydrogen chloride-ethyl acetate solution was added to the residue and concentrated. The residue was recrystallized from methanol to give the title compound (92 mg, 58%) as a pale yellow powder.
¹H-NMR (300MHz, DMSO-d₆) δ : 1.25 (3H, t, J = 7.2 Hz), 2.75 (3H, d, J = 3.9 Hz), 2.95 (2H, t, J = 7.2 Hz), 3.07-3.44 (4H, m), 6.79-6.88 (3H, m), 7.41 (1H, d, J = 8.1 Hz), 7.53-7.61 (2H, m), 7.81-7.84 (1H, m), 8.01 (1H, s), 8.08 (1H, dd, J = 1.8, 0.9 Hz), 8.14 (1H, s), 8.21-8.26 (1H, m), 9.97 (1H, br s), 10.65 (1H, s), 11.32 (1H, s), 12.33 (1H, d, J = 1.2 Hz).
Melting point: 223-224°C
Element analysis: Calculated (%) for C₂₉H₂₆N₅O₄F·1.0HCl·0.5H₂O: C, 60.79; H, 4.93; N, 12.22. Found (%): C, 60.46; H, 5.06; N, 12.00.

### Example 317

### N-[3-cyano-4-(3-{[3-(diethylamino)hexanoyl]amino}phenyl)-6-(4-fluoro-2-hydroxyphenyl)pyridin-2-yl]-2-furamide hydrochloride

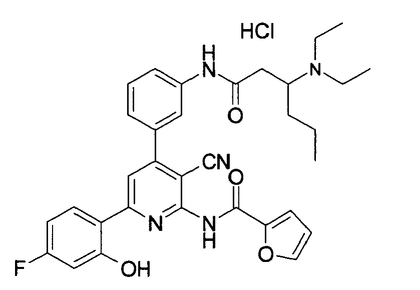

Sodium cyanoborohydride (66 mg, 1.05 mmol) was added to a solution of the compound of Example 265 (0.20 g, 0.35 mmol) in methanol (5 mL), acetaldehyde (0.20 mL), triethylamine (0.075 mL, 0.54 mmol) and acetic acid (0.5 mL) at room temperature, and stirred at room temperature for 2.5 hours. The reaction mixture was diluted with ethyl acetate, washed with saturated sodium hydrogencarbonate solution, water and saturated brine, dried over magnesium sulfate, and then concentrated under reduced pressure. The residue was purified by basic silica gel column chromatography (eluting solution: ethyl acetate to ethyl acetate/methanol = 9/1), and concentrated. An excessive amount of 4N hydrogen chloride-ethyl acetate solution was added to the residue and concentrated. The residue was recrystallized from methanol-diethylether to give the title compound (0.14 g, 65%) as a pale yellow powder.
¹H-NMR (300MHz, DMSO-d₆) δ : 0.91 (3H, t, J = 7.2 Hz), 1.28-1.42 (8H, m), 1.58-1.84 (2H, m), 2.75 (1H, dd, J = 16.5, 6.9 Hz), 3.05-3.29 (5H, m), 3.86 (1H, br s), 6.79-6.88 (3H, m), 7.43 (1H, d, J = 7.8 Hz), 7.54-7.61 (2H, m), 7.83 (1H, d, J = 8.1 Hz), 8.01 (1H, br s), 8.08 (1H, d, J = 1.2 Hz), 8.15 (1H, s), 8.21-8.26 (1H, m), 9.56 (1H, br s), 10.76 (1H, s), 11.31 (1H, s), 12.34 (1H, s).
Melting point: 195-197°C
Element analysis: Calculated (%) for C₃₃H₃₄N₅O₄F·1.0HCl·1.0H₂O: C, 62.11; H, 5.84; N, 10.97. Found (%): C, 62.02; H, 6.00; N, 10.81.

### Example 318

### N-[3-cyano-4-(3-{[3-(dipropylamino)butanoyl]amino}phenyl)-6-(4-fluoro-2-hydroxyphenyl)pyridin-2-yl]-2-furamide hydrochloride

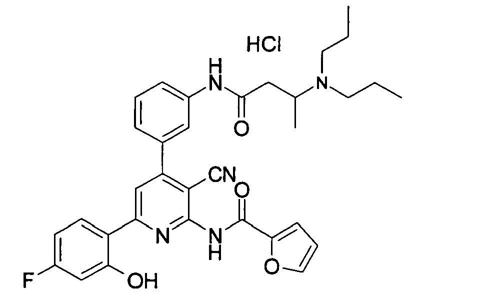

Sodium cyanoborohydride (70 mg, 1.11 mmol) was added to a solution of the compound of Example 337 (0.20 g, 0.37 mmol) in THF (5 ml)-methanol (5 mL), propionaldehyde (0.1 mL), triethylamine (0.08 mL, 0.57 mmol) and acetic acid (0.5 mL) at room temperature, and stirred at room temperature for 13.5 hours.

Propionaldehyde (0.1 mL) and sodium cyanoborohydride (70 mg, 1.11 mmol) were added, and stirred at room temperature for another 4 hours. The reaction mixture was diluted with ethyl acetate, washed with saturated sodium hydrogencarbonate solution, water and saturated brine, dried over magnesium sulfate, and then concentrated under reduced pressure. The residue was purified by basic silica gel column chromatography (eluting solution: hexane to ethyl acetate), and concentrated. An excessive amount of 4N hydrogen chloride-ethyl acetate solution was added to the residue and concentrated. The residue was recrystallized from methanol to give the title compound (0.15 g, 65%) as a yellow powder.
¹H-NMR (300MHz, DMSO-d₆) δ : 0.85-0.97 (6H, m), 1.34 (3H, d, J = 6.6 Hz), 1.74-1.80 (4H, m), 2.72-2.80 (1H, m), 2.99-3.19 (5H, m), 3.91 (1H, br s), 6.79-6.87 (3H, m), 7.43 (1H, d, J = 7.5 Hz), 7.54-7.61 (2H, m), 7.82 (1H, d, J = 8.1 Hz), 8.00 (1H, s), 8.08 (1H, s), 8.15 (1H, s), 8.23 (1H, t, J = 7.2 Hz), 9.82 (1H, br s), 10.71 (1H, s), 11.31 (1H, s), 12.35 (1H, s).
Melting point: 198-200°C
Element analysis: Calculated (%) for C₃₃H₃₄N₅O₄F·1.0HCl·0.5H₂O: C, 63.00; H, 5.77; N, 11.13. Found (%): C, 62.87; H, 5.83; N, 11.02.

### Example 319

### N-[3-cyano-4-(3-{[3-(diethylamino)pentanoyl]amino}phenyl)-6-(4-fluoro-2-hydroxyphenyl)pyridin-2-yl]-2-furamide hydrochloride

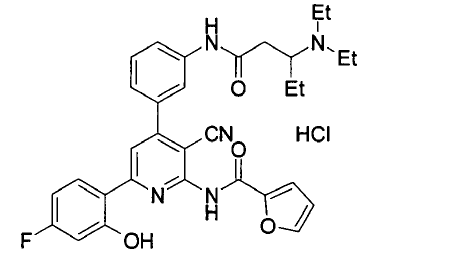

From the compound of Example 264 (150 mg, 0.27 mmol) and 97% acetaldehyde (268 mg, 5.46 mmol), the title compound (130 mg, 79%) was given as a pale yellow crystal in substantially the same manner as in Example 305.
¹H-NMR (300MHz, DMSO-d₆) δ:0.98 (3H, t, J = 7.2 Hz), 1.28-1.33 (6H, m), 1.60-1.78 (1H, m), 1.86-2.06 (1H, m), 2.76 (1H, dd, J = 16.2, 6.6 Hz), 3.04-3.39 (5H, m), 3.80 (1H, br s), 6.79-6.88 (3H, m), 7.42 (1H, d, J = 8.1 Hz), 7.54-7.61 (2H, m), 7.83 (1H, d, J = 8.7 Hz), 8.02 (1H, s), 8.08 (1H, d, J = 0.6 Hz), 8.15 (1H, s), 8.23 (1H, t, J = 7.8 Hz), 9.61 (1H, br s), 10.78 (1H, s), 11.31 (1H, s), 12.34 (1H, s).
Melting point: 171-175°C
Element analysis: Calculated (%) for C₃₂H₃₂N₅O₄F·1.0HCl·1.0H₂O: C, 61.58; H, 5.65; N, 11.22. Found (%): C, 61.43; H, 5.69; N, 11.20.

### Example 320

### N-[3-cyano-4-(3-{[3-(ethylamino)butanoyl]amino}phenyl)-6-(4-fluoro-2-hydroxyphenyl)pyridin-2-yl]-2-furamide hydrochloride

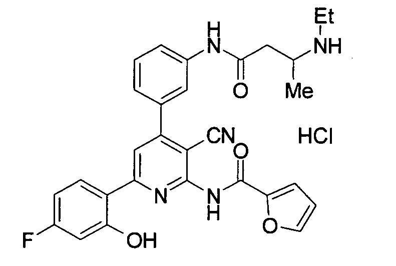

From the compound of Example 337 (100 mg, 0.19 mmol) and 97% acetaldehyde (14 mg, 0.28 mmol), the title compound (83 mg, 77%) was given as a yellow crystal in substantially the same manner as in Example 308.
¹H-NMR (300MHz, DMSO-d₆) δ : 1.23 (3H, t, J = 7.2 Hz), 1.30 (3H, d, J = 6.3 Hz), 2.69-2.77 (1H, m), 2.91-3.02 (3H, m), 3.60 (1H, q, J = 5.7 Hz), 6.78-6.86 (3H, m), 7.41 (1H, d, J = 7.5 Hz), 7.53-7.60 (2H, m), 7.83 (1H, d, J = 8.1 Hz), 8.01 (1H, d, J = 1.2 Hz), 8.14 (1H, s), 8.21 (1H, s), 8.23 (1H, t, J = 7.8 Hz), 8.60-8.90 (2H, br), 10.65 (1H, s), 11.30-11.50 (1H, br), 12.30-12.50 (1H, br).
Melting point: 249-255°C
Element analysis: Calculated (%) for C₂₉H₂₆N₅O₄F·1.0HCl: C, 61.76; H, 4.83; N, 12.42. Found (%): C, 61.39; H, 4.81; N, 12.38.

### Example 321

### N-[3-cyano-6-(4-fluoro-2-hydroxyphenyl)-4-(3-{[3-(isopropylamino)butanoyl]amino} phenyl)pyridin-2-yl]-2-furamide hydrochloride

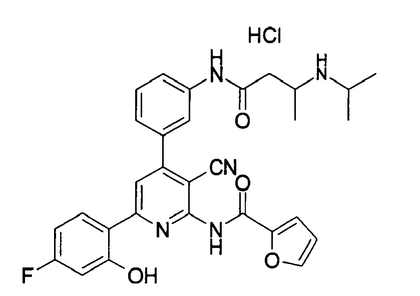

Sodium cyanoborohydride (70 mg, 1.11 mmol) was added to a solution of the compound of Example 337 (0.20 g, 0.37 mmol) in THF (5 ml)-methanol (5 mL), acetone (0.2 mL), triethylamine (0.08 mL, 0.57 mmol) and acetic acid (0.5 mL) at room temperature, and stirred at room temperature for 8 hours. The reaction mixture was diluted with ethyl acetate, washed with saturated sodium hydrogencarbonate solution, water and saturated brine, dried over magnesium sulfate, and then concentrated under reduced pressure. An excessive amount of 4N hydrogen chloride-ethyl acetate solution was added to the residue and concentrated. Then, the residue was recrystallized from methanol-diethylether to give the title compound (0.17 g, 79%) as a pale yellow powder.
¹H-NMR (300MHz, DMSO-d₆) δ : 1.26-1.33 (9H, m), 2.75 (1H, dd, J = 15.9, 7.8 Hz), 2.94 (1H, dd, J = 15.9, 4.8 Hz), 3.44 (1H, br s), 3.68 (1H, br s), 6.79-6.88 (3H, m), 7.42 (1H, d, J = 7.8 Hz), 7.53-7.61 (2H, m), 7.84 (1H, d, J = 8.1 Hz), 8.01 (1H, s), 8.08 (1H, d, J = 1.2 Hz), 8.14 (1H, s), 8.21-8.26 (1H, m), 8.62 (1H, br s), 8.76 (1H, br s), 10.65 (1H, s), 11.31 (1H, s), 12.34 (1H, s).
Melting point: 197-199°C
Element analysis: Calculated (%) for C₃₀H₂₈N₅O₄F·1.0HCl·1.5H₂O: C, 59.55; H, 5.33; N, 11.57. Found (%): C, 59.51; H, 5.32; N, 11.61.

### Example 322

### N-[3-cyano-4-(3-{[(1-ethylpiperidin-2-yl)acetyl]amino}phenyl)-6-(4-fluoro-2-hydroxyphenyl)pyridin-2-yl]-2-furamide hydrochloride

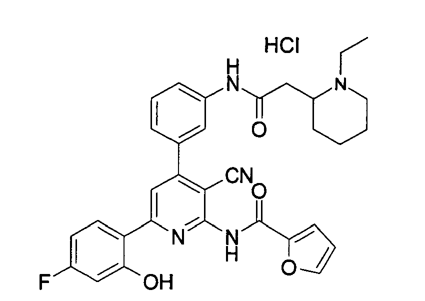

Sodium cyanoborohydride (49 mg, 0.78 mmol) was added to a solution of the compound of Example 290 (0.15 g, 0.26 mmol) in THF (5 ml)-methanol (5 mL), acetaldehyde (0.20 mL), triethylamine (0.055 mL, 0.39 mmol) and acetic acid (0.5 mL) at room temperature, and stirred at room temperature for 1 hour. The reaction mixture was diluted with ethyl acetate, washed with saturated sodium hydrogencarbonate solution, water and saturated brine, dried over magnesium sulfate, and then concentrated under reduced pressure. The residue was purified by basic silica gel column chromatography (eluting solution: ethyl acetate to ethyl acetate/methanol = 9/1), and concentrated. An excessive amount of 4N hydrogen chloride-ethyl acetate solution was added to the residue and concentrated. Then, the residue was recrystallized from methanol-diethylether to give the title compound (95 mg, 60%) as a colorless powder.
¹H-NMR (300MHz, DMSO-d₆) δ: 1.21-1.29 (3H, m), 1.50-1.96 (6H, m), 2.73-3.16 (5H, m), 3.25-3.42 (1H, m), 3.56 (1H, br s), 6.79-6.88 (3H, m), 7.42 (1H, d, J = 7.8 Hz), 7.53-7.61 (2H, m), 7.83 (1H, d, J = 8.7 Hz), 8.02 (1H, br s), 8.07-8.08 (1H, m), 8.15 (1H, s), 8.21-8.26 (1H, m), 10.03 (1H, br s), 10.72 (1H, br s), 11.31 (1H, s), 12.34 (1H, s).
Melting point: 201-203°C
Element analysis: Calculated (%) for C₃₂H₃₀N₅O₄F·1.0HCl·1.0H₂O: C, 61.78; H, 5.35; N, 11.26. Found (%): C, 61.42; H, 5.33; N, 11.25.

### Example 323 and Example 324

### N-{3-cyano-6-(4-fluoro-2-hydroxyphenyl)-4-[3-({2-[methyl(trifluoroacetyl)amino] ethyl} amino)phenyl]pyridin-2-yl -2-furamide

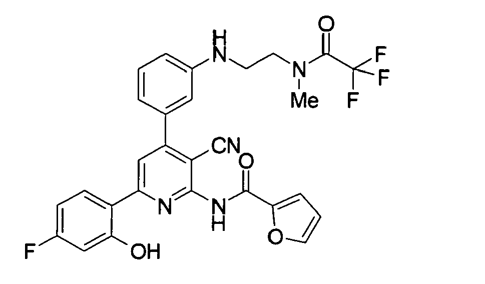

and

### N-[3-cyano-4-{3-[[2-(dimethylamino)ethyl](methyl)amino]phenyl}-6-(4-fluoro-2-hydroxyphenyl)pyridin-2-yl]-2-furamide

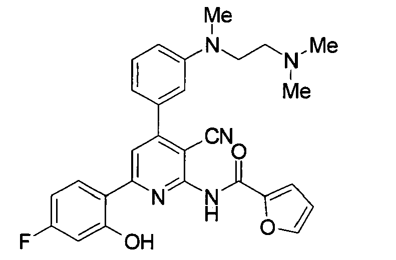

Sodium cyanoborohydride (38 mg, 0.60 mmol) was added to a solution of the compound of Example 340 (90 mg, 0.15 mmol) in methanol (10 mL), a 37% aqueous solution of formaldehyde (64 mg, 0.92 mmol) and acetic acid (0.1 mL) at room temperature, and stirred at room temperature for 3 days. The reaction mixture was diluted with saturated sodium hydrogencarbonate solution, and extracted with ethyl acetate. The extract was washed with saturated brine, dried over magnesium sulfate, and then concentrated under reduced pressure. The residue was purified by basic silica gel column chromatography (eluting solution: ethyl acetate to ethyl acetate/methanol = 10/1) to give N-{3-cyano-6-(4-fluoro-2-hydroxyphenyl)-4-[3-({2-[methyl(trifluoroacetyl)amino]ethyl}amino)phenyl]pyridin-2-yl}-2-furamide (30 mg, 35%) as a yellow solid, and N-[3-cyano-4-{3-[[2-(dimethylamino)ethyl](methyl)amino]phenyl}-6-(4-fluoro-2-hydroxyphenyl)pyridin-2-yl]-2-furamide (40 mg, 53%) as a yellow solid.

### N-{3-cyano-6-(4-fluoro-2-hydroxyphenyl)-4-[3-({2-[methyl(trifluoroacetyl)amino] ethyl}amino)phenyl]pyridin-2-yl}-2-furamide

¹H-NMR (300MHz, CDCl₃) δ: 3.08 (3H, s), 3.64-3.71 (4H, m), 6.59-6.66 (2H, m), 6.75 (1H, dd, J = 10.5, 2.4 Hz), 6.86-6.97 (3H, m), 7.12 (1H, t, J = 0.9 Hz), 7.40-7.46 (2H, m), 7.62-7.65 (2H, m), 7.81 (1H, dd, J = 9.0, 6.3 Hz), 9.15 (1H, s), 11.58 (1H, d, J = 1.5 Hz).
Melting point: 226-228°C

### N-[3-cyano-4-{3-[[2-(dimethylamino)ethyl](methyl)amino]phenyl}-6-(4-fluoro-2-hydroxyphenyl)pyridin-2-yl]-2-furamide

¹H-NMR (300MHz, CDCl₃) δ : 2.32 (6H, s), 2.55 (2H, t, J = 7.5 Hz), 3.06 (3H, s), 3.54 (2H, t, J = 7.5 Hz), 6.60-6.66 (2H, m), 6.80 (1H, dd, J = 10.5, 2.4 Hz), 6.85-6.90 (3H, m), 7.36-7.45 (2H, m), 7.62 (2H, s), 7.79 (1H, dd, J = 9.3, 6.6 Hz), 9.21 (1H, br s), 8.61 (1H, d, J = 1.2 Hz).
Melting point: 203-204°C

### Example 325

### N-{3-cyano-6-(4-fluoro-2-hydroxyphenyl)-4-[3-(piperazine-1-ylmethyl)phenyl] pyridin-2-yl}-2-furamide ditrifluoroacetic acid

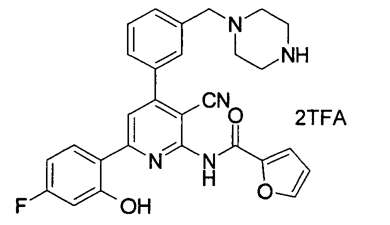

Trifluoroacetic acid (1 mL) was added to a solution of the compound of Reference Example 251 (50 mg, 0.078 mmol) in dichloromethane (2 mL), and stirred at room temperature for 1 hour. The reaction mixture was diluted with diethylether, and the precipitate was collected by filtration. The residue was recrystallized from methanol-diethylether to give the title compound (29 mg, 35%) as a white crystal.
¹H-NMR (300MHz, DMSO-d₆) δ : 2.66 (4H, br s), 3.13 (4H, br s), 3.73 (2H, s), 6.80-6.87 (3H, m), 7.52-7.74 (5H, m), 8.08 (1H, d, J = 0.9 Hz), 8.18 (1H, s), 8.26 (1H, dd, J = 9.6, 6.9 Hz), 8.59 (2H, br s), 11.29 (1H, s), 12.40 (1H, s).
Melting point: 144-150°C

### Example 326

### N-[3-cyano-4-[3-(1,4-diazepan-1-yl)phenyl]-6-(4-fluoro-2-hydroxyphenyl)pyridin-2-yl] -2-furamide hydrochloride

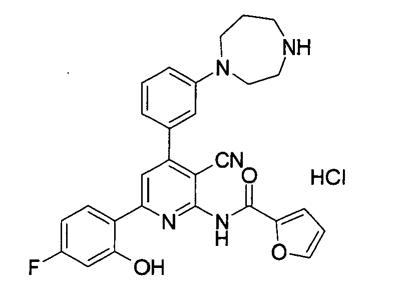

From the compound of Reference Example 243 (140 mg, 0.22 mmol), the title compound (108 mg, 86%) was given as a pale yellow crystal in substantially the same manner as in Example 271.
¹H-NMR (300MHz, DMSO-d₆) δ:2.09-2.20 (2H, br), 3.10-3.20 (2H, br), 3.30-3.40 (2H, br), 3.61 (2H, t, J = 5.7 Hz), 3.75-3.95 (2H, br), 6.79-6.86 (3H, m), 6.99-7.07 (3H, m), 7.43 (1H, t, J = 7.8 Hz), 7.53 (1H, d, J = 3.6 Hz), 8.08 (1H, t, J = 1.5 Hz), 8.13 (1H, s), 8.22 (1H, dd, J = 9.9, 6.9 Hz), 8.96 (2H, br s), 11.23 (1H, s), 12.41 (1H, s).
Melting point: 254-259°C
Element analysis: Calculated (%) for C₂₈H₂₄N₅O₃F·1.0HCl·0.5H₂O: C, 61.94; H, 4.83; N, 12.90. Found (%): C, 61.86; H, 4.82; N, 12.64.

### Example 327

### N-{3-cyano-6-(4-fluoro-2-hydroxyphenyl)-4-[3-(4-methylpiperazin-1-yl)phenyl] pyridin-2-yl}-2-furamide hydrochloride

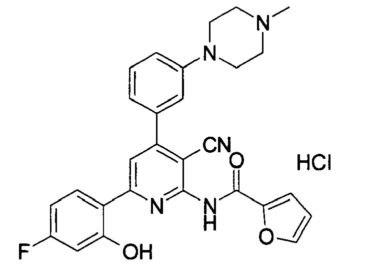

From the compound of Reference Example 239 (95 mg, 0.18 mmol), the title compound (82 mg, 80%) was given as a pale yellow crystal in substantially the same manner as in Example 271.
¹H-NMR (300MHz, DMSO-d₆) δ: 2.83 (3H, s), 3.10-3.30 (4H, br), 3.40-3.65 (2H, br), 3.80-4.15 (2H, br), 6.79-6.87 (3H, m), 7.20-7.27 (2H, m), 7.32 (1H, s), 7.48-7.54 (2H, m), 8.08 (1H, s), 8.16 (1H, s), 8.25 (1H, dd, J = 9.9, 6.9 Hz), 10.62 (1H, br s), 11.25 (1H, s), 11.44 (1H, s).
Melting point: 254-259°C
Element analysis: Calculated (%) for C₂₈H₂₄N₅O₃F·1.0HCl·1.0H₂O: C, 60.93; H, 4.93; N, 12.68. Found (%): C, 61.32; H, 4.85; N, 12.69.

### Example 328

### N-[3-cyano-4-{3-[(N,N-diethyl-β-alanyl)amino]phenyl}-6-(4-fluoro-2-hydroxyphenyl) pyridin-2-yl]-2-furamide hydrochloride

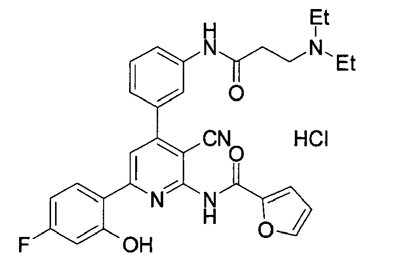

From the compound of Example 68 (200 mg, 0.38 mmol) and 97% acetaldehyde (0.6 mL), the title compound (194 mg, 88%) was given as a pale yellow crystal in substantially the same manner as in Example 305.
¹H-NMR (300MHz, DMSO-d₆) δ : 1.24 (6H, t, J = 7.2 Hz), 2.94 (2H, t, J = 7.2 Hz), 3.08-3.24 (4H, m), 3.34-3.39 (2H, m), 6.79-6.88 (3H, m), 7.41 (1H, d, J = 7.8 Hz), 7.54-7.60 (2H, m), 7.83 (1H, d, J = 8.4 Hz), 8.01 (1H, s), 8.08 (1H, s), 8.14 (1H, s), 8.23 (1H, t, J = 7.8 Hz), 10.07 (1H, br s), 10.67 (1H, s), 11.32 (1H, s), 12.34 (1H, s).
Melting point: 209-213°C
Element analysis: Calculated (%) for C₃₀H₂₈N₅O₄F·1.0HCl·0.5H₂O: C, 61.38; H, 5.15; N, 11.93. Found (%): C, 61.23; H, 4.95; N, 11.80.

### Example 329

### N-{3-cyano-6-(4-fluoro-2-hydroxyphenyl)-4-[3-(glycylamino)phenyl]pyridin-2-yl}-2-furamide hydrochloride

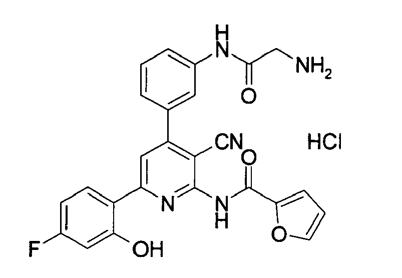

From the compound of Reference Example 257 (200 mg, 0.32 mmol), the title compound (162 mg, 99%) was given as a pale yellow crystal in substantially the same manner as in Example 271.
¹H-NMR (300MHz, DMSO-d₆) δ : 3.85 (2H, d, J = 5.7 Hz), 6.79-6.87 (3H, m), 7.47 (1H, d, J = 7.8 Hz), 7.54 (1H, d, J = 3.3 Hz), 7.62 (1H, t, J = 7.8 Hz), 7.82 (1H, d, J = 8.1 Hz), 8.00 (1H, s), 8.02 (1H, t, J = 0.9 Hz), 8.16-8.26 (5H, m), 10.87 (1H, s), 11.34 (1H, s), 12.33 (1H, s).
Melting point: 229-236°C
Element analysis: Calculated (%) for C₂₅H₁₈N₅O₄F·1.0HCl·1.0H₂O: C, 57.09; H, 4.02; N, 13.32. Found (%): C, 56.84; H, 3.83; N, 13.09.

### Example 330

### N-[3-cyano-4-[3-(4-ethylpiperazin-1-yl)phenyl]-6-(4-fluoro-2-hydroxyphenyl) pyridin-2-yl]-2-furamide hydrochloride

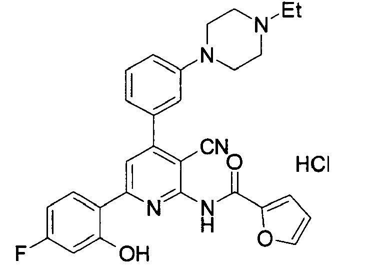

From the compound of Reference Example 240 (90 mg, 0.16 mmol), the title compound (77 mg, 82%) was given as a pale yellow crystal in substantially the same manner as in Example 271.
¹H-NMR (300MHz, DMSO-d₆) δ:1.29 (3H, t, J = 7.2 Hz), 3.10-3.25 (6H, m), 3.59 (2H, d, J = 11.4 Hz), 3.98 (2H, d, J = 11.4 Hz), 6.79-6.87 (3H, m), 7.20-7.27 (2H, m), 7.33 (1H, s), 7.48-7.54 (2H, m), 8.08 (1H, t, J = 0.9 Hz), 8.16 (1H, s), 8.25 (1H, dd, J = 9.6, 6.9 Hz), 10.53 (1H, br s), 11.25 (1H, s), 12.44 (1H, s).
Melting point: 218-222°C
Element analysis: Calculated (%) for C₂₉H₂₆N₅O₃F·1.0HCl·1.0H₂O: C, 61.54; H, 5.16; N, 12.37; Cl, 6.26. Found (%): C, 61.72; H, 5.30; N, 12.34; Cl, 6.07.

### Example 331

### N-[3-cyano-6-(4-fluoro-2-hydroxyphenyl)-4-(3-morpholin-4-ylphenyl)pyridin-2-yl]-2-f uramide hydrochloride

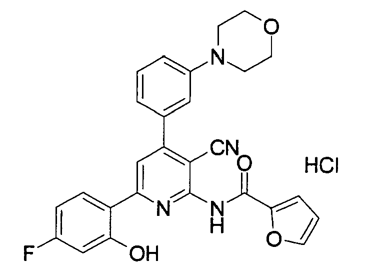

From the compound of Reference Example 241 (230 mg, 0.44 mmol), the title compound (195 mg, 85%) was given as a white crystal in substantially the same manner as in Example 271.
¹H-NMR (300MHz, DMSO-d₆) δ : 3.24 (4H, s), 3.78 (4H, s), 6.79-6.85 (3H, m), 7.16-7.26 (3H, m), 7.45-7.54 (2H, m), 8.08 (1H, s), 8.15 (1H, s), 8.26 (1H, dd, J = 9.6, 6.9 Hz), 11.22 (1H, s), 12.00-13.30 (1H, br).
Melting point: 244-246°C
Element analysis: Calculated (%) for C₂₇H₂₁N₄O₄F·1.0HCl: C, 62.25; H, 4.26; N, 10.75. Found (%): C, 62.24; H, 4.28; N, 10.81.

### Example 332

### N-[3-cyano-6-(4-fluoro-2-hydroxyphenyl)-4-(3-piperidin-1-ylphenyl)pyridin-2-yl]-2-furamide hydrochloride

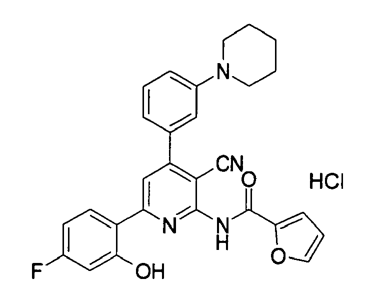

From the compound of Reference Example 242 (210 mg, 0.40 mmol), the title compound (179 mg, 86%) was given as a pale yellow crystal in substantially the same manner as in Example 271.
¹H-NMR (300MHz, DMSO-d₆) δ : 1.65 (2H, br s), 1.90 (4H, br s), 3.50 (4H, br s), 6.79-6.88 (3H, m), 7.54-8.00 (5H, m), 8.08 (1H, s), 8.20-8.27 (2H, m), 11.31 (1H, s), 12.00-12.70 (1H, br).
Melting point: 240-242°C
Element analysis: Calculated (%) for C₂₈H₂₃N₄O₃F·1.0HCl: C, 64.80; H, 4.66; N, 10.80. Found (%): C, 64.56; H, 4.67; N, 10.82.

### Example 333

### N-(3-cyano-6-(4-fluoro-2-hydroxyphenyl)-4-{3-[(4-methylpiperazin-1-yl)methyl] phenyl}pyridin-2-yl)-2-furamide dihydrochloride

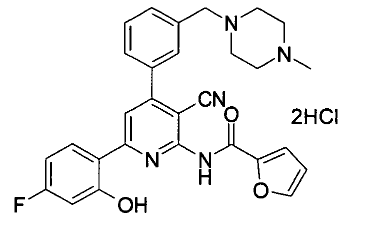

Sodium cyanoborate (75 mg, 1.19 mmol) was added to a solution of the compound of Example 325 (60 mg, 0.083 mmol) in methanol (10 mL), a 37% aqueous solution of formaldehyde (0.5 mL, 3.20 mmol) and acetic acid (1 mL), and stirred at room temperature for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure to give N-(3-cyano-6-(4-fluoro-2-hydroxyphenyl)-4-{3-[(4-methylpiperazine-1-yl)methyl]phenyl}pyridin-2-yl)-2-furamide.

A 4N hydrogen chloride-ethyl acetate solution (0.5 mL) was added to a solution of the entire amount of the resultant N-(3-cyano-6-(4-fluoro-2-hydroxyphenyl)-4-{3-[(4-methylpiperazine-1-yl)methyl]phenyl}pyridin-2-yl)-2-furamide in ethyl acetate (5 mL), and concentrated under reduced pressure. The residue was recrystallized from methanol-diethylether to give the title compound(40 mg, 82%) as a pale yellow crystal.
¹H-NMR (300MHz, DMSO-d₆) δ : 2.73 (3H, s), 3.30-4.50 (10H, m), 6.80-6.87 (3H, m), 7.54 (1H, d, J = 3.6 Hz), 7.62-7.81 (3H, m), 7.90-8.05 (1H, br), 8.08 (1H, s), 8.32-8.39 (2H, m), 11.32 (1H, s), 12.51 (1H, br s).
Melting point: 205-210°C
Element analysis: Calculated (%) for C₂₉H₂₆N₅O₃F·2.0HCl·1.5H₂O: C, 56.96; H, 5.11; N, 11.45. Found (%): C, 56.93; H, 5.16; N, 11.45.

### Example 334

N-[3-cyano-4-{3-[(4-ethylpiperazin-1-yl)methyl]phenyl}-6-(4-fluoro-2-hydroxyphenyl)pyridin-2-yl]-2-furamide dihydrochloride

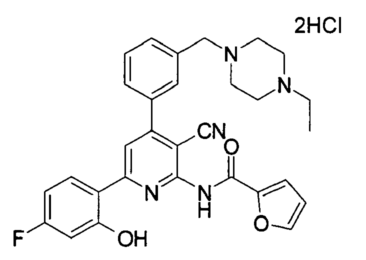

From the compound of Example 325 (60 mg, 0.083 mmol) and 97% acetaldehyde (0.5 mL, 9.53 mmol), the title compound (41 mg, 83%) was given as a pale yellow crystal in substantially the same manner as in Example 333.
¹H-NMR (300MHz, DMSO-d₆) δ : 1.25 (3H, t, J = 7.2 Hz), 3.10-4.50 (12H, m), 6.80-6.87 (3H, m), 7.54 (1H, d, J = 3.6 Hz), 7.68-7.82 (3H, m), 7.95-8.05 (1H, br), 8.09 (1H, s), 8.33-8.39 (2H, m), 11.33 (1H, s), 12.52 (1H, br s).
Melting point: 215-222°C
Element analysis: Calculated (%) for C₃₀H₂₈N₅O₃F·2.0HCl·1.5H₂O: C, 57.60; H, 5.32; N,11.20. Found (%): C, 57.88; H, 5.23; N, 11.14.

### Example 335

### N-[3-cyano-4-[3-(1,4-diazepan-1-ylmethyl)phenyl]-6-(4-fluoro-2-hydroxyphenyl) pyridin-2-yl]-2-furamide dihydrochloride

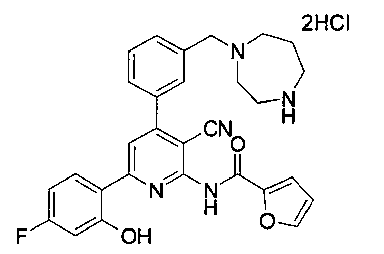

A 4N hydrogen chloride-ethyl acetate solution (1 mL) was added to an solution of the compound of Example 258 (75 mg, 0.11 mmol) in ethyl acetate (5 mL), and stirred at room temperature for 16 hours. The precipitated crystals were collected by filtration, and recrystallized from methanol-diethylether to give the title compound (63 mg, 98%) as a pale yellow crystal.
¹H-NMR (300MHz, DMSO-d₆) δ:2.10-2.30 (2H, br), 3.10-3.80 (8H, m), 4.53 (2H, br s), 6.80-6.87 (3H, m), 7.55 (1H, d, J = 3.6 Hz), 7.73 (1H, t, J = 7.5 Hz), 7.85 (2H, br s), 8.09 (2H, br s), 8.37-8.43 (2H, m), 9.32 (1H, br s), 9.60 (1H, br s), 11.34 (1H, s), 11.82 (1H, s), 12.54 (1H, s).
Melting point: 199-206°C
Element analysis: Calculated (%) for C₂₉H₂₆N₅O₃F·2.0HCl·1.5H₂O: C, 56.96; H, 5.11; N, 11.45. Found (%): C, 57.05; H, 5.15; N, 11.43.

### Example 336

### N-[3-cyano-4-[3-({[(2S)-1-ethylpyrrolidin-2-yl]acetyl}amino)phenyl]-6-(4-fluoro-2-hydroxyphenyl)pyridin-2-yl]-2-furamide hydrochloride

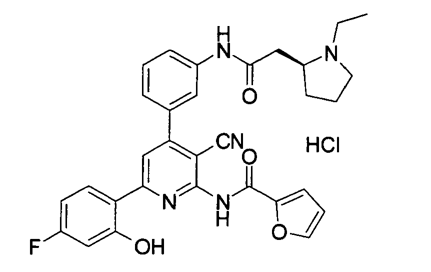

A solution of the compound of Example 279 (30 mg, 0.057 mmol), ethyl iodide (50 mg, 0.29 mmol) and N,N-diisopropylethylamine (22 mg, 0.17 mmol) in tert-butanol (2 mL) was stirred at 60°C for 16 hours. The reaction mixture was diluted with saturated aqueous sodium hydrogencarbonate, and extracted with ethyl acetate. The extract was washed with saturated brine, dried over magnesium sulfate, and then concentrated under reduced pressure to give N-[3-cyano-4-[3-({[(2S)-1-ethylpyrrolidin-2-yl]acetyl}amino)phenyl]-6-(4-fluoro-2-hydroxyphenyl)pyridin-2-yl]-2 -furamide.

A 4N hydrogen chloride-ethyl acetate solution (0.5 mL) was added to a solution of the entire amount of the resultant N-[3-cyano-4-[3-({[(2S)-1-ethylpyrrolidine-2-yl]acetyl}amino)phenyl]-6-(4-fluoro-2-hydroxyphenyl)pyridin-2-yl]-2-furamide in ethyl acetate (5 mL), and concentrated under reduced pressure. The residue was recrystallized from methanol-diethylether to give the title compound (24 mg, 71 %) as a white crystal.
¹H-NMR (300MHz, DMSO-d₆) δ : 1.26 (3H, t, J = 6.9 Hz), 1.70-1.77 (1H, m), 1.91-2.01 (2H, m), 2.22-2.31 (1H, m), 2.83-2.91 (1H, m), 3.00-3.17 (3H, m), 3.30-3.50 (1H, m), 3.50-3.60 (1H, m), 3.72-3.79 (1H, m), 6.79-6.86 (3H, m), 7.42 (1H, d, J = 7.2 Hz), 7.53-7.61 (2H, m), 7.82 (1H, d, J = 8.1 Hz), 7.99 (1H, s), 8.08 (1H, s), 8.14 (1H, s), 8.23 (1H, t, J = 7.8 Hz), 10.64 (1H, s), 11.31 (1H, s), 12.34 (1H, s).
Melting point: 182-187°C
Element analysis: Calculated (%) for C₃₁H₂₈N₅O₄F·1.0HCl·1.5H₂O: C, 60.34; H, 5.23; N, 11.35. Found (%): C, 60.41; H, 5.23; N, 11.34.

### Example 337

### N-[4-{3-[(3-aminobutanoyl)amino]phenyl}-3-cyano-6-(4-fluoro-2-hydroxyphenyl) pyridin-2-yl]-2-furamide hydrochloride

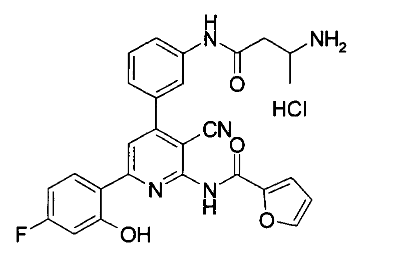

From the compound of Reference Example 135 (500 mg, 1.09 mmol), the title compound (411 mg, 70%) was given as a yellow powder in substantially the same manner as in Example 78.
¹H-NMR (300MHz, DMSO-d₆) δ : 1.26 (3H, d, J = 6.6 Hz), 2.60-2.85 (2H, m), 3.50-3.70 (1H, m), 6.75-6.90 (3H, m), 7.41 (1H, d, J = 8.1 Hz), 7.54 (1H, d, J = 3.9 Hz), 7.59 (1H, d, J = 7.8 Hz), 7.83 (1H, d, J = 8.1 Hz), 7.90-8.10 (5H, m), 8.14 (1H, s), 8.23 (1H, t, J = 7.5 Hz), 10.60 (1H, s), 11.31 (1H, s), 12.34 (1H, s).
Melting point: 213-215°C
Element analysis: Calculated (%) for C₂₇H₂₂N₅O₄F·1.0HCl·1.5H₂O: C, 57.60; H, 4.65; N. 12.44. Found (%): C, 57.49; H, 4.66; N, 12.38.

### Example 338

### N-[3-cyano-4-(3-{[3-(dimethylamino)butanoyl]amino}phenyl)-6-(4-fluoro-2-hydroxyphenyl)pyridin-2-yl]-2-furamide hydrochloride

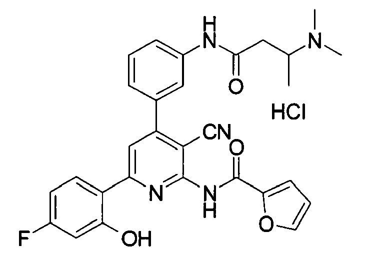

From the compound of Example 337 (100 mg, 0.19 mmol), the title compound (68 mg, 64%) was given as a yellow powder in substantially the same manner as in Example 333.
1H-NMR (300MHz, DMSO-d6) δ : 1.30 (3H, d, J = 6.6 Hz), 2.65-2.85 (7H, m), 2.90-3.10 (1H, m), 3.70-3.90 (1H, m), 6.75-6.90 (3H, m), 7.43 (1H, d, J = 7.5 Hz), 7.54 (1H, d, J = 3.3 Hz), 7.59 (1H, t, J = 7.8 Hz), 7.82 (1H, d, J = 9.0 Hz), 8.00 (1H, s), 8.08 (1H, s), 8.15 (1H, s), 8.20-8.30 (1H, m), 10.03 (1H, br s), 10.65 (1H, s), 11.32 (1H, s), 12.34 (1H, s).
Melting point: 233-234°C
Element analysis: Calculated (%) for C₂₉H₂₆N₅O₄F·1.0HCl·0.5H₂O: C, 60.79; H, 4.93; N. 12.22. Found (%): C, 60.84; H, 4.85; N, 12.27.

### Example 339

### N-[3-cyano-4-(3-{[3-(diethylamino)butanoyl]amino}phenyl)-6-(4-fluoro-2-hydroxyphenyl)pyridin-2-yl]-2-furamide hydrochloride

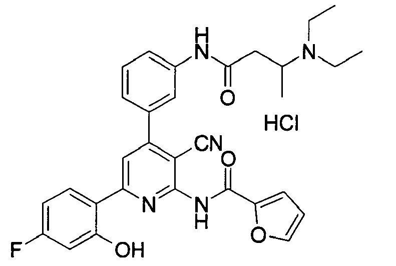

From the compound of Example 337 (100 mg, 0.19 mmol) and acetaldehyde (17 mg, 0.38 mmol), the title compound (60 mg, 54%) was given as a yellow powder in substantially the same manner as in Example 333.
¹H-NMR (300MHz, DMSO-d₆) δ: 1.25-1.45 (9H, m), 2.70-2.85 (1H, m), 3.05-3.30 (5H, m), 3.92 (1H, brs), 6.75-6.90 (3H, m), 7.43 (1H, d, J=7.8 Hz), 7.54 (1H, d, J=3.0 Hz), 7.58 (1H, t, J=7.8 Hz), 7.82 (1H, d, J=9.0 Hz), 8.01 (1H, s), 8.08 (1H, s), 8.15 (1H, s), 8.24 (1H, t, J=7.5 Hz), 9.79 (1H, s), 10.68 (1H, s), 11.31 (1H, s), 12.35 (1H, s).
Melting point: 193-195°C
Element analysis: Calculated (%) for C₃₁H₃₀N₅O₄F·1.0HCl·1.5H₂O: C, 60.14; H, 5.54; N. 11.31. Found (%): C, 60.51; H, 5.89; N, 11.25.

### Example 340

### N-[4-{3-[(2-aminoethyl)(trifluoroacetyl)amino]phenyl}-3-cyano-6-(4-fluoro-2-hydroxyphenyl)pyridin-2-yl]-2-furamide hydrochloride

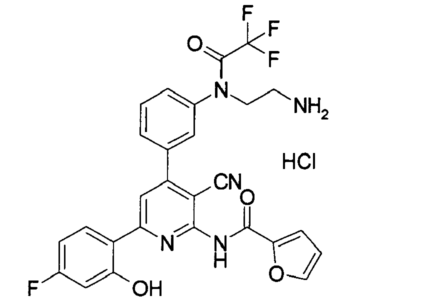

From the compound of Reference Example 245 (80 mg, 0.11 mmol), the title compound (55 mg, 85%) was given as a white crystal in substantially the same manner as in Example 8.
¹H-NMR (300MHz, DMSO-d₆) δ: 2.95-3.10 (2H, m), 4.00-4.12 (2H, m), 6.79-6.89 (3H, m), 7.54 (1H, d, J = 3.6 Hz), 7.79-7.90 (3H, m), 8.02-8.09 (5H, m), 8.19-8.27 (2H, m), 11.36 (1H, s), 12.27 (1H, s).
Melting point: 252-253°C

| Formulation Example 1 | | |
|---|---|---|
| (1) | Compound of Example 3 | 50 mg |
| (2) | Lactose | 34 mg |
| (3) | Cornstarch | 10.6 mg |
| (4) | Cornstarch (glue-like state) | 5 mg |
| (5) | Magnesium stearate | 0.4 mg |
| (6) | Carboxymethylcellulose calcium | 20 mg |
| | Total | 120 mg |

Components (1) through (6) are mixed in accordance with the usual method, and the resultant mixture is tableted by a tableting machine to obtain tablets.

| Formulation Example 2 | | |
|---|---|---|
| (1) | Compound of Example 3 | 10.0 mg |
| (2) | Lactose | 60.0 mg |
| (3) | Cornstarch | 35.0 mg |
| (4) | Gelatin | 3.0 mg |
| (5) | Magnesium stearate | 2.0 mg |

A mixture of 10.0 mg of the compound of Example 3, 60.0 mg lactose and 35.0 mg cornstarch is passed through a sieve having 1 mm meshes using 0.03 ml 10% aqueous solution of gelatin (3.0 mg as gelatin) and thus is granulated, dried at 40°C and then passed through the sieve again. Granules thus obtained are mixed with 2.0 mg magnesium stearate and compressed. The obtained core tablets are sugar-coated with an aqueous suspension of sucrose, titanium dioxide, talc and gum arabic. The tablets thus coated are polished with beeswax. Thus, coated tablets are obtained.

| Formulation Example 3 | | |
|---|---|---|
| (1) | Compound of Example 3 | 10.0 mg |
| (2) | Lactose | 70.0 mg |
| (3) | Cornstarch | 50.0 mg |
| (4) | Soluble starch | 7.0 mg |
| (5) | Magnesium stearate | 3.0 mg |

10.0 mg of the compound of Example 3 and 3.0 mg magnesium stearate are granulated with 0.07 ml aqueous solution of soluble starch (7.0 mg as soluble starch), dried, and mixed with 70.0 mg lactose and 50.0 mg cornstarch. The resultant mixture is compressed. Thus, tablets are obtained.

Test example 1

### (1) Preparation of receptor expressing CHO cells

cDNA fragments (SEQ ID NO: 2) encoding the entire length of human metastin receptor hOT7T 175 were incorporated into expression vector pAKK01.11H to produce animal cell expression plasmids pAKKO-hOT7T175. CHO/dhFr-cells (ATCC, #CRL-9096) were seeded onto 6 cm culture dishes (Becton Dickinson) at a density of 0.3 x 10⁶ cells/dish, and cultured in the conditions of 48 hours, 37°C and 5% CO₂. 5 µg pAKKO-hOT7T175 plasmid DNA was transfected to the resultant cells by a known method (described in Example 3 of WO 00/24890) using the Cellphect Transfection Kit (Amersham, #27-9268-01). The DNA-transfected cells were cultured in the Dulbecco's modified Eagle medium (DMEM) (Sigma, #D6046) containing 10% dialyzed FBS (Biowest, #S180D), lx Non-Essential Amino Acid (Invitrogen, #11140-050) and 50 µg/ml Gentamycin (Invitrogen, #15750-060; and cell strains stably expressing the plasmid genes were selected. The obtained 41 clones were treated with the FLIPR assay (in accordance with the method described in Example 3 of WO 00/24890), and the reactivity to the human metastin (45-54) (Nos. 40 to 54 of the human metastin sequence; Peptide Institute, Inc., #4389-v) was evaluated. In the end, h175KB34 cell strains were selected. By substantially the same method, h175-16 cell strains were also produced.

### (2) Preparation of a cell membrane fraction of the h175KB34 cells

The h175KB34 cells were cultured to be on the stage of 40-50% confluency using a cell factory (Nunc, #164327), and then cells were recovered in the following method.

First, the medium was disposed, and the cells were washed twice with 200 ml EDTA/PBS(-). Another 200 ml of EDTA/PBS(-) was added, and the cells were kept still for 20 minutes at room temperature until being peeled off. The cells were recovered into four 50 ml tubes (Becton Dickinson, #352070), and centrifuged (2,000 rpm, 10 min., 4°C) by a reduced performance centrifuge (TOMY, RL-101). The supernatant was disposed, and the pellets in the four tubes were each suspended with 10 ml EDTA/PBS(-) and collected into one tube (Becton Dickinson, #352070). The pellet was further centrifuged (2,000 rpm, 10 min., 4°C). The resultant pellet was suspended with 20 ml ice-cooled homogenizer buffer [10 mM NaHCO₃, 2 mM EGTA, 0.2 mM magnesium acetate, 10 µg/ml Leupeptin (Peptide Institute, Inc., #4041), 10 µg/ml E-64 (Peptide Institute, Inc., # 4096), 100 µg/ml o-phenanthroline (Dojindo Laboratories), 90 µg/ml PMSF (Wako Pure Chemical Industries, Ltd.), 10 µg/ml Pepstatin A (Peptide Institute, Inc., #4397), pH7.3]. The cell suspension was homogenized 3 times, each for 30 seconds, using a Polytron homogenizer. The resultant homogenate was centrifuged (2,500 rpm, 10 min, 4°C) using a high performance refrigerated centrifuge (Hitachi, Ltd., CR26H). The supernatant was recovered into a super-centrifuge tube, and super-centrifuged (30,000 rpm, 60 min, 4°C) using a super-centrifuge (Hitachi, Ltd., SCP70H). 3 ml homogenizer buffer was added to the resultant pellet, and the pellet was suspended by pipetting. 3 ml glycerol (Wako Pure Chemical Industries, Ltd.) was added to the resultant suspension and diluted 2-folds. The resultant substance was used as a cell membrane fraction of the h175KB34 cells. Each 100 µl of the membrane fraction was put into a 1.5 ml tube (Eppendorf, #0030120.086), stored in a freezer (-30C) so as not to be frozen, and used for a binding assay.

### (3) Preparation of an assay buffer

An assay buffer was prepared on the day of the test by adding, to each 100 ml of an assay basic buffer [20 mM Tris, 2.5 mM Magnesium Acetate, 2 mM EGTA, pH 7.4], 1 mg Leupeptin (Peptide Institute, Inc., 4041), 1 mg E-64 (Peptide Institute, Inc., 4096), 10 mg o-phenanthroline (Dojindo Laboratories), 9 mg PMSF (Wako Pure Chemical Industries, Ltd.) and 1 mg Pepstatin A (Peptide Institute, Inc., 4397). The prepared assay buffer was stored at 4°C until being used. When the measurement was to be performed with no BSA, the above-described assay buffer was used. When the measurement was to be performed with BSA, BSA (Fraction V, Serologicals protein Inc.) was added to the assay buffer before use at a ratio of 1 mg/ml.

### (4) Preparation of a membrane fraction suspension

Immediately before use, the cell membrane fraction of the h175KB34 cells prepared in section (2) was diluted 250-folds with the assay buffer.

### (5) Preparation of a ¹²⁵I-metastin (40-54)-diluted solution

Human metastin (40-54) (Nos.40 to 54 of the human metastin sequence) was synthesized in accordance with a known method (described in Example 3 of WO 00/24890) using a peptide synthesizer.

An eluate for HPLC, liquid A (0.1% trifluoroacetic acid), and liquid B (60% acetonitrile, 0.1% trifluoroacetic acid) were prepared as follows.

Liquid A was prepared by adding 1 ml trifluoroacetic acid (Wako Pure Chemical Industries, Ltd., #206-10731) to 1 L distilled water (Wako Pure Chemical Industries, Ltd., #042-16973). Liquid B was prepared by adding 400 ml distilled water (Wako Pure Chemical Industries, Ltd., #042-16973) to 600 ml acetonitrile (Wako Pure Chemical Industries, Ltd., #015-08633), and then adding 1 ml trifluoroacetic acid.

Radioactive iodo-labeling of metastin (40-54) by lactoperoxidase was performed as follows.

20 µl 1 mM human metastin (40-54), 20 µl 0.001% hydrogen peroxide water (Wako Pure Chemical Industries, Ltd., #081-04215), 20 µl 10 µg/ml lactoperoxidase (Sigma, #L-2005), and 20 µl [¹²⁵I]sodium iodide (Amersham, #IMS30, 74 MBq/20 µl) were put to a 1.5 ml tube (Eppendorf, #0030 120.086), and mixed to allow a reaction at room temperature for 10 minutes. Immediately after the reaction, 700 µl of liquid A (0.1% trifluoroacetic acid) was added to the reaction solution, and the reaction was stopped.

The reaction solution was injected into an HPLC column (Tosoh Corporation, TSKgel Super-ODS, 4.6 x 100 mm) which had been set to a high performance liquid chromatographer (liquid discharging pump: Shimadzu #LC-6A, system controller: Shimadzu #SLC-6A, UV detector: Shimadzu #SPD-6A, column oven: Shimadzu #CTO-10AS). Under the conditions of a flow rate of 1 ml/min. and a column temperature of 40°C, liquid B concentration was linearly increased within 40 minutes from liquid A 57%-liquid B 43% to liquid A 50%-liquid B 50%, and thus peptides were eluted. While observing the UV absorbance at 210 nm, an eluate containing a radioactive iodo-labeled metastin (40-54), i.e., ¹²⁵I-metastin (40-54), was sampled. The peptide eluate was diluted 10-folds using a ligand diluting buffer [50 mM Tris (Sigma, #T-8524), 5 mM MgCl₂ (Wako Pure Chemical Industries, Ltd., #135-00165), 150 mM NaCl (Wako Pure Chemical Industries, Ltd., #791-01665), 0.03% NaN₃, (Wako Pure Chemical Industries, Ltd., #195-11092), 10 µg/ml Leupeptin (Peptide Institute, Inc., #4041), 10 µg/ml E-64 (Peptide Institute, Inc., #4096), 100 µg/ml o-phenanthroline (Dojindo Laboratories, #346-02191), 90 µg/ml PMSF (Wako Pure Chemical Industries, Ltd., #162-12182), 10 µg/ml Pepstatin A (Peptide Institute, Inc., #4397), 0.1% BSA (Sigma, #A-2153), 0.05% CHAPS (Dojindo Laboratories, #345-04724), pH 7.4]. The resultant solution was used as an undiluted stock solution of ¹²⁵I-metastin (40-54).

The radioactivity of the undiluted stock solution of ¹²⁵I-metastin (40-54) was measured by a γ counter (Packard, CobraII), and the concentration of ¹²⁵I-metastin (40-54) was calculated. When prepared under the above-described conditions, the ¹²⁵I-metastin (40-54) generally had a concentration of about 50 nM.

The undiluted stock solution of ¹²⁵I-metastin (40-54) was divided into 400 µl, and stored at -70°C until immediately before use.

The undiluted stock solution of ¹²⁵I-metastin (40-54) was diluted with a filtering buffer [50 mM Tris, 5 mM MgCl₂, 150 mM NaCI, 0.1% BSA, 0.05% CHAPS, 0.03% NaN₃, pH 7.4] to prepare a diluted stock solution of ¹²⁵I-metastin (40-54), which was stored at -30°C. Immediately before use, the diluted stock solution of ¹²⁵I-metastin (40-54) was diluted with a ligand diluting buffer (20 ml assay buffer containing 20 mg BSA and 10 mg CHAPS) to prepare a 1.5 nM ¹²⁵I-metastin (40-54) solution. This dilution was performed regardless of whether the measurement was to be performed with or without BSA.

### (6) Binding reaction

178 µl of the membrane fraction suspension liquid prepared in section (4) was added to each of the wells of a 96-well plate (type 3363, Coming). Then, 2 µl of the test compound (the compound solution diluted with DMSO 100-folds with respect to the final measurement concentration) was added to each well. 2 µl DMSO was added to each well in the total binding control zone, whereas 2 µl 200 µM human metastin (45-54) solution (4389-v of Peptide Institute, Inc., which was diluted with DMSO so as to be 200 µM) was added to each well in the non-specific binding control zone. 20 µl of the 1.5 nM ¹²⁵I-metastin (40-54) solution prepared in section (5) was added to each well, and mixed by a micromixer (Sanko Junyaku Co., Ltd.) at room temperature for 60 minutes to allow a binding reaction.

### (7) Measurement

The post-binding-reaction solution in each well of the 96-well plate was transferred to a filter plate (UniFilterGF/B, PerkinElmer) which had been PEI-processed (had been immersed in 0.3% Polyethyleneimine, 20 mM Tris, pH 7.4), using a cell harvester (PerkinElmer), and then filtered. After filtering, the plate was washed three times with a washing buffer [50 mM Tris, 5 mM MgCl₂, 1 mM EDTA, 0.05% CHAPS, 0.05% NaN₃, 0.1% BSA, pH 7.4], and then dried by a dryer (50°C) for 45 minutes. 30 µl liquid scintillator (MicroScint O, PerkinElmer) was added to the dried filter plate, and the light emission of the scintillator was measured by the TopCount (PerkinElmer) for 1 minute.

The value of specific binding is obtained by subtracting the value of non-specific binding from the value of total binding. The binding inhibiting activity of a test compound is represented with a ratio of the value of total binding minus the measurement value obtained when the test compound was added, with respect to the value of specific binding. The concentration of a compound exhibiting a binding inhibiting activity of 50% (i.e., IC₅₀ value) was calculated from the dose reaction curve.

The binding inhibiting activity, represented by the IC₅₀ value, of each of the compounds of Examples 1, 2, 3, 5, 7, 12 14, 23 and 27 was 10 µM or less.

### Test Example 2

The h175-16 cell strains prepared in Test Example 1 were seeded onto a 96-well plate (Coming, #3904) at 3 x 10⁴ cell/well, and cultured for 24 hours on a CO₂ incubator (cell seeding plate).

A calcium kit buffer containing 0.1% BSA [20 mM HEPES, 115 mM NaCl, 5.4 mM KCl, 0.8 mM MgCl₂, 1.8 mM CaCl₂, 13.8 mM D-glucose, pH 7.4, with an attached calcium kit produced by Dojin] was prepared and kept warm at 37°C. Then, the medium in the wells of the cell seeding plate was removed, and 50 µl of the calcium kit buffer was added to each well. 50 µl 250 mM Probenecid (with an attached calcium kit produced by Dojin), 80 µl 5% Pluronic F-127 (with an attached calcium kit produced by Dojin) and 25 µl Fluo-3 AM (powder with an attached calcium kit produced by Dojin was dissolved in DMSO at 1 mg/ml) were added to the calcium kit buffer containing 5 ml 0.1% BSA (an amount for measuring one plate) to prepare a loading solution. 50 µl of the loading solution was added to each well to allow a reaction at 37°C for 1 hour. Thus, Fluo-3 AM was loaded onto the cells. Then, the solution in each well was removed, and 100 µl of the calcium kit buffer containing 1.25 mM Probenecid and 0.1% BSA was added to each well.

50 µl of a test compound diluted with the calcium kit buffer containing 0.1% BSA was added to each well, and stirred by a plate shaker. A cell plate, and a ligand plate provided with human metastin (40-54) (AnyGen) diluted to 400 pM with the calcium kit buffer containing 0.1% BSA, were set to the Fluorometric Imaging Plate Reader (FLIPR, Molecular Devices), and 50 µl was added to each well using an automatic divider in the FLIPR. A change in the calcium concentration in the cells, which corresponded to the ligand response, was measured by a CCD camera in the FLIPR as a change in the amount of fluorescence of Fluo-3. The obtained change in the amount of fluorescence was plotted against the concentration of the test compound, and the compound concentration at which 50% of the change in the amount of fluorescence (i.e., IC₅₀ value) was inhibited was calculated.

The IC₅₀ value (antagonist activity), obtained by this test, of each of the compounds of Examples 3, 12, 14, 23 and 27 was 10 µM or less.

From the above, it is understood that the compounds according to the present invention are superb antagonists to a metastin receptor.

### Test Example 3

The binding inhibiting activity of the compounds of the examples was measured in substantially the same manner as in Test Example 1. The binding inhibiting activity, represented by the IC₅₀ value, of each of the compounds of Examples 31, 41, 42, 43, 44, 45, 46, 50, 51, 56, 57, 58, 61, 63, 64, 65, 66, 67, 68, 69, 70, 71, 76, 77, 78, 79, 80, 81, 83, 84, 85, 86, 235, 237, 238, 239, 240, 241, 242, 243, 244, 245, 246, 247, 249, 250, 251, 252, 253, 254, 255, 257, 258, 259, 261, 263, 264, 265, 266, 267, 268, 269, 270, 271, 272, 273, 274, 275, 276, 277, 278, 280, 281, 283, 284, 285, 286, 287, 288, 289, 290, 291, 292, 293, 294, 295, 296, 298, 299, 300, 301, 302, 303, 304, 306, 307, 308, 309, 310, 312, 313, 314, 315, 316, 317, 318, 319, 320, 321, 322, 324, 325, 326, 327, 328, 329, 330, 333, 334, 335, 336, 337, 338 and 339 was 10 µM or less.

From the above, it is understood that the compounds according to the present invention are superb antagonists to a metastin receptor.

### Test Example 4

### Suppression of the blood LH level of castrated rats

The compound of Example 303 was intravenously administered to male castrated rats, and the blood LH level was measured.

Male Wistar rats (Japan SLC, Inc.), which were castrated 7 days before the experiment and were 11 weeks old at the time of the experiment, were used. To the test animals (n = 8), the compound of the example, which was dispersed to have a final concentration of 0.5 mmol/L with 20% DMSO-40% PEG400, was intravenously administered in an amount of 0.22 mg/kg (0.8 ml/kg) every 10 minutes, 5 times in succession. To the control animals (n = 8), only 20% DMSO-40% PEG400 used as a dispersion medium was intravenously administered in an amount of 0.8 ml/kg every 10 minutes, 5 times in succession. Immediately before the administration, and 25, 45, 60, 90, 120 and 180 minutes after the start of administration, blood was sampled from the external jugular vein as an aprotinin-EDTA plasma sample, and immediately frozen for storage. The LH concentration in the plasma was measured by radioimmunoassay.

FIG 1 shows the LH concentration (average value ± standard deviation) in each administration group.

The start time of the administration was set to 0 (represented with the arrow). In the control group, substantially no change was observed in the blood LH concentration even after the administration. By contrast, in the group to which the compound was administered, the blood LH concentration decreased to 68% of that of the control group 60 minutes after the start of administration (P < 0.01). FIG. 2 especially shows the plasma LH concentration (average value ± standard deviation) immediately before the administration and 60 minutes after the start of administration.

From the above results, it is shown that the compound of Example 303 has a function of significantly decreasing the blood LH concentration when intravenously administered.

### INDUSTRIAL APPLICABILITY

A compound according to the present invention has superb metastin receptor antagonist activity, gonadotropic hormone (e.g., FSH, LH, etc.) secretion suppression activity, sex hormone [e.g., androgen (e.g., testosterone, androstenedione, etc.), estrogen (e.g., estradiol, estrone, etc.), progesterone, etc.] secretion suppression activity, and the like; is low reduced in toxicity; and has very little side effects. Therefore, a compound according to the present invention or a prodrug thereof is useful as a safe pharmaceutical agent; for example, a metastin receptor antagonist (including an inverse agonist and a partial agonist), a gonadal function regulator, a gonadotropic hormone secretion suppressor, a sex hormone secretion suppressor, an ovulation inhibitor, an ovarium function regulator, or the like; more specifically, for example, a medicament such as a prophylactic or therapeutic agent for hormone-dependent cancer (e.g., prostate cancer, breast cancer, ovarian cancer, endometrial cancer, etc.), benign prostatomegaly (BPH), infertility, endometriosis, precocious puberty, uterine myoma or the like, a contraceptive, a follicle maturing inhibitor, a menstrual cycle-suspending agent or the like. A compound according to the present invention or a prodrug thereof is also useful for regulating ovulation, and is usable for infertility treatment such as, for example, IVF (in vitro fertilization), artificial insemination or the like.

## Claims

1. A compound represented by the formula: 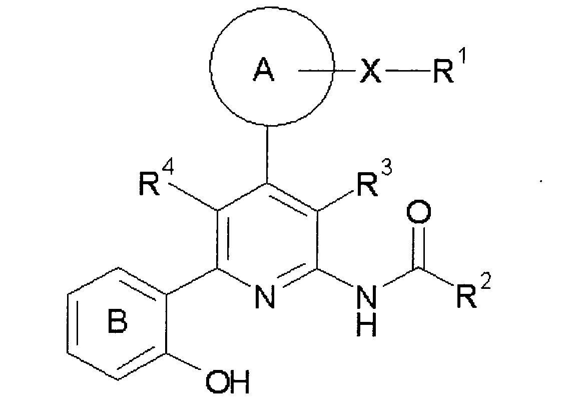 or a salt thereof, wherein
Ring A represents a 5- to 8-membered homocyclic or heterocyclic group optionally having a substituent other than a group represented by formula -X-R¹ wherein X represents a bond or a spacer, and R¹ represents optionally substituted amino or an optionally substituted nitrogen-containing heterocyclic group; and the group represented by formula -X-R¹ may be located at any position on Ring A;
Ring B represents a benzene ring optionally having a substituent other than hydroxy;
R² represents an optionally substituted homocyclic or heterocyclic group; and
R³ and R⁴ independently represent a hydrogen atom, cyano, acyl or an optionally substituted hydrocarbon group;
with proviso that the compound or the salt thereof excludes N-[4-(3-aminophenyl)-3-cyano-6-(2-hydroxyphenyl)pyridin-2-yl] thiophene-2-carboxamide, [(3-{3-cyano-6-(2-hydroxyphenyl)-2-[(2-thienylcarbonyl) amino]pyridin-4-yl}phenyl)amino](oxo)acetic acid ethylester, N-(3-cyano-6-(2-hydroxyphenyl)-4-{3-[methoxyacetyl)amino]phenyl}pyridin-2-yl) thiophene-2-carboxamide, N-(3-{3-cyano-6-(2-hydroxyphenyl)-2-[(2-thienylcarbonyl) amino]pyridin-4-yl}phenyl)-5-oxotetrahydrofuran-2-carboxamide, N-[3-(cyano-6-(2-hydroxyphenyl)-4-(3-{[(5-oxotetrahydrofuran-2-yl)carbonyl]amino} phenyl)pyridin-2-yl]isoxazole-5-carboxamide, and N- [3-cyano-6-(2-hydroxyphenyl)-4-(3-{[(5-oxotetrahydrofuran-2-yl)carbonyl]amino} phenyl)pyridin-2-yl] -2-furanamide).

2. The compound according to claim 1, wherein Ring A is a benzene ring optionally having halogen.

3. The compound according to claim 1, wherein the group of the formula: 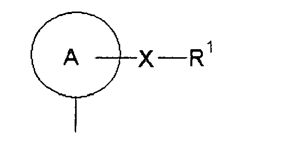 is a group represented by the formula: 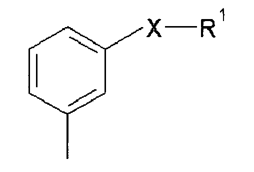 wherein each letter has the same significance as in claim 1.

4. The compound according to claim 1, wherein X is a group represented by the formula: 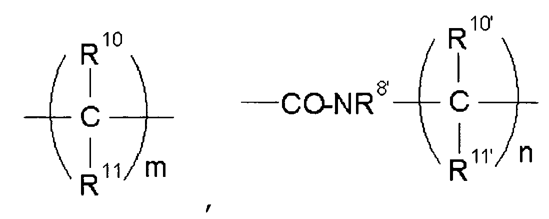 or 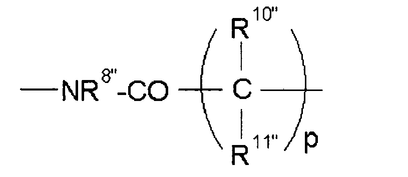 wherein
R^{8'}, R^{8"}, R¹⁰, R^{10'}, R^{10"}, R¹¹, R^{11'} and R^{11"} independently represent a hydrogen atom, an optionally substituted hydrocarbon group, or an optionally substituted heterocyclic group;
m represents an integer of 1 to 8; and
n and p independently represent an integer of 0 to 6 with proviso that if m, n and p are each 2 or greater, R¹⁰, R¹¹, R^{10'}, R^{11'}, R^{10"} and R^{11"} in the repeat unit may each be the same or different.

5. The compound according to claim 1, wherein R¹ is an optionally substituted 5-to 7-membered nitrogen-containing heterocyclic group.

6. The compound according to claim 1, wherein R¹ is an optionally substituted non-aromatic, 5- to 7-membered nitrogen-containing heterocyclic group.

7. The compound according to claim 5, wherein X is a group represented by a bond or the formula: 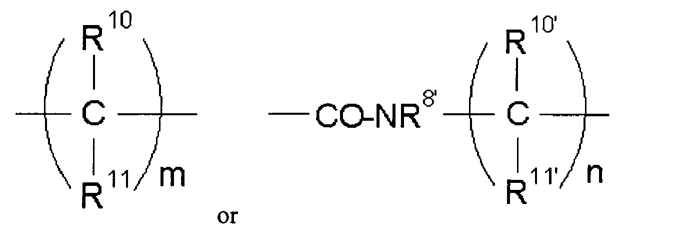 wherein
R^{8'}, R¹⁰, R^{10'}, R¹¹ and R^{11'} independently represent a hydrogen atom, an optionally substituted hydrocarbon group, or an optionally substituted heterocyclic group;
m represents an integer of 1 to 8; and
n represents an integer of 0 to 6 with proviso that if m and n are each 2 or greater, R¹⁰, R¹¹, R^{10'} and R^{11'} in the repeat unit may each be the same or different.

8. The compound according to claim 1, wherein Ring B is a benzene ring having halogen other than hydroxy.

9. The compound according to claim 1, wherein the group of the formula: 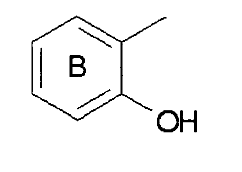 is a group represented by the formula: 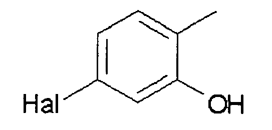 wherein Hal represents halogen.

10. The compound according to claim 1, wherein R² is an optionally substituted aromatic group.

11. The compound according to claim 1, wherein R³ is cyano.

12. The compound according to claim 1, which is represented by the formula: 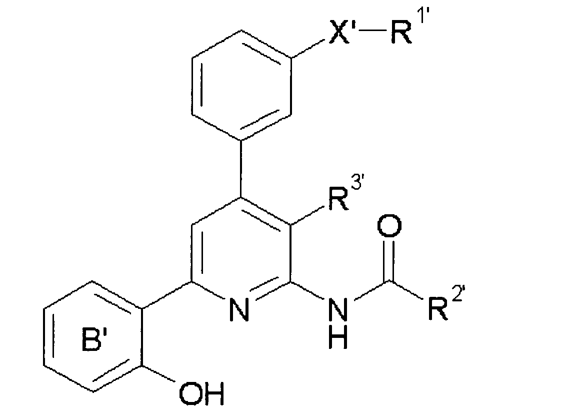 wherein Ring B' represents a benzene ring optionally having halogen other than hydroxy;
X' represents a group expressed by a bond or the formula: 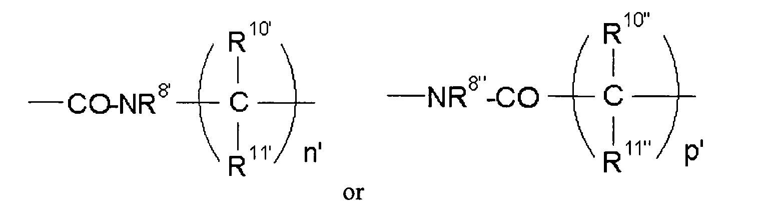 wherein R^{8'}, R^{8"}, R^{10'}, R^{10"}, R^{11'} and R^{11"} independently represent a hydrogen atom, an optionally substituted hydrocarbon group, or an optionally substituted heterocyclic group; and
n' and p' independently represent an integer of 1 to 4 with proviso that if n' and p' are each 2 or greater, R^{10'}, R^{11'} R^{10"} and R^{11"} in the repeat unit may each be the same or different;
R^{1'} represents optionally substituted amino or an optionally substituted 5- to 7-membered nitrogen-containing heterocyclic group;
R^{2'} represents an optionally substituted 5- to 10-membered homocyclic or heterocyclic group; and
R^{3'} represents a hydrogen atom or cyano.

13. The compound according to claim 12, which is represented by the formula: 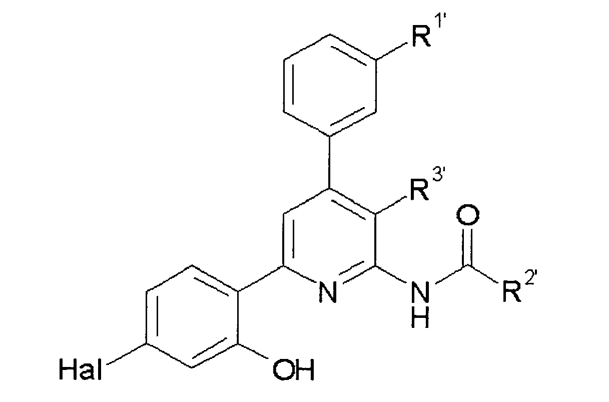 wherein Hal represents halogen, and R^{1'} represents an optionally substituted 5- to 7-membered nitrogen-containing heterocyclic group.

14. N-[4-(3-{[(2-aminoethyl)amino]carbonyl}phenyl)-3-cyano-6-(4-fluoro-2-hydroxyphenyl)pyridin-2-yl]thiophene-2-carboxamide hydrochloride;
N-[4-[3-({[(2S)-2-aminopropyl]amino}carbonyl)phenyl]-6-(4-chloro-2-hydroxyphenyl)-3-cyanopyridin-2-yl]isoxazole-5-carboxamide hydrochloride;
N-[3-cyano-6-(4-fluoro-2-hydroxyphenyl)-4-(3-piperazin-1-ylphenyl) pyridin-2-yl]-2-furamide hydrochloride;
N-[3-cyano-6-(4-fluoro-2-hydroxyphenyl)-4-(3-(((2R)-pyrrolidin-2-ylacetyl) amino)phenyl)pyridin-2-yl]-2-furamide hydrochloride; or
N-[3-cyano-4-[3-(1,4-diazepan-1-ylmethyl)phenyl]-6-(4-fluoro-2-hydroxyphenyl)pyridin-2-yl]-2-furamide dihydrochloride.

15. A prodrug of the compound according to claim 1.

16. A pharmaceutical agent comprising the compound according to claim 1 or a prodrug thereof.

17. The pharmaceutical agent according to claim 16, which is a metastin receptor antagonist.

18. The pharmaceutical agent according to claim 17, which is a gonadal function regulator.

19. The pharmaceutical agent according to claim 17, which is a gonadotropic hormone secretion suppressor and/or sex hormone secretion suppressor.

20. The pharmaceutical agent according to claim 16, which is a prophylactic or treating agent for hormone-dependent cancer, benign prostatomegaly, endometriosis, precocious puberty or uterine myoma.

21. A gonadal function regulator, comprising a compound represented by the formula: 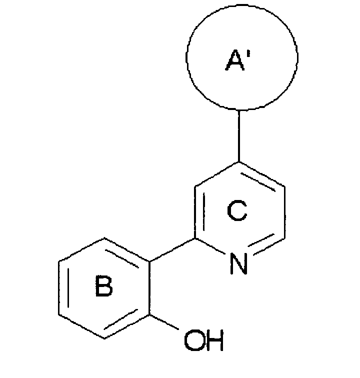 or a salt thereof, wherein:
Ring A' represents an optionally substituted 5- to 8-membered homocyclinc or heterocyclic group;
Ring B represents a benzene ring optionally having a substitutent other than hydroxy; and
Ring C represents an optionally substituted pyridine ring, which may optionally be condensed.

22. A medicament according to claim 21, which is a gonadotropic hormone secretion suppressor and/or sex hormone secretion suppressor.


Sequence Listing

<110> Takeda Pharmaceutical Company Limited

<120> Pyridyl-phenol compounds and use thereof

<130> G07-0147

<140> PCT/JP2006/316129
<141> 2006-08-10

<150> JP2005-232884
<151> 2005-08-11

<150> JP2005-368313
<151> 2005-12-21

<160> 6

<210> 1
<211> 398
<212> PRT
<213> Homo sapiens

<400> 1
Met His Thr Val Ala Thr Ser Gly Pro Asn Ala Ser Trp Gly Ala Pro
                5                   10                  15
Ala Asn Ala Ser Gly Cys Pro Gly Cys Gly Ala Asn Ala Ser Asp Gly
            20                  25                  30
Pro Val Pro Ser Pro Arg Ala Val Asp Ala Trp Leu Val Pro Leu Phe
        35                  40                  45
Phe Ala Ala Leu Met Leu Leu Gly Leu Val Gly Asn Ser Leu Val Ile
    50                  55                  60
Tyr Val Ile Cys Arg His Lys Pro Met Arg Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Gly Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu

145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Ala Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Ala Ala Met Leu Arg His Leu Gly Arg Val Ala Val Arg Pro Ala Pro
225                 230                 235                 240
Ala Asp Ser Ala Leu Gln Gly Gln Val Leu Ala Glu Arg Ala Gly Ala
                245                 250                 255
Val Arg Ala Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ala Gly Ser Trp His Pro Arg Ser Tyr Ala Ala Tyr Ala Leu
    290                 295                 300
Lys Thr Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Arg Arg
                325                 330                 335
Val Cys Pro Cys Ala Pro Arg Arg Pro Arg Arg Pro Arg Arg Pro Gly
            340                 345                 350
Pro Ser Asp Pro Ala Ala Pro His Ala Glu Leu His Arg Leu Gly Ser
        355                 360                 365
His Pro Ala Pro Ala Arg Ala Gln Lys Pro Gly Ser Ser Gly Leu Ala
    370                 375                 380
Ala Arg Gly Leu Cys Val Leu Gly Glu Asp Asn Ala Pro Leu
385                 390                 395

<210> 2
<211> 1194
<212> DNA
<213> Homo sapiens

<400> 2
atgcacaccg tggctacgtc cggacccaac gcgtcctggg gggcaccggc caacgcctcc      60
ggctgcccgg gctgtggcgc caacgcctcg gacggcccag tcccttcgcc gcgggccgtg     120
gacgcctggc tcgtgccgct cttcttcgcg gcgctgatgc tgctgggcct ggtggggaac     180
tcgctggtca tctacgtcat ctgccgccac aagccgatgc ggaccgtgac caacttctac     240
atcgccaacc tggcggccac ggacgtgacc ttcctcctgt gctgcgtccc cttcacggcc     300
ctgctgtacc cgctgcccgg ctgggtgctg ggcgacttca tgtgcaagtt cgtcaactac     360

atccagcagg tctcggtgca ggccacgtgt gccactctga ccgccatgag tgtggaccgc 420
tggtacgtga cggtgttccc gttgcgcgcc ctgcaccgcc gcacgccccg cctggcgctg 480
gctgtcagcc tcagcatctg ggtaggctct gcggcggtgt ctgcgccggt gctcgccctg 540
caccgcctgt cacccgggcc gcgcgcctac tgcagtgagg ccttccccag ccgcgccctg 600
gagcgcgcct tcgcactgta caacctgctg gcgctgtacc tgctgccgct gctcgccacc 660
tgcgcctgct atgcggccat gctgcgccac ctgggccggg tcgccgtgcg ccccgcgccc 720
gccgatagcg ccctgcaggg gcaggtgctg gcagagcgcg caggcgccgt gcgggccaag 780
gtctcgcggc tggtggcggc cgtggtcctg ctcttcgccg cctgctgggg ccccatccag 840
ctgttcctgg tgctgcaggc gctgggcccc gcgggctcct ggcacccacg cagctacgcc 900
gcctacgcgc ttaagacctg ggctcactgc atgtcctaca gcaactccgc gctgaacccg 960
ctgctctacg ccttcctggg ctcgcacttc cgacaggcct tccgccgcgt ctgcccctgc 1020
gcgccgcgcc gcccccgccg cccccgccgg cccggaccct cggaccccgc agccccacac 1080
gcggagctgc accgcctggg gtcccacccg gcccccgcca gggcgcagaa gccagggagc 1140
agtgggctgg ccgcgcgcgg gctgtgcgtc ctgggggagg acaacgcccc tctc 1194

<210> 3
<211> 396
<212> PRT
<213> Norvegicus rattus

<400> 3
Met Ala Ala Glu Ala Thr Leu Gly Pro Asn Val Ser Trp Trp Ala Pro
                5                   10                  15
Ser Asn Ala Ser Gly Cys Pro Gly Cys Gly Val Asn Ala Ser Asp Gly
            20                  25                  30
Pro Gly Ser Ala Pro Arg Pro Leu Asp Ala Trp Leu Val Pro Leu Phe
        35                  40                  45
Phe Ala Ala Leu Met Leu Leu Gly Leu Val Gly Asn Ser Leu Val Ile
    50                  55                  60
Phe Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Thr Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Thr Val Ser Leu Ser Ile Trp Val Gly Ser Ala Ala Val Ser Ala Pro
                165                 170                 175
Val Leu Ala Leu His Arg Leu Ser Pro Gly Pro His Thr Tyr Cys Ser

            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Leu
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Gly Pro Gln Arg Gln Arg Arg Pro His Ala Ser Ala
            340                 345                 350
His Ser Asp Arg Ala Ala Pro His Ser Val Pro His Ser Arg Ala Ala
        355                 360                 365
His Pro Val Arg Val Arg Thr Pro Glu Pro Gly Asn Pro Val Val Arg
    370                 375                 380
Ser Pro Ser Val Gln Asp Glu His Thr Ala Pro Leu
385                 390                 395

<210> 4
<211> 1188
<212> DNA
<213> Norvegicus rattus

<400> 4
atggccgcag aggcgacgtt gggtccgaac gtgagctggt gggctccgtc caacgcttcg 60
ggatgcccgg gctgcggtgt caatgcctcg gatggcccag gctccgcgcc aaggcccctg 120
gatgcctggc tggtgcccct gtttttcgct gccctaatgt tgctggggct agtcgggaac 180
tcactggtca tcttcgttat ctgccgccac aagcacatgc agaccgtcac caatttctac 240
atcgctaacc tggcggccac agatgtcact ttccttctgt gctgcgtacc cttcaccgcg 300
ctcctctatc cgctgcccac ctgggtgctg ggagacttca tgtgcaaatt cgtcaactac 360
atccagcagg tctcggtgca agccacatgt gccactttga cagccatgag tgtggaccgc 420
tggtacgtga ctgtgttccc gctgcgtgca cttcaccgcc gcactccgcg cctggccctg 480
actgtcagcc ttagcatctg ggtgggttcc gcagctgttt ccgccccggt gctggctctg 540
caccgcctgt cgcccgggcc tcacacctac tgcagtgagg cgtttcccag ccgtgccctg 600

gagcgcgctt tcgcgctcta caacctgctg gccctatacc tgctgccgct gctcgccacc 660
tgcgcctgct acggtgccat gctgcgccac ctgggccgcg ccgctgtacg ccccgcaccc 720
actgatggcg ccctgcaggg gcagctgcta gcacagcgcg ctggagcagt gcgcaccaag 780
gtctcccggc tggtggccgc tgtcgtcctg ctcttcgccg cctgctgggg cccgatccag 840
ctgttcctgg tgcttcaagc cctgggcccc tcgggggcct ggcaccctcg aagctatgcc 900
gcctacgcgc tcaagatctg ggctcactgc atgtcctaca gcaattctgc gctcaacccg 960
ctgctctatg ccttcctggg ttcccacttc agacaggcct tctgccgcgt gtgcccctgc 1020
ggcccgcaac gccagcgtcg gccccacgcg tcagcgcact cggaccgagc cgcaccccat 1080
agtgtgccgc acagccgggc tgcgcaccct gtccgggtca ggacccccga gcctgggaac 1140
cctgtggtgc gctcgccctc tgttcaggat gaacacactg ccccactc 1188

<210> 5
<211> 396
<212> PRT
<213> Mus musculus

<400> 5
Met Ala Thr Glu Ala Thr Leu Ala Pro Asn Val Thr Trp Trp Ala Pro
1               5                   10                  15
Ser Asn Ala Ser Gly Cys Pro Gly Cys Gly Val Asn Ala Ser Asp Asp
            20                  25                  30
Pro Gly Ser Ala Pro Arg Pro Leu Asp Ala Trp Leu Val Pro Leu Phe
        35                  40                  45
Phe Ala Thr Leu Met Leu Leu Gly Leu Val Gly Asn Ser Leu Val Ile
    50                  55                  60
Tyr Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Ala Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Thr Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr

210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Val
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Cys Arg Gln Arg Gln Arg Arg Pro His Thr Ser Ala
            340                 345                 350
His Ser Asp Arg Ala Ala Thr His Thr Val Pro His Ser Arg Ala Ala
        355                 360                 365
His Pro Val Arg Ile Arg Ser Pro Glu Pro Gly Asn Pro Val Val Arg
    370                 375                 380
Ser Pro Cys Ala Gln Ser Glu Arg Thr Ala Ser Leu
385                 390                 395

<210> 6
<211> 1188
<212> DNA
<213> Mus musculus

<400> 6
atggccaccg aggcgacatt ggctcccaat gtgacctggt gggctccgtc caacgcttca    60
ggatgcccag gctgcggtgt caacgcctcg gatgacccag gctctgcgcc aaggcccctg   120
gatgcctggc tggttcccct gtttttcgct acactcatgt tgcttgggct ggtcggaaac   180
tcattggtca tctacgttat ctgccgccac aagcacatgc agacagttac caacttctac   240
atcgctaacc tggctgccac agacgtcact ttcctactgt gctgcgtgcc cttcaccgca   300
ctcctctacc cgctgcccgc ctgggtgctg ggagacttca tgtgcaaatt cgtcaactac   360
atccagcagg tctcggtgca agccacatgt gccactctga cggccatgag tgtggaccgc   420
tggtatgtga ctgtgttccc gctgcgtgca cttcaccgcc gcactccgcg cctggccctg   480
gctgtcagcc tcagcatctg ggtggggtca gcagctgtgt ccgcccccgt gctggccctg   540
caccgcctgt cgccagggcc tcgcacctac tgcagcgagg cgtttcccag ccgcgccctg   600
gagcgcgcct tcgcgctcta caacctgctg gctctatatc tgctgccgct gctcgccacc   660
tgcgcctgct acggcgccat gctgcgccac ctgggccgtg cggctgtacg ccccgcaccc   720
actgacggcg ccctgcaggg acagctgcta gcacagcgcg ccggagcagt gcgcaccaag   780
gtctcccggc tggtggccgc tgtcgtcctg ctcttcgccg cctgctgggg cccgatccag   840

ctgttcctgg tgcttcaagc cctgggcccc tcgggggcct ggcaccctcg aagctatgcc 900
gcctacgcgg tcaagatctg ggctcactgc atgtcctaca gcaactcggc gctcaatccg 960
ctgctctatg ccttcctggg ttcacacttc agacaggcct tctgccgcgt gtgcccctgc 1020
tgccggcaac gccagcgccg gccccacacg tcagcgcact cggaccgagc tgcaactcac 1080
actgtgccgc acagccgtgc tgcgcaccct gtgcggatca ggagcccgga gcctgggaac 1140
cctgtggtgc gctcgccctg cgctcagagt gaacgcactg cctcactc 1188